# EUROPEAN PATENT APPLICATION

(11) **EP 2 053 126 A1**
(43) Date of publication of application: **29.04.2009**
(21) Application number: 09001462.2
(22) Date of filing: 27.07.1999
(51) Int. Cl.: C12N 15/31, C07K 14/315, C07K 16/12, G01N 33/50, A61K 39/09, C12Q 1/68

(54) **Streptococcus pneumoniae proteins and nucleic acid molecules**

(30) Priority: 27.07.1998 GB 9816337; 19.03.1999 US 125164
(62) Divisional of application: 07000636.6
(71) Applicant: Sanofi Pasteur Limited, Toronto, ON M2R 3T4 (CA)
(72) Inventor: Gilbert, Christophe Francois Guy, 69622 Villeurbanne Cedex (FR); Hansbro, Philip Michael, Newcastle, NSW 200 (AU)
(74) Representative: Chapman, Paul William

(57) **Abstract**

Novel protein antigens from *Streptococcus pneumoniae* are disclosed, together with nucleic acid sequences encoding them. Their use in vaccines and in screening methods is also described.

SEQUENCE LISTING

<110> Microbial Technics Limited
      Gilbert, Christophe FG
      Hansbro, Philip M

<120> Streptococcus pneumoniae proteins and nucleic acid molecules

<130> P21129EP5

<140> PCT/GB99/02451
<141> 1999-07-27

<150> GB 9816337.1
<151> 1998-03-27

<150> US 60/125164
<151> 1999-03-19

<160> 388

<170> PatentIn Ver. 2.1

<210> 1
<211> 207
<212> PRT
<213> Streptococcus pneumoniae

<400> 1
Met Glu Glu Leu Val Thr Leu Asp Cys Leu Phe Ile Asp Arg Thr Lys
  1               5                  10                  15

Ile Glu Ala Asn Ala Asn Lys Tyr Ser Phe Val Trp Lys Lys Thr Thr
             20                  25                  30

Glu Lys Phe Ser Ala Lys Leu Gln Glu Gln Ile Gln Val Tyr Phe Gln
         35                  40                  45

Glu Glu Ile Thr Pro Leu Leu Ile Lys Tyr Ala Met Phe Asp Lys Lys
     50                  55                  60

Gln Lys Arg Gly Tyr Lys Glu Ser Ala Lys Asn Leu Ala Asn Trp His
 65                  70                  75                  80

Tyr Asn Asp Lys Glu Asp Ser Tyr Thr His Pro Asp Gly Trp Tyr Tyr
                 85                  90                  95

Arg Phe His His Thr Lys Tyr Gln Lys Thr Gln Thr Asp Phe Gln Gln
            100                 105                 110

Glu Ile Lys Val Tyr Tyr Ala Asp Glu Pro Glu Ser Ala Pro Gln Lys
        115                 120                 125

Gly Leu Tyr Met Asn Glu Arg Tyr Gln Asn Leu Lys Ala Lys Glu Cys
    130                 135                 140

Gln Ala Leu Leu Ser Pro Gln Gly Arg Gln Ile Phe Ala Gln Arg Lys
145                 150                 155                 160

Ile Asp Val Glu Pro Val Phe Gly Gln Ile Lys Ala Ser Leu Gly Tyr
                165                 170                 175

Lys Arg Cys Asn Leu Arg Gly Lys Arg Gln Val Arg Ile Asp Met Gly
            180                 185                 190

Leu Val Leu Met Ala Asn Asn Leu Leu Lys Tyr Ser Lys Met Lys
        195                 200                 205

<210> 2
<211> 2233
<212> PRT
<213> Streptococcus pneumoniae

<400> 2
Met Gly Lys Gly His Trp Asn Arg Lys Arg Val Tyr Ser Ile Arg Lys
  1               5                  10                  15

Phe Ala Val Gly Ala Cys Ser Val Met Ile Gly Thr Cys Ala Val Leu
             20                  25                  30

Leu Gly Gly Asn Ile Ala Gly Glu Ser Val Val Tyr Ala Asp Glu Thr
         35                  40                  45

Leu Ile Thr His Thr Ala Glu Lys Pro Lys Glu Glu Lys Met Ile Val
     50                  55                  60

Glu Glu Lys Ala Asp Lys Ala Leu Glu Thr Lys Asn Ile Val Glu Arg
 65                  70                  75                  80

Thr Glu Gln Ser Glu Pro Ser Ser Thr Glu Ala Ile Ala Ser Glu Lys
                 85                  90                  95

Lys Glu Asp Glu Ala Val Thr Pro Lys Glu Glu Lys Val Ser Ala Lys
            100                 105                 110

Pro Glu Glu Lys Ala Pro Arg Ile Glu Ser Gln Ala Ser Asn Gln Glu
        115                 120                 125

Lys Pro Leu Lys Glu Asp Ala Lys Ala Val Thr Asn Glu Glu Val Asn
    130                 135                 140

Gln Met Ile Glu Asp Arg Lys Val Asp Phe Asn Gln Asn Trp Tyr Phe
145                 150                 155                 160

Lys Leu Asn Ala Asn Ser Lys Glu Ala Ile Lys Pro Asp Ala Asp Val
                165                 170                 175

Ser Thr Trp Lys Lys Leu Asp Leu Pro Tyr Asp Trp Ser Ile Phe Asn
            180                 185                 190

Asp Phe Asp His Glu Ser Pro Ala Gln Asn Glu Gly Gly Gln Leu Asn
        195                 200                 205

Gly Gly Glu Ala Trp Tyr Arg Lys Thr Phe Lys Leu Asp Glu Lys Asp
    210                 215                 220

Leu Lys Lys Asn Val Arg Leu Thr Phe Asp Gly Val Tyr Met Asp Ser
225                 230                 235                 240

Gln Val Tyr Val Asn Gly Gln Leu Val Gly His Tyr Pro Asn Gly Tyr

245                 250                 255

Asn Gln Phe Ser Tyr Asp Ile Thr Lys Tyr Leu Gln Lys Asp Gly Arg
            260                 265                 270

Glu Asn Val Ile Ala Val His Ala Val Asn Lys Gln Pro Ser Ser Arg
        275                 280                 285

Trp Tyr Ser Gly Ser Gly Ile Tyr Arg Asp Val Thr Leu Gln Val Thr
    290                 295                 300

Asp Lys Val His Val Glu Lys Asn Gly Thr Thr Ile Leu Thr Pro Lys
305                 310                 315                 320

Leu Glu Glu Gln Gln His Gly Lys Val Glu Thr His Val Thr Ser Lys
                325                 330                 335

Ile Val Asn Thr Asp Asp Lys Asp His Glu Leu Val Ala Glu Tyr Gln
            340                 345                 350

Ile Val Glu Arg Gly Gly His Ala Val Thr Gly Leu Val Arg Thr Ala
        355                 360                 365

Ser Arg Thr Leu Lys Ala His Glu Ser Thr Ser Leu Asp Ala Ile Leu
    370                 375                 380

Glu Val Glu Arg Pro Lys Leu Trp Thr Val Leu Asn Asp Lys Pro Ala
385                 390                 395                 400

Leu Tyr Glu Leu Ile Thr Arg Val Tyr Arg Asp Gly Gln Leu Val Asp
                405                 410                 415

Ala Lys Lys Asp Leu Phe Gly Tyr Arg Tyr Tyr His Trp Thr Pro Asn
            420                 425                 430

Glu Gly Phe Ser Leu Asn Gly Glu Arg Ile Lys Phe His Gly Val Ser
        435                 440                 445

Leu His His Asp His Gly Ala Leu Gly Ala Glu Glu Asn Tyr Lys Ala
    450                 455                 460

Glu Tyr Arg Arg Leu Lys Gln Met Lys Glu Met Gly Val Asn Ser Ile
465                 470                 475                 480

Arg Thr Thr His Asn Pro Ala Ser Glu Gln Thr Leu Gln Ile Ala Ala
                485                 490                 495

Glu Leu Gly Leu Leu Val Gln Glu Glu Ala Phe Asp Thr Trp Tyr Gly
            500                 505                 510

Gly Lys Lys Pro Tyr Asp Tyr Gly Arg Phe Phe Glu Lys Asp Ala Thr
        515                 520                 525

His Pro Glu Ala Arg Lys Gly Glu Lys Trp Ser Asp Phe Asp Leu Arg
    530                 535                 540

Thr Met Val Glu Arg Gly Lys Asn Asn Pro Ala Ile Phe Met Trp Ser
545                 550                 555                 560

Ile Gly Asn Glu Ile Gly Glu Ala Asn Gly Asp Ala His Ser Leu Ala
                565                 570                 575

Thr Val Lys Arg Leu Val Lys Val Ile Lys Asp Val Asp Lys Thr Arg
            580                 585                 590

Tyr Val Thr Met Gly Ala Asp Lys Phe Arg Phe Gly Asn Gly Ser Gly
        595                 600                 605

Gly His Glu Lys Ile Ala Asp Glu Leu Asp Ala Val Gly Phe Asn Tyr
    610                 615                 620

Ser Glu Asp Asn Tyr Lys Ala Leu Arg Ala Lys His Pro Lys Trp Leu
625                 630                 635                 640

Ile Tyr Gly Ser Glu Thr Ser Ser Ala Thr Arg Thr Arg Gly Ser Tyr
                645                 650                 655

Tyr Arg Pro Glu Arg Glu Leu Lys His Ser Asn Gly Pro Glu Arg Asn
            660                 665                 670

Tyr Glu Gln Ser Asp Tyr Gly Asn Asp Arg Val Gly Trp Gly Lys Thr
        675                 680                 685

Ala Thr Ala Ser Trp Thr Phe Asp Arg Asp Asn Ala Gly Tyr Ala Gly
    690                 695                 700

Gln Phe Ile Trp Thr Gly Thr Asp Tyr Ile Gly Glu Pro Thr Pro Trp
705                 710                 715                 720

His Asn Gln Asn Gln Thr Pro Val Lys Ser Ser Tyr Phe Gly Ile Val
                725                 730                 735

Asp Thr Ala Gly Ile Pro Lys His Asp Phe Tyr Leu Tyr Gln Ser Gln
            740                 745                 750

Trp Val Ser Val Lys Lys Lys Pro Met Val His Leu Leu Pro His Trp
        755                 760                 765

Asn Trp Glu Asn Lys Glu Leu Ala Ser Lys Val Ala Asp Ser Glu Gly
    770                 775                 780

Lys Ile Pro Val Arg Ala Tyr Ser Asn Ala Ser Ser Val Glu Leu Phe
785                 790                 795                 800

Leu Asn Gly Lys Ser Leu Gly Leu Lys Thr Phe Asn Lys Lys Gln Thr
                805                 810                 815

Ser Asp Gly Arg Thr Tyr Gln Glu Gly Ala Asn Ala Asn Glu Leu Tyr
            820                 825                 830

Leu Glu Trp Lys Val Ala Tyr Gln Pro Gly Thr Leu Glu Ala Ile Ala
        835                 840                 845

Arg Asp Glu Ser Gly Lys Glu Ile Ala Arg Asp Lys Ile Thr Thr Ala
    850                 855                 860

Gly Lys Pro Ala Ala Val Arg Leu Ile Lys Glu Asp His Ala Ile Ala
865                 870                 875                 880

Ala Asp Gly Lys Asp Leu Thr Tyr Ile Tyr Tyr Glu Ile Val Asp Ser
                885                 890                 895

Gln Gly Asn Val Val Pro Thr Ala Asn Asn Leu Val Arg Phe Gln Leu
            900                 905                 910

His Gly Gln Gly Gln Leu Val Gly Val Asp Asn Gly Glu Gln Ala Ser
        915                 920                 925

Arg Glu Arg Tyr Lys Ala Gln Ala Asp Gly Ser Trp Ile Arg Lys Ala
    930                 935                 940

Phe Asn Gly Lys Gly Val Ala Ile Val Lys Ser Thr Glu Gln Ala Gly
945                 950                 955                 960

Lys Phe Thr Leu Thr Ala His Ser Asp Leu Leu Lys Ser Asn Gln Val
                965                 970                 975

Thr Val Phe Thr Gly Lys Lys Glu Gly Gln Glu Lys Thr Val Leu Gly
            980                 985                 990

Thr Glu Val Pro Lys Val Gln Thr Ile Ile Gly Glu Ala Pro Glu Met
        995                 1000                1005

Pro Thr Thr Val Pro Phe Val Tyr Ser Asp Gly Ser Arg Ala Glu Arg
    1010                1015                1020

Pro Val Thr Trp Ser Ser Val Asp Val Ser Lys Pro Gly Ile Val Thr
1025                1030                1035                1040

Val Lys Gly Met Ala Asp Gly Arg Glu Val Glu Ala Arg Val Glu Val
                1045                1050                1055

Ile Ala Leu Lys Ser Glu Leu Pro Val Val Lys Arg Ile Ala Pro Asn
            1060                1065                1070

Thr Asp Leu Asn Ser Val Asp Lys Ser Val Ser Tyr Val Leu Ile Asp
        1075                1080                1085

Gly Ser Val Glu Glu Tyr Glu Val Asp Lys Trp Glu Ile Ala Glu Glu
    1090                1095                1100

Asp Lys Ala Lys Leu Ala Ile Pro Gly Ser Arg Ile Gln Ala Thr Gly
1105                1110                1115                1120

Tyr Leu Glu Gly Gln Pro Ile His Ala Thr Leu Val Val Glu Glu Gly
                1125                1130                1135

Asn Pro Ala Ala Pro Ala Val Pro Thr Val Thr Val Gly Gly Glu Ala
            1140                1145                1150

Val Thr Gly Leu Thr Ser Gln Lys Pro Met Gln Tyr Arg Thr Leu Ala
        1155                1160                1165

Tyr Gly Ala Lys Leu Pro Glu Val Thr Ala Ser Ala Lys Asn Ala Ala
    1170                1175                1180

Val Thr Val Leu Gln Ala Ser Ala Ala Asn Gly Met Arg Ala Ser Ile
1185                1190                1195                1200

Phe Ile Gln Pro Lys Asp Gly Gly Pro Leu Gln Thr Tyr Ala Ile Gln
                1205                1210                1215

Phe Leu Glu Glu Ala Pro Lys Ile Ala His Leu Ser Leu Gln Val Glu

1220                 1225                 1230

Lys Ala Asp Ser Leu Lys Glu Asp Gln Thr Val Lys Leu Ser Val Arg
    1235                 1240                 1245

Ala His Tyr Gln Asp Gly Thr Gln Ala Val Leu Pro Ala Asp Lys Val
    1250                 1255                 1260

Thr Phe Ser Thr Ser Gly Glu Gly Glu Val Ala Ile Arg Lys Gly Met
1265                 1270                 1275                 1280

Leu Glu Leu His Lys Pro Gly Ala Val Thr Leu Asn Ala Glu Tyr Glu
                1285                 1290                 1295

Gly Ala Lys Asp Gln Val Glu Leu Thr Ile Gln Ala Asn Thr Glu Lys
            1300                 1305                 1310

Lys Ile Ala Gln Ser Ile Arg Pro Val Asn Val Val Thr Asp Leu His
        1315                 1320                 1325

Gln Glu Pro Ser Leu Pro Ala Thr Val Thr Val Glu Tyr Asp Lys Gly
    1330                 1335                 1340

Phe Pro Lys Thr His Lys Val Thr Trp Gln Ala Ile Pro Lys Glu Lys
1345                 1350                 1355                 1360

Leu Asp Ser Tyr Gln Thr Phe Glu Val Leu Gly Lys Val Glu Gly Ile
                1365                 1370                 1375

Asp Leu Glu Ala Arg Ala Lys Val Ser Val Glu Gly Ile Val Ser Val
            1380                 1385                 1390

Glu Glu Val Ser Val Thr Thr Pro Ile Ala Glu Ala Pro Gln Leu Pro
        1395                 1400                 1405

Glu Ser Val Arg Thr Tyr Asp Ser Asn Gly His Val Ser Ser Ala Lys
    1410                 1415                 1420

Val Ala Trp Asp Ala Ile Arg Pro Glu Gln Tyr Ala Lys Glu Gly Val
1425                 1430                 1435                 1440

Phe Thr Val Asn Gly Arg Leu Glu Gly Thr Gln Leu Thr Thr Lys Leu
                1445                 1450                 1455

His Val Arg Val Ser Ala Gln Thr Glu Gln Gly Ala Asn Ile Ser Asp
            1460                 1465                 1470

Gln Trp Thr Gly Ser Glu Leu Pro Leu Ala Phe Ala Ser Asp Ser Asn
        1475                 1480                 1485

Pro Ser Asp Pro Val Ser Asn Val Asn Asp Lys Leu Ile Ser Tyr Asn
    1490                 1495                 1500

Asn Gln Pro Ala Asn Arg Trp Thr Asn Trp Asn Arg Thr Asn Pro Glu
1505                 1510                 1515                 1520

Ala Ser Val Gly Val Leu Phe Gly Asp Ser Gly Ile Leu Ser Lys Arg
                1525                 1530                 1535

Ser Val Asp Asn Leu Ser Val Gly Phe His Glu Asp His Gly Val Gly
            1540                 1545                 1550

Val Pro Lys Ser Tyr Val Ile Glu Tyr Tyr Val Gly Lys Thr Val Pro
        1555                1560                1565

Thr Ala Pro Lys Asn Pro Ser Phe Val Gly Asn Glu Asp His Val Phe
    1570                1575                1580

Asn Asp Ser Ala Asn Trp Lys Pro Val Thr Asn Leu Lys Ala Pro Ala
1585                1590                1595                1600

Gln Leu Lys Ala Gly Glu Met Asn His Phe Ser Phe Asp Lys Val Glu
                1605                1610                1615

Thr Tyr Ala Val Arg Ile Arg Met Val Lys Ala Asp Asn Lys Arg Gly
            1620                1625                1630

Thr Ser Ile Thr Glu Val Gln Ile Phe Ala Lys Gln Val Ala Ala Ala
        1635                1640                1645

Lys Gln Gly Gln Thr Arg Ile Gln Val Asp Gly Lys Asp Leu Ala Asn
    1650                1655                1660

Phe Asn Pro Asp Leu Thr Asp Tyr Tyr Leu Glu Ser Val Asp Gly Lys
1665                1670                1675                1680

Val Pro Ala Val Thr Ala Ser Val Ser Asn Asn Gly Leu Ala Thr Val
                1685                1690                1695

Val Pro Ser Val Arg Glu Gly Glu Pro Val Arg Val Ile Ala Lys Ala
            1700                1705                1710

Glu Asn Gly Asp Ile Leu Gly Glu Tyr Arg Leu His Phe Thr Lys Asp
        1715                1720                1725

Lys Ser Leu Leu Ser His Lys Pro Val Ala Ala Val Lys Gln Ala Arg
    1730                1735                1740

Leu Leu Gln Val Gly Gln Ala Leu Glu Leu Pro Thr Lys Val Pro Val
1745                1750                1755                1760

Tyr Phe Thr Gly Lys Asp Gly Tyr Glu Thr Lys Asp Leu Thr Val Glu
                1765                1770                1775

Trp Glu Glu Val Pro Ala Glu Asn Leu Thr Lys Ala Gly Gln Phe Thr
            1780                1785                1790

Val Arg Gly Arg Val Leu Gly Ser Asn Leu Val Ala Glu Ile Thr Val
        1795                1800                1805

Arg Val Thr Asp Lys Leu Gly Glu Thr Leu Ser Asp Asn Pro Asn Tyr
    1810                1815                1820

Asp Glu Asn Ser Asn Gln Ala Phe Ala Ser Ala Thr Asn Asp Ile Asp
1825                1830                1835                1840

Lys Asn Ser His Asp Arg Val Asp Tyr Leu Asn Asp Gly Asp His Ser
                1845                1850                1855

Glu Asn Arg Arg Trp Thr Asn Trp Ser Pro Thr Pro Ser Ser Asn Pro
            1860                1865                1870

Glu Val Ser Ala Gly Val Ile Phe Arg Glu Asn Gly Lys Ile Val Glu
        1875                1880                1885

Arg Thr Val Thr Gln Gly Lys Val Gln Phe Phe Ala Asp Ser Gly Thr
    1890                1895                1900

Asp Ala Pro Ser Lys Leu Val Leu Glu Arg Tyr Val Gly Pro Glu Phe
1905                1910                1915                1920

Glu Val Pro Thr Tyr Tyr Ser Asn Tyr Gln Ala Tyr Asp Ala Asp His
                1925                1930                1935

Pro Phe Asn Asn Pro Glu Asn Trp Glu Ala Val Pro Tyr Arg Ala Asp
            1940                1945                1950

Lys Asp Ile Ala Ala Gly Asp Glu Ile Asn Val Thr Phe Lys Ala Ile
        1955                1960                1965

Lys Ala Lys Ala Met Arg Trp Arg Met Glu Arg Lys Ala Asp Lys Ser
    1970                1975                1980

Gly Val Ala Met Ile Glu Met Thr Phe Leu Ala Pro Ser Glu Leu Pro
1985                1990                1995                2000

Gln Glu Ser Thr Gln Ser Lys Ile Leu Val Asp Gly Lys Glu Leu Ala
                2005                2010                2015

Asp Phe Ala Glu Asn Arg Gln Asp Tyr Gln Ile Thr Tyr Lys Gly Gln
            2020                2025                2030

Arg Pro Lys Val Ser Val Glu Glu Asn Asn Gln Val Ala Ser Thr Val
        2035                2040                2045

Val Asp Ser Gly Glu Asp Ser Phe Pro Val Leu Val Arg Leu Val Ser
    2050                2055                2060

Glu Ser Gly Lys Gln Val Lys Glu Tyr Arg Ile His Leu Thr Lys Glu
2065                2070                2075                2080

Lys Pro Val Ser Glu Lys Thr Val Ala Ala Val Gln Glu Asp Leu Pro
                2085                2090                2095

Lys Ile Glu Phe Val Glu Lys Asp Leu Ala Tyr Lys Thr Val Glu Lys
            2100                2105                2110

Lys Asp Ser Thr Leu Tyr Leu Gly Glu Thr Arg Val Glu Gln Glu Gly
        2115                2120                2125

Lys Val Gly Lys Glu Arg Ile Phe Thr Ala Ile Asn Pro Asp Gly Ser
    2130                2135                2140

Lys Glu Glu Lys Leu Arg Glu Val Val Glu Val Pro Thr Asp Arg Ile
2145                2150                2155                2160

Val Leu Val Gly Thr Lys Pro Val Ala Gln Glu Ala Lys Lys Pro Gln
                2165                2170                2175

Val Ser Glu Lys Ala Asp Thr Lys Pro Ile Asp Ser Ser Glu Ala Ser
            2180                2185                2190

Gln Thr Asn Lys Ala Gln Leu Pro Ser Thr Gly Ser Ala Ala Ser Gln

2195                2200                2205

Ala Ala Val Ala Ala Gly Leu Thr Leu Leu Gly Leu Ser Ala Gly Leu
    2210                2215                2220

Val Val Thr Lys Gly Lys Lys Glu Asp
2225                2230

<210> 3
<211> 470
<212> PRT
<213> Streptococcus pneumoniae

<400> 3
Met Lys Ile Met Lys Lys Lys Tyr Trp Thr Leu Ala Ile Leu Phe Phe
1               5                   10                  15

Cys Leu Phe Asn Asn Ser Val Thr Ala Gln Glu Ile Pro Lys Asn Leu
            20                  25                  30

Asp Gly Asn Ile Thr His Thr Gln Thr Ser Glu Ser Phe Ser Glu Ser
        35                  40                  45

Asp Glu Lys Gln Val Asp Tyr Ser Asn Lys Asn Gln Glu Glu Val Asp
    50                  55                  60

Gln Asn Lys Phe Arg Ile Gln Ile Asp Lys Thr Glu Leu Phe Val Thr
65                  70                  75                  80

Thr Asp Lys His Leu Glu Lys Asn Cys Cys Lys Leu Glu Leu Glu Pro
                85                  90                  95

Gln Ile Asn Asn Asp Ile Val Asn Ser Glu Ser Asn Asn Leu Leu Gly
            100                 105                 110

Glu Asp Asn Leu Asp Asn Lys Ile Lys Glu Asn Val Ser His Leu Asp
        115                 120                 125

Asn Arg Gly Gly Asn Ile Glu His Asp Lys Asp Asn Leu Glu Ser Ser
    130                 135                 140

Ile Val Arg Lys Tyr Glu Trp Asp Ile Asp Lys Val Thr Gly Gly Gly
145                 150                 155                 160

Glu Ser Tyr Lys Leu Tyr Ser Lys Ser Asn Ser Lys Val Ser Ile Ala
                165                 170                 175

Ile Leu Asp Ser Gly Val Asp Leu Gln Asn Thr Gly Leu Leu Lys Asn
            180                 185                 190

Leu Ser Asn His Ser Lys Asn Tyr Val Pro Asn Lys Gly Tyr Leu Gly
        195                 200                 205

Lys Glu Glu Gly Glu Glu Gly Ile Ile Ser Asp Ile Gln Asp Arg Leu
    210                 215                 220

Gly His Gly Thr Ala Val Val Ala Gln Ile Val Gly Asp Asp Asn Ile
225                 230                 235                 240

Asn Gly Val Asn Pro His Val Asn Ile Asn Val Tyr Arg Ile Phe Gly
                245                 250                 255

Lys Ser Ser Ala Ser Pro Asp Trp Ile Val Lys Ala Ile Phe Asp Ala
            260                 265                 270

Val Asp Asp Gly Asn Asp Ile Ile Asn Leu Ser Thr Gly Gln Tyr Leu
        275                 280                 285

Met Ile Asp Gly Glu Tyr Glu Asp Gly Thr Asn Asp Phe Glu Thr Phe
    290                 295                 300

Leu Lys Tyr Lys Lys Ala Ile Asp Tyr Ala Asn Gln Lys Gly Val Ile
305                 310                 315                 320

Ile Val Ala Ala Leu Gly Asn Asp Ser Leu Asn Val Ser Asn Gln Ser
                325                 330                 335

Asp Leu Leu Lys Leu Ile Ser Ser Arg Lys Lys Val Arg Lys Pro Gly
            340                 345                 350

Leu Val Val Asp Val Pro Ser Tyr Phe Ser Ser Thr Ile Ser Val Gly
        355                 360                 365

Gly Ile Asp Arg Leu Gly Asn Leu Ser Asp Phe Ser Asn Lys Gly Asp
    370                 375                 380

Ser Asp Ala Ile Tyr Ala Pro Ala Gly Ser Thr Leu Ser Leu Ser Glu
385                 390                 395                 400

Leu Gly Leu Asn Asn Phe Ile Asn Ala Glu Lys Tyr Lys Glu Asp Trp
                405                 410                 415

Ile Phe Ser Ala Thr Leu Gly Gly Tyr Thr Tyr Leu Tyr Gly Asn Ser
            420                 425                 430

Phe Ala Ala Pro Lys Val Ser Gly Ala Ile Ala Met Ile Ile Asp Lys
        435                 440                 445

Tyr Lys Leu Lys Asp Gln Pro Tyr Asn Tyr Met Phe Val Lys Lys Phe
    450                 455                 460

Trp Lys Lys His Tyr Gln
465                 470

<210> 4
<211> 335
<212> PRT
<213> Streptococcus pneumoniae

<400> 4
Met Lys Lys Thr Trp Lys Val Phe Leu Thr Leu Val Thr Ala Leu Val
  1               5                  10                  15

Ala Val Val Leu Val Ala Cys Gly Gln Gly Thr Ala Ser Lys Asp Asn
             20                  25                  30

Lys Glu Ala Glu Leu Lys Lys Val Asp Phe Ile Leu Asp Trp Thr Pro
         35                  40                  45

Asn Thr Asn His Thr Gly Leu Tyr Val Ala Lys Glu Lys Gly Tyr Phe
        50                  55                  60

Lys Glu Ala Gly Val Asp Val Asp Leu Lys Leu Pro Pro Glu Glu Ser
65                  70                  75                  80

Ser Ser Asp Leu Val Ile Asn Gly Lys Ala Pro Phe Ala Val Tyr Phe
                85                  90                  95

Gln Asp Tyr Met Ala Lys Lys Leu Glu Lys Gly Ala Gly Ile Thr Ala
            100                 105                 110

Val Ala Ala Ile Val Glu His Asn Thr Ser Gly Ile Ile Ser Arg Lys
        115                 120                 125

Ser Asp Asn Val Ser Ser Pro Lys Asp Leu Val Gly Lys Lys Tyr Gly
    130                 135                 140

Thr Trp Asn Asp Pro Thr Glu Leu Ala Met Leu Lys Thr Leu Val Glu
145                 150                 155                 160

Ser Gln Gly Gly Asp Phe Glu Lys Val Glu Lys Val Pro Asn Asn Asp
                165                 170                 175

Ser Asn Ser Ile Thr Pro Ile Ala Asn Gly Val Phe Asp Thr Ala Trp
            180                 185                 190

Ile Tyr Tyr Gly Trp Asp Gly Ile Leu Ala Lys Ser Gln Gly Val Asp
        195                 200                 205

Ala Asn Phe Met Tyr Leu Lys Asp Tyr Val Lys Glu Phe Asp Tyr Tyr
    210                 215                 220

Ser Pro Val Ile Ile Ala Asn Asn Asp Tyr Leu Lys Asp Asn Lys Glu
225                 230                 235                 240

Glu Ala Arg Lys Val Ile Gln Ala Ile Lys Lys Gly Tyr Gln Tyr Ala
                245                 250                 255

Met Glu His Pro Glu Glu Ala Ala Asp Ile Leu Ile Lys Asn Ala Pro
            260                 265                 270

Glu Leu Lys Glu Lys Arg Asp Phe Val Ile Glu Ser Gln Lys Tyr Leu
        275                 280                 285

Ser Lys Glu Tyr Ala Ser Asp Lys Glu Lys Trp Gly Gln Phe Asp Ala
    290                 295                 300

Ala Arg Trp Asn Ala Phe Tyr Lys Trp Asp Lys Glu Asn Gly Ile Leu
305                 310                 315                 320

Lys Glu Asp Leu Thr Asp Lys Gly Phe Thr Asn Glu Phe Val Lys
                325                 330                 335

<210> 5
<211> 335
<212> PRT
<213> Streptococcus pneumoniae

<400> 5
Met Lys Arg Thr Trp Arg Asn Ser Phe Val Thr Asn Leu Asn Thr Pro
1               5                   10                  15

Phe Met Ile Gly Asn Ile Glu Ile Pro Asn Arg Thr Val Leu Ala Pro
            20                  25                  30

Met Ala Gly Val Thr Asn Ser Ala Phe Arg Thr Ile Ala Lys Glu Leu
        35                  40                  45

Gly Ala Gly Leu Val Val Met Glu Met Val Ser Asp Lys Gly Ile Gln
    50                  55                  60

Tyr Asn Asn Glu Lys Thr Leu His Met Leu His Ile Asp Glu Gly Glu
65                  70                  75                  80

Asn Pro Val Ser Ile Gln Leu Phe Gly Ser Asp Glu Asp Ser Leu Ala
                85                  90                  95

Arg Ala Ala Glu Phe Ile Gln Glu Asn Thr Lys Thr Asp Ile Val Asp
            100                 105                 110

Ile Asn Met Gly Cys Pro Val Asn Lys Ile Val Lys Asn Glu Ala Gly
        115                 120                 125

Ala Met Trp Leu Lys Asp Pro Asp Lys Ile Tyr Ser Ile Ile Asn Lys
    130                 135                 140

Val Gln Ser Val Leu Asp Ile Pro Leu Thr Val Lys Met Arg Thr Gly
145                 150                 155                 160

Trp Ala Asp Pro Ser Leu Ala Val Glu Asn Ala Leu Ala Ala Glu Ala
                165                 170                 175

Ala Gly Val Ser Ala Leu Ala Met His Gly Arg Thr Arg Glu Gln Met
            180                 185                 190

Tyr Thr Gly His Ala Asp Leu Glu Thr Leu Tyr Lys Val Ala Gln Ala
        195                 200                 205

Leu Thr Lys Ile Pro Phe Ile Ala Asn Gly Asp Ile Arg Thr Val Gln
    210                 215                 220

Glu Ala Lys Gln Arg Ile Glu Glu Val Gly Ala Asp Ala Val Met Ile
225                 230                 235                 240

Gly Arg Ala Ala Met Gly Asn Pro Tyr Leu Phe Asn Gln Ile Asn His
                245                 250                 255

Tyr Phe Glu Thr Gly Glu Ile Leu Pro Asp Leu Thr Phe Glu Asp Lys
            260                 265                 270

Met Lys Ile Ala Tyr Glu His Leu Lys Arg Leu Ile Asn Leu Lys Gly
        275                 280                 285

Glu Asn Val Ala Val Arg Glu Phe Arg Gly Leu Ala Pro His Tyr Leu
    290                 295                 300

Arg Gly Thr Ser Gly Ala Ala Lys Leu Arg Gly Ala Ile Ser Gln Ala
305                 310                 315                 320

Ser Thr Leu Ala Glu Ile Glu Thr Leu Leu Gln Leu Glu Lys Ala
                325                 330                 335

<210> 6
<211> 442
<212> PRT
<213> Streptococcus pneumoniae

<400> 6
Met Ile Lys Asn Pro Lys Leu Leu Thr Lys Ser Phe Leu Arg Ser Phe
1               5                   10                  15

Ala Ile Leu Gly Gly Val Gly Leu Val Ile His Ile Ala Ile Tyr Leu
            20                  25                  30

Thr Phe Pro Phe Tyr Tyr Ile Gln Leu Glu Gly Glu Lys Phe Asn Glu
        35                  40                  45

Ser Ala Arg Val Phe Thr Glu Tyr Leu Lys Thr Lys Thr Ser Asp Glu
    50                  55                  60

Ile Pro Ser Leu Leu Gln Ser Tyr Ser Lys Ser Leu Thr Ile Ser Ala
65                  70                  75                  80

His Leu Lys Arg Asp Ile Val Asp Lys Arg Leu Pro Leu Val His Asp
                85                  90                  95

Leu Asp Ile Lys Asp Gly Lys Leu Ser Asn Tyr Ile Val Met Leu Asp
            100                 105                 110

Met Ser Val Ser Thr Ala Asp Gly Lys Gln Val Thr Val Gln Phe Val
        115                 120                 125

His Gly Val Asp Val Tyr Lys Glu Ala Lys Asn Ile Leu Leu Leu Tyr
    130                 135                 140

Leu Pro Tyr Thr Phe Leu Val Thr Ile Ala Phe Ser Phe Val Phe Ser
145                 150                 155                 160

Tyr Phe Tyr Thr Lys Arg Leu Leu Asn Pro Leu Phe Tyr Ile Ser Glu
                165                 170                 175

Val Thr Ser Lys Met Gln Asp Leu Asp Asp Asn Ile Arg Phe Asp Glu
            180                 185                 190

Ser Arg Lys Asp Glu Val Gly Glu Val Gly Lys Gln Ile Asn Gly Met
        195                 200                 205

Tyr Glu His Leu Leu Lys Val Ile Tyr Glu Leu Glu Ser Arg Asn Glu
    210                 215                 220

Gln Ile Val Lys Leu Gln Asn Gln Lys Val Ser Phe Val Arg Gly Ala
225                 230                 235                 240

Ser His Glu Leu Lys Thr Pro Leu Ala Ser Leu Arg Ile Ile Leu Glu
                245                 250                 255

Asn Met Gln His Asn Ile Gly Asp Tyr Lys Asp His Pro Lys Tyr Ile

            260                 265                 270

Ala Lys Ser Ile Asn Lys Ile Asp Gln Met Ser His Leu Leu Glu Glu
        275                 280                 285

Val Leu Glu Ser Ser Lys Phe Gln Glu Trp Thr Glu Cys Arg Glu Thr
    290                 295                 300

Thr Val Lys Pro Val Leu Val Asp Ile Leu Ser Arg Tyr Gln Glu Leu
305                 310                 315                 320

Ala His Ser Ile Gly Val Thr Ile Glu Asn Gln Leu Thr Asp Ala Thr
                325                 330                 335

Arg Val Val Met Ser Leu Arg Ala Leu Asp Lys Val Leu Thr Asn Leu
            340                 345                 350

Ile Ser Asn Ala Ile Lys Tyr Ser Asp Lys Asn Gly Arg Val Ile Ile
        355                 360                 365

Ser Glu Gln Asp Gly Tyr Leu Ser Ile Lys Asn Thr Cys Ala Pro Leu
    370                 375                 380

Ser Asp Gln Glu Leu Glu His Leu Phe Asp Ile Phe Tyr His Ser Gln
385                 390                 395                 400

Ile Val Thr Asp Lys Asp Glu Ser Ser Gly Leu Gly Leu Tyr Ile Val
                405                 410                 415

Asn Asn Ile Leu Glu Ser Tyr Gln Met Asp Tyr Ser Phe Leu Pro Tyr
            420                 425                 430

Glu His Gly Met Glu Phe Lys Ile Ser Leu
        435                 440

<210> 7
<211> 475
<212> PRT
<213> Streptococcus pneumoniae

<400> 7
Met Tyr Leu Gly Asp Leu Met Glu Lys Ala Glu Cys Gly Gln Phe Ser
1               5                   10                  15

Ile Leu Ser Phe Leu Leu Gln Glu Ser Gln Thr Thr Val Lys Ala Val
            20                  25                  30

Met Glu Glu Thr Gly Phe Ser Lys Ala Thr Leu Thr Lys Tyr Val Thr
        35                  40                  45

Leu Leu Asn Asp Lys Ala Leu Asp Ser Gly Leu Glu Leu Ala Ile His
    50                  55                  60

Ser Glu Asp Glu Asn Leu Arg Leu Ser Ile Gly Ala Ala Thr Lys Gly
65                  70                  75                  80

Arg Asp Ile Arg Ser Leu Phe Leu Glu Ser Ala Val Lys Tyr Gln Ile
                85                  90                  95

Leu Val Tyr Leu Leu Tyr His Gln Gln Phe Leu Ala His Gln Leu Ala
            100                 105                 110

Gln Glu Leu Val Ile Ser Glu Ala Thr Leu Gly Arg His Leu Ala Gly
        115                 120                 125

Leu Asn Gln Ile Leu Ser Glu Phe Asp Leu Ser Ile Gln Asn Gly Arg
    130                 135                 140

Trp Arg Gly Pro Glu His Gln Ile His Tyr Phe Tyr Phe Cys Leu Phe
145                 150                 155                 160

Arg Lys Val Trp Ser Ser Gln Glu Trp Glu Gly His Met Gln Lys Pro
                165                 170                 175

Glu Arg Lys Gln Glu Ile Ala Asn Leu Glu Glu Ile Cys Gly Ala Ser
            180                 185                 190

Leu Ser Ala Gly Gln Lys Leu Asp Leu Val Leu Trp Ala His Ile Ser
        195                 200                 205

Gln Gln Arg Leu Arg Val Asn Ala Cys Gln Phe Gln Val Ile Glu Glu
    210                 215                 220

Lys Met Arg Gly Tyr Phe Asp Asn Ile Phe Tyr Leu Arg Leu Leu Arg
225                 230                 235                 240

Lys Val Pro Ser Phe Phe Ala Gly Gln His Ile Pro Leu Gly Val Glu
                245                 250                 255

Asp Gly Glu Met Met Ile Phe Phe Ser Phe Leu Leu Ser His Arg Ile
            260                 265                 270

Leu Pro Leu His Thr Met Glu Tyr Ile Leu Gly Phe Gly Gly Gln Leu
        275                 280                 285

Ala Asp Leu Leu Thr Gln Leu Ile Gln Glu Met Lys Lys Glu Glu Leu
    290                 295                 300

Leu Gly Asp Tyr Thr Glu Asp His Val Thr Tyr Glu Leu Ser Gln Leu
305                 310                 315                 320

Cys Ala Gln Val Tyr Leu Tyr Lys Gly Tyr Ile Leu Gln Asp Arg Tyr
                325                 330                 335

Lys Tyr Gln Leu Glu Asn Arg His Pro Tyr Leu Leu Met Glu His Asp
            340                 345                 350

Phe Lys Glu Thr Ala Glu Glu Ile Phe His Ala Leu Pro Ala Phe Gln
        355                 360                 365

Gln Gly Thr Asp Leu Asp Lys Lys Ile Leu Trp Glu Trp Leu Gln Leu
    370                 375                 380

Ile Glu Tyr Met Ala Glu Asn Gly Gly Gln His Met Arg Ile Gly Leu
385                 390                 395                 400

Asp Leu Thr Ser Gly Phe Leu Val Phe Ser Arg Met Ala Ala Ile Leu
                405                 410                 415

Lys Arg Tyr Leu Glu Tyr Asn Arg Phe Ile Thr Ile Glu Ala Tyr Asp

420                 425                 430

Pro Ser Arg His Tyr Asp Leu Leu Val Thr Asn Asn Pro Ile His Lys
        435                 440                 445

Lys Glu Gln Thr Pro Val Tyr Tyr Leu Lys Asn Asp Leu Asp Met Glu
    450                 455                 460

Asp Leu Val Ala Ile Arg Gln Leu Leu Phe Thr
465                 470                 475

<210> 8
<211> 608
<212> PRT
<213> Streptococcus pneumoniae

<400> 8
Met Glu Phe Ser Lys Lys Thr Arg Glu Leu Ser Ile Lys Lys Met Gln
1               5                   10                  15

Glu Arg Thr Leu Asp Leu Leu Ile Ile Gly Gly Gly Ile Thr Gly Ala
            20                  25                  30

Gly Val Ala Leu Gln Ala Ala Ala Ser Gly Leu Glu Thr Gly Leu Ile
        35                  40                  45

Glu Met Gln Asp Phe Ala Glu Gly Thr Ser Ser Arg Ser Thr Lys Leu
    50                  55                  60

Val His Gly Gly Leu Arg Tyr Leu Lys Gln Phe Asp Val Glu Val Val
65                  70                  75                  80

Ser Asp Thr Val Ser Glu Arg Ala Val Val Gln Gln Ile Ala Pro His
                85                  90                  95

Ile Pro Lys Ser Asp Pro Met Leu Leu Pro Val Tyr Asp Glu Asp Gly
            100                 105                 110

Ala Thr Phe Ser Leu Phe Arg Leu Lys Val Ala Met Asp Leu Tyr Asp
        115                 120                 125

Leu Leu Ala Gly Val Ser Asn Thr Pro Ala Ala Asn Lys Val Leu Ser
    130                 135                 140

Lys Asp Gln Val Leu Glu Arg Gln Pro Asn Leu Lys Lys Glu Gly Leu
145                 150                 155                 160

Val Gly Gly Gly Val Tyr Leu Asp Phe Arg Asn Asn Asp Ala Arg Leu
                165                 170                 175

Val Ile Glu Asn Ile Lys Arg Ala Asn Gln Asp Gly Ala Leu Ile Ala
            180                 185                 190

Asn His Val Lys Ala Glu Gly Phe Leu Phe Asp Glu Ser Gly Lys Ile
        195                 200                 205

Thr Gly Val Val Ala Arg Asp Leu Leu Thr Asp Gln Val Phe Glu Ile
    210                 215                 220

Lys Ala Arg Leu Val Ile Asn Thr Thr Gly Pro Trp Ser Asp Lys Val
225                 230                 235                 240

Arg Asn Leu Ser Asn Lys Gly Thr Gln Phe Ser Gln Met Arg Pro Thr
                245                 250                 255

Lys Gly Val His Leu Val Val Asp Ser Ser Lys Ile Lys Val Ser Gln
            260                 265                 270

Pro Val Tyr Phe Asp Thr Gly Leu Gly Asp Gly Arg Met Val Phe Val
        275                 280                 285

Leu Pro Arg Glu Asn Lys Thr Tyr Phe Gly Thr Thr Asp Thr Asp Tyr
    290                 295                 300

Thr Gly Asp Leu Glu His Pro Lys Val Thr Gln Glu Asp Val Asp Tyr
305                 310                 315                 320

Leu Leu Gly Ile Val Asn Asn Arg Phe Pro Glu Ser Asn Ile Thr Ile
                325                 330                 335

Asp Asp Ile Glu Ser Ser Trp Ala Gly Leu Arg Pro Leu Ile Ala Gly
            340                 345                 350

Asn Ser Ala Ser Asp Tyr Asn Gly Gly Asn Asn Gly Thr Ile Ser Asp
        355                 360                 365

Glu Ser Phe Asp Asn Leu Ile Ala Thr Val Glu Ser Tyr Leu Ser Lys
    370                 375                 380

Glu Lys Thr Arg Glu Asp Val Glu Ser Ala Val Ser Lys Leu Glu Ser
385                 390                 395                 400

Ser Thr Ser Glu Lys His Leu Asp Pro Ser Ala Val Ser Arg Gly Ser
                405                 410                 415

Ser Leu Asp Arg Asp Asp Asn Gly Leu Leu Thr Leu Ala Gly Gly Lys
            420                 425                 430

Ile Thr Asp Tyr Arg Lys Met Ala Glu Gly Ala Met Glu Arg Val Val
        435                 440                 445

Asp Ile Leu Lys Ala Glu Phe Asp Arg Ser Phe Lys Leu Ile Asn Ser
    450                 455                 460

Lys Thr Tyr Pro Val Ser Gly Gly Glu Leu Asn Pro Ala Asn Val Asp
465                 470                 475                 480

Ser Glu Ile Glu Ala Phe Ala Gln Leu Gly Val Ser Arg Gly Leu Asp
                485                 490                 495

Ser Lys Glu Ala His Tyr Leu Ala Asn Leu Tyr Gly Ser Asn Ala Pro
            500                 505                 510

Lys Val Phe Ala Leu Ala His Ser Leu Glu Gln Ala Pro Gly Leu Ser
        515                 520                 525

Leu Ala Asp Thr Leu Ser Leu His Tyr Ala Met Arg Asn Glu Leu Thr
    530                 535                 540

Leu Ser Pro Val Asp Phe Leu Leu Arg Arg Thr Asn His Met Leu Phe

545                 550                 555                 560

Met Arg Asp Ser Leu Asp Ser Ile Val Glu Pro Ile Leu Asp Glu Met
                565                 570                 575

Gly Arg Phe Tyr Asp Trp Thr Glu Glu Glu Lys Ala Thr Tyr Arg Ala
            580                 585                 590

Asp Val Glu Ala Ala Leu Ala Asn Asn Asp Leu Ala Glu Leu Lys Asn
        595                 600                 605

<210> 9
<211> 234
<212> PRT
<213> Streptococcus pneumoniae

<400> 9
Met Met Asn Glu Leu Phe Gly Glu Phe Leu Gly Thr Leu Ile Leu Ile
1               5                   10                  15

Leu Leu Gly Asn Gly Val Val Ala Gly Val Val Leu Pro Lys Thr Lys
            20                  25                  30

Ser Asn Ser Ser Gly Trp Ile Val Ile Thr Met Gly Trp Gly Ile Ala
        35                  40                  45

Val Ala Val Ala Val Phe Val Ser Gly Lys Leu Ser Pro Ala Tyr Leu
    50                  55                  60

Asn Pro Ala Val Thr Ile Gly Val Ala Leu Lys Gly Gly Leu Pro Trp
65                  70                  75                  80

Ala Ser Val Leu Pro Tyr Ile Leu Ala Gln Phe Ala Gly Ala Met Leu
                85                  90                  95

Gly Gln Ile Leu Val Trp Leu Gln Phe Lys Pro His Tyr Glu Ala Glu
            100                 105                 110

Glu Asn Ala Gly Asn Ile Leu Ala Thr Phe Ser Thr Gly Pro Ala Ile
        115                 120                 125

Lys Asp Thr Val Ser Asn Leu Ile Ser Glu Ile Leu Gly Thr Phe Val
    130                 135                 140

Leu Val Leu Thr Ile Phe Ala Leu Gly Leu Tyr Asp Phe Gln Ala Gly
145                 150                 155                 160

Ile Gly Thr Phe Ala Val Gly Thr Leu Ile Val Gly Ile Gly Leu Ser
                165                 170                 175

Leu Gly Gly Thr Thr Gly Tyr Ala Leu Asn Pro Ala Arg Asp Leu Gly
            180                 185                 190

Pro Arg Ile Met His Ser Ile Leu Pro Ile Pro Asn Lys Gly Asp Gly
        195                 200                 205

Asp Trp Ser Tyr Ala Trp Ile Pro Val Val Gly Pro Val Ile Gly Ala
    210                 215                 220

Ala Leu Ala Val Leu Val Phe Ser Leu Phe
225                 230

<210> 10
<211> 136
<212> PRT
<213> Streptococcus pneumoniae

<400> 10
Met Thr Lys Lys Lys Ile Glu Arg Ile Ser Val Ile His Arg Glu Lys
1               5                   10                  15

Ile Leu Trp Leu Lys Trp Tyr Phe Met Arg Asp Lys Glu Gln Pro Lys
            20                  25                  30

Tyr Ser Val Leu Glu Arg Lys Met Phe Asp Ala Ala Lys Asn Gln Asp
        35                  40                  45

Met Leu Ala Tyr Gln Lys Tyr Ala Thr Ile Lys Gln Ile Thr Asp Ile
    50                  55                  60

Arg Val Gln Thr Ser Glu Ala Asp Ile Leu Glu Ala Val Lys Glu Val
65                  70                  75                  80

Tyr Val Tyr Asn His Met Asn Val Ile Gly Ala Cys Gln Arg Ile Leu
                85                  90                  95

Phe Ile Ser Gln Ser Pro Ala Tyr Asp Lys Leu Asn Lys Trp Phe Asn
            100                 105                 110

Ile Tyr Ser Asp Leu Tyr Phe Ser Val Val Pro Leu Pro Lys Met Gly
        115                 120                 125

Val Tyr His Glu Met Val Gly Ile
    130                 135

<210> 11
<211> 1099
<212> PRT
<213> Streptococcus pneumoniae

<400> 11
Met Lys Asn Ser Asn Glu Ala Glu Met Lys Leu Leu Tyr Thr Asp Ile
1               5                   10                  15

Arg Thr Ser Leu Thr Glu Ile Leu Thr Arg Glu Ala Glu Glu Leu Val
            20                  25                  30

Ala Ala Gly Lys Arg Val Phe Tyr Ile Ala Pro Asn Ser Leu Ser Phe
        35                  40                  45

Glu Lys Glu Arg Ala Val Leu Glu Tyr Leu Ser Gln Gln Ala Ser Phe
    50                  55                  60

Ser Ile Thr Val Thr Arg Phe Ala Gln Met Ala Arg Tyr Leu Val Leu
65                  70                  75                  80

Asn Asp Leu Pro Ala Lys Thr Thr Leu Asp Asp Ile Gly Leu Gly Leu
                85                  90                  95

Ala Phe Tyr Lys Cys Leu Ala Glu Leu Asp Pro Lys Asp Leu Arg Val
            100                 105                 110

Tyr Gly Ala Ile Lys Gln Asp Pro Gln Leu Ile Gln Gln Leu Ile Glu
        115                 120                 125

Leu Tyr His Glu Met Thr Lys Ser Gln Met Ser Phe Leu Asp Leu Glu
    130                 135                 140

Asn Leu Thr Asp Glu Asp Lys Arg Ala Asp Leu Leu Leu Ile Phe Glu
145                 150                 155                 160

Lys Val Thr Ala Tyr Leu Asn Gln Gly Gln Leu Ala Gln Glu Ser Gln
                165                 170                 175

Leu Ser His Leu Ile Glu Ala Ile Glu Asn Asp Lys Val Ser Ser Asp
            180                 185                 190

Phe Asn Gln Ile Ala Leu Val Ile Asp Gly Phe Thr Arg Phe Ser Ala
        195                 200                 205

Glu Glu Glu Arg Val Val Asp Leu Leu His Gly Lys Gly Val Glu Ile
    210                 215                 220

Val Ile Gly Ala Tyr Ala Ser Lys Lys Ala Tyr Thr Ser Pro Phe Ser
225                 230                 235                 240

Glu Gly Asn Leu Tyr Gln Ala Ser Val Lys Phe Leu His His Leu Ala
                245                 250                 255

Ser Lys Tyr Gln Thr Pro Ala Gln Asp Cys Ser Gln Thr His Glu Lys
            260                 265                 270

Met Asp Ser Phe Asp Lys Ala Ser Arg Leu Leu Glu Ser Ser Tyr Asp
        275                 280                 285

Phe Ser Glu Leu Ala Leu Asp Val Asp Glu Lys Asp Arg Glu Asn Leu
    290                 295                 300

Gln Ile Trp Ser Cys Leu Thr Gln Lys Glu Glu Leu Glu Leu Val Ala
305                 310                 315                 320

Arg Ser Ile Arg Gln Lys Leu His Glu Asn Ser Asp Leu Ser Tyr Lys
                325                 330                 335

His Phe Arg Ile Leu Leu Gly Asp Val Ala Ser Tyr Gln Leu Ser Leu
            340                 345                 350

Lys Thr Ile Phe Asp Gln Tyr Gln Ile Pro Phe Tyr Leu Gly Arg Ser
        355                 360                 365

Glu Ala Met Ala His His Pro Leu Thr Gln Phe Val Glu Ser Ile Leu
    370                 375                 380

Ala Leu Lys Arg Tyr Arg Phe Arg Gln Glu Asp Leu Ile Asn Leu Leu

385                 390                 395                 400

Arg Thr Asp Leu Tyr Thr Asp Leu Ser Gln Ser Asp Ile Asp Ala Phe
                405                 410                 415

Glu Gln Tyr Ile Arg Tyr Leu Gly Ile Asn Gly Leu Pro Ala Phe Gln
            420                 425                 430

Gln Thr Phe Thr Lys Ser His His Gly Lys Phe Asn Leu Glu Arg Leu
        435                 440                 445

Asn Val Leu Arg Leu Arg Ile Leu Ala Pro Leu Glu Thr Leu Phe Ala
    450                 455                 460

Ser Arg Lys Gln Lys Ala Glu Lys Leu Leu Gln Lys Trp Ser Val Phe
465                 470                 475                 480

Leu Lys Glu Gly Ala Val Thr Lys Gln Leu Gln Asp Leu Thr Thr Thr
                485                 490                 495

Leu Glu Ala Val Glu Gln Glu Arg Gln Ala Glu Val Trp Lys Ala Phe
            500                 505                 510

Cys His Val Leu Glu Gln Phe Ala Thr Val Phe Ala Gly Ser Gln Val
        515                 520                 525

Ser Leu Glu Asp Phe Leu Ala Leu Leu His Ser Gly Met Ser Leu Ser
    530                 535                 540

Gln Tyr Arg Thr Ile Pro Ala Thr Val Asp Thr Val Leu Val Gln Ser
545                 550                 555                 560

Tyr Asp Leu Ile Ala Pro Leu Thr Ala Asp Phe Val Tyr Ala Ile Gly
                565                 570                 575

Leu Thr Gln Asp Asn Leu Pro Lys Ile Ser Gln Asn Thr Ser Leu Leu
            580                 585                 590

Thr Asp Glu Glu Arg Gln Asn Leu Asn Gln Ala Thr Glu Glu Gly Val
        595                 600                 605

Gln Leu Leu Ile Ala Ser Ser Glu Asn Leu Lys Lys Asn Arg Tyr Thr
    610                 615                 620

Met Leu Ser Leu Val Asn Ser Ala Arg Lys Gln Leu Phe Leu Ser Ala
625                 630                 635                 640

Pro Ser Leu Phe Asn Glu Ser Glu Ser Lys Glu Ser Ala Tyr Leu Gln
                645                 650                 655

Glu Leu Ile His Phe Gly Phe Arg Arg Arg Glu Lys Arg Met Asn His
            660                 665                 670

Lys Gly Leu Ser Lys Glu Asp Met Gly Ser Tyr His Ser Leu Leu Ser
        675                 680                 685

Ser Leu Val Ala Tyr His Gln Gln Gly Glu Met Ser Asp Thr Glu Gln
    690                 695                 700

Asp Leu Thr Phe Val Lys Val Leu Ser Arg Val Ile Gly Lys Lys Leu
705                 710                 715                 720

Asp Gln Gln Gly Leu Glu Asn Pro Ala Ile Pro Thr Ser Pro Ser Ser
                725                 730                 735

Lys Thr Leu Ala Lys Asp Thr Leu Gln Ala Leu Tyr Pro Ala Lys Gln
            740                 745                 750

Glu Phe Tyr Leu Ser Thr Ser Gly Leu Thr Glu Phe Tyr Arg Asn Glu
        755                 760                 765

Tyr Ser Tyr Phe Leu Arg Tyr Val Leu Gly Leu Gln Glu Glu Leu Arg
    770                 775                 780

Leu His Pro Asp Ala Arg Ser His Gly Asn Phe Leu His Arg Ile Phe
785                 790                 795                 800

Glu Arg Ala Leu Gln Leu Pro Asn Glu Asp Ser Phe Asp Gln Arg Leu
                805                 810                 815

Glu Gln Ala Ile Gln Glu Thr Ser Gln Glu Arg Glu Phe Glu Ala Ile
            820                 825                 830

Tyr Gln Glu Ser Leu Glu Ala Gln Phe Thr Lys Glu Val Leu Leu Asp
        835                 840                 845

Val Ala Arg Thr Thr Gly His Ile Leu Arg His Asn Pro Ala Ile Glu
    850                 855                 860

Thr Ile Lys Glu Glu Ala Asn Phe Gly Gly Lys Asp Gln Ala Phe Ile
865                 870                 875                 880

Gln Leu Asp Asn Gly Arg Ser Val Phe Val Arg Gly Lys Val Asp Arg
                885                 890                 895

Ile Asp Arg Leu Lys Ala Asn Gly Ala Ile Gly Val Val Asp Tyr Lys
            900                 905                 910

Ser Ser Leu Thr Gln Phe Gln Phe Pro His Phe Phe Asn Gly Leu Asn
        915                 920                 925

Ser Gln Leu Pro Thr Tyr Leu Ala Ala Leu Lys Arg Glu Gly Glu Gln
    930                 935                 940

Asn Phe Phe Gly Ala Met Tyr Leu Glu Met Ala Glu Pro Val Gln Ser
945                 950                 955                 960

Leu Met Ala Val Lys Ser Leu Ala Gly Ala Val Val Glu Ala Ser Lys
                965                 970                 975

Ser Met Lys Tyr Gln Gly Leu Phe Leu Glu Lys Glu Ser Ser Tyr Leu
            980                 985                 990

Gly Glu Phe Tyr Asn Lys Asn Lys Ala Asn Gln Leu Thr Asp Glu Glu
        995                 1000                1005

Phe Gln Leu Leu Leu Asp Tyr Asn Ala Tyr Leu Tyr Lys Lys Ala Ala
    1010                1015                1020

Glu Lys Ile Leu Ala Gly Arg Phe Ala Ile Asn Pro Tyr Thr Glu Asn
1025                1030                1035                1040

Gly Arg Ser Ile Ala Pro Tyr Val Gln Gln His Gln Ala Ile Thr Gly
                1045                1050                1055

Phe Glu Ala Asn Tyr His Leu Gly Gln Ala Arg Phe Leu Glu Lys Leu
            1060                1065                1070

Asp Leu Ala Asp Gly Lys Arg Leu Val Gly Glu Lys Leu Lys Gln Ala
        1075                1080                1085

Trp Leu Glu Lys Ile Arg Glu Glu Leu Asn Arg
    1090                1095

<210> 12
<211> 1216
<212> PRT
<213> Streptococcus pneumoniae

<400> 12
Met Lys Leu Ile Pro Phe Leu Ser Glu Glu Glu Ile Gln Lys Leu Gln
1               5                   10                  15

Glu Ala Glu Ala Asn Ser Ser Lys Glu Gln Lys Lys Thr Ala Glu Gln
            20                  25                  30

Ile Glu Ala Ile Tyr Thr Ser Ala Gln Asn Ile Leu Val Ser Ala Ser
        35                  40                  45

Ala Gly Ser Gly Lys Thr Phe Val Met Ala Glu Arg Ile Leu Asp Gln
    50                  55                  60

Leu Ala Arg Gly Val Glu Ile Ser Gln Leu Phe Ile Ser Thr Phe Thr
65                  70                  75                  80

Val Lys Ala Ala Thr Glu Leu Lys Glu Arg Leu Glu Lys Lys Ile Ser
                85                  90                  95

Lys Lys Ile Gln Glu Thr Asp Asp Val Asp Leu Lys Gln His Leu Gly
            100                 105                 110

Arg Gln Leu Ala Asp Leu Pro Asn Ala Ala Ile Gly Thr Met Asp Ser
        115                 120                 125

Phe Thr Gln Lys Phe Leu Gly Lys His Gly Tyr Leu Leu Asp Ile Ala
    130                 135                 140

Pro Asn Phe Arg Ile Leu Gln Asn Gln Ser Glu Gln Leu Ile Leu Glu
145                 150                 155                 160

Asn Glu Val Phe His Glu Val Phe Glu Ala His Tyr Gln Gly Lys Gln
                165                 170                 175

Lys Glu Thr Phe Ser His Leu Leu Lys Asn Phe Ala Gly Arg Gly Lys
            180                 185                 190

Asp Glu Arg Gly Leu Arg Gln Gln Val Tyr Lys Ile Tyr Asp Phe Leu
        195                 200                 205

Gln Ser Thr Ser Asn Pro Gln Lys Trp Leu Ser Glu Ser Phe Leu Lys
    210                 215                 220

Gly Phe Glu Lys Ala Asp Phe Thr Ser Glu Lys Glu Lys Leu Thr Glu
225                 230                 235                 240

Gln Ile Lys Gln Ala Leu Trp Asp Leu Glu Ser Phe Phe Arg Tyr His
                245                 250                 255

Leu Asp Asn Asp Ala Lys Glu Phe Ala Lys Ala Ala Tyr Leu Glu Asn
            260                 265                 270

Val Gln Leu Ile Leu Asp Glu Ile Gly Ser Leu Asn Gln Glu Ser Asp
        275                 280                 285

Ser Gln Ala Tyr Gln Ala Val Leu Ala Arg Val Val Ala Ile Ser Lys
    290                 295                 300

Glu Lys Asn Gly Arg Ala Leu Thr Asn Ala Ser Arg Lys Ala Asp Leu
305                 310                 315                 320

Lys Pro Leu Ala Asp Ala Tyr Asn Glu Glu Arg Lys Thr Gln Phe Ala
                325                 330                 335

Lys Leu Gly Gln Leu Ser Asp Gln Ile Ala Ile Leu Asp Tyr Gln Glu
            340                 345                 350

Arg Tyr His Gly Asp Thr Trp Lys Leu Ala Lys Thr Phe Gln Ser Phe
        355                 360                 365

Met Ser Asp Phe Val Glu Ala Tyr Arg Gln Arg Lys Arg Gln Glu Asn
    370                 375                 380

Ala Phe Glu Phe Ala Asp Ile Ser His Tyr Thr Ile Glu Ile Leu Glu
385                 390                 395                 400

Asn Phe Pro Gln Val Arg Glu Ser Tyr Gln Glu Arg Phe His Glu Val
                405                 410                 415

Met Val Asp Glu Tyr Gln Asp Thr Asn His Ile Gln Glu Arg Met Leu
            420                 425                 430

Glu Leu Leu Ser Asn Gly His Asn Arg Phe Met Val Gly Asp Ile Lys
        435                 440                 445

Gln Ser Ile Tyr Arg Phe Arg Gln Ala Asp Pro Gln Ile Phe Asn Glu
    450                 455                 460

Lys Phe Gln Arg Tyr Ala Gln Asn Pro Gln Glu Gly Arg Leu Ile Ile
465                 470                 475                 480

Leu Lys Glu Asn Phe Arg Ser Ser Ser Glu Val Leu Ser Ala Thr Asn
                485                 490                 495

Asp Val Phe Glu Arg Leu Met Asp Gln Glu Val Gly Glu Ile Asn Tyr
            500                 505                 510

Asp Asn Lys His Gln Leu Val Phe Ala Asn Thr Lys Leu Thr Pro Asn
        515                 520                 525

Pro Asp Asn Lys Ala Ala Phe Leu Leu Tyr Asp Lys Asp Asp Thr Gly
    530                 535                 540

Glu Glu Glu Glu Ser Gln Thr Glu Thr Lys Leu Thr Gly Glu Met Arg
545                 550                 555                 560

Leu Val Ile Lys Glu Ile Leu Lys Leu His Gln Glu Lys Gly Val Ala
                565                 570                 575

Phe Lys Glu Ile Ala Leu Leu Thr Ser Ser Arg Ser Arg Asn Asp Gln
            580                 585                 590

Ile Leu Leu Ala Leu Ser Glu Tyr Gly Ile Pro Val Lys Thr Asp Gly
        595                 600                 605

Glu Gln Asn Asn Tyr Leu Gln Ser Leu Glu Val Gln Val Met Leu Asp
    610                 615                 620

Thr Leu Arg Val Ile His Asn Pro Leu Gln Asp Tyr Ala Leu Val Ala
625                 630                 635                 640

Leu Met Lys Ser Pro Met Phe Gly Phe Asp Glu Asp Glu Leu Ala Arg
                645                 650                 655

Leu Ser Leu Gln Lys Ala Glu Asp Lys Val His Glu Asn Leu Tyr Glu
            660                 665                 670

Lys Leu Val Asn Ala Gln Lys Met Ala Ser Ser Gln Lys Gly Leu Ile
        675                 680                 685

His Thr Ala Leu Ala Glu Lys Leu Lys Gln Phe Met Asp Ile Leu Ala
    690                 695                 700

Ser Trp Arg Leu Tyr Ala Lys Thr His Ser Leu Tyr Asp Leu Ile Trp
705                 710                 715                 720

Lys Ile Tyr Asn Asp Arg Phe Tyr Tyr Asp Tyr Val Gly Ala Leu Pro
                725                 730                 735

Asn Gly Pro Ala Arg Gln Ala Asn Leu Tyr Ala Leu Ala Leu Arg Ala
            740                 745                 750

Asp Gln Phe Glu Lys Ser Asn Phe Lys Gly Leu Ser Arg Phe Ile Arg
        755                 760                 765

Met Ile Asp Gln Val Leu Glu Ala Gln His Asp Leu Ala Ser Val Ala
    770                 775                 780

Val Ala Pro Pro Lys Asp Ala Val Glu Leu Met Thr Ile His Lys Ser
785                 790                 795                 800

Lys Gly Leu Glu Phe Pro Tyr Val Phe Ile Leu Asn Met Asp Gln Asp
                805                 810                 815

Phe Asn Lys Gln Asp Ser Met Ser Glu Val Ile Leu Ser Arg Gln Asn
            820                 825                 830

Gly Leu Gly Val Lys Tyr Ile Ala Lys Met Glu Thr Gly Ala Val Glu
        835                 840                 845

Asp His Tyr Pro Lys Thr Ile Lys Leu Ser Ile Pro Ser Leu Thr Tyr
    850                 855                 860

Arg Gln Asn Glu Glu Glu Leu Gln Leu Ala Ser Tyr Ser Glu Gln Met

865                 870                 875                 880

Arg Leu Leu Tyr Val Ala Met Thr Arg Ala Glu Lys Lys Leu Tyr Leu
                885                 890                 895

Val Gly Lys Gly Ser Arg Glu Lys Leu Glu Ser Lys Glu Tyr Pro Ala
            900                 905                 910

Ala Lys Asn Gly Lys Leu Asn Ser Asn Thr Arg Leu Gln Ala Arg Asn
        915                 920                 925

Phe Gln Asp Trp Leu Trp Ala Ile Ser Lys Val Phe Thr Lys Asp Lys
    930                 935                 940

Leu Asn Phe Ser Tyr Arg Phe Ile Gly Glu Asp Gln Leu Thr Arg Glu
945                 950                 955                 960

Ala Ile Gly Glu Leu Glu Thr Lys Ser Pro Leu Gln Asp Ser Ser Gln
                965                 970                 975

Ala Asp Asn Arg Gln Ser Asp Thr Ile Lys Glu Ala Leu Glu Met Leu
            980                 985                 990

Lys Glu Val Glu Val Tyr Asn Thr Leu His Arg Ala Ala Ile Glu Leu
        995                 1000                1005

Pro Ser Val Gln Thr Pro Ser Gln Ile Lys Lys Phe Tyr Glu Pro Val
    1010                1015                1020

Met Asp Met Glu Gly Val Glu Ile Ala Gly Gln Gly Gln Ser Val Gly
1025                1030                1035                1040

Lys Lys Ile Ser Phe Asp Leu Pro Asp Phe Ser Thr Lys Glu Lys Val
                1045                1050                1055

Thr Gly Ala Glu Ile Gly Ser Ala Thr His Glu Leu Met Gln Arg Ile
            1060                1065                1070

Asp Leu Ser Gln Gln Leu Thr Leu Ala Ser Leu Thr Glu Thr Leu Lys
        1075                1080                1085

Gln Val Gln Thr Ser Gln Ala Val Arg Asp Lys Ile Asn Leu Asp Lys
    1090                1095                1100

Ile Leu Ala Phe Phe Asp Thr Val Leu Gly Gln Glu Ile Leu Ala Asn
1105                1110                1115                1120

Thr Asp His Leu Tyr Arg Glu Gln Pro Phe Ser Met Leu Lys Arg Asp
                1125                1130                1135

Gln Lys Ser Gln Glu Asp Phe Val Val Arg Gly Ile Leu Asp Gly Tyr
            1140                1145                1150

Leu Leu Tyr Glu Asn Lys Ile Val Leu Phe Asp Tyr Lys Thr Asp Arg
        1155                1160                1165

Tyr Asp Glu Pro Ser Gln Leu Val Asp Arg Tyr Arg Gly Gln Leu Ala
    1170                1175                1180

Leu Tyr Glu Glu Ala Leu Ser Arg Ala Tyr Ser Ile Glu Asn Ile Glu
1185                1190                1195                1200

Lys Tyr Leu Ile Leu Leu Gly Lys Asp Glu Val Gln Val Val Lys Val
                1205                1210                1215

<210> 13
<211> 215
<212> PRT
<213> Streptococcus pneumoniae

<400> 13
Met Glu Leu Ala Arg His Ala Glu Ser Leu Gly Val Asp Ala Ile Ala
1               5                   10                  15

Thr Ile Pro Pro Ile Tyr Phe Arg Leu Pro Glu Tyr Ser Val Ala Lys
            20                  25                  30

Tyr Trp Asn Asp Ile Ser Ser Ala Ala Pro Asn Thr Asp Tyr Val Ile
        35                  40                  45

Tyr Asn Ile Pro Gln Leu Ala Gly Val Ala Leu Thr Pro Ser Leu Tyr
    50                  55                  60

Thr Glu Met Leu Lys Asn Pro Arg Val Ile Gly Val Lys Asn Ser Ser
65                  70                  75                  80

Met Pro Val Gln Asp Ile Gln Thr Phe Val Ser Leu Gly Gly Glu Asp
                85                  90                  95

His Ile Val Phe Asn Gly Pro Asp Glu Gln Phe Leu Gly Gly Arg Leu
            100                 105                 110

Met Gly Ala Arg Ala Gly Ile Gly Gly Thr Tyr Gly Ala Met Pro Glu
        115                 120                 125

Leu Phe Leu Lys Leu Asn Gln Leu Ile Ala Asp Lys Asp Leu Glu Thr
    130                 135                 140

Ala Arg Glu Leu Gln Tyr Ala Ile Asn Ala Ile Ile Gly Lys Leu Thr
145                 150                 155                 160

Ser Ala His Gly Asn Met Tyr Gly Val Ile Lys Glu Val Leu Lys Ile
                165                 170                 175

Asn Glu Gly Leu Asn Ile Gly Ser Val Arg Ser Pro Leu Thr Pro Val
            180                 185                 190

Thr Glu Glu Asp Arg Pro Val Val Glu Ala Ala Ala Ala Leu Ile Arg
        195                 200                 205

Glu Thr Lys Glu Arg Phe Leu
    210                 215

<210> 14
<211> 407

<212> PRT
<213> Streptococcus pneumoniae

<400> 14
Met Tyr Lys Thr Lys Cys Leu Arg Glu Lys Leu Val Leu Phe Leu Lys
1               5                   10                  15

Ile Phe Phe Pro Ile Leu Ile Tyr Gln Phe Ala Asn Tyr Ser Ala Ser
            20                  25                  30

Phe Val Asp Thr Ala Met Thr Gly Gln Tyr Asn Thr Met Asp Leu Ala
        35                  40                  45

Gly Val Ser Met Ala Thr Ser Ile Trp Asn Pro Phe Phe Thr Phe Leu
    50                  55                  60

Thr Gly Ile Val Ser Ala Leu Val Pro Ile Ile Gly His His Leu Gly
65                  70                  75                  80

Arg Gly Lys Lys Glu Glu Val Ala Ser Asp Phe Tyr Gln Phe Ile Tyr
                85                  90                  95

Leu Ala Leu Gly Leu Ser Val Val Leu Leu Gly Met Val Leu Phe Leu
            100                 105                 110

Ala Pro Ile Ile Leu Asn His Ile Gly Leu Glu Ala Ala Val Ala Ala
        115                 120                 125

Val Ala Val Arg Tyr Leu Trp Phe Leu Ser Ile Gly Ile Ile Pro Leu
    130                 135                 140

Leu Leu Phe Ser Val Ile Arg Ser Leu Leu Asp Ser Leu Gly Leu Thr
145                 150                 155                 160

Lys Leu Ser Met Tyr Leu Met Leu Leu Leu Leu Pro Leu Asn Ser Gly
                165                 170                 175

Phe Asn Tyr Leu Leu Ile Tyr Gly Ala Phe Gly Val Pro Glu Leu Gly
            180                 185                 190

Gly Ala Gly Ala Gly Leu Gly Thr Ser Leu Ala Tyr Trp Val Leu Leu
        195                 200                 205

Gly Ile Ser Val Leu Val Leu Phe Lys Gln Glu Lys Leu Lys Ala Leu
    210                 215                 220

His Leu Glu Lys Arg Ile Pro Leu Asn Met Asp Lys Ile Lys Glu Gly
225                 230                 235                 240

Val Arg Leu Gly Leu Pro Ile Gly Gly Thr Val Phe Ala Glu Val Ala
                245                 250                 255

Ile Phe Ser Val Val Gly Leu Ile Met Ala Lys Phe Ser Pro Leu Ile
            260                 265                 270

Ile Ala Ser His Gln Ser Ala Met Asn Phe Ser Ser Leu Met Tyr Ala
        275                 280                 285

Phe Pro Met Ser Ile Ser Ser Ala Met Ala Ile Val Val Ser Tyr Glu
    290                 295                 300

Val Gly Ala Lys Arg Phe Asp Asp Ala Lys Thr Tyr Ile Gly Leu Gly
305                 310                 315                 320

Arg Trp Thr Ala Leu Ile Phe Ala Ala Phe Thr Leu Thr Phe Leu Tyr
                325                 330                 335

Ile Phe Arg Gly Asn Val Ala Ser Leu Tyr Gly Asn Asp Pro Lys Phe
            340                 345                 350

Ile Asp Leu Thr Val Arg Phe Leu Thr Tyr Ser Leu Phe Phe Gln Leu
        355                 360                 365

Ala Asp Thr Phe Ala Ala Pro Leu Gln Gly Ile Leu Arg Gly Tyr Lys
    370                 375                 380

Asp Thr Val Ile Pro Phe Tyr Leu Gly Leu Leu Gly Tyr Trp Gly Val
385                 390                 395                 400

Ala Ile Pro Val Tyr Ala Ile
                405

<210> 15
<211> 189
<212> PRT
<213> Streptococcus pneumoniae

<400> 15
Met Ser Thr Leu Ala Lys Ile Glu Ala Leu Leu Phe Val Ala Gly Glu
  1               5                  10                  15

Asp Gly Ile Arg Val Arg Gln Leu Ala Glu Leu Leu Ser Leu Pro Pro
             20                  25                  30

Thr Gly Ile Gln Gln Ser Leu Gly Lys Leu Ala Gln Lys Tyr Glu Lys
         35                  40                  45

Asp Pro Asp Ser Ser Leu Ala Leu Ile Glu Thr Ser Gly Ala Tyr Arg
     50                  55                  60

Leu Val Thr Lys Pro Gln Phe Ala Glu Ile Leu Lys Glu Tyr Ser Lys
 65                  70                  75                  80

Ala Pro Ile Asn Gln Ser Leu Ser Arg Ala Ala Leu Glu Thr Leu Ser
                 85                  90                  95

Ile Ile Ala Tyr Lys Gln Pro Ile Thr Arg Ile Glu Ile Asp Ala Ile
            100                 105                 110

Arg Gly Val Asn Ser Ser Gly Ala Leu Ala Lys Leu Gln Ala Phe Asp
        115                 120                 125

Leu Ile Lys Glu Asp Gly Lys Lys Glu Val Leu Gly Arg Pro Asn Leu
    130                 135                 140

Tyr Val Thr Thr Asp Tyr Phe Leu Asp Tyr Met Gly Ile Asn His Leu
145                 150                 155                 160

Glu Glu Leu Pro Val Ile Asp Glu Leu Glu Ile Gln Ala Gln Glu Ser
                165                 170                 175

Gln Leu Phe Gly Glu Arg Ile Glu Glu Asp Glu Asn Gln
            180                 185

<210> 16
<211> 311
<212> PRT
<213> Streptococcus pneumoniae

<400> 16
Met Asp Thr Met Ile Ser Arg Phe Phe Arg His Leu Phe Glu Ala Leu
1               5                   10                  15

Lys Ser Leu Lys Arg Asn Gly Trp Met Thr Val Ala Ala Val Ser Ser
            20                  25                  30

Val Met Ile Thr Leu Thr Leu Val Ala Ile Phe Ala Ser Val Ile Phe
        35                  40                  45

Asn Thr Ala Lys Leu Ala Thr Asp Ile Glu Asn Asn Val Arg Val Val
    50                  55                  60

Val Tyr Ile Arg Lys Asp Val Glu Asp Asn Ser Gln Thr Ile Glu Lys
65                  70                  75                  80

Glu Gly Gln Thr Val Thr Asn Asn Asp Tyr His Lys Val Tyr Asp Ser
                85                  90                  95

Leu Lys Asn Met Ser Thr Val Lys Ser Val Thr Phe Ser Ser Lys Glu
            100                 105                 110

Glu Gln Tyr Glu Lys Leu Thr Glu Ile Met Gly Asp Asn Trp Lys Ile
        115                 120                 125

Phe Glu Gly Asp Ala Asn Pro Leu Tyr Asp Ala Tyr Ile Val Glu Ala
    130                 135                 140

Asn Thr Pro Asn Asp Val Lys Thr Ile Ala Glu Asp Ala Lys Lys Ile
145                 150                 155                 160

Glu Gly Val Ser Glu Val Gln Asp Gly Gly Ala Asn Thr Glu Arg Leu
                165                 170                 175

Phe Lys Leu Ala Ser Phe Ile Arg Val Trp Gly Leu Gly Ile Ala Ala
            180                 185                 190

Leu Leu Ile Phe Ile Ala Val Phe Leu Ile Ser Asn Thr Ile Arg Ile
        195                 200                 205

Thr Ile Ile Ser Arg Ser Arg Glu Ile Gln Ile Met Arg Leu Val Gly
    210                 215                 220

Ala Lys Asn Ser Tyr Ile Arg Gly Pro Phe Leu Leu Glu Gly Ala Phe
225                 230                 235                 240

Ile Gly Leu Leu Gly Ala Ile Ala Pro Ser Val Leu Val Phe Ile Val
                245                 250                 255

Tyr Gln Ile Val Tyr Gln Ser Val Asn Lys Ser Leu Val Gly Gln Asn

260                 265                 270

Leu Ser Met Ile Ser Pro Asp Leu Phe Ser Pro Leu Met Ile Ala Leu
        275                 280                 285

Leu Phe Val Ile Gly Val Phe Ile Gly Ser Leu Gly Ser Gly Ile Ser
    290                 295                 300

Met Arg Arg Phe Leu Lys Ile
305                 310

<210> 17
<211> 658
<212> PRT
<213> Streptococcus pneumoniae

<400> 17
Met Lys Lys Val Arg Phe Ile Phe Leu Ala Leu Leu Phe Phe Leu Ala
  1               5                  10                  15

Ser Pro Glu Gly Ala Met Ala Ser Asp Gly Thr Trp Gln Gly Lys Gln
             20                  25                  30

Tyr Leu Lys Glu Asp Gly Ser Gln Ala Ala Asn Glu Trp Val Phe Asp
         35                  40                  45

Thr His Tyr Gln Ser Trp Phe Tyr Ile Lys Ala Asp Ala Asn Tyr Ala
     50                  55                  60

Glu Asn Glu Trp Leu Lys Gln Gly Asp Asp Tyr Phe Tyr Leu Lys Ser
 65                  70                  75                  80

Gly Gly Tyr Met Ala Lys Ser Glu Trp Val Glu Asp Lys Gly Ala Phe
                 85                  90                  95

Tyr Tyr Leu Asp Gln Asp Gly Lys Met Lys Arg Asn Ala Trp Val Gly
            100                 105                 110

Thr Ser Tyr Val Gly Ala Thr Gly Ala Lys Val Ile Glu Asp Trp Val
        115                 120                 125

Tyr Asp Ser Gln Tyr Asp Ala Trp Phe Tyr Ile Lys Ala Asp Gly Gln
    130                 135                 140

His Ala Glu Lys Glu Trp Leu Gln Ile Lys Gly Lys Asp Tyr Tyr Phe
145                 150                 155                 160

Lys Ser Gly Gly Tyr Leu Leu Thr Ser Gln Trp Ile Asn Gln Ala Tyr
                165                 170                 175

Val Asn Ala Ser Gly Ala Lys Val Gln Gln Gly Trp Leu Phe Asp Lys
            180                 185                 190

Gln Tyr Gln Ser Trp Phe Tyr Ile Lys Glu Asn Gly Asn Tyr Ala Asp
        195                 200                 205

Lys Glu Trp Ile Phe Glu Asn Gly His Tyr Tyr Tyr Leu Lys Ser Gly
    210                 215                 220

Gly Tyr Met Ala Ala Asn Glu Trp Ile Trp Asp Lys Glu Ser Trp Phe
225                 230                 235                 240

Tyr Leu Lys Phe Asp Gly Lys Met Ala Glu Lys Glu Trp Val Tyr Asp
                245                 250                 255

Ser His Ser Gln Ala Trp Tyr Tyr Phe Lys Ser Gly Gly Tyr Met Thr
            260                 265                 270

Ala Asn Glu Trp Ile Trp Asp Lys Glu Ser Trp Phe Tyr Leu Lys Ser
        275                 280                 285

Asp Gly Lys Ile Ala Glu Lys Glu Trp Val Tyr Asp Ser His Ser Gln
    290                 295                 300

Ala Trp Tyr Tyr Phe Lys Ser Gly Gly Tyr Met Thr Ala Asn Glu Trp
305                 310                 315                 320

Ile Trp Asp Lys Glu Ser Trp Phe Tyr Leu Lys Ser Asp Gly Lys Ile
                325                 330                 335

Ala Glu Lys Glu Trp Val Tyr Asp Ser His Ser Gln Ala Trp Tyr Tyr
            340                 345                 350

Phe Lys Ser Gly Gly Tyr Met Ala Lys Asn Glu Thr Val Asp Gly Tyr
        355                 360                 365

Gln Leu Gly Ser Asp Gly Lys Trp Leu Gly Gly Lys Thr Thr Asn Glu
    370                 375                 380

Asn Ala Ala Tyr Tyr Gln Val Val Pro Val Thr Ala Asn Val Tyr Asp
385                 390                 395                 400

Ser Asp Gly Glu Lys Leu Ser Tyr Ile Ser Gln Gly Ser Val Val Trp
                405                 410                 415

Leu Asp Lys Asp Arg Lys Ser Asp Asp Lys Arg Leu Ala Ile Thr Ile
            420                 425                 430

Ser Gly Leu Ser Gly Tyr Met Lys Thr Glu Asp Leu Gln Ala Leu Asp
        435                 440                 445

Ala Ser Lys Asp Phe Ile Pro Tyr Tyr Glu Ser Asp Gly His Arg Phe
    450                 455                 460

Tyr His Tyr Val Ala Gln Asn Ala Ser Ile Pro Val Ala Ser His Leu
465                 470                 475                 480

Ser Asp Met Glu Val Gly Lys Lys Tyr Tyr Ser Ala Asp Gly Leu His
                485                 490                 495

Phe Asp Gly Phe Lys Leu Glu Asn Pro Phe Leu Phe Lys Asp Leu Thr
            500                 505                 510

Glu Ala Thr Asn Tyr Ser Ala Glu Glu Leu Asp Lys Val Phe Ser Leu
        515                 520                 525

Leu Asn Ile Asn Asn Ser Leu Leu Glu Asn Lys Gly Ala Thr Phe Lys
    530                 535                 540

Glu Ala Glu Glu His Tyr His Ile Asn Ala Leu Tyr Leu Leu Ala His

545                 550                 555                 560

Ser Ala Leu Glu Ser Asn Trp Gly Arg Ser Lys Ile Ala Lys Asp Lys
                565                 570                 575

Asn Asn Phe Phe Gly Ile Thr Ala Tyr Asp Thr Thr Pro Tyr Leu Ser
            580                 585                 590

Ala Lys Thr Phe Asp Asp Val Asp Lys Gly Ile Leu Gly Ala Thr Lys
        595                 600                 605

Trp Ile Lys Glu Asn Tyr Ile Asp Arg Gly Arg Thr Phe Leu Gly Asn
    610                 615                 620

Lys Ala Ser Gly Met Asn Val Glu Tyr Ala Ser Asp Pro Tyr Trp Gly
625                 630                 635                 640

Glu Lys Ile Ala Ser Val Met Met Lys Ile Asn Glu Lys Leu Gly Gly
                645                 650                 655

Lys Asp

<210> 18
<211> 288
<212> PRT
<213> Streptococcus pneumoniae

<400> 18
Met Lys Lys Val Leu Gln Lys Tyr Trp Ala Trp Ala Phe Val Val Ile
1               5                   10                  15

Pro Leu Leu Leu Gln Ala Ile Phe Phe Tyr Val Pro Met Phe Gln Gly
            20                  25                  30

Ala Phe Tyr Ser Phe Thr Asn Trp Thr Gly Leu Thr Tyr Asn Tyr Lys
        35                  40                  45

Phe Val Gly Leu Asn Asn Phe Lys Leu Leu Phe Met Asp Pro Lys Phe
    50                  55                  60

Met Asn Ala Ile Gly Phe Thr Ala Ile Ile Ala Ile Ala Met Val Val
65                  70                  75                  80

Gly Glu Ile Ala Leu Gly Ile Phe Ile Ala Arg Val Leu Asn Ser Lys
                85                  90                  95

Ile Lys Gly Gln Thr Phe Phe Arg Ala Trp Phe Phe Phe Pro Ala Val
            100                 105                 110

Leu Ser Gly Leu Thr Val Ala Leu Ile Phe Lys Gln Val Phe Asn Tyr
        115                 120                 125

Gly Leu Pro Ala Ile Gly Asn Ala Leu His Ile Glu Phe Phe Gln Thr
    130                 135                 140

Ser Leu Leu Gly Thr Lys Trp Gly Ala Ile Phe Ala Ala Val Phe Val
145                 150                 155                 160

Leu Leu Trp Gln Gly Val Ala Met Pro Ile Ile Ile Phe Leu Ala Gly
                165                 170                 175

Leu Gln Ser Ile Pro Thr Glu Ile Thr Glu Ala Ala Arg Ile Asp Gly
            180                 185                 190

Ala Thr Ser Lys Gln Val Phe Trp Asn Ile Glu Leu Pro Tyr Leu Leu
        195                 200                 205

Pro Ser Val Ser Met Val Phe Ile Leu Ala Leu Lys Gly Gly Leu Thr
    210                 215                 220

Ala Phe Asp Gln Val Phe Ala Met Thr Gly Gly Gly Pro Asn Asn Ala
225                 230                 235                 240

Thr Thr Ser Leu Gly Leu Leu Val Tyr Asn Tyr Ala Phe Lys Asn Asn
                245                 250                 255

Gln Phe Gly Tyr Ala Asn Ala Ile Ala Val Ile Leu Phe Phe Leu Ile
            260                 265                 270

Val Val Ile Ser Ile Ile Gln Leu Arg Val Ser Lys Lys Phe Glu Ile
        275                 280                 285

<210> 19
<211> 278
<212> PRT
<213> Streptococcus pneumoniae

<400> 19
Met Met Lys Gln Asp Glu Arg Lys Ala Leu Ile Gly Lys Tyr Ile Leu
  1               5                  10                  15

Leu Ile Leu Gly Ser Val Leu Ile Leu Val Pro Leu Leu Ala Thr Leu
            20                  25                  30

Phe Ser Ser Phe Lys Pro Thr Lys Asp Ile Val Asp Asn Phe Phe Gly
        35                  40                  45

Phe Pro Thr Asn Phe Thr Trp Asp Asn Phe Ser Arg Leu Leu Ala Asp
    50                  55                  60

Gly Ile Gly Gly Tyr Tyr Trp Asn Ser Val Val Ile Thr Val Leu Ser
65                  70                  75                  80

Leu Leu Ala Val Met Ile Phe Ile Pro Met Ala Ala Tyr Ser Ile Ala
                85                  90                  95

Arg Asn Met Ser Lys Arg Lys Ala Phe Thr Ile Met Tyr Thr Leu Leu
            100                 105                 110

Ile Leu Gly Ile Phe Val Pro Phe Gln Val Ile Met Ile Pro Ile Thr
        115                 120                 125

Val Met Met Ser Lys Leu Gly Leu Ala Asn Thr Phe Gly Leu Ile Leu
    130                 135                 140

Leu Tyr Leu Thr Tyr Ala Ile Pro Gln Thr Leu Phe Leu Tyr Val Gly
145                 150                 155                 160

Tyr Ile Lys Ile Ser Ile Pro Glu Ser Leu Asp Glu Ala Ala Glu Ile
                165                 170                 175

Asp Gly Ala Asn Gln Phe Thr Thr Tyr Phe Arg Ile Ile Phe Pro Met
            180                 185                 190

Met Lys Pro Met His Ala Thr Thr Met Ile Ile Asn Ala Leu Trp Phe
        195                 200                 205

Trp Asn Asp Phe Met Leu Pro Leu Leu Val Leu Asn Arg Asp Ser Lys
    210                 215                 220

Met Trp Thr Leu Pro Leu Phe Gln Tyr Asn Tyr Ala Gly Gln Tyr Phe
225                 230                 235                 240

Asn Asp Tyr Gly Pro Ser Phe Ala Ser Tyr Val Val Gly Ile Ile Ser
                245                 250                 255

Ile Thr Ile Val Tyr Leu Phe Phe Gln Arg His Ile Ile Ser Gly Met
            260                 265                 270

Ser Asn Gly Ala Val Lys
        275

<210> 20
<211> 347
<212> PRT
<213> Streptococcus pneumoniae

<400> 20
Met Lys Ser Ile Leu Gln Lys Met Gly Glu His Pro Met Leu Leu Leu
1               5                   10                  15

Phe Leu Ser Tyr Ser Thr Val Ile Ser Ile Leu Ala Gln Asn Trp Met
            20                  25                  30

Gly Leu Val Ala Ser Val Gly Met Phe Leu Phe Thr Ile Phe Phe Leu
        35                  40                  45

His Tyr Gln Ser Ile Leu Ser His Lys Phe Phe Arg Leu Ile Leu Gln
    50                  55                  60

Phe Val Leu Phe Gly Ser Val Leu Ser Ala Ala Phe Ala Ser Leu Glu
65                  70                  75                  80

His Phe Gln Ile Val Lys Lys Phe Asn Tyr Ala Phe Leu Ser Pro Asn
                85                  90                  95

Met Gln Val Trp His Gln Asn Arg Ala Glu Val Thr Phe Phe Asn Pro
            100                 105                 110

Asn Tyr Tyr Gly Ile Ile Cys Cys Phe Cys Ile Met Ile Ala Phe Tyr
        115                 120                 125

Leu Phe Thr Thr Thr Lys Leu Asn Trp Leu Lys Val Phe Cys Val Ile

130                 135                 140

Ala Gly Phe Val Asn Leu Phe Gly Leu Asn Phe Thr Gln Asn Arg Thr
145                 150                 155                 160

Ala Phe Pro Ala Ile Ile Ala Gly Ala Ile Ile Tyr Leu Phe Thr Thr
                165                 170                 175

Ile Lys Asn Trp Lys Ala Phe Trp Leu Ser Ile Gly Val Phe Ala Ile
            180                 185                 190

Gly Leu Ser Phe Leu Phe Ser Ser Asp Leu Gly Val Arg Met Gly Thr
        195                 200                 205

Leu Asp Ser Ser Met Glu Glu Arg Ile Ser Ile Trp Asp Ala Gly Met
    210                 215                 220

Ala Leu Phe Lys Gln Asn Pro Phe Trp Gly Glu Gly Pro Leu Thr Tyr
225                 230                 235                 240

Met Asn Ser Tyr Pro Arg Ile His Ala Pro Tyr His Glu His Ala His
                245                 250                 255

Ser Leu Tyr Ile Asp Thr Ile Leu Ser Tyr Gly Ile Val Gly Thr Ile
            260                 265                 270

Leu Leu Val Leu Ser Ser Val Ala Pro Val Arg Leu Met Met Asp Met
        275                 280                 285

Ser Gln Glu Ser Gly Lys Arg Pro Ile Ile Gly Leu Tyr Leu Ser Phe
    290                 295                 300

Leu Thr Val Val Ala Val His Gly Ile Phe Asp Leu Ala Leu Phe Trp
305                 310                 315                 320

Ile Gln Ser Gly Phe Ile Phe Leu Leu Val Met Cys Ser Ile Pro Leu
                325                 330                 335

Glu His Arg Met Leu Val Ser Asp Met Thr Asp
            340                 345

<210> 21
<211> 432
<212> PRT
<213> Streptococcus pneumoniae

<400> 21
Met Ser Lys Met Asp Val Gln Lys Ile Ile Ala Pro Met Met Lys Phe
1               5                   10                  15

Val Asn Met Arg Gly Ile Ile Ala Leu Lys Asp Gly Met Leu Ala Ile
            20                  25                  30

Leu Pro Leu Thr Val Val Gly Ser Leu Phe Leu Ile Met Gly Gln Leu
        35                  40                  45

Pro Phe Glu Gly Leu Asn Lys Ser Ile Ala Ser Val Phe Gly Ala Asn
    50                  55                  60

Trp Thr Glu Pro Phe Met Gln Val Tyr Ser Gly Thr Phe Ala Ile Met
65                  70                  75                  80

Gly Leu Ile Ser Cys Phe Ser Ile Ala Tyr Ser Tyr Ala Lys Asn Ser
                85                  90                  95

Gly Val Glu Ala Leu Pro Ala Gly Val Leu Ser Val Ser Ala Phe Phe
            100                 105                 110

Ile Leu Leu Arg Ser Ser Tyr Ile Pro Lys Gln Gly Glu Ala Ile Gly
        115                 120                 125

Asp Ala Ile Ser Lys Val Trp Phe Gly Gly Gln Gly Ile Ile Gly Ala
    130                 135                 140

Ile Ile Ile Gly Leu Val Val Gly Ser Ile Tyr Thr Phe Phe Ile Lys
145                 150                 155                 160

Arg Lys Ile Val Ile Lys Met Pro Glu Gln Val Pro Gln Ala Ile Ala
                165                 170                 175

Lys Gln Phe Glu Ala Met Ile Pro Ala Phe Val Ile Phe Leu Ser Ser
            180                 185                 190

Met Ile Val Tyr Ile Leu Ala Lys Ser Leu Thr Asn Gly Gly Thr Phe
        195                 200                 205

Ile Glu Met Ile Tyr Ser Ala Ile Gln Val Pro Leu Gln Gly Leu Thr
    210                 215                 220

Gly Ser Leu Tyr Gly Ala Ile Gly Ile Ala Phe Phe Ile Ser Phe Leu
225                 230                 235                 240

Trp Trp Phe Gly Val His Gly Gln Ser Val Val Asn Gly Val Val Thr
                245                 250                 255

Ala Leu Leu Leu Ser Asn Leu Asp Ala Asn Lys Ala Met Leu Ala Ser
            260                 265                 270

Ala Asn Leu Ser Leu Glu Asn Gly Ala His Ile Val Thr Gln Gln Phe
        275                 280                 285

Leu Asp Ser Phe Leu Ile Leu Ser Gly Ser Gly Ile Thr Phe Gly Leu
    290                 295                 300

Val Val Ala Met Leu Phe Ala Ala Lys Ser Lys Gln Tyr Gln Ala Leu
305                 310                 315                 320

Gly Lys Val Ala Ala Phe Pro Ala Ile Phe Asn Val Asn Glu Pro Val
                325                 330                 335

Val Phe Gly Phe Pro Ile Val Met Asn Pro Val Met Phe Val Pro Phe
            340                 345                 350

Ile Leu Val Pro Val Leu Ala Ala Val Ile Val Tyr Gly Ala Ile Ala
        355                 360                 365

Thr Gly Phe Met Gln Pro Phe Ser Gly Val Thr Leu Pro Trp Ser Thr
    370                 375                 380

Pro Ala Ile Leu Ser Gly Phe Leu Val Gly Gly Trp Gln Gly Val Ile

385                 390                 395                 400

Thr Gln Leu Val Ile Leu Ala Met Ser Thr Leu Val Tyr Phe Pro Phe
                405                 410                 415

Phe Lys Val Gln Asp Arg Leu Ala Tyr Gln Asn Glu Ile Lys Gln Ser
            420                 425                 430

<210> 22
<211> 100
<212> PRT
<213> Streptococcus pneumoniae

<400> 22
Met Lys Lys Lys Asp Leu Val Asp Gln Leu Val Ser Glu Ile Glu Thr
1               5                   10                  15

Gly Lys Val Arg Thr Leu Gly Ile Tyr Gly His Gly Ala Ser Gly Lys
            20                  25                  30

Ser Thr Phe Ala Gln Glu Leu Tyr Gln Ala Leu Asp Ser Thr Thr Val
        35                  40                  45

Asn Leu Leu Glu Thr Asp Pro Tyr Ile Thr Ser Gly Arg His Leu Val
    50                  55                  60

Val Pro Lys Asp Ala Pro Asn Gln Lys Val Thr Ala Ser Leu Pro Val
65                  70                  75                  80

Ala His Glu Leu Glu Ser Leu Gln Arg Asp Ile Leu Ala Cys Arg Arg
                85                  90                  95

Val Trp Met Ser
            100

<210> 23
<211> 238
<212> PRT
<213> Streptococcus pneumoniae

<400> 23
Met Lys Lys Arg Tyr Leu Val Leu Thr Ala Leu Leu Ala Leu Ser Leu
1               5                   10                  15

Ala Ala Cys Ser Gln Glu Lys Thr Lys Asn Glu Asp Gly Glu Thr Lys
            20                  25                  30

Thr Glu Gln Thr Ala Lys Ala Asp Gly Thr Val Gly Ser Lys Ser Gln
        35                  40                  45

Gly Ala Ala Gln Lys Lys Ala Glu Val Val Asn Lys Gly Asp Tyr Tyr
    50                  55                  60

Ser Ile Gln Gly Lys Tyr Asp Glu Ile Ile Val Ala Asn Lys His Tyr

65                  70                  75                  80

Pro Leu Ser Lys Asp Tyr Asn Pro Gly Glu Asn Pro Thr Ala Lys Ala
                85                  90                  95

Glu Leu Val Lys Leu Ile Lys Ala Met Gln Glu Ala Gly Phe Pro Ile
            100                 105                 110

Ser Asp His Tyr Ser Gly Phe Arg Ser Tyr Glu Thr Gln Thr Lys Leu
        115                 120                 125

Tyr Gln Asp Tyr Val Asn Gln Asp Gly Lys Ala Ala Ala Asp Arg Tyr
    130                 135                 140

Ser Ala Arg Pro Gly Tyr Ser Glu His Gln Thr Gly Leu Ala Phe Asp
145                 150                 155                 160

Val Ile Gly Thr Asp Gly Asp Leu Val Thr Glu Glu Lys Ala Ala Gln
                165                 170                 175

Trp Leu Leu Asp His Ala Ala Asp Tyr Gly Phe Val Val Arg Tyr Leu
            180                 185                 190

Lys Gly Lys Glu Lys Glu Thr Gly Tyr Met Ala Glu Glu Trp His Leu
        195                 200                 205

Arg Tyr Val Gly Lys Glu Ala Lys Glu Ile Ala Ala Ser Gly Leu Ser
    210                 215                 220

Leu Glu Glu Tyr Tyr Gly Phe Glu Gly Gly Asp Tyr Val Asp
225                 230                 235

<210> 24
<211> 425
<212> PRT
<213> Streptococcus pneumoniae

<400> 24
Met Arg Glu Pro Asp Phe Leu Asn His Phe Leu Lys Lys Gly Tyr Phe
1               5                   10                  15

Lys Lys His Ala Lys Ala Val Leu Ala Leu Ser Gly Gly Leu Asp Ser
            20                  25                  30

Met Phe Leu Phe Lys Val Leu Ser Thr Tyr Gln Lys Glu Leu Glu Ile
        35                  40                  45

Glu Leu Ile Leu Ala His Val Asn His Lys Gln Arg Ile Glu Ser Asp
    50                  55                  60

Trp Glu Glu Lys Glu Leu Arg Lys Leu Ala Ala Glu Ala Glu Leu Pro
65                  70                  75                  80

Ile Tyr Ile Ser Asn Phe Ser Gly Glu Phe Ser Glu Ala Arg Ala Arg
                85                  90                  95

Asn Phe Arg Tyr Asp Phe Phe Gln Glu Val Met Lys Lys Thr Gly Ala
            100                 105                 110

Thr Ala Leu Val Thr Ala His His Ala Asp Asp Gln Val Glu Thr Ile
        115                 120                 125

Phe Met Arg Leu Ile Arg Gly Thr Arg Leu Arg Tyr Leu Ser Gly Ile
    130                 135                 140

Lys Glu Lys Gln Val Val Gly Glu Ile Glu Ile Ile Arg Pro Phe Leu
145                 150                 155                 160

His Phe Gln Lys Lys Asp Phe Pro Ser Ile Phe His Phe Glu Asp Thr
                165                 170                 175

Ser Asn Gln Glu Asn His Tyr Phe Arg Asn Arg Ile Arg Asn Ser Tyr
            180                 185                 190

Leu Pro Glu Leu Glu Lys Glu Asn Pro Arg Phe Arg Asp Ala Ile Leu
        195                 200                 205

Gly Ile Gly Asn Glu Ile Leu Asp Tyr Asp Leu Ala Ile Ala Glu Leu
    210                 215                 220

Ser Asn Asn Ile Asn Val Glu Asp Leu Gln Gln Leu Phe Ser Tyr Ser
225                 230                 235                 240

Glu Ser Thr Gln Arg Val Leu Leu Gln Thr Tyr Leu Asn Arg Phe Pro
                245                 250                 255

Asp Leu Asn Leu Thr Lys Ala Gln Phe Ala Glu Val Gln Gln Ile Leu
            260                 265                 270

Lys Ser Lys Ser Gln Tyr Arg His Pro Ile Lys Asn Gly Tyr Glu Leu
        275                 280                 285

Ile Lys Glu Tyr Gln Gln Phe Gln Ile Cys Lys Ile Ser Pro Gln Ala
    290                 295                 300

Asp Glu Lys Glu Asp Glu Leu Val Leu His Tyr Gln Asn Gln Val Ala
305                 310                 315                 320

Tyr Gln Gly Tyr Leu Phe Ser Phe Gly Leu Pro Leu Glu Gly Glu Leu
                325                 330                 335

Ile Gln Gln Ile Pro Val Ser Arg Glu Thr Ser Ile His Ile Arg His
            340                 345                 350

Arg Lys Thr Gly Asp Val Leu Ile Lys Asn Gly His Arg Lys Lys Leu
        355                 360                 365

Arg Arg Leu Phe Ile Asp Leu Lys Ile Pro Met Glu Lys Arg Asn Ser
    370                 375                 380

Ala Leu Ile Ile Glu Gln Phe Gly Glu Ile Val Ser Ile Leu Gly Ile
385                 390                 395                 400

Ala Thr Asn Asn Leu Ser Lys Lys Thr Lys Asn Asp Ile Met Asn Thr
                405                 410                 415

Val Leu Tyr Ile Glu Lys Ile Asp Arg
            420                 425

<210> 25
<211> 422
<212> PRT
<213> Streptococcus pneumoniae

<400> 25
Met Arg Lys Phe Leu Ile Ile Leu Leu Leu Pro Ser Phe Leu Thr Ile
1               5                   10                  15

Ser Lys Val Val Ser Thr Glu Lys Glu Val Val Tyr Thr Ser Lys Glu
            20                  25                  30

Ile Tyr Tyr Leu Ser Gln Ser Asp Phe Gly Ile Tyr Phe Arg Glu Lys
        35                  40                  45

Leu Ser Ser Pro Met Val Tyr Gly Glu Val Pro Val Tyr Ala Asn Glu
    50                  55                  60

Asp Leu Val Val Glu Ser Gly Lys Leu Thr Pro Lys Thr Ser Phe Gln
65                  70                  75                  80

Ile Thr Glu Trp Arg Leu Asn Lys Gln Gly Ile Pro Val Phe Lys Leu
                85                  90                  95

Ser Asn His Gln Phe Ile Ala Ala Asp Lys Arg Phe Leu Tyr Asp Gln
            100                 105                 110

Ser Glu Val Thr Pro Thr Ile Lys Lys Val Trp Leu Glu Ser Asp Phe
        115                 120                 125

Lys Leu Tyr Asn Ser Pro Tyr Asp Leu Lys Glu Val Lys Ser Ser Leu
    130                 135                 140

Ser Ala Tyr Ser Gln Val Ser Ile Asp Lys Thr Met Phe Val Glu Gly
145                 150                 155                 160

Arg Glu Phe Leu His Ile Asp Gln Ala Gly Trp Val Ala Lys Glu Ser
                165                 170                 175

Thr Ser Glu Glu Asp Asn Arg Met Ser Lys Val Gln Glu Met Leu Ser
            180                 185                 190

Glu Lys Tyr Gln Lys Asp Ser Phe Ser Ile Tyr Val Lys Gln Leu Thr
        195                 200                 205

Thr Gly Lys Glu Ala Gly Ile Asn Gln Asp Glu Lys Met Tyr Ala Ala
    210                 215                 220

Ser Val Leu Lys Leu Ser Tyr Leu Tyr Tyr Thr Gln Glu Lys Ile Asn
225                 230                 235                 240

Glu Gly Leu Tyr Gln Leu Asp Thr Thr Val Lys Tyr Val Ser Ala Val
                245                 250                 255

Asn Asp Phe Pro Gly Ser Tyr Lys Pro Glu Gly Ser Gly Ser Leu Pro
            260                 265                 270

Lys Lys Glu Asp Asn Lys Glu Tyr Ser Leu Lys Asp Leu Ile Thr Lys
        275                 280                 285

Val Ser Lys Glu Ser Asp Asn Val Ala His Asn Leu Leu Gly Tyr Tyr
    290                 295                 300

Ile Ser Asn Gln Ser Asp Ala Thr Phe Lys Ser Lys Met Ser Ala Ile
305                 310                 315                 320

Met Gly Asp Asp Trp Asp Pro Lys Glu Lys Leu Ile Ser Ser Lys Met
                325                 330                 335

Ala Gly Lys Phe Met Glu Ala Ile Tyr Asn Gln Asn Gly Phe Val Leu
            340                 345                 350

Glu Ser Leu Thr Lys Thr Asp Phe Asp Ser Gln Arg Ile Ala Lys Gly
        355                 360                 365

Val Ser Val Lys Val Ala His Lys Ile Gly Asp Ala Asp Glu Phe Lys
    370                 375                 380

His Asp Thr Gly Val Val Tyr Ala Asp Ser Pro Phe Ile Leu Ser Ile
385                 390                 395                 400

Phe Thr Lys Asn Ser Asp Tyr Asp Thr Ile Ser Lys Ile Ala Lys Asp
                405                 410                 415

Val Tyr Glu Val Leu Lys
            420

<210> 26
<211> 652
<212> PRT
<213> Streptococcus pneumoniae

<400> 26
Met Lys Lys Gln Asn Asn Gly Leu Ile Lys Asn Pro Phe Leu Trp Leu
  1               5                  10                  15

Leu Phe Ile Phe Phe Leu Val Thr Gly Phe Gln Tyr Phe Tyr Ser Gly
            20                  25                  30

Asn Asn Ser Gly Gly Ser Gln Gln Ile Asn Tyr Thr Glu Leu Val Gln
        35                  40                  45

Glu Ile Thr Asp Gly Asn Val Lys Glu Leu Thr Tyr Gln Pro Asn Gly
    50                  55                  60

Ser Val Ile Glu Val Ser Gly Val Tyr Lys Asn Pro Lys Thr Ser Lys
 65                  70                  75                  80

Glu Glu Thr Gly Ile Gln Phe Phe Thr Pro Ser Val Thr Lys Val Glu
                85                  90                  95

Lys Phe Thr Ser Thr Ile Leu Pro Ala Asp Thr Thr Val Ser Glu Leu
            100                 105                 110

Gln Lys Leu Ala Thr Asp His Lys Ala Glu Val Thr Val Lys His Glu
        115                 120                 125

Ser Ser Ser Gly Ile Trp Ile Asn Leu Leu Val Ser Ile Val Pro Phe
    130                 135                 140

Gly Ile Leu Phe Phe Phe Leu Phe Ser Met Met Gly Asn Met Gly Gly
145                 150                 155                 160

Gly Asn Gly Arg Asn Pro Met Ser Phe Gly Arg Ser Lys Ala Lys Ala
                165                 170                 175

Ala Asn Lys Glu Asp Ile Lys Val Arg Phe Ser Asp Val Ala Gly Ala
            180                 185                 190

Glu Glu Glu Lys Gln Glu Leu Val Glu Val Val Glu Phe Leu Lys Asp
        195                 200                 205

Pro Lys Arg Phe Thr Lys Leu Gly Ala Arg Ile Pro Ala Gly Val Leu
    210                 215                 220

Leu Glu Gly Pro Pro Gly Thr Gly Lys Thr Leu Leu Ala Lys Ala Val
225                 230                 235                 240

Ala Gly Glu Ala Gly Val Pro Phe Phe Ser Ile Ser Gly Ser Asp Phe
                245                 250                 255

Val Glu Met Phe Val Gly Val Gly Ala Ser Arg Val Arg Ser Leu Phe
            260                 265                 270

Glu Asp Ala Lys Lys Ala Ala Pro Ala Ile Ile Phe Ile Asp Glu Ile
        275                 280                 285

Asp Ala Val Gly Arg Gln Arg Gly Val Gly Leu Gly Gly Gly Asn Asp
    290                 295                 300

Glu Arg Glu Gln Thr Leu Asn Gln Leu Leu Ile Glu Met Asp Gly Phe
305                 310                 315                 320

Glu Gly Asn Glu Gly Ile Ile Val Ile Ala Ala Thr Asn Arg Ser Asp
                325                 330                 335

Val Leu Asp Pro Ala Leu Leu Arg Pro Gly Arg Phe Asp Arg Lys Val
            340                 345                 350

Leu Val Gly Arg Pro Asp Val Lys Gly Arg Glu Ala Ile Leu Lys Val
        355                 360                 365

His Ala Lys Asn Lys Pro Leu Ala Glu Asp Val Asp Leu Lys Leu Val
    370                 375                 380

Ala Gln Gln Thr Pro Gly Phe Val Gly Ala Asp Leu Glu Asn Val Leu
385                 390                 395                 400

Asn Glu Ala Ala Leu Val Ala Ala Arg Arg Asn Lys Ser Ile Ile Asp
                405                 410                 415

Ala Ser Asp Ile Asp Glu Ala Glu Asp Arg Val Ile Ala Gly Pro Ser
            420                 425                 430

Lys Lys Asp Lys Thr Val Ser Gln Lys Glu Arg Glu Leu Val Ala Tyr
        435                 440                 445

His Glu Ala Gly His Thr Ile Val Gly Leu Val Leu Ser Asn Ala Arg
    450                 455                 460

Val Val His Lys Val Thr Ile Val Pro Arg Gly Arg Ala Gly Gly Tyr
465                 470                 475                 480

Met Ile Ala Leu Pro Lys Glu Asp Gln Met Leu Leu Ser Lys Glu Asp
                485                 490                 495

Met Lys Glu Gln Leu Ala Gly Leu Met Gly Gly Arg Val Ala Glu Glu
            500                 505                 510

Ile Ile Phe Asn Val Gln Thr Thr Gly Ala Ser Asn Asp Phe Glu Gln
        515                 520                 525

Ala Thr Gln Met Ala Arg Ala Met Val Thr Glu Tyr Gly Met Ser Glu
    530                 535                 540

Lys Leu Gly Pro Val Gln Tyr Glu Gly Asn His Ala Met Leu Gly Ala
545                 550                 555                 560

Gln Ser Pro Gln Lys Ser Ile Ser Glu Gln Thr Ala Tyr Glu Ile Asp
                565                 570                 575

Glu Glu Val Arg Ser Leu Leu Asn Glu Ala Arg Asn Lys Ala Ala Glu
            580                 585                 590

Ile Ile Gln Ser Asn Arg Glu Thr His Lys Leu Ile Ala Glu Ala Leu
        595                 600                 605

Leu Lys Tyr Glu Thr Leu Asp Ser Thr Gln Ile Lys Ala Leu Tyr Glu
    610                 615                 620

Thr Gly Lys Met Pro Glu Ala Val Glu Glu Glu Ser His Ala Leu Ser
625                 630                 635                 640

Tyr Asp Glu Val Lys Ser Lys Met Asn Asp Glu Lys
                645                 650

<210> 27
<211> 563
<212> PRT
<213> Streptococcus pneumoniae

<400> 27
Met Lys Arg Ser Ser Leu Leu Val Arg Met Val Ile Ser Ile Phe Leu
1               5                   10                  15

Val Phe Leu Ile Leu Leu Ala Leu Val Gly Thr Phe Tyr Tyr Gln Ser
            20                  25                  30

Ser Ser Ser Ala Ile Glu Ala Thr Ile Glu Gly Asn Ser Gln Thr Thr
        35                  40                  45

Ile Ser Gln Thr Ser His Phe Ile Gln Ser Tyr Ile Lys Lys Leu Glu
    50                  55                  60

Thr Thr Ser Thr Gly Leu Thr Gln Gln Thr Asp Val Leu Ala Tyr Ala
65                  70                  75                  80

Glu Asn Pro Ser Gln Asp Lys Val Glu Gly Ile Arg Asp Leu Phe Leu
                85                  90                  95

Thr Ile Leu Lys Ser Asp Lys Asp Leu Lys Thr Val Val Leu Val Thr
            100                 105                 110

Lys Ser Gly Gln Val Ile Ser Thr Asp Asp Ser Val Gln Met Lys Thr
        115                 120                 125

Ser Ser Asp Met Met Ala Glu Asp Trp Tyr Gln Lys Ala Ile His Gln
    130                 135                 140

Gly Ala Met Pro Val Leu Thr Pro Ala Arg Lys Ser Asp Ser Gln Trp
145                 150                 155                 160

Val Ile Ser Val Thr Gln Glu Leu Val Asp Ala Lys Gly Ala Asn Leu
                165                 170                 175

Gly Val Leu Arg Leu Asp Ile Ser Tyr Glu Thr Leu Glu Ala Tyr Leu
            180                 185                 190

Asn Gln Leu Gln Leu Gly Gln Gln Gly Phe Ala Phe Ile Ile Asn Glu
        195                 200                 205

Asn His Glu Phe Val Tyr His Pro Gln His Thr Val Tyr Ser Ser Ser
    210                 215                 220

Ser Lys Met Glu Ala Met Lys Pro Tyr Ile Asp Thr Gly Gln Gly Tyr
225                 230                 235                 240

Thr Pro Gly His Lys Ser Tyr Val Ser Gln Glu Lys Ile Ala Gly Thr
                245                 250                 255

Asp Trp Thr Val Leu Gly Val Ser Ser Leu Glu Lys Leu Asp Gln Val
            260                 265                 270

Arg Ser Gln Leu Leu Trp Thr Leu Leu Gly Ala Ser Val Thr Ser Leu
        275                 280                 285

Leu Val Cys Leu Cys Leu Val Trp Phe Ser Leu Lys Arg Trp Ile Ala
    290                 295                 300

Pro Leu Lys Asp Leu Arg Glu Thr Met Leu Glu Ile Ala Ser Gly Ala
305                 310                 315                 320

Gln Asn Leu Arg Ala Lys Glu Val Gly Ala Tyr Glu Leu Arg Glu Val
                325                 330                 335

Thr Arg Gln Phe Asn Ala Met Leu Asp Gln Ile Asp Gln Leu Met Val
            340                 345                 350

Ala Ile Arg Ser Gln Glu Glu Thr Thr Arg Gln Tyr Gln Leu Gln Ala
        355                 360                 365

Leu Ser Ser Gln Ile Asn Pro His Phe Leu Tyr Asn Thr Leu Asp Thr
    370                 375                 380

Ile Ile Trp Met Ala Glu Phe His Asp Ser Gln Arg Val Val Gln Val
385                 390                 395                 400

Thr Lys Ser Leu Ala Thr Tyr Phe Arg Leu Ala Leu Asn Gln Gly Lys
                405                 410                 415

Asp Leu Ile Cys Leu Ser Asp Glu Ile Asn His Val Arg Gln Tyr Leu
            420                 425                 430

Phe Ile Gln Lys Gln Arg Tyr Gly Asp Lys Leu Glu Tyr Glu Ile Asn
        435                 440                 445

Glu Asn Val Ala Phe Asp Asn Leu Val Leu Pro Lys Leu Val Leu Gln
    450                 455                 460

Pro Leu Val Glu Asn Ala Leu Tyr His Gly Ile Lys Glu Lys Glu Gly
465                 470                 475                 480

Gln Gly His Ile Lys Leu Ser Val Gln Lys Gln Asp Ser Gly Leu Val
                485                 490                 495

Ile Arg Ile Glu Asp Asp Gly Val Gly Phe Gln Asp Ala Gly Asp Ser
            500                 505                 510

Ser Gln Ser Gln Leu Lys Arg Gly Gly Val Gly Leu Gln Asn Val Asp
        515                 520                 525

Gln Arg Leu Lys Leu His Phe Gly Ala Asn Tyr His Met Lys Ile Asp
    530                 535                 540

Ser Arg Pro Gln Lys Gly Thr Lys Val Glu Ile Tyr Ile Asn Arg Ile
545                 550                 555                 560

Glu Thr Ser

<210> 28
<211> 563
<212> PRT
<213> Streptococcus pneumoniae

<400> 28
Met Lys Arg Ser Ser Leu Leu Val Arg Met Val Ile Ser Ile Phe Leu
  1               5                  10                  15

Val Phe Leu Ile Leu Leu Ala Leu Val Gly Thr Phe Tyr Tyr Gln Ser
            20                  25                  30

Ser Ser Ser Ala Ile Glu Ala Thr Ile Glu Gly Asn Ser Gln Thr Thr
        35                  40                  45

Ile Ser Gln Thr Ser His Phe Ile Gln Ser Tyr Ile Lys Lys Leu Glu
    50                  55                  60

Thr Thr Ser Thr Gly Leu Thr Gln Gln Thr Asp Val Leu Ala Tyr Ala
 65                  70                  75                  80

Glu Asn Pro Ser Gln Asp Lys Val Glu Gly Ile Arg Asp Leu Phe Leu
                 85                  90                  95

Thr Ile Leu Lys Ser Asp Lys Asp Leu Lys Thr Val Val Leu Val Thr
            100                 105                 110

Lys Ser Gly Gln Val Ile Ser Thr Asp Asp Ser Val Gln Met Lys Thr
        115                 120                 125

Ser Ser Asp Met Met Ala Glu Asp Trp Tyr Gln Lys Ala Ile His Gln
        130                 135                 140

Gly Ala Met Pro Val Leu Thr Pro Ala Arg Lys Ser Asp Ser Gln Trp
145                 150                 155                 160

Val Ile Ser Val Thr Gln Glu Leu Val Asp Ala Lys Gly Ala Asn Leu
                165                 170                 175

Gly Val Leu Arg Leu Asp Ile Ser Tyr Glu Thr Leu Glu Ala Tyr Leu
            180                 185                 190

Asn Gln Leu Gln Leu Gly Gln Gln Gly Phe Ala Phe Ile Ile Asn Glu
        195                 200                 205

Asn His Glu Phe Val Tyr His Pro Gln His Thr Val Tyr Ser Ser Ser
    210                 215                 220

Ser Lys Met Glu Ala Met Lys Pro Tyr Ile Asp Thr Gly Gln Gly Tyr
225                 230                 235                 240

Thr Pro Gly His Lys Ser Tyr Val Ser Gln Glu Lys Ile Ala Gly Thr
                245                 250                 255

Asp Trp Thr Val Leu Gly Val Ser Ser Leu Glu Lys Leu Asp Gln Val
            260                 265                 270

Arg Ser Gln Leu Leu Trp Thr Leu Leu Gly Ala Ser Val Thr Ser Leu
        275                 280                 285

Leu Val Cys Leu Cys Leu Val Trp Phe Ser Leu Lys Arg Trp Ile Ala
    290                 295                 300

Pro Leu Lys Asp Leu Arg Glu Thr Met Leu Glu Ile Ala Ser Gly Ala
305                 310                 315                 320

Gln Asn Leu Arg Ala Lys Glu Val Gly Ala Tyr Glu Leu Arg Glu Val
                325                 330                 335

Thr Arg Gln Phe Asn Ala Met Leu Asp Gln Ile Asp Gln Leu Met Val
            340                 345                 350

Ala Ile Arg Ser Gln Glu Glu Thr Thr Arg Gln Tyr Gln Leu Gln Ala
        355                 360                 365

Leu Ser Ser Gln Ile Asn Pro His Phe Leu Tyr Asn Thr Leu Asp Thr
    370                 375                 380

Ile Ile Trp Met Ala Glu Phe His Asp Ser Gln Arg Val Val Gln Val
385                 390                 395                 400

Thr Lys Ser Leu Ala Thr Tyr Phe Arg Leu Ala Leu Asn Gln Gly Lys
                405                 410                 415

Asp Leu Ile Cys Leu Ser Asp Glu Ile Asn His Val Arg Gln Tyr Leu
            420                 425                 430

Phe Ile Gln Lys Gln Arg Tyr Gly Asp Lys Leu Glu Tyr Glu Ile Asn
        435                 440                 445

Glu Asn Val Ala Phe Asp Asn Leu Val Leu Pro Lys Leu Val Leu Gln
    450                 455                 460

Pro Leu Val Glu Asn Ala Leu Tyr His Gly Ile Lys Glu Lys Glu Gly
465                 470                 475                 480

Gln Gly His Ile Lys Leu Ser Val Gln Lys Gln Asp Ser Gly Leu Val
                485                 490                 495

Ile Arg Ile Glu Asp Asp Gly Val Gly Phe Gln Asp Ala Gly Asp Ser
            500                 505                 510

Ser Gln Ser Gln Leu Lys Arg Gly Gly Val Gly Leu Gln Asn Val Asp
        515                 520                 525

Gln Arg Leu Lys Leu His Phe Gly Ala Asn Tyr His Met Lys Ile Asp
    530                 535                 540

Ser Arg Pro Gln Lys Gly Thr Lys Val Glu Ile Tyr Ile Asn Arg Ile
545                 550                 555                 560

Glu Thr Ser

<210> 29
<211> 379
<212> PRT
<213> Streptococcus pneumoniae

<400> 29
Met Phe Phe Lys Leu Leu Arg Glu Ala Leu Lys Val Lys Gln Val Arg
  1               5                  10                  15

Ser Lys Ile Leu Phe Thr Ile Phe Ile Val Leu Val Phe Arg Ile Gly
             20                  25                  30

Thr Ser Ile Thr Val Pro Gly Val Asn Ala Asn Ser Leu Asn Ala Leu
         35                  40                  45

Ser Gly Leu Ser Phe Leu Asn Met Leu Ser Leu Val Ser Gly Asn Ala
     50                  55                  60

Leu Lys Asn Phe Ser Ile Phe Ala Leu Gly Val Ser Pro Tyr Ile Thr
 65                  70                  75                  80

Ala Ser Ile Val Val Gln Leu Leu Gln Met Asp Ile Leu Pro Lys Phe
                 85                  90                  95

Val Glu Trp Gly Lys Gln Gly Glu Val Gly Arg Arg Lys Leu Asn Gln
            100                 105                 110

Ala Thr Arg Tyr Ile Ala Leu Val Leu Ala Phe Val Gln Ser Ile Gly
        115                 120                 125

Ile Thr Ala Gly Phe Asn Thr Leu Ala Gly Ala Gln Leu Ile Lys Thr
    130                 135                 140

Ala Leu Thr Pro Gln Val Phe Leu Thr Ile Gly Ile Ile Leu Thr Ala
145                 150                 155                 160

Gly Ser Met Ile Val Thr Trp Leu Gly Glu Gln Ile Thr Asp Lys Gly
                165                 170                 175

Tyr Gly Asn Gly Val Ser Met Ile Ile Phe Ala Gly Ile Val Ser Ser
            180                 185                 190

Ile Pro Glu Met Ile Gln Gly Ile Tyr Val Asp Tyr Phe Val Asn Val
        195                 200                 205

Pro Ser Ser Arg Ile Thr Ser Ser Ile Ile Phe Val Ile Ile Leu Ile
    210                 215                 220

Ile Thr Val Leu Leu Ile Ile Tyr Phe Thr Thr Tyr Val Gln Gln Ala
225                 230                 235                 240

Glu Tyr Lys Ile Pro Ile Gln Tyr Thr Lys Val Ala Gln Gly Ala Pro
                245                 250                 255

Ser Ser Ser Tyr Leu Pro Leu Lys Val Asn Pro Ala Gly Val Ile Pro
            260                 265                 270

Val Ile Phe Ala Ser Ser Ile Thr Ala Ala Pro Ala Ala Ile Leu Gln
        275                 280                 285

Phe Leu Ser Ala Thr Gly His Asp Trp Ala Trp Val Arg Val Ala Gln
    290                 295                 300

Glu Met Leu Ala Thr Thr Ser Pro Thr Gly Ile Ala Met Tyr Ala Leu
305                 310                 315                 320

Leu Ile Ile Leu Phe Thr Phe Phe Tyr Thr Phe Val Gln Ile Asn Pro
                325                 330                 335

Glu Lys Ala Ala Glu Thr Tyr Lys Arg Val Val Pro Ile Ser Met Glu
            340                 345                 350

Phe Val Leu Val Lys Val Gln Lys Asn Ile Cys Leu Asn Phe Phe Val
        355                 360                 365

Val Leu Gln Leu Leu Val Pro Ser Ser Leu Val
    370                 375

<210> 30
<211> 445
<212> PRT
<213> Streptococcus pneumoniae

<400> 30
Met Asp Ile Arg Gln Val Thr Glu Thr Ile Ala Met Ile Glu Glu Gln
  1               5                  10                  15

Asn Phe Asp Ile Arg Thr Ile Thr Met Gly Ile Ser Leu Leu Asp Cys
             20                  25                  30

Ile Asp Pro Asp Ile Asn Arg Ala Ala Glu Lys Ile Tyr Gln Lys Ile
         35                  40                  45

Thr Thr Lys Ala Ala Asn Leu Val Ala Val Gly Asp Glu Ile Ala Ala

50                  55                  60

Glu Leu Gly Ile Pro Ile Val Asn Lys Arg Val Ser Val Thr Pro Ile
65                  70                  75                  80

Ser Leu Ile Gly Ala Ala Thr Asp Ala Thr Asp Tyr Val Val Leu Ala
                85                  90                  95

Lys Ala Leu Asp Lys Ala Ala Lys Glu Ile Gly Val Asp Phe Ile Gly
            100                 105                 110

Gly Phe Ser Ala Leu Val Gln Lys Gly Tyr Gln Lys Gly Asp Glu Ile
        115                 120                 125

Leu Ile Asn Ser Ile Pro Arg Ala Leu Ala Glu Thr Asp Lys Val Cys
    130                 135                 140

Ser Ser Val Asn Ile Gly Ser Thr Lys Ser Gly Ile Asn Met Thr Ala
145                 150                 155                 160

Val Ala Asp Met Gly Arg Ile Ile Lys Glu Thr Ala Asn Leu Ser Asp
                165                 170                 175

Met Gly Val Ala Lys Leu Val Val Phe Ala Asn Ala Val Glu Asp Asn
            180                 185                 190

Pro Phe Met Ala Gly Ala Phe His Gly Val Gly Glu Ala Asp Val Ile
        195                 200                 205

Ile Asn Val Gly Val Ser Gly Pro Gly Val Val Lys Arg Ala Leu Glu
    210                 215                 220

Lys Val Arg Gly Gln Ser Phe Asp Val Val Ala Glu Thr Val Lys Lys
225                 230                 235                 240

Thr Ala Phe Lys Ile Thr Arg Ile Gly Gln Leu Val Gly Gln Met Ala
                245                 250                 255

Ser Glu Arg Leu Gly Val Glu Phe Gly Ile Val Asp Leu Ser Leu Ala
            260                 265                 270

Pro Thr Pro Ala Val Gly Asp Ser Val Ala Arg Val Leu Glu Glu Met
        275                 280                 285

Gly Leu Glu Thr Val Gly Thr His Gly Thr Thr Ala Ala Leu Ala Leu
    290                 295                 300

Leu Asn Asp Gln Val Lys Lys Gly Gly Val Met Ala Cys Asn Gln Val
305                 310                 315                 320

Gly Gly Leu Ser Gly Ala Phe Ile Pro Val Ser Glu Asp Glu Gly Met
                325                 330                 335

Ile Ala Ala Val Gln Asn Gly Ser Leu Asn Leu Glu Lys Leu Glu Ala
            340                 345                 350

Met Thr Ala Ile Cys Ser Val Gly Leu Asp Met Ile Ala Ile Pro Glu
        355                 360                 365

Asp Thr Pro Ala Glu Thr Ile Ala Ala Met Ile Ala Asp Glu Ala Ala
    370                 375                 380

Ile Gly Val Ile Asn Met Lys Thr Thr Ala Val Arg Ile Ile Pro Lys
385                 390                 395                 400

Gly Lys Glu Gly Asp Met Ile Glu Phe Gly Gly Leu Leu Gly Thr Ala
                405                 410                 415

Pro Val Met Lys Val Asn Gly Ala Ser Ser Val Asp Phe Ile Ser Arg
            420                 425                 430

Gly Gly Gln Ile Pro Ala Pro Ile His Ser Phe Lys Asn
        435                 440                 445

<210> 31
<211> 285
<212> PRT
<213> Streptococcus pneumoniae

<400> 31
Met Thr Gln Ile Ile Asp Gly Lys Ala Leu Ala Ala Lys Leu Gln Gly
1               5                   10                  15

Gln Leu Ala Glu Lys Thr Ala Lys Leu Lys Glu Glu Thr Gly Leu Val
            20                  25                  30

Pro Gly Leu Val Val Ile Leu Val Gly Asp Asn Pro Ala Ser Gln Val
        35                  40                  45

Tyr Val Arg Asn Lys Glu Arg Ser Ala Leu Ala Ala Gly Phe Arg Ser
    50                  55                  60

Glu Val Val Arg Val Pro Glu Thr Ile Thr Gln Glu Glu Leu Leu Asp
65                  70                  75                  80

Leu Ile Ala Lys Tyr Asn Gln Asp Pro Ala Trp His Gly Ile Leu Val
                85                  90                  95

Gln Leu Pro Leu Pro Lys His Ile Asp Glu Glu Ala Val Leu Leu Ala
            100                 105                 110

Ile Asp Pro Glu Lys Asp Val Asp Gly Phe His Pro Leu Asn Met Gly
        115                 120                 125

Arg Leu Trp Ser Gly His Pro Val Met Ile Pro Ser Thr Pro Ala Gly
    130                 135                 140

Ile Met Glu Met Phe His Glu Tyr Gly Ile Asp Leu Glu Gly Lys Asn
145                 150                 155                 160

Ala Val Val Ile Gly Arg Ser Asn Ile Val Gly Lys Pro Met Ala Gln
                165                 170                 175

Leu Leu Leu Ala Lys Asn Ala Thr Val Thr Leu Thr His Ser Arg Thr
            180                 185                 190

His Asn Leu Ser Lys Val Ala Ala Lys Ala Asp Ile Leu Val Val Ala
        195                 200                 205

Ile Gly Arg Ala Lys Phe Val Thr Ala Asp Phe Val Lys Pro Gly Ala

210                 215                 220

Val Val Ile Asp Val Gly Met Asn Arg Asp Glu Asn Gly Lys Leu Cys
225                 230                 235                 240

Gly Asp Val Asp Tyr Glu Ala Val Ala Pro Leu Ala Ser His Ile Thr
                245                 250                 255

Pro Val Pro Gly Gly Val Gly Pro Met Thr Ile Thr Met Leu Met Glu
            260                 265                 270

Gln Thr Tyr Gln Ala Ala Leu Arg Thr Leu Asp Arg Lys
        275                 280                 285

<210> 32
<211> 403
<212> PRT
<213> Streptococcus pneumoniae

<400> 32
Met Ser Lys Phe Asn Arg Ile His Leu Val Val Leu Asp Ser Val Gly
  1               5                  10                  15

Ile Gly Ala Ala Pro Asp Ala Asn Asn Phe Val Asn Ala Gly Val Pro
             20                  25                  30

Asp Gly Ala Ser Asp Thr Leu Gly His Ile Ser Lys Thr Val Gly Leu
         35                  40                  45

Asn Val Pro Asn Met Ala Lys Ile Gly Leu Gly Asn Ile Pro Arg Glu
     50                  55                  60

Thr Pro Leu Lys Thr Val Ala Ala Glu Ser Asn Pro Thr Gly Tyr Ala
 65                  70                  75                  80

Thr Lys Leu Glu Glu Val Ser Leu Gly Lys Asp Thr Met Thr Gly His
                 85                  90                  95

Trp Glu Ile Met Gly Leu Asn Ile Thr Glu Pro Phe Asp Thr Phe Trp
            100                 105                 110

Asn Gly Phe Pro Glu Glu Ile Leu Thr Lys Ile Glu Glu Phe Ser Gly
        115                 120                 125

Arg Lys Val Ile Arg Glu Ala Asn Lys Pro Tyr Ser Gly Thr Ala Val
    130                 135                 140

Ile Tyr Asp Phe Gly Pro Arg Gln Met Glu Thr Gly Glu Leu Ile Ile
145                 150                 155                 160

Tyr Thr Ser Ala Asp Pro Val Leu Gln Ile Ala Ala His Glu Asp Ile
                165                 170                 175

Ile Pro Leu Asp Glu Leu Tyr Arg Ile Cys Glu Tyr Ala Arg Ser Ile
            180                 185                 190

Thr Leu Glu Arg Pro Ala Leu Leu Gly Arg Ile Ile Ala Arg Pro Tyr
        195                 200                 205

Val Gly Glu Pro Gly Asn Phe Thr Arg Thr Ala Asn Arg Arg Asp Leu
    210                 215                 220

Ala Val Ser Pro Phe Phe Pro Thr Val Leu Asp Lys Leu Asn Glu Ala
225                 230                 235                 240

Gly Ile Asp Thr Tyr Ala Val Gly Lys Ile Asn Asp Ile Phe Asn Gly
                245                 250                 255

Ala Gly Ile Asn His Asp Met Gly His Asn Lys Ser Asn Ser His Gly
            260                 265                 270

Ile Asp Thr Leu Leu Lys Thr Met Gly Leu Ala Glu Phe Glu Lys Gly
        275                 280                 285

Phe Ser Phe Thr Asn Leu Val Asp Phe Asp Ala Leu Tyr Gly His Arg
    290                 295                 300

Arg Asn Ala His Gly Tyr Arg Asp Cys Leu His Glu Phe Asp Glu Arg
305                 310                 315                 320

Leu Pro Glu Ile Ile Ala Ala Met Arg Glu Asn Asp Leu Leu Leu Ile
                325                 330                 335

Thr Ala Asp His Gly Asn Asp Pro Thr Tyr Ala Gly Thr Asp His Thr
            340                 345                 350

Arg Glu Tyr Ile Pro Leu Leu Ala Tyr Ser Pro Ala Phe Lys Gly Asn
        355                 360                 365

Gly Leu Ile Pro Val Gly His Phe Ala Asp Ile Ser Ala Thr Val Ala
    370                 375                 380

Asp Asn Phe Gly Val Glu Thr Ala Met Ile Gly Glu Ser Phe Leu Asp
385                 390                 395                 400

Lys Leu Val

<210> 33
<211> 326
<212> PRT
<213> Streptococcus pneumoniae

<400> 33
Met Phe Ile Ser Ile Ser Ala Gly Ile Val Thr Phe Leu Leu Thr Leu
1               5                   10                  15

Val Glu Ile Pro Ala Phe Ile Gln Phe Tyr Arg Lys Ala Gln Ile Thr
            20                  25                  30

Gly Gln Gln Met His Glu Asp Val Lys Gln His Gln Ala Lys Ala Gly
        35                  40                  45

Thr Pro Thr Met Gly Gly Leu Val Phe Leu Ile Thr Ser Val Leu Val
    50                  55                  60

Ala Phe Phe Phe Ala Leu Phe Ser Ser Gln Phe Ser Asn Asn Val Gly
65                  70                  75                  80

Met Ile Leu Phe Ile Leu Val Leu Tyr Gly Leu Val Gly Phe Leu Asp
                85                  90                  95

Asp Phe Leu Lys Val Phe Arg Lys Ile Asn Glu Gly Leu Asn Pro Lys
            100                 105                 110

Gln Lys Leu Ala Leu Gln Leu Leu Gly Gly Val Ile Phe Tyr Leu Phe
        115                 120                 125

Tyr Glu Arg Gly Gly Asp Ile Leu Ser Val Phe Gly Tyr Pro Val His
    130                 135                 140

Leu Gly Phe Phe Tyr Ile Phe Phe Ala Leu Phe Trp Leu Val Gly Phe
145                 150                 155                 160

Ser Asn Ala Val Asn Leu Thr Asp Gly Val Asp Gly Leu Ala Ser Ile
                165                 170                 175

Ser Val Val Ile Ser Leu Ser Ala Tyr Gly Val Ile Ala Tyr Val Gln
            180                 185                 190

Gly Gln Met Asp Ile Leu Leu Val Ile Leu Ala Met Ile Gly Gly Leu
        195                 200                 205

Leu Gly Phe Phe Ile Phe Asn His Lys Pro Ala Lys Val Phe Met Gly
    210                 215                 220

Asp Val Gly Ser Leu Ala Leu Gly Gly Met Leu Ala Ala Ile Ser Met
225                 230                 235                 240

Ala Leu His Gln Glu Trp Thr Leu Leu Ile Ile Gly Ile Val Tyr Val
                245                 250                 255

Phe Glu Thr Thr Ser Val Met Met Gln Val Ser Tyr Phe Lys Leu Thr
            260                 265                 270

Gly Gly Lys Arg Ile Phe Arg Met Thr Pro Val His His His Phe Glu
        275                 280                 285

Leu Gly Gly Leu Ser Gly Lys Gly Asn Pro Trp Ser Glu Trp Lys Val
    290                 295                 300

Asp Phe Phe Phe Trp Gly Val Gly Leu Leu Ala Ser Leu Leu Thr Leu
305                 310                 315                 320

Ala Ile Leu Tyr Leu Met
                325

<210> 34
<211> 426
<212> PRT
<213> Streptococcus pneumoniae

<400> 34
Leu Phe Lys Lys Asn Lys Asp Ile Leu Asn Ile Ala Leu Pro Ala Met
1               5                   10                  15

Gly Glu Asn Phe Leu Gln Met Leu Met Gly Met Val Asp Ser Tyr Leu

20                  25                  30

Val Ala His Leu Gly Leu Ile Ala Ile Ser Gly Val Ser Val Ala Gly
        35                  40                  45

Asn Ile Ile Thr Ile Tyr Gln Ala Ile Phe Ile Ala Leu Gly Ala Ala
    50                  55                  60

Ile Ser Ser Val Ile Ser Lys Ser Ile Gly Gln Lys Asp Gln Ser Lys
65                  70                  75                  80

Leu Ala Tyr His Val Thr Glu Ala Leu Lys Ile Thr Leu Leu Leu Ser
                85                  90                  95

Phe Leu Leu Gly Phe Leu Ser Ile Phe Ala Gly Lys Glu Met Ile Gly
            100                 105                 110

Leu Leu Gly Thr Glu Arg Asp Val Ala Glu Ser Gly Gly Leu Tyr Leu
        115                 120                 125

Ser Leu Val Gly Gly Ser Ile Val Leu Leu Gly Leu Met Thr Ser Leu
    130                 135                 140

Gly Ala Leu Ile Arg Ala Thr His Asn Pro Arg Leu Pro Leu Tyr Val
145                 150                 155                 160

Ser Phe Leu Ser Asn Ala Leu Asn Ile Leu Phe Ser Ser Leu Ala Ile
                165                 170                 175

Phe Val Leu Asp Met Gly Ile Ala Gly Val Ala Trp Gly Thr Ile Val
            180                 185                 190

Ser Arg Leu Val Gly Leu Val Ile Leu Trp Ser Gln Leu Lys Leu Pro
        195                 200                 205

Tyr Gly Lys Pro Thr Phe Gly Leu Asp Lys Glu Leu Leu Thr Leu Ala
    210                 215                 220

Leu Pro Ala Ala Gly Glu Arg Leu Met Met Arg Ala Gly Asp Val Val
225                 230                 235                 240

Ile Ile Ala Leu Val Val Ser Phe Gly Thr Glu Ala Val Ala Gly Asn
                245                 250                 255

Ala Ile Gly Glu Val Leu Thr Gln Phe Asn Tyr Met Pro Ala Phe Gly
            260                 265                 270

Val Ala Thr Ala Thr Val Met Leu Leu Ala Arg Ala Val Gly Glu Asp
        275                 280                 285

Asp Trp Lys Arg Val Ala Ser Leu Ser Lys Gln Thr Phe Trp Leu Ser
    290                 295                 300

Leu Phe Leu Met Leu Pro Leu Ser Phe Ser Ile Tyr Val Leu Gly Val
305                 310                 315                 320

Pro Leu Thr His Leu Tyr Thr Thr Asp Ser Leu Ala Val Glu Ala Ser
                325                 330                 335

Val Leu Val Thr Leu Phe Ser Leu Leu Gly Thr Pro Met Thr Thr Gly
            340                 345                 350

Thr Val Ile Tyr Thr Ala Val Trp Gln Gly Leu Gly Asn Ala Arg Leu
        355                 360                 365

Pro Phe Tyr Ala Thr Ser Ile Gly Met Trp Cys Ile Arg Ile Gly Thr
    370                 375                 380

Gly Tyr Leu Met Gly Ile Val Leu Gly Trp Gly Leu Pro Gly Ile Trp
385                 390                 395                 400

Ala Gly Ser Leu Leu Asp Asn Gly Phe Arg Trp Leu Phe Leu Arg Tyr
                405                 410                 415

Arg Tyr Gln Arg Tyr Met Ser Leu Lys Gly
            420                 425

<210> 35
<211> 420
<212> PRT
<213> Streptococcus pneumoniae

<400> 35
Met Gln Thr Gln Glu Lys His Ser Gln Ala Ala Val Leu Gly Leu Gln
1               5                   10                  15

His Leu Leu Ala Met Tyr Ser Gly Ser Ile Leu Val Pro Ile Met Ile
            20                  25                  30

Ala Thr Ala Leu Gly Tyr Ser Ala Glu Gln Leu Thr Tyr Leu Ile Ser
        35                  40                  45

Thr Asp Ile Phe Met Cys Gly Val Ala Thr Phe Leu Gln Leu Gln Leu
    50                  55                  60

Asn Lys Tyr Phe Gly Ile Gly Leu Pro Val Val Leu Gly Val Ala Phe
65                  70                  75                  80

Gln Ser Val Ala Pro Leu Ile Met Ile Gly Gln Ser His Gly Ser Gly
                85                  90                  95

Ala Met Phe Gly Ala Leu Ile Ala Ser Gly Ile Tyr Val Val Leu Val
            100                 105                 110

Ser Gly Ile Phe Ser Lys Val Ala Asn Leu Phe Pro Ser Ile Val Thr
        115                 120                 125

Gly Ser Val Ile Thr Thr Ile Gly Leu Thr Leu Ile Pro Val Ala Ile
    130                 135                 140

Gly Asn Met Gly Asn Asn Val Pro Glu Pro Thr Gly Gln Ser Leu Leu
145                 150                 155                 160

Leu Ala Ala Ile Thr Val Leu Ile Ile Leu Leu Ile Asn Ile Phe Thr
                165                 170                 175

Lys Gly Phe Ile Lys Ser Ile Ser Ile Leu Ile Gly Leu Val Val Gly
            180                 185                 190

Thr Ala Ile Ala Ala Thr Met Gly Leu Val Asp Phe Ser Pro Val Ala

195                 200                 205

Val Ala Pro Leu Val His Val Pro Thr Pro Leu Tyr Phe Gly Met Pro
    210                 215                 220

Thr Phe Glu Ile Ser Ser Ile Val Met Met Cys Ile Ile Ala Thr Val
225                 230                 235                 240

Ser Met Val Glu Ser Thr Gly Val Tyr Leu Ala Leu Ser Asp Ile Thr
                245                 250                 255

Lys Asp Pro Ile Asp Ser Thr Arg Leu Arg Asn Gly Tyr Arg Ala Glu
            260                 265                 270

Gly Leu Ala Val Leu Leu Gly Gly Ile Phe Asn Thr Phe Pro Tyr Thr
        275                 280                 285

Gly Phe Ser Gln Asn Val Gly Leu Val Lys Leu Ser Gly Ile Lys Lys
    290                 295                 300

Arg Leu Pro Ile Tyr Tyr Ala Ala Gly Phe Leu Val Leu Leu Gly Leu
305                 310                 315                 320

Leu Pro Lys Phe Gly Ala Leu Ala Gln Ile Ile Pro Ser Ser Val Leu
                325                 330                 335

Gly Gly Ala Met Leu Val Met Phe Gly Phe Val Ser Ile Gln Gly Met
            340                 345                 350

Gln Ile Leu Ala Arg Val Asp Phe Ala Asn Asn Glu His Asn Phe Leu
        355                 360                 365

Ile Ala Ala Val Ser Ile Ala Ala Gly Val Gly Leu Asn Asn Ser Asn
    370                 375                 380

Leu Phe Val Ser Met Pro Thr Ala Phe Gln Met Phe Phe Ser Asn Gly
385                 390                 395                 400

Ile Val Val Ala Ser Leu Leu Ala Ile Val Leu Asn Ala Val Leu Asn
                405                 410                 415

His Lys Lys Lys
            420

<210> 36
<211> 784
<212> PRT
<213> Streptococcus pneumoniae

<400> 36
Met Lys Asp Arg Ile Lys Glu Tyr Leu Gln Asp Lys Gly Lys Val Thr
1               5                   10                  15

Val Asn Asp Leu Ala Gln Ala Leu Gly Lys Asp Ser Ser Lys Asp Phe
            20                  25                  30

Arg Glu Leu Ile Lys Thr Leu Ser Leu Met Glu Arg Lys His Gln Ile
        35                  40                  45

Arg Phe Glu Glu Asp Gly Ser Leu Thr Leu Glu Ile Lys Lys Lys His
    50                  55                  60

Glu Ile Thr Leu Lys Gly Ile Phe His Ala His Lys Asn Gly Phe Gly
65                  70                  75                  80

Phe Val Ser Leu Glu Gly Glu Glu Asp Asp Leu Phe Val Gly Lys Asn
                85                  90                  95

Asp Val Asn Tyr Ala Ile Asp Gly Asp Thr Val Glu Val Val Ile Lys
            100                 105                 110

Lys Val Ala Asp Arg Asn Lys Gly Thr Ala Ala Glu Ala Lys Ile Ile
        115                 120                 125

Asp Ile Leu Glu His Ser Leu Thr Thr Val Val Gly Gln Ile Val Leu
    130                 135                 140

Asp Gln Glu Lys Pro Lys Tyr Ala Gly Tyr Ile Arg Ser Lys Asn Gln
145                 150                 155                 160

Lys Ile Ser Gln Pro Ile Tyr Val Lys Lys Pro Ala Leu Lys Leu Glu
                165                 170                 175

Gly Thr Glu Val Leu Lys Val Phe Ile Asp Lys Tyr Pro Ser Lys Lys
            180                 185                 190

His Asp Phe Phe Val Ala Ser Val Leu Asp Val Val Gly His Ser Thr
        195                 200                 205

Asp Val Gly Ile Asp Val Leu Glu Val Leu Glu Ser Met Asp Ile Val
    210                 215                 220

Ser Glu Phe Pro Glu Ala Val Val Lys Glu Ala Glu Ser Val Pro Asp
225                 230                 235                 240

Ala Pro Ser Gln Lys Asp Met Glu Gly Arg Leu Asp Leu Arg Asp Glu
                245                 250                 255

Ile Thr Phe Thr Ile Asp Gly Ala Asp Ala Lys Asp Leu Asp Asp Ala
            260                 265                 270

Val His Ile Lys Ala Leu Lys Asn Gly Asn Leu Glu Phe Gly Val His
        275                 280                 285

Ile Ala Asp Val Ser Tyr Tyr Val Thr Glu Gly Ser Ala Leu Asp Lys
    290                 295                 300

Glu Ala Leu Asn Arg Ala Thr Ser Val Tyr Val Thr Asp Arg Val Val
305                 310                 315                 320

Pro Met Leu Pro Glu Arg Leu Ser Asn Gly Ile Cys Ser Leu Asn Pro
                325                 330                 335

Gln Val Asp Arg Leu Thr Gln Ser Ala Ile Met Glu Ile Asp Lys His
            340                 345                 350

Gly Arg Val Val Asn Tyr Thr Ile Thr Gln Thr Val Ile Lys Thr Ser
        355                 360                 365

Phe Arg Met Thr Tyr Ser Asp Val Asn Asp Ile Leu Ala Gly Asp Glu

370                 375                 380

Glu Lys Arg Lys Glu Tyr His Lys Ile Val Ser Ser Ile Glu Leu Met
385                 390                 395                 400

Ala Lys Leu His Glu Thr Leu Glu Asn Met Arg Val Lys Arg Gly Ala
                405                 410                 415

Leu Asn Phe Asp Thr Asn Glu Ala Lys Ile Leu Val Asp Lys Gln Gly
            420                 425                 430

Lys Pro Val Asp Ile Val Leu Arg Gln Arg Gly Ile Ala Glu Arg Met
        435                 440                 445

Ile Glu Ser Phe Met Leu Met Ala Asn Glu Thr Val Ala Glu His Phe
    450                 455                 460

Ser Lys Leu Asp Leu Pro Phe Ile Tyr Arg Ile His Glu Glu Pro Lys
465                 470                 475                 480

Ala Glu Lys Val Gln Lys Phe Ile Asp Tyr Ala Ser Ser Phe Gly Leu
                485                 490                 495

Arg Ile Tyr Gly Thr Ala Ser Glu Ile Ser Gln Glu Ala Leu Gln Asp
            500                 505                 510

Ile Met Arg Ala Val Glu Gly Glu Pro Tyr Ala Asp Val Leu Ser Met
        515                 520                 525

Met Leu Leu Arg Ser Met Gln Gln Ala Arg Tyr Ser Glu His Asn His
    530                 535                 540

Gly His Tyr Gly Leu Ala Ala Asp Tyr Tyr Thr His Phe Thr Ser Pro
545                 550                 555                 560

Ile Arg Arg Tyr Pro Asp Leu Leu Val His Arg Met Ile Arg Asp Tyr
                565                 570                 575

Gly Arg Ser Lys Glu Ile Ala Glu His Phe Glu Gln Val Ile Pro Glu
            580                 585                 590

Ile Ala Thr Gln Ser Ser Asn Arg Glu Arg Arg Ala Ile Glu Ala Glu
        595                 600                 605

Arg Glu Val Glu Ala Met Lys Lys Ala Glu Tyr Met Glu Glu Tyr Val
    610                 615                 620

Gly Glu Glu Tyr Asp Ala Val Val Ser Ser Ile Val Lys Phe Gly Leu
625                 630                 635                 640

Phe Val Glu Leu Pro Asn Thr Val Glu Gly Leu Ile His Ile Thr Asn
                645                 650                 655

Leu Pro Glu Phe Tyr His Phe Asn Glu Arg Asp Leu Thr Leu Arg Gly
            660                 665                 670

Glu Lys Ser Gly Ile Thr Phe Arg Val Gly Gln Gln Ile Arg Ile Arg
        675                 680                 685

Val Glu Arg Ala Asp Lys Met Thr Gly Glu Ile Asp Phe Ser Phe Val
    690                 695                 700

Pro Ser Glu Phe Asp Val Ile Glu Lys Gly Leu Lys Gln Ser Ser Arg
705                 710                 715                 720

Ser Gly Arg Gly Arg Asp Ser Asn Arg Arg Ser Asp Lys Lys Glu Asp
                725                 730                 735

Lys Arg Lys Ser Gly Arg Ser Asn Asp Lys Arg Lys His Ser Gln Lys
            740                 745                 750

Asp Lys Lys Lys Lys Gly Lys Lys Pro Phe Tyr Lys Glu Val Ala Lys
        755                 760                 765

Lys Gly Ala Lys His Gly Lys Gly Arg Gly Lys Gly Arg Arg Thr Lys
    770                 775                 780

<210> 37
<211> 513
<212> PRT
<213> Streptococcus pneumoniae

<400> 37
Met Gly Thr Thr Gly Phe Thr Ile Ile Asp Leu Ile Ile Leu Ile Val
  1               5                  10                  15

Tyr Leu Leu Ala Val Leu Val Ala Gly Ile Tyr Phe Ser Lys Lys Glu
             20                  25                  30

Met Lys Gly Lys Glu Phe Phe Lys Gly Asp Gly Ser Val Pro Trp Tyr
         35                  40                  45

Val Thr Ser Val Ser Ile Phe Ala Thr Met Leu Ser Pro Ile Ser Phe
     50                  55                  60

Leu Gly Leu Ala Gly Ser Ser Tyr Ala Gly Ser Trp Ile Leu Trp Phe
 65                  70                  75                  80

Ala Gln Leu Gly Met Val Val Ala Ile Pro Leu Thr Ile Arg Phe Ile
                 85                  90                  95

Leu Pro Ile Phe Ala Arg Ile Asp Ile Asp Thr Ala Tyr Asp Tyr Leu
            100                 105                 110

Asp Lys Arg Phe Asn Ser Lys Ala Leu Arg Ile Ile Ser Ala Leu Leu
        115                 120                 125

Phe Ile Ile Tyr Gln Leu Gly Arg Met Ser Ile Ile Met Tyr Leu Pro
    130                 135                 140

Ser Ala Gly Leu Ser Val Leu Thr Gly Ile Asp Ile Asn Ile Leu Ile
145                 150                 155                 160

Ile Leu Met Gly Val Val Ala Ile Val Tyr Ser Tyr Thr Gly Gly Leu
                165                 170                 175

Lys Ser Val Leu Trp Thr Asp Phe Ile Gln Gly Val Ile Leu Ile Ser

            180                 185                 190

Gly Val Val Leu Ala Leu Phe Val Leu Ile Ala Asn Ile Lys Gly Gly
        195                 200                 205

Phe Gly Ala Val Ala Glu Thr Leu Ala Asn Gly Lys Phe Leu Ala Ala
    210                 215                 220

Asn Glu Lys Leu Phe Asp Pro Asn Leu Leu Ser Asn Ser Ile Phe Leu
225                 230                 235                 240

Ile Val Met Gly Ser Gly Phe Thr Ile Leu Ser Ser Tyr Ala Ser Ser
                245                 250                 255

Gln Asp Leu Val Gln Arg Phe Thr Thr Thr Gln Asn Ile Lys Lys Leu
            260                 265                 270

Asn Lys Met Leu Phe Thr Asn Gly Val Leu Ser Leu Ala Thr Ala Thr
        275                 280                 285

Val Phe Tyr Leu Ile Gly Thr Gly Leu Tyr Val Phe Tyr Gln Val Gln
    290                 295                 300

Asn Ala Asp Ser Ala Ala Ser Asn Ile Pro Gln Asp Gln Ile Phe Met
305                 310                 315                 320

Tyr Phe Ile Ala Tyr Gln Leu Pro Val Gly Ile Thr Gly Leu Ile Leu
                325                 330                 335

Ala Ala Ile Tyr Ala Ala Ser Gln Ser Thr Ile Ser Thr Gly Leu Asn
            340                 345                 350

Ser Val Ala Thr Ser Trp Thr Leu Asp Ile Gln Asp Val Ile Ser Lys
        355                 360                 365

Asn Met Ser Asp Asn Arg Arg Thr Lys Ile Ala Gln Phe Val Ser Leu
    370                 375                 380

Ala Val Gly Leu Phe Ser Ile Gly Val Ser Ile Val Met Ala His Ser
385                 390                 395                 400

Asp Ile Lys Ser Ala Tyr Glu Trp Phe Asn Ser Phe Met Gly Leu Val
                405                 410                 415

Leu Gly Leu Leu Gly Gly Val Phe Ile Leu Gly Phe Val Ser Lys Lys
            420                 425                 430

Ala Asn Lys Gln Gly Ala Tyr Ala Ala Leu Ile Val Ser Thr Ile Val
        435                 440                 445

Met Val Phe Ile Lys Tyr Phe Leu Pro Pro Thr Ala Val Ser Tyr Trp
    450                 455                 460

Ala Tyr Ser Leu Ile Ser Ile Ser Val Ser Val Val Ser Gly Tyr Ile
465                 470                 475                 480

Val Ser Val Leu Thr Gly Asn Lys Val Ser Ala Pro Lys Tyr Thr Thr
                485                 490                 495

Ile His Asp Ile Thr Glu Ile Lys Ala Asp Ser Ser Trp Glu Val Arg
            500                 505                 510

His

<210> 38
<211> 484
<212> PRT
<213> Streptococcus pneumoniae

<400> 38
Met Lys Phe Ser Lys Lys Tyr Ile Ala Ala Gly Ser Ala Val Ile Val
1               5                   10                  15

Ser Leu Ser Leu Cys Ala Tyr Ala Leu Asn Gln His Arg Ser Gln Glu
            20                  25                  30

Asn Lys Asp Asn Asn Arg Val Ser Tyr Val Asp Gly Ser Gln Ser Ser
        35                  40                  45

Gln Lys Ser Glu Asn Leu Thr Pro Asp Gln Val Ser Gln Lys Glu Gly
    50                  55                  60

Ile Gln Ala Glu Gln Ile Val Ile Lys Ile Thr Asp Gln Gly Tyr Val
65                  70                  75                  80

Thr Ser His Gly Asp His Tyr His Tyr Tyr Asn Gly Lys Val Pro Tyr
                85                  90                  95

Asp Ala Leu Phe Ser Glu Glu Leu Leu Met Lys Asp Pro Asn Tyr Gln
            100                 105                 110

Leu Lys Asp Ala Asp Ile Val Asn Glu Val Lys Gly Gly Tyr Ile Ile
        115                 120                 125

Lys Val Asp Gly Lys Tyr Tyr Val Tyr Leu Lys Asp Ala Ala His Ala
    130                 135                 140

Asp Asn Val Arg Thr Lys Asp Glu Ile Asn Arg Gln Lys Gln Glu His
145                 150                 155                 160

Val Lys Asp Asn Glu Lys Val Asn Ser Asn Val Ala Val Ala Arg Ser
                165                 170                 175

Gln Gly Arg Tyr Thr Thr Asn Asp Gly Tyr Val Phe Asn Pro Ala Asp
            180                 185                 190

Ile Ile Glu Asp Thr Gly Asn Ala Tyr Ile Val Pro His Gly Gly His
        195                 200                 205

Tyr His Tyr Ile Pro Lys Ser Asp Leu Ser Ala Ser Glu Leu Ala Ala
    210                 215                 220

Ala Lys Ala His Leu Ala Gly Lys Asn Met Gln Pro Ser Gln Leu Ser
225                 230                 235                 240

Tyr Ser Ser Thr Ala Ser Asp Asn Asn Thr Gln Ser Val Ala Lys Gly
                245                 250                 255

Ser Thr Ser Lys Pro Ala Asn Lys Ser Glu Asn Leu Gln Ser Leu Leu

260                 265                 270

Lys Glu Leu Tyr Asp Ser Pro Ser Ala Gln Arg Tyr Ser Glu Ser Asp
        275                 280                 285

Gly Leu Val Phe Asp Pro Ala Lys Ile Ile Ser Arg Thr Pro Asn Gly
    290                 295                 300

Val Ala Ile Pro His Gly Asp His Tyr His Phe Ile Pro Tyr Ser Lys
305                 310                 315                 320

Leu Ser Ala Leu Glu Glu Lys Ile Ala Arg Met Val Pro Ile Ser Gly
                325                 330                 335

Thr Gly Ser Thr Val Ser Thr Asn Ala Lys Pro Asn Glu Val Val Ser
            340                 345                 350

Ser Leu Gly Ser Leu Ser Ser Asn Pro Ser Ser Leu Thr Thr Ser Lys
        355                 360                 365

Glu Leu Ser Ser Ala Ser Asp Gly Tyr Ile Phe Asn Pro Lys Asp Ile
    370                 375                 380

Val Glu Glu Thr Ala Thr Ala Tyr Ile Val Arg His Gly Asp His Phe
385                 390                 395                 400

His Tyr Ile Pro Lys Ser Asn Gln Ile Gly Gln Pro Thr Leu Pro Asn
                405                 410                 415

Asn Ser Leu Ala Thr Pro Ser Pro Ser Leu Pro Ile Asn Pro Gly Thr
            420                 425                 430

Ser His Glu Lys His Glu Glu Asp Gly Tyr Gly Phe Asp Ala Asn Arg
        435                 440                 445

Ile Ile Ala Glu Asp Glu Ser Gly Phe Val Met Ser His Gly Asp His
    450                 455                 460

Asn His Tyr Phe Phe Lys Lys Asp Leu Thr Glu Glu Gln Ile Lys Val
465                 470                 475                 480

Arg Lys Asn Ile

<210> 39
<211> 153
<212> PRT
<213> Streptococcus pneumoniae

<400> 39
Met Lys Lys Arg Ala Ile Val Ala Val Ile Val Leu Leu Leu Ile Gly
1               5                   10                  15

Leu Asp Gln Leu Val Lys Ser Tyr Ile Val Gln Gln Ile Pro Leu Gly
            20                  25                  30

Glu Val Arg Ser Trp Ile Pro Asn Phe Val Ser Leu Thr Tyr Leu Gln
        35                  40                  45

Asn Arg Gly Ala Ala Phe Ser Ile Leu Gln Asp Gln Gln Leu Leu Phe
        50                  55                  60

Ala Val Ile Thr Leu Val Val Val Ile Gly Ala Ile Trp Tyr Leu His
 65                  70                  75                  80

Lys His Met Glu Asp Ser Phe Trp Met Val Leu Gly Leu Thr Leu Ile
                 85                  90                  95

Ile Ala Gly Gly Leu Gly Asn Phe Ile Asp Arg Val Ser Gln Gly Phe
            100                 105                 110

Val Val Asp Met Phe His Leu Asp Phe Ile Asn Phe Ala Ile Phe Asn
        115                 120                 125

Val Ala Asp Ser Tyr Leu Thr Val Gly Val Ile Ile Leu Leu Ile Ala
    130                 135                 140

Met Leu Lys Glu Glu Ile Asn Gly Asn
145                 150

<210> 40
<211> 284
<212> PRT
<213> Streptococcus pneumoniae

<400> 40
Met Asn Thr Asn Leu Ala Ser Phe Ile Val Gly Leu Ile Ile Asp Glu
  1               5                  10                  15

Asn Asp Arg Phe Tyr Phe Val Gln Lys Asp Gly Gln Thr Tyr Ala Leu
             20                  25                  30

Ala Lys Glu Glu Gly Gln His Thr Val Gly Asp Thr Val Lys Gly Phe
         35                  40                  45

Ala Tyr Thr Asp Met Lys Gln Lys Leu Arg Leu Thr Thr Leu Glu Val
     50                  55                  60

Thr Ala Thr Gln Asp Gln Phe Gly Trp Gly Arg Val Thr Glu Val Arg
 65                  70                  75                  80

Lys Asp Leu Gly Val Phe Val Asp Thr Gly Leu Pro Asp Lys Glu Ile
                 85                  90                  95

Val Val Ser Leu Asp Ile Leu Pro Glu Leu Lys Glu Leu Trp Pro Lys
            100                 105                 110

Lys Gly Asp Gln Leu Tyr Ile Arg Leu Glu Val Asp Lys Lys Asp Arg
        115                 120                 125

Ile Trp Gly Leu Leu Ala Tyr Gln Glu Asp Phe Gln Arg Leu Ala Arg
    130                 135                 140

Pro Ala Tyr Asn Asn Met Gln Asn Gln Asn Trp Pro Ala Ile Val Tyr
145                 150                 155                 160

Arg Leu Lys Leu Ser Gly Thr Phe Val Tyr Leu Pro Glu Asn Asn Met
                165                 170                 175

Leu Gly Phe Ile His Pro Ser Glu Arg Tyr Ala Glu Pro Arg Leu Gly
            180                 185                 190

Gln Val Leu Asp Ala Arg Val Ile Gly Phe Arg Glu Val Asp Arg Thr
        195                 200                 205

Leu Asn Leu Ser Leu Lys Pro Arg Ser Phe Glu Met Leu Glu Asn Asp
    210                 215                 220

Ala Gln Met Ile Leu Thr Tyr Leu Glu Ser Asn Gly Gly Phe Met Thr
225                 230                 235                 240

Leu Asn Asp Lys Ser Ser Pro Asp Asp Ile Lys Ala Thr Phe Gly Ile
                245                 250                 255

Ser Lys Gly Gln Phe Lys Lys Ala Leu Gly Gly Leu Met Lys Ala Gly
            260                 265                 270

Lys Ile Lys Gln Asp Gln Phe Gly Thr Glu Leu Ile
        275                 280

<210> 41
<211> 394
<212> PRT
<213> Streptococcus pneumoniae

<400> 41
Met Lys Asp Val Ser Leu Phe Leu Leu Lys Lys Val Phe Lys Ser Arg
1               5                   10                  15

Leu Asn Trp Ile Val Leu Ala Leu Phe Val Ser Val Leu Gly Val Thr
            20                  25                  30

Phe Tyr Leu Asn Ser Gln Thr Ala Asn Ser His Ser Leu Glu Ser Arg
        35                  40                  45

Leu Glu Ser Arg Ile Ala Ala Asn Glu Arg Ala Ile Asn Glu Asn Glu
    50                  55                  60

Glu Lys Leu Ser Gln Met Ser Asp Thr Ser Ser Glu Glu Tyr Gln Phe
65                  70                  75                  80

Ala Lys Asn Asn Leu Asp Val Gln Lys Asn Leu Leu Thr Arg Lys Thr
                85                  90                  95

Glu Ile Leu Thr Leu Leu Lys Glu Gly Arg Trp Lys Glu Ala Tyr Tyr
            100                 105                 110

Leu Gln Trp Gln Asp Glu Glu Lys Asn Tyr Glu Phe Val Ser Asn Asp
        115                 120                 125

Pro Thr Ala Ser Pro Gly Leu Lys Met Gly Val Asp Arg Glu Arg Lys
    130                 135                 140

Ile Tyr Gln Ala Leu Tyr Pro Leu Asn Ile Lys Ala His Thr Leu Glu
145                 150                 155                 160

Phe Pro Thr His Gly Ile Asp Gln Ile Val Trp Ile Leu Glu Val Ile

                    165                 170                 175

Ile Pro Ser Leu Phe Val Val Ala Ile Ile Phe Met Leu Thr Gln Leu
            180                 185                 190

Phe Ala Glu Arg Tyr Gln Asn His Leu Asp Thr Ala His Leu Tyr Pro
        195                 200                 205

Val Ser Lys Val Thr Phe Ala Ile Ser Ser Leu Gly Val Gly Val Gly
    210                 215                 220

Tyr Val Thr Val Leu Phe Ile Gly Ile Cys Gly Phe Ser Phe Leu Val
225                 230                 235                 240

Gly Ser Leu Ile Ser Gly Phe Gly Gln Leu Asp Tyr Pro Tyr Pro Ile
                245                 250                 255

Tyr Ser Leu Val Asn Gln Glu Val Thr Ile Gly Lys Ile Gln Asp Val
            260                 265                 270

Leu Phe Pro Gly Leu Leu Leu Ala Phe Leu Ala Phe Ile Val Ile Val
        275                 280                 285

Glu Val Val Tyr Leu Ile Ala Tyr Phe Phe Lys Gln Lys Met Pro Val
    290                 295                 300

Leu Phe Leu Ser Leu Ile Gly Ile Val Gly Leu Leu Phe Gly Ile Gln
305                 310                 315                 320

Thr Ile Gln Pro Leu Gln Arg Ile Ala His Leu Ile Pro Phe Thr Tyr
                325                 330                 335

Leu Arg Ser Val Glu Ile Leu Ser Gly Arg Leu Pro Lys Gln Ile Asp
            340                 345                 350

Asn Val Asp Leu Asn Trp Ser Met Gly Met Val Leu Leu Pro Cys Leu
        355                 360                 365

Ile Ile Phe Leu Leu Leu Gly Ile Leu Phe Ile Glu Arg Trp Gly Ser
    370                 375                 380

Ser Gln Lys Lys Glu Phe Phe Asn Arg Phe
385                 390

<210> 42
<211> 448
<212> PRT
<213> Streptococcus pneumoniae

<400> 42
Met Met Lys Phe Ile Leu Asp Ile Val Ser Thr Pro Ala Ile Leu Val
1               5                   10                  15

Ala Leu Ile Ala Ile Leu Gly Leu Val Leu Gln Lys Lys Lys Leu Pro
            20                  25                  30

Asp Ile Ile Lys Gly Gly Ile Lys Thr Phe Val Gly Phe Leu Val Val
        35                  40                  45

Ser Gly Gly Ala Gly Ile Val Gln Asn Ser Leu Asn Pro Phe Gly Thr
    50                  55                  60

Met Phe Glu His Ala Phe His Leu Ser Gly Val Val Pro Asn Asn Glu
65                  70                  75                  80

Ala Ile Val Ala Val Ala Leu Thr Thr Tyr Gly Ser Ala Thr Ala Met
                85                  90                  95

Ile Met Phe Ala Gly Met Val Phe Asn Ile Leu Ile Ala Arg Phe Thr
            100                 105                 110

Arg Phe Lys Tyr Ile Phe Leu Thr Gly His His Thr Leu Tyr Met Ala
        115                 120                 125

Cys Met Ile Ala Val Ile Leu Ser Val Ala Gly Phe Thr Ser Leu Pro
    130                 135                 140

Leu Ile Leu Leu Gly Gly Leu Ala Leu Gly Ile Ile Met Ser Ile Ser
145                 150                 155                 160

Pro Ala Phe Val Gln Lys Tyr Met Val Gln Leu Thr Gly Asn Asp Lys
                165                 170                 175

Val Ala Leu Gly His Phe Ser Ser Leu Gly Tyr Trp Leu Ser Gly Phe
            180                 185                 190

Thr Gly Ser Leu Ile Gly Asp Lys Ser Lys Ser Thr Glu Asp Ile Lys
        195                 200                 205

Phe Pro Lys Ser Leu Ala Phe Leu Arg Asp Ser Thr Val Ser Ile Thr
    210                 215                 220

Leu Ser Met Ala Val Ile Tyr Ile Ile Val Ala Ile Phe Ala Gly Ser
225                 230                 235                 240

Glu Tyr Ile Glu Lys Glu Ile Ser Ser Gly Thr Ser Gly Leu Val Tyr
                245                 250                 255

Ala Leu Gln Leu Ala Gly Gln Phe Ala Ala Gly Val Phe Val Ile Leu
            260                 265                 270

Ala Gly Val Arg Leu Ile Leu Gly Glu Ile Val Pro Ala Phe Lys Gly
        275                 280                 285

Ile Ser Glu Arg Leu Val Pro Asn Ser Lys Pro Ala Leu Asp Cys Pro
    290                 295                 300

Ile Val Tyr Thr Tyr Ala Pro Asn Ala Val Leu Ile Gly Phe Ile Ser
305                 310                 315                 320

Ser Phe Val Gly Gly Leu Val Ser Met Val Ile Met Ile Ala Ser Gly
                325                 330                 335

Thr Val Val Ile Leu Pro Gly Val Val Pro His Phe Phe Cys Gly Ala
            340                 345                 350

Thr Ala Gly Val Ile Gly Asn Ala Ser Gly Gly Val Arg Gly Ala Thr
        355                 360                 365

Ile Gly Ala Phe Leu Gln Gly Ile Leu Ile Ser Phe Leu Pro Val Phe

370                 375                 380

Leu Met Pro Val Leu Gly Gly Leu Gly Phe Gln Gly Ser Thr Phe Ser
385                 390                 395                 400

Asp Ala Asp Phe Gly Leu Ser Gly Ile Ile Leu Gly Met Leu Asn Gln
                405                 410                 415

Phe Gly Ser Gln Ala Gly Ile Val Ile Gly Leu Val Leu Ile Leu Ala
            420                 425                 430

Val Met Phe Gly Val Ser Phe Ile Lys Lys Pro Ser Ala Thr Glu Glu
        435                 440                 445

<210> 43
<211> 333
<212> PRT
<213> Streptococcus pneumoniae

<400> 43
Met Ile Lys Thr Phe Leu Ser Ala Leu Ser Val Ile Leu Phe Ser Ile
  1               5                  10                  15

Pro Ile Ile Thr Tyr Ser Phe Phe Pro Ser Ser Asn Leu Asn Ile Trp
             20                  25                  30

Leu Ser Thr Gln Pro Ile Leu Ala Gln Ile Tyr Ala Phe Pro Leu Ala
         35                  40                  45

Thr Ala Thr Met Ala Ala Ile Leu Ser Phe Leu Phe Phe Phe Leu Ser
     50                  55                  60

Phe Tyr Lys Lys Asn Lys Gln Ile Arg Phe Tyr Ser Gly Ile Leu Leu
 65                  70                  75                  80

Leu Leu Ser Leu Ile Leu Leu Leu Phe Gly Thr Asp Lys Thr Leu Ser
                 85                  90                  95

Ser Ala Ser Asn Lys Thr Lys Thr Leu Lys Leu Val Thr Trp Asn Val
            100                 105                 110

Ala Asn Gln Ile Glu Ala Gln His Ile Glu Arg Ile Phe Ser His Phe
        115                 120                 125

Asp Ala Asp Met Ala Ile Phe Pro Glu Leu Ala Thr Asn Ile Arg Gly
    130                 135                 140

Glu Gln Glu Asn Gln Arg Ile Lys Leu Leu Phe His Gln Val Gly Leu
145                 150                 155                 160

Ser Met Ala Asn Tyr Asp Ile Phe Thr Ser Pro Pro Thr Asn Ser Gly
                165                 170                 175

Ile Ala Pro Val Thr Val Ile Val Lys Lys Ser Tyr Gly Phe Tyr Thr
            180                 185                 190

Glu Ala Lys Thr Phe His Thr Thr Arg Phe Gly Thr Ile Val Leu His
        195                 200                 205

Ser Arg Lys Gln Asn Ile Pro Asp Ile Ile Ala Leu His Thr Ala Pro
    210                 215                 220

Pro Leu Pro Gly Leu Met Glu Ile Trp Lys Gln Asp Leu Asn Ile Ile
225                 230                 235                 240

His Asn Gln Leu Ala Ser Lys Tyr Pro Lys Ala Ile Ile Ala Gly Asp
                245                 250                 255

Phe Asn Ala Thr Met Arg His Gly Ala Leu Ala Lys Ile Ser Ser His
            260                 265                 270

Arg Asp Ala Leu Asn Ala Leu Pro Pro Phe Glu Arg Gly Thr Trp Asn
        275                 280                 285

Ser Gln Ser Pro Lys Leu Phe Asn Ala Thr Ile Asp His Ile Leu Leu
    290                 295                 300

Pro Lys Asn His Tyr Tyr Val Lys Asp Leu Asp Ile Val Ser Phe Gln
305                 310                 315                 320

Asn Ser Asp His Arg Cys Ile Phe Thr Glu Ile Thr Phe
                325                 330

<210> 44
<211> 425
<212> PRT
<213> Streptococcus pneumoniae

<400> 44
Met Asn Pro Ile Gln Arg Ser Trp Ala Tyr Val Ser Arg Lys Arg Leu
1               5                   10                  15

Arg Ser Phe Ile Leu Phe Leu Ile Leu Leu Val Leu Leu Ala Gly Ile
            20                  25                  30

Ser Ala Cys Leu Thr Leu Met Lys Ser Asn Lys Thr Val Glu Ser Asn
        35                  40                  45

Leu Tyr Lys Ser Leu Asn Thr Ser Phe Ser Ile Lys Lys Ile Glu Asn
    50                  55                  60

Gly Gln Thr Phe Lys Leu Ser Asp Leu Ala Ser Val Ser Lys Ile Lys
65                  70                  75                  80

Gly Leu Glu Asn Val Ser Pro Glu Leu Glu Thr Val Ala Lys Leu Lys
                85                  90                  95

Asp Lys Glu Ala Val Thr Gly Glu Gln Ser Val Glu Arg Asp Asp Leu
            100                 105                 110

Ser Ala Ala Asp Asn Asn Leu Val Ser Leu Thr Ala Leu Glu Asp Ser
        115                 120                 125

Ser Lys Asp Val Thr Phe Thr Ser Ser Ala Phe Asn Leu Lys Glu Gly
    130                 135                 140

Arg His Leu Gln Lys Gly Asp Ser Lys Lys Ile Leu Ile His Glu Glu
145                 150                 155                 160

Leu Ala Lys Lys Asn Gly Leu Ser Leu His Asp Lys Ile Gly Leu Asp
                165                 170                 175

Ala Gly Gln Ser Glu Ser Gly Lys Gly Gln Thr Val Glu Phe Glu Ile
            180                 185                 190

Ile Gly Ile Phe Ser Gly Lys Lys Gln Glu Lys Phe Thr Gly Leu Ser
        195                 200                 205

Ser Asp Phe Ser Glu Asn Gln Val Phe Thr Asp Tyr Glu Ser Ser Gln
    210                 215                 220

Thr Leu Leu Gly Asn Ser Glu Ala Gln Val Ser Ala Ala Arg Phe Tyr
225                 230                 235                 240

Val Glu Asn Pro Lys Glu Met Asp Gly Leu Met Lys Gln Val Glu Asn
                245                 250                 255

Leu Ala Leu Glu Asn Gln Gly Tyr Gln Val Glu Lys Glu Asn Lys Ala
            260                 265                 270

Phe Glu Gln Ile Lys Asp Ser Val Ala Thr Phe Gln Thr Phe Leu Thr
        275                 280                 285

Ile Phe Leu Tyr Gly Met Leu Ile Ala Gly Ala Gly Ala Leu Ile Leu
    290                 295                 300

Val Leu Ser Leu Trp Leu Arg Glu Arg Val Tyr Glu Val Gly Ile Leu
305                 310                 315                 320

Leu Ala Leu Gly Lys Gly Lys Ser Ser Ile Phe Leu Gln Phe Cys Leu
                325                 330                 335

Glu Val Val Leu Val Ser Leu Gly Ala Leu Leu Pro Ala Phe Val Ala
            340                 345                 350

Gly Asn Ala Ile Thr Thr Tyr Leu Leu Gln Thr Leu Leu Ala Ser Gly
        355                 360                 365

Asp Gln Ala Ser Leu Gln Asp Thr Leu Ala Lys Ala Ser Ser Leu Ser
    370                 375                 380

Thr Ser Ile Leu Ser Phe Ala Glu Ser Tyr Val Phe Leu Val Leu Leu
385                 390                 395                 400

Ser Cys Leu Ser Val Ala Leu Cys Phe Leu Phe Leu Phe Arg Lys Ser
                405                 410                 415

Pro Lys Glu Ile Leu Ser Ser Ile Ser
            420                 425

<210> 45
<211> 459
<212> PRT
<213> Streptococcus pneumoniae

<400> 45
Met Leu His Asn Ala Phe Ala Tyr Val Thr Arg Lys Phe Phe Lys Ser
1               5                   10                  15

Ile Val Ile Phe Leu Ile Ile Leu Leu Met Ala Ser Leu Ser Leu Val
            20                  25                  30

Gly Leu Ser Ile Lys Gly Ala Thr Ala Lys Ala Ser Gln Glu Thr Phe
        35                  40                  45

Lys Asn Ile Thr Asn Ser Phe Ser Met Gln Ile Asn Arg Arg Val Asn
    50                  55                  60

Gln Gly Thr Pro Arg Gly Ala Gly Asn Ile Lys Gly Glu Asp Ile Lys
65                  70                  75                  80

Lys Ile Thr Glu Asn Lys Ala Ile Glu Ser Tyr Val Lys Arg Ile Asn
                85                  90                  95

Ala Ile Gly Asp Leu Thr Gly Tyr Asp Leu Ile Glu Thr Pro Glu Thr
            100                 105                 110

Lys Lys Asn Leu Thr Ala Asp Arg Ala Lys Arg Phe Gly Ser Ser Leu
        115                 120                 125

Met Ile Thr Gly Val Asn Asp Ser Ser Lys Glu Asp Lys Phe Val Ser
    130                 135                 140

Gly Ser Tyr Lys Leu Val Glu Gly Glu His Leu Thr Asn Asp Asp Lys
145                 150                 155                 160

Asp Lys Ile Leu Leu His Lys Asp Leu Ala Ala Lys His Gly Trp Lys
                165                 170                 175

Val Gly Asp Lys Val Lys Leu Asp Ser Asn Ile Tyr Asp Ala Asp Asn
            180                 185                 190

Glu Lys Gly Ala Lys Glu Thr Val Glu Val Thr Ile Lys Gly Leu Phe
        195                 200                 205

Asp Gly His Asn Lys Ser Ala Val Thr Tyr Ser Gln Glu Leu Tyr Glu
    210                 215                 220

Asn Thr Ala Ile Thr Asp Ile His Thr Ala Ala Lys Leu Tyr Gly Tyr
225                 230                 235                 240

Thr Glu Asp Thr Ala Ile Tyr Gly Asp Ala Thr Phe Phe Val Thr Ala
                245                 250                 255

Asp Lys Asn Leu Asp Asp Val Met Lys Glu Leu Asn Gly Ile Ser Gly
            260                 265                 270

Ile Asn Trp Lys Ser Tyr Thr Leu Val Lys Ser Ser Ser Asn Tyr Pro
        275                 280                 285

Ala Leu Glu Gln Ser Ile Ser Gly Met Tyr Lys Met Ala Asn Leu Leu
    290                 295                 300

Phe Trp Gly Ser Leu Ser Phe Ser Val Leu Leu Leu Ala Leu Leu Leu
305                 310                 315                 320

Ser Leu Trp Ile Asn Ala Arg Arg Lys Glu Val Gly Ile Leu Leu Ser
                325                 330                 335

Ile Gly Leu Lys Gln Ala Ser Ile Leu Gly Gln Phe Ile Thr Glu Ser
            340                 345                 350

Ile Leu Ile Ala Ile Pro Ala Leu Val Ser Ala Tyr Phe Leu Ala Asn
        355                 360                 365

Tyr Thr Ala Arg Ala Ile Gly Asn Thr Val Leu Ala Asn Val Thr Ser
    370                 375                 380

Gly Val Ala Lys Gln Ala Ser Lys Ala Ala Gln Ala Ser Asn Leu Gly
385                 390                 395                 400

Gly Gly Ala Glu Val Asp Gly Phe Ser Lys Thr Leu Ser Ser Leu Asp
                405                 410                 415

Ile Ser Ile Gln Thr Ser Asp Phe Ile Ile Ile Phe Val Leu Ala Leu
            420                 425                 430

Val Leu Val Val Leu Val Met Ala Leu Ala Ser Ser Asn Leu Leu Arg
        435                 440                 445

Lys Gln Pro Lys Glu Leu Leu Leu Asp Gly Glu
    450                 455

<210> 46
<211> 205
<212> PRT
<213> Streptococcus pneumoniae

<400> 46
Met Ser Gln Asp Lys Gln Met Lys Ala Val Ser Pro Leu Leu Gln Arg
  1               5                  10                  15

Val Ile Asn Ile Ser Ser Ile Val Gly Gly Val Gly Ser Leu Ile Phe
             20                  25                  30

Cys Ile Trp Ala Tyr Gln Ala Gly Ile Leu Gln Ser Lys Glu Thr Leu
         35                  40                  45

Ser Ala Phe Ile Gln Gln Ala Gly Ile Trp Gly Pro Pro Leu Phe Ile
     50                  55                  60

Phe Leu Gln Ile Leu Gln Thr Val Val Pro Ile Ile Pro Gly Ala Leu
 65                  70                  75                  80

Thr Ser Val Ala Gly Val Phe Ile Tyr Gly His Ile Ile Gly Thr Ile
                 85                  90                  95

Tyr Asn Tyr Ile Gly Ile Val Ile Gly Cys Ala Ile Ile Phe Tyr Leu
            100                 105                 110

Val Arg Leu Tyr Gly Ala Ala Phe Val Gln Ser Val Val Ser Lys Arg
        115                 120                 125

Thr Tyr Asp Lys Tyr Ile Asp Trp Leu Asp Lys Gly Asn Arg Phe Asp

130                 135                 140

Arg Phe Phe Ile Phe Met Met Ile Trp Pro Ile Ser Pro Ala Asp Phe
145                 150                 155                 160

Leu Cys Met Leu Ala Ala Leu Thr Lys Met Ser Phe Lys Arg Tyr Met
                165                 170                 175

Thr Ile Ile Ile Leu Thr Lys Pro Phe Thr Leu Val Val Tyr Thr Tyr
            180                 185                 190

Gly Leu Thr Tyr Ile Ile Asp Phe Phe Trp Gln Met Leu
        195                 200                 205

<210> 47
<211> 399
<212> PRT
<213> Streptococcus pneumoniae

<400> 47
Met Arg Asn Met Trp Val Val Ile Lys Glu Thr Tyr Leu Arg His Val
1               5                   10                  15

Glu Ser Trp Ser Phe Phe Phe Met Val Ile Ser Pro Phe Leu Phe Leu
            20                  25                  30

Gly Ile Ser Val Gly Ile Gly His Leu Gln Gly Ser Ser Met Ala Lys
        35                  40                  45

Asn Asn Lys Val Ala Val Val Thr Thr Val Pro Ser Val Ala Glu Gly
    50                  55                  60

Leu Lys Asn Val Asn Gly Val Asn Phe Asp Tyr Lys Asp Glu Ala Ser
65                  70                  75                  80

Ala Lys Glu Ala Ile Lys Glu Glu Lys Leu Lys Gly Tyr Leu Thr Ile
                85                  90                  95

Asp Gln Glu Asp Ser Val Leu Lys Ala Val Tyr His Gly Glu Thr Ser
            100                 105                 110

Leu Glu Asn Gly Ile Lys Phe Glu Val Thr Gly Thr Leu Asn Glu Leu
        115                 120                 125

Gln Asn Gln Leu Asn Arg Ser Thr Ala Ser Leu Ser Gln Glu Gln Glu
    130                 135                 140

Lys Arg Leu Ala Gln Thr Ile Gln Phe Thr Glu Lys Ile Asp Glu Ala
145                 150                 155                 160

Lys Glu Asn Lys Lys Phe Ile Gln Thr Ile Ala Ala Gly Ala Leu Gly
                165                 170                 175

Phe Phe Leu Tyr Met Ile Leu Ile Thr Tyr Ala Gly Val Thr Ala Gln
            180                 185                 190

Glu Val Ala Ser Glu Lys Gly Thr Lys Ile Met Glu Val Val Phe Ser
        195                 200                 205

Ser Ile Arg Ala Ser His Tyr Phe Tyr Ala Arg Met Met Ala Leu Phe
    210                 215                 220

Leu Val Ile Leu Thr His Ile Gly Ile Tyr Val Val Gly Gly Leu Ala
225                 230                 235                 240

Ala Val Leu Leu Phe Lys Asp Leu Pro Phe Leu Ala Gln Ser Gly Ile
                245                 250                 255

Leu Asp His Leu Gly Asp Ala Ile Ser Leu Asn Thr Leu Leu Phe Ile
            260                 265                 270

Leu Ile Ser Leu Phe Met Tyr Val Val Leu Ala Ala Phe Leu Gly Ser
        275                 280                 285

Met Val Ser Arg Pro Glu Asp Ser Gly Lys Ala Leu Ser Pro Leu Met
    290                 295                 300

Ile Leu Ile Met Gly Gly Phe Phe Gly Val Thr Ala Leu Gly Ala Ala
305                 310                 315                 320

Gly Asp Asn Leu Leu Leu Lys Ile Gly Ser Tyr Ile Pro Phe Ile Ser
                325                 330                 335

Thr Phe Phe Met Pro Phe Arg Thr Ile Asn Asp Tyr Ala Gly Gly Ala
            340                 345                 350

Glu Ala Trp Ile Ser Leu Ala Ile Thr Val Ile Phe Ala Val Val Ala
        355                 360                 365

Thr Gly Phe Ile Gly Arg Met Tyr Ala Ser Leu Val Leu Gln Thr Asp
    370                 375                 380

Asp Leu Gly Ile Trp Lys Thr Phe Lys Arg Ala Leu Ser Tyr Lys
385                 390                 395

<210> 48
<211> 438
<212> PRT
<213> Streptococcus pneumoniae

<400> 48
Met Thr Glu Thr Ile Lys Leu Met Lys Ala His Thr Ser Val Arg Arg
1               5                   10                  15

Phe Lys Glu Gln Glu Ile Pro Gln Val Asp Leu Asn Glu Ile Leu Thr
            20                  25                  30

Ala Ala Gln Met Ala Ser Ser Trp Lys Asn Phe Gln Ser Tyr Ser Val
        35                  40                  45

Ile Val Val Arg Ser Gln Glu Lys Lys Asp Ala Leu Tyr Glu Leu Val
    50                  55                  60

Pro Gln Glu Ala Ile Arg Gln Ser Ala Val Phe Leu Leu Phe Val Gly
65                  70                  75                  80

Asp Leu Asn Arg Ala Glu Lys Gly Ala Arg Leu His Thr Asp Thr Phe
                85                  90                  95

Gln Pro Gln Gly Val Glu Gly Leu Leu Ile Ser Ser Val Asp Ala Ala
            100                 105                 110
Leu Ala Gly Gln Asn Ala Leu Leu Ala Ala Glu Ser Leu Gly Tyr Gly
        115                 120                 125
Gly Val Ile Ile Gly Leu Val Arg Tyr Lys Ser Glu Glu Val Ala Glu
    130                 135                 140
Leu Phe Asn Leu Pro Asp Tyr Thr Tyr Ser Val Phe Gly Met Ala Leu
145                 150                 155                 160
Gly Val Pro Asn Gln His His Asp Met Lys Pro Arg Leu Pro Leu Glu
                165                 170                 175
Asn Val Val Phe Glu Glu Glu Tyr Gln Glu Gln Ser Thr Glu Ala Ile
            180                 185                 190
Gln Ala Tyr Asp Arg Val Gln Ala Asp Tyr Ala Gly Ala Arg Ala Thr
        195                 200                 205
Thr Ser Trp Ser Gln Arg Leu Ala Glu Gln Phe Gly Gln Ala Glu Pro
    210                 215                 220
Ser Ser Thr Arg Lys Asn Leu Glu Gln Lys Lys Leu Leu Met Leu Lys
225                 230                 235                 240
Leu Ile Ala Ile Val Gly Thr Asn Ser Lys Arg Ser Thr Asn Arg Gln
                245                 250                 255
Leu Leu Gln Tyr Met Gln Lys His Phe Thr Asp Lys Ala Glu Ile Glu
            260                 265                 270
Leu Val Glu Ile Lys Ala Ile Pro Val Phe Asn Lys Pro Ala Asp Lys
        275                 280                 285
Gln Val Pro Ala Glu Ile Leu Glu Ile Ala Ala Lys Ile Glu Glu Ala
    290                 295                 300
Asp Gly Val Ile Ile Gly Thr Pro Glu Tyr Asp His Ser Ile Pro Ala
305                 310                 315                 320
Val Leu Met Ser Ala Leu Ala Trp Leu Ser Tyr Gly Ile Tyr Pro Leu
                325                 330                 335
Leu Asn Lys Pro Ile Met Ile Thr Gly Ala Ser Tyr Gly Thr Leu Gly
            340                 345                 350
Ser Ser Arg Ala Gln Leu Gln Leu Arg Gln Ile Leu Asn Ala Pro Glu
        355                 360                 365
Ile Lys Ala Asn Val Leu Pro Asp Glu Phe Leu Leu Ser His Ser Leu
    370                 375                 380
Gln Ala Phe Asn Pro Ser Gly Asp Leu Val Asp Leu Asp Val Ile Lys
385                 390                 395                 400
Lys Leu Asp Ala Ile Phe Asp Asp Phe Arg Ile Phe Val Lys Ile Thr
                405                 410                 415

Glu Lys Leu Arg Asn Ala Gln Glu Leu Leu Arg Lys Asp Ala Glu Asp
            420                 425                 430

Phe Asp Trp Glu Asn Leu
        435

<210> 49
<211> 280
<212> PRT
<213> Streptococcus pneumoniae

<400> 49
Met Asn Thr Tyr Gln Leu Asn Asn Gly Val Glu Ile Pro Val Leu Gly
1               5                   10                  15

Phe Gly Thr Phe Lys Ala Lys Asp Gly Glu Glu Ala Tyr Arg Ala Val
            20                  25                  30

Leu Glu Ala Leu Lys Ala Gly Tyr Arg His Ile Asp Thr Ala Ala Ile
        35                  40                  45

Tyr Gln Asn Glu Glu Ser Val Gly Gln Ala Ile Lys Asp Ser Gly Val
    50                  55                  60

Pro Arg Glu Glu Met Phe Val Thr Thr Lys Leu Trp Asn Ser Gln Gln
65                  70                  75                  80

Thr Tyr Glu Gln Thr Arg Gln Ala Leu Glu Lys Ser Ile Glu Lys Leu
                85                  90                  95

Gly Leu Asp Tyr Leu Asp Leu Tyr Leu Ile His Trp Pro Asn Pro Lys
            100                 105                 110

Pro Leu Arg Glu Asn Asp Ala Trp Lys Thr Arg Asn Ala Glu Val Trp
        115                 120                 125

Arg Ala Met Glu Asp Leu Tyr Gln Glu Gly Lys Ile Arg Ala Ile Gly
    130                 135                 140

Val Ser Asn Phe Leu Pro His His Leu Asp Ala Leu Leu Glu Thr Ala
145                 150                 155                 160

Thr Ile Val Pro Ala Val Asn Gln Val Arg Leu Ala Pro Gly Val Tyr
                165                 170                 175

Gln Asp Gln Val Val Ala Tyr Cys Arg Glu Lys Gly Ile Leu Leu Glu
            180                 185                 190

Ala Trp Gly Pro Phe Gly Gln Gly Glu Leu Phe Asp Ser Lys Gln Val
        195                 200                 205

Gln Glu Ile Ala Ala Asn His Gly Lys Ser Val Ala Gln Ile Ala Leu
    210                 215                 220

Ala Trp Ser Leu Ala Glu Gly Phe Leu Pro Leu Pro Lys Ser Val Thr
225                 230                 235                 240

Thr Ser Arg Ile Gln Ala Asn Leu Asp Cys Phe Gly Ile Glu Leu Ser
                245                 250                 255

His Glu Glu Arg Glu Thr Leu Lys Thr Ile Ala Val Gln Ser Gly Ala
            260                 265                 270

Pro Arg Val Asp Asp Val Asp Phe
        275                 280

<210> 50
<211> 266
<212> PRT
<213> Streptococcus pneumoniae

<400> 50
Met Arg Cys Lys Met Leu Asp Pro Ile Ala Ile Gln Leu Gly Pro Leu
1               5                   10                  15

Ala Ile Arg Trp Tyr Ala Leu Cys Ile Val Thr Gly Leu Ile Leu Ala
            20                  25                  30

Val Tyr Leu Thr Met Lys Glu Ala Pro Arg Lys Lys Ile Ile Pro Asp
        35                  40                  45

Asp Ile Leu Asp Phe Ile Leu Val Ala Phe Pro Leu Ala Ile Leu Gly
    50                  55                  60

Ala Arg Leu Tyr Tyr Val Ile Phe Arg Phe Asp Tyr Tyr Ser Gln Asn
65                  70                  75                  80

Leu Gly Glu Ile Phe Ala Ile Trp Asn Gly Gly Leu Ala Ile Tyr Gly
                85                  90                  95

Gly Leu Ile Thr Gly Ala Leu Val Leu Tyr Ile Phe Ala Asp Arg Lys
            100                 105                 110

Leu Ile Asn Thr Trp Asp Phe Leu Asp Ile Ala Ala Pro Ser Val Met
        115                 120                 125

Ile Ala Gln Ser Leu Gly Arg Trp Gly Asn Phe Phe Asn Gln Glu Ala
    130                 135                 140

Tyr Gly Ala Thr Val Asp Asn Leu Asp Tyr Leu Pro Gly Phe Ile Arg
145                 150                 155                 160

Asp Gln Met Tyr Ile Glu Gly Ser Tyr Arg Gln Pro Thr Phe Leu Tyr
                165                 170                 175

Glu Ser Leu Trp Asn Leu Leu Gly Phe Ala Leu Ile Leu Ile Phe Arg
            180                 185                 190

Arg Lys Trp Lys Ser Leu Arg Arg Gly His Ile Thr Ala Phe Tyr Leu
        195                 200                 205

Ile Trp Tyr Gly Phe Gly Arg Met Val Ile Glu Gly Met Arg Thr Asp
    210                 215                 220

Ser Leu Met Phe Phe Gly Phe Arg Val Ser Gln Trp Leu Ser Val Val
225                 230                 235                 240

Leu Ile Gly Leu Gly Ile Met Ile Val Ile Tyr Gln Asn Arg Lys Lys

245                 250                 255

Ala Pro Tyr Tyr Ile Thr Glu Glu Glu Asn
            260                 265

<210> 51
<211> 129
<212> PRT
<213> Streptococcus pneumoniae

<400> 51
Met Gly Lys Leu Ser Ser Ile Leu Leu Gly Thr Val Ser Gly Ala Ala
1               5                   10                  15

Leu Ala Leu Phe Leu Thr Ser Asp Lys Gly Lys Gln Val Cys Ser Gln
            20                  25                  30

Ala Gln Asp Phe Leu Asp Asp Leu Arg Glu Asp Pro Glu Tyr Ala Lys
        35                  40                  45

Glu Gln Val Cys Glu Lys Leu Thr Glu Val Lys Glu Gln Ala Thr Asp
    50                  55                  60

Phe Val Leu Lys Thr Lys Glu Gln Val Glu Ser Gly Glu Ile Thr Val
65                  70                  75                  80

Asp Ser Ile Leu Ala Gln Thr Lys Ser Tyr Ala Phe Gln Ala Thr Glu
                85                  90                  95

Ala Ser Lys Asn Gln Leu Asn Asn Leu Lys Glu Gln Trp Gln Glu Lys
            100                 105                 110

Ala Glu Ala Leu Asp Asp Ser Glu Glu Ile Val Ile Asp Ile Thr Glu
        115                 120                 125

Glu

<210> 52
<211> 205
<212> PRT
<213> Streptococcus pneumoniae

<400> 52
Met Lys Thr Lys Leu Ile Phe Trp Gly Ser Met Leu Phe Leu Leu Ser
1               5                   10                  15

Leu Ser Ile Leu Leu Thr Ile Tyr Leu Ala Trp Ile Phe Tyr Pro Met
            20                  25                  30

Glu Ile Gln Trp Leu Asn Leu Thr Asn Arg Val Tyr Leu Lys Pro Glu
        35                  40                  45

Thr Ile Gln Tyr Asn Phe His Ile Leu Met Asn Tyr Leu Thr Asn Pro
    50                  55                  60

Phe Ser Gln Val Leu Gln Met Pro Asp Phe Arg Ser Ser Ala Ala Gly

65                  70                  75                  80

Leu His His Phe Ala Val Val Lys Asn Leu Phe His Leu Val Gln Leu
                85                  90                  95

Val Ala Leu Val Thr Leu Pro Ser Phe Tyr Val Phe Val Asn Arg Ile
            100                 105                 110

Val Lys Lys Asp Phe Leu Ser Leu Tyr Arg Lys Ser Leu Leu Ala Leu
        115                 120                 125

Val Val Leu Pro Val Met Ile Gly Leu Gly Gly Val Leu Ile Gly Phe
    130                 135                 140

Asp Gln Phe Phe Thr Leu Phe His Gln Ile Leu Phe Val Gly Asp Asp
145                 150                 155                 160

Thr Trp Leu Phe Asp Pro Ala Lys Asp Pro Val Ile Met Ile Leu Pro
                165                 170                 175

Glu Thr Phe Phe Leu His Ala Phe Leu Leu Phe Phe Ala Leu Tyr Glu
            180                 185                 190

Asn Phe Phe Gly Tyr Leu Tyr Leu Lys Ser Arg Arg Lys
        195                 200                 205

<210> 53
<211> 637
<212> PRT
<213> Streptococcus pneumoniae

<400> 53
Met Thr Tyr His Phe Thr Glu Glu Tyr Asp Ile Ile Val Ile Gly Ala
1               5                   10                  15

Gly His Ala Gly Val Glu Ala Ser Leu Ala Ala Ser Arg Met Gly Cys
            20                  25                  30

Lys Val Leu Leu Ala Thr Ile Asn Ile Glu Met Leu Ala Phe Met Pro
        35                  40                  45

Cys Asn Pro Ser Ile Gly Gly Ser Ala Lys Gly Ile Val Val Arg Glu
    50                  55                  60

Val Asp Ala Leu Gly Gly Glu Met Ala Lys Thr Ile Asp Lys Thr Tyr
65                  70                  75                  80

Ile Gln Met Lys Met Leu Asn Thr Gly Lys Gly Pro Ala Val Arg Ala
                85                  90                  95

Leu Arg Ala Gln Ala Asp Lys Glu Leu Tyr Ser Lys Glu Met Arg Lys
            100                 105                 110

Thr Val Glu Asn Gln Glu Asn Leu Thr Leu Arg Gln Thr Met Ile Asp
        115                 120                 125

Glu Ile Leu Val Glu Asp Gly Lys Val Val Gly Val Arg Thr Ala Thr
    130                 135                 140

His Gln Glu Tyr Ala Ala Lys Ala Val Ile Val Thr Thr Gly Thr Ala
145                 150                 155                 160

Leu Arg Gly Glu Ile Ile Ile Gly Asp Leu Lys Tyr Ser Ser Gly Pro
                165                 170                 175

Asn His Ser Leu Ala Ser Ile Asn Leu Ala Asp Asn Leu Lys Glu Leu
            180                 185                 190

Gly Leu Glu Ile Gly Arg Phe Lys Thr Gly Thr Pro Pro Arg Val Lys
        195                 200                 205

Ala Ser Ser Ile Asn Tyr Asp Val Thr Glu Ile Gln Pro Gly Asp Glu
    210                 215                 220

Val Pro Asn His Phe Ser Tyr Thr Ser Arg Asp Glu Asp Tyr Val Lys
225                 230                 235                 240

Asp Gln Val Pro Cys Trp Leu Thr Tyr Thr Asn Gly Thr Ser His Glu
                245                 250                 255

Ile Ile Gln Asn Asn Leu His Arg Ala Pro Met Phe Thr Gly Val Val
            260                 265                 270

Lys Gly Val Gly Pro Arg Tyr Cys Pro Ser Ile Glu Asp Lys Ile Val
        275                 280                 285

Arg Phe Ala Asp Lys Glu Arg His Gln Leu Phe Leu Glu Pro Glu Gly
    290                 295                 300

Arg Asn Thr Glu Glu Val Tyr Val Gln Gly Leu Ser Thr Ser Leu Pro
305                 310                 315                 320

Glu Asp Val Gln Arg Asp Leu Val His Ser Ile Lys Gly Leu Glu Asn
                325                 330                 335

Ala Glu Met Met Arg Thr Gly Tyr Ala Ile Glu Tyr Asp Met Val Leu
            340                 345                 350

Pro His Gln Leu Arg Ala Thr Leu Glu Thr Lys Lys Ile Ser Gly Leu
        355                 360                 365

Phe Thr Ala Gly Gln Thr Asn Gly Thr Ser Gly Tyr Glu Glu Ala Ala
    370                 375                 380

Gly Gln Gly Ile Ile Ala Gly Ile Asn Ala Ala Leu Lys Ile Gln Gly
385                 390                 395                 400

Lys Pro Glu Leu Ile Leu Lys Arg Ser Asp Gly Tyr Ile Gly Val Met
                405                 410                 415

Ile Asp Asp Leu Val Thr Lys Gly Thr Ile Glu Pro Tyr Arg Leu Leu
            420                 425                 430

Thr Ser Arg Ala Glu Tyr Arg Leu Ile Leu Arg His Asp Asn Ala Asp
        435                 440                 445

Met Arg Leu Thr Glu Met Gly Arg Glu Ile Gly Leu Val Asp Asp Glu
    450                 455                 460

Arg Trp Ala Arg Phe Glu Ile Lys Lys Asn Gln Phe Asp Asn Glu Met

465                 470                 475                 480

Lys Arg Leu Asp Ser Ile Lys Leu Lys Pro Val Lys Glu Thr Asn Ala
                485                 490                 495

Lys Val Glu Glu Met Gly Phe Lys Pro Leu Thr Asp Ala Val Thr Ala
            500                 505                 510

Lys Glu Phe Leu Arg Arg Pro Glu Val Ser Tyr Gln Asp Val Val Ala
        515                 520                 525

Phe Ile Gly Pro Ala Ala Glu Asp Leu Asp Asp Lys Ile Ile Glu Leu
    530                 535                 540

Ile Glu Thr Glu Ile Lys Tyr Glu Gly Tyr Ile Ser Lys Ala Met Asp
545                 550                 555                 560

Gln Val Ala Lys Met Lys Arg Met Glu Glu Lys Arg Ile Pro Ala Asn
                565                 570                 575

Ile Asp Trp Asp Asp Ile Asp Ser Ile Ala Thr Glu Ala Arg Gln Lys
            580                 585                 590

Phe Lys Leu Ile Asn Pro Glu Thr Ile Gly Gln Ala Ser Arg Ile Ser
        595                 600                 605

Gly Val Asn Pro Ala Asp Ile Ser Ile Leu Met Val Tyr Leu Glu Gly
    610                 615                 620

Lys Asn Arg Ser Ile Ser Lys Thr Leu Gln Lys Ser Lys
625                 630                 635

<210> 54
<211> 185
<212> PRT
<213> Streptococcus pneumoniae

<400> 54
Met Thr Lys Gln Val Leu Leu Val Asp Asp Glu Glu His Ile Leu Lys
1               5                   10                  15

Leu Leu Asp Tyr His Leu Ser Lys Glu Gly Phe Ser Thr Gln Leu Val
            20                  25                  30

Thr Asn Gly Arg Lys Ala Leu Ala Leu Ala Glu Thr Glu Pro Phe Asp
        35                  40                  45

Phe Ile Leu Leu Asp Ile Met Leu Pro Gln Leu Asp Gly Met Glu Val
    50                  55                  60

Cys Lys Arg Leu Arg Ala Lys Gly Val Lys Thr Pro Ile Met Met Val
65                  70                  75                  80

Ser Ala Lys Ser Asp Glu Phe Asp Lys Val Leu Ala Leu Glu Leu Gly
                85                  90                  95

Ala Asp Asp Tyr Leu Thr Lys Pro Phe Ser Pro Arg Glu Leu Leu Ala
            100                 105                 110

Arg Val Lys Ala Val Leu Arg Arg Thr Lys Gly Glu Gln Glu Gly Asp
        115                 120                 125

Asp Ser Asp Asn Ile Ala Asp Asp Ser Trp Leu Phe Gly Thr Leu Lys
    130                 135                 140

Val Tyr Pro Glu Arg His Glu Val Tyr Lys Ala Asn Lys Leu Leu Ser
145                 150                 155                 160

Leu Thr Pro Lys Glu Phe Glu Ser Asp Lys Asn Pro Phe Phe Glu Val
                165                 170                 175

Phe Lys Val Ser Lys Val Thr Ala Gln
            180                 185

<210> 55
<211> 478
<212> PRT
<213> Streptococcus pneumoniae

<400> 55
Met Thr Thr Phe Lys Asp Gly Phe Leu Trp Gly Gly Ala Val Ala Ala
1               5                   10                  15

His Gln Leu Glu Gly Gly Trp Gln Glu Gly Gly Lys Gly Ile Ser Val
            20                  25                  30

Ala Asp Val Met Thr Ala Gly Arg His Gly Val Ala Arg Glu Ile Thr
        35                  40                  45

Leu Gly Val Leu Glu Gly Lys Tyr Tyr Pro Asn His Glu Ala Ile Asp
    50                  55                  60

Phe Tyr His Arg Tyr Lys Glu Asp Ile Ala Leu Phe Ala Glu Met Gly
65                  70                  75                  80

Phe Lys Cys Phe Arg Thr Ser Ile Ala Trp Thr Arg Ile Phe Pro Lys
                85                  90                  95

Gly Asp Glu Leu Glu Pro Asn Glu Glu Gly Leu Gln Phe Tyr Asp Asn
            100                 105                 110

Leu Phe Asp Glu Cys Leu Lys Asn Gly Ile Glu Pro Val Ile Thr Leu
        115                 120                 125

Ser His Phe Glu Met Pro Tyr His Leu Val Thr Glu Tyr Gly Gly Trp
    130                 135                 140

Lys Asn Arg Lys Leu Ile Asp Phe Phe Ala Arg Phe Ala Glu Val Val
145                 150                 155                 160

Phe Lys Arg Tyr Lys Asp Lys Val Lys Tyr Trp Met Thr Phe Asn Glu
                165                 170                 175

Ile Asn Asn Gln Ala Asn Tyr Gln Glu Asp Phe Ala Pro Phe Thr Asn
            180                 185                 190

Ser Gly Ile Val Tyr Glu Glu Gly Asp Asn Arg Glu Ala Ile Met Tyr
        195                 200                 205

Gln Ala Ala His Tyr Glu Leu Val Ala Ser Ala Arg Ala Val Lys Ile
    210                 215                 220

Gly His Glu Ile Asn Pro Asp Phe Gln Ile Gly Cys Met Ile Ala Met
225                 230                 235                 240

Cys Pro Ile Tyr Pro Val Thr Cys Asn Pro Lys Asp Ile Leu Met Ala
                245                 250                 255

Met Lys Ala Met Gln Lys Arg Tyr Tyr Phe Ala Asp Val His Val Leu
            260                 265                 270

Gly Lys Tyr Pro Glu His Ile Phe Lys Tyr Trp Glu Arg Lys Gly Ile
        275                 280                 285

Ser Val Asp Phe Thr Ala Gln Asp Lys Glu Asp Leu Leu Gly Gly Thr
    290                 295                 300

Val Asp Tyr Ile Gly Phe Ser Tyr Tyr Met Ser Phe Ala Ile Asp Ser
305                 310                 315                 320

His Arg Glu Asn Asn Pro Tyr Phe Asp Tyr Leu Glu Thr Glu Asp Leu
                325                 330                 335

Val Lys Asn Asn Tyr Val Lys Ala Ser Glu Trp Glu Trp Gln Ile Asp
            340                 345                 350

Pro Glu Gly Leu Arg Tyr Ala Leu Asn Trp Phe Thr Asp His Tyr His
        355                 360                 365

Leu Pro Leu Phe Ile Val Glu Asn Gly Phe Gly Ala Ile Asp Gln Val
    370                 375                 380

Ala Ala Asp Gly Met Val His Asp Asp Tyr Arg Ile Glu Tyr Leu Gly
385                 390                 395                 400

Ala His Ile Arg Glu Met Lys Lys Ala Val Val Glu Asp Gly Val Asp
                405                 410                 415

Leu Met Gly Tyr Thr Pro Trp Gly Cys Ile Asp Leu Val Ser Ala Gly
            420                 425                 430

Thr Gly Glu Met Arg Lys Arg Tyr Gly Phe Ile Tyr Val Asp Lys Asp
        435                 440                 445

Asp Asn Gly Lys Gly Ser Tyr Asn Arg Ser Pro Lys Lys Ser Phe Gly
    450                 455                 460

Trp Tyr Lys Glu Val Ile Ser Ser Asn Gly Glu Ser Val Glu
465                 470                 475

<210> 56
<211> 448
<212> PRT
<213> Streptococcus pneumoniae

<400> 56
   Met Asp Gln Gln Asn Gly Leu Phe Gly Phe Leu Glu Asn His Val Met
   1               5                   10                  15
   Gly Pro Met Gly Lys Leu Ala Gln Phe Lys Val Val Arg Ala Ile Thr
               20                  25                  30
   Ala Ala Gly Met Ala Ala Val Pro Phe Thr Ile Val Gly Ser Met Phe
           35                  40                  45
   Leu Val Phe Ser Ile Leu Pro Gln Ala Phe Ser Phe Trp Pro Ile Val
       50                  55                  60
   Ala Asp Ile Phe Ser Ala Ser Phe Asp Lys Phe Thr Ser Leu Tyr Met
   65                  70                  75                  80
   Val Ala Asn Tyr Ala Thr Met Gly Ser Leu Ser Leu Tyr Phe Val Leu
                   85                  90                  95
   Ser Leu Ala Tyr Glu Leu Thr Lys Ile Tyr Ala Glu Glu Glu Glu Leu
               100                 105                 110
   Asn Met Asn Pro Leu Asn Gly Ala Leu Leu Ala Leu Met Ala Phe Val
           115                 120                 125
   Met Thr Val Pro Gln Ile Ile Phe Asp Gly Gly Met Met Lys Thr Val
       130                 135                 140
   Thr Ser Leu Lys Glu Gly Ala Val Ile Ala Asp Gly Trp Ala Met Gly
   145                 150                 155                 160
   Asn Val Val Ala Arg Phe Gly Thr Thr Gly Ile Phe Thr Ala Ile Ile
                   165                 170                 175
   Met Ala Ile Val Thr Val Leu Ile Tyr Arg Met Cys Val Lys His Asn
               180                 185                 190
   Trp Val Ile Lys Met Pro Glu Ala Val Pro Glu Gly Val Ser Arg Gly
           195                 200                 205
   Phe Thr Ala Leu Val Pro Gly Phe Val Val Ala Phe Val Val Ile Phe
       210                 215                 220
   Ile Asn Gly Leu Leu Val Ala Met Gly Thr Asp Ile Phe Lys Val Ile
   225                 230                 235                 240
   Ala Ile Pro Phe Gly Phe Val Ser Asn Leu Thr Asn Ser Trp Ile Gly
                   245                 250                 255
   Leu Met Ile Ile Tyr Leu Leu Thr Gln Leu Leu Trp Ile Val Gly Ile
               260                 265                 270
   His Gly Ala Asn Ile Val Phe Ala Phe Val Ser Pro Ile Ala Leu Ala
           275                 280                 285
   Asn Met Ala Glu Asn Ala Ala Gly Gly His Phe Ala Val Ala Gly Glu
       290                 295                 300
   Phe Ser Asn Met Phe Val Ile Ala Gly Gly Ser Gly Ala Thr Leu Gly
   305                 310                 315                 320
   Leu Cys Leu Tyr Ile Ala Phe Ala Ser Lys Ser Glu Gln Leu Lys Ala
                   325                 330                 335
   Ile Gly Arg Ala Ser Val Val Pro Ala Leu Phe Asn Ile Asn Glu Pro
               340                 345                 350
   Leu Ile Phe Gly Leu Pro Ile Ile Tyr Asn Pro Ala Leu Ala Ile Pro
           355                 360                 365
   Phe Ile Leu Ala Pro Met Val Thr Ala Thr Ile Tyr Tyr Val Ala Asn
       370                 375                 380
   Ser Leu Asn Phe Ile Lys Pro Ile Ile Ala Gln Val Pro Trp Pro Thr
   385                 390                 395                 400
   Pro Val Gly Ile Gly Ala Phe Leu Gly Thr Ala Asp Leu Arg Ala Val
                   405                 410                 415
   Leu Val Ala Leu Val Cys Ala Phe Ala Ala Phe Leu Val Tyr Leu Pro
               420                 425                 430
   Phe Ile Arg Val Tyr Asp Gln Lys Leu Val Lys Glu Glu Gln Gly Ile
           435                 440                 445

<210> 57
<211> 657
<212> PRT
<213> Streptococcus pneumoniae

<400> 57
Met Lys Lys Phe Tyr Val Ser Pro Ile Phe Pro Ile Leu Val Gly Leu
  1               5                  10                  15

Ile Ala Phe Gly Val Leu Ser Thr Phe Ile Ile Phe Val Asn Asn Asn
             20                  25                  30

Leu Leu Thr Val Leu Ile Leu Phe Leu Phe Val Gly Gly Tyr Val Phe
         35                  40                  45

Leu Phe Lys Lys Leu Arg Val His Tyr Thr Arg Ser Asp Val Glu Gln
     50                  55                  60

Ile Gln Tyr Val Asn His Gln Ala Glu Glu Ser Leu Thr Ala Leu Leu
 65                  70                  75                  80

Glu Gln Met Pro Val Gly Val Met Lys Leu Asn Leu Ser Ser Gly Glu
                 85                  90                  95

Val Glu Trp Phe Asn Pro Tyr Ala Glu Leu Ile Leu Thr Lys Glu Asp
            100                 105                 110

Gly Asp Phe Asp Leu Glu Ala Val Gln Thr Ile Ile Lys Ala Ser Val
        115                 120                 125

Gly Asn Pro Ser Thr Tyr Ala Lys Leu Gly Glu Lys Arg Tyr Ala Val
    130                 135                 140

His Met Asp Ala Ser Ser Gly Val Leu Tyr Phe Val Asp Val Ser Arg

145                 150                 155                 160

Glu Gln Ala Ile Thr Asp Glu Leu Val Thr Ser Arg Pro Val Ile Gly
                165                 170                 175

Ile Val Ser Val Asp Asn Tyr Asp Asp Leu Glu Asp Glu Thr Ser Glu
            180                 185                 190

Ser Asp Ile Ser Gln Ile Asn Ser Phe Val Ala Asn Phe Ile Ser Glu
        195                 200                 205

Phe Ser Glu Lys His Met Met Phe Ser Arg Arg Val Ser Met Asp Arg
    210                 215                 220

Phe Tyr Leu Phe Thr Asp Tyr Thr Val Leu Glu Gly Leu Met Asn Asp
225                 230                 235                 240

Lys Phe Ser Val Ile Asp Ala Phe Arg Glu Glu Ser Lys Gln Arg Gln
                245                 250                 255

Leu Pro Leu Thr Leu Ser Met Gly Phe Ser Tyr Gly Asp Gly Asn His
            260                 265                 270

Asp Glu Ile Gly Lys Val Ala Leu Leu Asn Leu Asn Leu Ala Glu Val
        275                 280                 285

Arg Gly Gly Asp Gln Val Val Val Lys Glu Asn Asp Glu Thr Lys Asn
    290                 295                 300

Pro Val Tyr Phe Gly Gly Gly Ser Ala Ala Ser Ile Lys Arg Thr Arg
305                 310                 315                 320

Thr Arg Thr Arg Ala Met Met Thr Ala Ile Ser Asp Lys Ile Arg Ser
                325                 330                 335

Val Asp Gln Val Phe Val Val Gly His Lys Asn Leu Asp Met Asp Ala
            340                 345                 350

Leu Gly Ser Ala Val Gly Met Gln Leu Phe Ala Ser Asn Val Ile Glu
        355                 360                 365

Asn Ser Tyr Ala Leu Tyr Asp Glu Glu Gln Met Ser Pro Asp Ile Glu
    370                 375                 380

Arg Ala Val Ser Phe Ile Glu Lys Glu Gly Val Thr Lys Leu Leu Ser
385                 390                 395                 400

Val Lys Asp Ala Met Gly Met Val Thr Asn Arg Ser Leu Leu Ile Leu
                405                 410                 415

Val Asp His Ser Lys Thr Ala Leu Thr Leu Ser Lys Glu Phe Tyr Asp
            420                 425                 430

Leu Phe Thr Gln Thr Ile Val Ile Asp His His Arg Arg Asp Gln Asp
        435                 440                 445

Phe Pro Asp Asn Ala Val Ile Thr Tyr Ile Glu Ser Gly Ala Ser Ser
    450                 455                 460

Ala Ser Glu Leu Val Thr Glu Leu Ile Gln Phe Gln Asn Ser Lys Lys
465                 470                 475                 480

Asn Arg Leu Ser Arg Met Gln Ala Ser Val Leu Met Ala Gly Met Met
                485                 490                 495

Leu Asp Thr Lys Asn Phe Thr Ser Arg Val Thr Ser Arg Thr Phe Asp
            500                 505                 510

Val Ala Ser Tyr Leu Arg Thr Arg Gly Ser Asp Ser Ile Ala Ile Gln
        515                 520                 525

Glu Ile Ala Ala Thr Asp Phe Glu Glu Tyr Arg Glu Val Asn Glu Leu
    530                 535                 540

Ile Leu Gln Gly Arg Lys Leu Gly Ser Asp Val Leu Ile Ala Glu Ala
545                 550                 555                 560

Lys Asp Met Lys Cys Tyr Asp Thr Val Val Ile Ser Lys Ala Ala Asp
                565                 570                 575

Ala Met Leu Ala Met Ser Gly Ile Glu Ala Ser Phe Val Leu Ala Lys
            580                 585                 590

Asn Thr Gln Gly Phe Ile Ser Ile Ser Ala Arg Ser Arg Ser Lys Leu
        595                 600                 605

Asn Val Gln Arg Ile Met Glu Glu Leu Gly Gly Gly Gly His Phe Asn
    610                 615                 620

Leu Ala Ala Ala Gln Ile Lys Asp Val Thr Leu Ser Glu Ala Gly Glu
625                 630                 635                 640

Lys Leu Thr Glu Ile Val Leu Asn Glu Met Lys Glu Lys Glu Lys Glu
                645                 650                 655

Glu

<210> 58
<211> 235
<212> PRT
<213> Streptococcus pneumoniae

<400> 58
Met Lys Glu Lys Asn Met Trp Lys Glu Leu Leu Asn Arg Ala Gly Trp
  1               5                  10                  15

Ile Leu Val Phe Leu Leu Ala Val Leu Leu Tyr Gln Val Pro Leu Val
             20                  25                  30

Val Thr Ser Ile Leu Thr Leu Lys Glu Val Ala Leu Leu Gln Ser Gly
         35                  40                  45

Leu Ile Val Ala Gly Leu Ser Ile Val Val Leu Ala Leu Phe Ile Met
     50                  55                  60

Gly Ala Arg Lys Thr Lys Leu Ala Ser Phe Asn Phe Ser Phe Phe Arg
 65                  70                  75                  80

Ala Lys Asp Leu Ala Arg Leu Gly Leu Ser Tyr Leu Val Ile Val Gly

85                  90                  95

Ser Asn Ile Leu Gly Ser Ile Leu Leu Gln Leu Ser Asn Glu Thr Thr
            100                 105                 110

Thr Ala Asn Gln Ser Gln Ile Asn Asp Met Val Gln Asn Ser Ser Leu
        115                 120                 125

Ile Ser Ser Phe Phe Leu Leu Ala Leu Leu Ala Pro Ile Cys Glu Glu
    130                 135                 140

Ile Leu Cys Arg Gly Ile Val Pro Lys Lys Ile Phe Arg Gly Lys Glu
145                 150                 155                 160

Asn Leu Gly Phe Val Val Gly Thr Ile Val Phe Ala Leu Leu His Gln
                165                 170                 175

Pro Ser Asn Leu Pro Ser Leu Leu Ile Tyr Gly Gly Met Ser Thr Val
            180                 185                 190

Leu Ser Trp Thr Ala Tyr Lys Thr Gln Arg Leu Glu Met Ser Ile Leu
        195                 200                 205

Leu His Met Ile Val Asn Gly Ile Ala Phe Cys Leu Leu Ala Leu Val
    210                 215                 220

Val Ile Met Ser Arg Thr Leu Gly Ile Ser Val
225                 230                 235

<210> 59
<211> 235
<212> PRT
<213> Streptococcus pneumoniae

<400> 59
Met Lys Glu Lys Asn Met Trp Lys Glu Leu Leu Asn Arg Ala Gly Trp
1               5                   10                  15

Ile Leu Val Phe Leu Leu Ala Val Leu Leu Tyr Gln Val Pro Leu Val
            20                  25                  30

Val Thr Ser Ile Leu Thr Leu Lys Glu Val Ala Leu Leu Gln Ser Gly
        35                  40                  45

Leu Ile Val Ala Gly Leu Ser Ile Val Val Leu Ala Leu Phe Ile Met
    50                  55                  60

Gly Ala Arg Lys Thr Lys Leu Ala Ser Phe Asn Phe Ser Phe Phe Arg
65                  70                  75                  80

Ala Lys Asp Leu Ala Arg Leu Gly Leu Ser Tyr Leu Val Ile Val Gly
                85                  90                  95

Ser Asn Ile Leu Gly Ser Ile Leu Leu Gln Leu Ser Asn Glu Thr Thr
            100                 105                 110

Thr Ala Asn Gln Ser Gln Ile Asn Asp Met Val Gln Asn Ser Ser Leu
        115                 120                 125

Ile Ser Ser Phe Phe Leu Leu Ala Leu Leu Ala Pro Ile Cys Glu Glu
    130                 135                 140

Ile Leu Cys Arg Gly Ile Val Pro Lys Lys Ile Phe Arg Gly Lys Glu
145                 150                 155                 160

Asn Leu Gly Phe Val Val Gly Thr Ile Val Phe Ala Leu Leu His Gln
                165                 170                 175

Pro Ser Asn Leu Pro Ser Leu Leu Ile Tyr Gly Gly Met Ser Thr Val
            180                 185                 190

Leu Ser Trp Thr Ala Tyr Lys Thr Gln Arg Leu Glu Met Ser Ile Leu
        195                 200                 205

Leu His Met Ile Val Asn Gly Ile Ala Phe Cys Leu Leu Ala Leu Val
    210                 215                 220

Val Ile Met Ser Arg Thr Leu Gly Ile Ser Val
225                 230                 235

<210> 60
<211> 533
<212> PRT
<213> Streptococcus pneumoniae

<400> 60
Met Asp Thr Gln Lys Ile Glu Ala Ala Val Lys Met Ile Ile Glu Ala
  1               5                  10                  15

Val Gly Glu Asp Ala Asn Arg Glu Gly Leu Gln Glu Thr Pro Ala Arg
             20                  25                  30

Val Ala Arg Met Tyr Gln Glu Ile Phe Ser Gly Leu Gly Gln Thr Ala
         35                  40                  45

Glu Glu His Leu Ser Lys Ser Phe Glu Ile Ile Asp Asp Asn Met Val
     50                  55                  60

Val Glu Lys Asp Ile Phe Phe His Thr Met Cys Glu His His Phe Leu
 65                  70                  75                  80

Pro Phe Tyr Gly Arg Ala His Ile Ala Tyr Ile Pro Asp Gly Arg Val
                 85                  90                  95

Ala Gly Leu Ser Lys Leu Ala Arg Thr Val Glu Val Tyr Ser Lys Lys
            100                 105                 110

Pro Gln Ile Gln Glu Arg Leu Asn Ile Glu Val Ala Asp Ala Leu Met
        115                 120                 125

Asp Tyr Leu Gly Ala Lys Gly Ala Phe Val Val Ile Glu Ala Glu His
    130                 135                 140

Met Cys Met Ser Met Arg Gly Val Arg Lys Pro Gly Thr Ala Thr Leu
145                 150                 155                 160

Thr Thr Val Ala Arg Gly Leu Phe Glu Thr Asp Lys Asp Leu Arg Asp
                165                 170                 175

Gln Ala Tyr Arg Leu Met Gly Leu Met Lys Asp Leu Phe Leu Lys Arg
            180                 185                 190

Lys Gln Ala Phe Arg Lys Glu Cys Leu Gly Tyr Leu Arg Tyr Val Leu
        195                 200                 205

Asn Asp His Phe Val Leu Phe Leu Leu Val Leu Leu Gly Phe Leu Ala
    210                 215                 220

Tyr Gln Tyr Ser Gln Leu Leu Gln His Phe Pro Glu Asn His Trp Pro
225                 230                 235                 240

Ile Leu Leu Phe Val Gly Ile Thr Ser Val Leu Leu Leu Leu Trp Gly
                245                 250                 255

Gly Thr Ala Thr Tyr Met Glu Ala Pro Asp Lys Leu Phe Leu Leu Val
            260                 265                 270

Gly Glu Glu Glu Ile Lys Leu His Leu Lys Arg Gln Thr Gly Ile Ser
        275                 280                 285

Leu Val Phe Trp Leu Phe Val Gln Thr Leu Phe Leu Leu Leu Phe Ala
    290                 295                 300

Pro Leu Phe Leu Ala Met Gly Tyr Gly Leu Pro Val Phe Leu Leu Tyr
305                 310                 315                 320

Val Leu Leu Leu Gly Val Gly Lys Tyr Phe His Phe Cys Gln Lys Ala
                325                 330                 335

Ser Lys Phe Phe Thr Glu Thr Gly Leu Asp Trp Asp Tyr Val Ile Ser
            340                 345                 350

Gln Glu Ser Lys Arg Lys Gln Val Leu Leu Arg Phe Phe Ala Leu Phe
        355                 360                 365

Thr Gln Val Lys Gly Ile Ser Asn Ser Val Lys Arg Arg Ala Tyr Leu
    370                 375                 380

Asp Phe Ile Leu Lys Ala Val Gln Lys Val Pro Gly Lys Ile Trp Gln
385                 390                 395                 400

Asn Leu Tyr Leu Arg Ser Tyr Leu Arg Asn Gly Asp Leu Phe Ala Leu
                405                 410                 415

Ser Leu Arg Leu Leu Leu Leu Ser Leu Leu Ala Gln Val Phe Ile Glu
            420                 425                 430

Gln Ala Trp Ile Ala Thr Ala Val Val Val Leu Phe Asn Tyr Leu Leu
        435                 440                 445

Leu Phe Gln Leu Leu Ala Leu Tyr His Ala Phe Asp Tyr Gln Tyr Leu
    450                 455                 460

Thr Gln Leu Phe Pro Leu Asp Lys Gly Gln Lys Glu Lys Gly Leu Gln
465                 470                 475                 480

Glu Val Val Arg Gly Leu Thr Ser Phe Val Leu Leu Val Glu Leu Val
                485                 490                 495

Val Gly Leu Ile Thr Phe Gln Glu Lys Leu Ala Leu Leu Ala Leu Leu
            500                 505                 510

Gly Ala Gly Leu Val Leu Leu Val Leu Tyr Leu Pro Tyr Gln Val Lys
        515                 520                 525

Arg Gln Met Gln Asp
    530

<210> 61
<211> 398
<212> PRT
<213> Streptococcus pneumoniae

<400> 61
Met Arg Lys Ser Ile Val Leu Ala Ala Asp Asn Ala Tyr Leu Ile Pro
1               5                   10                  15

Leu Glu Thr Thr Ile Lys Ser Val Leu Tyr His Asn Arg Asp Val Asp
            20                  25                  30

Phe Tyr Ile Leu Asn Ser Asp Ile Ala Pro Glu Trp Phe Lys Leu Leu
        35                  40                  45

Gly Arg Lys Met Glu Val Val Asn Ser Thr Ile Arg Ser Val His Ile
    50                  55                  60

Asp Lys Glu Leu Phe Glu Ser Tyr Lys Thr Gly Pro His Ile Asn Tyr
65                  70                  75                  80

Ala Ser Tyr Phe Arg Phe Phe Ala Thr Glu Val Val Glu Ser Asp Arg
                85                  90                  95

Val Leu Tyr Leu Asp Ser Asp Ile Ile Val Thr Gly Glu Leu Ala Thr
            100                 105                 110

Leu Phe Glu Ile Asp Leu Lys Gly Tyr Ser Ile Gly Ala Val Asp Asp
        115                 120                 125

Val Tyr Ala Tyr Glu Gly Arg Lys Ser Gly Phe Asn Thr Gly Met Leu
    130                 135                 140

Leu Met Asp Val Ala Lys Trp Lys Glu His Ser Ile Val Asn Ser Leu
145                 150                 155                 160

Leu Glu Leu Ala Ala Glu Gln Asn Gln Val Val His Leu Gly Asp Gln
                165                 170                 175

Ser Ile Leu Asn Ile Tyr Phe Glu Asp Asn Trp Leu Ala Leu Asp Lys
            180                 185                 190

Thr Tyr Asn Tyr Met Val Gly Ile Asp Ile Tyr His Leu Ala Gln Glu
        195                 200                 205

Cys Glu Arg Leu Asp Asp Asn Pro Pro Thr Ile Val His Tyr Ala Ser
    210                 215                 220

His Asp Lys Pro Trp Asn Thr Tyr Ser Ile Ser Arg Leu Arg Glu Leu
225                 230                 235                 240

Trp Trp Val Tyr Arg Asp Leu Asp Trp Ser Glu Ile Ala Phe Gln Arg
                245                 250                 255

Ser Asp Leu Asn Tyr Phe Glu Arg Ser Asn Gln Ser Lys Lys Gln Val
            260                 265                 270

Met Leu Val Thr Trp Ser Ala Asp Ile Lys His Leu Glu Tyr Leu Val
        275                 280                 285

Gln Arg Leu Pro Asp Trp His Phe His Leu Ala Ala Pro Cys Asp Cys
    290                 295                 300

Ser Glu Glu Leu Thr Ser Leu Ser Gln Tyr Thr Asn Val Thr Val Tyr
305                 310                 315                 320

Gln Asn Val Leu His Ser Arg Ile Asp Trp Leu Leu Asp Asp Ser Ile
                325                 330                 335

Val Tyr Leu Asp Ile Asn Thr Gly Gly Glu Val Phe Asn Val Val Thr
            340                 345                 350

Arg Ala Gln Glu Ser Gly Lys Lys Ile Phe Ala Phe Asp Ile Thr Arg
        355                 360                 365

Lys Ser Met Asp Asp Gly Leu Tyr Asp Gly Ile Phe Ser Val Glu Arg
    370                 375                 380

Pro Asp Asp Leu Val Asp Arg Met Lys Asn Ile Glu Ile Glu
385                 390                 395

<210> 62
<211> 405
<212> PRT
<213> Streptococcus pneumoniae

<400> 62
Met Thr Lys Ile Tyr Ser Ser Ile Ala Val Lys Lys Gly Leu Phe Thr
1               5                   10                  15

Ser Phe Leu Leu Phe Ile Tyr Val Leu Gly Ser Arg Ile Ile Leu Pro
            20                  25                  30

Phe Val Asp Leu Asn Thr Lys Asp Phe Leu Gly Gly Ser Thr Ala Tyr
        35                  40                  45

Leu Ala Phe Ser Ala Ala Leu Thr Gly Gly Asn Leu Arg Ser Leu Ser
    50                  55                  60

Ile Phe Ser Val Gly Leu Ser Pro Trp Met Ser Ala Met Ile Leu Trp
65                  70                  75                  80

Gln Met Phe Ser Phe Ser Lys Arg Leu Gly Leu Thr Ser Thr Ser Ile
                85                  90                  95

Glu Ile Gln Asp Arg Arg Lys Met Tyr Leu Thr Leu Leu Ile Ala Val
            100                 105                 110

Ile Gln Ser Leu Ala Val Ser Leu Arg Leu Pro Val Gln Ser Ser Tyr

115                 120                 125

Ser Ala Ile Leu Val Val Leu Met Asn Thr Ile Leu Leu Ile Ala Gly
    130                 135                 140

Thr Phe Phe Leu Val Trp Leu Ser Asp Leu Asn Ala Ser Met Gly Ile
145                 150                 155                 160

Gly Gly Ser Ile Val Ile Leu Leu Ser Ser Met Val Leu Asn Ile Pro
                165                 170                 175

Gln Asp Val Leu Glu Thr Phe Gln Thr Val His Ile Pro Thr Gly Ile
            180                 185                 190

Ile Val Leu Leu Ala Leu Leu Thr Leu Val Phe Ser Tyr Leu Leu Ala
        195                 200                 205

Leu Met Tyr Arg Ala Arg Tyr Leu Val Pro Val Asn Lys Ile Gly Leu
    210                 215                 220

His Asn Arg Phe Lys Arg Tyr Ser Tyr Leu Glu Ile Met Leu Asn Pro
225                 230                 235                 240

Ala Gly Gly Met Pro Tyr Met Tyr Val Met Ser Phe Leu Ser Val Pro
                245                 250                 255

Ala Tyr Leu Phe Ile Leu Leu Gly Phe Ile Phe Pro Asn His Ser Gly
            260                 265                 270

Leu Ala Ala Leu Ser Lys Glu Phe Met Val Gly Lys Pro Leu Trp Val
        275                 280                 285

Tyr Val Tyr Ile Ser Val Leu Phe Leu Phe Ser Ile Ile Phe Ala Phe
    290                 295                 300

Val Thr Met Asn Gly Glu Glu Ile Ala Asp Arg Met Lys Lys Ser Gly
305                 310                 315                 320

Glu Tyr Ile Tyr Gly Ile Tyr Pro Gly Ala Asp Thr Ser Arg Phe Ile
                325                 330                 335

Asn Arg Leu Val Leu Arg Phe Ser Val Ile Gly Gly Leu Phe Asn Val
            340                 345                 350

Ile Met Ala Gly Gly Pro Met Leu Phe Val Leu Phe Asp Glu Lys Leu
        355                 360                 365

Leu Arg Leu Ala Met Ile Pro Gly Leu Phe Met Met Phe Gly Gly Met
    370                 375                 380

Ile Phe Thr Ile Arg Asp Glu Val Lys Ala Leu Arg Leu Asn Glu Thr
385                 390                 395                 400

Tyr Arg Pro Leu Ile
                405

<210> 63
<211> 763
<212> PRT

<213> Streptococcus pneumoniae

<400> 63
Met Ser Ser Leu Ser Asp Gln Glu Leu Val Ala Lys Thr Val Glu Phe
1               5                   10                  15

Arg Gln Arg Leu Ser Glu Gly Glu Ser Leu Asp Asp Ile Leu Val Glu
            20                  25                  30

Ala Phe Ala Val Val Arg Glu Ala Asp Lys Arg Ile Leu Gly Met Phe
        35                  40                  45

Pro Tyr Asp Val Gln Val Met Gly Ala Ile Val Met His Tyr Gly Asn
    50                  55                  60

Val Ala Glu Met Asn Thr Gly Glu Gly Lys Thr Leu Thr Ala Thr Met
65                  70                  75                  80

Pro Val Tyr Leu Asn Ala Phe Ser Gly Glu Gly Val Met Val Val Thr
                85                  90                  95

Pro Asn Glu Tyr Leu Ser Lys Arg Asp Ala Glu Glu Met Gly Gln Val
            100                 105                 110

Tyr Arg Phe Leu Gly Leu Thr Ile Gly Val Pro Phe Thr Glu Asp Pro
        115                 120                 125

Lys Lys Glu Met Lys Ala Glu Glu Lys Lys Leu Ile Tyr Ala Ser Asp
    130                 135                 140

Ile Ile Tyr Thr Thr Asn Ser Asn Leu Gly Phe Asp Tyr Leu Asn Asp
145                 150                 155                 160

Asn Leu Ala Ser Asn Glu Glu Gly Lys Phe Leu Arg Pro Phe Asn Tyr
                165                 170                 175

Val Ile Ile Asp Glu Ile Asp Asp Ile Leu Leu Asp Ser Ala Gln Thr
            180                 185                 190

Pro Leu Ile Ile Ala Gly Ser Pro Arg Val Gln Ser Asn Tyr Tyr Ala
        195                 200                 205

Ile Ile Asp Thr Leu Val Thr Thr Leu Val Glu Gly Glu Asp Tyr Ile
    210                 215                 220

Phe Lys Glu Glu Lys Glu Glu Val Trp Leu Thr Thr Lys Gly Ala Lys
225                 230                 235                 240

Ser Ala Glu Asn Phe Leu Gly Ile Asp Asn Leu Tyr Lys Glu Glu His
                245                 250                 255

Ala Ser Phe Ala Arg His Leu Val Tyr Ala Ile Arg Ala His Lys Leu
            260                 265                 270

Phe Thr Lys Asp Lys Asp Tyr Ile Ile Arg Gly Asn Glu Met Val Leu
        275                 280                 285

Val Asp Lys Gly Thr Gly Arg Leu Met Glu Met Thr Lys Leu Gln Gly
    290                 295                 300

Gly Leu His Gln Ala Ile Glu Ala Lys Glu His Val Lys Leu Ser Pro

305                 310                 315                 320

Glu Thr Arg Ala Met Ala Ser Ile Thr Tyr Gln Ser Leu Phe Lys Met
                325                 330                 335

Phe Asn Lys Ile Ser Gly Met Thr Gly Thr Gly Lys Val Ala Glu Lys
            340                 345                 350

Glu Phe Ile Glu Thr Tyr Asn Met Ser Val Val Arg Ile Pro Thr Asn
        355                 360                 365

Arg Pro Arg Gln Arg Ile Asp Tyr Pro Asp Asn Leu Tyr Ile Thr Leu
    370                 375                 380

Pro Glu Lys Val Tyr Ala Ser Leu Glu Tyr Ile Lys Gln Tyr His Ala
385                 390                 395                 400

Lys Gly Asn Pro Leu Leu Val Phe Val Gly Ser Val Glu Met Ser Gln
                405                 410                 415

Leu Tyr Ser Ser Leu Leu Phe Arg Glu Gly Ile Ala His Asn Val Leu
            420                 425                 430

Asn Ala Asn Asn Ala Ala Arg Glu Ala Gln Ile Ile Ser Glu Ser Gly
        435                 440                 445

Gln Met Gly Ala Val Thr Val Ala Thr Ser Met Ala Gly Arg Gly Thr
    450                 455                 460

Asp Ile Lys Leu Gly Lys Gly Val Ala Glu Leu Gly Gly Leu Ile Val
465                 470                 475                 480

Ile Gly Thr Glu Arg Met Glu Ser Gln Arg Ile Asp Leu Gln Ile Arg
                485                 490                 495

Gly Arg Ser Gly Arg Gln Gly Asp Pro Gly Met Ser Lys Phe Phe Val
            500                 505                 510

Ser Leu Glu Asp Asp Val Ile Lys Lys Phe Gly Pro Ser Trp Val His
        515                 520                 525

Lys Lys Tyr Lys Asp Tyr Gln Val Gln Asp Met Thr Gln Pro Glu Val
    530                 535                 540

Leu Lys Gly Arg Lys Tyr Arg Lys Leu Val Glu Lys Ala Gln His Ala
545                 550                 555                 560

Ser Asp Ser Ala Gly Arg Ser Ala Arg Arg Gln Thr Leu Glu Tyr Ala
                565                 570                 575

Glu Ser Met Asn Ile Gln Arg Asp Ile Val Tyr Lys Glu Arg Asn Arg
            580                 585                 590

Leu Ile Asp Gly Ser Arg Asp Leu Glu Asp Val Val Val Asp Ile Ile
        595                 600                 605

Glu Arg Tyr Thr Glu Glu Val Ala Ala Asp His Tyr Ala Ser Arg Glu
    610                 615                 620

Leu Leu Phe His Phe Ile Val Thr Asn Ile Ser Phe His Val Lys Glu
625                 630                 635                 640

Val Pro Asp Tyr Ile Asp Val Thr Asp Lys Thr Ala Val Arg Ser Phe
                645                 650                 655

Met Lys Gln Val Ile Asp Lys Glu Leu Ser Glu Lys Lys Glu Leu Leu
            660                 665                 670

Asn Gln His Asp Leu Tyr Glu Gln Phe Leu Arg Leu Ser Leu Leu Lys
        675                 680                 685

Ala Ile Asp Asp Asn Trp Val Glu Gln Val Asp Tyr Leu Gln Gln Leu
    690                 695                 700

Ser Met Ala Ile Gly Gly Gln Ser Ala Ser Gln Lys Asn Pro Ile Val
705                 710                 715                 720

Glu Tyr Tyr Gln Glu Ala Tyr Ala Gly Phe Glu Ala Met Lys Glu Gln
                725                 730                 735

Ile His Ala Asp Met Val Arg Asn Leu Leu Met Gly Leu Val Glu Val
            740                 745                 750

Thr Pro Lys Gly Glu Ile Val Thr His Phe Pro
        755                 760

<210> 64
<211> 319
<212> PRT
<213> Streptococcus pneumoniae

<400> 64
Met Ile Gly Thr Phe Ala Ala Ala Leu Val Ala Val Leu Ala Asn Phe
1               5                   10                  15

Ile Val Pro Ile Glu Ile Thr Pro Asn Ser Ala Asn Thr Glu Ile Ala
            20                  25                  30

Pro Pro Asp Gly Ile Gly Gln Val Leu Ser Asn Leu Leu Leu Lys Leu
        35                  40                  45

Val Asp Asn Pro Val Asn Ala Leu Leu Thr Ala Asn Tyr Ile Arg Ile
    50                  55                  60

Leu Ser Trp Ala Val Ile Phe Gly Ile Ala Met Arg Glu Ala Ser Lys
65                  70                  75                  80

Asn Ser Gln Glu Leu Leu Lys Thr Ile Ala Asp Val Thr Ser Lys Ile
                85                  90                  95

Val Glu Trp Ile Ile Asn Leu Ala Pro Phe Gly Ile Leu Gly Leu Val
            100                 105                 110

Phe Lys Thr Ile Ser Asp Lys Gly Val Gly Ser Leu Ala Asn Tyr Gly
        115                 120                 125

Ile Leu Leu Val Leu Leu Val Thr Thr Met Leu Phe Val Ala Pro Val
    130                 135                 140

Val Asn Pro Leu Ile Ala Phe Phe Phe Met Arg Arg Asn Pro Tyr Pro

145                 150                 155                 160

Leu Val Trp Asn Cys Leu Arg Val Ser Gly Val Thr Ala Phe Phe Thr
                165                 170                 175

Arg Ser Ser Ala Thr Asn Ile Pro Val Asn Met Lys Leu Cys His Asp
            180                 185                 190

Leu Gly Leu Asn Pro Asp Thr Tyr Ser Val Ser Ile Pro Leu Gly Ser
        195                 200                 205

Thr Ile Asn Met Ala Gly Val Ala Ile Thr Ile Asn Leu Leu Thr Leu
    210                 215                 220

Ala Ala Val Asn Thr Leu Gly Ile Pro Val Asp Phe Ala Thr Ala Phe
225                 230                 235                 240

Val Leu Ser Val Val Ala Ala Ile Ser Ser Cys Asp Ala Ser Gly Ile
                245                 250                 255

Ala Gly Gly Ser Leu Leu Leu Ile Pro Val Ala Cys Ser Leu Phe Gly
            260                 265                 270

Ile Ser Asn Asp Ile Ala Ile Gln Ile Val Gly Val Gly Phe Val Ile
        275                 280                 285

Gly Val Ile Gln Asp Ser Cys Glu Thr Ala Leu Asn Ser Ser Thr Asp
    290                 295                 300

Val Leu Phe Thr Ala Val Ala Glu Tyr Ala Ala Thr Arg Lys Lys
305                 310                 315

<210> 65
<211> 317
<212> PRT
<213> Streptococcus pneumoniae

<400> 65
Met Ser Ile Ser Gln Arg Thr Thr Lys Leu Ile Leu Ala Thr Cys Leu
1               5                   10                  15

Ala Cys Leu Leu Ala Tyr Phe Leu Asn Leu Ser Ser Ala Val Ser Ala
            20                  25                  30

Gly Ile Ile Ala Leu Leu Ser Leu Ser Asp Thr Arg Arg Ser Thr Leu
        35                  40                  45

Lys Leu Ala Arg Asn Arg Leu Phe Ser Met Leu Leu Ala Leu Ala Ile
    50                  55                  60

Gly Val Leu Ala Phe His Leu Ser Gly Phe His Ile Trp Ser Leu Gly
65                  70                  75                  80

Leu Tyr Leu Ala Phe Tyr Val Pro Leu Ala Tyr Lys Met Gly Trp Glu
                85                  90                  95

Ile Gly Ile Thr Pro Ser Thr Val Leu Val Ser His Leu Leu Val Gln
            100                 105                 110

Glu Ser Thr Ser Pro Asp Leu Leu Val Asn Glu Phe Leu Leu Phe Ala
        115                 120                 125

Ile Gly Thr Gly Phe Ala Leu Leu Val Asn Leu Tyr Met Pro Ser Arg
    130                 135                 140

Glu Glu Glu Ile Gln His Tyr His Thr Leu Val Glu Glu Lys Leu Lys
145                 150                 155                 160

Asp Ile Leu Gln Arg Phe Lys Tyr Tyr Leu Ser Arg Gly Asp Gly Arg
                165                 170                 175

Asn Arg Ala Gln Leu Val Ala Glu Leu Asp Thr Leu Leu Lys Glu Ala
            180                 185                 190

Leu Arg Leu Val Tyr Leu Asp His Ser Asp His Leu Phe His Gln Thr
        195                 200                 205

Asp Tyr His Ile His Tyr Phe Glu Met Arg Gln Arg Gln Ser Arg Ile
    210                 215                 220

Leu Arg Asn Met Ala Gln Gln Ile Asn Thr Cys His Leu Ala Ala Ser
225                 230                 235                 240

Glu Ser Leu Ile Leu Ala Gln Leu Phe Ser Lys Ile Ala Gly Gln Leu
                245                 250                 255

Ser Gln Thr Asn Pro Ala Ser Asp Leu Leu Asp Glu Ile Glu Arg Tyr
            260                 265                 270

Leu Glu Val Phe Arg Asn Arg Ser Leu Pro Lys Thr Arg Glu Glu Phe
        275                 280                 285

Glu Thr Arg Ala Thr Leu Leu Gln Leu Leu Arg Glu Ala Lys Thr Phe
    290                 295                 300

Ile Gln Val Lys Val Asp Phe Tyr Gln Lys Tyr Arg Gln
305                 310                 315

<210> 66
<211> 537
<212> PRT
<213> Streptococcus pneumoniae

<400> 66
Met Glu Ile Met Ser Leu Ala Ile Ala Val Phe Ala Val Ile Ile Gly
1               5                   10                  15

Leu Val Ile Gly Tyr Val Ser Ile Ser Ala Lys Met Lys Ser Ser Gln
            20                  25                  30

Glu Ala Ala Glu Leu Met Leu Leu Asn Ala Glu Gln Glu Ala Thr Asn
        35                  40                  45

Leu Arg Gly Gln Ala Glu Arg Glu Ala Asp Leu Leu Val Asn Glu Ala
    50                  55                  60

Lys Arg Glu Ser Lys Ser Leu Lys Lys Glu Ala Leu Leu Glu Ala Lys
65                  70                  75                  80

Glu Glu Ala Arg Lys Tyr Arg Glu Glu Val Asp Ala Glu Phe Lys Ser
                85                  90                  95

Glu Arg Gln Glu Leu Lys Gln Ile Glu Ser Arg Leu Thr Glu Arg Ala
            100                 105                 110

Thr Ser Leu Asp Arg Lys Asp Asp Asn Leu Thr Ser Lys Glu Gln Thr
        115                 120                 125

Leu Glu Gln Lys Glu Gln Ser Ile Ser Asp Arg Ala Lys Asn Leu Asp
    130                 135                 140

Ala Arg Glu Glu Gln Leu Glu Glu Val Glu Arg Gln Lys Glu Ala Glu
145                 150                 155                 160

Leu Glu Arg Ile Gly Ala Leu Ser Gln Ala Glu Ala Arg Asp Ile Ile
                165                 170                 175

Leu Ala Gln Thr Glu Glu Asn Leu Thr Arg Glu Ile Ala Ser Arg Ile
            180                 185                 190

Arg Glu Ala Glu Gln Glu Val Lys Glu Arg Ser Asp Lys Met Ala Lys
        195                 200                 205

Asp Ile Leu Val Gln Ala Met Gln Arg Ile Ala Gly Glu Tyr Val Ala
    210                 215                 220

Glu Ser Thr Asn Ser Thr Val His Leu Pro Asp Asp Thr Met Lys Gly
225                 230                 235                 240

Arg Ile Ile Gly Arg Glu Gly Arg Asn Ile Arg Thr Phe Glu Ser Leu
                245                 250                 255

Thr Gly Val Asp Val Ile Ile Asp Asp Thr Pro Glu Val Val Thr Leu
            260                 265                 270

Ser Gly Phe Asp Pro Ile Arg Arg Glu Ile Ala Arg Met Thr Met Glu
        275                 280                 285

Met Leu Leu Lys Asp Gly Arg Ile His Pro Ala Arg Ile Glu Glu Leu
    290                 295                 300

Val Glu Lys Asn Arg Gln Glu Ile Asp Asn Lys Ile Arg Glu Tyr Gly
305                 310                 315                 320

Glu Ala Ala Ala Tyr Glu Ile Gly Ala Pro Asn Leu His Pro Asp Leu
                325                 330                 335

Met Lys Ile Met Gly Arg Leu Gln Phe Arg Thr Ser Tyr Gly Gln Asn
            340                 345                 350

Val Leu Arg His Ser Ile Glu Val Ala Lys Leu Ala Gly Ile Met Ala
        355                 360                 365

Ser Glu Leu Gly Glu Asn Ala Ala Leu Ala Arg Arg Ala Gly Phe Leu
    370                 375                 380

His Asp Ile Gly Lys Ala Ile Asp His Glu Val Glu Gly Ser His Val
385                 390                 395                 400

Glu Ile Gly Met Glu Leu Ala Arg Lys Tyr Lys Glu Pro Pro Val Val
                405                 410                 415

Val Asn Thr Ile Ala Ser His His Gly Asp Val Glu Ala Glu Ser Val
            420                 425                 430

Ile Ala Val Ile Val Ala Ala Ala Asp Ala Leu Ser Ala Ala Arg Pro
        435                 440                 445

Gly Ala Arg Ser Glu Ser Leu Glu Ser Tyr Ile Lys Arg Leu His Asp
    450                 455                 460

Leu Glu Glu Ile Ala Asn Gly Phe Glu Gly Val Gln Thr Ser Phe Ala
465                 470                 475                 480

Leu Gln Ala Gly Arg Glu Ile Arg Ile Met Val Asn Pro Gly Lys Ile
                485                 490                 495

Lys Asp Asp Lys Val Thr Ile Leu Ala His Lys Val Arg Lys Lys Ile
            500                 505                 510

Glu Asn Asn Leu Asp Tyr Pro Gly Asn Ile Lys Val Thr Val Ile Arg
        515                 520                 525

Glu Leu Arg Ala Val Asp Tyr Ala Lys
    530                 535

<210> 67
<211> 104
<212> PRT
<213> Streptococcus pneumoniae

<400> 67
Met Met Leu Lys Pro Ser Ile Asp Thr Leu Leu Asp Lys Val Pro Ser
1               5                   10                  15

Lys Tyr Ser Leu Val Ile Leu Glu Ala Lys Arg Ala His Glu Leu Glu
            20                  25                  30

Ala Gly Ala Pro Ala Thr Gln Gly Phe Lys Ser Glu Lys Ser Thr Leu
        35                  40                  45

Arg Ala Leu Glu Glu Ile Glu Ser Gly Asn Val Thr Ile His Pro Asp
    50                  55                  60

Pro Glu Gly Lys Arg Glu Ala Val Arg Arg Arg Ile Glu Glu Glu Lys
65                  70                  75                  80

Arg Arg Lys Glu Glu Glu Glu Lys Lys Ile Lys Glu Gln Ile Ala Lys
                85                  90                  95

Glu Lys Glu Asp Gly Glu Lys Ile
            100

<210> 68
<211> 263
<212> PRT

<213> Streptococcus pneumoniae

<400> 68
Met Ser Ala Tyr Gln Leu Pro Thr Val Trp Gln Asp Glu Ala Ser Asn
1               5                   10                  15

Gln Gly Ala Phe Thr Gly Leu Asn Arg Pro Thr Ala Gly Ala Arg Phe
            20                  25                  30

Glu Gln Asn Leu Pro Lys Gly Glu Gln Ala Phe Gln Leu Tyr Ser Leu
        35                  40                  45

Gly Thr Pro Asn Gly Val Lys Val Thr Ile Leu Leu Glu Glu Leu Leu
    50                  55                  60

Glu Ala Gly Phe Lys Glu Ala Ala Tyr Asp Leu Tyr Lys Ile Ala Ile
65                  70                  75                  80

Met Asp Gly Asp Gln Phe Gly Ser Asp Phe Val Lys Leu Asn Pro Asn
                85                  90                  95

Ser Lys Ile Pro Ala Leu Leu Asp Gln Ser Gly Thr Glu Asn Val Arg
            100                 105                 110

Val Phe Glu Ser Ala His Ile Leu Leu Tyr Leu Ala Glu Lys Phe Gly
        115                 120                 125

Ala Phe Leu Pro Ser Asn Pro Val Glu Lys Val Glu Val Leu Asn Trp
    130                 135                 140

Leu Phe Trp Gln Ala Gly Ala Ala Pro Phe Leu Gly Gly Gly Phe Gly
145                 150                 155                 160

His Phe Phe Asn Tyr Ala Pro Glu Lys Leu Glu Tyr Pro Ile Asn Arg
                165                 170                 175

Phe Thr Met Glu Val Lys Arg Gln Leu Asp Leu Leu Asp Lys Glu Leu
            180                 185                 190

Ala Gln Lys Pro Tyr Ile Ala Gly Asn Asp Tyr Thr Ile Ala Asp Ile
        195                 200                 205

Ala Ile Trp Ser Trp Tyr Gly Gln Leu Val Gln Gly Asn Leu Tyr Gln
    210                 215                 220

Gly Ser Ala Lys Phe Leu Asp Ala Ser Ser Tyr Gln Asn Leu Val Lys
225                 230                 235                 240

Trp Ala Glu Lys Ile Ala Asn Arg Pro Ala Val Lys Arg Gly Leu Glu
                245                 250                 255

Val Thr Tyr Thr Glu Ile Lys
            260


<210> 69
<211> 312
<212> PRT
<213> Streptococcus pneumoniae

<400> 69
Leu Ala Ser Leu Ile Thr Ser Ile Ile Met Phe Tyr Val Gly Phe Asp
1               5                   10                  15

Val Leu Arg Asp Thr Ile Gln Lys Ile Leu Ser Arg Glu Glu Thr Val
            20                  25                  30

Ile Asp Pro Leu Gly Ala Thr Leu Gly Ile Ile Ser Ala Ala Ile Met
        35                  40                  45

Phe Val Val Tyr Leu Tyr Asn Thr Arg Leu Ser Lys Lys Ser Asn Ser
    50                  55                  60

Asn Ala Leu Lys Ala Ala Ala Lys Asp Asn Leu Ser Asp Ala Val Thr
65                  70                  75                  80

Ser Leu Gly Thr Ala Ile Ala Ile Leu Ala Ser Ser Phe Asn Tyr Pro
                85                  90                  95

Ile Val Asp Lys Leu Val Ala Ile Ile Ile Thr Phe Phe Ile Leu Lys
            100                 105                 110

Thr Ala Tyr Asp Ile Phe Ile Glu Ser Ser Phe Ser Leu Ser Asp Gly
        115                 120                 125

Phe Asp Asp Arg Leu Leu Glu Asp Tyr Gln Lys Ala Ile Met Glu Ile
    130                 135                 140

Pro Lys Ile Ser Lys Val Lys Ser Gln Arg Gly Arg Thr Tyr Gly Ser
145                 150                 155                 160

Asn Ile Tyr Leu Asp Ile Thr Leu Glu Met Asn Pro Asp Leu Ser Val
                165                 170                 175

Phe Glu Ser His Glu Ile Ala Asp Gln Val Glu Ser Met Leu Glu Glu
            180                 185                 190

Arg Phe Gly Val Phe Asp Thr Asp Val His Ile Glu Pro Ala Pro Ile
        195                 200                 205

Pro Glu Asp Glu Ile Leu Asp Asn Val Tyr Lys Lys Leu Leu Met Arg
    210                 215                 220

Glu Gln Leu Ile Asp Gln Gly Asn Gln Leu Glu Glu Leu Leu Thr Asp
225                 230                 235                 240

Asp Phe Val Tyr Ile Arg Gln Asp Gly Glu Gln Met Asp Lys Glu Ala
                245                 250                 255

Tyr Lys Thr Lys Lys Glu Leu Asn Ser Ala Ile Lys Asp Ile Gln Ile
            260                 265                 270

Thr Ser Ile Ser Gln Lys Thr Lys Leu Ile Cys Tyr Glu Leu Asp Gly
        275                 280                 285

Ile Ile His Thr Ser Ile Trp Arg Arg His Glu Thr Trp Gln Asn Ile
    290                 295                 300

Phe His Gln Glu Thr Lys Lys Glu
305                 310

<210> 70
<211> 264
<212> PRT
<213> Streptococcus pneumoniae

<400> 70
Met Thr Ile Lys Leu Val Ala Thr Asp Met Asp Gly Thr Phe Leu Asp
1               5                   10                  15

Gly Asn Gly Arg Phe Asp Met Asp Arg Leu Lys Ser Leu Leu Val Ser
            20                  25                  30

Tyr Lys Glu Lys Gly Ile Tyr Phe Ala Val Ala Ser Gly Arg Gly Phe
        35                  40                  45

Leu Ser Leu Glu Lys Leu Phe Ala Gly Val Arg Asp Asp Ile Ile Phe
    50                  55                  60

Ile Ala Glu Asn Gly Ser Leu Val Glu Tyr Gln Gly Gln Asp Leu Tyr
65                  70                  75                  80

Glu Ala Thr Met Ser Arg Asp Phe Tyr Leu Ala Thr Phe Glu Lys Leu
                85                  90                  95

Lys Thr Ser Pro Tyr Val Asp Ile Asn Lys Leu Leu Leu Thr Gly Lys
            100                 105                 110

Lys Gly Ser Tyr Val Leu Asp Thr Val Asp Glu Thr Tyr Leu Lys Val
        115                 120                 125

Ser Gln His Tyr Asn Glu Asn Ile Gln Lys Val Ala Ser Leu Glu Asp
    130                 135                 140

Ile Thr Asp Asp Ile Phe Lys Phe Thr Thr Asn Phe Thr Glu Glu Thr
145                 150                 155                 160

Leu Glu Asp Gly Glu Ala Trp Val Asn Glu Asn Val Pro Gly Val Lys
                165                 170                 175

Ala Met Thr Thr Gly Phe Glu Ser Ile Asp Ile Val Leu Asp Tyr Val
            180                 185                 190

Asp Lys Gly Val Ala Ile Val Glu Leu Val Lys Lys Leu Gly Ile Thr
        195                 200                 205

Met Asp Gln Val Met Ala Phe Gly Asp Asn Leu Asn Asp Leu His Met
    210                 215                 220

Met Gln Val Val Gly His Pro Val Ala Pro Glu Asn Ala Arg Pro Glu
225                 230                 235                 240

Ile Leu Glu Leu Ala Lys Thr Val Ile Gly His His Lys Glu Arg Ser
                245                 250                 255

Val Ile Ala Tyr Met Glu Gly Leu
            260

<210> 71
<211> 272
<212> PRT
<213> Streptococcus pneumoniae

<400> 71
Met Ala Asp Ile Lys Leu Ile Ala Leu Asp Leu Asp Gly Thr Leu Leu
1               5                   10                  15

Thr Thr Asp Lys Arg Leu Thr Asp Arg Thr Lys Glu Thr Leu Gln Ala
            20                  25                  30

Ala Arg Asp Arg Gly Ile Lys Val Val Leu Thr Thr Gly Arg Pro Leu
        35                  40                  45

Lys Ala Met Asp Phe Phe Leu His Glu Leu Gly Thr Asp Gly Gln Glu
    50                  55                  60

Asp Glu Tyr Thr Ile Thr Phe Asn Gly Gly Leu Val Gln Lys Asn Thr
65                  70                  75                  80

Gly Glu Ile Leu Asp Lys Thr Val Phe Ser Tyr Asp Asp Val Ala Arg
                85                  90                  95

Leu Tyr Glu Glu Thr Glu Lys Leu Ser Leu Pro Leu Asp Ala Ile Ser
            100                 105                 110

Glu Gly Thr Val Tyr Gln Ile Gln Ser Asp Gln Glu Ser Leu Tyr Ala
        115                 120                 125

Lys Phe Asn Pro Ala Leu Thr Phe Val Pro Val Asp Phe Glu Asp Leu
    130                 135                 140

Ser Ser Gln Met Thr Tyr Asn Lys Cys Val Thr Ala Phe Ala Gln Glu
145                 150                 155                 160

Pro Leu Asp Ala Ala Ile Gln Lys Ile Ser Pro Glu Leu Phe Asp Gln
                165                 170                 175

Tyr Glu Ile Phe Lys Ser Arg Glu Met Leu Leu Glu Trp Ser Pro Lys
            180                 185                 190

Asn Val His Lys Ala Thr Gly Leu Ala Lys Leu Ile Ser His Leu Gly
        195                 200                 205

Ile Asp Gln Ser Gln Val Met Ala Cys Gly Asp Glu Ala Asn Asp Leu
    210                 215                 220

Ser Met Ile Glu Trp Ala Gly Leu Gly Val Ala Met Gln Asn Ala Val
225                 230                 235                 240

Pro Glu Val Lys Ala Ala Ala Asn Val Val Thr Pro Met Thr Asn Asp
                245                 250                 255

Glu Glu Ala Val Ala Trp Ala Ile Glu Glu Tyr Val Leu Lys Glu Asn
            260                 265                 270

<210> 72
<211> 418
<212> PRT
<213> Streptococcus pneumoniae

<400> 72
Met Glu Ser Leu Leu Ile Leu Leu Leu Ile Ala Asn Leu Ala Gly Leu
1               5                   10                  15

Phe Leu Ile Trp Gln Arg Gln Asp Arg Gln Glu Lys His Leu Ser Lys
            20                  25                  30

Ser Leu Glu Asp Gln Ala Asp His Leu Ser Asp Gln Leu Asp Tyr Arg
        35                  40                  45

Phe Asp Gln Ala Arg Gln Ala Ser Gln Leu Asp Gln Lys Asp Leu Glu
    50                  55                  60

Val Val Val Ser Asp Arg Leu Gln Glu Val Arg Ile Glu Leu His Gln
65                  70                  75                  80

Gly Leu Thr Gln Val Arg Gln Glu Met Thr Asp Asn Leu Leu Gln Thr
                85                  90                  95

Arg Asp Lys Thr Asp Gln Arg Leu Gln Ala Leu Gln Glu Ser Asn Glu
            100                 105                 110

Gln Arg Leu Glu Gln Met Arg Gln Thr Val Glu Glu Lys Leu Glu Lys
        115                 120                 125

Thr Leu Gln Thr Arg Leu Gln Ala Ser Phe Glu Thr Val Ser Lys Gln
    130                 135                 140

Leu Glu Ser Val Asn Arg Gly Leu Gly Glu Met Gln Thr Val Ala Arg
145                 150                 155                 160

Asp Val Gly Ala Leu Asn Lys Val Leu Ser Gly Thr Lys Thr Arg Gly
                165                 170                 175

Ile Leu Gly Glu Leu Gln Leu Gly Gln Ile Ile Glu Asp Ile Met Thr
            180                 185                 190

Pro Ala Gln Tyr Glu Arg Glu Tyr Ala Thr Val Glu Asn Ser Ser Glu
        195                 200                 205

Arg Val Glu Tyr Ala Ile Lys Leu Pro Gly Gln Gly Asp Gln Glu Tyr
    210                 215                 220

Val Tyr Leu Pro Ile Asp Ser Lys Phe Pro Leu Ala Asp Tyr Tyr Arg
225                 230                 235                 240

Leu Glu Glu Ala Tyr Glu Thr Gly Asp Lys Asp Glu Ile Glu Arg Cys
                245                 250                 255

Arg Lys Ser Leu Leu Ala Ser Val Lys Arg Phe Ala Arg Asp Ile Arg
            260                 265                 270

Asn Lys Tyr Ile Ala Pro Pro Arg Thr Thr Asn Phe Gly Val Leu Phe
        275                 280                 285

Val Pro Thr Glu Gly Leu Tyr Ser Glu Ile Val Arg Asn Pro Val Phe
    290                 295                 300

Phe Asp Asp Leu Arg Arg Glu Glu Gln Ile Ile Val Ala Gly Pro Ser
305                 310                 315                 320

Thr Leu Ser Ala Leu Leu Asn Ser Leu Ser Val Gly Phe Lys Thr Leu
                325                 330                 335

Asn Ile Gln Lys Ser Ala Asp His Ile Ser Lys Thr Leu Ala Ser Val
            340                 345                 350

Lys Thr Glu Phe Gly Lys Phe Gly Gly Ile Leu Val Lys Ala Gln Lys
        355                 360                 365

His Leu Gln His Ala Ser Gly Asn Ile Asp Glu Leu Leu Asn Arg Arg
    370                 375                 380

Thr Ile Ala Ile Glu Arg Thr Leu Arg His Ile Glu Leu Ser Glu Gly
385                 390                 395                 400

Glu Pro Ala Leu Asp Leu Leu His Phe Gln Glu Asn Glu Glu Glu Tyr
                405                 410                 415

Glu Asp

<210> 73
<211> 313
<212> PRT
<213> Streptococcus pneumoniae

<400> 73
Met Lys Ile Ser His Met Lys Lys Asp Glu Leu Phe Glu Gly Phe Tyr
1               5                   10                  15

Leu Ile Lys Ser Ala Asp Leu Arg Gln Thr Arg Ala Gly Lys Asn Tyr
            20                  25                  30

Leu Ala Phe Thr Phe Gln Asp Asp Ser Gly Glu Ile Asp Gly Lys Leu
        35                  40                  45

Trp Asp Ala Gln Pro His Asn Ile Glu Ala Phe Thr Ala Gly Lys Val
    50                  55                  60

Val His Met Lys Gly Arg Arg Glu Val Tyr Asn Asn Thr Pro Gln Val
65                  70                  75                  80

Asn Gln Ile Thr Leu Arg Leu Pro Gln Ala Gly Glu Pro Asn Asp Pro
                85                  90                  95

Ala Asp Phe Lys Val Lys Ser Pro Val Asp Val Lys Glu Ile Arg Asp
            100                 105                 110

Tyr Met Ser Gln Met Ile Phe Lys Ile Glu Asn Pro Val Trp Gln Arg
        115                 120                 125

Ile Val Arg Asn Leu Tyr Thr Lys Tyr Asp Lys Glu Phe Tyr Ser Tyr
    130                 135                 140

Pro Ala Ala Lys Thr Asn His His Ala Phe Glu Thr Gly Leu Ala Tyr
145                 150                 155                 160

His Thr Ala Thr Met Val Arg Leu Ala Asp Ala Ile Ser Glu Val Tyr
                165                 170                 175

Pro Gln Leu Asn Lys Ser Leu Leu Tyr Ala Gly Ile Met Leu His Asp
            180                 185                 190

Leu Ala Lys Val Ile Glu Leu Thr Gly Pro Asp Gln Thr Glu Tyr Thr
        195                 200                 205

Val Arg Gly Asn Leu Leu Gly His Ile Ala Leu Ile Asp Ser Glu Ile
    210                 215                 220

Thr Lys Thr Val Met Glu Leu Gly Ile Asp Asp Thr Lys Glu Glu Val
225                 230                 235                 240

Val Leu Leu Arg His Val Ile Leu Ser His His Gly Leu Leu Glu Tyr
                245                 250                 255

Gly Ser Pro Val Arg Pro Arg Ile Met Glu Ala Glu Ile Ile His Met
            260                 265                 270

Ile Asp Asn Leu Asp Ala Ser Met Met Met Met Ser Thr Ala Leu Ala
        275                 280                 285

Leu Val Asp Lys Gly Glu Met Thr Asn Lys Ile Phe Ala Met Asp Asn
    290                 295                 300

Arg Ser Phe Tyr Lys Pro Asp Leu Asp
305                 310

<210> 74
<211> 503
<212> PRT
<213> Streptococcus pneumoniae

<400> 74
Met Ser Glu Lys Ala Lys Lys Gly Phe Lys Met Pro Ser Ser Tyr Thr
1               5                   10                  15

Val Leu Leu Ile Ile Ile Ala Ile Met Ala Val Leu Thr Trp Phe Ile
            20                  25                  30

Pro Ala Gly Ala Phe Ile Glu Gly Ile Tyr Glu Thr Gln Pro Gln Asn
        35                  40                  45

Pro Gln Gly Ile Trp Asp Val Leu Met Ala Pro Ile Arg Ala Met Leu
    50                  55                  60

Gly Thr His Pro Glu Glu Gly Ser Leu Ile Lys Glu Thr Ser Ala Ala
65                  70                  75                  80

Ile Asp Val Ala Phe Phe Ile Leu Met Val Gly Gly Phe Leu Gly Ile
                85                  90                  95

Val Asn Lys Thr Gly Ala Leu Asp Val Gly Ile Ala Ser Ile Val Lys

            100                 105                 110

Lys Tyr Lys Gly Arg Glu Lys Met Leu Ile Leu Val Leu Met Pro Leu
        115                 120                 125

Phe Ala Leu Gly Gly Thr Thr Tyr Gly Met Gly Glu Glu Thr Met Ala
    130                 135                 140

Phe Tyr Pro Leu Leu Val Pro Val Met Met Ala Val Gly Phe Asp Ser
145                 150                 155                 160

Leu Thr Gly Val Ala Ile Ile Leu Leu Gly Ser Gln Ile Gly Cys Leu
                165                 170                 175

Ala Ser Thr Leu Asn Pro Phe Ala Thr Gly Ile Ala Ser Ala Thr Ala
            180                 185                 190

Gly Val Gly Thr Gly Asp Gly Ile Val Leu Arg Leu Ile Phe Trp Val
        195                 200                 205

Thr Leu Thr Ala Leu Ser Thr Trp Phe Val Tyr Arg Tyr Ala Asp Lys
    210                 215                 220

Ile Gln Lys Asp Pro Thr Lys Ser Leu Val Tyr Ser Thr Arg Lys Glu
225                 230                 235                 240

Asp Leu Lys His Phe Asn Val Glu Glu Ser Ser Ser Val Glu Ser Thr
                245                 250                 255

Leu Ser Ser Lys Gln Lys Ser Val Leu Phe Leu Phe Val Leu Thr Phe
            260                 265                 270

Ile Leu Met Val Leu Ser Phe Ile Pro Trp Thr Asp Leu Gly Val Thr
        275                 280                 285

Ile Phe Asp Asp Phe Asn Thr Trp Leu Thr Gly Leu Pro Val Ile Gly
    290                 295                 300

Asn Ile Val Gly Ser Ser Thr Ser Ala Leu Gly Thr Trp Tyr Phe Pro
305                 310                 315                 320

Glu Gly Ala Met Leu Phe Ala Phe Met Gly Ile Leu Ile Gly Val Ile
                325                 330                 335

Tyr Gly Leu Lys Glu Asp Lys Ile Ile Ser Ser Phe Met Asn Gly Ala
            340                 345                 350

Ala Asp Leu Leu Ser Val Ala Leu Ile Val Ala Ile Ala Arg Gly Ile
        355                 360                 365

Gln Val Ile Met Asn Asp Gly Met Ile Thr Asp Thr Ile Leu Asn Trp
    370                 375                 380

Gly Lys Glu Gly Leu Ser Gly Leu Ser Ser Gln Val Phe Ile Val Val
385                 390                 395                 400

Thr Tyr Ile Phe Tyr Leu Pro Met Ser Phe Leu Ile Pro Ser Ser Ser
                405                 410                 415

Gly Leu Ala Ser Ala Thr Met Gly Ile Met Ala Pro Leu Gly Glu Phe
            420                 425                 430

Val Asn Val Arg Pro Ser Leu Ile Ile Thr Ala Tyr Gln Ser Ala Ser
        435                 440                 445

Gly Val Leu Asn Leu Ile Ala Pro Thr Ser Gly Ile Val Met Gly Ala
    450                 455                 460

Leu Ala Leu Gly Arg Ile Asn Ile Gly Thr Trp Trp Lys Phe Met Gly
465                 470                 475                 480

Lys Leu Val Val Ala Ile Ile Val Val Thr Ile Ala Leu Leu Leu Leu
                485                 490                 495

Gly Thr Phe Leu Pro Phe Leu
            500

<210> 75
<211> 782
<212> PRT
<213> Streptococcus pneumoniae

<400> 75
Met Ser Asn Ser Phe Val Lys Leu Leu Val Ser Gln Leu Phe Ala Asn
  1               5                  10                  15

Leu Ala Asp Ile Phe Phe Arg Val Thr Ile Ile Ala Asn Ile Tyr Ile
             20                  25                  30

Ile Ser Lys Ser Val Ile Ala Thr Ser Leu Val Pro Ile Leu Ile Gly
         35                  40                  45

Ile Ser Ser Phe Val Ala Ser Leu Leu Val Pro Leu Val Thr Lys Arg
     50                  55                  60

Leu Ala Leu Asn Arg Val Leu Ser Leu Ser Gln Phe Gly Lys Thr Ile
 65                  70                  75                  80

Leu Leu Ala Ile Leu Val Gly Met Phe Thr Val Met Gln Ser Val Ala
                 85                  90                  95

Pro Leu Val Thr Tyr Leu Phe Val Val Ala Ile Ser Ile Leu Asp Gly
            100                 105                 110

Phe Ala Ala Pro Val Ser Tyr Ala Ile Val Pro Arg Tyr Ala Thr Asp
        115                 120                 125

Leu Gly Lys Ala Asn Ser Ala Leu Ser Met Thr Gly Glu Ala Val Gln
    130                 135                 140

Leu Ile Gly Trp Gly Leu Gly Gly Leu Leu Phe Ala Thr Ile Gly Leu
145                 150                 155                 160

Leu Pro Thr Thr Cys Ile Asn Leu Val Leu Tyr Ile Ile Ser Ser Phe
                165                 170                 175

Leu Met Leu Phe Leu Pro Asn Ala Glu Val Glu Val Leu Glu Ser Glu
            180                 185                 190

Thr Asn Leu Glu Ile Leu Leu Lys Gly Trp Lys Leu Val Ala Arg Asn

195                 200                 205

Pro Arg Leu Arg Leu Phe Val Ser Ala Asn Leu Leu Glu Ile Phe Ser
    210                 215                 220

Asn Thr Ile Trp Val Ser Ser Ile Ile Leu Val Phe Val Thr Glu Leu
225                 230                 235                 240

Leu Asn Lys Thr Glu Ser Tyr Trp Gly Tyr Ser Asn Thr Ala Tyr Ser
                245                 250                 255

Ile Gly Ile Ile Ile Ser Gly Leu Ile Ala Phe Arg Leu Ser Glu Lys
            260                 265                 270

Phe Leu Ala Ala Lys Trp Glu Pro Gln Leu Phe Thr Pro Asn Leu Lys
        275                 280                 285

Thr Ile Gln Asn Pro Cys Leu Ser Leu Asp Pro Gly Trp Phe Leu Phe
    290                 295                 300

Ser Pro Asn Gly Cys Phe Leu Leu Asp Lys Lys Glu Phe Pro Leu Tyr
305                 310                 315                 320

Gly Ile Ser Val Glu Lys Asn Thr Lys Arg Lys Glu Thr His Met Asn
                325                 330                 335

Ser Leu Pro Asn His His Phe Gln Asn Lys Ser Phe Tyr Gln Leu Ser
            340                 345                 350

Phe Asp Gly Gly His Leu Thr Gln Tyr Gly Gly Leu Ile Phe Phe Gln
        355                 360                 365

Glu Leu Phe Ser Gln Leu Lys Leu Lys Glu Arg Ile Ser Lys Tyr Leu
    370                 375                 380

Val Thr Asn Asp Gln Arg Arg Tyr Cys Arg Tyr Ser Asp Ser Asp Ile
385                 390                 395                 400

Leu Val Gln Phe Leu Phe Gln Leu Leu Thr Gly Tyr Gly Thr Asp Tyr
                405                 410                 415

Ala Cys Lys Glu Leu Ser Ala Asp Ala Tyr Phe Pro Lys Leu Leu Glu
            420                 425                 430

Gly Gly Gln Leu Ala Ser Gln Pro Thr Leu Ser Arg Phe Leu Ser Arg
        435                 440                 445

Thr Asp Glu Glu Thr Val His Ser Leu Arg Cys Leu Asn Leu Glu Leu
    450                 455                 460

Val Glu Phe Phe Leu Gln Phe His Gln Leu Asn Gln Leu Ile Val Asp
465                 470                 475                 480

Ile Asp Ser Thr His Phe Thr Thr Tyr Gly Lys Gln Glu Gly Val Ala
                485                 490                 495

Tyr Asn Ala His Tyr Arg Ala His Gly Tyr His Pro Leu Tyr Ala Phe
            500                 505                 510

Glu Gly Lys Thr Gly Tyr Cys Phe Asn Ala Gln Leu Arg Pro Gly Asn
        515                 520                 525

Arg Tyr Cys Ser Glu Glu Ala Asp Ser Phe Ile Thr Pro Val Leu Glu
    530                 535                 540

Arg Phe Asn Gln Leu Leu Phe Arg Met Asp Ser Gly Phe Ala Thr Pro
545                 550                 555                 560

Lys Leu Tyr Asp Leu Ile Glu Lys Thr Gly Gln Tyr Tyr Leu Ile Lys
                565                 570                 575

Leu Lys Lys Asn Thr Val Leu Ser Arg Leu Gly Asp Leu Ser Leu Pro
            580                 585                 590

Cys Pro Gln Asp Glu Asp Leu Thr Ile Leu Pro His Ser Ala Tyr Ser
        595                 600                 605

Glu Thr Leu Tyr Gln Ala Gly Ser Trp Ser His Lys Arg Arg Val Cys
    610                 615                 620

Gln Phe Ser Glu Arg Lys Glu Gly Asn Leu Phe Tyr Asp Val Ile Ser
625                 630                 635                 640

Leu Val Thr Asn Met Thr Ser Gly Thr Ser Gln Asp Gln Phe Gln Leu
                645                 650                 655

Tyr Arg Gly Arg Gly Gln Ala Glu Asn Phe Ile Lys Glu Met Lys Glu
            660                 665                 670

Gly Phe Phe Gly Asp Lys Thr Asp Ser Ser Thr Leu Ile Lys Asn Glu
        675                 680                 685

Val Arg Met Met Met Ser Cys Ile Ala Tyr Asn Leu Tyr Leu Phe Leu
    690                 695                 700

Lys His Leu Ala Gly Gly Asp Phe Gln Thr Leu Thr Ile Lys Arg Phe
705                 710                 715                 720

Arg His Leu Phe Leu His Val Val Gly Lys Cys Val Arg Thr Gly Arg
                725                 730                 735

Lys Gln Leu Leu Lys Leu Ser Ser Leu Tyr Ala Tyr Ser Glu Leu Phe
            740                 745                 750

Ser Ala Leu Tyr Ser Arg Ile Arg Lys Val Asn Leu Asn Leu Pro Val
        755                 760                 765

Pro Tyr Glu Pro Pro Arg Arg Lys Ala Ser Leu Met Met His
    770                 775                 780

<210> 76
<211> 414
<212> PRT
<213> Streptococcus pneumoniae

<400> 76
Met Met Glu Phe Phe Gln Gln Leu Pro His Leu Glu Pro Tyr Gly Asn
  1               5                  10                  15

Pro Gln Tyr Phe Val Tyr Val Ile Ala Ala Thr Leu Pro Ile Phe Ile

            20                  25                  30

Gly Leu Phe Phe Lys Lys Arg Phe Ala Trp Tyr Glu Val Leu Val Ser
        35                  40                  45

Leu Phe Phe Ile Val Thr Met Leu Val Gly Gly Lys Thr Asn Gln Leu
    50                  55                  60

Ala Ala Leu Gly Ile Tyr Leu Cys Trp Glu Ile Leu Leu Leu Leu Phe
65                  70                  75                  80

Tyr Lys His Tyr Arg Lys Ser Lys Asp Gly Lys Trp Val Phe Tyr Leu
                85                  90                  95

Val Ser Phe Leu Ser Leu Leu Pro Ile Ile Phe Val Lys Val Gln Pro
            100                 105                 110

Ala Ile Asn Gly Thr Gln Ser Leu Leu Gly Phe Leu Gly Ile Ser Tyr
        115                 120                 125

Leu Thr Phe Arg Ser Val Gly Ile Val Ile Glu Leu Arg Asp Gly Val
    130                 135                 140

Ile Lys Asp Phe Thr Leu Trp Glu Phe Leu Arg Phe Leu Leu Phe Met
145                 150                 155                 160

Pro Thr Phe Ser Ser Gly Pro Ile Asp Arg Phe Lys Arg Phe Asn Glu
                165                 170                 175

Asn Tyr Gln Ala Ile Pro Glu Arg Asp Glu Leu Met Asp Met Leu Asp
            180                 185                 190

Glu Ser Val Arg Tyr Ile Met Trp Gly Phe Leu Tyr Lys Phe Ile Leu
        195                 200                 205

Ala His Val Leu Gly Glu Thr Leu Leu Pro Pro Leu Lys Asn Leu Ala
    210                 215                 220

Leu Gln Ser Gly Gly Phe Phe Asn Leu Tyr Ala Leu Ala Val Met Tyr
225                 230                 235                 240

Thr Phe Gly Leu Glu Leu Phe Phe Asp Phe Ala Gly Tyr Ser Met Phe
                245                 250                 255

Ala Leu Ala Ile Ser Asn Leu Met Gly Ile Arg Ser Pro Ile Asn Phe
            260                 265                 270

Asn Lys Pro Phe Leu Ser Arg Asp Leu Lys Glu Phe Trp Asn Arg Trp
        275                 280                 285

His Met Ser Leu Ser Phe Trp Phe Arg Asp Phe Val Phe Met Arg Met
    290                 295                 300

Val Met Val Leu Thr Arg Lys Lys Val Phe Lys Asn Arg Asn Val Thr
305                 310                 315                 320

Ser Ser Met Ala Tyr Ile Val Asn Met Leu Ile Met Gly Phe Trp His
                325                 330                 335

Gly Val Thr Trp Tyr Tyr Ile Ala Tyr Gly Leu Phe His Gly Leu Gly
            340                 345                 350

Leu Val Ile Asn Asp Ala Trp Val Arg Lys Lys Lys Thr Leu Asn Lys
        355                 360                 365

Glu Arg Lys Lys Ala Gly Lys Ala Ala Leu Pro Glu Asn Arg Trp Ile
    370                 375                 380

Gln Leu Leu Gly Met Val Val Thr Phe His Val Val Met Leu Ser Phe
385                 390                 395                 400

Leu Ile Phe Ser Gly Phe Leu Asn Asn Leu Trp Phe Lys Lys
                405                 410

<210> 77
<211> 422
<212> PRT
<213> Streptococcus pneumoniae

<400> 77
Met Leu Lys Arg Leu Trp Met Ile Phe Gly Pro Val Leu Ile Ala Gly
1               5                   10                  15

Leu Leu Val Phe Leu Leu Ile Phe Phe Tyr Pro Thr Glu Met His His
            20                  25                  30

Asn Leu Gly Ala Glu Lys Arg Ser Ala Val Ala Thr Thr Ile Asp Ser
        35                  40                  45

Phe Lys Glu Arg Ser Gln Lys Val Arg Ala Leu Ser Asp Pro Asn Val
    50                  55                  60

Arg Phe Val Pro Phe Phe Gly Ser Ser Glu Trp Leu Arg Phe Asp Gly
65                  70                  75                  80

Ala His Pro Ala Val Leu Ala Glu Lys Tyr Asn Arg Ser Tyr Arg Pro
                85                  90                  95

Tyr Leu Leu Gly Gln Gly Gly Ala Ala Ser Leu Asn Gln Tyr Phe Gly
            100                 105                 110

Met Gln Gln Met Leu Pro Gln Leu Glu Asn Lys Gln Val Val Tyr Val
        115                 120                 125

Ile Ser Pro Gln Trp Phe Ser Lys Asn Gly Tyr Asp Pro Ala Ala Phe
    130                 135                 140

Gln Gln Tyr Phe Asn Gly Asp Gln Leu Thr Ser Phe Leu Lys His Gln
145                 150                 155                 160

Ser Gly Asp Gln Ala Ser Gln Tyr Ala Ala Thr Arg Leu Leu Gln Gln
                165                 170                 175

Phe Pro Asn Val Ala Met Lys Asp Leu Val Gln Lys Leu Ala Ser Lys
            180                 185                 190

Glu Glu Leu Ser Thr Ala Asp Asn Glu Met Ile Glu Leu Leu Ala Arg
        195                 200                 205

Phe Asn Glu Arg Gln Ala Ser Phe Phe Gly Gln Phe Ser Val Arg Gly

210                     215                     220

Tyr Val Asn Tyr Asp Lys His Val Ala Lys Tyr Leu Lys Ile Leu Pro
225                 230                 235                 240

Asp Gln Phe Ser Tyr Gln Ala Ile Glu Asp Val Val Lys Ala Asp Ala
                245                 250                 255

Glu Lys Asn Thr Ser Asn Asn Glu Met Gly Met Glu Asn Tyr Phe Tyr
            260                 265                 270

Asn Glu Gln Ile Lys Lys Asp Leu Lys Lys Leu Lys Asp Ser Gln Lys
        275                 280                 285

Ser Phe Thr Tyr Leu Lys Ser Pro Glu Tyr Asn Asp Leu Gln Leu Val
    290                 295                 300

Leu Thr Gln Phe Ser Lys Ser Lys Val Asn Pro Ile Phe Ile Ile Pro
305                 310                 315                 320

Pro Val Asn Lys Lys Trp Met Asn Tyr Ala Gly Leu Arg Glu Asp Met
                325                 330                 335

Tyr Gln Gln Thr Val Gln Lys Ile Arg Tyr Gln Leu Glu Ser Gln Gly
            340                 345                 350

Phe Thr Asn Ile Ala Asp Phe Ser Lys Asp Gly Gly Glu Pro Phe Phe
        355                 360                 365

Met Lys Asp Thr Ile His Leu Gly Trp Leu Gly Trp Leu Ala Phe Asp
    370                 375                 380

Lys Ala Val Asp Pro Phe Leu Ser Asn Pro Thr Pro Ala Pro Thr Tyr
385                 390                 395                 400

His Leu Asn Glu Arg Phe Phe Ser Lys Asp Trp Ala Thr Tyr Asp Gly
                405                 410                 415

Asp Val Lys Glu Phe Gln
            420

<210> 78
<211> 206
<212> PRT
<213> Streptococcus pneumoniae

<400> 78
Met Glu Lys Asn Leu Lys Ala Leu Lys Gln Thr Thr Asp Gln Glu Gly
1               5                   10                  15

Pro Ala Ile Glu Pro Glu Lys Ala Glu Asp Thr Lys Thr Val Gln Asn
            20                  25                  30

Gly Tyr Phe Glu Asp Ala Ala Val Lys Asp Arg Thr Leu Ser Asp Tyr
        35                  40                  45

Ala Gly Asn Trp Gln Ser Val Tyr Pro Phe Leu Glu Asp Gly Thr Phe
    50                  55                  60

Asp Gln Val Phe Asp Tyr Lys Ala Lys Leu Thr Gly Lys Met Thr Gln
65                  70                  75                  80

Ala Glu Tyr Lys Ala Tyr Tyr Thr Lys Gly Tyr His Thr Asp Val Thr
                85                  90                  95

Lys Ile Asn Ile Thr Asp Asn Thr Met Glu Phe Val Gln Gly Gly Gln
            100                 105                 110

Ser Lys Lys Tyr Thr Tyr Lys Tyr Val Gly Lys Lys Ile Leu Thr Tyr
        115                 120                 125

Lys Lys Gly Asn Arg Gly Val Arg Phe Leu Phe Glu Ala Thr Asp Ala
    130                 135                 140

Asp Ala Gly Gln Phe Lys Tyr Val Gln Phe Ser Asp His Asn Val Ala
145                 150                 155                 160

Pro Val Lys Ala Glu His Phe His Ile Phe Phe Gly Gly Thr Ser Gln
                165                 170                 175

Glu Ala Leu Phe Glu Glu Met Asp Asn Trp Pro Thr Tyr Tyr Pro Asp
            180                 185                 190

Asn Leu Ser Gly Gln Glu Ile Ala Gln Glu Met Leu Ala His
        195                 200                 205

<210> 79
<211> 147
<212> PRT
<213> Streptococcus pneumoniae

<400> 79
Met Lys Asp Gly His Leu Leu Ala His His Ile Arg Leu Leu Asn Gly
 1               5                  10                  15

Arg Ile Phe Gln Lys Leu Leu Ser Gln Asp Pro Glu Ala Leu Tyr Arg
            20                  25                  30

Gly Glu Gln Gly Lys Ile Leu Ala Val Leu Trp Asn Ser Glu Thr Gly
        35                  40                  45

Cys Ala Thr Ala Thr Asp Ile Ala Leu Ala Thr Gly Leu Ala Asn Asn
    50                  55                  60

Thr Leu Thr Thr Met Ile Lys Lys Leu Glu Glu Gln Lys Leu Val Ile
65                  70                  75                  80

Val Ser Pro Cys Gly Lys Asp Lys Arg Lys Lys Tyr Leu Val Leu Thr
                85                  90                  95

Glu Leu Gly Lys Ser Gln Lys Glu Val Gly His Arg Val Ser Gln Lys
            100                 105                 110

Leu Asp Thr Ile Phe Tyr Lys Gly Phe Ser Glu Glu Glu Ile His Gln
        115                 120                 125

Phe Glu Gly Phe Gln Glu Arg Ile Leu Ala Asn Leu Lys Glu Lys Gly
    130                 135                 140

Asn Glu Val
145

<210> 80
<211> 506
<212> PRT
<213> Streptococcus pneumoniae

<400> 80
Met Thr Asn Leu Ile Ala Thr Phe Gln Asp Arg Phe Ser Asp Trp Leu
1               5                   10                  15

Thr Ala Leu Ser Gln His Leu Gln Leu Ser Leu Leu Thr Leu Leu Leu
            20                  25                  30

Ala Ile Leu Leu Ala Ile Pro Leu Ala Val Phe Leu Arg Tyr His Glu
        35                  40                  45

Lys Leu Ala Asp Trp Val Leu Gln Ile Ala Gly Ile Phe Gln Thr Ile
    50                  55                  60

Pro Ser Leu Ala Leu Leu Gly Leu Phe Ile Pro Leu Met Gly Ile Gly
65                  70                  75                  80

Thr Leu Pro Ala Leu Thr Ala Leu Val Ile Tyr Ala Ile Phe Pro Ile
                85                  90                  95

Leu Gln Asn Thr Ile Thr Gly Leu Lys Gly Ile Asp Pro Asn Leu Gln
            100                 105                 110

Glu Ala Gly Ile Ala Phe Gly Met Thr Arg Trp Glu Arg Leu Lys Lys
        115                 120                 125

Phe Glu Ile Pro Leu Ala Met Pro Val Ile Met Ser Gly Ile Arg Thr
    130                 135                 140

Ala Ala Val Leu Ile Ile Gly Thr Ala Thr Leu Ala Ala Leu Ile Gly
145                 150                 155                 160

Ala Gly Gly Leu Gly Ser Phe Ile Leu Leu Gly Ile Asp Arg Asn Asn
                165                 170                 175

Ala Ser Leu Ile Leu Ile Gly Ala Leu Ser Ser Ala Val Leu Ala Ile
            180                 185                 190

Ala Phe Asn Phe Leu Leu Lys Val Met Glu Lys Ala Lys Leu Arg Thr
        195                 200                 205

Ile Phe Ser Gly Phe Ala Leu Val Ala Leu Leu Leu Gly Leu Ser Tyr
    210                 215                 220

Ser Pro Ala Leu Leu Val Gln Lys Glu Lys Glu Asn Leu Val Ile Ala
225                 230                 235                 240

Gly Lys Ile Gly Pro Glu Pro Glu Ile Leu Ala Asn Met Tyr Lys Leu
                245                 250                 255

Leu Ile Glu Glu Asn Thr Ser Met Thr Ala Thr Val Lys Pro Asn Phe

260                 265                 270

Gly Lys Thr Ser Phe Leu Tyr Glu Ala Leu Lys Lys Gly Asp Ile Asp
        275                 280                 285

Ile Tyr Pro Glu Phe Thr Gly Thr Val Thr Glu Ser Leu Leu Gln Pro
    290                 295                 300

Ser Pro Lys Val Ser His Glu Pro Glu Gln Val Tyr Gln Val Ala Arg
305                 310                 315                 320

Asp Gly Ile Ala Lys Gln Asp His Leu Ala Tyr Leu Lys Pro Met Ser
                325                 330                 335

Tyr Gln Asn Thr Tyr Ala Val Ala Val Pro Lys Lys Ile Ala Gln Glu
            340                 345                 350

Tyr Gly Leu Lys Thr Ile Ser Asp Leu Lys Lys Val Glu Gly Gln Leu
        355                 360                 365

Lys Ala Gly Phe Thr Leu Glu Phe Asn Asp Arg Glu Asp Gly Asn Lys
    370                 375                 380

Gly Leu Gln Ser Met Tyr Gly Leu Asn Leu Asn Val Ala Thr Ile Glu
385                 390                 395                 400

Pro Ala Leu Arg Tyr Gln Ala Ile Gln Ser Gly Asp Ile Gln Ile Thr
                405                 410                 415

Asp Ala Tyr Ser Thr Asp Ala Glu Leu Glu Arg Tyr Asp Leu Gln Val
            420                 425                 430

Leu Glu Asp Asp Lys Gln Leu Phe Pro Pro Tyr Gln Gly Ala Pro Leu
        435                 440                 445

Met Lys Glu Ala Leu Leu Lys Lys His Pro Glu Leu Glu Arg Val Leu
    450                 455                 460

Asn Thr Leu Ala Gly Lys Ile Thr Glu Ser Gln Met Ser Gln Leu Asn
465                 470                 475                 480

Tyr Gln Val Gly Val Glu Gly Lys Ser Ala Lys Gln Val Ala Lys Glu
                485                 490                 495

Phe Leu Gln Glu Gln Gly Leu Leu Lys Lys
            500                 505

<210> 81
<211> 269
<212> PRT
<213> Streptococcus pneumoniae

<400> 81
Met Met His Thr Tyr Leu Gln Lys Lys Ile Glu Asn Ile Lys Thr Thr
1               5                   10                  15

Leu Gly Glu Met Ser Gly Gly Tyr Arg Arg Met Val Ala Ala Met Ala
            20                  25                  30

Asp Leu Gly Phe Ser Gly Thr Met Lys Ala Ile Trp Asp Asp Leu Phe
        35                  40                  45

Ala His Arg Ser Phe Ala Gln Trp Ile Tyr Leu Leu Val Leu Gly Ser
    50                  55                  60

Phe Pro Leu Trp Leu Glu Leu Val Tyr Glu His Arg Ile Val Asp Trp
65                  70                  75                  80

Ile Gly Met Ile Cys Ser Leu Thr Gly Ile Ile Cys Val Ile Phe Val
                85                  90                  95

Ser Glu Gly Arg Ala Ser Asn Tyr Leu Phe Gly Leu Ile Asn Ser Val
            100                 105                 110

Ile Tyr Leu Ile Leu Ala Leu Gln Lys Gly Phe Tyr Gly Glu Val Leu
        115                 120                 125

Thr Thr Leu Tyr Phe Thr Val Met Gln Pro Ile Gly Leu Leu Val Trp
    130                 135                 140

Ile Tyr Gln Ala Gln Phe Lys Lys Glu Lys Gln Glu Phe Val Ala Arg
145                 150                 155                 160

Lys Leu Asp Gly Lys Gly Trp Thr Lys Tyr Leu Ser Ile Ser Val Leu
                165                 170                 175

Trp Trp Leu Ala Phe Gly Phe Ile Tyr Gln Ser Ile Gly Ala Asn Arg
            180                 185                 190

Pro Tyr Arg Asp Ser Ile Thr Asp Ala Thr Asn Gly Val Gly Gln Ile
        195                 200                 205

Leu Met Thr Ala Val Tyr Arg Glu Gln Trp Ile Phe Trp Ala Ala Thr
    210                 215                 220

Asn Val Phe Ser Ile Tyr Leu Trp Trp Gly Glu Ser Leu Gln Ile Gln
225                 230                 235                 240

Gly Lys Tyr Leu Ile Tyr Leu Ile Asn Ser Leu Val Gly Trp Tyr Gln
                245                 250                 255

Trp Ser Lys Ala Ala Lys Gln Asn Thr Asp Leu Leu Asn
            260                 265

<210> 82
<211> 299
<212> PRT
<213> Streptococcus pneumoniae

<400> 82
Met Arg Asn Met Lys Ala Lys Tyr Ala Val Trp Val Ala Phe Phe Leu
1               5                   10                  15

Asn Leu Thr Tyr Ala Ile Val Glu Phe Ile Ala Gly Gly Val Phe Gly
            20                  25                  30

Ser Ser Ala Val Leu Ala Asp Ser Val His Asp Leu Gly Asp Ala Ile
        35                  40                  45

Ala Ile Gly Ile Ser Ala Phe Leu Glu Thr Ile Ser Asn Arg Glu Glu
    50                  55                  60

Asp Asn Gln Tyr Thr Leu Gly Tyr Lys Arg Phe Ser Leu Leu Gly Ala
65                  70                  75                  80

Leu Val Thr Ala Val Ile Leu Val Thr Gly Ser Val Leu Val Ile Leu
                85                  90                  95

Glu Asn Val Thr Lys Ile Leu His Pro Gln Pro Val Asn Asp Glu Gly
            100                 105                 110

Ile Leu Trp Leu Gly Ile Ile Ala Ile Thr Ile Asn Leu Leu Ala Ser
        115                 120                 125

Leu Val Val Gly Lys Gly Lys Thr Lys Asn Glu Ser Ile Leu Ser Leu
    130                 135                 140

His Phe Leu Glu Asp Thr Leu Gly Trp Val Ala Val Ile Leu Met Ala
145                 150                 155                 160

Ile Val Leu Arg Phe Thr Asp Trp Tyr Ile Leu Asp Pro Leu Leu Ser
                165                 170                 175

Leu Val Ile Ser Phe Phe Ile Leu Ser Lys Ala Leu Pro Arg Phe Trp
            180                 185                 190

Ser Thr Leu Lys Ile Phe Leu Asp Ala Val Pro Glu Gly Leu Asp Ile
        195                 200                 205

Lys Gln Val Lys Ser Gly Leu Glu Arg Leu Asp Asn Val Ala Ser Leu
    210                 215                 220

Asn Gln Leu Asn Leu Trp Thr Met Asp Ala Leu Glu Lys Asn Ala Ile
225                 230                 235                 240

Val His Val Cys Leu Lys Glu Met Glu His Met Glu Thr Cys Lys Glu
                245                 250                 255

Ser Ile Arg Ile Phe Leu Lys Asp Cys Gly Phe Gln Asn Ile Thr Ile
            260                 265                 270

Glu Ile Asp Ala Asp Leu Glu Thr His Gln Thr His Lys Arg Lys Val
        275                 280                 285

Cys Asp Leu Glu Arg Ser Tyr Glu His Gln His
    290                 295

<210> 83
<211> 242
<212> PRT
<213> Streptococcus pneumoniae

<400> 83
Met Ile Glu Tyr Lys Asn Val Ala Leu Arg Tyr Thr Glu Lys Asp Val
1               5                   10                  15

Leu Arg Asp Val Asn Leu Gln Ile Glu Asp Gly Glu Phe Met Val Leu

20                  25                  30

Val Gly Pro Ser Gly Ser Gly Lys Thr Thr Met Leu Lys Met Ile Asn
        35                  40                  45

Arg Leu Leu Glu Pro Thr Asp Gly Asn Ile Tyr Met Asp Gly Lys Arg
    50                  55                  60

Ile Lys Asp Tyr Asp Glu Arg Glu Leu Arg Leu Ser Thr Gly Tyr Val
65                  70                  75                  80

Leu Gln Ala Ile Ala Leu Phe Pro Asn Leu Thr Val Ala Glu Asn Ile
                85                  90                  95

Ala Leu Ile Pro Glu Met Lys Gly Trp Ser Lys Glu Glu Ile Thr Lys
            100                 105                 110

Lys Thr Glu Glu Leu Leu Ala Lys Val Gly Leu Pro Val Ala Glu Tyr
        115                 120                 125

Gly His Arg Leu Pro Ser Glu Leu Ser Gly Gly Glu Gln Gln Arg Val
    130                 135                 140

Gly Ile Val Arg Ala Met Ile Gly Gln Pro Lys Ile Phe Leu Met Asp
145                 150                 155                 160

Glu Pro Phe Ser Ala Leu Asp Ala Ile Ser Arg Lys Gln Leu Gln Val
                165                 170                 175

Leu Thr Lys Glu Leu His Lys Glu Phe Gly Met Thr Thr Ile Phe Val
            180                 185                 190

Thr His Asp Thr Asp Glu Ala Leu Lys Leu Ala Asp Arg Ile Ala Val
        195                 200                 205

Leu Gln Asp Gly Glu Ile Arg Gln Val Ala Asn Pro Glu Thr Ile Leu
    210                 215                 220

Lys Ala Pro Ala Thr Asp Phe Val Ala Asp Leu Phe Gly Gly Ser Val
225                 230                 235                 240

His Asp

<210> 84
<211> 360
<212> PRT
<213> Streptococcus pneumoniae

<400> 84
Met Ser Ala Val Ala Ile Ser Ala Met Thr Lys Val Met Gln Glu Thr
1               5                   10                  15

His Gly Asn Pro Ser Ser Ile His Gly His Gly Arg Gln Ala Gly Lys
            20                  25                  30

Leu Leu Arg Glu Ala Arg Gln Glu Leu Ala Gln Leu Leu Arg Thr Lys
        35                  40                  45

Pro Gln His Ile Phe Phe Thr Ser Gly Gly Thr Glu Gly Asn Asn Thr
50                  55                  60

Thr Ile Ile Gly Tyr Cys Leu Arg His Gln Glu Gln Gly Lys His Ile
65                  70                  75                  80

Ile Thr Thr Ala Ile Glu His His Ala Val Leu Glu Thr Ile Asp Tyr
                85                  90                  95

Leu Val Gln His Phe Gly Phe Glu Ala Thr Ile Ile Gln Pro Glu Asn
            100                 105                 110

Gln Glu Ile Thr Ala Gln Gln Ile Gln Lys Ala Leu Arg Asp Asp Thr
        115                 120                 125

Ile Leu Val Ser Thr Met Phe Val Asn Asn Glu Thr Gly Asn Leu Leu
    130                 135                 140

Pro Ile Ala Glu Ile Gly Gln Ile Leu Lys Gln His Pro Ala Ala Tyr
145                 150                 155                 160

His Val Asp Ala Val Gln Ala Ile Gly Lys Ile Pro Ile His Ser Glu
                165                 170                 175

Glu Leu Gly Ile Asp Phe Leu Thr Ala Ser Ala His Lys Phe His Gly
            180                 185                 190

Pro Lys Gly Ile Gly Phe Leu Tyr Ala Ser Ser Met Asp Phe Asp Ser
        195                 200                 205

Tyr Leu His Gly Gly Asp Gln Glu Gln Lys Lys Arg Ala Gly Thr Glu
    210                 215                 220

Asn Leu Pro Ala Ile Val Gly Met Val Ala Ala Leu Lys Glu Asp Leu
225                 230                 235                 240

Glu Lys Gln Glu Glu His Phe Gln His Val Gln Asn Leu Glu Thr Ala
                245                 250                 255

Phe Leu Ala Glu Leu Glu Gly Ile Gln Tyr Tyr Leu Asn Arg Gly Lys
            260                 265                 270

His His Leu Pro Tyr Val Leu Asn Ile Gly Phe Pro Gly Gln Lys Asn
        275                 280                 285

Asp Leu Leu Leu Leu Arg Leu Asp Leu Ala Gly Ile Ser Ile Ser Thr
    290                 295                 300

Gly Ser Ala Cys Thr Ala Gly Val Val Gln Ser Ser His Val Leu Glu
305                 310                 315                 320

Ala Met Tyr Gly Ala Asn Ser Glu Arg Leu Lys Glu Ser Leu Arg Ile
                325                 330                 335

Ser Leu Ser Pro Gln Asn Thr Val Glu Asp Leu Gln Thr Leu Ala Lys
            340                 345                 350

Thr Leu Lys Glu Ile Ile Gly Gly
        355                 360

<210> 85
<211> 459
<212> PRT
<213> Streptococcus pneumoniae

<400> 85
Met Leu Phe Lys Leu Ser Lys Glu Lys Ile Glu Leu Gly Leu Ser Arg
1               5                   10                  15

Leu Ser Pro Ala Arg Arg Ile Phe Leu Ser Phe Ala Leu Val Ile Leu
            20                  25                  30

Leu Gly Ser Leu Leu Leu Ser Leu Pro Phe Val Gln Val Glu Ser Ser
        35                  40                  45

Arg Ala Thr Tyr Phe Asp His Leu Phe Thr Ala Val Ser Ala Val Cys
    50                  55                  60

Val Thr Gly Leu Ser Thr Leu Pro Val Ala His Thr Tyr Asn Ile Trp
65                  70                  75                  80

Gly Gln Ile Ile Cys Leu Leu Leu Ile Gln Ile Gly Gly Leu Gly Leu
                85                  90                  95

Met Thr Phe Ile Gly Val Phe Tyr Ile Gln Ser Lys Gln Lys Leu Ser
            100                 105                 110

Leu Arg Ser Arg Ala Thr Ile Gln Asp Ser Phe Ser Tyr Gly Glu Thr
        115                 120                 125

Arg Ser Leu Arg Lys Phe Val Tyr Ser Ile Phe Leu Thr Thr Phe Leu
    130                 135                 140

Val Glu Ser Leu Gly Ala Ile Leu Leu Ser Phe Arg Leu Ile Pro Gln
145                 150                 155                 160

Leu Gly Trp Gly Arg Gly Leu Phe Ser Ser Ile Phe Leu Ala Ile Ser
                165                 170                 175

Ala Phe Cys Asn Ala Gly Phe Asp Asn Leu Gly Ser Thr Ser Leu Phe
            180                 185                 190

Ala Phe Gln Thr Asp Leu Leu Val Asn Leu Val Ile Ala Gly Leu Ile
        195                 200                 205

Ile Thr Gly Gly Leu Gly Phe Met Val Trp Phe Asp Leu Ala Gly His
    210                 215                 220

Val Gly Arg Lys Lys Lys Gly Arg Leu His Phe His Thr Lys Leu Val
225                 230                 235                 240

Leu Leu Leu Thr Ile Gly Leu Leu Leu Phe Gly Thr Ala Thr Thr Leu
                245                 250                 255

Phe Leu Glu Trp Asn Asn Ala Gly Thr Ile Gly Asn Leu Pro Val Ala
            260                 265                 270

Asp Lys Val Leu Val Ser Phe Phe Gln Thr Val Thr Met Arg Thr Ala
        275                 280                 285

Gly Phe Ser Thr Ile Asp Tyr Thr Gln Ala His Pro Val Thr Leu Leu
    290                 295                 300

Ile Tyr Ile Leu Gln Met Phe Leu Gly Gly Ala Pro Gly Gly Thr Ala
305                 310                 315                 320

Gly Gly Leu Lys Ile Thr Thr Phe Phe Val Leu Leu Val Phe Ala Arg
                325                 330                 335

Ser Glu Leu Leu Gly Leu Pro His Ala Asn Val Ala Arg Arg Thr Ile
            340                 345                 350

Ala Pro Arg Thr Val Gln Lys Ser Phe Ser Val Phe Ile Ile Phe Leu
        355                 360                 365

Met Ser Phe Leu Ile Gly Leu Ile Leu Leu Gly Ile Thr Ala Lys Gly
    370                 375                 380

Asn Pro Pro Phe Ile His Leu Val Phe Glu Thr Ile Ser Ala Leu Ser
385                 390                 395                 400

Thr Val Gly Val Thr Ala Asn Leu Thr Pro Asp Leu Gly Lys Leu Ala
                405                 410                 415

Leu Ser Val Ile Met Pro Leu Met Phe Met Gly Arg Ile Gly Pro Leu
            420                 425                 430

Thr Leu Phe Val Ser Leu Ala Asp Tyr His Pro Glu Lys Lys Asp Met
        435                 440                 445

Ile His Tyr Met Lys Ala Asp Ile Ser Ile Gly
    450                 455

<210> 86
<211> 221
<212> PRT
<213> Streptococcus pneumoniae

<400> 86
Met Ser Asp Arg Thr Ile Gly Ile Leu Gly Leu Gly Ile Phe Gly Ser
  1               5                  10                  15

Ser Val Leu Ala Ala Leu Ala Lys Gln Asp Met Asn Ile Ile Ala Ile
             20                  25                  30

Asp Asp His Ala Glu Arg Ile Asn Gln Phe Glu Pro Val Leu Ala Arg
         35                  40                  45

Gly Val Ile Gly Asp Ile Thr Asp Glu Glu Leu Leu Arg Ser Ala Gly
     50                  55                  60

Ile Asp Thr Cys Asp Thr Val Val Val Ala Thr Gly Glu Asn Leu Glu
 65                  70                  75                  80

Ser Ser Val Leu Ala Val Met His Cys Lys Ser Leu Gly Val Pro Thr
                 85                  90                  95

Val Ile Ala Lys Val Lys Ser Gln Thr Ala Lys Lys Val Leu Glu Lys
            100                 105                 110

Ile Gly Ala Asp Ser Val Ile Ser Pro Glu Tyr Glu Met Gly Gln Ser
            115                 120                 125

Leu Ala Gln Thr Ile Leu Phe His Asn Ser Val Asp Val Phe Gln Leu
    130                 135                 140

Asp Lys Asn Val Ser Ile Val Glu Met Lys Ile Pro Gln Ser Trp Ala
145                 150                 155                 160

Gly Gln Ser Leu Ser Lys Leu Asp Leu Arg Gly Lys Tyr Asn Leu Asn
                165                 170                 175

Ile Leu Gly Phe Arg Glu Gln Glu Asn Ser Pro Leu Asp Val Glu Phe
            180                 185                 190

Gly Pro Asp Asp Leu Leu Lys Ala Asp Thr Tyr Ile Leu Ala Val Ile
        195                 200                 205

Asn Asn Gln Tyr Leu Asp Thr Leu Val Ala Leu Asn Ser
    210                 215                 220

<210> 87
<211> 179
<212> PRT
<213> Streptococcus pneumoniae

<400> 87
Met Lys Leu Leu Ser Ile Ala Ile Ser Ser Tyr Asn Ala Ala Ala Tyr
1               5                   10                  15

Leu His Tyr Cys Val Glu Ser Leu Val Ile Gly Gly Glu Gln Val Gly
            20                  25                  30

Ile Leu Ile Ile Asn Asp Gly Ser Gln Asp Gln Thr Gln Glu Ile Ala
        35                  40                  45

Glu Cys Leu Ala Ser Lys Tyr Pro Asn Ile Val Arg Ala Ile Tyr Gln
    50                  55                  60

Glu Asn Lys Cys His Gly Gly Ala Val Asn Arg Gly Leu Val Glu Ala
65                  70                  75                  80

Ser Gly Arg Tyr Phe Lys Val Val Asp Ser Asp Asp Trp Val Asp Pro
                85                  90                  95

Arg Ala Tyr Leu Lys Ile Leu Glu Thr Leu Gln Glu Leu Glu Ser Lys
            100                 105                 110

Gly Gln Glu Val Asp Val Phe Val Thr Asn Phe Val Tyr Glu Lys Glu
        115                 120                 125

Gly Gln Ser Arg Lys Lys Ser Met Ser Tyr Asp Ser Val Leu Pro Val
    130                 135                 140

Arg Gln Ile Phe Gly Trp Asp Gln Val Gly Asn Phe Ser Lys Gly Gln
145                 150                 155                 160

Tyr Thr Met Met His Ser Leu Ile Tyr Arg Thr Asp Leu Leu Arg Ala

165                 170                 175

Ser Gln Phe

<210> 88
<211> 623
<212> PRT
<213> Streptococcus pneumoniae

<400> 88
Met Lys Phe Asn Pro Asn Gln Arg Tyr Thr Arg Trp Ser Ile Arg Arg
1               5                   10                  15

Leu Ser Val Gly Val Ala Ser Val Val Val Ala Ser Gly Phe Phe Val
            20                  25                  30

Leu Val Gly Gln Pro Ser Ser Val Arg Ala Asp Gly Leu Asn Pro Thr
        35                  40                  45

Pro Gly Gln Val Leu Pro Glu Glu Thr Ser Gly Thr Lys Glu Gly Asp
    50                  55                  60

Leu Ser Glu Lys Pro Gly Asp Thr Val Leu Thr Gln Ala Lys Pro Glu
65                  70                  75                  80

Gly Val Thr Gly Asn Thr Asn Ser Leu Pro Thr Pro Thr Glu Arg Thr
                85                  90                  95

Glu Val Ser Glu Glu Thr Ser Pro Ser Ser Leu Asp Thr Leu Phe Glu
            100                 105                 110

Lys Asp Glu Glu Ala Gln Lys Asn Pro Glu Leu Thr Asp Val Leu Lys
        115                 120                 125

Glu Thr Val Asp Thr Ala Asp Val Asp Gly Thr Gln Ala Ser Pro Ala
    130                 135                 140

Glu Thr Thr Pro Glu Gln Val Lys Gly Gly Val Lys Glu Asn Thr Lys
145                 150                 155                 160

Asp Ser Ile Asp Val Pro Ala Ala Tyr Leu Glu Lys Ala Glu Gly Lys
                165                 170                 175

Gly Pro Phe Thr Ala Gly Val Asn Gln Val Ile Pro Tyr Glu Leu Phe
            180                 185                 190

Ala Gly Asp Gly Met Leu Thr Arg Leu Leu Leu Lys Ala Ser Asp Asn
        195                 200                 205

Ala Pro Trp Ser Asp Asn Gly Thr Ala Lys Asn Pro Ala Leu Pro Pro
    210                 215                 220

Leu Glu Gly Leu Thr Lys Gly Lys Tyr Phe Tyr Glu Val Asp Leu Asn
225                 230                 235                 240

Gly Asn Thr Val Gly Lys Gln Gly Gln Ala Leu Ile Asp Gln Leu Arg
                245                 250                 255

Ala Asn Gly Thr Gln Thr Tyr Lys Ala Thr Val Lys Val Tyr Gly Asn
            260                 265                 270

Lys Asp Gly Lys Ala Asp Leu Thr Asn Leu Val Ala Thr Lys Asn Val
        275                 280                 285

Asp Ile Asn Ile Asn Gly Leu Val Ala Lys Glu Thr Val Gln Lys Ala
    290                 295                 300

Val Ala Asp Asn Val Lys Asp Ser Ile Asp Val Pro Ala Ala Tyr Leu
305                 310                 315                 320

Glu Lys Ala Lys Gly Glu Gly Pro Phe Thr Ala Gly Val Asn His Val
                325                 330                 335

Ile Pro Tyr Glu Leu Phe Ala Gly Asp Gly Met Leu Thr Arg Leu Leu
            340                 345                 350

Leu Lys Ala Ser Asp Lys Ala Pro Trp Ser Asp Asn Gly Asp Ala Lys
        355                 360                 365

Asn Pro Ala Leu Ser Pro Leu Gly Glu Asn Val Lys Thr Lys Gly Gln
    370                 375                 380

Tyr Phe Tyr Gln Val Ala Leu Asp Gly Asn Val Ala Gly Lys Glu Lys
385                 390                 395                 400

Gln Ala Leu Ile Asp Gln Phe Arg Ala Asn Gly Thr Gln Thr Tyr Ser
                405                 410                 415

Ala Thr Val Asn Val Tyr Gly Asn Lys Asp Gly Lys Pro Asp Leu Asp
            420                 425                 430

Asn Ile Val Ala Thr Lys Lys Val Thr Ile Asn Ile Asn Gly Leu Ile
        435                 440                 445

Ser Lys Glu Thr Val Gln Lys Ala Val Ala Asp Asn Val Lys Asp Ser
    450                 455                 460

Ile Asp Val Pro Ala Ala Tyr Leu Glu Lys Ala Lys Gly Glu Gly Pro
465                 470                 475                 480

Phe Thr Ala Gly Val Asn His Val Ile Pro Tyr Glu Leu Phe Ala Gly
                485                 490                 495

Asp Gly Met Leu Thr Arg Leu Leu Leu Lys Ala Ser Asp Lys Ala Pro
            500                 505                 510

Trp Ser Asp Asn Gly Asp Ala Lys Asn Pro Ala Leu Ser Pro Leu Gly
        515                 520                 525

Glu Asn Val Lys Thr Lys Gly Gln Tyr Phe Tyr Gln Leu Ala Leu Asp
    530                 535                 540

Gly Asn Val Ala Gly Lys Glu Lys Gln Ala Leu Ile Asp Gln Phe Arg
545                 550                 555                 560

Ala Asn Gly Thr Gln Thr Tyr Ser Ala Thr Val Asn Val Tyr Gly Asn
                565                 570                 575

Lys Asp Gly Lys Pro Asp Leu Asp Asn Ile Val Ala Thr Lys Lys Val

580                 585                 590

Thr Ile Asn Ile Asn Gly Leu Ile Ser Lys Glu Thr Val Gln Lys Ala
        595                 600                 605

Val Ala Asp Asn Val Lys Thr Val Ser Met Phe Gln Gln Pro Thr
    610                 615                 620

<210> 89
<211> 350
<212> PRT
<213> Streptococcus pneumoniae

<400> 89
Met Lys Leu Lys Ser Tyr Ile Leu Val Gly Tyr Ile Ile Ser Thr Leu
1               5                   10                  15

Leu Thr Ile Leu Val Val Phe Trp Ala Val Gln Lys Met Leu Ile Ala
            20                  25                  30

Lys Gly Glu Ile Tyr Phe Leu Leu Gly Met Thr Ile Val Ala Ser Leu
        35                  40                  45

Val Gly Ala Gly Ile Ser Leu Phe Leu Leu Leu Pro Val Phe Thr Ser
    50                  55                  60

Leu Gly Lys Leu Lys Glu His Ala Lys Arg Val Ala Ala Lys Asp Phe
65                  70                  75                  80

Pro Ser Asn Leu Glu Val Gln Gly Pro Val Glu Phe Gln Gln Leu Gly
                85                  90                  95

Gln Thr Phe Asn Glu Met Ser His Asp Leu Gln Val Ser Phe Asp Ser
            100                 105                 110

Leu Glu Glu Ser Glu Arg Glu Lys Gly Leu Met Ile Ala Gln Leu Ser
        115                 120                 125

His Asp Ile Lys Thr Pro Ile Thr Ser Ile Gln Ala Thr Val Glu Gly
    130                 135                 140

Ile Leu Asp Gly Ile Ile Lys Glu Ser Glu Gln Ala His Tyr Leu Ala
145                 150                 155                 160

Thr Ile Gly Arg Gln Thr Glu Arg Leu Asn Lys Leu Val Glu Glu Leu
                165                 170                 175

Asn Phe Leu Thr Leu Asn Thr Ala Arg Asn Gln Val Glu Thr Thr Ser
            180                 185                 190

Lys Asp Ser Ile Phe Leu Asp Lys Leu Leu Ile Glu Cys Met Ser Glu
        195                 200                 205

Phe Gln Phe Leu Ile Glu Gln Glu Arg Arg Asp Val His Leu Gln Val
    210                 215                 220

Ile Pro Glu Ser Ala Arg Ile Glu Gly Asp Tyr Ala Lys Leu Ser Arg
225                 230                 235                 240

Ile Leu Val Asn Leu Val Asp Asn Ala Phe Lys Tyr Ser Ala Pro Gly
                245                 250                 255

Thr Lys Leu Glu Val Val Ala Lys Leu Glu Lys Asp Gln Leu Ser Ile
            260                 265                 270

Ser Val Thr Asp Glu Gly Gln Gly Ile Ala Pro Glu Asp Leu Glu Asn
        275                 280                 285

Ile Phe Lys Arg Leu Tyr Arg Val Glu Thr Ser Arg Asn Met Lys Thr
    290                 295                 300

Gly Gly His Gly Leu Gly Leu Ala Ile Ala Arg Glu Leu Ala His Gln
305                 310                 315                 320

Leu Gly Gly Glu Ile Thr Val Ser Ser Gln Tyr Gly Leu Gly Ser Thr
                325                 330                 335

Phe Thr Leu Val Leu Asn Leu Ser Gly Ser Glu Asn Lys Ala
            340                 345                 350

<210> 90
<211> 175
<212> PRT
<213> Streptococcus pneumoniae

<400> 90
Met Phe Gly Gln Thr Ala Gln His Gly Leu Thr Asn Ser Leu Lys Asp
  1               5                  10                  15

Phe Trp Ile Phe Leu Leu Asn Ile Gly Pro Gln Leu Ala Phe Phe Cys
             20                  25                  30

Gln Met Leu Arg Cys Ser Arg Ser Val Glu Gln Gly Thr Gly Asn His
         35                  40                  45

Arg Arg Glu Phe Asn Met Ile Gln Gln Ile Phe Ser His Phe Gly Met
     50                  55                  60

Thr His Leu Gly Gln Ile Lys Leu Val Tyr Gln Glu Ser Ile Asp Leu
 65                  70                  75                  80

Glu Leu Leu Val Asn Ala Leu Asn His His Leu Leu Ile Asp Arg Leu
                 85                  90                  95

Val Leu Thr Pro Asn Gln Ile Thr Ile Glu Ile Asp Arg Gln Ile Val
            100                 105                 110

His Gly Leu Asp Leu Leu Lys Gly Arg Lys Asp Lys Glu Ile Ile Asp
        115                 120                 125

Ile Lys Ser Met Phe Arg Gln Leu Glu Leu Ala Ser Thr Gln Gln Ile
    130                 135                 140

Cys Pro Ile Asn Gln Arg Val His His Gly Ile Leu Ala Phe Gly Glu
145                 150                 155                 160

Ile Ser Asp Leu Val Pro Ala Lys Asn Leu Pro Asn Arg Gln Asp
                165                 170                 175

<210> 91
<211> 158
<212> PRT
<213> Streptococcus pneumoniae

<400> 91
Met Glu His Leu Ala Thr Tyr Phe Ser Thr Tyr Gly Gly Ala Phe Phe
1               5                   10                  15

Ala Ala Leu Gly Ile Val Leu Ala Val Gly Leu Ser Gly Met Gly Ser
            20                  25                  30

Ala Tyr Gly Val Gly Lys Ala Gly Gln Ser Ala Ala Ala Leu Leu Lys
        35                  40                  45

Glu Gln Pro Glu Lys Phe Ala Ser Ala Leu Ile Leu Gln Leu Leu Pro
    50                  55                  60

Gly Thr Gln Gly Leu Tyr Gly Phe Val Ile Gly Ile Leu Ile Trp Leu
65                  70                  75                  80

Gln Leu Thr Pro Glu Leu Pro Leu Glu Lys Gly Val Ala Tyr Phe Phe
                85                  90                  95

Val Ala Leu Pro Ile Ala Ile Val Gly Tyr Phe Ser Ala Lys His Gln
            100                 105                 110

Gly Asn Val Ala Val Ala Gly Met Gln Ile Leu Ala Lys Arg Pro Lys
        115                 120                 125

Glu Phe Met Lys Gly Ala Ile Leu Ala Ala Met Val Glu Thr Tyr Ala
    130                 135                 140

Ile Leu Ala Phe Val Val Ser Phe Ile Leu Thr Leu Arg Val
145                 150                 155

<210> 92
<211> 165
<212> PRT
<213> Streptococcus pneumoniae

<400> 92
Met Leu Lys Ser Glu Lys Gln Ser Arg Tyr Gln Met Leu Asn Glu Glu
1               5                   10                  15

Leu Ser Phe Leu Leu Glu Gly Glu Thr Asn Val Leu Ala Asn Leu Ser
            20                  25                  30

Asn Ala Ser Ala Leu Ile Lys Ser Arg Phe Pro Asn Thr Val Phe Ala
        35                  40                  45

Gly Phe Tyr Leu Phe Asp Gly Lys Glu Leu Val Leu Gly Pro Phe Gln
    50                  55                  60

Gly Gly Val Ser Cys Ile Arg Ile Ala Leu Gly Lys Gly Val Cys Gly
65                  70                  75                  80

Glu Ala Ala His Phe Gln Glu Thr Val Ile Val Gly Asp Val Thr Thr
                85                  90                  95

Tyr Leu Asn Tyr Ile Ser Cys Asp Ser Leu Ala Lys Ser Glu Ile Val
            100                 105                 110

Val Pro Met Met Lys Asn Gly Gln Leu Leu Gly Val Leu Asp Leu Asp
        115                 120                 125

Ser Ser Glu Ile Glu Asp Tyr Asp Ala Met Asp Arg Asp Tyr Leu Glu
    130                 135                 140

Gln Phe Val Ala Ile Leu Leu Glu Lys Thr Ala Trp Asp Phe Thr Met
145                 150                 155                 160

Phe Glu Glu Lys Ser
                165

<210> 93
<211> 256
<212> PRT
<213> Streptococcus pneumoniae

<400> 93
Met Ser Val Leu Glu Ile Lys Asp Leu His Val Glu Ile Glu Gly Lys
  1               5                  10                  15

Glu Ile Leu Lys Gly Val Asn Leu Thr Leu Lys Thr Gly Glu Ile Ala
            20                  25                  30

Ala Ile Met Gly Pro Asn Gly Thr Gly Lys Ser Thr Leu Ser Ala Ala
        35                  40                  45

Ile Met Gly Asn Pro Asn Tyr Glu Val Thr Lys Gly Glu Val Leu Phe
    50                  55                  60

Asp Gly Val Asn Ile Leu Glu Leu Glu Val Asp Glu Arg Ala Arg Met
 65                  70                  75                  80

Gly Leu Phe Leu Ala Met Gln Tyr Pro Ser Glu Ile Pro Gly Ile Thr
                85                  90                  95

Asn Ala Glu Phe Leu Arg Ala Ala Met Asn Ala Gly Lys Glu Asp Asp
            100                 105                 110

Glu Lys Ile Ser Val Arg Glu Phe Ile Thr Lys Leu Asp Glu Lys Met
        115                 120                 125

Glu Leu Leu Asn Met Lys Glu Glu Met Ala Glu Arg Tyr Leu Asn Glu
    130                 135                 140

Gly Phe Ser Gly Gly Glu Lys Lys Arg Asn Glu Ile Leu Gln Leu Leu
145                 150                 155                 160

Met Leu Glu Pro Thr Phe Ala Leu Leu Asp Glu Ile Asp Ser Gly Leu
                165                 170                 175

Asp Ile Asp Ala Leu Lys Val Val Ser Lys Gly Val Asn Ala Met Arg

180                 185                 190

Gly Glu Gly Phe Gly Ala Met Ile Ile Thr His Tyr Gln Arg Leu Leu
        195                 200                 205

Asn Tyr Ile Thr Pro Asp Val Val His Val Met Met Glu Gly Arg Val
    210                 215                 220

Val Leu Ser Gly Gly Pro Glu Leu Ala Ala Arg Leu Glu Arg Glu Gly
225                 230                 235                 240

Tyr Ala Lys Leu Ala Glu Glu Leu Gly Tyr Asp Tyr Lys Glu Glu Leu
                245                 250                 255

<210> 94
<211> 156
<212> PRT
<213> Streptococcus pneumoniae

<400> 94
Met Pro Tyr Lys Arg Gln Arg Ser Phe Ser Met Ala Leu Ser Lys Leu
  1               5                  10                  15

Asp Ser Leu Tyr Met Ala Val Val Ala Asp His Ser Lys Asn Pro His
             20                  25                  30

His Gln Gly Lys Leu Glu Asp Ala Glu Gln Ile Ser Leu Asn Asn Pro
         35                  40                  45

Thr Cys Gly Asp Val Ile Asn Leu Ser Val Lys Phe Asp Ala Glu Asp
     50                  55                  60

Arg Leu Glu Asp Ile Ala Phe Leu Asn Ser Gly Cys Thr Ile Ser Thr
 65                  70                  75                  80

Ala Ser Ala Ser Met Met Thr Asp Ala Val Leu Gly Lys Thr Lys Gln
                 85                  90                  95

Glu Ile Leu Glu Leu Ala Thr Ile Phe Ser Glu Met Val Gln Gly Gln
            100                 105                 110

Lys Asp Glu Arg Gln Asp Gln Leu Gly Asp Ala Ala Phe Leu Ser Gly
        115                 120                 125

Val Ala Lys Phe Pro Gln Arg Ile Lys Cys Ala Thr Leu Ala Trp Asn
    130                 135                 140

Ala Leu Lys Lys Thr Ile Glu Asn Gln Glu Lys Gln
145                 150                 155

<210> 95
<211> 650
<212> PRT
<213> Streptococcus pneumoniae

<400> 95
Met Lys Ile Gln Asp Leu Leu Arg Lys Asp Val Met Leu Leu Asp Leu
1               5                   10                  15

Gln Ala Thr Glu Lys Thr Ala Val Ile Asp Glu Met Ile Lys Asn Leu
            20                  25                  30

Thr Asp His Gly Tyr Val Thr Asp Phe Glu Thr Phe Lys Glu Gly Ile
        35                  40                  45

Leu Ala Arg Glu Ala Leu Thr Ser Thr Gly Leu Gly Asp Gly Ile Ala
    50                  55                  60

Met Pro His Ser Lys Asn Ala Ala Val Lys Glu Ala Thr Val Leu Phe
65                  70                  75                  80

Ala Lys Ser Asn Lys Gly Val Asp Tyr Glu Ser Leu Asp Gly Gln Ala
                85                  90                  95

Thr Asp Leu Phe Phe Met Ile Ala Ala Pro Glu Gly Ala Asn Asp Thr
            100                 105                 110

His Leu Ala Ala Leu Ala Glu Leu Ser Gln Tyr Leu Met Lys Asp Gly
        115                 120                 125

Phe Ala Asp Lys Leu Arg Gln Ala Thr Ser Ala Asp Gln Val Ile Glu
    130                 135                 140

Leu Phe Asp Gln Ala Ser Glu Lys Thr Glu Glu Leu Val Gln Ala Pro
145                 150                 155                 160

Ala Asn Asp Ser Gly Asp Phe Ile Val Ala Val Thr Ala Cys Thr Thr
                165                 170                 175

Gly Ile Ala His Thr Tyr Met Ala Gln Glu Ala Leu Gln Lys Val Ala
            180                 185                 190

Ala Glu Met Gly Val Gly Ile Lys Val Glu Thr Asn Gly Ala Ser Gly
        195                 200                 205

Val Gly Asn Gln Leu Thr Ala Glu Asp Ile Arg Lys Ala Lys Ala Ile
    210                 215                 220

Ile Ile Ala Ala Asp Lys Ala Val Glu Met Asp Arg Phe Asp Gly Lys
225                 230                 235                 240

Pro Leu Ile Asn Arg Pro Val Ala Asp Gly Ile Arg Lys Thr Glu Glu
                245                 250                 255

Leu Ile Asn Leu Ala Leu Ser Gly Asp Thr Glu Val Tyr Arg Ala Ala
            260                 265                 270

Asn Gly Ala Lys Ala Ala Thr Ala Ser Asn Glu Lys Gln Ser Leu Gly
        275                 280                 285

Gly Ala Leu Tyr Lys His Leu Met Ser Gly Val Ser Gln Met Leu Pro
    290                 295                 300

Phe Val Ile Gly Gly Gly Ile Met Ile Ala Leu Ala Phe Leu Ile Asp
305                 310                 315                 320

Gly Ala Leu Gly Val Pro Asn Glu Asn Leu Gly Asn Leu Gly Ser Tyr
                325                 330                 335

His Glu Leu Ala Ser Met Phe Met Lys Ile Gly Gly Ala Ala Phe Gly
            340                 345                 350

Leu Met Leu Pro Val Phe Ala Gly Tyr Val Ala Tyr Ser Ile Ala Glu
        355                 360                 365

Lys Pro Gly Leu Val Ala Gly Phe Val Ala Gly Ala Ile Ala Lys Glu
    370                 375                 380

Gly Phe Ala Phe Gly Lys Ile Pro Tyr Ala Ala Gly Gly Glu Ala Thr
385                 390                 395                 400

Ser Thr Leu Ala Gly Val Ser Ser Gly Phe Leu Gly Ala Leu Val Gly
                405                 410                 415

Gly Phe Ile Ala Gly Ala Leu Val Leu Ala Ile Lys Lys Tyr Val Lys
            420                 425                 430

Val Pro Arg Ser Leu Glu Gly Ala Lys Ser Ile Leu Leu Leu Pro Leu
        435                 440                 445

Leu Gly Thr Ile Leu Thr Gly Phe Val Met Leu Ala Val Asn Ile Pro
    450                 455                 460

Met Ala Ala Ile Asn Thr Ala Met Asn Asp Phe Leu Gly Gly Leu Gly
465                 470                 475                 480

Gly Gly Ser Ala Val Leu Leu Gly Ile Val Leu Gly Gly Met Met Ala
                485                 490                 495

Val Asp Met Gly Gly Pro Val Asn Lys Ala Ala Tyr Val Phe Gly Thr
            500                 505                 510

Gly Thr Leu Ala Ala Thr Val Ser Ser Gly Gly Ser Val Ala Met Ala
        515                 520                 525

Ala Val Met Ala Gly Gly Met Val Pro Pro Leu Ala Ile Phe Val Ala
    530                 535                 540

Thr Leu Leu Phe Lys Asp Lys Phe Thr Lys Glu Glu Arg Asn Ser Gly
545                 550                 555                 560

Leu Thr Asn Ile Ile Met Gly Leu Ser Phe Ile Thr Glu Gly Ala Ile
                565                 570                 575

Pro Phe Gly Ala Ala Asp Pro Ala Arg Ala Ile Pro Ser Phe Ile Leu
            580                 585                 590

Gly Ser Ala Val Ala Gly Gly Leu Val Gly Leu Thr Gly Ile Lys Leu
        595                 600                 605

Met Ala Pro His Gly Gly Ile Phe Val Ile Ala Leu Thr Ser Asn Ala
    610                 615                 620

Leu Leu Tyr Leu Val Ser Val Leu Val Gly Ala Ile Val Ser Gly Val
625                 630                 635                 640

Val Tyr Gly Tyr Leu Arg Lys Pro Gln Ala
                645                 650

<210> 96
<211> 767
<212> PRT
<213> Streptococcus pneumoniae

<400> 96
Met Ala Asn Lys Asn Thr Ser Thr Thr Arg Arg Arg Pro Ser Lys Ala
1               5                   10                  15

Glu Leu Glu Arg Lys Glu Ala Ile Gln Arg Met Leu Ile Ser Leu Gly
            20                  25                  30

Ile Ala Ile Leu Leu Ile Phe Ala Ala Phe Lys Leu Gly Ala Ala Gly
        35                  40                  45

Ile Thr Leu Tyr Asn Leu Ile Arg Leu Leu Val Gly Ser Leu Ala Tyr
    50                  55                  60

Leu Ala Ile Phe Gly Leu Leu Ile Tyr Leu Phe Phe Phe Lys Trp Ile
65                  70                  75                  80

Arg Lys Gln Glu Gly Leu Leu Ser Gly Phe Phe Thr Ile Phe Ala Gly
                85                  90                  95

Leu Leu Leu Ile Phe Glu Ala Tyr Leu Val Trp Lys Tyr Gly Leu Asp
            100                 105                 110

Lys Ser Val Leu Lys Gly Thr Met Ala Gln Val Val Thr Asp Leu Thr
        115                 120                 125

Gly Phe Arg Thr Thr Ser Phe Ala Gly Gly Gly Leu Ile Gly Val Ala
    130                 135                 140

Leu Tyr Ile Pro Thr Ala Phe Leu Phe Ser Asn Ile Gly Thr Tyr Phe
145                 150                 155                 160

Ile Gly Ser Ile Leu Ile Leu Val Gly Ser Leu Leu Val Ser Pro Trp
                165                 170                 175

Ser Val Tyr Asp Ile Ala Glu Phe Phe Ser Arg Gly Phe Ala Lys Trp
            180                 185                 190

Trp Glu Gly His Glu Arg Arg Lys Glu Glu Arg Phe Val Lys Gln Glu
        195                 200                 205

Glu Lys Ala Arg Gln Lys Ala Glu Lys Glu Ala Arg Leu Glu Gln Glu
    210                 215                 220

Glu Thr Glu Lys Ala Leu Leu Asp Leu Pro Pro Val Asp Met Glu Thr
225                 230                 235                 240

Gly Glu Ile Leu Thr Glu Glu Ala Val Gln Asn Leu Pro Pro Ile Pro
                245                 250                 255

Glu Glu Lys Trp Val Glu Pro Glu Ile Ile Leu Pro Gln Ala Glu Leu
            260                 265                 270

Lys Phe Pro Glu Gln Glu Asp Asp Ser Asp Asp Glu Asp Val Gln Val
        275                 280                 285

Asp Phe Ser Ala Lys Glu Ala Leu Glu Tyr Lys Leu Pro Ser Leu Gln
    290                 295                 300

Leu Phe Ala Pro Asp Lys Pro Lys Asp Gln Ser Lys Glu Lys Lys Ile
305                 310                 315                 320

Val Arg Glu Asn Ile Lys Ile Leu Glu Ala Thr Phe Ala Ser Phe Gly
                325                 330                 335

Ile Lys Val Thr Val Glu Arg Ala Glu Ile Gly Pro Ser Val Thr Lys
            340                 345                 350

Tyr Glu Val Lys Pro Ala Val Gly Val Arg Val Asn Arg Ile Ser Asn
        355                 360                 365

Leu Ser Asp Asp Leu Ala Leu Ala Leu Ala Ala Lys Asp Val Arg Ile
    370                 375                 380

Glu Ala Pro Ile Pro Gly Lys Ser Leu Ile Gly Ile Glu Val Pro Asn
385                 390                 395                 400

Ser Asp Ile Ala Thr Val Ser Phe Arg Glu Leu Trp Glu Gln Ser Gln
                405                 410                 415

Thr Lys Ala Glu Asn Phe Leu Glu Ile Pro Leu Gly Lys Ala Val Asn
            420                 425                 430

Gly Thr Ala Arg Ala Phe Asp Leu Ser Lys Met Pro His Leu Leu Val
        435                 440                 445

Ala Gly Ser Thr Gly Ser Gly Lys Ser Val Ala Val Asn Gly Ile Ile
    450                 455                 460

Ala Ser Ile Leu Met Lys Ala Arg Pro Asp Gln Val Lys Phe Met Met
465                 470                 475                 480

Val Asp Pro Lys Met Val Glu Leu Ser Val Tyr Asn Asp Ile Pro His
                485                 490                 495

Leu Leu Ile Pro Val Val Thr Asn Pro Arg Lys Ala Ser Lys Ala Leu
            500                 505                 510

Gln Lys Val Val Asp Glu Met Glu Asn Arg Tyr Glu Leu Phe Ala Lys
        515                 520                 525

Val Gly Val Arg Asn Ile Ala Gly Phe Asn Ala Lys Val Glu Glu Phe
    530                 535                 540

Asn Ser Gln Ser Glu Tyr Lys Gln Ile Pro Leu Pro Phe Ile Val Val
545                 550                 555                 560

Ile Val Asp Glu Leu Ala Asp Leu Met Met Val Ala Ser Lys Glu Val
                565                 570                 575

Glu Asp Ala Ile Ile Arg Leu Gly Gln Lys Ala Arg Ala Ala Gly Ile
            580                 585                 590

His Met Ile Leu Ala Thr Gln Arg Pro Ser Val Asp Val Ile Ser Gly
        595                 600                 605

Leu Ile Lys Ala Asn Val Pro Ser Arg Val Ala Phe Ala Val Ser Ser
    610                 615                 620

Gly Thr Asp Ser Arg Thr Ile Leu Asp Glu Asn Gly Ala Glu Lys Leu
625                 630                 635                 640

Leu Gly Arg Gly Asp Met Leu Phe Lys Pro Ile Asp Glu Asn His Pro
                645                 650                 655

Val Arg Leu Gln Gly Ser Phe Ile Ser Asp Asp Asp Val Glu Arg Ile
            660                 665                 670

Val Asn Phe Ile Lys Thr Gln Ala Asp Ala Asp Tyr Asp Glu Ser Phe
        675                 680                 685

Asp Pro Gly Glu Val Ser Glu Asn Glu Gly Glu Phe Ser Asp Gly Asp
    690                 695                 700

Ala Gly Gly Asp Pro Leu Phe Glu Glu Ala Lys Ser Leu Val Ile Glu
705                 710                 715                 720

Thr Gln Lys Ala Ser Ala Ser Met Ile Gln Arg Arg Leu Ser Val Gly
                725                 730                 735

Phe Asn Arg Ala Thr Arg Leu Met Glu Glu Leu Glu Ile Ala Gly Val
            740                 745                 750

Ile Gly Pro Ala Glu Gly Thr Lys Pro Arg Lys Val Leu Gln Gln
        755                 760                 765

<210> 97
<211> 175
<212> PRT
<213> Streptococcus pneumoniae

<400> 97
Met Ser Tyr Phe Lys Lys Tyr Lys Phe Asp Lys Ser Gln Phe Lys Leu
1               5                   10                  15

Gly Met Arg Thr Phe Lys Thr Gly Ile Ala Val Phe Leu Val Leu Leu
            20                  25                  30

Ile Phe Gly Phe Phe Gly Trp Lys Gly Leu Gln Ile Gly Ala Leu Thr
        35                  40                  45

Ala Val Phe Ser Leu Arg Glu Ser Phe Asp Glu Ser Val His Phe Gly
    50                  55                  60

Thr Ser Arg Ile Leu Gly Asn Ser Ile Gly Gly Leu Tyr Ala Leu Val
65                  70                  75                  80

Phe Phe Leu Leu Asn Thr Phe Phe His Glu Ala Phe Trp Val Thr Leu
                85                  90                  95

Val Val Val Pro Ile Cys Thr Met Leu Thr Ile Met Thr Asn Val Ala
            100                 105                 110

Met Asn Asn Lys Ala Gly Val Ile Gly Gly Val Ala Ala Met Leu Ile
        115                 120                 125

Ile Thr Leu Ser Ile Pro Ser Gly Glu Thr Ile Leu Tyr Val Phe Val
    130                 135                 140

Arg Val Leu Glu Thr Phe Met Gly Val Phe Val Ala Ile Ile Val Asn
145                 150                 155                 160

Tyr Asp Ile Asp Arg Ile Arg Leu Phe Leu Glu Lys Lys Glu Lys
                165                 170                 175

<210> 98
<211> 156
<212> PRT
<213> Streptococcus pneumoniae

<400> 98
Met Asn Lys Ser Glu His Arg His Gln Leu Ile Arg Ala Leu Ile Thr
1               5                   10                  15

Lys Asn Lys Ile His Thr Gln Ala Glu Leu Gln Ala Leu Leu Ala Glu
            20                  25                  30

Asn Asp Ile Gln Val Thr Gln Ala Thr Leu Ser Arg Asp Ile Lys Asn
        35                  40                  45

Met Asn Leu Ser Lys Val Arg Glu Glu Asp Ser Ala Tyr Tyr Val Leu
    50                  55                  60

Asn Asn Gly Ser Ile Ser Lys Trp Glu Lys Arg Leu Glu Leu Tyr Met
65                  70                  75                  80

Glu Asp Ala Leu Val Trp Met Arg Pro Val Gln His Gln Val Leu Leu
                85                  90                  95

Lys Thr Leu Pro Gly Leu Ala Gln Ser Phe Gly Ser Ile Ile Asp Thr
            100                 105                 110

Leu Ser Phe Pro Asp Ala Ile Ala Thr Leu Cys Gly Asn Asp Val Cys
        115                 120                 125

Leu Ile Ile Cys Glu Asp Ala Asp Thr Ala Gln Lys Cys Phe Glu Glu
    130                 135                 140

Leu Lys Lys Phe Ala Pro Pro Phe Phe Phe Glu Glu
145                 150                 155

<210> 99
<211> 495
<212> PRT
<213> Streptococcus pneumoniae

<400> 99
Met Lys Ser Ile Lys Leu Asn Ala Leu Ser Tyr Met Gly Ile Arg Val
1               5                   10                  15

Leu Asn Ile Ile Phe Pro Ile Leu Thr Gly Thr Tyr Val Ala Arg Val
            20                  25                  30

Leu Asp Arg Thr Asp Tyr Gly Tyr Phe Asn Ser Val Asp Thr Ile Leu
        35                  40                  45

Ser Phe Phe Leu Pro Phe Ala Thr Tyr Gly Val Tyr Asn Tyr Gly Leu
    50                  55                  60

Arg Ala Ile Ser Asn Val Lys Asp Asn Lys Lys Asp Leu Asn Arg Thr
65                  70                  75                  80

Phe Ser Ser Leu Phe Tyr Leu Cys Ile Ala Cys Thr Ile Leu Thr Thr
                85                  90                  95

Ala Val Tyr Ile Leu Ala Tyr Pro Leu Phe Phe Thr Asp Asn Pro Ile
            100                 105                 110

Val Lys Lys Val Tyr Leu Val Met Gly Ile Gln Leu Ile Ala Gln Ile
        115                 120                 125

Phe Ser Ile Glu Trp Val Asn Glu Ala Leu Glu Asn Tyr Ser Phe Leu
    130                 135                 140

Phe Tyr Lys Thr Ala Phe Ile Arg Ile Leu Met Leu Val Ser Ile Phe
145                 150                 155                 160

Leu Phe Val Lys Asn Glu His Asp Ile Val Val Tyr Thr Leu Val Met
                165                 170                 175

Ser Leu Ser Thr Leu Ile Asn Tyr Leu Ile Ser Tyr Phe Trp Ile Lys
            180                 185                 190

Arg Asp Ile Lys Leu Val Lys Ile His Leu Ser Asp Phe Lys Pro Leu
        195                 200                 205

Phe Leu Pro Leu Thr Ala Met Leu Val Phe Ala Asn Ala Asn Met Leu
    210                 215                 220

Phe Thr Phe Leu Asp Arg Leu Phe Leu Val Lys Thr Gly Ile Asp Val
225                 230                 235                 240

Asn Val Ser Tyr Tyr Thr Ile Ala Gln Arg Ile Val Thr Val Ile Ala
                245                 250                 255

Gly Val Val Thr Gly Ala Ile Gly Val Ser Val Pro Arg Leu Ser Tyr
            260                 265                 270

Tyr Leu Gly Lys Gly Asp Lys Glu Ala Tyr Val Ser Leu Val Asn Arg
        275                 280                 285

Gly Ser Arg Ile Phe Asn Phe Phe Ile Ile Pro Leu Ser Phe Gly Leu
    290                 295                 300

Met Val Leu Gly Pro Asn Ala Ile Leu Leu Tyr Gly Ser Glu Lys Tyr
305                 310                 315                 320

Ile Gly Gly Gly Ile Leu Thr Ser Leu Phe Ala Phe Arg Thr Ile Ile
                325                 330                 335

Leu Ala Leu Asp Thr Ile Leu Gly Ser Gln Ile Leu Phe Thr Asn Gly
            340                 345                 350

Tyr Glu Lys Arg Ile Thr Val Tyr Thr Val Phe Ala Gly Leu Leu Asn
        355                 360                 365

Leu Gly Leu Asn Ser Leu Leu Phe Phe Asn His Ile Val Ala Pro Glu
    370                 375                 380

Tyr Tyr Leu Leu Thr Thr Met Leu Ser Glu Thr Ser Leu Leu Val Phe
385                 390                 395                 400

Tyr Ile Ile Phe Ile His Arg Lys Gln Leu Ile His Leu Gly His Ile
                405                 410                 415

Phe Ser Tyr Thr Val Arg Tyr Ser Leu Phe Ser Leu Ser Phe Val Ala
            420                 425                 430

Ile Tyr Phe Leu Ile Asn Phe Val Tyr Pro Val Asp Met Val Ile Asn
        435                 440                 445

Leu Pro Phe Leu Ile Asn Thr Gly Leu Ile Val Leu Leu Ser Ala Ile
    450                 455                 460

Ser Tyr Ile Ser Leu Leu Val Phe Thr Lys Asp Ser Ile Phe Tyr Glu
465                 470                 475                 480

Phe Leu Asn His Val Leu Ala Leu Lys Asn Lys Phe Lys Lys Ser
                485                 490                 495

<210> 100
<211> 286
<212> PRT
<213> Streptococcus pneumoniae

<400> 100
Met Glu Leu Phe Met Lys Ile Thr Asn Tyr Glu Ile Tyr Lys Leu Lys
1               5                   10                  15

Lys Ser Gly Leu Thr Asn Gln Gln Ile Leu Lys Val Leu Glu Tyr Gly
            20                  25                  30

Glu Asn Val Asp Gln Glu Leu Leu Leu Gly Asp Ile Ala Asp Ile Ser
        35                  40                  45

Gly Cys Arg Asn Pro Ala Val Phe Met Glu Arg Tyr Phe Gln Ile Asp
    50                  55                  60

Asp Ala His Leu Ser Lys Glu Phe Gln Lys Phe Pro Ser Phe Ser Ile
65                  70                  75                  80

Leu Asp Asp Cys Tyr Pro Trp Asp Leu Ser Glu Ile Tyr Asp Ala Pro
                85                  90                  95

Val Leu Leu Phe Tyr Lys Gly Asn Leu Asp Leu Leu Lys Phe Pro Lys
            100                 105                 110

Val Ala Val Val Gly Ser Arg Ala Cys Ser Lys Gln Gly Ala Lys Ser
        115                 120                 125

Val Glu Lys Val Ile Gln Gly Leu Glu Asn Glu Leu Val Ile Val Ser
    130                 135                 140

Gly Leu Ala Lys Gly Ile Asp Thr Ala Ala His Met Ala Ala Leu Gln
145                 150                 155                 160

Asn Gly Gly Lys Thr Ile Ala Val Ile Gly Thr Gly Leu Asp Val Phe
                165                 170                 175

Tyr Pro Lys Ala Asn Lys Arg Leu Gln Asp Tyr Ile Gly Asn Asp His
            180                 185                 190

Leu Val Leu Ser Glu Tyr Gly Pro Gly Glu Gln Pro Leu Lys Phe His
        195                 200                 205

Phe Pro Ala Arg Asn Arg Ile Ile Ala Gly Leu Cys Arg Gly Val Ile
    210                 215                 220

Val Ala Glu Ala Lys Met Arg Ser Gly Ser Leu Ile Thr Cys Glu Arg
225                 230                 235                 240

Ala Met Glu Glu Gly Arg Asp Val Phe Ala Ile Pro Gly Ser Ile Leu
                245                 250                 255

Asp Gly Leu Ser Asp Gly Cys His His Leu Ile Gln Glu Gly Ala Lys
            260                 265                 270

Leu Val Thr Ser Gly Gln Asp Val Leu Ala Glu Phe Glu Phe
        275                 280                 285

<210> 101
<211> 267
<212> PRT
<213> Streptococcus pneumoniae

<400> 101
Met Lys Gln Leu Thr Val Glu Asp Ala Lys Gln Ile Glu Leu Glu Ile
  1               5                  10                  15

Leu Asp Tyr Ile Asp Thr Leu Cys Lys Lys His Asn Ile Asn Tyr Ile
            20                  25                  30

Ile Asn Tyr Gly Thr Leu Ile Gly Ala Val Arg His Glu Gly Phe Ile
        35                  40                  45

Pro Trp Asp Asp Asp Ile Asp Leu Ser Met Pro Arg Glu Asp Tyr Gln
    50                  55                  60

Arg Phe Ile Asn Ile Phe Gln Lys Glu Lys Ser Lys Tyr Lys Leu Leu
 65                  70                  75                  80

Ser Leu Glu Thr Asp Lys Asn Tyr Phe Asn Asn Phe Ile Lys Ile Thr
                85                  90                  95

Asp Ser Thr Thr Lys Ile Ile Asp Thr Arg Asn Thr Lys Thr Tyr Glu
            100                 105                 110

Ser Gly Ile Phe Ile Asp Ile Phe Pro Ile Asp Arg Phe Asp Asp Pro

115                 120                 125

Lys Val Ile Asp Thr Cys Tyr Lys Leu Glu Ser Phe Lys Leu Leu Ser
    130                 135                 140

Phe Ser Lys His Lys Asn Ile Val Tyr Lys Asp Ser Leu Leu Lys Asp
145                 150                 155                 160

Trp Ile Arg Thr Ala Phe Trp Leu Leu Leu Arg Pro Val Ser Pro Arg
                165                 170                 175

Tyr Phe Ala Asn Lys Ile Glu Lys Glu Ile Gln Lys Tyr Ser Arg Glu
            180                 185                 190

Asn Gly Gln Tyr Met Ala Phe Ile Pro Ser Lys Phe Lys Glu Lys Glu
        195                 200                 205

Val Phe Pro Ser Gly Thr Phe Asp Lys Thr Ile Asp Leu Pro Phe Glu
    210                 215                 220

Asn Leu Ser Leu Pro Ala Pro Glu Lys Phe Asp Thr Ile Leu Thr Gln
225                 230                 235                 240

Phe Tyr Gly Asp Tyr Met Thr Leu Pro Pro Glu Glu Lys Arg Phe Tyr
                245                 250                 255

Ser His Glu Phe His Ala Tyr Lys Leu Glu Asp
            260                 265

<210> 102
<211> 508
<212> PRT
<213> Streptococcus pneumoniae

<400> 102
Met Ile Lys Ile Asn His Leu Thr Ile Thr Gln Asn Lys Asp Leu Arg
 1               5                  10                  15

Asp Leu Val Ser Asp Leu Thr Met Thr Ile Gln Asp Gly Glu Lys Val
            20                  25                  30

Ala Ile Ile Gly Glu Glu Gly Asn Gly Lys Ser Thr Leu Leu Lys Ile
        35                  40                  45

Leu Met Gly Glu Ala Leu Ser Asp Phe Thr Ile Lys Gly Asn Ile Gln
    50                  55                  60

Ser Asp Tyr Gln Ser Leu Ala Tyr Ile Pro Gln Lys Val Pro Glu Asp
65                  70                  75                  80

Leu Lys Lys Lys Thr Leu His Asp Tyr Phe Phe Leu Asp Ser Ile Asp
                85                  90                  95

Leu Asp Tyr Ser Ile Leu Tyr Arg Leu Ala Glu Glu Leu His Phe Asp
            100                 105                 110

Ser Asn Arg Phe Ala Ser Asp Gln Glu Ile Gly Asn Leu Ser Gly Gly
        115                 120                 125

Glu Ala Leu Lys Ile Gln Leu Ile His Glu Leu Ala Lys Pro Phe Glu
    130                 135                 140

Ile Leu Phe Leu Asp Glu Pro Ser Asn Asp Leu Asp Leu Glu Thr Val
145                 150                 155                 160

Asp Trp Leu Lys Gly Gln Ile Gln Lys Thr Arg Gln Thr Val Ile Phe
                165                 170                 175

Ile Ser His Asp Glu Asp Phe Leu Ser Glu Thr Ala Asp Thr Ile Val
            180                 185                 190

His Leu Arg Leu Val Lys His Arg Lys Glu Ala Glu Thr Leu Val Glu
        195                 200                 205

His Leu Asp Tyr Asp Ser Tyr Ser Glu Gln Arg Lys Ala Asn Phe Ala
    210                 215                 220

Lys Gln Ser Gln Gln Ala Ala Asn Asn Gln Arg Ala Tyr Asp Lys Thr
225                 230                 235                 240

Met Glu Lys His Arg Arg Val Lys Gln Asn Val Glu Thr Ala Leu Arg
                245                 250                 255

Ala Thr Lys Asp Ser Thr Ala Gly Arg Leu Leu Ala Lys Lys Met Lys
            260                 265                 270

Thr Val Leu Ser Gln Glu Lys Arg Tyr Glu Lys Ala Ala Gln Ser Met
        275                 280                 285

Thr Gln Lys Pro Leu Glu Glu Glu Gln Ile Gln Leu Phe Phe Ser Asp
    290                 295                 300

Ile Gln Pro Leu Pro Ala Ser Lys Val Leu Val Gln Leu Glu Lys Glu
305                 310                 315                 320

Asn Leu Ser Ile Asp Asp Arg Val Leu Val Gln Lys Leu Gln Leu Thr
                325                 330                 335

Val Arg Gly Gln Glu Lys Ile Gly Ile Ile Gly Pro Asn Gly Val Gly
            340                 345                 350

Lys Ser Thr Leu Leu Ala Lys Leu Gln Arg Leu Leu Asn Asp Lys Arg
        355                 360                 365

Glu Ile Ser Leu Gly Phe Met Pro Gln Asp Tyr His Lys Lys Leu Gln
    370                 375                 380

Leu Asp Leu Ser Pro Ile Ala Tyr Leu Ser Lys Thr Gly Glu Lys Glu
385                 390                 395                 400

Glu Leu Gln Lys Ile Gln Ser His Leu Ala Ser Leu Asn Phe Ser Tyr
                405                 410                 415

Pro Glu Met Gln His Gln Ile Arg Ser Leu Ser Gly Gly Gln Gln Gly
            420                 425                 430

Lys Leu Leu Leu Leu Asp Leu Val Leu Arg Lys Pro Asn Phe Leu Leu
        435                 440                 445

Leu Asp Glu Pro Thr Arg Asn Phe Ser Pro Thr Ser Gln Pro Gln Ile

450                 455                 460

Arg Lys Leu Phe Ala Thr Tyr Pro Gly Gly Leu Ile Thr Val Ser His
465                 470                 475                 480

Asp Arg Arg Phe Leu Lys Glu Val Cys Ser Ile Ile Tyr Arg Met Thr
                485                 490                 495

Glu His Gly Leu Lys Leu Val Asn Leu Glu Asp Leu
            500                 505

<210> 103
<211> 237
<212> PRT
<213> Streptococcus pneumoniae

<400> 103
Met Lys Pro Lys Thr Phe Tyr Asn Leu Leu Ala Glu Gln Asn Leu Pro
  1               5                  10                  15

Leu Ser Asp Gln Gln Lys Glu Gln Phe Glu Arg Tyr Phe Glu Leu Leu
             20                  25                  30

Val Glu Trp Asn Glu Lys Ile Asn Leu Thr Ala Ile Thr Asp Lys Glu
         35                  40                  45

Glu Val Tyr Leu Lys His Phe Tyr Asp Ser Ile Ala Pro Ile Leu Gln
     50                  55                  60

Gly Leu Ile Pro Asn Glu Thr Ile Lys Leu Leu Asp Ile Gly Ala Gly
 65                  70                  75                  80

Ala Gly Phe Pro Ser Leu Pro Met Lys Ile Leu Tyr Pro Glu Leu Asp
                 85                  90                  95

Val Thr Ile Ile Asp Ser Leu Asn Lys Arg Ile Asn Phe Leu Gln Leu
            100                 105                 110

Leu Ala Gln Glu Leu Asp Leu Asn Gly Val His Phe Tyr His Gly Arg
        115                 120                 125

Ala Glu Asp Phe Ala Gln Asp Lys Asn Phe Arg Ala Gln Tyr Asp Phe
    130                 135                 140

Val Thr Ala Arg Ala Val Ala Arg Met Gln Val Leu Ser Glu Leu Thr
145                 150                 155                 160

Ile Pro Tyr Leu Lys Val Gly Gly Lys Leu Leu Ala Leu Lys Ala Ser
                165                 170                 175

Asn Ala Pro Glu Glu Leu Leu Glu Ala Lys Asn Ala Leu Asn Leu Leu
            180                 185                 190

Phe Ser Lys Val Glu Asp Asn Leu Ser Tyr Ala Leu Pro Asn Arg Asp
        195                 200                 205

Pro Arg Tyr Ile Thr Val Val Glu Lys Lys Lys Glu Thr Pro Asn Lys
    210                 215                 220

Tyr Pro Arg Lys Ala Gly Met Pro Asn Lys Arg Pro Leu
225                 230                 235

<210> 104
<211> 268
<212> PRT
<213> Streptococcus pneumoniae

<400> 104
Met Ser Ile Lys Leu Ile Ala Val Asp Ile Asp Gly Thr Leu Val Asn
1               5                   10                  15

Ser Gln Lys Glu Ile Thr Pro Glu Val Phe Ser Ala Ile Gln Asp Ala
            20                  25                  30

Lys Glu Ala Gly Val Lys Val Val Ile Ala Thr Gly Arg Pro Ile Ala
        35                  40                  45

Gly Val Ala Lys Leu Leu Asp Asp Leu Gln Leu Arg Asp Glu Gly Asp
    50                  55                  60

Tyr Val Val Thr Phe Asn Gly Ala Leu Val Gln Glu Thr Ala Thr Gly
65                  70                  75                  80

His Glu Ile Ile Ser Glu Ser Leu Thr Tyr Glu Asp Tyr Leu Asp Met
                85                  90                  95

Glu Phe Leu Ser Arg Lys Leu Gly Val His Met His Ala Ile Thr Lys
            100                 105                 110

Asp Gly Ile Tyr Thr Ala Asn Arg Asn Ile Gly Lys Tyr Thr Val His
        115                 120                 125

Glu Ser Thr Leu Val Ser Met Pro Ile Phe Tyr Arg Thr Pro Glu Glu
    130                 135                 140

Met Ala Gly Lys Glu Ile Val Lys Cys Met Phe Ile Asp Glu Pro Glu
145                 150                 155                 160

Ile Leu Asp Ala Ala Ile Glu Lys Ile Pro Ala Glu Phe Tyr Glu Arg
                165                 170                 175

Tyr Ser Ile Asn Lys Ser Ala Pro Phe Tyr Leu Glu Leu Leu Lys Lys
            180                 185                 190

Asn Val Asp Lys Gly Ser Ala Ile Thr His Leu Ala Glu Lys Leu Gly
        195                 200                 205

Leu Thr Lys Asp Glu Thr Met Ala Ile Gly Asp Glu Glu Asn Asp Arg
    210                 215                 220

Ala Met Leu Glu Val Val Gly Asn Pro Val Val Met Glu Asn Gly Asn
225                 230                 235                 240

Pro Glu Ile Lys Lys Ile Ala Lys Tyr Ile Thr Lys Thr Asn Asp Glu
                245                 250                 255

Ser Gly Val Ala His Ala Ile Arg Thr Trp Val Leu
            260                 265

<210> 105
<211> 186
<212> PRT
<213> Streptococcus pneumoniae

<400> 105
Met Thr Trp Ile Ile Leu Gly Val Ile Ala Leu Ile Val Ile Phe Val
1               5                   10                  15

Ile Val Ser Tyr Asn Gly Leu Val Lys Asn Arg Met Gln Thr Lys Glu
            20                  25                  30

Ala Trp Ser Gln Ile Asp Val Gln Leu Lys Arg Arg Asn Asp Leu Leu
        35                  40                  45

Pro Asn Leu Ile Glu Thr Val Lys Gly Tyr Ala Lys Tyr Glu Gly Ser
    50                  55                  60

Thr Leu Glu Lys Val Ala Glu Leu Arg Asn Gln Val Ala Ala Ala Thr
65                  70                  75                  80

Ser Pro Ala Glu Ala Met Lys Ala Ser Asp Ala Leu Thr Arg Gln Val
                85                  90                  95

Ser Gly Ile Phe Ala Val Ala Glu Ser Tyr Pro Asp Leu Lys Ala Ser
            100                 105                 110

Ala Asn Phe Val Lys Leu Gln Glu Glu Leu Thr Asn Thr Glu Asn Lys
        115                 120                 125

Ile Ser Tyr Ser Arg Gln Leu Tyr Asn Ser Val Val Ser Asn Tyr Asn
    130                 135                 140

Val Lys Leu Glu Thr Phe Pro Ser Asn Ile Ile Ala Gly Met Phe Gly
145                 150                 155                 160

Phe Lys Ala Ala Asp Phe Leu Gln Thr Pro Glu Glu Glu Lys Ser Val
                165                 170                 175

Pro Lys Val Asp Phe Ser Gly Leu Gly Asp
            180                 185

<210> 106
<211> 299
<212> PRT
<213> Streptococcus pneumoniae

<400> 106
Met Leu Phe Asp Gln Ile Ala Ser Asn Lys Arg Lys Thr Trp Ile Leu
1               5                   10                  15

Leu Leu Val Phe Phe Leu Leu Leu Ala Leu Val Gly Tyr Ala Val Gly
            20                  25                  30

Tyr Leu Phe Ile Arg Ser Gly Leu Gly Gly Leu Val Ile Ala Leu Ile
        35                  40                  45

Ile Gly Phe Ile Tyr Ala Leu Ser Met Ile Phe Gln Ser Thr Glu Ile
    50                  55                  60

Val Met Ser Met Asn Gly Ala Arg Glu Val Asp Glu Gln Thr Ala Pro
65                  70                  75                  80

Asp Leu Tyr His Val Val Glu Asp Met Ala Leu Val Ala Gln Ile Pro
                85                  90                  95

Met Pro Arg Val Phe Ile Ile Asp Asp Pro Ala Leu Asn Ala Phe Ala
            100                 105                 110

Thr Gly Ser Asn Pro Gln Asn Ala Ala Val Ala Ala Thr Ser Gly Leu
        115                 120                 125

Leu Ala Ile Met Asn Arg Glu Glu Leu Glu Ala Val Met Gly His Glu
    130                 135                 140

Val Ser His Ile Arg Asn Tyr Asp Ile Arg Ile Ser Thr Ile Ala Val
145                 150                 155                 160

Ala Leu Ala Ser Ala Ile Thr Met Leu Ser Ser Met Ala Gly Arg Met
                165                 170                 175

Met Trp Trp Gly Gly Ala Gly Arg Arg Arg Ser Asp Asp Asp Arg Asp
            180                 185                 190

Gly Asn Gly Leu Glu Ile Ile Met Leu Val Val Ser Leu Leu Ala Ile
        195                 200                 205

Val Leu Ala Pro Leu Ala Ala Thr Leu Val Gln Leu Ala Ile Ser Arg
    210                 215                 220

Gln Arg Glu Phe Leu Ala Asp Ala Ser Ser Val Glu Leu Thr Arg Asn
225                 230                 235                 240

Pro Gln Gly Met Ile Asn Ala Leu Asp Lys Leu Asp Asn Ser Lys Pro
                245                 250                 255

Met Ser Arg His Val Asp Asp Ala Ser Ser Ala Leu Tyr Ile Asn Asp
            260                 265                 270

Pro Lys Lys Gly Gly Gly Phe Gln Lys Leu Phe Tyr Thr His Pro Pro
        275                 280                 285

Ile Ser Glu Arg Ile Glu Arg Leu Lys Gln Met
    290                 295

<210> 107
<211> 180
<212> PRT
<213> Streptococcus pneumoniae

<400> 107
Met Lys Leu Asn Ile Gln Glu Ile Arg Lys Gln Ser Glu Gly Leu Asn
1               5                   10                  15

Phe Glu Gln Thr Leu Asp Leu Val Asp Asp Leu Arg Ala Arg Asn Gln

                20                  25                  30

Glu Ile Leu Asp Val Lys Asp Ile Leu Ala Val Gly Lys Val Gln Tyr
            35                  40                  45

Glu Asp Arg Met Tyr Phe Leu Asp Tyr Gln Leu Ser Tyr Thr Ile Val
    50                  55                  60

Leu Ala Ser Ser Arg Ser Met Glu Pro Val Glu Leu Val Glu Ser Tyr
65                  70                  75                  80

Pro Val Thr Glu Val Phe Met Glu Gly Ala Thr Asn Gln Leu Asp Gln
                85                  90                  95

Glu Val Leu Asp Asp Asp Leu Val Leu Pro Ile Glu Asn Gly Glu Leu
            100                 105                 110

Asp Leu Ala Glu Ser Val Ser Asp Asn Ile Leu Leu Asn Ile Pro Ile
        115                 120                 125

Lys Val Leu Thr Ala Glu Glu Glu Ala Gly Gln Gly Phe Ile Ser Gly
    130                 135                 140

Asn Asp Trp Gln Ile Met Thr Glu Glu Glu Tyr Gln Ala Gln Lys Ala
145                 150                 155                 160

Val Lys Lys Glu Glu Asn Ser Pro Phe Ala Gly Leu Gln Gly Leu Phe
                165                 170                 175

Asp Gly Asp Glu
            180

<210> 108
<211> 177
<212> PRT
<213> Streptococcus pneumoniae

<400> 108
Met Lys Arg Gln Leu Ala Leu Val Val Phe Ser Gly Gly Gln Asp Ser
1               5                   10                  15

Thr Thr Cys Leu Phe Trp Val Met Gln His Tyr Glu Thr Val Glu Ala
            20                  25                  30

Val Thr Phe Ala Tyr Gly Gln Arg His His Leu Glu Ile Gln Ile Thr
        35                  40                  45

Arg Glu Ile Ala Lys Glu Gln Gly Ile Arg His His Ile Leu Asp Met
    50                  55                  60

Ser Leu Leu Gly Gln Ile Thr Ala Gln Pro Asp Phe Ala Thr Ile His
65                  70                  75                  80

Ile Ser Tyr Ile Pro Asp Lys Leu Cys Val Glu Ser Lys Ser Leu Lys
                85                  90                  95

Leu Tyr Leu Phe Ser Tyr Arg Asn His Gly Asp Phe His Glu Asn Cys
            100                 105                 110

Ile Asn Thr Ile Gly Lys Asp Leu Val Asn Leu Leu Asp Pro Arg Tyr
        115                 120                 125

Leu Glu Val Trp Gly Lys Phe Thr Pro Arg Gly Gly Ile Ser Ile Asp
    130                 135                 140

Pro Tyr Tyr Asn Tyr Gly Lys Gln Gly Thr Lys Tyr Glu Gly Leu Ala
145                 150                 155                 160

Glu Gln Arg Leu Phe Gln His Asp Leu Tyr Pro Glu Lys Ile Asp Asn
                165                 170                 175

Arg

<210> 109
<211> 1236
<212> PRT
<213> Streptococcus pneumoniae

<400> 109
Met Thr Glu Thr Val Glu Asp Lys Val Ser His Ser Ile Thr Gly Leu
1               5                   10                  15

Asp Ile Leu Lys Gly Ile Val Ala Ala Gly Ala Val Ile Ser Gly Thr
            20                  25                  30

Val Ala Thr Gln Thr Lys Val Phe Thr Asn Glu Ser Ala Val Leu Glu
        35                  40                  45

Lys Thr Val Glu Lys Thr Asp Ala Leu Ala Thr Asn Asp Thr Val Val
    50                  55                  60

Leu Gly Thr Ile Ser Thr Ser Asn Ser Ala Ser Ser Thr Ser Leu Ser
65                  70                  75                  80

Ala Ser Glu Ser Ala Ser Thr Ser Ala Ser Glu Ser Ala Ser Thr Ser
                85                  90                  95

Ala Ser Thr Ser Ala Ser Thr Ser Ala Ser Glu Ser Ala Ser Thr Ser
            100                 105                 110

Ala Ser Thr Ser Ile Ser Ala Ser Ser Thr Val Val Gly Ser Gln Thr
        115                 120                 125

Ala Ala Ala Thr Glu Ala Thr Ala Lys Lys Val Glu Glu Asp Arg Lys
    130                 135                 140

Lys Pro Ala Ser Asp Tyr Val Ala Ser Val Thr Asn Val Asn Leu Gln
145                 150                 155                 160

Ser Tyr Ala Lys Arg Arg Lys Arg Ser Val Asp Ser Ile Glu Gln Leu
                165                 170                 175

Leu Ala Ser Ile Lys Asn Ala Ala Val Phe Ser Gly Asn Thr Ile Val
            180                 185                 190

Asn Gly Ala Pro Ala Ile Asn Ala Ser Leu Asn Ile Ala Lys Ser Glu
        195                 200                 205

Thr Lys Val Tyr Thr Gly Glu Gly Val Asp Ser Val Tyr Arg Val Pro
    210                 215                 220

Ile Tyr Tyr Lys Leu Lys Val Thr Asn Asp Gly Ser Lys Leu Thr Phe
225                 230                 235                 240

Thr Tyr Thr Val Thr Tyr Val Asn Pro Lys Thr Asn Asp Leu Gly Asn
                245                 250                 255

Ile Ser Ser Met Arg Pro Gly Tyr Ser Ile Tyr Asn Ser Gly Thr Ser
            260                 265                 270

Thr Gln Thr Met Leu Thr Leu Gly Ser Asp Leu Gly Lys Pro Ser Gly
        275                 280                 285

Val Lys Asn Tyr Ile Thr Asp Lys Asn Gly Arg Gln Val Leu Ser Tyr
    290                 295                 300

Asn Thr Ser Thr Met Thr Thr Gln Gly Ser Gly Tyr Thr Trp Gly Asn
305                 310                 315                 320

Gly Ala Gln Met Asn Gly Phe Phe Ala Lys Lys Gly Tyr Gly Leu Thr
                325                 330                 335

Ser Ser Trp Thr Val Pro Ile Thr Gly Thr Asp Thr Ser Phe Thr Phe
            340                 345                 350

Thr Pro Tyr Ala Ala Arg Thr Asp Arg Ile Gly Ile Asn Tyr Phe Asn
        355                 360                 365

Gly Gly Gly Lys Val Val Glu Ser Ser Thr Thr Ser Gln Ser Leu Ser
    370                 375                 380

Gln Ser Lys Ser Leu Ser Val Ser Ala Ser Gln Ser Ala Ser Ala Ser
385                 390                 395                 400

Ala Ser Thr Ser Ala Ser Ala Ser Ala Ser Thr Ser Ala Ser Ala Ser
                405                 410                 415

Ala Ser Thr Ser Ala Ser Ala Ser Ala Ser Thr Ser Ala Ser Val Ser
            420                 425                 430

Ala Ser Thr Ser Ala Ser Ala Ser Ala Ser Thr Ser Ala Ser Ala Ser
        435                 440                 445

Ala Ser Thr Ser Ala Ser Glu Ser Ala Ser Thr Ser Ala Ser Ala Ser
    450                 455                 460

Ala Ser Thr Ser Ala Ser Ala Ser Ala Ser Thr Ser Ala Ser Ala Ser
465                 470                 475                 480

Ala Ser Thr Ser Ala Ser Glu Ser Ala Ser Thr Ser Ala Ser Ala Ser
                485                 490                 495

Ala Ser Thr Ser Ala Ser Glu Ser Ala Ser Thr Ser Ala Ser Ala Ser
            500                 505                 510

Ala Ser Thr Ser Ala Ser Ala Ser Ala Ser Thr Ser Ala Ser Gly Ser
        515                 520                 525

Ala Ser Thr Ser Thr Ser Ala Ser Ala Ser Thr Ser Ala Ser Ala Ser
    530                 535                 540

Ala Ser Thr Ser Ala Ser Ala Ser Ala Ser Ile Ser Ala Ser Glu Ser
545                 550                 555                 560

Ala Ser Thr Ser Ala Ser Glu Ser Ala Ser Thr Ser Thr Ser Ala Ser
                565                 570                 575

Ala Ser Thr Ser Ala Ser Glu Ser Ala Ser Thr Ser Ala Ser Ala Ser
            580                 585                 590

Ala Ser Thr Ser Ala Ser Ala Ser Ala Ser Thr Ser Ala Ser Ala Ser
        595                 600                 605

Ala Ser Thr Ser Ala Ser Ala Ser Thr Ser Ala Ser Glu Ser Ala Ser
    610                 615                 620

Thr Ser Ala Ser Ala Ser Ala Ser Thr Ser Ala Ser Ala Ser Ala Ser
625                 630                 635                 640

Thr Ser Ala Ser Ala Ser Ala Ser Thr Ser Ala Ser Ala Ser Ala Ser
                645                 650                 655

Thr Ser Ala Ser Val Ser Ala Ser Thr Ser Ala Ser Ala Ser Ala Ser
            660                 665                 670

Thr Ser Ala Ser Ala Ser Ala Ser Thr Ser Ala Ser Glu Ser Ala Ser
        675                 680                 685

Thr Ser Ala Ser Ala Ser Ala Ser Thr Ser Ala Ser Ala Ser Ala Ser
    690                 695                 700

Thr Ser Ala Ser Ala Ser Ala Ser Thr Ser Ala Ser Glu Ser Ala Ser
705                 710                 715                 720

Thr Ser Ala Ser Ala Ser Ala Ser Thr Ser Ala Ser Glu Ser Ala Ser
                725                 730                 735

Thr Ser Ala Ser Ala Ser Ala Ser Thr Ser Ala Ser Ala Ser Ala Ser
            740                 745                 750

Thr Ser Ala Ser Gly Ser Ala Ser Thr Ser Thr Ser Ala Ser Ala Ser
        755                 760                 765

Thr Ser Ala Ser Ala Ser Ala Ser Thr Ser Ala Ser Ala Ser Ala Ser
    770                 775                 780

Ile Ser Ala Ser Glu Ser Ala Ser Thr Ser Ala Ser Glu Ser Ala Ser
785                 790                 795                 800

Thr Ser Thr Ser Ala Ser Ala Ser Thr Ser Ala Ser Glu Ser Ala Ser
                805                 810                 815

Thr Ser Ala Ser Ala Ser Ala Ser Thr Ser Ala Ser Ala Ser Ala Ser
            820                 825                 830

Thr Ser Ala Ser Ala Ser Ala Arg Gln Val Arg Arg Pro Gln Pro Val
        835                 840                 845

His Leu Asn Arg His Gln Pro Val Arg Gln Pro Gln Gln Val Leu Val

850                 855                 860

His Gln Leu Gln His Gln Arg Val His Arg Leu Gln His Gln Pro Val
865                 870                 875                 880

Pro Arg Leu Gln Arg Gln Pro Val Arg Gln Leu Gln Gln Val Pro Val
                885                 890                 895

Leu Gln Ser Gln His Gln Gln Val Leu Gln Pro Gln His Arg Gln Val
            900                 905                 910

Pro Arg Leu Gln Gln Ala His Gln His Leu Asn Gln Arg Arg Gln Ala
        915                 920                 925

Pro Gln Leu Gln Gln Val Pro Val Arg Gln Pro Gln Arg Arg Gln Val
    930                 935                 940

Arg Gln Pro Gln Gln Val Leu Val His Gln Leu Gln His Gln Arg Val
945                 950                 955                 960

His Arg Leu Arg Arg Gln Pro Val His Gln Ser Gln Gln Val Pro Val
                965                 970                 975

Arg Gln Leu Pro His Gln Gln Val Pro Arg Leu Gln Gln Ala Pro Val
            980                 985                 990

Arg Arg Leu Gln Gln Val Leu Ala Pro Gln Pro Gln Pro Gln Pro Val
        995                 1000                1005

Arg Gln Pro Gln Gln Val Ser Gln Arg Leu Asn Arg His Gln Arg Val
    1010                1015                1020

Arg Pro Leu Gln Gln Val Leu Ala Pro Gln Pro Gln Arg Gln Gln Val
1025                1030                1035                1040

His Arg Leu Gln Arg Gln Arg Val Arg Leu Asn Arg His Gln Arg Val
                1045                1050                1055

Arg Pro Leu Gln Gln Val Leu Ala Pro Gln Pro Gln Arg Gln Gln Val
            1060                1065                1070

His Arg Leu Gln His Gln Arg Val Arg Pro Leu Gln Gln Val Leu Ala
        1075                1080                1085

Pro Gln Pro Gln Arg Gln Gln Val His Arg Leu Gln Arg Gln Arg Val
    1090                1095                1100

Arg Leu Ser Gln His Gln Arg Val Arg Gln Pro Gln Gln Ala His Gln
1105                1110                1115                1120

Leu Leu Asn Leu His Gln Pro Val Arg Gln Pro Gln His Arg Gln Ala
                1125                1130                1135

Pro Gln Leu Gln Gln Val Pro Val Arg Gln Pro Gln Arg Arg Gln Val
            1140                1145                1150

Arg Arg Leu Gln Gln Val Pro Val Arg Gln Pro Gln Gln Val Pro Val
        1155                1160                1165

Arg Gln Pro Gln Arg Arg Gln Val Arg Arg Pro Gln Pro Val His Leu
    1170                1175                1180

Asn Arg His Gln Pro Val Arg Gln Pro Gln Gln Val Leu Val His Gln
1185                1190                1195                1200

Leu Gln His Gln Arg Val His Arg Leu Gln His Gln Pro Val His Gln
                1205                1210                1215

Ser Gln Gln Val Pro Val Arg Gln Phe Arg Ile Asn Lys Cys Leu Gly
            1220                1225                1230

Phe Ser Lys Tyr
        1235

<210> 110
<211> 449
<212> PRT
<213> Streptococcus pneumoniae

<400> 110
Met Gly Val Glu Thr Trp Phe Tyr Ser Ser Ile Cys Trp Leu Ala Ile
1               5                   10                  15

Gly Leu Gly Ser Val Trp Lys Phe Pro Tyr Met Thr Ala Ala Asn Gly
            20                  25                  30

Gly Gly Gly Phe Leu Leu Ile Phe Leu Ile Ser Thr Ile Leu Ile Gly
        35                  40                  45

Phe Pro Leu Leu Leu Ala Glu Phe Ala Leu Gly Arg Ser Ala Gly Val
    50                  55                  60

Ser Ala Ile Lys Thr Phe Gly Lys Leu Gly Lys Asn Asn Lys Tyr Asn
65                  70                  75                  80

Phe Ile Gly Trp Ile Gly Ala Phe Ala Leu Phe Ile Leu Leu Ser Phe
                85                  90                  95

Tyr Ser Val Ile Gly Gly Trp Ile Leu Val Tyr Leu Gly Ile Glu Phe
            100                 105                 110

Gly Lys Leu Phe Gln Leu Gly Gly Thr Gly Asp Tyr Ala Gln Leu Phe
        115                 120                 125

Thr Ser Ile Ile Ser Asn Pro Ala Ile Ala Leu Gly Ala Gln Ala Ala
    130                 135                 140

Phe Ile Leu Leu Asn Ile Phe Ile Val Ser Arg Gly Val Gln Lys Gly
145                 150                 155                 160

Ile Glu Arg Ala Ser Lys Val Met Met Pro Leu Leu Phe Ile Val Phe
                165                 170                 175

Val Phe Ile Ile Gly Arg Ser Leu Ser Leu Pro Asn Ala Met Glu Gly
            180                 185                 190

Val Leu Tyr Phe Leu Lys Pro Asp Phe Ser Lys Leu Thr Ser Thr Gly
        195                 200                 205

Leu Leu Tyr Ala Leu Gly Gln Ser Phe Phe Ala Leu Ser Leu Gly Val

210                 215                 220

Thr Val Met Leu Thr Tyr Ala Ser Tyr Leu Asp Lys Lys Thr Asn Leu
225                 230                 235                 240

Val Gln Ser Gly Ile Ser Ile Val Ala Met Asn Ile Ser Ile Ser Ile
                245                 250                 255

Met Ala Gly Leu Ala Ile Phe Gln Ala Arg Ser Pro Phe Asn Ile Gln
            260                 265                 270

Ser Glu Gly Gly Pro Ser Leu Leu Phe Ile Val Leu Pro Gln Leu Phe
        275                 280                 285

Asp Lys Met Pro Phe Gly Thr Ile Phe Tyr Val Leu Phe Leu Leu Leu
    290                 295                 300

Phe Leu Phe Ala Thr Val Thr Phe Ser Val Val Met Leu Glu Ile Asn
305                 310                 315                 320

Val Asp Asn Ile Thr Asn Gln Asp Asn Ser Lys Arg Ala Lys Trp Ser
                325                 330                 335

Val Ile Leu Gly Ile Leu Thr Phe Val Phe Gly Ile Pro Ser Ala Leu
            340                 345                 350

Ser Tyr Gly Val Met Ala Asp Val His Ile Phe Gly Lys Thr Phe Phe
        355                 360                 365

Asp Ala Met Asp Phe Leu Val Ser Asn Leu Leu Met Pro Phe Gly Ala
    370                 375                 380

Leu Tyr Leu Ser Leu Phe Thr Gly Tyr Ile Phe Lys Lys Ala Leu Ala
385                 390                 395                 400

Met Glu Glu Leu His Leu Asp Glu Arg Ala Trp Lys Gln Gly Leu Phe
                405                 410                 415

Gln Val Trp Leu Phe Leu Leu Arg Phe Phe Val Ser Ser Phe Gln Ser
            420                 425                 430

Ser Ser Leu Trp Ser Ser Leu Pro Asn Leu Cys Asn Gln Lys Gly Leu
        435                 440                 445

Glu

<210> 111
<211> 186
<212> PRT
<213> Streptococcus pneumoniae

<400> 111
Met Leu Lys Lys Trp Gln Leu Lys Asp Val Ile Leu Leu Ala Phe Leu
1               5                   10                  15

Ser Ile Phe Phe Gly Gly Val Phe Val Gly Ser Gly Tyr Val Tyr Asn
            20                  25                  30

Ile Leu Ser Leu Leu Leu Thr Pro Leu Gly Leu Gln Ala Phe Ala Asn
        35                  40                  45

Glu Ile Leu Phe Gly Leu Trp Cys Met Ala Ala Pro Ile Ala Ala Ile
    50                  55                  60

Phe Val Pro Arg Val Gly Ser Ala Thr Ile Gly Glu Val Leu Ala Ala
65                  70                  75                  80

Leu Ala Glu Val Leu Tyr Gly Ser Gln Phe Gly Leu Gly Ala Leu Leu
                85                  90                  95

Ser Gly Phe Val Gln Gly Leu Gly Ser Glu Phe Gly Phe Ile Val Thr
            100                 105                 110

Lys Asn Arg Tyr Glu Ser Trp Leu Ser Leu Thr Ala Asn Ser Ile Gly
        115                 120                 125

Ile Thr Leu Val Ser Phe Val Tyr Glu Tyr Ile Lys Leu Gly Tyr Tyr
    130                 135                 140

Ala Phe Ser Leu Pro Phe Val Leu Ser Leu Leu Val Val Arg Phe Ile
145                 150                 155                 160

Ser Val Tyr Phe Phe Cys Thr Ile Leu Val Arg Ala Ile Val Lys Leu
                165                 170                 175

Tyr His Gln Phe Ala Thr Gly Gly Lys Ala
            180                 185

<210> 112
<211> 216
<212> PRT
<213> Streptococcus pneumoniae

<400> 112
Met Val Lys Val Ala Thr Gln Thr Pro Ile Ile Ser Leu Phe Leu Leu
1               5                   10                  15

Ile Leu Ser Leu Glu Thr Ser Phe Ile Pro Ser Ile Ala Leu Thr Leu
            20                  25                  30

Ser Val Val Ala Phe Cys Ile Leu Phe Met Leu Tyr Tyr Arg Arg Phe
        35                  40                  45

Lys Met Leu Ala Trp Met Ile Ile Leu Ala Ile Leu Pro Ser Phe Ala
    50                  55                  60

Asn Tyr Trp Ala Val Gln Leu His Gly Asp Ala Ser Gln Ala Val Met
65                  70                  75                  80

Leu Gly Thr Arg Ala Phe Val Thr Val Cys Ile Gly Leu Val Phe Val
                85                  90                  95

Ser Ser Val Ser Leu Lys Glu Leu Leu Leu Tyr Leu Ala Gln Lys Gly
            100                 105                 110

Leu Ser Arg Ser Trp Ser Tyr Ala Leu Ile Val Val Phe Asn Ser Phe
        115                 120                 125

Pro Leu Ile Gln Gln Glu Ile Lys Ser Leu Lys Glu Ala Cys Leu Leu
    130                 135                 140

Arg Gly Gln Glu Leu His Phe Trp Ser Pro Leu Ile Tyr Ser Lys Val
145                 150                 155                 160

Leu Met Thr Val Phe Arg Trp Arg His Leu Tyr Leu Arg Ala Leu Ser
                165                 170                 175

Ala His Gly Tyr Asp Glu His Ala Gln Leu Lys Asn Ser Tyr Arg Thr
            180                 185                 190

Phe Tyr Ile Pro Lys Lys Thr Lys Leu Ile Tyr Leu Leu Phe Phe Leu
        195                 200                 205

Leu Leu Gln Thr Ser Leu Phe Leu
    210                 215

<210> 113
<211> 174
<212> PRT
<213> Streptococcus pneumoniae

<400> 113
Met Arg Lys His Gln Leu Gln Val His Lys Leu Thr Ile Leu Ser Met
  1               5                  10                  15

Met Ile Ala Leu Asp Val Val Leu Thr Pro Ile Phe Arg Ile Glu Gly
             20                  25                  30

Met Ala Pro Met Ser Ser Val Val Asn Ile Leu Ala Gly Ile Met Met
         35                  40                  45

Gly Pro Val Tyr Ala Leu Ala Met Ala Thr Val Thr Ala Phe Ile Arg
     50                  55                  60

Met Thr Thr Gln Gly Ile Pro Pro Leu Ala Leu Thr Gly Ala Thr Phe
 65                  70                  75                  80

Gly Ala Leu Leu Ala Gly Leu Phe Tyr Lys Tyr Gly Arg Lys Phe His
                 85                  90                  95

Tyr Ser Ala Leu Gly Glu Ile Leu Gly Thr Gly Ile Ile Gly Ser Ile
            100                 105                 110

Val Ser Tyr Pro Val Met Val Leu Phe Thr Gly Ser Ala Ala Lys Leu
        115                 120                 125

Ser Trp Phe Ile Tyr Thr Pro Arg Phe Phe Gly Ala Thr Leu Ile Gly
    130                 135                 140

Thr Ala Ile Ser Phe Ile Ala Phe Arg Phe Leu Ile Lys Gln Glu Phe
145                 150                 155                 160

Phe Lys Lys Val Gln Gly Tyr Phe Phe Ser Glu Arg Ile Asp
                165                 170

<210> 114
<211> 267
<212> PRT
<213> Streptococcus pneumoniae

<400> 114
Met Gln Glu Phe Thr Asn Pro Phe Pro Ile Gly Ser Ser Ser Leu Ile
1               5                   10                  15

His Cys Ile Thr Asn Glu Ile Ser Cys Glu Met Leu Ala Asn Gly Ile
            20                  25                  30

Leu Ala Leu Gly Cys Lys Pro Val Met Ala Asp Asp Ser Arg Glu Val
        35                  40                  45

Leu Asp Phe Thr Lys Gln Ser Gln Ala Leu Phe Ile Asn Leu Gly His
    50                  55                  60

Leu Ser Ala Glu Lys Glu Lys Ala Ile Arg Met Ala Ala Ser Tyr Ala
65                  70                  75                  80

Asn Gln Ser Ser Leu Pro Met Val Val Asp Ala Val Gly Val Thr Thr
                85                  90                  95

Ser Ser Ile Arg Lys Ser Leu Val Lys Asp Leu Leu Asp Tyr Arg Pro
            100                 105                 110

Thr Val Leu Lys Gly Asn Met Ser Glu Ile Arg Ser Leu Val Gly Leu
        115                 120                 125

Lys His His Gly Val Gly Val Asp Ala Ser Ala Lys Asp Gln Glu Thr
    130                 135                 140

Glu Asp Leu Leu Gln Val Leu Lys Asp Trp Cys Gln Thr Tyr Pro Gly
145                 150                 155                 160

Met Ser Phe Leu Val Thr Gly Pro Lys Asp Leu Val Val Ser Lys Asn
                165                 170                 175

Gln Val Ala Val Leu Gly Asn Gly Cys Thr Glu Leu Asp Trp Ile Thr
            180                 185                 190

Gly Thr Gly Asp Leu Val Gly Ala Leu Thr Ala Val Phe Leu Ser Gln
        195                 200                 205

Gly Lys Thr Gly Phe Glu Ala Ser Cys Leu Ala Val Ser Tyr Leu Asn
    210                 215                 220

Ile Ala Ala Glu Lys Ile Val Val Gln Gly Met Gly Leu Glu Glu Phe
225                 230                 235                 240

Arg Tyr Gln Val Leu Asn Gln Leu Ser Leu Leu Arg Arg Asp Glu Asn
                245                 250                 255

Trp Leu Asp Thr Ile Lys Gly Glu Val Tyr Glu
            260                 265

<210> 115

<211> 277
<212> PRT
<213> Streptococcus pneumoniae

<400> 115
Met Asn His Lys Ile Ala Ile Leu Ser Asp Val His Gly Asn Ala Thr
1               5                   10                  15

Ala Leu Glu Ala Val Ile Ala Asp Ala Lys Asn Gln Gly Ala Ser Glu
            20                  25                  30

Tyr Trp Leu Leu Gly Asp Ile Phe Leu Pro Gly Pro Gly Ala Asn Asp
        35                  40                  45

Leu Val Ala Leu Leu Lys Asp Leu Pro Ile Thr Ala Ser Val Arg Gly
    50                  55                  60

Asn Trp Asp Asp Arg Val Leu Glu Ala Leu Asp Gly Gln Tyr Gly Leu
65                  70                  75                  80

Glu Asp Pro Gln Glu Val Gln Leu Leu Arg Met Thr Gln Tyr Leu Met
                85                  90                  95

Glu Arg Met Asp Pro Ala Thr Ile Val Trp Leu Arg Ser Leu Pro Leu
            100                 105                 110

Leu Glu Lys Lys Glu Ile Asp Gly Leu Arg Phe Ser Ile Ser His Asn
        115                 120                 125

Leu Pro Asp Lys Asn Tyr Gly Gly Asp Leu Leu Val Glu Asn Asp Thr
    130                 135                 140

Glu Lys Phe Asp Gln Leu Leu Asp Ala Glu Thr Asp Val Ala Val Tyr
145                 150                 155                 160

Gly His Val His Lys Gln Leu Leu Arg Tyr Gly Ser Gln Gly Gln Gln
                165                 170                 175

Ile Ile Asn Pro Gly Ser Ile Gly Met Pro Tyr Phe Asn Trp Glu Ala
            180                 185                 190

Leu Lys Asn His Arg Ser Gln Tyr Ala Val Ile Glu Val Glu Asp Gly
        195                 200                 205

Glu Leu Leu Asn Ile Gln Phe Arg Lys Val Ala Tyr Asp Tyr Glu Ala
    210                 215                 220

Glu Leu Glu Leu Ala Lys Ser Lys Gly Leu Pro Phe Ile Glu Met Tyr
225                 230                 235                 240

Glu Glu Leu Arg Arg Asp Asp Asn Tyr Gln Gly His Asn Leu Glu Leu
                245                 250                 255

Leu Ala Ser Leu Ile Glu Lys His Gly Tyr Val Glu Asp Val Lys Asn
            260                 265                 270

Phe Phe Asp Phe Leu
        275

<210> 116
<211> 241
<212> PRT
<213> Streptococcus pneumoniae

<400> 116
Met Asn Val Asn Gln Ile Val Arg Ile Ile Pro Thr Leu Lys Ala Asn
1               5                   10                  15

Asn Arg Lys Leu Asn Glu Thr Phe Tyr Ile Glu Thr Leu Gly Met Lys
            20                  25                  30

Ala Leu Leu Glu Glu Ser Ala Phe Leu Ser Leu Gly Asp Gln Thr Gly
        35                  40                  45

Leu Glu Lys Leu Val Leu Glu Glu Ala Pro Ser Met Arg Thr Arg Lys
    50                  55                  60

Val Glu Gly Arg Lys Lys Leu Ala Arg Leu Ile Val Lys Val Glu Asn
65                  70                  75                  80

Pro Leu Glu Ile Glu Gly Ile Leu Ser Lys Thr Asp Ser Ile His Arg
                85                  90                  95

Leu Tyr Lys Gly Gln Asn Gly Tyr Ala Phe Glu Ile Phe Ser Pro Glu
            100                 105                 110

Asp Asp Leu Ile Leu Ile His Ala Glu Asp Asp Ile Ala Ser Leu Val
        115                 120                 125

Glu Val Gly Glu Lys Pro Glu Phe Gln Thr Asp Leu Ala Ser Ile Ser
    130                 135                 140

Leu Ser Lys Phe Glu Ile Ser Met Glu Leu His Leu Pro Thr Asp Ile
145                 150                 155                 160

Glu Ser Phe Leu Glu Ser Ser Glu Ile Gly Ala Ser Leu Asp Phe Ile
                165                 170                 175

Pro Ala Gln Gly Gln Asp Leu Thr Val Asp Asn Thr Val Thr Trp Asp
            180                 185                 190

Leu Ser Met Leu Lys Phe Leu Val Asn Glu Leu Asp Ile Ala Ser Leu
        195                 200                 205

Arg Gln Lys Phe Glu Ser Thr Glu Tyr Phe Ile Pro Lys Ser Glu Lys
    210                 215                 220

Phe Phe Leu Gly Lys Asp Arg Asn Asn Val Glu Leu Trp Phe Glu Glu
225                 230                 235                 240

Val

<210> 117
<211> 311
<212> PRT
<213> Streptococcus pneumoniae

<400> 117
Met Lys Trp Thr Lys Ile Ile Lys Lys Ile Glu Glu Gln Ile Glu Ala
1               5                   10                  15

Gly Ile Tyr Pro Gly Ala Ser Phe Ala Tyr Phe Lys Asp Asn Gln Trp
            20                  25                  30

Thr Glu Phe Tyr Leu Gly Gln Ser Asp Pro Glu His Gly Leu Gln Thr
        35                  40                  45

Glu Ala Gly Leu Val Tyr Asp Leu Ala Ser Val Ser Lys Val Val Gly
    50                  55                  60

Val Gly Thr Val Cys Thr Phe Leu Trp Glu Ile Gly Gln Leu Asp Ile
65                  70                  75                  80

Asp Arg Leu Val Ile Asp Phe Leu Pro Glu Ser Asp Tyr Pro Asp Ile
                85                  90                  95

Thr Ile Arg Gln Leu Leu Thr His Ala Thr Asp Leu Asp Pro Phe Ile
            100                 105                 110

Pro Asn Arg Asp Leu Leu Thr Ala Pro Glu Leu Lys Glu Ala Met Phe
        115                 120                 125

His Leu Asn Arg Arg Ser Gln Pro Ala Phe Leu Tyr Ser Asp Val His
    130                 135                 140

Phe Leu Leu Leu Gly Phe Ile Leu Glu Arg Ile Phe Asn Gln Asp Leu
145                 150                 155                 160

Asp Val Ile Leu Lys Asp Gln Val Trp Lys Pro Trp Gly Met Thr Glu
                165                 170                 175

Thr Lys Phe Gly Pro Val Glu Leu Ala Val Pro Thr Val Arg Gly Val
            180                 185                 190

Glu Ala Gly Ile Val His Asp Pro Lys Ala Arg Leu Leu Gly Arg His
        195                 200                 205

Ala Gly Ser Ala Gly Leu Phe Ser Thr Ile Lys Asp Leu Gln Ile Phe
    210                 215                 220

Leu Glu His Tyr Leu Ala Asp Asp Phe Ala Arg Asp Leu Asn Gln Asn
225                 230                 235                 240

Phe Ser Pro Leu Asp Asp Lys Glu Arg Ser Leu Ala Trp Asn Leu Glu
                245                 250                 255

Gly Asp Trp Leu Asp His Thr Gly Tyr Thr Gly Thr Phe Ile Met Trp
            260                 265                 270

Asn Arg Gln Lys Gln Glu Ala Thr Ile Phe Leu Ser Asn Arg Thr Tyr
        275                 280                 285

Glu Lys Asp Glu Arg Ala Gln Trp Ile Leu Asp Arg Asn Gln Val Met
    290                 295                 300

Asn Leu Ile Arg Lys Glu Glu
305                 310

<210> 118
<211> 160
<212> PRT
<213> Streptococcus pneumoniae

<400> 118
Met Met Lys Lys Thr Tyr Asn His Ile Leu Val Trp Gly Val Ile Phe
1               5                   10                  15

Tyr Ser Ile Cys Ile Val Cys Phe Cys Phe Thr Pro Gln Glu Gln Ser
            20                  25                  30

Thr Val Gly Val Gly Thr Pro Gly Ile Gln His Leu Gly Arg Leu Val
        35                  40                  45

Phe Leu Leu Thr Pro Phe Asn Ser Leu Trp Lys Leu Gly Glu Val Ser
    50                  55                  60

Asp Ile Gly Gln Leu Cys Trp Ile Phe Leu Gln Asn Ile Leu Asn Val
65                  70                  75                  80

Phe Leu Phe Phe Pro Leu Ile Phe Gln Leu Leu Tyr Leu Phe Pro Asn
                85                  90                  95

Leu Arg Lys Thr Lys Lys Val Leu Leu Phe Ser Phe Leu Val Ser Leu
            100                 105                 110

Gly Ile Glu Cys Thr Gln Leu Ile Leu Asp Phe Phe Phe Asp Phe Asn
        115                 120                 125

Arg Val Phe Glu Ile Asp Asp Leu Trp Thr Asn Thr Leu Gly Gly Tyr
    130                 135                 140

Leu Ala Trp Leu Leu Tyr Lys Arg Leu His Lys Asn Lys Val Arg Asn
145                 150                 155                 160

<210> 119
<211> 263
<212> PRT
<213> Streptococcus pneumoniae

<400> 119
Met Lys Ile Pro Leu Leu Thr Phe Ala Arg His Lys Phe Val Tyr Val
1               5                   10                  15

Leu Leu Thr Leu Leu Phe Leu Ala Leu Val Tyr Arg Asp Val Leu Met
            20                  25                  30

Thr Tyr Phe Phe Phe Asp Ile His Ala Pro Asp Leu Ala Lys Phe Asp
        35                  40                  45

Gly Gln Ala Ile Lys Asn Asp Leu Leu Lys Ser Ala Leu Asp Phe Arg
    50                  55                  60

Ile Leu Gln Phe Asn Leu Gly Phe Tyr Gln Ser Phe Ile Ile Pro Ile
65                  70                  75                  80

Ile Ile Val Leu Leu Gly Phe Gln Tyr Ile Glu Leu Lys Asn Lys Val
                85                  90                  95

Leu Arg Leu Ser Ile Gly Arg Glu Val Ser Tyr Gln Gly Leu Lys Arg
            100                 105                 110

Lys Leu Thr Leu Gln Val Ala Ser Ile Pro Cys Leu Ile Tyr Leu Val
        115                 120                 125

Thr Val Leu Ile Ile Ala Ile Ile Thr Tyr Phe Phe Gly Thr Phe Ser
    130                 135                 140

Pro Leu Gly Trp Asn Ser Leu Phe Ser Asp Gly Ser Gly Leu Gln Arg
145                 150                 155                 160

Leu Leu Asp Gly Glu Ile Lys Ser Tyr Leu Phe Phe Thr Cys Val Leu
                165                 170                 175

Leu Ile Gly Ile Phe Ile Asn Ala Ile Tyr Phe Leu Gln Ile Val Asp
            180                 185                 190

Tyr Val Gly Asn Val Thr Arg Ser Ala Ile Thr Tyr Leu Met Phe Leu
        195                 200                 205

Trp Leu Gly Ser Met Leu Leu Tyr Ser Ala Leu Pro Tyr Tyr Met Val
    210                 215                 220

Pro Met Thr Ser Leu Met Gln Ala Ser Tyr Gly Asp Val Ser Leu Met
225                 230                 235                 240

Lys Leu Phe Thr Pro Tyr Ile Leu Tyr Ile Val Pro Tyr Met Val Leu
                245                 250                 255

Glu Lys Tyr Glu Asp Asn Val
            260

<210> 120
<211> 505
<212> PRT
<213> Streptococcus pneumoniae

<400> 120
Met Phe Lys Val Leu Gln Lys Val Gly Lys Ala Phe Met Leu Pro Ile
1               5                   10                  15

Ala Ile Leu Pro Ala Ala Gly Leu Leu Leu Gly Ile Gly Gly Ala Leu
            20                  25                  30

Ser Asn Pro Thr Thr Ile Ala Thr Tyr Pro Ile Leu Asp Asn Ser Ile
        35                  40                  45

Phe Gln Ser Ile Phe Gln Val Met Ser Ser Ala Gly Glu Val Val Phe
    50                  55                  60

Ser Asn Leu Ser Leu Leu Leu Cys Val Gly Leu Cys Ile Gly Leu Ala
65                  70                  75                  80

Lys Arg Asp Lys Gly Thr Ala Ala Leu Ala Gly Val Thr Gly Tyr Leu
                85                  90                  95

Val Met Thr Ala Thr Ile Lys Ala Leu Val Lys Leu Phe Met Ala Glu
            100                 105                 110

Gly Ser Ala Ile Asp Thr Gly Val Ile Gly Ala Leu Val Val Gly Ile
        115                 120                 125

Val Ala Val Tyr Leu His Asn Arg Tyr Asn Asn Ile Gln Leu Pro Ser
    130                 135                 140

Ala Leu Gly Phe Phe Gly Gly Ser Arg Phe Val Pro Ile Val Thr Ser
145                 150                 155                 160

Phe Ser Ser Ile Leu Ile Gly Phe Val Phe Phe Val Ile Trp Pro Pro
                165                 170                 175

Phe Gln Gln Leu Leu Val Ser Thr Gly Gly Tyr Ile Ser Gln Ala Gly
            180                 185                 190

Pro Ile Gly Thr Phe Leu Tyr Gly Phe Leu Met Arg Leu Ser Gly Ala
        195                 200                 205

Val Gly Leu His His Ile Ile Tyr Pro Met Phe Trp Tyr Thr Glu Leu
    210                 215                 220

Gly Gly Val Glu Thr Val Ala Gly Gln Thr Val Val Gly Ala Gln Lys
225                 230                 235                 240

Ile Phe Phe Ala Gln Leu Ala Asp Leu Ala His Ser Gly Leu Phe Thr
                245                 250                 255

Glu Gly Thr Arg Phe Phe Ala Gly Arg Phe Ser Thr Met Met Phe Gly
            260                 265                 270

Leu Pro Ala Ala Cys Leu Ala Met Tyr His Ser Val Pro Lys Asn Arg
        275                 280                 285

Arg Lys Lys Tyr Ala Gly Leu Phe Phe Gly Val Ala Leu Thr Ser Phe
    290                 295                 300

Ile Thr Gly Ile Thr Glu Pro Ile Glu Phe Met Phe Leu Phe Val Ser
305                 310                 315                 320

Pro Val Leu Tyr Val Val His Ala Phe Leu Asp Gly Val Ser Phe Phe
                325                 330                 335

Ile Ala Asp Val Leu Asn Ile Ser Ile Gly Asn Thr Phe Ser Gly Gly
            340                 345                 350

Val Ile Asp Phe Thr Leu Phe Gly Ile Leu Gln Gly Asn Ala Lys Thr
        355                 360                 365

Asn Trp Val Leu Gln Ile Pro Phe Gly Leu Ile Trp Ser Val Leu Tyr
    370                 375                 380

Tyr Ile Ile Phe Arg Trp Phe Ile Thr Gln Phe Asn Val Leu Thr Pro
385                 390                 395                 400

Gly Arg Gly Glu Glu Val Asp Ser Lys Glu Ile Ser Glu Ser Ala Asp
                405                 410                 415

Ser Thr Ser Asn Thr Ala Asp Tyr Leu Lys Gln Asp Ser Leu Gln Ile
            420                 425                 430

Ile Arg Ala Leu Gly Gly Ser Asn Asn Ile Glu Asp Val Asp Ala Cys
        435                 440                 445

Val Thr Arg Leu Arg Val Ala Val Lys Glu Val Asn Gln Val Asp Lys
    450                 455                 460

Ala Leu Leu Lys Gln Ile Gly Ala Val Asp Val Leu Glu Val Lys Gly
465                 470                 475                 480

Gly Ile Gln Ala Ile Tyr Gly Ala Lys Ala Ile Leu Tyr Lys Asn Ser
                485                 490                 495

Ile Asn Glu Ile Leu Gly Val Asp Asp
            500                 505

<210> 121
<211> 442
<212> PRT
<213> Streptococcus pneumoniae

<400> 121
Met Lys Phe Arg Lys Leu Ala Cys Thr Val Leu Ala Gly Ala Ala Val
1               5                   10                  15

Leu Gly Leu Ala Ala Cys Gly Asn Ser Gly Gly Ser Lys Asp Ala Ala
            20                  25                  30

Lys Ser Gly Gly Asp Gly Ala Lys Thr Glu Ile Thr Trp Trp Ala Phe
        35                  40                  45

Pro Val Phe Thr Gln Glu Lys Thr Gly Asp Gly Val Gly Thr Tyr Glu
    50                  55                  60

Lys Ser Ile Ile Glu Ala Phe Glu Lys Ala Asn Pro Asp Ile Lys Val
65                  70                  75                  80

Lys Leu Glu Thr Ile Asp Phe Lys Ser Gly Pro Glu Lys Ile Thr Thr
                85                  90                  95

Ala Ile Glu Ala Gly Thr Ala Pro Asp Val Leu Phe Asp Ala Pro Gly
            100                 105                 110

Arg Ile Ile Gln Tyr Gly Lys Asn Gly Lys Leu Ala Glu Leu Asn Asp
        115                 120                 125

Leu Phe Thr Asp Glu Phe Val Lys Asp Val Asn Asn Glu Asn Ile Val
    130                 135                 140

Gln Ala Ser Lys Ala Gly Asp Lys Ala Tyr Met Tyr Pro Ile Ser Ser
145                 150                 155                 160

Ala Pro Phe Tyr Met Ala Met Asn Lys Lys Met Leu Glu Asp Ala Gly
                165                 170                 175

Val Ala Asn Leu Val Lys Glu Gly Trp Thr Thr Asp Asp Phe Glu Lys
            180                 185                 190

Val Leu Lys Ala Leu Lys Asp Lys Gly Tyr Thr Pro Gly Ser Leu Phe
        195                 200                 205

Ser Ser Gly Gln Gly Gly Asp Gln Gly Thr Arg Ala Phe Ile Ser Asn
    210                 215                 220

Leu Tyr Ser Gly Ser Val Thr Asp Glu Lys Val Ser Lys Tyr Thr Thr
225                 230                 235                 240

Asp Asp Pro Lys Phe Val Lys Gly Leu Glu Lys Ala Thr Ser Trp Ile
                245                 250                 255

Lys Asp Asn Leu Ile Asn Asn Gly Ser Gln Phe Asp Gly Gly Ala Asp
            260                 265                 270

Ile Gln Asn Phe Ala Asn Gly Gln Thr Ser Tyr Thr Ile Leu Trp Ala
        275                 280                 285

Pro Ala Gln Asn Gly Ile Gln Ala Lys Leu Leu Glu Ala Ser Lys Val
    290                 295                 300

Glu Val Val Glu Val Pro Phe Pro Ser Asp Glu Gly Lys Pro Ala Leu
305                 310                 315                 320

Glu Tyr Leu Val Asn Gly Phe Ala Val Phe Asn Asn Lys Asp Asp Lys
                325                 330                 335

Lys Val Ala Ala Ser Lys Lys Phe Ile Gln Phe Ile Ala Asp Asp Lys
            340                 345                 350

Glu Trp Gly Pro Lys Asp Val Val Arg Thr Gly Ala Phe Pro Val Arg
        355                 360                 365

Thr Ser Phe Gly Lys Leu Tyr Glu Asp Lys Arg Met Glu Thr Ile Ser
    370                 375                 380

Gly Trp Thr Gln Tyr Tyr Ser Pro Tyr Tyr Asn Thr Ile Asp Gly Phe
385                 390                 395                 400

Ala Glu Met Arg Thr Leu Trp Phe Pro Met Leu Gln Ser Val Ser Asn
                405                 410                 415

Gly Asp Glu Lys Pro Ala Asp Ala Leu Lys Ala Phe Thr Glu Lys Ala
            420                 425                 430

Asn Glu Thr Ile Lys Lys Ala Met Lys Gln
        435                 440

<210> 122
<211> 279
<212> PRT
<213> Streptococcus pneumoniae

<400> 122
Met Gln Ser Thr Glu Lys Lys Pro Leu Thr Ala Phe Thr Val Ile Ser

1                   5                   10                  15

Thr Ile Ile Leu Leu Leu Leu Thr Val Leu Phe Ile Phe Pro Phe Tyr
                20                  25                  30

Trp Ile Leu Thr Gly Ala Phe Lys Ser Gln Pro Asp Thr Ile Val Ile
            35                  40                  45

Pro Pro Gln Trp Phe Pro Lys Met Pro Thr Met Glu Asn Phe Gln Gln
    50                  55                  60

Leu Met Val Gln Asn Pro Ala Leu Gln Trp Met Trp Asn Ser Val Phe
65                  70                  75                  80

Ile Ser Leu Val Thr Met Phe Leu Val Cys Ala Thr Ser Ser Leu Ala
                85                  90                  95

Gly Tyr Val Leu Ala Lys Lys Arg Phe Tyr Gly Gln Arg Ile Leu Phe
            100                 105                 110

Ala Ile Phe Ile Ala Ala Met Ala Leu Pro Lys Gln Val Val Leu Val
        115                 120                 125

Pro Leu Val Arg Ile Val Asn Phe Met Gly Ile His Asp Thr Leu Trp
    130                 135                 140

Ala Val Ile Leu Pro Leu Ile Gly Trp Pro Phe Gly Val Phe Leu Met
145                 150                 155                 160

Lys Gln Phe Ser Glu Asn Ile Pro Thr Glu Leu Leu Glu Ser Ala Lys
                165                 170                 175

Ile Asp Gly Cys Gly Glu Ile Arg Thr Phe Trp Ser Val Ala Phe Pro
            180                 185                 190

Ile Val Lys Pro Gly Phe Ala Ala Leu Ala Ile Phe Thr Phe Ile Asn
        195                 200                 205

Thr Trp Asn Asp Tyr Phe Met Gln Leu Val Met Leu Thr Ser Arg Asn
    210                 215                 220

Asn Leu Thr Ile Ser Leu Gly Val Ala Thr Met Gln Ala Glu Met Ala
225                 230                 235                 240

Thr Asn Tyr Gly Leu Ile Met Ala Gly Ala Ala Leu Ala Ala Val Pro
                245                 250                 255

Ile Val Thr Val Phe Leu Val Phe Gln Lys Ser Phe Thr Gln Gly Ile
            260                 265                 270

Thr Met Gly Ala Val Lys Gly
        275

<210> 123
<211> 216
<212> PRT
<213> Streptococcus pneumoniae

<400> 123

Met Lys Ile Met Phe Lys Asn Phe Asn Asn Ile Leu Leu Asn Arg Lys
1               5                   10                  15

Ile Val Leu Leu Leu Arg Ile Val Leu Met Met Ile Leu Ile Asn His
            20                  25                  30

Leu Leu Ser Thr Ala Val Gln Lys Gln Asp Ala Val Ile Phe Phe Lys
        35                  40                  45

Arg Glu Leu Ile Ser Ile Phe Ser Tyr Asn Asp Tyr Ser Glu Ala Asn
    50                  55                  60

Leu Glu Ile Pro Lys Leu Leu Leu Asn Leu Ser Leu Phe Met Val Gly
65                  70                  75                  80

Trp Leu Ser Val Ile Leu Leu Glu Ser Asp Leu Ala Asp His Tyr His
                85                  90                  95

His Leu Ile Arg Tyr Gln Ser Ser Ser Phe Phe Asp Tyr Thr Arg Lys
            100                 105                 110

Arg Leu Val Val Ile Ser Lys Phe Phe Thr Gln Asp Leu Phe Val Trp
        115                 120                 125

Phe Leu Gly Leu Leu Pro Leu Gly Ile His Phe Lys Thr Val Ala Leu
    130                 135                 140

Phe Phe Leu Leu Ala Gln Leu Met Met Leu Tyr Leu Leu Leu Ser Tyr
145                 150                 155                 160

Leu Ile Ala Leu Ile Ser Ala Gly Ala Gly Phe Ser Phe Phe Leu Tyr
                165                 170                 175

Phe Leu Ala Phe Val Gly Gln Glu Trp Met Met Asp His Ile Val Thr
            180                 185                 190

Val Tyr Leu Val Leu Leu Ser Leu Leu Val Met Leu Ile Val Ser Arg
        195                 200                 205

Leu Glu Glu Lys Phe Lys Lys Gly
    210                 215

<210> 124
<211> 174
<212> PRT
<213> Streptococcus pneumoniae

<400> 124
Met Gly Lys Gly Glu Met Gly Lys Gly Val Ile Gly Leu Glu Phe Asp
1               5                   10                  15

Ser Glu Val Leu Val Asn Lys Ala Pro Thr Leu Gln Leu Ala Asn Gly
            20                  25                  30

Lys Thr Ala Thr Phe Leu Thr Gln Tyr Asp Ser Lys Thr Leu Leu Phe
        35                  40                  45

Ala Val Asp Lys Glu Asp Ile Gly Gln Glu Ile Ile Gly Ile Ala Lys
    50                  55                  60

Gly Ser Ile Glu Ser Met His Asn Leu Pro Val Asn Leu Ala Gly Ala
65                  70                  75                  80

Arg Val Pro Gly Gly Val Asn Gly Ser Lys Ala Ala Val His Glu Val
                85                  90                  95

Pro Glu Phe Thr Gly Gly Val Asn Gly Thr Glu Pro Ala Val His Glu
            100                 105                 110

Ile Ala Glu Tyr Lys Gly Ser Asp Ser Leu Val Thr Leu Thr Thr Lys
        115                 120                 125

Lys Asp Tyr Thr Tyr Lys Ala Pro Leu Ala Gln Gln Ala Leu Pro Glu
    130                 135                 140

Thr Gly Asn Lys Glu Ser Asp Leu Leu Ala Ser Leu Gly Leu Thr Ala
145                 150                 155                 160

Phe Phe Leu Gly Leu Phe Thr Leu Gly Lys Lys Arg Glu Gln
                165                 170

<210> 125
<211> 629
<212> PRT
<213> Streptococcus pneumoniae

<400> 125
   Met Lys Lys Thr Phe Phe Leu Leu Val Leu Gly Leu Phe Cys Leu Leu
   1               5                   10                  15
   Pro Leu Ser Val Phe Ala Ile Asp Phe Lys Ile Asn Ser Tyr Gln Gly
               20                  25                  30
   Asp Leu Tyr Ile His Ala Asp Asn Thr Ala Glu Phe Arg Gln Lys Ile
           35                  40                  45
   Val Tyr Gln Phe Glu Glu Asp Phe Lys Gly Gln Ile Val Gly Leu Gly
       50                  55                  60
   Arg Ala Gly Lys Met Pro Ser Gly Phe Asp Ile Asp Pro His Pro Lys
   65                  70                  75                  80
   Ile Gln Ala Ala Lys Asn Gly Ala Glu Leu Ala Asp Val Thr Ser Glu
                   85                  90                  95
   Val Thr Glu Glu Ala Asp Gly Tyr Thr Val Arg Val Tyr Asn Pro Gly
               100                 105                 110
   Gln Glu Gly Asp Ile Val Glu Val Asp Leu Val Trp Asn Leu Lys Asn
           115                 120                 125
   Leu Leu Phe Leu Tyr Asp Asp Ile Ala Glu Leu Asn Trp Gln Pro Leu
       130                 135                 140
   Thr Asp Ser Ser Glu Ser Ile Glu Lys Phe Glu Phe His Val Arg Gly
   145                 150                 155                 160
   Asp Lys Gly Ala Glu Lys Leu Phe Phe His Thr Gly Lys Leu Phe Arg
   165 170 175
   Glu Gly Thr Ile Glu Lys Ser Asn Leu Asp Tyr Thr Ile Arg Leu Asp
   180 185 190
   Asn Leu Pro Ala Lys Arg Gly Val Glu Leu His Ala Tyr Trp Pro Arg
   195 200 205
   Thr Asp Phe Ala Ser Ala Arg Asp Gln Gly Leu Lys Gly Asn Arg Leu
   210 215 220
   Glu Glu Phe Asn Lys Ile Glu Asp Ser Ile Val Arg Glu Lys Asp Gln
   225 230 235 240
   Ser Lys Gln Leu Val Thr Trp Val Leu Pro Ser Ile Leu Ser Ile Ser
   245 250 255
   Leu Leu Leu Ser Val Cys Phe Tyr Phe Ile Tyr Arg Arg Lys Thr Thr
   260 265 270
   Pro Ser Val Lys Tyr Ala Lys Asn His Arg Leu Tyr Glu Pro Pro Met
   275 280 285
   Glu Leu Glu Pro Met Val Leu Ser Glu Ala Val Tyr Ser Thr Ser Leu
   290 295 300
   Glu Glu Val Ser Pro Leu Val Lys Gly Ala Gly Lys Phe Thr Phe Asp
   305 310 315 320
   Gln Leu Ile Gln Ala Thr Leu Leu Asp Val Ile Asp Arg Gly Asn Val
   325 330 335
   Ser Ile Ile Ser Glu Gly Asp Ala Val Gly Leu Arg Leu Val Lys Glu
   340 345 350
   Asp Gly Leu Ser Ser Phe Glu Lys Asp Cys Leu Asn Leu Ala Phe Ser
   355 360 365
   Gly Lys Lys Glu Glu Thr Leu Ser Asn Leu Phe Ala Asp Tyr Lys Val
   370 375 380
   Ser Asp Ser Leu Tyr Arg Arg Ala Lys Val Ser Asp Glu Lys Arg Ile
   385 390 395 400
   Gln Ala Arg Gly Leu Gln Leu Lys Ser Ser Phe Glu Glu Val Leu Asn
   405 410 415
   Gln Met Gln Glu Gly Val Arg Lys Arg Val Ser Phe Trp Gly Leu Pro
   420 425 430
   Asp Tyr Tyr Arg Pro Leu Thr Gly Gly Glu Lys Ala Leu Gln Val Gly
   435 440 445
   Met Gly Ala Leu Thr Ile Leu Pro Leu Phe Ile Gly Phe Gly Leu Phe
   450 455 460
   Leu Tyr Ser Leu Asp Val His Gly Tyr Leu Tyr Leu Pro Leu Pro Ile
   465 470 475 480
   Leu Gly Phe Leu Gly Leu Val Leu Ser Val Phe Tyr Tyr Trp Lys Leu
   485 490 495
   Arg Leu Asp Asn Arg Asp Gly Val Leu Asn Glu Ala Gly Ala Glu Val
               500                 505                 510
   Tyr Tyr Leu Trp Thr Ser Phe Glu Asn Met Leu Arg Glu Ile Ala Arg
           515                 520                 525
   Leu Asp Gln Ala Glu Leu Glu Ser Ile Val Val Trp Asn Arg Leu Leu
       530                 535                 540
   Val Tyr Ala Thr Leu Phe Gly Tyr Ala Asp Lys Val Ser His Leu Met
   545                 550                 555                 560
   Lys Val His Gln Ile Gln Val Glu Asn Pro Asp Ile Asn Leu Tyr Val
                   565                 570                 575
   Ala Tyr Gly Trp His Ser Thr Phe Tyr His Ser Thr Ala Gln Met Ser
               580                 585                 590
   His Tyr Ala Ser Val Ala Asn Thr Ala Ser Thr Tyr Ser Val Ser Ser
           595                 600                 605
   Gly Ser Gly Ser Ser Gly Gly Gly Phe Ser Gly Gly Gly Gly Gly Gly
       610                 615                 620
   Ser Ile Gly Ala Phe
   625

<210> 126
<211> 886
<212> PRT
<213> Streptococcus pneumoniae

<400> 126
Met Lys Lys Val Arg Lys Ile Phe Gln Lys Ala Val Ala Gly Leu Cys
  1               5                  10                  15

Cys Ile Ser Gln Leu Thr Ala Phe Ser Ser Ile Val Ala Leu Ala Glu
             20                  25                  30

Thr Pro Glu Thr Ser Pro Ala Ile Gly Lys Val Val Ile Lys Glu Thr
         35                  40                  45

Gly Glu Gly Gly Ala Leu Leu Gly Asp Ala Val Phe Glu Leu Lys Asn
     50                  55                  60

Asn Thr Asp Gly Thr Thr Val Ser Gln Arg Thr Glu Ala Gln Thr Gly
 65                  70                  75                  80

Glu Ala Ile Phe Ser Asn Ile Lys Pro Gly Thr Tyr Thr Leu Thr Glu
                 85                  90                  95

Ala Gln Pro Pro Val Gly Tyr Lys Pro Ser Thr Lys Gln Trp Thr Val
            100                 105                 110

Glu Val Glu Lys Asn Gly Arg Thr Thr Val Gln Gly Glu Gln Val Glu
        115                 120                 125

Asn Arg Glu Glu Ala Leu Ser Asp Gln Tyr Pro Gln Thr Gly Thr Tyr

130                 135                 140

Pro Asp Val Gln Thr Pro Tyr Gln Ile Ile Lys Val Asp Gly Ser Glu
145                 150                 155                 160

Lys Asn Gly Gln His Lys Ala Leu Asn Pro Asn Pro Tyr Glu Arg Val
                165                 170                 175

Ile Pro Glu Gly Thr Leu Ser Lys Arg Ile Tyr Gln Val Asn Asn Leu
            180                 185                 190

Asp Asp Asn Gln Tyr Gly Ile Glu Leu Thr Val Ser Gly Lys Thr Val
        195                 200                 205

Tyr Glu Gln Lys Asp Lys Ser Val Pro Leu Asp Val Val Ile Leu Leu
    210                 215                 220

Asp Asn Ser Asn Ser Met Ser Asn Ile Arg Asn Lys Asn Ala Arg Arg
225                 230                 235                 240

Ala Glu Arg Ala Gly Glu Ala Thr Arg Ser Leu Ile Asp Lys Ile Thr
                245                 250                 255

Ser Asp Ser Glu Asn Arg Val Ala Leu Val Thr Tyr Ala Ser Thr Ile
            260                 265                 270

Phe Asp Gly Thr Glu Phe Thr Val Glu Lys Gly Val Ala Asp Lys Asn
        275                 280                 285

Gly Lys Arg Leu Asn Asp Ser Leu Phe Trp Asn Tyr Asp Gln Thr Ser
    290                 295                 300

Phe Thr Thr Asn Thr Lys Asp Tyr Ser Tyr Leu Lys Leu Thr Asn Asp
305                 310                 315                 320

Lys Asn Asp Ile Val Glu Leu Lys Asn Lys Val Pro Thr Glu Ala Glu
                325                 330                 335

Asp His Asp Gly Asn Arg Leu Met Tyr Gln Phe Gly Ala Thr Phe Thr
            340                 345                 350

Gln Lys Ala Leu Met Lys Ala Asp Glu Ile Leu Thr Gln Gln Ala Arg
        355                 360                 365

Gln Asn Ser Gln Lys Val Ile Phe His Ile Thr Asp Gly Val Pro Thr
    370                 375                 380

Met Ser Tyr Pro Ile Asn Phe Asn His Ala Thr Phe Ala Pro Ser Tyr
385                 390                 395                 400

Gln Asn Gln Leu Asn Ala Phe Phe Ser Lys Ser Pro Asn Lys Asp Gly
                405                 410                 415

Ile Leu Leu Ser Asp Phe Ile Thr Gln Ala Thr Ser Gly Glu His Thr
            420                 425                 430

Ile Val Arg Gly Asp Gly Gln Ser Tyr Gln Met Phe Thr Asp Lys Thr
        435                 440                 445

Val Tyr Glu Lys Gly Ala Pro Ala Ala Phe Pro Val Lys Pro Glu Lys
    450                 455                 460

Tyr Ser Glu Met Lys Ala Ala Gly Tyr Ala Val Ile Gly Asp Pro Ile
465                 470                 475                 480

Asn Gly Gly Tyr Ile Trp Leu Asn Trp Arg Glu Ser Ile Leu Ala Tyr
            485                 490                 495

Pro Phe Asn Ser Asn Thr Ala Lys Ile Thr Asn His Gly Asp Pro Thr
        500                 505                 510

Arg Trp Tyr Tyr Asn Gly Asn Ile Ala Pro Asp Gly Tyr Asp Val Phe
            515                 520                 525

Thr Val Gly Ile Gly Ile Asn Gly Asp Pro Gly Thr Asp Glu Ala Thr
    530                 535                 540

Ala Thr Ser Phe Met Gln Ser Ile Ser Ser Lys Pro Glu Asn Tyr Thr
545                 550                 555                 560

Asn Val Thr Asp Thr Thr Lys Ile Leu Glu Gln Leu Asn Arg Tyr Phe
            565                 570                 575

His Thr Ile Val Thr Glu Lys Lys Ser Ile Glu Asn Gly Thr Ile Thr
        580                 585                 590

Asp Pro Met Gly Glu Leu Ile Asp Leu Gln Leu Gly Thr Asp Gly Arg
            595                 600                 605

Phe Asp Pro Ala Asp Tyr Thr Leu Thr Ala Asn Asp Gly Ser Arg Leu
    610                 615                 620

Glu Asn Gly Gln Ala Val Gly Gly Pro Gln Asn Asp Gly Gly Leu Leu
625                 630                 635                 640

Lys Asn Ala Lys Val Leu Tyr Asp Thr Thr Glu Lys Arg Ile Arg Val
            645                 650                 655

Thr Gly Leu Tyr Leu Gly Thr Asp Glu Lys Val Thr Leu Thr Tyr Asn
        660                 665                 670

Val Arg Leu Asn Asp Glu Phe Val Ser Asn Lys Phe Tyr Asp Thr Asn
            675                 680                 685

Gly Arg Thr Thr Leu His Pro Lys Glu Val Glu Gln Asn Thr Val Arg
    690                 695                 700

Asp Phe Pro Ile Pro Lys Ile Arg Asp Val Arg Lys Tyr Pro Glu Ile
705                 710                 715                 720

Thr Ile Ser Lys Glu Lys Lys Leu Gly Asp Ile Glu Phe Ile Lys Val
            725                 730                 735

Asn Lys Asn Asp Lys Lys Pro Leu Arg Gly Ala Val Phe Ser Leu Gln
        740                 745                 750

Lys Gln His Pro Asp Tyr Pro Asp Ile Tyr Gly Ala Ile Asp Gln Asn
            755                 760                 765

Gly Thr Tyr Gln Asn Val Arg Thr Gly Glu Asp Gly Lys Leu Thr Phe
    770                 775                 780

Lys Asn Leu Ser Asp Gly Lys Tyr Arg Leu Phe Glu Asn Ser Glu Pro
785                 790                 795                 800

Ala Gly Tyr Lys Pro Val Gln Asn Lys Pro Ile Val Ala Phe Gln Ile
                805                 810                 815

Val Asn Gly Glu Val Arg Asp Val Thr Ser Ile Val Pro Gln Asp Ile
            820                 825                 830

Pro Ala Gly Tyr Glu Phe Thr Asn Asp Lys His Tyr Ile Thr Asn Glu
        835                 840                 845

Pro Ile Pro Pro Lys Arg Glu Tyr Pro Arg Thr Gly Gly Ile Gly Met
    850                 855                 860

Leu Pro Phe Tyr Leu Ile Gly Cys Met Met Met Gly Gly Val Leu Leu
865                 870                 875                 880

Tyr Thr Arg Lys His Pro
                885

<210> 127
<211> 665
<212> PRT
<213> Streptococcus pneumoniae

<400> 127
Met Lys Ser Ile Asn Lys Phe Leu Thr Met Leu Ala Ala Leu Leu Leu
  1               5                  10                  15

Thr Ala Ser Ser Leu Phe Ser Ala Ala Thr Val Phe Ala Ala Gly Thr
             20                  25                  30

Thr Thr Thr Ser Val Thr Val His Lys Leu Leu Ala Thr Asp Gly Asp
         35                  40                  45

Met Asp Lys Ile Ala Asn Glu Leu Glu Thr Gly Asn Tyr Ala Gly Asn
     50                  55                  60

Lys Val Gly Val Leu Pro Ala Asn Ala Lys Glu Ile Ala Gly Val Met
 65                  70                  75                  80

Phe Val Trp Thr Asn Thr Asn Asn Glu Ile Ile Asp Glu Asn Gly Gln
                 85                  90                  95

Thr Leu Gly Val Asn Ile Asp Pro Gln Thr Phe Lys Leu Ser Gly Ala
            100                 105                 110

Met Pro Ala Thr Ala Met Lys Lys Leu Thr Glu Ala Glu Gly Ala Lys
        115                 120                 125

Phe Asn Thr Ala Asn Leu Pro Ala Ala Lys Tyr Lys Ile Tyr Glu Ile
    130                 135                 140

His Ser Leu Ser Thr Tyr Val Gly Glu Asp Gly Ala Thr Leu Thr Gly
145                 150                 155                 160

Ser Lys Ala Val Pro Ile Glu Ile Glu Leu Pro Leu Asn Asp Val Val
                165                 170                 175

Asp Ala His Val Tyr Pro Lys Asn Thr Glu Ala Lys Pro Lys Ile Asp
            180                 185                 190

Lys Asp Phe Lys Gly Lys Ala Asn Pro Asp Thr Pro Arg Val Asp Lys
        195                 200                 205

Asp Thr Pro Val Asn His Gln Val Gly Asp Val Val Glu Tyr Glu Ile
    210                 215                 220

Val Thr Lys Ile Pro Ala Leu Ala Asn Tyr Ala Thr Ala Asn Trp Ser
225                 230                 235                 240

Asp Arg Met Thr Glu Gly Leu Ala Phe Asn Lys Gly Thr Val Lys Val
                245                 250                 255

Thr Val Asp Asp Val Ala Leu Glu Ala Gly Asp Tyr Ala Leu Thr Glu
            260                 265                 270

Val Ala Thr Gly Phe Asp Leu Lys Leu Thr Asp Ala Gly Leu Ala Lys
        275                 280                 285

Val Asn Asp Gln Asn Ala Glu Lys Thr Val Lys Ile Thr Tyr Ser Ala
    290                 295                 300

Thr Leu Asn Asp Lys Ala Ile Val Glu Val Pro Glu Ser Asn Asp Val
305                 310                 315                 320

Thr Phe Asn Tyr Gly Asn Asn Pro Asp His Gly Asn Thr Pro Lys Pro
                325                 330                 335

Asn Lys Pro Asn Glu Asn Gly Asp Leu Thr Leu Thr Lys Thr Trp Val
            340                 345                 350

Asp Ala Thr Gly Ala Pro Ile Pro Ala Gly Ala Glu Ala Thr Phe Asp
        355                 360                 365

Leu Val Asn Ala Gln Thr Gly Lys Val Val Gln Thr Val Thr Leu Thr
    370                 375                 380

Thr Asp Lys Asn Thr Val Thr Val Asn Gly Leu Asp Lys Asn Thr Glu
385                 390                 395                 400

Tyr Lys Phe Val Glu Arg Ser Ile Lys Gly Tyr Ser Ala Asp Tyr Gln
                405                 410                 415

Glu Ile Thr Thr Ala Gly Glu Ile Ala Val Lys Asn Trp Lys Asp Glu
            420                 425                 430

Asn Pro Lys Pro Leu Asp Pro Thr Glu Pro Lys Val Val Thr Tyr Gly
        435                 440                 445

Lys Lys Phe Val Lys Val Asn Asp Lys Asp Asn Arg Leu Ala Gly Ala
    450                 455                 460

Glu Phe Val Ile Ala Asn Ala Asp Asn Ala Gly Gln Tyr Leu Ala Arg
465                 470                 475                 480

Lys Ala Asp Lys Val Ser Gln Glu Glu Lys Gln Leu Val Val Thr Thr
                485                 490                 495

Lys Asp Ala Leu Asp Arg Ala Val Ala Ala Tyr Asn Ala Leu Thr Ala
            500                 505                 510

Gln Gln Gln Thr Gln Gln Glu Lys Glu Lys Val Asp Lys Ala Gln Ala
        515                 520                 525

Ala Tyr Asn Ala Ala Val Ile Ala Ala Asn Asn Ala Phe Glu Trp Val
    530                 535                 540

Ala Asp Lys Asp Asn Glu Asn Val Val Lys Leu Val Ser Asp Ala Gln
545                 550                 555                 560

Gly Arg Phe Glu Ile Thr Gly Leu Leu Ala Gly Thr Tyr Tyr Leu Glu
                565                 570                 575

Glu Thr Lys Gln Pro Ala Gly Tyr Ala Leu Leu Thr Ser Arg Gln Lys
            580                 585                 590

Phe Glu Val Thr Ala Thr Ser Tyr Ser Ala Thr Gly Gln Gly Ile Glu
        595                 600                 605

Tyr Thr Ala Gly Ser Gly Lys Asp Asp Ala Thr Lys Val Val Asn Lys
    610                 615                 620

Lys Ile Thr Ile Pro Gln Thr Gly Gly Ile Gly Thr Ile Ile Phe Ala
625                 630                 635                 640

Val Ala Gly Ala Ala Ile Met Gly Ile Ala Val Tyr Ala Tyr Val Lys
                645                 650                 655

Asn Asn Lys Asp Glu Asp Gln Leu Ala
            660                 665

<210> 128
<211> 390
<212> PRT
<213> Streptococcus pneumoniae

<400> 128
Met Thr Met Gln Lys Met Gln Lys Met Ile Ser Arg Ile Phe Phe Val
1               5                   10                  15

Met Ala Leu Cys Phe Ser Leu Val Trp Gly Ala His Ala Val Gln Ala
            20                  25                  30

Gln Glu Asp His Thr Leu Val Leu Gln Leu Glu Asn Tyr Gln Glu Val
        35                  40                  45

Val Ser Gln Leu Pro Ser Arg Asp Gly His Arg Leu Gln Val Trp Lys
    50                  55                  60

Leu Asp Asp Ser Tyr Ser Tyr Asp Asp Arg Val Gln Ile Val Arg Asp
65                  70                  75                  80

Leu His Ser Trp Asp Glu Asn Lys Leu Ser Ser Phe Lys Lys Thr Ser
                85                  90                  95

Phe Glu Met Thr Phe Leu Glu Asn Gln Ile Glu Val Ser His Ile Pro
            100                 105                 110

Asn Gly Leu Tyr Tyr Val Arg Ser Ile Ile Gln Thr Asp Ala Val Ser
        115                 120                 125

Tyr Pro Ala Glu Phe Leu Phe Glu Met Thr Asp Gln Thr Val Glu Pro
    130                 135                 140

Leu Val Ile Val Ala Lys Lys Thr Asp Thr Met Thr Thr Lys Val Lys
145                 150                 155                 160

Leu Ile Lys Val Asp Gln Asp His Asn Arg Leu Glu Gly Val Gly Phe
                165                 170                 175

Lys Leu Val Ser Val Ala Arg Asp Val Ser Glu Lys Glu Val Pro Leu
            180                 185                 190

Ile Gly Glu Tyr Arg Tyr Ser Ser Ser Gly Gln Val Gly Arg Thr Leu
        195                 200                 205

Tyr Thr Asp Lys Asn Gly Glu Ile Phe Val Thr Asn Leu Pro Leu Gly
    210                 215                 220

Asn Tyr Arg Phe Lys Glu Val Glu Pro Leu Ala Gly Tyr Ala Val Thr
225                 230                 235                 240

Thr Leu Asp Thr Asp Val Gln Leu Val Asp His Gln Leu Val Thr Ile
                245                 250                 255

Thr Val Val Asn Gln Lys Leu Pro Arg Gly Asn Val Asp Phe Met Lys
            260                 265                 270

Val Asp Gly Arg Thr Asn Thr Ser Leu Gln Gly Ala Met Phe Lys Val
        275                 280                 285

Met Lys Glu Glu Ser Gly His Tyr Thr Pro Val Leu Gln Asn Gly Lys
    290                 295                 300

Glu Val Val Val Thr Ser Gly Lys Asp Gly Arg Phe Arg Val Glu Gly
305                 310                 315                 320

Leu Glu Tyr Gly Thr Tyr Tyr Leu Trp Glu Leu Gln Ala Pro Thr Gly
                325                 330                 335

Tyr Val Gln Leu Thr Ser Pro Val Ser Phe Thr Ile Gly Lys Asp Thr
            340                 345                 350

Arg Lys Glu Leu Val Thr Val Val Lys Asn Asn Lys Arg Pro Arg Ile
        355                 360                 365

Asp Val Pro Asp Thr Gly Glu Glu Thr Leu Val Tyr Leu Asp Ala Cys
    370                 375                 380

Cys His Phe Val Val Trp
385                 390

<210> 129
<211> 521
<212> PRT
<213> Streptococcus pneumoniae

<400> 129
Met Ser His Ile Tyr Leu Ser Ile Phe Thr Ser Leu Leu Leu Met Leu
1               5                   10                  15

Gly Leu Val Asn Val Ala Gln Ala Asp Glu Tyr Leu Arg Ile Gly Met
            20                  25                  30

Glu Ala Ala Tyr Ala Pro Phe Asn Trp Thr Gln Asp Asp Asp Ser Asn
        35                  40                  45

Gly Ala Val Lys Ile Asp Gly Thr Asn Gln Tyr Ala Asn Gly Tyr Asp
    50                  55                  60

Val Gln Ile Ala Lys Lys Ile Ala Lys Asp Leu Gly Lys Glu Pro Leu
65                  70                  75                  80

Val Val Lys Thr Lys Trp Glu Gly Leu Val Pro Ala Leu Thr Ser Gly
                85                  90                  95

Lys Ile Asp Met Ile Ile Ala Gly Met Ser Pro Thr Ala Glu Arg Lys
            100                 105                 110

Gln Glu Ile Ala Phe Ser Ser Ser Tyr Tyr Thr Ser Glu Pro Val Leu
        115                 120                 125

Leu Val Lys Lys Asp Ser Ala Tyr Ala Ser Ala Lys Ser Leu Asp Asp
    130                 135                 140

Phe Asn Gly Ala Lys Ile Thr Ser Gln Gln Gly Val Tyr Leu Tyr Asn
145                 150                 155                 160

Leu Ile Ala Gln Ile Pro Gly Ala Lys Lys Glu Thr Ala Met Gly Asp
                165                 170                 175

Phe Ala Gln Met Arg Gln Ala Leu Glu Ala Gly Val Ile Asp Ala Tyr
            180                 185                 190

Val Ser Glu Arg Pro Glu Ala Leu Thr Ala Glu Ala Ala Asn Ser Lys
        195                 200                 205

Phe Lys Met Ile Gln Val Glu Pro Gly Phe Lys Thr Gly Glu Glu Asp
    210                 215                 220

Thr Ala Ile Ala Ile Gly Leu Arg Lys Asn Asp Asn Arg Ile Ser Gln
225                 230                 235                 240

Ile Asn Ala Ser Ile Glu Thr Ile Ser Lys Asp Asp Gln Val Ala Leu
                245                 250                 255

Met Asp Arg Met Ile Lys Glu Gln Pro Ala Glu Ala Thr Thr Thr Glu
            260                 265                 270

Glu Thr Ser Ser Ser Phe Phe Ser Gln Val Ala Lys Ile Leu Ser Glu
        275                 280                 285

Asn Trp Gln Gln Leu Leu Arg Gly Ala Gly Ile Thr Leu Leu Ile Ser
    290                 295                 300

Ile Val Gly Thr Ile Ile Gly Leu Ile Ile Gly Leu Ala Ile Gly Val
305                 310                 315                 320

Phe Arg Thr Ala Pro Leu Ser Glu Asn Lys Val Ile Tyr Gly Leu Gln
                325                 330                 335

Lys Leu Val Gly Trp Val Leu Asn Val Tyr Ile Glu Ile Phe Arg Gly
            340                 345                 350

Thr Pro Met Ile Val Gln Ser Met Val Ile Tyr Tyr Gly Thr Ala Gln
        355                 360                 365

Ala Phe Gly Ile Asn Leu Asp Arg Thr Leu Ala Ala Ile Phe Ile Val
    370                 375                 380

Ser Ile Asn Thr Gly Ala Tyr Met Thr Glu Ile Val Arg Gly Gly Ile
385                 390                 395                 400

Leu Ala Val Asp Lys Gly Gln Phe Glu Ala Ala Thr Ala Leu Gly Met
                405                 410                 415

Thr His Asn Gln Thr Met Arg Lys Ile Val Leu Pro Gln Val Val Arg
            420                 425                 430

Asn Ile Leu Pro Ala Thr Gly Asn Glu Phe Val Ile Asn Ile Lys Asp
        435                 440                 445

Thr Ser Val Leu Asn Val Ile Ser Val Val Glu Leu Tyr Phe Ser Gly
    450                 455                 460

Asn Thr Val Ala Thr Gln Thr Tyr Gln Tyr Phe Gln Thr Phe Thr Ile
465                 470                 475                 480

Ile Ala Val Ile Tyr Phe Val Leu Thr Phe Thr Val Thr Arg Ile Leu
                485                 490                 495

Arg Phe Ile Glu Arg Arg Met Asp Met Asp Thr Tyr Thr Thr Gly Ala
            500                 505                 510

Asn Gln Met Gln Thr Glu Asp Leu Lys
        515                 520

<210> 130
<211> 246
<212> PRT
<213> Streptococcus pneumoniae

<400> 130
Met Thr Gln Ala Ile Leu Glu Ile Lys His Leu Lys Lys Ser Tyr Gly
  1               5                  10                  15

Gln Asn Glu Val Leu Lys Asp Ile Ser Leu Thr Val His Lys Gly Glu
             20                  25                  30

Val Ile Ser Ile Ile Gly Ser Ser Gly Ser Gly Lys Ser Thr Phe Leu
         35                  40                  45

Arg Ser Ile Asn Leu Leu Glu Thr Pro Thr Asp Gly Gln Ile Leu Tyr
     50                  55                  60

His Gly Gln Asn Val Leu Glu Lys Gly Tyr Asp Leu Thr Gln Tyr Arg

65                  70                  75                  80

Glu Lys Leu Gly Met Val Phe Gln Ser Phe Asn Leu Phe Glu Asn Leu
                85                  90                  95

Asn Val Leu Glu Asn Thr Ile Val Ala Gln Thr Thr Val Leu Lys Arg
            100                 105                 110

Glu Arg Thr Glu Ala Glu Lys Ile Ala Lys Glu Asn Leu Glu Lys Val
        115                 120                 125

Gly Met Gly Glu Arg Tyr Trp Gln Ala Lys Pro Lys Gln Leu Ser Gly
    130                 135                 140

Gly Gln Lys Gln Arg Val Ala Ile Ala Arg Ala Leu Ser Met Asn Pro
145                 150                 155                 160

Asp Ala Ile Leu Phe Asp Glu Pro Thr Ser Ala Leu Asp Pro Glu Met
                165                 170                 175

Val Gly Glu Val Leu Lys Ile Met Gln Asp Leu Ala Gln Glu Gly Leu
            180                 185                 190

Thr Met Ile Val Val Thr His Glu Met Glu Phe Ala Arg Asp Val Ser
        195                 200                 205

His Arg Val Ile Phe Met Asp Lys Gly Val Ile Ala Glu Glu Gly Lys
    210                 215                 220

Pro Glu Asp Leu Phe Thr Asn Pro Lys Glu Asp Arg Thr Lys Glu Phe
225                 230                 235                 240

Leu Gln Arg Tyr Leu Lys
                245

<210> 131
<211> 229
<212> PRT
<213> Streptococcus pneumoniae

<400> 131
Met Lys Lys Tyr Gln Leu Leu Phe Lys Ile Ser Ala Val Phe Ser Tyr
1               5                   10                  15

Leu Phe Phe Val Phe Ser Leu Ser Gln Leu Thr Leu Ile Val Gln Asn
            20                  25                  30

Tyr Trp Gln Phe Ser Ser Gln Ile Gly Asn Leu Phe Trp Ile Gln Asn
        35                  40                  45

Ile Leu Ser Leu Leu Phe Ile Gly Val Met Ile Val Val Leu Val Lys
    50                  55                  60

Thr Gly His Gly Tyr Leu Phe Arg Ile Pro Arg Lys Lys Trp Leu Trp
65                  70                  75                  80

Tyr Ser Ile Leu Thr Val Leu Val Leu Val Phe Gln Ile Ser Phe Asn
                85                  90                  95

Val Gln Thr Ala Lys His Val Gln Ser Thr Ala Glu Gly Trp Ala Val
            100                 105                 110

Leu Ile Gly Tyr Ser Gly Thr Asn Phe Ala Glu Leu Gly Ile Tyr Ile
        115                 120                 125

Ala Leu Phe Phe Leu Val Pro Leu Met Glu Glu Leu Ile Tyr Arg Gly
    130                 135                 140

Leu Leu Gln His Ala Phe Phe Lys His Ser Arg Phe Gly Leu Asp Leu
145                 150                 155                 160

Leu Leu Pro Ser Ile Leu Phe Ala Leu Pro His Phe Ser Ser Leu Pro
                165                 170                 175

Ser Leu Leu Asp Ile Phe Val Phe Ala Thr Val Gly Ile Ile Phe Ala
            180                 185                 190

Gly Leu Thr Arg Tyr Thr Lys Ser Ile Tyr Pro Ser Tyr Ala Val His
        195                 200                 205

Val Ile Asn Asn Ile Val Ala Thr Phe Pro Phe Leu Leu Thr Phe Leu
    210                 215                 220

His Arg Val Leu Gly
225

<210> 132
<211> 350
<212> PRT
<213> Streptococcus pneumoniae

<400> 132
Met Asn Lys Lys Gln Trp Leu Gly Leu Gly Leu Val Ala Val Ala Ala
  1               5                  10                  15

Val Gly Leu Ala Ala Cys Gly Asn Arg Ser Ser Arg Asn Ala Ala Ser
             20                  25                  30

Ser Ser Asp Val Lys Thr Lys Ala Ala Ile Val Thr Asp Thr Gly Gly
         35                  40                  45

Val Asp Asp Lys Ser Phe Asn Gln Ser Ala Trp Glu Gly Leu Gln Ala
     50                  55                  60

Trp Gly Lys Glu His Asn Leu Ser Lys Asp Asn Gly Phe Thr Tyr Phe
 65                  70                  75                  80

Gln Ser Thr Ser Glu Ala Asp Tyr Ala Asn Asn Leu Gln Gln Ala Ala
                 85                  90                  95

Gly Ser Tyr Asn Leu Ile Phe Gly Val Gly Phe Ala Leu Asn Asn Ala
            100                 105                 110

Val Lys Asp Ala Ala Lys Glu His Thr Asp Leu Asn Tyr Val Leu Ile
        115                 120                 125

Asp Asp Val Ile Lys Asp Gln Lys Asn Val Ala Ser Val Thr Phe Ala
    130                 135                 140

Asp Asn Glu Ser Gly Tyr Leu Ala Gly Val Ala Ala Ala Lys Thr Thr
145                 150                 155                 160

Lys Thr Lys Gln Val Gly Phe Val Gly Gly Ile Glu Ser Glu Val Ile
                165                 170                 175

Ser Arg Phe Glu Ala Gly Phe Lys Ala Gly Val Ala Ser Val Asp Pro
            180                 185                 190

Ser Ile Lys Val Gln Val Asp Tyr Ala Gly Ser Phe Gly Asp Ala Ala
        195                 200                 205

Lys Gly Lys Thr Ile Ala Ala Ala Gln Tyr Ala Ala Gly Ala Asp Ile
    210                 215                 220

Val Tyr Gln Val Ala Gly Gly Thr Gly Ala Gly Val Phe Ala Glu Ala
225                 230                 235                 240

Lys Ser Leu Asn Glu Ser Arg Pro Glu Asn Glu Lys Val Trp Val Ile
                245                 250                 255

Gly Val Asp Arg Asp Gln Glu Ala Glu Gly Lys Tyr Thr Ser Lys Asp
            260                 265                 270

Gly Lys Glu Ser Asn Phe Val Leu Val Ser Thr Leu Lys Gln Val Gly
        275                 280                 285

Thr Thr Val Lys Asp Ile Ser Asn Lys Ala Glu Arg Gly Glu Phe Pro
    290                 295                 300

Gly Gly Gln Val Ile Val Tyr Ser Leu Lys Asp Lys Gly Val Asp Leu
305                 310                 315                 320

Ala Val Thr Asn Leu Ser Glu Glu Gly Lys Lys Ala Val Glu Asp Ala
                325                 330                 335

Lys Ala Lys Ile Leu Asp Gly Ser Val Lys Val Pro Glu Lys
            340                 345                 350

<210> 133
<211> 352
<212> PRT
<213> Streptococcus pneumoniae

<400> 133
Met Ser Lys Lys Leu Gln Gln Ile Ser Val Pro Leu Ile Ser Val Phe
  1               5                  10                  15

Leu Gly Ile Leu Leu Gly Ala Ile Val Met Trp Ile Phe Gly Tyr Asp
             20                  25                  30

Ala Ile Trp Gly Tyr Glu Glu Leu Phe Tyr Thr Ala Phe Gly Ser Leu
         35                  40                  45

Arg Gly Ile Gly Glu Ile Phe Arg Ala Met Gly Pro Leu Val Leu Ile
     50                  55                  60

Gly Leu Gly Phe Ala Val Ala Ser Arg Ala Gly Phe Phe Asn Val Gly

65                  70                  75                  80

Leu Pro Gly Gln Ala Leu Ala Gly Trp Ile Leu Ser Gly Trp Phe Ala
                85                  90                  95

Leu Ser His Pro Asp Met Pro Arg Pro Leu Met Ile Leu Ala Thr Ile
            100                 105                 110

Val Ile Ala Leu Ile Ala Gly Gly Ile Val Gly Ala Ile Pro Gly Ile
        115                 120                 125

Leu Arg Ala Tyr Leu Gly Thr Ser Glu Val Ile Val Thr Ile Met Met
    130                 135                 140

Asn Tyr Ile Val Leu Tyr Val Gly Asn Ala Phe Ile His Ala Phe Pro
145                 150                 155                 160

Lys Asp Phe Met Gln Ser Thr Asp Ser Thr Ile Arg Val Gly Ala Asn
                165                 170                 175

Ala Thr Tyr Gln Thr Pro Trp Leu Ala Glu Leu Thr Gly Asn Ser Arg
            180                 185                 190

Met Asn Ile Gly Ile Phe Phe Ala Ile Ile Ala Val Ala Val Ile Trp
        195                 200                 205

Phe Met Leu Lys Lys Thr Thr Leu Gly Phe Glu Ile Arg Ala Val Gly
    210                 215                 220

Leu Asn Pro His Ala Ser Glu Tyr Ala Gly Ile Ser Ala Lys Arg Thr
225                 230                 235                 240

Ile Ile Leu Ser Met Ile Ile Ser Gly Ala Leu Ala Gly Leu Gly Gly
                245                 250                 255

Ala Val Glu Gly Leu Gly Thr Phe Gln Asn Val Tyr Val Gln Gly Ser
            260                 265                 270

Ser Leu Ala Ile Gly Phe Asn Gly Met Ala Val Ser Leu Leu Ala Ala
        275                 280                 285

Asn Ser Pro Ile Gly Ile Leu Phe Ala Ala Phe Leu Phe Gly Val Leu
    290                 295                 300

Gln Val Gly Ala Pro Gly Met Asn Ala Ala Gln Val Pro Ser Glu Leu
305                 310                 315                 320

Val Ser Ile Val Thr Ala Ser Ile Ile Phe Phe Val Ser Val His Tyr
                325                 330                 335

Leu Ile Glu Arg Phe Val Lys Pro Lys Lys Gln Val Lys Gly Gly Lys
            340                 345                 350

<210> 134
<211> 276
<212> PRT

<213> Streptococcus pneumoniae

<400> 134
Met Gly Val Lys Lys Lys Leu Lys Leu Thr Ser Leu Leu Gly Leu Ser
1               5                   10                  15

Leu Leu Ile Met Thr Ala Cys Ala Thr Asn Gly Val Thr Ser Asp Ile
            20                  25                  30

Thr Ala Glu Ser Ala Asp Phe Trp Ser Lys Leu Val Tyr Phe Phe Ala
        35                  40                  45

Glu Ile Ile Arg Phe Leu Ser Phe Asp Ile Ser Ile Gly Val Gly Ile
    50                  55                  60

Ile Leu Phe Thr Val Leu Ile Arg Thr Val Leu Leu Pro Val Phe Gln
65                  70                  75                  80

Val Gln Met Val Ala Ser Arg Lys Met Gln Glu Ala Gln Pro Arg Ile
                85                  90                  95

Lys Ala Leu Arg Glu Gln Tyr Pro Gly Arg Asp Met Glu Ser Arg Thr
            100                 105                 110

Lys Leu Glu Gln Glu Met Arg Lys Val Phe Lys Glu Met Gly Val Arg
        115                 120                 125

Gln Ser Asp Ser Leu Trp Pro Ile Leu Ile Gln Met Pro Val Ile Leu
    130                 135                 140

Ala Leu Phe Gln Ala Leu Ser Arg Val Asp Phe Leu Lys Thr Gly His
145                 150                 155                 160

Phe Leu Trp Ile Asn Leu Gly Ser Val Asp Thr Thr Leu Val Leu Pro
                165                 170                 175

Ile Leu Ala Ala Val Phe Thr Phe Leu Ser Thr Trp Leu Ser Asn Lys
            180                 185                 190

Ala Leu Ser Glu Arg Asn Gly Ala Thr Thr Ala Met Met Tyr Gly Ile
        195                 200                 205

Pro Val Leu Ile Phe Ile Phe Ala Val Tyr Ala Pro Gly Gly Val Ala
    210                 215                 220

Leu Tyr Trp Thr Val Ser Asn Ala Tyr Gln Val Leu Gln Thr Tyr Phe
225                 230                 235                 240

Leu Asn Asn Pro Phe Lys Ile Ile Ala Glu Arg Glu Ala Val Val Gln
                245                 250                 255

Ala Gln Lys Asp Leu Glu Asn Arg Lys Arg Lys Ala Lys Lys Lys Ala
            260                 265                 270

Gln Lys Thr Lys
        275

<210> 135
<211> 264

<212> PRT
<213> Streptococcus pneumoniae

<400> 135
Met Val Ile Asp Pro Phe Ala Ile Asn Glu Leu Asp Tyr Tyr Leu Val
1               5                   10                  15

Ser His Phe His Ser Asp His Ile Asp Pro Tyr Thr Ala Ala Ala Ile
            20                  25                  30

Leu Asn Asn Pro Lys Leu Glu His Val Lys Phe Ile Gly Pro Tyr His
        35                  40                  45

Cys Gly Arg Ile Trp Glu Gly Trp Gly Val Pro Lys Glu Arg Ile Ile
    50                  55                  60

Val Val Lys Pro Gly Asp Thr Ile Glu Leu Lys Asp Met Lys Ile His
65                  70                  75                  80

Ala Val Glu Ser Phe Asp Arg Thr Cys Leu Val Thr Leu Pro Val Asn
                85                  90                  95

Gly Ala Asp Glu Thr Gly Gly Glu Leu Ala Gly Leu Ala Val Thr Asp
            100                 105                 110

Glu Glu Met Ala Gln Lys Ala Val Asn Tyr Ile Phe Glu Thr Pro Gly
        115                 120                 125

Gly Thr Ile Tyr His Gly Ala Asp Ser His Phe Ser Asn Tyr Phe Ala
    130                 135                 140

Lys His Gly Lys Asp Phe Lys Ile Asp Val Ala Leu Asn Asn Tyr Gly
145                 150                 155                 160

Glu Asn Pro Val Gly Ile Gln Asp Lys Met Thr Ser Ile Asp Leu Leu
                165                 170                 175

Arg Met Ala Glu Asn Leu Arg Thr Lys Val Ile Ile Pro Val His Tyr
            180                 185                 190

Asp Ile Trp Ser Asn Phe Met Ala Ser Thr Asn Glu Ile Leu Glu Leu
        195                 200                 205

Trp Lys Met Arg Lys Asp Arg Leu Gln Tyr Asp Phe His Pro Phe Ile
    210                 215                 220

Trp Glu Val Gly Gly Lys Tyr Thr Tyr Pro Gln Asp Gln His Leu Val
225                 230                 235                 240

Glu Tyr His His Pro Arg Gly Phe Asp Asp Cys Phe Glu Gln Asp Ser
                245                 250                 255

Asn Ile Gln Phe Lys Ala Leu Leu
            260


<210> 136
<211> 194
<212> PRT
<213> Streptococcus pneumoniae

<400> 136
Met Phe Leu Ser Gly Trp Leu Ser Ser Phe Ala Asn Thr Tyr Ile His
1               5                   10                  15

Asp Leu Leu Gly Val Leu Phe Pro Asp Ser Pro Phe Leu Asn Ala Phe
            20                  25                  30

Glu Ser Ala Ile Ala Ala Pro Leu Val Glu Glu Pro Leu Lys Leu Leu
        35                  40                  45

Ser Leu Val Phe Val Leu Ala Leu Ile Pro Val Arg Lys Leu Lys Ser
    50                  55                  60

Leu Phe Leu Leu Gly Ile Ala Ser Gly Leu Gly Phe Gln Met Ile Lys
65                  70                  75                  80

Asp Ile Gly Tyr Ile Arg Thr Asp Leu Pro Glu Gly Phe Asp Phe Thr
                85                  90                  95

Ile Ser Arg Ile Leu Glu Arg Ile Ile Ser Gly Ile Ala Ser His Trp
            100                 105                 110

Thr Phe Ser Gly Leu Ala Val Val Gly Val Tyr Leu Leu Tyr Arg Ala
        115                 120                 125

Tyr Lys Gly Gln Lys Val Gly Lys Lys Gln Gly Leu Ile Phe Leu Gly
    130                 135                 140

Leu Ala Leu Gly Thr His Phe Leu Phe Asn Ser Pro Phe Val Glu Leu
145                 150                 155                 160

Glu Thr Glu Leu Pro Leu Ala Ile Pro Val Val Thr Ala Ile Ala Leu
                165                 170                 175

Tyr Gly Phe Tyr His Ala Tyr Cys Phe Val Glu Lys His Asn Glu Leu
            180                 185                 190

Met Thr

<210> 137
<211> 129
<212> PRT
<213> Streptococcus pneumoniae

<400> 137
Met Lys Val Glu Pro Arg Cys Asp Val Leu Ser Arg Met Ser His Phe
1               5                   10                  15

Phe Ile Arg Ile Leu Ile Met Glu Leu Gln Glu Leu Val Glu Arg Ser
            20                  25                  30

Trp Ala Ile Arg Gln Ala Tyr His Glu Leu Glu Val Lys His His Asp
        35                  40                  45

Ser Lys Trp Thr Val Glu Glu Asp Leu Leu Ala Leu Ser Asn Asp Ile
    50                  55                  60

Gly Asn Phe Gln Arg Leu Val Met Thr Lys Gln Gly Arg Tyr Tyr Asp
65                  70                  75                  80

Glu Thr Pro Tyr Thr Leu Glu Gln Lys Leu Ser Glu Asn Ile Trp Trp
                85                  90                  95

Leu Leu Glu Leu Ser Gln Arg Leu Asp Ile Asp Ile Leu Thr Glu Met
            100                 105                 110

Glu Asn Phe Leu Ser Asp Lys Glu Lys Gln Leu Asn Val Arg Thr Trp
        115                 120                 125

Lys

<210> 138
<211> 324
<212> PRT
<213> Streptococcus pneumoniae

<400> 138
Met Leu Asp Trp Lys Gln Phe Phe Leu Ala Tyr Leu Arg Ser Arg Ser
1               5                   10                  15

Arg Leu Phe Ile Tyr Leu Leu Ser Leu Ala Phe Leu Val Leu Leu Phe
            20                  25                  30

Gln Phe Leu Phe Ala Ser Leu Gly Ile Tyr Phe Leu Tyr Phe Phe Phe
        35                  40                  45

Leu Cys Cys Phe Val Thr Ile Leu Phe Phe Thr Trp Asp Ile Leu Val
    50                  55                  60

Glu Thr Gln Val Tyr Arg Gln Glu Leu Leu Tyr Gly Glu Arg Glu Ala
65                  70                  75                  80

Lys Ser Pro Leu Glu Ile Ala Leu Ala Glu Lys Leu Glu Ala Arg Glu
                85                  90                  95

Met Glu Leu Tyr Gln Gln Arg Ser Lys Ala Glu Arg Lys Leu Thr Asp
            100                 105                 110

Leu Leu Asp Tyr Tyr Thr Leu Trp Val His Gln Ile Lys Thr Pro Ile
        115                 120                 125

Ala Ala Ser Gln Leu Leu Val Ala Glu Val Val Asp Arg Gln Leu Lys
    130                 135                 140

Gln Gln Leu Glu Gln Glu Ile Phe Lys Ile Asp Ser Tyr Thr Asn Leu
145                 150                 155                 160

Val Leu Gln Tyr Leu Arg Leu Glu Ser Phe His Asp Asp Leu Val Leu
                165                 170                 175

Lys Gln Val Gln Ile Glu Asp Leu Val Lys Glu Ile Ile Arg Lys Tyr
            180                 185                 190

Ala Leu Phe Phe Ile Gln Lys Gly Leu Asn Val Asn Leu His Asp Leu
        195                 200                 205

Asp Lys Glu Ile Val Thr Asp Lys Lys Trp Leu Leu Val Val Ile Glu
    210                 215                 220

Gln Ile Ile Ser Asn Ser Leu Lys Tyr Thr Lys Glu Gly Gly Leu Glu
225                 230                 235                 240

Ile Tyr Met Asp Asp Gln Glu Leu Cys Ile Lys Asp Thr Gly Ile Gly
                245                 250                 255

Ile Lys Asn Ser Asp Val Leu Arg Val Phe Glu Arg Gly Phe Ser Gly
            260                 265                 270

Tyr Asn Gly Arg Leu Thr Gln Gln Ser Ser Gly Leu Gly Leu Tyr Leu
        275                 280                 285

Ser Lys Lys Ile Ser Glu Glu Leu Gly His Gln Ile Arg Ile Glu Ser
    290                 295                 300

Glu Val Gly Lys Gly Thr Thr Val Arg Ile Gln Phe Ala Gln Val Asn
305                 310                 315                 320

Leu Val Leu Glu

<210> 139
<211> 195
<212> PRT
<213> Streptococcus pneumoniae

<400> 139
Met Glu Leu Asn Thr His Asn Ala Glu Ile Leu Leu Ser Ala Ala Asn
  1               5                  10                  15

Lys Ser His Tyr Pro Gln Asp Glu Leu Pro Glu Ile Ala Leu Ala Gly
             20                  25                  30

Arg Ser Asn Val Gly Lys Ser Ser Phe Ile Asn Thr Met Leu Asn Arg
         35                  40                  45

Lys Asn Leu Ala Arg Thr Ser Gly Lys Pro Gly Lys Thr Gln Leu Leu
     50                  55                  60

Asn Phe Phe Asn Ile Asp Asp Lys Met Arg Phe Val Asp Val Pro Gly
 65                  70                  75                  80

Tyr Gly Tyr Ala Arg Val Ser Lys Lys Glu Arg Glu Lys Trp Gly Cys
                 85                  90                  95

Met Ile Glu Glu Tyr Leu Thr Thr Arg Glu Asn Leu Arg Ala Val Val
            100                 105                 110

Ser Leu Val Asp Leu Arg His Asp Pro Ser Ala Asp Asp Val Gln Met
        115                 120                 125

Tyr Glu Phe Leu Lys Tyr Tyr Glu Ile Pro Val Ile Ile Val Ala Thr
    130                 135                 140

Lys Ala Asp Lys Ile Pro Arg Gly Lys Trp Asn Lys His Glu Ser Ala

145                 150                 155                 160

Ile Lys Lys Lys Leu Asn Phe Asp Pro Ser Asp Asp Phe Ile Leu Phe
                165                 170                 175

Ser Ser Val Ser Lys Ala Gly Met Asp Glu Ala Trp Asp Ala Ile Leu
            180                 185                 190

Glu Lys Leu
        195

<210> 140
<211> 296
<212> PRT
<213> Streptococcus pneumoniae

<400> 140
Met Thr Lys Lys Gln Leu His Leu Val Ile Val Thr Gly Met Ser Gly
1               5                   10                  15

Ala Gly Lys Thr Val Ala Ile Gln Ser Phe Glu Asp Leu Gly Tyr Phe
            20                  25                  30

Thr Ile Asp Asn Met Pro Pro Ala Leu Leu Pro Lys Phe Leu Gln Leu
        35                  40                  45

Val Glu Ile Lys Glu Asp Asn Pro Lys Leu Ala Leu Val Val Asp Met
    50                  55                  60

Arg Ser Arg Ser Phe Phe Ser Glu Ile Gln Ala Val Leu Asp Glu Leu
65                  70                  75                  80

Glu Asn Gln Asp Gly Leu Asp Phe Lys Ile Leu Phe Leu Asp Ala Ala
                85                  90                  95

Asp Lys Glu Leu Val Ala Arg Tyr Lys Glu Thr Arg Arg Ser His Pro
            100                 105                 110

Leu Ala Ala Asp Gly Arg Ile Leu Asp Gly Ile Lys Leu Glu Arg Glu
        115                 120                 125

Leu Leu Ala Pro Leu Lys Asn Met Ser Gln Asn Val Val Asp Thr Thr
    130                 135                 140

Glu Leu Thr Pro Arg Glu Leu Arg Lys Thr Leu Ala Glu Gln Phe Ser
145                 150                 155                 160

Asp Gln Glu Gln Ala Gln Ser Phe Arg Ile Glu Val Met Ser Phe Gly
                165                 170                 175

Phe Lys Tyr Gly Ile Pro Ile Asp Ala Asp Leu Val Phe Asp Val Arg
            180                 185                 190

Phe Leu Pro Asn Pro Tyr Tyr Leu Pro Glu Leu Arg Asn Gln Thr Gly
        195                 200                 205

Val Asp Glu Pro Val Tyr Asp Tyr Val Met Asn His Pro Glu Ser Glu
    210                 215                 220

Asp Phe Tyr Gln His Leu Leu Ala Leu Ile Glu Pro Ile Leu Pro Ser
225                 230                 235                 240

Tyr Gln Lys Glu Gly Lys Ser Val Leu Thr Ile Ala Met Gly Cys Thr
                245                 250                 255

Gly Gly Gln His Arg Ser Val Ala Phe Ala Lys Arg Leu Ala Gln Asp
            260                 265                 270

Leu Ser Lys Asn Trp Ser Val Asn Glu Gly His Arg Asp Lys Asp Arg
        275                 280                 285

Arg Lys Glu Thr Val Asn Arg Ser
    290                 295

<210> 141
<211> 325
<212> PRT
<213> Streptococcus pneumoniae

<400> 141
Met Arg Lys Pro Lys Ile Thr Val Ile Gly Gly Gly Thr Gly Ser Pro
  1               5                  10                  15

Val Ile Leu Lys Ser Leu Arg Glu Lys Asp Val Glu Ile Ala Ala Ile
             20                  25                  30

Val Thr Val Ala Asp Asp Gly Gly Ser Ser Gly Glu Leu Arg Lys Asn
         35                  40                  45

Met Gln Gln Leu Thr Pro Pro Gly Asp Leu Arg Asn Val Leu Val Ala
     50                  55                  60

Met Ser Asp Met Pro Lys Phe Tyr Glu Lys Val Phe Gln Tyr Arg Phe
 65                  70                  75                  80

Ser Glu Asp Ala Gly Ala Phe Ala Gly His Pro Leu Gly Asn Leu Ile
                 85                  90                  95

Ile Ala Gly Leu Ser Glu Met Gln Gly Ser Thr Tyr Asn Ala Met Gln
            100                 105                 110

Leu Leu Ser Lys Phe Phe His Thr Thr Gly Lys Ile Tyr Pro Ser Ser
        115                 120                 125

Asp His Pro Leu Thr Leu His Ala Val Phe Gln Asp Gly Thr Glu Val
    130                 135                 140

Ala Gly Glu Ser His Ile Val Asp His Arg Gly Ile Ile Asp Asn Val
145                 150                 155                 160

Tyr Val Thr Asn Ala Leu Asn Asp Asp Thr Pro Leu Ala Ser Arg Arg
                165                 170                 175

Val Val Gln Thr Ile Leu Glu Ser Asp Met Ile Val Leu Gly Pro Gly
            180                 185                 190

Ser Leu Phe Thr Ser Ile Leu Pro Asn Ile Val Ile Lys Glu Ile Gly
        195                 200                 205

Arg Ala Leu Leu Glu Thr Lys Ala Glu Ile Ala Tyr Val Cys Asn Ile
    210                 215                 220

Met Thr Gln Arg Gly Glu Thr Glu His Phe Thr Asp Ser Asp His Val
225                 230                 235                 240

Glu Val Leu His Arg His Leu Gly Arg Pro Phe Ile Asp Thr Val Leu
                245                 250                 255

Val Asn Ile Glu Lys Val Pro Gln Glu Tyr Met Asn Ser Asn Arg Phe
            260                 265                 270

Asp Glu Tyr Leu Val Gln Val Glu His Asp Phe Val Gly Leu Cys Lys
        275                 280                 285

Gln Val Ser Arg Val Ile Ser Ser Asn Phe Leu Arg Leu Glu Asn Gly
    290                 295                 300

Gly Ala Phe His Asp Gly Asp Leu Ile Val Asp Glu Leu Met Arg Ile
305                 310                 315                 320

Ile Gln Val Lys Lys
                325

<210> 142
<211> 392
<212> PRT
<213> Streptococcus pneumoniae

<400> 142
Met Lys Asn Leu Ile Lys Leu Leu Ile Ile Arg Leu Ile Val Asn Leu
  1               5                  10                  15

Ala Asp Ser Val Phe Tyr Ile Val Ala Leu Trp His Val Ser Asn Asn
             20                  25                  30

Tyr Ser Ser Ser Met Phe Leu Gly Ile Phe Ile Ala Val Asn Tyr Leu
         35                  40                  45

Pro Asp Leu Leu Leu Ile Phe Phe Gly Pro Val Ile Asp Arg Val Asn
     50                  55                  60

Pro Gln Lys Ile Leu Ile Ile Ser Ile Leu Val Gln Leu Ala Val Ala
 65                  70                  75                  80

Val Ile Phe Leu Leu Leu Leu Asn Gln Ile Ser Phe Trp Val Ile Met
                 85                  90                  95

Ser Leu Val Phe Ile Ser Val Met Ala Ser Ser Ile Ser Tyr Val Ile
            100                 105                 110

Glu Asp Val Leu Ile Pro Gln Val Val Glu Tyr Asp Lys Ile Val Phe
        115                 120                 125

Ala Asn Ser Leu Phe Ser Ile Ser Tyr Lys Val Leu Asp Ser Ile Phe
    130                 135                 140

Asn Ser Phe Ala Ser Phe Leu Gln Val Ala Val Gly Phe Ile Leu Leu

145                 150                 155                 160

Val Lys Ile Asp Ile Gly Ile Phe Leu Leu Ala Leu Phe Ile Leu Leu
                165                 170                 175

Leu Leu Lys Phe Arg Thr Ser Asn Ala Asn Ile Glu Asn Phe Ser Phe
            180                 185                 190

Lys Tyr Tyr Lys Arg Glu Val Leu Gln Gly Thr Lys Phe Ile Leu Asn
        195                 200                 205

Asn Lys Leu Leu Phe Lys Thr Ser Ile Ser Leu Thr Leu Ile Asn Phe
    210                 215                 220

Phe Tyr Ser Phe Gln Thr Val Val Val Pro Ile Phe Ser Ile Arg Tyr
225                 230                 235                 240

Phe Asp Gly Pro Ile Phe Tyr Gly Ile Phe Leu Thr Ile Ala Gly Leu
                245                 250                 255

Gly Gly Ile Leu Gly Asn Met Leu Ala Pro Ile Val Ile Lys Tyr Leu
            260                 265                 270

Lys Ser Asn Gln Ile Val Gly Val Phe Leu Phe Leu Asn Gly Ser Ser
        275                 280                 285

Trp Leu Val Ala Ile Val Ile Lys Asp Tyr Thr Leu Ser Leu Ile Leu
    290                 295                 300

Phe Phe Val Cys Phe Met Ser Lys Gly Val Phe Asn Ile Ile Phe Asn
305                 310                 315                 320

Ser Leu Tyr Gln Gln Ile Pro Pro His Gln Leu Leu Gly Arg Val Asn
                325                 330                 335

Thr Thr Ile Asp Ser Ile Ile Ser Phe Gly Met Pro Ile Gly Ser Leu
            340                 345                 350

Val Ala Gly Thr Leu Ile Asp Leu Asn Ile Glu Leu Val Leu Ile Ala
        355                 360                 365

Ile Ser Ile Pro Tyr Phe Leu Phe Ser Tyr Ile Phe Tyr Thr Asp Asn
    370                 375                 380

Gly Leu Lys Glu Phe Ser Ile Tyr
385                 390

<210> 143
<211> 225
<212> PRT
<213> Streptococcus pneumoniae

<400> 143
Met Met Ser Asn Lys Asn Lys Glu Ile Leu Ile Phe Ala Ile Leu Tyr
  1               5                  10                  15

Thr Val Leu Phe Met Phe Asp Gly Val Lys Leu Leu Ala Ser Leu Met
             20                  25                  30

Pro Ser Ala Ile Ala Asn Tyr Leu Val Tyr Val Val Leu Ala Leu Tyr
        35                  40                  45

Gly Ser Phe Leu Phe Lys Asp Arg Leu Ile Gln Gln Trp Lys Glu Ile
    50                  55                  60

Arg Lys Thr Lys Arg Lys Phe Phe Phe Gly Val Leu Thr Gly Trp Leu
65                  70                  75                  80

Phe Leu Ile Leu Met Thr Val Val Phe Glu Phe Val Ser Glu Met Leu
                85                  90                  95

Lys Gln Phe Val Gly Leu Asp Gly Gln Gly Leu Asn Gln Ser Asn Ile
            100                 105                 110

Gln Ser Thr Phe Gln Glu Gln Pro Leu Leu Ile Ala Val Phe Ala Cys
        115                 120                 125

Val Ile Gly Pro Leu Val Glu Glu Leu Phe Phe Arg Gln Val Leu Leu
    130                 135                 140

His Tyr Leu Gln Glu Arg Leu Ser Gly Leu Leu Ser Ile Ile Leu Val
145                 150                 155                 160

Gly Leu Val Phe Ala Leu Thr His Met His Ser Leu Ala Leu Ser Glu
                165                 170                 175

Trp Ile Gly Ala Val Gly Tyr Leu Gly Gly Gly Leu Ala Phe Ser Ile
            180                 185                 190

Ile Tyr Val Lys Glu Lys Glu Asn Ile Tyr Tyr Pro Leu Leu Val His
        195                 200                 205

Met Leu Ser Asn Ser Leu Ser Leu Ile Ile Leu Ala Ile Ser Ile Val
    210                 215                 220

Lys
225

<210> 144
<211> 385
<212> PRT
<213> Streptococcus pneumoniae

<400> 144
Leu Lys Lys Pro Ile Ile Glu Phe Lys Asn Val Ser Lys Val Phe Glu
1               5                   10                  15

Asp Ser Asn Thr Lys Val Leu Lys Asp Ile Asn Phe Glu Leu Glu Glu
            20                  25                  30

Gly Lys Phe Tyr Thr Leu Leu Gly Ala Ser Gly Ser Gly Lys Ser Thr
        35                  40                  45

Ile Leu Asn Ile Ile Ala Gly Leu Leu Asp Ala Thr Thr Gly Asp Ile
    50                  55                  60

Met Leu Asp Gly Val Arg Ile Asn Asp Ile Pro Thr Asn Lys Arg Asp
65                  70                  75                  80

Val His Thr Val Phe Gln Ser Tyr Ala Leu Phe Pro His Met Asn Val
                85                  90                  95

Phe Glu Asn Val Ala Phe Pro Leu Arg Leu Arg Lys Ile Asp Lys Lys
            100                 105                 110

Glu Ile Glu Gln Arg Val Ala Glu Val Leu Lys Met Val Gln Leu Glu
        115                 120                 125

Gly Tyr Glu Lys Arg Ser Ile Arg Lys Leu Ser Gly Gly Gln Arg Gln
    130                 135                 140

Arg Val Ala Ile Ala Arg Ala Ile Ile Asn Gln Pro Arg Val Val Leu
145                 150                 155                 160

Leu Asp Glu Pro Leu Ser Ala Leu Asp Leu Lys Leu Arg Thr Asp Met
                165                 170                 175

Gln Tyr Glu Leu Arg Glu Leu Gln Gln Arg Leu Gly Ile Thr Phe Val
            180                 185                 190

Phe Val Thr His Asp Gln Glu Glu Ala Leu Ala Met Ser Asp Trp Ile
        195                 200                 205

Phe Val Met Asn Asp Gly Glu Ile Val Gln Ser Gly Thr Pro Val Asp
    210                 215                 220

Ile Tyr Asp Glu Pro Ile Asn His Phe Val Ala Thr Phe Ile Gly Glu
225                 230                 235                 240

Ser Asn Ile Leu Pro Gly Thr Met Ile Glu Asp Tyr Leu Val Glu Phe
                245                 250                 255

Asn Gly Lys Arg Phe Glu Ala Val Asp Gly Gly Met Lys Pro Asn Glu
            260                 265                 270

Pro Val Glu Val Val Ile Arg Pro Glu Asp Leu Arg Ile Thr Leu Pro
        275                 280                 285

Glu Glu Gly Lys Leu Gln Val Lys Val Asp Thr Gln Leu Phe Arg Gly
    290                 295                 300

Val His Tyr Glu Ile Ile Ala Tyr Asp Glu Leu Gly Asn Glu Trp Met
305                 310                 315                 320

Ile His Ser Thr Arg Lys Ala Ile Val Gly Glu Glu Ile Gly Leu Asp
                325                 330                 335

Phe Glu Pro Glu Asp Ile His Ile Met Arg Leu Asn Glu Thr Glu Glu
            340                 345                 350

Glu Phe Asp Ala Arg Ile Glu Glu Tyr Val Glu Ile Glu Glu Gln Glu
        355                 360                 365

Ala Gly Leu Ile Asn Ala Ile Glu Glu Glu Arg Asp Glu Glu Asn Lys
    370                 375                 380

Leu
385

<210> 145
<211> 206
<212> PRT
<213> Streptococcus pneumoniae

<400> 145
Met Lys Ser Met Arg Ile Leu Phe Leu Leu Ala Leu Ile Gln Ile Ser
1               5                   10                  15

Leu Ser Ser Cys Phe Leu Trp Lys Glu Cys Ile Leu Ser Phe Lys Gln
            20                  25                  30

Ser Thr Ala Phe Phe Ile Gly Ser Met Val Phe Val Ser Gly Ile Cys
        35                  40                  45

Ala Gly Val Asn Tyr Leu Tyr Thr Arg Lys Gln Glu Val His Ser Val
    50                  55                  60

Leu Ala Ser Lys Lys Ser Val Lys Leu Phe Tyr Ser Met Leu Leu Leu
65                  70                  75                  80

Ile Asn Leu Leu Gly Ala Val Leu Val Leu Ser Asp Asn Leu Phe Ile
                85                  90                  95

Lys Asn Thr Leu Gln Gln Glu Leu Val Asp Phe Leu Leu Pro Ser Phe
            100                 105                 110

Phe Phe Leu Phe Gly Leu Asp Leu Leu Ile Phe Leu Pro Leu Lys Lys
        115                 120                 125

Tyr Val Arg Asp Phe Leu Ala Met Leu Asp Arg Lys Lys Thr Val Leu
    130                 135                 140

Val Thr Ile Leu Ala Thr Leu Leu Phe Leu Arg Asn Pro Met Thr Ile
145                 150                 155                 160

Val Ser Leu Leu Ile Tyr Ile Gly Leu Gly Leu Phe Phe Ala Ala Tyr
                165                 170                 175

Leu Val Pro Asn Ser Val Lys Lys Glu Val Ser Phe Tyr Gly His Ile
            180                 185                 190

Phe Arg Asp Leu Val Leu Val Ile Val Thr Leu Ile Phe Phe
        195                 200                 205

<210> 146
<211> 338
<212> PRT
<213> Streptococcus pneumoniae

<400> 146
Met Val Lys Lys Ile Ile Gly Met Val Leu Ala Leu Leu Ser Val Thr
1               5                   10                  15

Val Val Gly Val Gly Val Phe Ala Tyr Thr Ile Tyr Gln Gln Gly Thr
            20                  25                  30

Glu Thr Leu Ala Lys Thr Tyr Lys Lys Ile Gly Glu Glu Thr Lys Val
        35                  40                  45

Ile Glu Ala Thr Glu Pro Leu Thr Ile Leu Leu Met Gly Val Asp Thr
    50                  55                  60

Gly Asn Val Glu Arg Thr Glu Thr Trp Val Gly Arg Ser Asp Ser Met
65                  70                  75                  80

Ile Leu Met Thr Val Asn Pro Lys Thr Lys Lys Thr Thr Met Met Ser
                85                  90                  95

Leu Glu Arg Asp Ile Leu Thr Arg Ile Glu Ser Gly Asn Gly Gln Ala
            100                 105                 110

His Glu Ala Lys Leu Asn Ser Ala Tyr Ala Asp Gly Gly Ala Glu Leu
        115                 120                 125

Ala Ile Glu Thr Ile Gln Lys Met Met Asn Ile His Ile Asp Arg Tyr
    130                 135                 140

Val Met Val Asn Met Arg Gly Leu Gln Lys Leu Val Asp Ala Val Gly
145                 150                 155                 160

Gly Ile Thr Val Asn Asn Ile Leu Gly Phe Pro Ile Ser Ile Ser Asp
                165                 170                 175

Gln Glu Glu Phe Asn Thr Ile Ser Ile Gly Val Gly Glu Gln His Ile
            180                 185                 190

Gly Gly Glu Glu Ala Leu Val Tyr Ala Arg Met Arg Tyr Gln Asp Pro
        195                 200                 205

Glu Gly Asp Tyr Gly Arg Gln Lys Arg Gln Arg Glu Val Ile Gln Lys
    210                 215                 220

Val Met Glu Lys Ala Leu Ser Leu Asn Ser Ile Gly His Tyr Gln Glu
225                 230                 235                 240

Ile Leu Lys Ala Leu Ser Asp Asn Met Gln Thr Asn Ile Asp Leu Ser
                245                 250                 255

Ala Lys Ser Ile Pro Asn Leu Leu Gly Tyr Lys Asp Ser Phe Lys Thr
            260                 265                 270

Ile Glu Thr Gln Gln Leu Gln Gly Glu Gly Glu Ile Leu Gln Gly Val
        275                 280                 285

Ser Tyr Gln Ile Val Ser Arg Ala His Met Leu Glu Met Gln Asn Leu
    290                 295                 300

Leu Arg Arg Ser Leu Gly Gln Glu Glu Val Thr Gln Leu Glu Thr Asn
305                 310                 315                 320

Ala Val Leu Phe Glu Asp Leu Phe Gly Arg Ala Pro Val Gly Asp Glu
                325                 330                 335

Asp Asn

<210> 147
<211> 331
<212> PRT
<213> Streptococcus pneumoniae

<400> 147
Met Lys Lys Gln Ala Tyr Val Ile Ile Ala Leu Thr Ser Phe Leu Phe
1               5                   10                  15

Val Phe Phe Phe Ser His Ser Leu Leu Glu Ile Leu Asp Phe Asp Trp
            20                  25                  30

Ser Ile Phe Leu His Asp Val Glu Lys Thr Glu Lys Phe Val Phe Leu
        35                  40                  45

Leu Leu Val Phe Ser Met Ser Met Thr Cys Leu Leu Ala Leu Phe Trp
    50                  55                  60

Arg Gly Ile Glu Glu Leu Ser Leu Arg Lys Met Gln Ala Asn Leu Lys
65                  70                  75                  80

Arg Leu Leu Ala Gly Gln Glu Val Val Gln Val Ala Asp Pro Asp Leu
                85                  90                  95

Asp Ala Ser Phe Lys Ser Leu Ser Gly Lys Leu Asn Leu Leu Thr Glu
            100                 105                 110

Ala Leu Gln Lys Ala Glu Asn Gln Ser Leu Ala Gln Glu Glu Glu Ile
        115                 120                 125

Ile Glu Lys Glu Arg Lys Arg Ile Ala Arg Asp Leu His Asp Thr Val
    130                 135                 140

Ser Gln Glu Leu Phe Ala Ala His Met Ile Leu Ser Gly Ile Ser Gln
145                 150                 155                 160

Gln Ala Leu Lys Leu Asp Arg Glu Lys Met Gln Thr Gln Leu Gln Ser
                165                 170                 175

Val Thr Ala Ile Leu Glu Thr Ala Gln Lys Asp Leu Arg Val Leu Leu
            180                 185                 190

Leu His Leu Arg Pro Val Glu Leu Glu Gln Lys Ser Leu Ile Glu Gly
        195                 200                 205

Ile Gln Ile Leu Leu Lys Glu Leu Glu Asp Lys Ser Asp Leu Arg Val
    210                 215                 220

Ser Leu Lys Gln Asn Met Thr Lys Leu Pro Lys Lys Ile Glu Glu His
225                 230                 235                 240

Ile Phe Arg Ile Leu Gln Glu Leu Ile Ser Asn Thr Leu Arg His Ala
                245                 250                 255

Gln Ala Ser Cys Leu Asp Val Tyr Leu Tyr Gln Thr Asp Val Glu Leu
            260                 265                 270

Gln Leu Lys Val Val Asp Asn Gly Ile Gly Phe Gln Leu Gly Ser Leu
        275                 280                 285

Asp Asp Leu Ser Tyr Gly Leu Arg Asn Ile Lys Glu Arg Val Glu Asp
    290                 295                 300

Met Ala Gly Thr Val Gln Leu Leu Thr Ala Pro Lys Gln Gly Leu Ala
305                 310                 315                 320

Val Asp Ile Arg Ile Pro Leu Leu Asp Lys Glu
                325                 330

<210> 148
<211> 288
<212> PRT
<213> Streptococcus pneumoniae

<400> 148
Met Ile Val Ser Ile Ile Ser Gln Gly Phe Val Trp Ala Ile Leu Gly
1               5                   10                  15

Leu Gly Ile Phe Met Thr Phe Arg Ile Leu Asn Phe Pro Asp Met Thr
            20                  25                  30

Thr Glu Gly Ser Phe Pro Leu Gly Gly Ala Val Ala Val Thr Leu Ile
        35                  40                  45

Thr Lys Gly Val Asn Pro Phe Leu Ala Thr Leu Val Ala Val Gly Ala
    50                  55                  60

Gly Cys Leu Ala Gly Met Ala Ala Gly Leu Leu Tyr Thr Lys Gly Lys
65                  70                  75                  80

Ile Pro Thr Leu Leu Ser Gly Ile Leu Val Met Thr Ser Cys His Ser
                85                  90                  95

Ile Met Leu Leu Ile Met Gly Arg Ala Asn Leu Gly Leu Leu Gly Thr
            100                 105                 110

Lys Gln Ile Gln Asp Val Leu Pro Phe Asp Ser Asp Leu Asn Gln Leu
        115                 120                 125

Leu Thr Gly Leu Ile Phe Val Ser Ile Val Ile Ala Leu Met Leu Phe
    130                 135                 140

Phe Leu Asp Thr Lys Leu Gly Gln Ala Tyr Ile Ala Thr Gly Asp Asn
145                 150                 155                 160

Pro Asp Met Ala Arg Ser Phe Gly Ile His Thr Gly Arg Met Glu Leu
                165                 170                 175

Met Gly Leu Val Leu Ser Asn Gly Val Ile Ala Leu Ala Gly Ala Leu
            180                 185                 190

Ile Ala Gln Gln Glu Gly Tyr Ala Asp Val Ser Arg Gly Ile Gly Val
        195                 200                 205

Ile Val Val Gly Leu Ala Ser Leu Ile Ile Gly Glu Val Ile Phe Lys
    210                 215                 220

Ser Leu Ser Leu Ala Glu Arg Leu Val Thr Ile Val Val Gly Ser Ile
225                 230                 235                 240

Ala Tyr Gln Phe Leu Val Trp Ala Val Ile Ala Leu Gly Phe Asn Thr
                245                 250                 255

Ser Tyr Leu Arg Leu Tyr Ser Ala Leu Ile Leu Ala Val Cys Leu Met
            260                 265                 270

Ile Pro Thr Phe Lys Gln Thr Ile Leu Lys Gly Ala Lys Leu Ser Lys
        275                 280                 285

<210> 149
<211> 355
<212> PRT
<213> Streptococcus pneumoniae

<400> 149
Met Lys Lys Met Lys Val Trp Ser Thr Val Leu Ala Thr Gly Val Ala
1               5                   10                  15

Leu Thr Thr Leu Ala Ala Cys Ser Gly Gly Ser Asn Ser Thr Thr Ala
            20                  25                  30

Ser Ser Ser Glu Glu Lys Ala Asp Lys Ser Gln Glu Leu Val Ile Tyr
        35                  40                  45

Ser Asn Ser Val Ser Asn Gly Arg Gly Asp Trp Leu Thr Ala Lys Ala
    50                  55                  60

Lys Glu Ala Gly Phe Asn Ile Lys Met Val Asp Ile Ala Gly Ala Gln
65                  70                  75                  80

Leu Ala Asp Arg Val Ile Ala Glu Lys Asn Asn Ala Val Ala Asp Met
                85                  90                  95

Val Phe Gly Ile Gly Ala Val Asp Ser Asn Lys Ile Arg Asp Gln Lys
            100                 105                 110

Leu Leu Val Gln Tyr Lys Pro Lys Trp Leu Asp Lys Ile Asp Gln Ser
        115                 120                 125

Leu Ser Asp Lys Asp Asn Tyr Tyr Asn Pro Val Ile Val Gln Pro Leu
    130                 135                 140

Val Leu Ile Gly Ala Pro Asp Val Lys Glu Met Pro Lys Asp Trp Thr
145                 150                 155                 160

Glu Leu Gly Ser Lys Tyr Lys Gly Lys Tyr Ser Ile Ser Gly Leu Gln
                165                 170                 175

Gly Gly Thr Gly Arg Ala Ile Leu Ala Ser Ile Leu Val Arg Tyr Leu
            180                 185                 190

Asp Asp Lys Gly Glu Leu Gly Val Ser Glu Lys Gly Trp Glu Val Ala
        195                 200                 205

Lys Glu Tyr Leu Lys Asn Ala Tyr Thr Leu Gln Lys Gly Glu Ser Ser

210                 215                 220

Ile Val Lys Met Leu Asp Lys Glu Asp Pro Ile Gln Tyr Gly Met Met
225                 230                 235                 240

Trp Gly Ser Gly Ala Leu Val Gly Gln Lys Glu Gln Asn Val Val Phe
                245                 250                 255

Lys Val Met Thr Pro Glu Ile Gly Val Pro Phe Val Thr Glu Gln Thr
            260                 265                 270

Met Val Leu Ser Thr Ser Lys Lys Gln Ala Leu Ala Lys Glu Phe Ile
        275                 280                 285

Asp Trp Phe Gly Gln Ser Glu Ile Gln Val Glu Tyr Ser Lys Asn Phe
    290                 295                 300

Gly Ser Ile Pro Ala Asn Lys Asp Ala Leu Lys Asp Leu Pro Glu Asp
305                 310                 315                 320

Thr Lys Lys Phe Val Asp Gln Val Lys Pro Gln Asn Ile Asp Trp Glu
                325                 330                 335

Ala Val Gly Lys His Leu Asp Glu Trp Val Glu Lys Ala Glu Leu Glu
            340                 345                 350

Tyr Val Gln
        355

<210> 150
<211> 336
<212> PRT
<213> Streptococcus pneumoniae

<400> 150
Met Ile Lys Phe Asp Asn Ile Gln Ile Lys Tyr Gly Asp Phe Val Ala
1               5                   10                  15

Ile Asp Asn Leu Asn Leu Asp Ile His Glu Gly Glu Phe Phe Thr Phe
            20                  25                  30

Leu Gly Pro Ser Gly Cys Gly Lys Ser Thr Thr Leu Arg Ala Leu Val
        35                  40                  45

Gly Phe Leu Asp Pro Ser Ser Gly Ser Ile Glu Val Asn Gly Thr Asp
    50                  55                  60

Val Thr His Leu Glu Pro Glu Lys Arg Gly Ile Gly Ile Val Phe Gln
65                  70                  75                  80

Ser Tyr Ala Leu Phe Pro Thr Met Thr Val Phe Asp Asn Ile Ala Phe
                85                  90                  95

Gly Leu Lys Val Lys Lys Val Ala Pro Asp Val Ile Lys Ala Lys Val
            100                 105                 110

Ser Ala Val Ala Ala Lys Ile Lys Ile Ser Asp Gln Gln Leu Gln Arg
        115                 120                 125

Asn Val Ser Glu Leu Ser Gly Gly Gln Gln Gln Arg Val Ala Leu Ala
    130                 135                 140

Arg Ala Leu Val Leu Glu Pro Lys Ile Leu Cys Leu Asp Glu Pro Leu
145                 150                 155                 160

Ser Asn Leu Asp Ala Lys Leu Arg Val Asp Leu Arg Lys Glu Leu Lys
                165                 170                 175

Arg Leu Gln Lys Glu Leu Gly Ile Thr Thr Leu Tyr Val Thr His Asp
            180                 185                 190

Gln Glu Glu Ala Leu Thr Leu Ser Asp Arg Ile Ala Val Phe Asn Asn
        195                 200                 205

Gly Tyr Ile Glu Gln Val Gly Thr Pro Val Glu Ile Tyr His Asn Ser
    210                 215                 220

Gln Thr Glu Phe Val Cys Asp Phe Ile Gly Asp Ile Asn Val Leu Thr
225                 230                 235                 240

Asp Glu Thr Val His Glu Val Leu Leu Lys Asn Thr Ser Val Phe Leu
                245                 250                 255

Glu Asp Lys Lys Gly Tyr Ile Arg Leu Glu Lys Val Arg Phe Asn Arg
            260                 265                 270

Glu Thr Glu Gln Asp Phe Ile Leu Lys Gly Thr Ile Ile Asp Val Glu
        275                 280                 285

Phe Ser Gly Val Thr Ile His Tyr Thr Ile Lys Val Ser Glu Ser Gln
    290                 295                 300

Ile Leu Asn Val Thr Ser Ile Asp Ser Gln Ala Ala Ile Arg Ser Val
305                 310                 315                 320

Gly Glu Ser Val Glu Leu Phe Ile Thr Pro Ser Asp Val Leu Gln Phe
                325                 330                 335

<210> 151
<211> 563
<212> PRT
<213> Streptococcus pneumoniae

<400> 151
Met Arg His Lys Leu Asn Leu Lys Asp Trp Leu Ile Arg Leu Gly Leu
  1               5                  10                  15

Ile Trp Phe Leu Val Thr Phe Ile Ile Tyr Pro Asn Phe Asp Leu Val
             20                  25                  30

Val Asn Val Phe Val Lys Gly Gly Glu Phe Ser Leu Asp Ala Val His
         35                  40                  45

Arg Val Leu Lys Ser Gln Arg Ala Leu Gln Ser Ile Met Asn Ser Phe
     50                  55                  60

Lys Leu Ala Phe Ser Leu Ile Ile Thr Val Asn Val Val Gly Ile Leu
65                  70                  75                  80

Cys Val Leu Phe Thr Glu Tyr Phe Asp Ile Lys Gly Ala Lys Ile Leu
                85                  90                  95

Lys Leu Gly Tyr Met Thr Ser Leu Ile Tyr Gly Gly Val Val Leu Ala
            100                 105                 110

Thr Gly Tyr Lys Phe Val Tyr Gly Pro Tyr Gly Leu Ile Thr Lys Phe
        115                 120                 125

Leu Gln Asn Val Ile Pro Ser Leu Asp Pro Asn Trp Phe Ile Gly Tyr
    130                 135                 140

Gly Ala Val Leu Phe Ile Met Thr Phe Ser Gly Thr Ala Asn His Thr
145                 150                 155                 160

Leu Phe Leu Thr Asn Thr Ile Arg Ser Val Asp Tyr His Thr Ile Glu
                165                 170                 175

Ala Ala Arg Asn Met Gly Ala Lys Pro Phe Thr Val Phe Arg Lys Val
            180                 185                 190

Val Leu Pro Thr Leu Ile Pro Thr Leu Phe Ala Leu Thr Ile Met Val
        195                 200                 205

Phe Leu Ser Gly Leu Ser Ala Val Ala Ala Pro Met Ile Val Gly Gly
    210                 215                 220

Lys Glu Phe Gln Thr Ile Asn Pro Met Ile Ile Thr Phe Ala Gly Met
225                 230                 235                 240

Gly Asn Ser Arg Asp Leu Ala Ala Leu Leu Ala Ile Ile Leu Gly Ile
                245                 250                 255

Ala Thr Thr Ile Leu Leu Thr Ile Met Asn Lys Ile Glu Lys Gly Gly
            260                 265                 270

Asn Tyr Ile Ser Ile Ser Lys Thr Lys Ala Pro Leu Lys Lys Gln Lys
        275                 280                 285

Ile Ala Ser Lys Pro Trp Asn Ile Ile Ala His Ile Val Ala Tyr Gly
    290                 295                 300

Leu Phe Thr Val Phe Met Leu Pro Leu Ile Phe Ile Val Leu Tyr Ser
305                 310                 315                 320

Phe Thr Asp Pro Val Ala Ile Gln Thr Gly Asn Leu Thr Leu Ser Asn
                325                 330                 335

Phe Thr Leu Glu Asn Tyr Arg Leu Phe Phe Ser Asn Ser Ala Ala Phe
            340                 345                 350

Ser Pro Phe Leu Val Ser Phe Ile Tyr Ser Ile Ile Ala Ala Thr Thr
        355                 360                 365

Ala Thr Ile Leu Ala Val Val Phe Ala Arg Val Val Arg Lys His Lys
    370                 375                 380

Ser Arg Phe Asp Phe Leu Phe Glu Tyr Gly Ala Leu Leu Pro Trp Leu
385                 390                 395                 400

Leu Pro Ser Thr Leu Leu Ala Val Ser Leu Leu Phe Thr Phe Asn Gln
                405                 410                 415

Pro Gln Phe Leu Val Leu Asn Gln Ile Leu Val Gly Ser Leu Val Ile
            420                 425                 430

Leu Leu Ile Ala Tyr Ile Val Val Lys Ile Pro Phe Ser Tyr Arg Met
        435                 440                 445

Val Arg Ala Ile Leu Phe Ser Val Asp Asp Glu Met Glu Asp Ala Ala
    450                 455                 460

Arg Ser Met Gly Ala Ser Pro Phe Tyr Thr Met Met Lys Val Ile Ile
465                 470                 475                 480

Pro Phe Ile Leu Pro Val Val Leu Ser Val Ile Ala Leu Asn Phe Asn
                485                 490                 495

Ser Leu Leu Thr Asp Phe Asp Leu Ser Val Phe Leu Tyr His Pro Leu
            500                 505                 510

Ala Gln Pro Leu Gly Ile Thr Ile Arg Ser Ala Gly Asp Glu Thr Ala
        515                 520                 525

Thr Ser Asn Ala Gln Ala Leu Val Phe Val Tyr Thr Ile Val Leu Met
    530                 535                 540

Ile Ile Ser Gly Thr Val Leu Tyr Phe Thr Gln Arg Pro Gly Arg Lys
545                 550                 555                 560

Val Arg Lys

<210> 152
<211> 443
<212> PRT
<213> Streptococcus pneumoniae

<400> 152
Met Ile Lys Asn Pro Lys Leu Leu Thr Lys Ser Phe Leu Arg Ser Phe
1               5                   10                  15

Ala Ile Leu Gly Gly Val Gly Leu Val Ile His Ile Ala Ile Tyr Leu
            20                  25                  30

Thr Phe Pro Phe Tyr Tyr Ile Gln Leu Glu Gly Glu Lys Phe Asn Glu
        35                  40                  45

Ser Ala Arg Val Phe Thr Glu Tyr Leu Lys Thr Lys Thr Ser Asp Glu
    50                  55                  60

Ile Pro Ser Leu Leu Gln Ser Tyr Ser Lys Ser Leu Thr Ile Ser Ala
65                  70                  75                  80

His Leu Lys Arg Asp Ile Val Asp Lys Arg Leu Pro Leu Val His Asp
                85                  90                  95

Leu Asp Ile Lys Asp Gly Lys Leu Ser Asn Tyr Ile Val Met Leu Asp
            100                 105                 110

Met Ser Val Ser Thr Ala Asp Gly Lys Gln Val Thr Val Gln Phe Val
        115                 120                 125

His Gly Val Asp Val Tyr Lys Glu Ala Lys Asn Ile Leu Leu Leu Tyr
    130                 135                 140

Leu Pro Tyr Thr Phe Leu Val Thr Ile Ala Phe Ser Phe Val Phe Ser
145                 150                 155                 160

Tyr Phe Tyr Thr Lys Arg Leu Leu Asn Pro Leu Phe Tyr Ile Ser Glu
                165                 170                 175

Val Thr Ser Lys Met Gln Asp Leu Asp Asp Asn Ile Arg Phe Asp Glu
            180                 185                 190

Ser Arg Lys Asp Glu Val Gly Glu Val Gly Lys Gln Ile Asn Gly Met
        195                 200                 205

Tyr Glu His Leu Leu Lys Val Ile Tyr Glu Leu Glu Ser Arg Asn Glu
    210                 215                 220

Gln Ile Val Lys Leu Gln Asn Gln Lys Val Ser Phe Val Arg Gly Ala
225                 230                 235                 240

Ser His Glu Leu Lys Thr Pro Leu Ala Ser Leu Arg Ile Ile Leu Glu
                245                 250                 255

Asn Met Gln His Asn Ile Gly Asp Tyr Lys Asp His Pro Lys Tyr Ile
            260                 265                 270

Ala Lys Ser Ile Asn Lys Ile Asp Gln Met Ser His Leu Leu Glu Glu
        275                 280                 285

Val Leu Glu Ser Ser Lys Phe Gln Glu Trp Thr Glu Cys Arg Glu Thr
    290                 295                 300

Leu Thr Val Lys Pro Val Leu Val Asp Ile Leu Ser Arg Tyr Gln Glu
305                 310                 315                 320

Leu Ala His Ser Ile Gly Val Thr Ile Glu Asn Gln Leu Thr Asp Ala
                325                 330                 335

Thr Arg Val Val Met Ser Leu Arg Ala Leu Asp Lys Val Leu Thr Asn
            340                 345                 350

Leu Ile Ser Asn Ala Ile Lys Tyr Ser Asp Lys Asn Gly Arg Val Ile
        355                 360                 365

Ile Ser Glu Gln Asp Gly Tyr Leu Ser Ile Lys Asn Thr Cys Ala Pro
    370                 375                 380

Leu Ser Asp Gln Glu Leu Glu His Leu Phe Asp Ile Phe Tyr His Ser
385                 390                 395                 400

Gln Ile Val Thr Asp Lys Asp Glu Ser Ser Gly Leu Gly Leu Tyr Ile
                405                 410                 415

Val Asn Asn Ile Leu Glu Ser Tyr Gln Met Asp Tyr Ser Phe Leu Pro
            420                 425                 430

Tyr Glu His Gly Met Glu Phe Lys Ile Ser Leu
        435                 440

<210> 153
<211> 290
<212> PRT
<213> Streptococcus pneumoniae

<400> 153
Met Asp Lys Ile Ile Lys Thr Ile Ser Glu Ser Gly Ala Phe Arg Ala
1               5                   10                  15

Phe Val Leu Asp Ser Thr Glu Thr Val Arg Thr Ala Gln Glu Lys His
            20                  25                  30

Gln Thr Gln Ala Ser Ser Thr Val Ala Leu Gly Arg Thr Leu Ile Ala
        35                  40                  45

Ser Gln Ile Leu Ala Ala Asn Glu Lys Gly Asn Thr Lys Leu Thr Val
    50                  55                  60

Lys Val Leu Gly Ser Ser Ser Leu Gly Ala Ile Ile Thr Val Ala Asp
65                  70                  75                  80

Thr Lys Gly Asn Val Lys Gly Tyr Val Gln Asn Pro Gly Val Asp Ile
                85                  90                  95

Lys Lys Thr Ala Thr Gly Glu Val Leu Val Gly Pro Phe Val Gly Asn
            100                 105                 110

Gly Gln Phe Leu Val Ile Thr Asp Tyr Gly Thr Gly Asn Pro Tyr Asn
        115                 120                 125

Ser Ile Thr Pro Leu Ile Ser Gly Glu Ile Gly Glu Asp Leu Ala Phe
    130                 135                 140

Tyr Leu Thr Glu Ser Gln Gln Thr Pro Ser Ala Val Gly Leu Asn Val
145                 150                 155                 160

Leu Leu Asp Glu Glu Asp Lys Val Lys Val Ala Gly Gly Phe Leu Val
                165                 170                 175

Gln Val Leu Pro Gly Ala Lys Lys Glu Glu Ile Ala Arg Phe Glu Lys
            180                 185                 190

Arg Ile Gln Glu Met Pro Ala Ile Ser Thr Leu Leu Glu Ser Asp Asp
        195                 200                 205

His Ile Glu Ala Leu Leu Lys Ala Ile Tyr Gly Asp Glu Ala Tyr Lys
    210                 215                 220

Arg Leu Ser Glu Glu Glu Ile Arg Phe Gln Cys Asp Cys Ser His Glu
225                 230                 235                 240

Arg Phe Met Asn Ala Leu Ala Ser Leu Pro Ser Ser Asp Leu Gln Glu
                245                 250                 255

Met Lys Glu Glu Asp His Gly Ala Glu Ile Thr Cys Gln Phe Cys Gln
            260                 265                 270

Thr Thr Tyr Asn Phe Asp Glu Lys Asp Leu Glu Glu Leu Ile Arg Asp
        275                 280                 285

Lys Ser
    290

<210> 154
<211> 152
<212> PRT
<213> Streptococcus pneumoniae

<400> 154
Met Lys Ser Ile Thr Lys Lys Ile Lys Ala Thr Leu Ala Gly Val Ala
1               5                   10                  15

Ala Leu Phe Ala Val Phe Ala Pro Ser Phe Val Ser Ala Gln Glu Ser
            20                  25                  30

Ser Thr Tyr Thr Val Lys Glu Gly Asp Thr Leu Ser Glu Ile Ala Glu
        35                  40                  45

Thr His Asn Thr Thr Val Glu Lys Leu Ala Glu Asn Asn His Ile Asp
    50                  55                  60

Asn Ile His Leu Ile Tyr Val Asp Gln Glu Leu Val Ile Asp Gly Pro
65                  70                  75                  80

Val Ala Pro Val Ala Thr Pro Ala Pro Ala Thr Tyr Ala Ala Pro Ala
                85                  90                  95

Ala Gln Asp Glu Thr Val Ser Ala Pro Val Ala Glu Thr Pro Val Val
            100                 105                 110

Ser Glu Thr Val Val Ser Thr Val Ser Gly Ser Glu Ala Glu Ala Lys
        115                 120                 125

Glu Trp Ile Ala Gln Lys Glu Ser Gly Gly Ser Ile Gln Leu Gln Met
    130                 135                 140

Asp Val Ile Ser Asp Val Thr Asn
145                 150

<210> 155
<211> 677
<212> PRT
<213> Streptococcus pneumoniae

<400> 155
Met Asn Leu Gly Glu Phe Trp Tyr Asn Lys Ile Asn Lys Asn Arg Gly
1               5                   10                  15

Arg Arg Leu Met Lys Lys Val Arg Phe Ile Phe Leu Ala Leu Leu Phe
            20                  25                  30

Phe Leu Ala Ser Pro Glu Gly Ala Met Ala Ser Asp Gly Thr Trp Gln
        35                  40                  45

Gly Lys Gln Tyr Leu Lys Glu Asp Gly Ser Gln Ala Ala Asn Glu Trp
    50                  55                  60

Val Phe Asp Thr His Tyr Gln Ser Trp Phe Tyr Ile Lys Ala Asp Ala
65                  70                  75                  80

Asn Tyr Ala Glu Asn Glu Trp Leu Lys Gln Gly Asp Asp Tyr Phe Tyr
                85                  90                  95

Leu Lys Ser Gly Gly Tyr Met Ala Lys Ser Glu Trp Val Glu Asp Lys
            100                 105                 110

Gly Ala Phe Tyr Tyr Leu Asp Gln Asp Gly Lys Met Lys Arg Asn Ala
        115                 120                 125

Trp Val Gly Thr Ser Tyr Val Gly Ala Thr Gly Ala Lys Val Ile Glu
    130                 135                 140

Asp Trp Val Tyr Asp Ser Gln Tyr Asp Ala Trp Phe Tyr Ile Lys Ala
145                 150                 155                 160

Asp Gly Gln His Ala Glu Lys Glu Trp Leu Gln Ile Lys Gly Lys Asp
                165                 170                 175

Tyr Tyr Phe Lys Ser Gly Gly Tyr Leu Leu Thr Ser Gln Trp Ile Asn
            180                 185                 190

Gln Ala Tyr Val Asn Ala Ser Gly Ala Lys Val Gln Gln Gly Trp Leu
        195                 200                 205

Phe Asp Lys Gln Tyr Gln Ser Trp Phe Tyr Ile Lys Glu Asn Gly Asn
    210                 215                 220

Tyr Ala Asp Lys Glu Trp Ile Phe Glu Asn Gly His Tyr Tyr Tyr Leu
225                 230                 235                 240

Lys Ser Gly Gly Tyr Met Ala Ala Asn Glu Trp Ile Trp Asp Lys Glu
                245                 250                 255

Ser Trp Phe Tyr Leu Lys Phe Asp Gly Lys Met Ala Glu Lys Glu Trp
            260                 265                 270

Val Tyr Asp Ser His Ser Gln Ala Trp Tyr Tyr Phe Lys Ser Gly Gly
        275                 280                 285

Tyr Met Thr Ala Asn Glu Trp Ile Trp Asp Lys Glu Ser Trp Phe Tyr
    290                 295                 300

Leu Lys Ser Asp Gly Lys Ile Ala Glu Lys Glu Trp Val Tyr Asp Ser
305                 310                 315                 320

His Ser Gln Ala Trp Tyr Tyr Phe Lys Ser Gly Gly Tyr Met Thr Ala
                325                 330                 335

Asn Glu Trp Ile Trp Asp Lys Glu Ser Trp Phe Tyr Leu Lys Ser Asp
            340                 345                 350

Gly Lys Ile Ala Glu Lys Glu Trp Val Tyr Asp Ser His Ser Gln Ala
        355                 360                 365

Trp Tyr Tyr Phe Lys Ser Gly Gly Tyr Met Ala Lys Asn Glu Thr Val
    370                 375                 380

Asp Gly Tyr Gln Leu Gly Ser Asp Gly Lys Trp Leu Gly Gly Lys Thr
385                 390                 395                 400

Thr Asn Glu Asn Ala Ala Tyr Tyr Gln Val Val Pro Val Thr Ala Asn
                405                 410                 415

Val Tyr Asp Ser Asp Gly Glu Lys Leu Ser Tyr Ile Ser Gln Gly Ser
            420                 425                 430

Val Val Trp Leu Asp Lys Asp Arg Lys Ser Asp Asp Lys Arg Leu Ala
        435                 440                 445

Ile Thr Ile Ser Gly Leu Ser Gly Tyr Met Lys Thr Glu Asp Leu Gln
    450                 455                 460

Ala Leu Asp Ala Ser Lys Asp Phe Ile Pro Tyr Tyr Glu Ser Asp Gly
465                 470                 475                 480

His Arg Phe Tyr His Tyr Val Ala Gln Asn Ala Ser Ile Pro Val Ala
                485                 490                 495

Ser His Leu Ser Asp Met Glu Val Gly Lys Lys Tyr Tyr Ser Ala Asp
            500                 505                 510

Gly Leu His Phe Asp Gly Phe Lys Leu Glu Asn Pro Phe Leu Phe Lys
        515                 520                 525

Asp Leu Thr Glu Ala Thr Asn Tyr Ser Ala Glu Glu Leu Asp Lys Val
    530                 535                 540

Phe Ser Leu Leu Asn Ile Asn Asn Ser Leu Leu Glu Asn Lys Gly Ala
545                 550                 555                 560

Thr Phe Lys Glu Ala Glu Glu His Tyr His Ile Asn Ala Leu Tyr Leu
                565                 570                 575

Leu Ala His Ser Ala Leu Glu Ser Asn Trp Gly Arg Ser Lys Ile Ala
            580                 585                 590

Lys Asp Lys Asn Asn Phe Phe Gly Ile Thr Ala Tyr Asp Thr Thr Pro
        595                 600                 605

Tyr Leu Ser Ala Lys Thr Phe Asp Asp Val Asp Lys Gly Ile Leu Gly
    610                 615                 620

Ala Thr Lys Trp Ile Lys Glu Asn Tyr Ile Asp Arg Gly Arg Thr Phe
625                 630                 635                 640

Leu Gly Asn Lys Ala Ser Gly Met Asn Val Glu Tyr Ala Ser Asp Pro
                645                 650                 655

Tyr Trp Gly Glu Lys Ile Ala Ser Val Met Met Lys Ile Asn Glu Lys
            660                 665                 670

Leu Gly Gly Lys Asp

        675

<210> 156
<211> 309
<212> PRT
<213> Streptococcus pneumoniae

<400> 156
Met Lys Lys Leu Gly Thr Leu Leu Val Leu Phe Leu Ser Ala Ile Ile
1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asn Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr
                165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210> 157
<211> 419
<212> PRT
<213> Streptococcus pneumoniae

<400> 157
Met Glu Trp Tyr Lys Lys Ile Gly Leu Leu Ala Thr Thr Gly Leu Ala
1               5                   10                  15

Leu Phe Gly Leu Gly Ala Cys Ser Asn Tyr Gly Lys Ser Ala Asp Gly
            20                  25                  30

Thr Val Thr Ile Glu Tyr Phe Asn Gln Lys Lys Glu Met Thr Lys Thr
        35                  40                  45

Leu Glu Glu Ile Thr Arg Asp Phe Glu Lys Glu Asn Pro Lys Ile Lys
    50                  55                  60

Val Lys Val Val Asn Val Pro Asn Ala Gly Glu Val Leu Lys Thr Arg
65                  70                  75                  80

Val Leu Ala Gly Asp Val Pro Asp Val Val Asn Ile Tyr Pro Gln Ser
                85                  90                  95

Ile Glu Leu Gln Glu Trp Ala Lys Ala Gly Val Phe Glu Asp Leu Ser
            100                 105                 110

Asn Lys Asp Tyr Leu Lys Arg Val Lys Asn Gly Tyr Ala Glu Lys Tyr
        115                 120                 125

Ala Val Asn Glu Lys Val Tyr Asn Val Pro Phe Thr Ala Asn Ala Tyr
    130                 135                 140

Gly Ile Tyr Tyr Asn Lys Asp Lys Phe Glu Glu Leu Gly Leu Lys Val
145                 150                 155                 160

Pro Glu Thr Trp Asp Glu Phe Glu Gln Leu Val Lys Asp Ile Val Ala
                165                 170                 175

Lys Gly Gln Thr Pro Phe Gly Ile Ala Gly Ala Asp Ala Trp Thr Leu
            180                 185                 190

Asn Gly Tyr Asn Gln Leu Ala Phe Ala Thr Ala Thr Gly Gly Gly Lys
        195                 200                 205

Glu Ala Asn Gln Tyr Leu Arg Tyr Ser Gln Pro Asn Ala Ile Lys Leu
    210                 215                 220

Ser Asp Pro Ile Met Lys Asp Asp Ile Lys Val Met Asp Ile Leu Arg
225                 230                 235                 240

Ile Asn Gly Ser Lys Gln Lys Asn Trp Glu Gly Ala Gly Tyr Thr Asp
                245                 250                 255

Val Ile Gly Ala Phe Ala Arg Gly Asp Val Leu Met Thr Pro Asn Gly
            260                 265                 270

Ser Trp Ala Ile Thr Ala Ile Asn Glu Gln Lys Pro Asn Phe Lys Ile
        275                 280                 285

Gly Thr Phe Met Ile Pro Gly Lys Glu Lys Gly Gln Ser Leu Thr Val
    290                 295                 300

Gly Ala Gly Asp Leu Ala Trp Ser Ile Ser Ala Thr Thr Lys His Pro
305                 310                 315                 320

Lys Glu Ala Asn Ala Phe Val Glu Tyr Met Thr Arg Pro Glu Val Met
                325                 330                 335

Gln Lys Tyr Tyr Asp Val Asp Gly Ser Pro Thr Ala Ile Glu Gly Val
            340                 345                 350

Lys Gln Ala Gly Glu Asp Ser Pro Leu Ala Gly Met Thr Glu Tyr Ala
        355                 360                 365

Phe Thr Asp Arg His Leu Val Trp Leu Gln Gln Tyr Trp Thr Ser Glu
    370                 375                 380

Ala Asp Phe His Thr Leu Thr Met Asn Tyr Val Leu Thr Gly Asp Lys
385                 390                 395                 400

Gln Gly Met Val Asn Asp Leu Asn Ala Phe Phe Asn Pro Met Lys Ala
                405                 410                 415

Asp Val Asp

<210> 158
<211> 559
<212> PRT
<213> Streptococcus pneumoniae

<400> 158
Met Lys Lys Ile Lys Pro His Gly Pro Leu Pro Ser Gln Thr Gln Leu
  1               5                  10                  15

Ala Tyr Leu Gly Asp Glu Leu Ala Ala Phe Ile His Phe Gly Pro Asn
             20                  25                  30

Thr Phe Tyr Asp Gln Glu Trp Gly Thr Gly Gln Glu Asp Pro Glu Arg
         35                  40                  45

Phe Asn Pro Ser Gln Leu Asp Ala Arg Glu Trp Val Arg Val Leu Lys
     50                  55                  60

Glu Thr Gly Phe Lys Lys Leu Ile Leu Val Val Lys His His Asp Gly
 65                  70                  75                  80

Phe Val Leu Tyr Pro Thr Ala His Thr Asp Tyr Ser Val Lys Val Ser

                85                  90                  95

Pro Trp Arg Arg Gly Lys Gly Asp Leu Leu Leu Glu Val Ser Gln Ala
            100                 105                 110

Ala Thr Glu Phe Asp Met Asp Met Gly Val Tyr Leu Ser Pro Trp Asp
        115                 120                 125

Ala His Ser Pro Leu Tyr His Val Asp Arg Glu Ala Asp Tyr Asn Ala
    130                 135                 140

Tyr Tyr Leu Ala Gln Leu Lys Glu Ile Leu Ser Asn Pro Asn Tyr Gly
145                 150                 155                 160

Asn Ala Gly Lys Phe Ala Glu Val Trp Met Asp Gly Ala Arg Gly Glu
                165                 170                 175

Gly Ala Gln Lys Val Asn Tyr Glu Phe Glu Lys Trp Phe Glu Thr Ile
            180                 185                 190

Arg Asp Leu Gln Gly Asp Cys Leu Ile Phe Ser Thr Glu Gly Thr Ser
        195                 200                 205

Ile Arg Trp Ile Gly Asn Glu Arg Gly Tyr Ala Gly Asp Pro Leu Trp
    210                 215                 220

Gln Lys Val Asn Pro Asp Lys Leu Gly Thr Glu Ala Glu Leu Asn Tyr
225                 230                 235                 240

Leu Gln His Gly Asp Pro Ser Gly Thr Ile Phe Ser Ile Gly Glu Ala
                245                 250                 255

Asp Val Ser Ile Arg Pro Gly Trp Phe Tyr His Glu Asp Gln Asp Pro
            260                 265                 270

Lys Ser Leu Glu Glu Leu Val Glu Ile Tyr Phe His Ser Val Gly Arg
        275                 280                 285

Gly Thr Pro Leu Leu Leu Asn Ile Pro Pro Asn Gln Ala Gly Leu Phe
    290                 295                 300

Asp Ala Lys Asp Ile Glu Arg Leu Tyr Glu Phe Ala Thr Tyr Arg Asn
305                 310                 315                 320

Glu Leu Tyr Lys Glu Asp Leu Ala Leu Gly Ala Glu Val Ser Gly Pro
                325                 330                 335

Ala Leu Ser Ala Asp Phe Ala Cys Arg His Leu Thr Asp Gly Leu Glu
            340                 345                 350

Thr Ser Ser Trp Ala Ser Asp Ala Asp Leu Pro Ile Gln Leu Glu Leu
        355                 360                 365

Asp Leu Gly Ser Pro Lys Thr Phe Asp Val Ile Glu Leu Arg Glu Asp
    370                 375                 380

Leu Lys Leu Gly Gln Arg Ile Ala Ala Phe His Val Gln Val Glu Val
385                 390                 395                 400

Asp Gly Val Trp Gln Glu Phe Gly Ser Gly His Thr Val Gly Tyr Lys
                405                 410                 415

Arg Leu Leu Arg Gly Ala Val Val Glu Ala Gln Lys Ile Arg Val Val
            420                 425                 430

Ile Thr Glu Ser Gln Ala Leu Pro Leu Leu Thr Lys Ile Ser Leu Tyr
        435                 440                 445

Lys Thr Pro Gly Leu Ser Lys Lys Glu Val Val Gln Glu Leu Ala Phe
    450                 455                 460

Ala Glu Lys Ser Leu Ala Val Ala Lys Gly Glu Asn Ala Tyr Phe Thr
465                 470                 475                 480

Val Lys Arg Arg Glu Cys Ser Gly Pro Leu Glu Ala Lys Ile Ser Ile
                485                 490                 495

Gln Pro Gly Thr Gly Val His Gly Val Ala Tyr Gln Asp Glu Ile Gln
            500                 505                 510

Val Leu Ala Phe Gln Thr Gly Glu Thr Glu Lys Ser Leu Thr Leu Pro
        515                 520                 525

Thr Leu Tyr Phe Ala Gly Asp Lys Thr Leu Asp Phe Tyr Leu Asn Leu
    530                 535                 540

Thr Val Asp Gly Gln Leu Val Asp Gln Leu Gln Val Gln Val Ser
545                 550                 555

<210> 159
<211> 227
<212> PRT
<213> Streptococcus pneumoniae

<400> 159
Met Leu Glu Arg Leu Lys Arg Ile His Tyr Met Phe Trp Ile Ser Leu
  1               5                  10                  15

Ile Phe Met Ile Phe Pro Ile Leu Ser Val Val Thr Gly Trp Leu Ser
             20                  25                  30

Ala Trp His Leu Leu Ile Asp Ile Leu Phe Val Val Ala Tyr Leu Gly
         35                  40                  45

Val Leu Thr Thr Lys Ser Gln Arg Leu Ser Trp Leu Tyr Trp Gly Leu
     50                  55                  60

Met Leu Thr Tyr Val Val Gly Asn Thr Ala Phe Val Ala Val Asn Tyr
 65                  70                  75                  80

Ile Trp Phe Phe Phe Phe Leu Ser Asn Leu Leu Ser Tyr His Phe Ser
                 85                  90                  95

Val Arg Ser Leu Lys Ser Leu His Val Trp Thr Phe Leu Leu Ala Gln
            100                 105                 110

Val Leu Val Val Gly Gln Leu Leu Ile Phe Gln Arg Ile Glu Val Glu
        115                 120                 125

Phe Leu Phe Tyr Leu Leu Val Ile Leu Thr Phe Val Asp Leu Met Thr

130                 135                 140

Phe Gly Leu Val Arg Ile Arg Ile Val Glu Asp Leu Lys Glu Ala Gln
145                 150                 155                 160

Val Lys Gln Asn Ala Gln Ile Asn Leu Leu Leu Ala Glu Asn Glu Arg
                165                 170                 175

Ser Arg Ile Gly Gln Asp Leu His Asp Ser Leu Gly His Thr Phe Ala
            180                 185                 190

Met Leu Ser Val Lys Thr Asp Leu Ala Leu Gln Leu Phe Gln Met Glu
        195                 200                 205

Ala Tyr Pro Gln Val Glu Lys Glu Leu Lys Glu Ile His Gln Ile Ser
    210                 215                 220

Lys Asp Pro
225

<210> 160
<211> 370
<212> PRT
<213> Streptococcus pneumoniae

<400> 160
Met Asn Asp Lys Leu Lys Ile Phe Leu Leu Leu Gly Val Phe Phe Leu
1               5                   10                  15

Ala Ile Thr Gly Phe Tyr Val Leu Leu Ile Arg Asn Ala Gly Gln Thr
            20                  25                  30

Asp Ala Ser Gln Ile Glu Lys Ala Ala Val Ser Gln Gly Gly Lys Ala
        35                  40                  45

Val Lys Lys Thr Glu Ile Ser Lys Asp Ala Asp Leu His Glu Ile Tyr
    50                  55                  60

Leu Ala Gly Gly Cys Phe Trp Gly Val Glu Glu Tyr Phe Ser Arg Val
65                  70                  75                  80

Pro Gly Val Thr Asp Ala Val Ser Gly Tyr Ala Asn Gly Arg Gly Glu
                85                  90                  95

Thr Thr Lys Tyr Glu Leu Ile Asn Gln Thr Gly His Ala Glu Thr Val
            100                 105                 110

His Val Thr Tyr Asp Ala Lys Gln Ile Ser Leu Lys Glu Ile Leu Leu
        115                 120                 125

His Tyr Phe Arg Ile Ile Asn Pro Thr Ser Lys Asn Lys Gln Gly Asn
    130                 135                 140

Asp Val Gly Thr Gln Tyr Arg Thr Gly Val Tyr Tyr Thr Asp Asp Lys
145                 150                 155                 160

Asp Leu Glu Val Ile Asn Gln Val Phe Asp Glu Val Ala Lys Lys Tyr
                165                 170                 175

Asp Gln Pro Leu Ala Val Glu Lys Glu Asn Leu Lys Asn Phe Val Val
            180                 185                 190

Ala Glu Asp Tyr His Gln Asp Tyr Leu Lys Lys Asn Pro Asn Gly Tyr
        195                 200                 205

Cys His Ile Asn Val Asn Gln Ala Ala Tyr Pro Val Ile Asp Ala Ser
    210                 215                 220

Lys Tyr Pro Lys Pro Ser Asp Glu Glu Leu Lys Lys Thr Leu Ser Pro
225                 230                 235                 240

Glu Glu Tyr Ala Val Thr Gln Glu Asn Gln Thr Glu Arg Ala Phe Ser
                245                 250                 255

Asn Arg Tyr Trp Asp Lys Phe Glu Ser Gly Ile Tyr Val Asp Ile Ala
            260                 265                 270

Thr Gly Glu Pro Leu Phe Ser Ser Lys Asp Lys Phe Glu Ser Gly Cys
        275                 280                 285

Gly Trp Pro Ser Phe Thr Gln Pro Ile Ser Pro Asp Val Val Thr Tyr
    290                 295                 300

Lys Glu Asp Lys Ser Tyr Asn Met Thr Arg Met Glu Val Arg Ser Arg
305                 310                 315                 320

Val Gly Asp Ser His Leu Gly His Val Phe Thr Asp Gly Pro Gln Asp
                325                 330                 335

Lys Gly Gly Leu Arg Tyr Cys Ile Asn Ser Leu Ser Ile Arg Phe Ile
            340                 345                 350

Pro Lys Asp Gln Met Glu Glu Lys Gly Tyr Ala Tyr Leu Leu Asp Tyr
        355                 360                 365

Val Asp
    370

<210> 161
<211> 338
<212> PRT
<213> Streptococcus pneumoniae

<400> 161
Met Lys Lys Lys Trp Met Tyr Tyr Ala Ala Cys Ser Ser Asn Glu Ser
1               5                   10                  15

Ala Asp Asp Ser Ser Ser Asp Lys Gly Asp Gly Gly Ser Leu Val Val
            20                  25                  30

Tyr Ser Pro Asn Ser Glu Gly Leu Ile Gly Ala Thr Ile Pro Ala Phe
        35                  40                  45

Glu Glu Lys Tyr Gly Ile Lys Val Glu Leu Ile Gln Ala Gly Thr Gly
    50                  55                  60

Glu Leu Phe Lys Lys Leu Glu Ser Glu Lys Glu Val Pro Val Ala Asp
65                  70                  75                  80

Val Ile Phe Gly Gly Ser Tyr Thr Gln Tyr Thr Thr His Gly Glu Leu
                85                  90                  95

Phe Glu Asn Tyr Thr Ser Lys Glu Asn Asp Asn Val Ile Lys Glu Tyr
            100                 105                 110

Gln Asn Thr Thr Gly Tyr Ser Thr Pro Tyr Thr Leu Asp Gly Ser Val
        115                 120                 125

Leu Ile Val Asn Pro Asp Leu Thr Lys Gly Met Asn Ile Glu Gly Tyr
    130                 135                 140

Asn Asp Leu Phe Lys Pro Glu Leu Lys Gly Lys Ile Ala Thr Ala Asp
145                 150                 155                 160

Pro Ala Asn Ser Ser Ser Ala Phe Ala Gln Leu Thr Asn Met Leu Gln
                165                 170                 175

Ala Gln Gly Gly Tyr Lys Asp Asp Lys Ala Trp Ser Tyr Val Lys Asp
            180                 185                 190

Leu Phe Thr Leu Ile Asp Gly Lys Ile Gly Ser Ser Ser Ser Ser Val
        195                 200                 205

Tyr Lys Val Val Ala Asp Gly Glu Met Ala Val Gly Leu Ser Tyr Glu
    210                 215                 220

Asp Pro Ala Val Lys Leu Leu Asn Asp Gly Ala Asn Ile Lys Val Val
225                 230                 235                 240

Tyr Pro Lys Glu Gly Thr Val Phe Leu Pro Ala Ser Ala Ala Ile Val
                245                 250                 255

Lys Lys Ser Lys Asn Met Glu Asn Ala Lys Lys Phe Ile Asp Phe Ile
            260                 265                 270

Ile Ser Gln Glu Val Gln Asp Thr Leu Gly Thr Thr Thr Thr Asn Arg
        275                 280                 285

Pro Val Arg Lys Asn Ala Lys Thr Ser Glu Asn Met Lys Pro Ile Asp
    290                 295                 300

Lys Ile Lys Thr Leu Thr Glu Asp Tyr Asp Tyr Val Ile Lys Asn Lys
305                 310                 315                 320

Ser Asp Ile Val Lys Lys Tyr Asn Glu Val Phe Thr Asp Ile Gln Ser
                325                 330                 335

Lys Gln

<210> 162
<211> 363
<212> PRT
<213> Streptococcus pneumoniae

<400> 162
Met Ser Glu Ile Lys Ile Ile Asn Ala Lys Lys Ile Tyr His Asp Val

1               5                   10                  15

Pro Val Ile Glu Asn Leu Asn Ile Thr Ile Pro Lys Gly Ser Leu Phe
                20                  25                  30

Thr Leu Leu Gly Ala Ser Gly Cys Gly Lys Thr Thr Leu Leu Arg Met
            35                  40                  45

Ile Ala Gly Phe Asn Ser Ile Glu Gly Gly Glu Phe Tyr Phe Asp Asp
    50                  55                  60

Thr Lys Ile Asn Asn Met Glu Pro Ser Lys Arg Asn Ile Gly Met Val
65                  70                  75                  80

Phe Gln Asn Tyr Ala Ile Phe Pro His Leu Thr Val Arg Asp Asn Val
                85                  90                  95

Ala Phe Gly Leu Met Gln Lys Lys Val Pro Lys Glu Glu Leu Ile Gln
            100                 105                 110

Gln Thr Asn Lys Tyr Leu Glu Leu Met Gln Ile Ala Gln Tyr Ala Asp
        115                 120                 125

Arg Lys Pro Asp Lys Leu Ser Gly Gly Gln Gln Gln Arg Val Thr Leu
    130                 135                 140

Ala Cys Ala Leu Ala Val Asn Pro Ser Val Leu Leu Met Asp Glu Pro
145                 150                 155                 160

Leu Ser Asn Leu Glu Ala Lys Leu Arg Leu Asp Met Arg Gln Ala Ile
                165                 170                 175

Arg Glu Ile Gln His Glu Val Gly Ile Thr Thr Val Tyr Val Thr His
            180                 185                 190

Asp Gln Glu Glu Ala Met Ala Ile Ser Asp Gln Ile Ala Val Met Lys
        195                 200                 205

Asp Gly Val Ile Gln Gln Ile Gly Arg Pro Lys Glu Leu Tyr His Lys
    210                 215                 220

Pro Ala Asn Glu Phe Val Ala Thr Phe Ile Gly Arg Thr Asn Ile Ile
225                 230                 235                 240

Pro Ala Asn Leu Glu Lys Arg Ser Asp Gly Ala Tyr Ile Val Phe Ser
                245                 250                 255

Asp Gly Tyr Ala Leu Arg Met Pro Ala Leu Asp Gln Val Glu Glu Gln
            260                 265                 270

Ala Ile His Val Ser Ile Arg Pro Glu Glu Phe Ile Lys Asp Glu Ser
        275                 280                 285

Gly Asp Ile Glu Gly Thr Ile Arg Asp Ser Val Tyr Leu Gly Leu Asn
    290                 295                 300

Thr Asp Tyr Phe Ile Glu Thr Gly Phe Ala Ser Lys Ile Gln Val Ser
305                 310                 315                 320

Glu Glu Ser Thr Phe Glu Glu Asp Leu Gln Lys Gly Asn Arg Ile Arg
                325                 330                 335

Leu Arg Ile Asn Thr Gln Lys Leu Asn Ile Phe Ser Ala Asp Gly Ser
            340                 345                 350

Gln Asn Leu Ile Lys Gly Val Asn His Gly Thr
        355                 360

<210> 163
<211> 313
<212> PRT
<213> Streptococcus pneumoniae

<400> 163
Met Lys Lys Lys Leu Leu Ala Gly Ala Ile Thr Leu Leu Ser Val Ala
1               5                   10                  15

Thr Leu Ala Ala Cys Ser Lys Gly Ser Glu Gly Ala Asp Leu Ile Ser
            20                  25                  30

Met Lys Gly Asp Val Ile Thr Glu His Gln Phe Tyr Glu Gln Val Lys
        35                  40                  45

Ser Asn Pro Ser Ala Gln Gln Val Leu Leu Asn Met Thr Ile Gln Lys
    50                  55                  60

Val Phe Glu Lys Gln Tyr Gly Ser Glu Leu Asp Asp Lys Glu Val Asp
65                  70                  75                  80

Asp Thr Ile Ala Glu Glu Lys Lys Gln Tyr Gly Glu Asn Tyr Gln Arg
                85                  90                  95

Val Leu Ser Gln Ala Gly Met Thr Leu Glu Thr Arg Lys Ala Gln Ile
            100                 105                 110

Arg Thr Ser Lys Leu Val Glu Leu Ala Val Lys Lys Val Ala Glu Ala
        115                 120                 125

Glu Leu Thr Asp Glu Ala Tyr Lys Lys Ala Phe Asp Glu Tyr Thr Pro
    130                 135                 140

Asp Val Thr Ala Gln Ile Ile Arg Leu Asn Asn Glu Asp Lys Ala Lys
145                 150                 155                 160

Glu Val Leu Glu Lys Ala Lys Ala Glu Gly Ala Asp Phe Ala Gln Leu
                165                 170                 175

Ala Lys Asp Asn Ser Thr Asp Glu Lys Thr Lys Glu Asn Gly Gly Glu
            180                 185                 190

Ile Thr Phe Asp Ser Ala Ser Thr Glu Val Pro Glu Gln Val Lys Lys
        195                 200                 205

Ala Ala Phe Ala Leu Asp Val Asp Gly Val Ser Asp Val Ile Thr Ala
    210                 215                 220

Thr Gly Thr Gln Ala Tyr Ser Ser Gln Tyr Tyr Ile Val Lys Leu Thr
225                 230                 235                 240

Lys Lys Thr Glu Lys Ser Ser Asn Ile Asp Asp Tyr Lys Glu Lys Leu

245                 250                 255

Lys Thr Val Ile Leu Thr Gln Lys Gln Asn Asp Ser Thr Phe Val Gln
            260                 265                 270

Ser Ile Ile Gly Lys Glu Leu Gln Ala Ala Asn Ile Lys Val Lys Asp
        275                 280                 285

Gln Ala Phe Gln Asn Ile Phe Thr Gln Tyr Ile Gly Gly Gly Asp Ser
    290                 295                 300

Ser Ser Ser Ser Ser Thr Ser Asn Glu
305                 310

<210> 164
<211> 465
<212> PRT
<213> Streptococcus pneumoniae

<400> 164
Met Lys Lys Asn Ile Lys Gln Tyr Val Thr Leu Gly Thr Val Val Val
  1               5                  10                  15

Leu Ser Ala Phe Val Ala Asn Ser Val Ala Ala Gln Glu Thr Glu Thr
             20                  25                  30

Ser Glu Val Ser Thr Pro Lys Leu Val Gln Pro Val Ala Pro Thr Thr
         35                  40                  45

Pro Ile Ser Glu Val Gln Pro Thr Ser Asp Asn Ser Ser Glu Val Thr
     50                  55                  60

Val Gln Pro Arg Thr Val Glu Thr Thr Val Lys Asp Pro Ser Ser Thr
 65                  70                  75                  80

Ala Glu Glu Thr Pro Val Leu Glu Lys Asn Asn Val Thr Leu Thr Gly
                 85                  90                  95

Gly Gly Glu Asn Val Thr Lys Glu Leu Lys Asp Lys Phe Thr Ser Gly
            100                 105                 110

Asp Phe Thr Val Val Ile Lys Tyr Asn Gln Ser Ser Glu Lys Gly Leu
        115                 120                 125

Gln Ala Leu Phe Gly Ile Ser Asn Ser Lys Pro Gly Gln Gln Asn Ser
    130                 135                 140

Tyr Val Asp Val Phe Leu Arg Asp Asn Gly Glu Leu Gly Met Glu Ala
145                 150                 155                 160

Arg Asp Thr Ser Ser Asn Lys Asn Asn Leu Val Ser Arg Pro Ala Ser
                165                 170                 175

Val Trp Gly Lys Tyr Lys Gln Glu Ala Val Thr Asn Thr Val Ala Val
            180                 185                 190

Val Ala Asp Ser Val Lys Lys Thr Tyr Ser Leu Tyr Ala Asn Gly Thr
        195                 200                 205

Lys Val Val Glu Lys Lys Val Asp Asn Phe Leu Asn Ile Lys Asp Ile
    210                 215                 220

Lys Gly Ile Asp Tyr Tyr Met Leu Gly Gly Val Lys Arg Ala Gly Lys
225                 230                 235                 240

Thr Ala Phe Gly Phe Asn Gly Thr Leu Glu Asn Ile Lys Phe Phe Asn
                245                 250                 255

Ser Ala Leu Asp Glu Glu Thr Val Lys Lys Met Thr Thr Asn Ala Val
            260                 265                 270

Thr Gly His Leu Ile Tyr Thr Ala Asn Asp Thr Thr Gly Ser Asn Tyr
        275                 280                 285

Phe Arg Ile Pro Val Leu Tyr Thr Phe Ser Asn Gly Arg Val Phe Ser
    290                 295                 300

Ser Ile Asp Ala Arg Tyr Gly Gly Thr His Asp Phe Leu Asn Lys Ile
305                 310                 315                 320

Asn Ile Ala Thr Ser Tyr Ser Asp Asp Asn Gly Lys Thr Trp Thr Lys
                325                 330                 335

Pro Lys Leu Thr Leu Ala Phe Asp Asp Phe Ala Pro Val Pro Leu Glu
            340                 345                 350

Trp Pro Arg Glu Val Gly Gly Arg Asp Leu Gln Ile Ser Gly Gly Ala
        355                 360                 365

Thr Tyr Ile Asp Ser Val Ile Val Glu Lys Lys Asn Lys Gln Val Leu
    370                 375                 380

Met Phe Ala Asp Val Met Pro Ala Gly Val Ser Phe Arg Glu Ala Thr
385                 390                 395                 400

Arg Lys Asp Ser Gly Tyr Lys Gln Ile Asp Gly Asn Tyr Tyr Leu Lys
                405                 410                 415

Leu Arg Lys Gln Gly Asp Thr Asp Tyr Asn Tyr Thr Ile Arg Glu Asn
            420                 425                 430

Gly Thr Val Tyr Asp Asp Arg Thr Asn Arg Pro Thr Glu Phe Ser Val
        435                 440                 445

Asp Lys Asn Phe Gly Ile Lys Gln Asn Gly Asn Tyr Leu Thr Val Glu
    450                 455                 460

Arg
465

<210> 165
<211> 266
<212> PRT
<213> Streptococcus pneumoniae

<400> 165
Met Lys Lys Phe Ser Leu Leu Leu Ala Ile Leu Pro Phe Leu Val Ala
1               5                   10                  15

Cys Glu Asn Gln Ala Thr Pro Lys Glu Thr Ser Ala Gln Lys Thr Ile
            20                  25                  30

Val Leu Ala Thr Ala Gly Asp Val Pro Pro Phe Asp Tyr Glu Asp Lys
        35                  40                  45

Gly Asn Leu Thr Gly Phe Asp Ile Glu Val Leu Lys Ala Val Asp Glu
    50                  55                  60

Lys Leu Ser Asp Tyr Glu Ile Gln Phe Gln Arg Thr Ala Trp Glu Ser
65                  70                  75                  80

Ile Phe Pro Gly Leu Asp Ser Gly His Tyr Gln Ala Ala Ala Asn Asn
                85                  90                  95

Leu Ser Tyr Thr Lys Glu Arg Ala Glu Lys Tyr Leu Tyr Ser Leu Pro
            100                 105                 110

Ile Ser Asn Asn Pro Leu Val Leu Val Ser Asn Lys Lys Asn Pro Leu
        115                 120                 125

Thr Ser Leu Asp Gln Ile Ala Gly Lys Thr Thr Gln Glu Asp Thr Gly
    130                 135                 140

Thr Ser Asn Ala Gln Phe Ile Asn Asn Trp Asn Gln Lys His Thr Asp
145                 150                 155                 160

Asn Pro Ala Thr Ile Asn Phe Ser Gly Glu Asp Ile Gly Lys Arg Ile
                165                 170                 175

Leu Asp Leu Ala Asn Gly Glu Phe Asp Phe Leu Val Phe Asp Lys Val
            180                 185                 190

Ser Val Gln Lys Ile Ile Lys Asp Arg Gly Leu Asp Leu Ser Val Val
        195                 200                 205

Asp Leu Pro Ser Ala Asp Ser Pro Ser Asn Tyr Ile Ile Phe Ser Ser
    210                 215                 220

Asp Gln Lys Glu Phe Lys Glu Gln Phe Asp Lys Ala Leu Lys Glu Leu
225                 230                 235                 240

Tyr Gln Asp Gly Thr Leu Glu Lys Leu Ser Asn Thr Tyr Leu Gly Gly
                245                 250                 255

Ser Tyr Leu Pro Asp Gln Ser Gln Leu Gln
            260                 265

<210> 166
<211> 259
<212> PRT
<213> Streptococcus pneumoniae

<400> 166
Met Lys Lys Asn Ser Leu Tyr Ile Ile Ser Ser Leu Phe Phe Ala Cys
1               5                   10                  15

Val Leu Phe Val Tyr Ala Thr Ala Thr Asn Phe Gln Asn Ser Thr Ser

20                  25                  30

Ala Arg Gln Val Lys Thr Glu Thr Tyr Thr Asn Thr Val Thr Asn Val
        35                  40                  45

Pro Ile Asp Ile Arg Tyr Asn Ser Asp Lys Tyr Phe Ile Ser Gly Phe
    50                  55                  60

Ala Ser Glu Val Ser Val Val Leu Thr Gly Ala Asn Arg Leu Ser Leu
65                  70                  75                  80

Ala Ser Glu Met Gln Glu Ser Thr Arg Lys Phe Lys Val Thr Ala Asp
                85                  90                  95

Leu Thr Asp Ala Gly Val Gly Thr Ile Glu Val Pro Leu Ser Ile Glu
            100                 105                 110

Asp Leu Pro Asn Gly Leu Thr Ala Val Ala Thr Pro Gln Lys Ile Thr
        115                 120                 125

Val Lys Ile Gly Lys Lys Ala Gln Lys Asp Lys Val Lys Ile Val Pro
    130                 135                 140

Glu Ile Asp Pro Ser Gln Ile Asp Ser Arg Val Gln Ile Glu Asn Val
145                 150                 155                 160

Met Val Ser Asp Lys Glu Val Ser Ile Thr Ser Asp Gln Glu Thr Leu
                165                 170                 175

Asp Arg Ile Asp Lys Ile Ile Ala Val Leu Pro Thr Ser Glu Arg Ile
            180                 185                 190

Thr Gly Asn Tyr Ser Gly Ser Val Pro Leu Gln Ala Ile Asp Arg Asn
        195                 200                 205

Gly Val Val Leu Pro Ala Val Ile Thr Pro Phe Asp Thr Ile Met Lys
    210                 215                 220

Val Thr Thr Lys Pro Val Ala Pro Ser Ser Ser Thr Ser Asn Ser Ser
225                 230                 235                 240

Thr Ser Ser Ser Ser Glu Thr Ser Ser Ser Thr Lys Ala Thr Ser Ser
                245                 250                 255

Lys Thr Asn

<210> 167
<211> 263
<212> PRT
<213> Streptococcus pneumoniae

<400> 167
Met Leu Ile Gly Glu Gly Tyr Arg Thr Phe Pro Val Leu Ile Tyr Thr
1               5                   10                  15

Gln Phe Ile Ser Glu Val Gly Gly Asn Ser Ala Phe Ala Ile Met Ala
            20                  25                  30

Ile Ile Ile Ala Leu Ala Ile Phe Leu Ile Gln Lys His Ile Ala Asn
        35                  40                  45

Arg Tyr Ser Phe Ser Met Asn Leu Leu His Pro Ile Glu Pro Lys Lys
    50                  55                  60

Thr Thr Lys Gly Lys Met Ala Ala Ile Tyr Ala Thr Val Tyr Gly Ile
65                  70                  75                  80

Ile Phe Ile Ser Val Leu Pro Gln Ile Tyr Leu Ile Tyr Thr Ser Phe
                85                  90                  95

Leu Lys Thr Ser Gly Met Val Ser Val Lys Gly Tyr Ser Pro Asn Ser
            100                 105                 110

Tyr Lys Val Ala Phe His Arg Met Gly Ser Ala Ile Phe Asn Thr Ile
        115                 120                 125

Arg Ile Pro Leu Ile Ala Leu Val Leu Val Val Leu Phe Ala Thr Phe
    130                 135                 140

Ile Ser Tyr Leu Ala Val Arg Lys Arg Asn Leu Phe Thr Asn Leu Ile
145                 150                 155                 160

Asp Ser Leu Ser Met Val Pro Tyr Ile Val Pro Gly Thr Val Leu Gly
                165                 170                 175

Ile Ala Phe Ile Ser Ser Phe Asn Thr Gly Leu Phe Gly Ser Gly Phe
            180                 185                 190

Leu Met Ile Thr Gly Thr Ala Phe Ile Leu Ile Met Ser Leu Ser Ala
        195                 200                 205

Arg Arg Leu Pro Tyr Thr Ile Arg Ser Ser Val Ala Ser Leu Gln Gln
    210                 215                 220

Ile Ala Pro Ser Ile Glu Glu Ala Ala Glu Ser Leu Gly Ser Ser Arg
225                 230                 235                 240

Leu Asn Thr Phe Ala Lys Ile Thr Thr Pro Met Met Leu Ser Gly Ile
                245                 250                 255

Ile Ser Gly Ala Ile Leu Ser
            260

<210> 168
<211> 1332
<212> DNA
<213> Streptococcus pneumoniae

<400> 168
atgataaaaa atcctaaatt attaaccaag tcttttttaa gaagttttgc aattctaggt 60
ggtgttggtc tagtcattca tatagctatt tatttgacct ttccttttta ttatattcaa 120
ctggaggggg aaaagtttaa tgagagcgca agagtgttta cggagtattt aaagactaag 180
acatctgatg aaattccaag cttactccag tcttattcaa agtccttgac catatctgct 240
caccttaaaa gagatattgt agataagcgg ctccctcttg tgcatgactt ggatattaaa 300
gatggaaagc tatcaaatta tatcgtgatg ttagatatgt ctgttagtac agcagatggt 360
aaacaggtaa ccgtgcaatt tgttcacggg gtggatgtct acaaagaagc aaagaatatt 420
ttgcttttgt atctcccata tacatttttg gttacaattg ctttttcctt tgttttttct 480

tatttttata ctaaacgctt gctcaatcct cttttttaca tttcagaagt gactagtaaa 540
atgcaagatt tggatgacaa tattcgtttt gatgaaagta ggaaagatga agttggtgaa 600
gttggaaaac agattaatgg tatgtatgag cacttgttga aggttattta tgagttggaa 660
agtcgtaatg agcaaattgt aaaattgcaa aatcaaaagg tttcctttgt ccgcggagca 720
tcacatgagt tgaaaacccc tttagccagt cttagaatta tcctagagaa tatgcagcat 780
aatattggag attacaaaga tcatccaaaa tatattgcaa agagtataaa taagattgac 840
cagatgagcc acttattaga agaagtactg gagtcttcta aattccaaga gtggacagag 900
tgtcgtgaga ccttgactgt taagccagtt ttagtagata ttttatcacg ttatcaagaa 960
ttagctcatt caataggtgt tacaattgaa aatcaattga cagatgctac cagggtcgtc 1020
atgagtctta gggcattgga taaggttttg acaaacctga ttagtaatgc aattaaatat 1080
tcagataaaa atgggcgtgt aatcatatcc gagcaagatg gctatctctc tatcaaaaat 1140
acatgtgcgc ctctaagtga ccaagaacta gaacatttat ttgatatatt ctatcattct 1200
caaatcgtga cagataagga tgaaagttcc ggtttgggtc tttacattgt gaataatatt 1260
ttagaaagct atcaaatgga ttatagtttt ctcccttatg aacacggtat ggaatttaag 1320
attagcttgt ag 1332

<210> 169
<211> 873
<212> DNA
<213> Streptococcus pneumoniae

<400> 169
atggataaaa ttattaaaac tatatcagaa agcggagcct ttcgtgcttt tgtccttgat 60
agcactgaaa ccgtccgcac tgctcaagaa aaacatcaaa cccaagctag ctcaactgta 120
gcgcttggtc gaactcttat cgctagccag attctcgcag ccaatgaaaa aggaaatacc 180
aaacttacag ttaaggtgtt gggatctagc tctctaggtg ctattatcac cgtcgctgat 240
accaagggga acgtcaaagg ctatgttcaa aatcctggtg ttgacatcaa aaagactgcg 300
actggtgaag tcctagtcgg accttttgtt ggaaatggtc aattcctcgt tatcacagac 360
tacggtactg gaaatcctta caactctata actcccctca tctctggaga aatcggtgaa 420
gaccttgcct tttaccttac tgaaagccaa caaacgcctt cagcggtcgg cctcaatgtc 480
cttttggacg aggaagacaa ggtcaaggtt gcaggtggtt tcctagttca agtcttgcca 540
ggagccaaga aagaagagat tgctcgcttt gaaaaacgca tccaagaaat gccagctatc 600
tctactcttc tcgaaagcga cgaccatatc gaagccctcc tcaaggctat ctacggggac 660
gaagcctaca agcgtctttc tgaagaagaa atccgtttcc aatgtgactg tagccatgaa 720
cgctttatga acgctcttgc cagccttcca agctcagact tacaggaaat gaaagaggaa 780
gaccacgggg cagaaatcac ttgtcaattc tgccaaacta cttacaactt tgatgaaaag 840
gacctggagg aactcattcg tgacaaatct taa 873

<210> 170
<211> 459
<212> DNA
<213> Streptococcus pneumoniae

<400> 170
atgaaatcaa taactaaaaa gattaaagca actcttgcag gagtagctgc cttgtttgca 60
gtatttgctc catcatttgt atctgctcaa gaatcatcaa cttacactgt taaagaaggt 120
gatacacttt cagaaatcgc tgaaactcac aacacaacag ttgaaaaatt ggcagaaaac 180
aaccacattg ataacattca tttgatttat gttgatcaag agttggttat cgatggccct 240
gtagcgcctg ttgcaacacc agcgccagct acttatgcgg caccagccgc tcaagatgaa 300
actgtttcag ctccagtagc agaaactcca gtagtaagtg aaacagttgt ttcaactgta 360
agcggatctg aagcagaagc caaagaatgg atcgctcaaa aagaatcagg tggtagtata 420
cagctacaaa tggacgttat atcggacgtt accaattaa 459

<210> 171
<211> 2034
<212> DNA
<213> Streptococcus pneumoniae

<400> 171
atgaatttag gagaattttg gtacaataaa ataaataaga acagaggaag aaggttaatg 60
aagaaagtaa gatttatttt tttagctctg ctatttttct tagctagtcc agagggtgca 120
atggctagtg atggtacttg gcaaggaaaa cagtatctga aagaagatgg cagtcaagca 180
gcaaatgagt gggtttttga tactcattat caatcttggt tctatataaa agcagatgct 240
aactatgctg aaaatgaatg gctaaagcaa ggtgacgact atttttacct caaatctggt 300
ggctatatgg ccaaatcaga atgggtagaa gacaagggag ccttttatta tcttgaccaa 360
gatggaaaga tgaaaagaaa tgcttgggta ggaacttcct atgttggtgc aacaggtgcc 420
aaagtaatag aagactgggt ctatgattct caatacgatg cttggtttta tatcaaagca 480
gatggacagc acgcagagaa agaatggctc caaattaaag ggaaggacta ttatttcaaa 540
tccggtggtt atctactgac aagtcagtgg attaatcaag cttatgtgaa tgctagtggt 600
gccaaagtac agcaaggttg gctttttgac aaacaatacc aatcttggtt ttacatcaaa 660
gaaaatggaa actatgctga taaagaatgg atttcgagaa atggtcacta ttattatcta 720
aaatccggtg gctacatggc agccaatgaa tggatttggg ataaggaatc ttggttttat 780
ctcaaatttg atgggaaaat ggctgaaaaa gaatgggtct acgattctca tagtcaagct 840
tggtactact tcaaatccgg tggttacatg acagccaatg aatggatttg ggataaggaa 900
tcttggtttt atctcaaatc tgatgggaaa atagctgaaa aagaatgggt ctacgattct 960
catagtcaag cttggtacta cttcaaatcc ggtggttaca tgacagccaa tgaatggatt 1020
tgggataagg aatcttggtt ttacctcaaa tctgatggga aaatagctga aaaagaatgg 1080
gtctacgatt ctcatagtca agcttggtac tacttcaaat ctggtggcta catggcgaaa 1140
aatgagacag tagatggtta tcagcttgga agcgatggta aatggcttgg aggaaaaact 1200
acaaatgaaa atgctgctta ctatcaagta gtgcctgtta cagccaatgt ttatgattca 1260
gatggtgaaa agctttccta tatatcgcaa ggtagtgtcg tatggctaga taaggataga 1320
aaaagtgatg acaagcgctt ggctattact atttctggtt tgtcaggcta tatgaaaaca 1380
gaagatttac aagcgctaga tgctagtaag gactttatcc cttattatga gagtgatggc 1440
caccgttttt atcactatgt ggctcagaat gctagtatcc cagtagcttc tcatctttct 1500
gatatggaag taggcaagaa atattattcg gcagatggcc tgcattttga tggttttaag 1560
cttgagaatc ccttcctttt caaagattta acagaggcta caaactacag tgctgaagaa 1620
ttggataagg tatttagttt gctaaacatt aacaatagcc ttttggagaa caagggcgct 1680
acttttaagg aagccgaaga acattaccat atcaatgctc tttatctcct tgcccatagt 1740
gccctagaaa gtaactgggg aagaagtaaa attgccaaag ataagaataa tttctttggc 1800
attacagcct atgatacgac cccttacctt tctgctaaga catttgatga tgtggataag 1860
ggaattttag gtgcaaccaa gtggattaag gaaaattata tcgatagggg aagaactttc 1920
cttggaaaca aggcttctgg tatgaatgtg gaatatgctt cagaccctta ttggggcgaa 1980
aaaattgcta gtgtgatgat gaaaatcaat gagaagctag gtggcaaaga ttag 2034

<210> 172
<211> 930
<212> DNA
<213> Streptococcus pneumoniae

<400> 172
atgaaaaaat taggtacatt actcgttctc tttctttctg caatcattct tgtagcatgt 60
gctagcggaa aaaaagatac aacttctggt caaaaactaa aagttgttgc tacaaactca 120
atcatcgctg atattactaa aaatattgct ggtgacaaaa ttgaccttca tagtatcgtt 180
ccgattgggc aagacccaca cgaatacgaa ccacttcctg aagacgttaa gaaaacttct 240
gaggctaatt tgattttcta taacggtatc aaccttgaaa caggtggcaa tgcttggttt 300
acaaaattgg tagaaaatgc caagaaaact gaaaacaaag actacttcgc agtcagcgac 360
ggcgttgatg ttatctacct tgaaggtcaa aatgaaaaag gaaaagaaga cccacacgct 420
tggcttaacc ttgaaaacgg tattattttt gctaaaaata tcgccaaaca attgagcgcc 480
aaagacccta acaataaaga attctatgaa aaaaatctca aagaatatac tgataagtta 540
gacaaacttg ataaagaaag taaggataaa tttaataaga tccctgctga aaagaaactc 600
attgtaacca gcgaaggagc attcaaatac ttctctaaag cctatggtgt cccaagtgct 660
tacatctggg aaatcaatac tgaagaagaa ggaactcctg aacaaatcaa gaccttggtt 720
gaaaaacttc gccaaacaaa agttccatca ctctttgtag aatcaagtgt ggatgaccgt 780
ccaatgaaaa ctgtttctca agacacaaac atcccaatct acgctcaaat ctttactgac 840
tctatcgcag aacaaggtaa agaaggcgac agctactaca gcatgatgaa atacaacctt 900
gacaagattg ctgaaggatt ggcaaaataa 930

<210> 173
<211> 1260
<212> DNA
<213> Streptococcus pneumoniae

<400> 173
atggaatggt ataaaaaaat cggacttctt gcaactacag gtttagcttt gtttgggctc 60
ggcgcttgct ccaactatgg taaatctgcg gatggcacag tgaccatcga gtatttcaac 120
cagaaaaaag aaatgaccaa aaccttggaa gaaatcactc gtgattttga gaaggaaaac 180
cctaagatca aggtcaaagt cgtcaatgta ccaaatgctg gtgaagtatt gaagacacgc 240
gttctcgcag gagatgtgcc tgatgtggtc aatatttacc cacagtccat cgaactgcaa 300
gaatgggcaa aagcaggtgt ttttgaagat ttgagcaaca aagactacct gaaacgcgtg 360
aaaaatggct acgctgaaaa atatgctgta aacgaaaaag tttacaacgt tccttttaca 420
gctaatgctt atggaattta ctacaacaaa gataaattcg aagaactggg cttgaaggtt 480
cctgaaacct gggatgaatt tgaacagtta gtcaaagata tcgttgctaa aggacaaaca 540
ccatttggaa ttgcaggtgc agatgcttgg acactcaatg gttacaatca attagccttt 600
gcgacagcaa caggtggagg aaaagaagca aatcaatacc ttcgttattc tcaaccaaat 660
gccattaaat tgtcggatcc gattatgaaa gatgatatca aggtcatgga catccttcgc 720
atcaatggat ctaagcaaaa gaactgggaa ggtgctggct ataccgatgt tatcggagcc 780
ttcgcacgtg gggatgtcct catgacacca aatgggtctt gggcgatcac agcgattaat 840
gaacaaaaac cgaactttaa gattgggacc ttcatgattc caggaaaaga aaaaggacaa 900
agcttaaccg ttggtgcggg agacttggca tggtctatct cagccaccac caaacatcca 960
aaagaagcca atgcctttgt ggaatatatg acccgtccag aagtcatgca aaaatactac 1020
gatgtggacg gatctccaac agcgatcgaa ggggtcaaac aagcaggaga agattcaccg 1080
cttgctggta tgaccgaata tgcctttacg gatcgtcact tggtctggtt gcaacaatac 1140
tggaccagtg aagcagactt ccataccttg accatgaact atgtcttgac cggtgataaa 1200
caaggcatgg tcaatgattt gaatgccttc tttaacccga tgaaagcgga tgtggattag 1260

<210> 174
<211> 1680
<212> DNA
<213> Streptococcus pneumoniae

<400> 174
atgaagaaaa tcaaaccgca tggaccgtta ccaagtcaga ctcagctagc ttatctggga 60
gatgaactag cagcttttat ccacttcggt cctaatacct tttatgacca agaatggggg 120
actggacagg aggatcctga gcgctttaac ccgagtcagt tggatgcgcg tgagtgggtt 180
cgtgtgctca aggaaacggg cttcaaaaag ttgattttgg tggtcaagca ccacgatggc 240
tttgtccttt atccgacagc tcacacagat tattcggtta aggtcagtcc ttggaggaga 300
ggaaagggcg acttgctcct tgaagtatcc caagctgcca cagagtttga tatggatatg 360
ggggtctacc tgtcaccgtg ggatgcccat agtcccctct atcatgtgga ccgagaagcg 420
gactacaatg cctattatct ggctcagttg aaggaaatct tatcaaatcc taactatggg 480
aatgctggta agttcgctga ggtttggatg gatggtgcca gaggagaggg cgcgcaaaag 540
gttaattatg aatttgaaaa atggtttgaa accattcgtg acctgcaggg cgattgcttg 600
attttttcaa cagaaggcac cagtatccgc tggattggca atgaacgagg gtatgcaggt 660
gatccactgt ggcaaaaggt gaatcctgat aaactaggaa cagaagcaga gctgaactat 720
cttcagcacg gggatccctc gggcacgatt ttttcaatcg gagaggcaga tgtttccatc 780
cgtccaggct ggttctacca tgaggatcag gatcctaagt ctctcgagga gttggtcgaa 840
atctactttc actcagtagg gcgaggaact ccactcttgc ttaatattcc gccgaatcaa 900
gctgggctct ttgatgcaaa ggatattgaa cgactttatg aatttgcgac ctatcgcaat 960
gagctctata aagaagattt ggctctggga gctgaggtat ctggtccagc tctttccgca 1020
gactttgctt gtcgccattt gacagacggc cttgagacca gctcttgggc aagcgatgca 1080
gacttgccca tccagttaga actcgactta ggttctccta aaacttttga tgtaattgag 1140
ttaagagaag atttgaagct agggcaacga atcgctgctt ttcatgtgca agtagaggtg 1200
gatggtgtct ggcaggagtt tggttcgggt catactgttg gttacaaacg tctcttacga 1260
ggagcagttg ttgaggcaca gaagatacgt gtagtcatta cagaatcaca ggctttgcct 1320
ttgttgacca agatttccct ttataaaact cctggattat caaaaaaaga agttgttcag 1380
gaactagcat ttgcagaaaa aagcctagct gtggcaaagg gagaaaatgc ctattttaca 1440
gttaagcgca gagaatgtag tggtccttta gaagctaaga tttcgattca accggggaca 1500
ggtgtccatg gtgtcgccta tcaggatgag attcaagtcc ttgcgtttca aactggtgag 1560

actgaaaaaa gtctgacgct accaaccttg tatttcgcag gagataaaac cttggatttc 1620
tatctgaacc taacggtgga tggtcagctt gtggatcaac ttcaagtcca agtttcataa 1680

<210> 175
<211> 684
<212> DNA
<213> Streptococcus pneumoniae

<400> 175
atgcttgaaa gactgaaaag aatacattat atgttttgga tcagtttaat ttttatgatt 60
ttccccatcc tgtctgtagt gactgggtgg ctttctgcct ggcatttatt gattgatatt 120
ctatttgtag tggcatattt gggtgtttta acaactaaga gccagcgcct atcttggcta 180
tattggggcc tcatgctgac ttatgtagtt gggaatactg cctttgttgc tgttaattat 240
atctggtttt tctttttcct atccaatctc ttaagttatc atttcagcgt acgtagttta 300
aagtctttac atgtctggac ttttcttctt gctcaagtcc ttgttgtggg gcaactgttg 360
atttttcaga gaatcgaagt tgagtttcta ttctatctac ttgtaattct tacttttgtc 420
gatttaatga cttttggatt ggttcggatt cgtattgtcg aggatttgaa agaagctcag 480
gtcaagcaaa atgctcagat aaatctattg cttgctgaaa atgaacgtag tcgtatcggt 540
caggatttgc atgatagtct gggacatacc tttgctatgc tgagtgtcaa gacagattta 600
gccttgcagt tatttcagat ggaggcttat ccacaggtgg aaaaggaatt aaaagaaatt 660
caccagatca gcaaggatcc atga 684

<210> 176
<211> 1113
<212> DNA
<213> Streptococcus pneumoniae

<400> 176
atgaatgata agttaaaaat cttcttgttg ctaggagtat tttttctagc cataaccggt 60
ttctatgttc tattgatacg aaatgcaggg cagacagatg cctcgcaaat tgaaaaggcg 120
gcagttagcc aaggaggaaa agcagtgaaa aaaacagaaa ttagtaaaga cgcagacttg 180
cacgaaattt atctagctgg aggttgtttc tggggagtgg aggaatattt ctcacgtgtt 240
cccggggtga cggatgccgt ttcaggctat gcaaatggta gaggagaaac aaccaagtac 300
gaattgatta accaaacagg tcatgcagaa accgtccatg tcacctatga tgccaagcaa 360
atttctctca aggaaatcct gcttcactat ttccgcatta tcaatccaac cagcaaaaat 420
aaacaaggaa atgatgtggg gacccagtac cgtactggtg tttattacac agatgacaag 480
gatttggaag tgattaacca agtctttgat gaggtggcta agaaatacga tcaacctcta 540
gcagttgaaa aggaaaactt gaagaatttt gtggtggctg aggattacca tcaagactat 600
ctcaagaaaa atccaaatgg ctactgccat atcaatgtta atcaggcggc ctatcctgtc 660
attgatgcca gcaaatatcc aaaaccaagt gatgaggaat tgaaaaagac cctgtcacct 720
gaggagtatg cagttaccca ggaaaatcaa acagaacgag ctttctcaaa ccgttactgg 780
gataaatttg aatccggtat ctatgtggat atagcaactg gggaacctct cttttcatca 840
aaagacaaat ttgagtctgg ttgtggctgg cctagtttta cccaacccat cagtccagat 900
gttgtcacct acaaggaaga taagtcctac aatatgacgc gtatggaagt gcggagccga 960
gtaggagatt ctcaccttgg gcatgtcttt acggatggtc cacaggacaa gggcggctta 1020
cgttactgta tcaatagcct ctctatccgc tttattccca aagaccaaat ggaagaaaaa 1080
ggctacgctt atttactaga ttatgttgat taa 1113

<210> 177
<211> 1017
<212> DNA
<213> Streptococcus pneumoniae

<400> 177
atgaaaaaga aatggatgta ttatgctgct tgttcttcta atgaatctgc cgatgacagt 60
tcatctgata aaggagacgg cggttcgcta gtcgtttatt caccaaactc agagggctta 120
attggagcaa ctattcctgc ctttgaagaa aaatatggta tcaaagtaga actgattcaa 180
gctggtactg gagaactttt caaaaaacta gagtcagaaa aagaagttcc tgtagctgat 240

gttatctttg gtggttctta tacacaatat actacccacg gagaactctt tgaaaactat 300
acttcaaaag aaaatgataa tgttatcaaa gaatatcaaa acacaactgg ctactctact 360
ccttatacac tagatggtag tgttttaatc gtcaaccctg atttaactaa aggcatgaac 420
atcgaaggat ataacgatct tttcaaacct gaactaaaag gaaaaatcgc aactgctgac 480
ccagcaaact cttctagcgc ctttgctcaa ttaacaaata tgctacaagc tcaaggtggt 540
tacaaagatg ataaggcttg gtcttatgta aaagatcttt tcacacttat tgatggtaaa 600
atcggttcaa gttcatctag tgtctataaa gtagtcgctg atggagaaat ggctgttggt 660
ctctcttatg aagatccagc agttaaactc ttaaatgacg gagctaacat taaggtagtc 720
tatccaaaag aaggaaccgt cttcctacct gctagtgctg ctatcgttaa aaaatctaaa 780
aatatggaaa atgccaagaa atttatcgat tttattatct ctcaagaagt acaagataca 840
cttggtacaa ccactactaa ccgtcctgtt cgtaaaaatg ctaaaacaag cgaaaacatg 900
aaaccaattg acaaaatcaa aacactcact gaagattatg attatgtcat caagaataaa 960
tcagatatcg ttaagaaata caacgaagtc tttacagata tccaatctaa acagtaa 1017

<210> 178
<211> 1092
<212> DNA
<213> Streptococcus pneumoniae

<400> 178
atgagtgaga tcaaaattat taacgccaaa aaaatctacc acgatgtccc tgttattgag 60
aatttgaaca ttacaattcc aaaaggaagt ctctttaccc ttcttggagc ttcaggatgt 120
gggaaaacga cccttcttcg tatgattgca ggtttcaaca gtatcgaagg tggagaattt 180
tacttcgatg atacaaaaat caataatatg gaacccagca aacgcaatat cgggatggtt 240
ttccaaaact acgctatttt cccacatttg actgtccgag acaacgttgc ttttggtctt 300
atgcaaaaga aggttccaaa agaagaattg attcaacaga ccaacaagta tcttgaactc 360
atgcaaattg ctcaatatgc ggatcgaaag cccgataaac tcagtggtgg acaacaacaa 420
cgtgtcacct tggcatgcgc cttagcggtt aatccaagtg ttctcctcat ggacgagcca 480
cttagtaatc tggaggccaa acttcgcttg gatatgcgtc aagccatccg agaaatccaa 540
cacgaagtgg gaattacaac tgtttatgta acccacgacc aagaagaagc catggctatt 600
tcagaccaaa ttgctgttat gaaagatggg gtgatccaac aaatcggccg accaaaagaa 660
ctctatcata aaccagctaa tgagtttgtg gcaaccttta tcggacgcac aaatattatc 720
cctgccaatc ttgaaaaacg gagcgacggc gcttatatcg tcttttcaga tggctatgcc 780
cttcgaatgc cagctcttga tcaggttgag gagcaagcta ttcatgtaag cattcgtccc 840
gaagagttta tcaaagatga atctggagat attgaaggaa ctattagaga tagcgtctat 900
cttggactaa atacggatta tttcattgag acaggttttg cctcaaaaat tcaagttagt 960
gaagaatcaa cttttgaaga agatctacaa aaaggcaatc gtattcgtct acgaatcaat 1020
acgcaaaaat taaacatctt ttctgcagat ggttcccaaa acctgataaa aggagtcaac 1080
catggaacgt aa 1092

<210> 179
<211> 942
<212> DNA
<213> Streptococcus pneumoniae

<400> 179
atgaagaaaa aattattggc aggtgccatc acactattat cagtagcaac tttagcagct 60
tgttcgaaag ggtcagaagg tgcagacctt atcagcatga aaggggatgt cattacagaa 120
catcaatttt atgagcaagt gaaaagcaac ccttcagccc aacaagtctt gttaaatatg 180
accatccaaa aagtttttga aaaacaatat ggctcagagc ttgatgataa agaggttgat 240
gatactattg ccgaagaaaa aaaacaatat ggcgaaaact accaacgtgt cttgtcacaa 300
gcaggtatga ctcttgaaac acgtaaagct caaattcgta caagtaaatt agttgagttg 360
gcagttaaga aggtagcaga agctgaattg acagatgaag cctataagaa agcctttgat 420
gagtacactc cagatgtaac ggctcaaatc atccgtctta ataatgaaga taaggccaaa 480
gaagttctcg aaaaagccaa ggcagaaggt gctgattttg ctcaattagc caaagataat 540
tcaactgatg aaaaaacaaa agaaaatggt ggagaaatta cctttgattc tgcttcaaca 600
gaagtacctg agcaagtcaa aaaagccgct ttcgctttag atgtggatgg tgtttctgat 660
gtgattacag caactggcac acaagcctac agtagccaat attacattgt aaaactcact 720
aagaaaacag aaaaatcatc taatattgat gactacaaag aaaaattaaa aactgttatc 780

ttgactcaaa aacaaaatga ttcaacattt gttcaaagca ttatcggaaa agaattgcaa 840
gcagccaata tcaaggttaa ggaccaagcc ttccaaaata tctttaccca atatatcggt 900
ggtggagatt caagctcaag cagtagtaca tcaaacgaat ag 942

<210> 180
<211> 1395
<212> DNA
<213> Streptococcus pneumoniae

<400> 180
atgaaaaaaa atattaaaca atatgtaacc ttaggtactg tagtggtatt atcagcattt 60
gttgctaact cagttgcagc tcaggagact gaaacttctg aagtatcaac accaaagttg 120
gtgcaacctg ttgcaccaac gactccgatt tcggaagtac aacctacatc ggataactct 180
tcggaagtta ctgtacaacc tcgaacagtt gaaactactg ttaaggatcc atcttctaca 240
gcggaagaaa ctcctgtctt agaaaaaaat aatgttactt taacaggggg cggagaaaat 300
gttactaaag agttaaagga taaatttact agcggtgact ttactgtagt gattaagtac 360
aatcagtcaa gtgagaaagg cttacaagct ctgtttggaa tatctaattc caaacccggt 420
caacaaaata gttatgtaga tgtgttcctt agagacaatg gtgagttggg gatggaagcg 480
cgtgatactt cttccaataa aaataaccta gtatccagac ctgcttcagt ttggggtaag 540
tacaaacaag aggctgtgac taacactgtt gcagtagtag cagattcagt caaaaaaaca 600
tattctttat acgcaaatgg tacaaaagta gtagaaaaga aagtggataa tttcctaaac 660
atcaaggata ttaaaggtat tgattactat atgcttgggg gagtgaaacg tgcaggaaaa 720
acggcgtttg gttttaacgg aacactagaa aatatcaaat tctttaatag tgcattggat 780
gaagaaactg ttaaaaagat gacaacaaac gctgttactg gacatttaat ttatacggct 840
aatgatacaa caggttctaa ctatttccgt attccagttc tgtatacttt tagcaatggt 900
cgggtatttt caagcattga cgctcgttac ggtggaactc atgatttctt gaataaaatt 960
aatattgcta caagttatag tgatgataat ggtaagacat ggactaaacc aaaattaaca 1020
ttggcattcg atgattttgc gccagtacca ttagaatggc ctcgtgaagt tggtggacgt 1080
gacttacaaa tcagcggtgg tgcaacctat attgactctg ttattgttga aaaaaagaac 1140
aaacaagtac tcatgtttgc tgatgtgatg cctgctggag taagttttag agaagcaact 1200
agaaaagatt caggttataa acaaattgat ggtaattatt accttaaatt aaggaaacaa 1260
ggtgatactg attacaatta tactattcgt gagaatggta ctgtatacga cgatcgtacc 1320
aacagaccaa ctgaattttc agtagataaa aatttcggta ttaaacaaaa tggtaattat 1380
ttgacggtag agcgg 1395

<210> 181
<211> 801
<212> DNA
<213> Streptococcus pneumoniae

<400> 181
atgaaaaaat ttagcctatt actagctatc ctaccatttt tggttgcctg tgagaatcaa 60
gctacaccca aagagactag cgctcaaaag acaatcgtcc ttgctacagc tggcgacgtg 120
ccaccatttg actacgaaga caagggcaat ctgacaggct ttgatatcga agttttaaag 180
gcagtagatg aaaaactcag cgactacgag attcaattcc aaagaaccgc ctgggagagc 240
atcttcccag gacttgattc tggtcactat caggctgcgg ccaataactt gagttacaca 300
aaagagcgtg ctgaaaaata cctttactcg cttccaattt ccaacaatcc cctcgtcctt 360
gtcagcaaca agaaaaatcc tttgacttct cttgaccaga tcgctggtaa aacaacacaa 420
gaggataccg gaacttctaa cgctcaattc atcaataact ggaatcagaa acacactgat 480
aatcccgcta caattaattt ttctggtgag gatattggta aacgaatcct agaccttgct 540
aacggagagt ttgatttcct agtttttgac aaggtatccg ttcaaaagat tatcaaggac 600
cgtggtttag acctctcagt cgttgattta ccttctgcag atagccccag caattatatc 660
attttctcaa gcgaccaaaa agagtttaaa gagcaatttg ataaagcgct caaagaactc 720
tatcaagacg gaacccttga aaaactcagc aatacctatc taggtggttc ttacctccca 780
gatcaatctc agttacaata a 801

<210> 182
<211> 780

<212> DNA
<213> Streptococcus pneumoniae

<400> 182
atgaaaaaaa atagtttata tatcatatcc tcactctttt ttgcttgtgt cttatttgtc 60
tatgctacgg cgacgaattt tcaaaacagt accagtgcta ggcaggtaaa aacggaaacc 120
tatactaata cagtaacaaa tgtccctatt gacatacgct ataatagtga taagtatttt 180
attagcggtt ttgcttcaga agtatcagtg gtcttgactg gtgcaaatcg cctatcgcta 240
gctagtgaaa tgcaagaaag tacacgtaaa ttcaaggtta ctgctgacct aacagatgcc 300
ggtgttggaa cgattgaagt tcctttgagc attgaagatt tacccaatgg gctgaccgct 360
gtggcgactc cgcaaaaaat tacagtcaag attggtaaga aggctcagaa ggataaggta 420
aagattgtac cagagattga ccctagtcaa attgatagtc gggtacaaat tgaaaatgtc 480
atggtgtcag ataaagaagt gtctattacg agtgaccaag agacattgga tagaattgat 540
aagattatcg ctgttttgcc aactagcgaa cgtataacag gtaattacag tggttcagta 600
cctttgcagg caatcgaccg caatggtgtt gtcttaccgg cagttatcac tccgtttgat 660
acaataatga aggtgactac aaaaccagta gcaccaagtt caagcacatc aaattcaagt 720
acaagcagtt catcggagac atcttcgtca acgaaagcaa ctagttcaaa aacgaattaa 780

<210> 183
<211> 792
<212> DNA
<213> Streptococcus pneumoniae

<400> 183
atgttgattg gcgaagggta tcggactttc cctgtcctga tttataccca atttattagc 60
gaggttggag gaaattctgc ttttgcaatt atggcgatta tcattgcctt ggcaattttc 120
cttatccaaa aacacattgc aaaccgctac agtttcagca tgaatctgct ccatccaatt 180
gagcctaaaa aaactacaaa aggaaaaatg gctgccattt atgcaacagt ctacggaatt 240
atctttatct ctgttttacc tcaaatctac ttaatttata cctctttcct aaaaacatca 300
ggtatggtat ctgttaaagg ttattctcca aacagttaca aggtagcttt ccatcgtatg 360
ggatctgcta ttttcaatac cattcgtatc cctttgattg ccttagttct agttgttcta 420
tttgcgacat ttatctccta cctagccgtt agaaaacgga atttgtttac aaacttaatt 480
gacagcctca gtatggtacc ttatattgta ccaggaaccg ttctagggat tgccttcatt 540
tcttccttca atactggtct atttggaagt ggatttctta tgattacagg gactgctttc 600
atcttgatta tgtctctatc tgccagaaga ttaccttata ctattcgctc atctgttgct 660
agcttacaac aaatagcacc aagtattgaa gaagctgctg aaagcttagg aagtagtcgt 720
ctcaatacct ttgctaagat tacaactcca atgatgctat ctggtatcat ttctggagcc 780
atcttatctt ga 792

<210> 184
<211> 744
<212> PRT
<213> Streptococcus pneumoniae

<400> 184
Met Asn Lys Lys Lys Met Ile Leu Thr Ser Leu Ala Ser Val Ala Ile
1               5                   10                  15

Leu Gly Ala Gly Phe Val Thr Ser Gln Pro Thr Phe Val Arg Ala Glu
            20                  25                  30

Glu Ser Pro Gln Val Val Glu Lys Ser Ser Leu Glu Lys Lys Tyr Glu
        35                  40                  45

Glu Ala Lys Ala Lys Ala Asp Thr Ala Lys Lys Asp Tyr Glu Thr Ala
    50                  55                  60

Lys Lys Lys Ala Glu Asp Ala Gln Lys Lys Tyr Glu Asp Asp Gln Lys
65                  70                  75                  80

Arg Thr Glu Glu Lys Ala Arg Lys Glu Ala Glu Ala Ser Gln Lys Leu
                85                  90                  95

Asn Asp Val Ala Leu Val Val Gln Asn Ala Tyr Lys Glu Tyr Arg Glu
            100                 105                 110

Val Gln Asn Gln Arg Ser Lys Tyr Lys Ser Asp Ala Glu Tyr Gln Lys
        115                 120                 125

Lys Leu Thr Glu Val Asp Ser Lys Ile Glu Lys Ala Arg Lys Glu Gln
    130                 135                 140

Gln Asp Leu Gln Asn Lys Phe Asn Glu Val Arg Ala Val Val Val Pro
145                 150                 155                 160

Glu Pro Asn Ala Leu Ala Glu Thr Lys Lys Lys Ala Glu Glu Ala Lys
                165                 170                 175

Ala Glu Glu Lys Val Ala Lys Arg Lys Tyr Asp Tyr Ala Thr Leu Lys
            180                 185                 190

Val Ala Leu Ala Lys Lys Glu Val Glu Ala Lys Glu Leu Glu Ile Glu
        195                 200                 205

Lys Leu Gln Tyr Glu Ile Ser Thr Leu Glu Gln Glu Val Ala Thr Ala
    210                 215                 220

Gln His Gln Val Asp Asn Leu Lys Lys Leu Leu Ala Gly Ala Asp Pro
225                 230                 235                 240

Asp Asp Gly Thr Glu Val Ile Glu Ala Lys Leu Lys Lys Gly Glu Ala
                245                 250                 255

Glu Leu Asn Ala Lys Gln Ala Glu Leu Ala Lys Lys Gln Thr Glu Leu
            260                 265                 270

Glu Lys Leu Leu Asp Ser Leu Asp Pro Glu Gly Lys Thr Gln Asp Glu
        275                 280                 285

Leu Asp Lys Glu Ala Glu Glu Ala Glu Leu Asp Lys Lys Ala Asp Glu
    290                 295                 300

Leu Gln Asn Lys Val Ala Asp Leu Glu Lys Glu Ile Ser Asn Leu Glu
305                 310                 315                 320

Ile Leu Leu Gly Gly Ala Asp Pro Glu Asp Asp Thr Ala Ala Leu Gln
                325                 330                 335

Asn Lys Leu Ala Ala Lys Lys Ala Glu Leu Ala Lys Lys Gln Thr Glu
            340                 345                 350

Leu Glu Lys Leu Leu Asp Ser Leu Asp Pro Glu Gly Lys Thr Gln Asp
        355                 360                 365

Glu Leu Asp Lys Glu Ala Glu Glu Ala Glu Leu Asp Lys Lys Ala Asp
    370                 375                 380

Glu Leu Gln Asn Lys Val Ala Asp Leu Glu Lys Glu Ile Ser Asn Leu
385                 390                 395                 400

Glu Ile Leu Leu Gly Gly Ala Asp Ser Glu Asp Asp Thr Ala Ala Leu
                405                 410                 415

Gln Asn Lys Leu Ala Thr Lys Lys Ala Glu Leu Glu Lys Thr Gln Lys
            420                 425                 430

Glu Leu Asp Ala Ala Leu Asn Glu Leu Gly Pro Asp Gly Asp Glu Glu
        435                 440                 445

Glu Thr Pro Ala Pro Ala Pro Gln Pro Glu Gln Pro Ala Pro Ala Pro
    450                 455                 460

Lys Pro Glu Gln Pro Ala Pro Ala Pro Lys Pro Glu Gln Pro Ala Pro
465                 470                 475                 480

Ala Pro Lys Pro Glu Gln Pro Ala Pro Ala Pro Lys Pro Glu Gln Pro
                485                 490                 495

Ala Pro Ala Pro Lys Pro Glu Gln Pro Ala Lys Pro Glu Lys Pro Ala
            500                 505                 510

Glu Glu Pro Thr Gln Pro Glu Lys Pro Ala Thr Pro Lys Thr Gly Trp
        515                 520                 525

Lys Gln Glu Asn Gly Met Trp Tyr Phe Tyr Asn Thr Asp Gly Ser Met
    530                 535                 540

Ala Ile Gly Trp Leu Gln Asn Asn Gly Ser Trp Tyr Tyr Leu Asn Ala
545                 550                 555                 560

Asn Gly Ala Met Ala Thr Gly Trp Val Lys Asp Gly Asp Thr Trp Tyr
                565                 570                 575

Tyr Leu Glu Ala Ser Gly Ala Met Lys Ala Ser Gln Trp Phe Lys Val
            580                 585                 590

Ser Asp Lys Trp Tyr Tyr Val Asn Ser Asn Gly Ala Met Ala Thr Gly
        595                 600                 605

Trp Leu Gln Tyr Asn Gly Ser Trp Tyr Tyr Leu Asn Ala Asn Gly Asp
    610                 615                 620

Met Ala Thr Gly Trp Leu Gln Tyr Asn Gly Ser Trp Tyr Tyr Leu Asn
625                 630                 635                 640

Ala Asn Gly Asp Met Ala Thr Gly Trp Ala Lys Val Asn Gly Ser Trp
                645                 650                 655

Tyr Tyr Leu Asn Ala Asn Gly Ala Met Ala Thr Gly Trp Ala Lys Val
            660                 665                 670

Asn Gly Ser Trp Tyr Tyr Leu Asn Ala Asn Gly Ser Met Ala Thr Gly
        675                 680                 685

Trp Val Lys Asp Gly Asp Thr Trp Tyr Tyr Leu Glu Ala Ser Gly Ala
    690                 695                 700

Met Lys Ala Ser Gln Trp Phe Lys Val Ser Asp Lys Trp Tyr Tyr Val
705                 710                 715                 720

Asn Gly Leu Gly Ala Leu Ala Val Asn Thr Thr Val Asp Gly Tyr Lys

                725                 730                 735

Val Asn Ala Asn Gly Glu Trp Val
            740

<210> 185
<211> 693
<212> PRT
<213> Streptococcus pneumoniae

<400> 185
Met Phe Ala Ser Lys Ser Glu Arg Lys Val His Tyr Ser Ile Arg Lys
1               5                   10                  15

Phe Ser Val Gly Val Ala Ser Val Val Val Ala Ser Leu Val Met Gly
            20                  25                  30

Ser Val Val His Ala Thr Glu Asn Glu Gly Ala Thr Gln Val Pro Thr
        35                  40                  45

Ser Ser Asn Arg Ala Asn Glu Ser Gln Ala Glu Gln Gly Glu Gln Pro
    50                  55                  60

Lys Lys Leu Asp Ser Glu Arg Asp Lys Ala Arg Lys Glu Val Glu Glu
65                  70                  75                  80

Tyr Val Lys Lys Ile Val Gly Glu Ser Tyr Ala Lys Ser Thr Lys Lys
                85                  90                  95

Arg His Thr Ile Thr Val Ala Leu Val Asn Glu Leu Asn Asn Ile Lys
            100                 105                 110

Asn Glu Tyr Leu Asn Lys Ile Val Glu Ser Thr Ser Glu Ser Gln Leu
        115                 120                 125

Gln Ile Leu Met Met Glu Ser Arg Ser Lys Val Asp Glu Ala Val Ser
    130                 135                 140

Lys Phe Glu Lys Asp Ser Ser Ser Ser Ser Ser Ser Asp Ser Ser Thr
145                 150                 155                 160

Lys Pro Glu Ala Ser Asp Thr Ala Lys Pro Asn Lys Pro Thr Glu Pro
                165                 170                 175

Gly Glu Lys Val Ala Glu Ala Lys Lys Lys Val Glu Glu Ala Glu Lys
            180                 185                 190

Lys Ala Lys Asp Gln Lys Glu Glu Asp Arg Arg Asn Tyr Pro Thr Ile
        195                 200                 205

Thr Tyr Lys Thr Leu Glu Leu Glu Ile Ala Glu Ser Asp Val Glu Val
    210                 215                 220

Lys Lys Ala Glu Leu Glu Leu Val Lys Val Lys Ala Asn Glu Pro Arg
225                 230                 235                 240

Asp Glu Gln Lys Ile Lys Gln Ala Glu Ala Glu Val Glu Ser Lys Gln
                245                 250                 255

Ala Glu Ala Thr Arg Leu Lys Lys Ile Lys Thr Asp Arg Glu Glu Ala
            260                 265                 270

Glu Glu Glu Ala Lys Arg Arg Ala Asp Ala Lys Glu Gln Gly Lys Pro
        275                 280                 285

Lys Gly Arg Ala Lys Arg Gly Val Pro Gly Glu Leu Ala Thr Pro Asp
    290                 295                 300

Lys Lys Glu Asn Asp Ala Lys Ser Ser Asp Ser Ser Val Gly Glu Glu
305                 310                 315                 320

Thr Leu Pro Ser Pro Ser Leu Lys Pro Glu Lys Lys Val Ala Glu Ala
                325                 330                 335

Glu Lys Lys Val Glu Glu Ala Lys Lys Lys Ala Glu Asp Gln Lys Glu
            340                 345                 350

Glu Asp Arg Arg Asn Tyr Pro Thr Asn Thr Tyr Lys Thr Leu Glu Leu
        355                 360                 365

Glu Ile Ala Glu Ser Asp Val Glu Val Lys Lys Ala Glu Leu Glu Leu
    370                 375                 380

Val Lys Glu Glu Ala Lys Glu Pro Arg Asn Glu Glu Lys Val Lys Gln
385                 390                 395                 400

Ala Lys Ala Glu Val Glu Ser Lys Lys Ala Glu Ala Thr Arg Leu Glu
                405                 410                 415

Lys Ile Lys Thr Asp Arg Lys Lys Ala Glu Glu Glu Ala Lys Arg Lys
            420                 425                 430

Ala Ala Glu Glu Asp Lys Val Lys Glu Lys Pro Ala Glu Gln Pro Gln
        435                 440                 445

Pro Ala Pro Ala Pro Lys Ala Glu Lys Pro Ala Pro Ala Pro Lys Pro
    450                 455                 460

Glu Asn Pro Ala Glu Gln Pro Lys Ala Glu Lys Pro Ala Asp Gln Gln
465                 470                 475                 480

Ala Glu Glu Asp Tyr Ala Arg Arg Ser Glu Glu Glu Tyr Asn Arg Leu
                485                 490                 495

Thr Gln Gln Gln Pro Pro Lys Thr Glu Lys Pro Ala Gln Pro Ser Thr
            500                 505                 510

Pro Lys Thr Gly Trp Lys Gln Glu Asn Gly Met Trp Tyr Phe Tyr Asn
        515                 520                 525

Thr Asp Gly Ser Met Ala Thr Gly Trp Leu Gln Asn Asn Gly Ser Trp
    530                 535                 540

Tyr Tyr Leu Asn Ser Asn Gly Ala Met Ala Thr Gly Trp Leu Gln Asn
545                 550                 555                 560

Asn Gly Ser Trp Tyr Tyr Leu Asn Ala Asn Gly Ser Met Ala Thr Gly
                565                 570                 575

Trp Leu Gln Asn Asn Gly Ser Trp Tyr Tyr Leu Asn Ala Asn Gly Ser

580                 585                 590

Met Ala Thr Gly Trp Leu Gln Tyr Asn Gly Ser Trp Tyr Tyr Leu Asn
        595                 600                 605

Ala Asn Gly Ser Met Ala Thr Gly Trp Leu Gln Tyr Asn Gly Ser Trp
    610                 615                 620

Tyr Tyr Leu Asn Ala Asn Gly Asp Met Ala Thr Gly Trp Val Lys Asp
625                 630                 635                 640

Gly Asp Thr Trp Tyr Tyr Leu Glu Ala Ser Gly Ala Met Lys Ala Ser
                645                 650                 655

Gln Trp Phe Lys Val Ser Asp Lys Trp Tyr Tyr Val Asn Gly Ser Gly
            660                 665                 670

Ala Leu Ala Val Asn Thr Thr Val Asp Gly Tyr Gly Val Asn Ala Asn
        675                 680                 685

Gly Glu Trp Val Asn
    690

<210> 186
<211> 129
<212> PRT
<213> Streptococcus pneumoniae

<400> 186
Met Val Lys Arg Arg Ile Arg Arg Gly Thr Arg Glu Pro Glu Lys Val
1               5                   10                  15

Val Val Pro Glu Gln Ser Ser Ile Pro Ser Tyr Pro Val Ser Val Thr
            20                  25                  30

Ser Asn Gln Gly Thr Asp Val Ala Val Glu Pro Ala Lys Ala Val Ala
        35                  40                  45

Pro Thr Thr Asp Trp Lys Gln Glu Asn Gly Met Trp Tyr Phe Tyr Asn
    50                  55                  60

Thr Asp Gly Ser Met Ala Thr Gly Trp Val Gln Val Asn Ser Ser Trp
65                  70                  75                  80

Tyr Tyr Leu Asn Ser Asn Gly Ser Met Lys Val Asn Gln Trp Phe Gln
                85                  90                  95

Val Gly Gly Lys Trp Tyr Tyr Val Asn Thr Ser Gly Glu Leu Ala Val
            100                 105                 110

Asn Thr Ser Ile Asp Gly Tyr Arg Val Asn Asp Asn Gly Glu Trp Val
        115                 120                 125

Arg

<210> 187

<211> 230
<212> PRT
<213> Streptococcus pneumoniae

<400> 187
Leu Asn Thr Ser Phe Val His Ala Ala Asp Gly Ile Gln Tyr Val Arg
1               5                   10                  15

Asp Asp Thr Arg Asp Lys Glu Glu Gly Ile Glu Tyr Asp Asp Ala Asp
            20                  25                  30

Asn Gly Asp Ile Ile Val Lys Val Ala Thr Lys Pro Lys Val Val Thr
        35                  40                  45

Lys Lys Ile Ser Ser Thr Arg Ile Arg Tyr Glu Lys Asp Glu Thr Lys
    50                  55                  60

Asp Arg Ser Glu Asn Pro Val Thr Ile Asp Gly Glu Asp Gly Tyr Val
65                  70                  75                  80

Thr Thr Thr Arg Thr Tyr Asp Val Asn Pro Glu Thr Gly Tyr Val Thr
                85                  90                  95

Glu Gln Val Thr Val Asp Arg Lys Glu Ala Thr Asp Thr Val Ile Lys
            100                 105                 110

Val Pro Ala Lys Ser Lys Val Glu Glu Val Leu Val Pro Phe Ala Thr
        115                 120                 125

Lys Tyr Glu Ala Asp Asn Asp Leu Ser Ala Gly Gln Glu Gln Glu Ile
    130                 135                 140

Thr Leu Gly Lys Asn Gly Lys Thr Val Thr Thr Ile Thr Tyr Asn Val
145                 150                 155                 160

Asp Gly Lys Ser Gly Gln Val Thr Glu Ser Thr Leu Ser Gln Lys Lys
                165                 170                 175

Asp Ser Gln Thr Arg Val Val Lys Lys Arg Thr Lys Pro Gln Val Leu
            180                 185                 190

Val Gln Glu Ile Pro Ile Glu Thr Glu Tyr Leu Asp Gly Pro Thr Leu
        195                 200                 205

Asp Lys Ser Gln Glu Val Glu Glu Val Gly Glu Ile Gly Lys Leu Leu
    210                 215                 220

Leu Leu Gln Ser Ile Leu
225                 230


<210> 188
<211> 340
<212> PRT
<213> Streptococcus pneumoniae

<400> 188
Met Lys Leu Leu Lys Lys Met Met Gln Ile Ala Leu Ala Thr Phe Phe
1               5                   10                  15

Phe Gly Leu Leu Ala Thr Asn Thr Val Phe Ala Asp Asp Ser Glu Gly
            20                  25                  30

Trp Gln Phe Val Gln Glu Asn Gly Arg Thr Tyr Tyr Lys Lys Gly Asp
        35                  40                  45

Leu Lys Glu Thr Tyr Trp Arg Val Ile Asp Gly Lys Tyr Tyr Tyr Phe
    50                  55                  60

Asp Pro Leu Ser Gly Glu Met Val Val Gly Trp Gln Tyr Ile Pro Ala
65                  70                  75                  80

Pro His Lys Gly Val Thr Ile Gly Pro Ser Pro Arg Ile Glu Ile Ala
                85                  90                  95

Leu Arg Pro Asp Trp Phe Tyr Phe Gly Gln Asp Gly Val Leu Gln Glu
            100                 105                 110

Phe Val Gly Lys Gln Val Leu Glu Ala Lys Thr Ala Thr Asn Thr Asn
        115                 120                 125

Lys His His Gly Glu Glu Tyr Asp Ser Gln Ala Glu Lys Arg Val Tyr
    130                 135                 140

Tyr Phe Glu Asp Gln Arg Ser Tyr His Thr Leu Lys Thr Gly Trp Ile
145                 150                 155                 160

Tyr Glu Glu Gly His Trp Tyr Tyr Leu Gln Lys Asp Gly Gly Phe Asp
                165                 170                 175

Ser Arg Ile Asn Arg Leu Thr Val Gly Glu Leu Ala Arg Gly Trp Val
            180                 185                 190

Lys Asp Tyr Pro Leu Thr Tyr Asp Glu Glu Lys Leu Lys Ala Ala Pro
        195                 200                 205

Trp Tyr Tyr Leu Asn Pro Ala Thr Gly Ile Met Gln Thr Gly Trp Gln
    210                 215                 220

Tyr Leu Gly Asn Arg Trp Tyr Tyr Leu His Ser Ser Gly Ala Met Ala
225                 230                 235                 240

Thr Gly Trp Tyr Lys Glu Gly Ser Thr Trp Tyr Tyr Leu Asp Ala Glu
                245                 250                 255

Asn Gly Asp Met Arg Thr Gly Trp Gln Asn Leu Gly Asn Lys Trp Tyr
            260                 265                 270

Tyr Leu Arg Ser Ser Gly Ala Met Ala Thr Gly Trp Tyr Gln Glu Ser
        275                 280                 285

Ser Thr Trp Tyr Tyr Leu Asn Ala Ser Asn Gly Asp Met Lys Thr Gly
    290                 295                 300

Trp Phe Gln Val Asn Gly Asn Trp Tyr Tyr Ala Tyr Asp Ser Gly Ala
305                 310                 315                 320

Leu Ala Val Asn Thr Thr Val Gly Gly Tyr Tyr Leu Asn Tyr Asn Gly
                325                 330                 335

Glu Trp Val Lys

            340

<210> 189
<211> 181
<212> PRT
<213> Streptococcus pneumoniae

<400> 189
Leu Ala Gly Arg Tyr Gly Ser Ala Val Gln Cys Thr Glu Val Thr Ala
1               5                   10                  15

Ser Asn Leu Ser Thr Val Lys Thr Lys Ala Thr Val Val Glu Lys Pro
            20                  25                  30

Leu Lys Asp Phe Arg Ala Ser Thr Ser Asp Gln Ser Gly Trp Val Glu
        35                  40                  45

Ser Asn Gly Lys Trp Tyr Phe Tyr Glu Ser Gly Asp Val Lys Thr Gly
    50                  55                  60

Trp Val Lys Thr Asp Gly Lys Trp Tyr Tyr Leu Asn Asp Leu Gly Val
65                  70                  75                  80

Met Gln Thr Gly Phe Val Lys Phe Ser Gly Ser Trp Tyr Tyr Leu Ser
                85                  90                  95

Asn Ser Gly Ala Met Phe Thr Gly Trp Gly Thr Asp Gly Ser Arg Trp
            100                 105                 110

Phe Tyr Phe Asp Gly Ser Gly Ala Met Lys Thr Gly Trp Tyr Lys Glu
        115                 120                 125

Asn Gly Thr Trp Tyr Tyr Leu Asp Glu Ala Gly Ile Met Lys Thr Gly
    130                 135                 140

Trp Phe Lys Val Gly Pro His Trp Tyr Tyr Ala Tyr Gly Ser Gly Ala
145                 150                 155                 160

Leu Ala Val Ser Thr Thr Thr Pro Asp Gly Tyr Arg Val Asn Gly Asn
                165                 170                 175

Gly Glu Trp Val Asn
            180

<210> 190
<211> 332
<212> PRT
<213> Streptococcus pneumoniae

<400> 190
Met Lys Ile Leu Lys Lys Thr Met Gln Val Gly Leu Thr Val Phe Phe
1               5                   10                  15

Phe Gly Leu Leu Gly Thr Ser Thr Val Phe Ala Asp Asp Ser Glu Gly
            20                  25                  30

Trp Gln Phe Val Gln Glu Asn Gly Arg Thr Tyr Tyr Lys Lys Gly Asp

        35                  40                  45

Leu Lys Glu Thr Tyr Trp Arg Val Ile Asp Gly Lys Tyr Tyr Tyr Phe
    50                  55                  60

Asp Ser Leu Ser Gly Glu Met Val Val Gly Trp Gln Tyr Ile Pro Phe
65                  70                  75                  80

Pro Ser Lys Gly Ser Thr Ile Gly Pro Tyr Pro Asn Gly Ile Arg Leu
                85                  90                  95

Glu Gly Phe Pro Lys Ser Glu Trp Tyr Tyr Phe Asp Lys Asn Gly Val
            100                 105                 110

Leu Gln Glu Phe Val Gly Trp Lys Thr Leu Glu Ile Lys Thr Lys Asp
        115                 120                 125

Ser Val Gly Arg Lys Tyr Gly Glu Lys Arg Glu Asp Ser Glu Asp Lys
    130                 135                 140

Glu Glu Lys Arg Tyr Tyr Thr Asn Tyr Tyr Phe Asn Gln Asn His Ser
145                 150                 155                 160

Leu Glu Thr Gly Trp Leu Tyr Asp Gln Ser Asn Trp Tyr Tyr Leu Ala
                165                 170                 175

Lys Thr Glu Ile Asn Gly Glu Asn Tyr Leu Gly Gly Glu Arg Arg Ala
            180                 185                 190

Gly Trp Ile Asn Asp Asp Ser Thr Trp Tyr Tyr Leu Asp Pro Thr Thr
        195                 200                 205

Gly Ile Met Gln Thr Gly Trp Gln Tyr Leu Gly Asn Lys Trp Tyr Tyr
    210                 215                 220

Leu Arg Ser Ser Gly Ala Met Ala Thr Gly Trp Tyr Gln Glu Gly Thr
225                 230                 235                 240

Thr Trp Tyr Tyr Leu Asp His Pro Asn Gly Asp Met Lys Thr Gly Trp
                245                 250                 255

Gln Asn Leu Gly Asn Lys Trp Tyr Tyr Leu Arg Ser Ser Gly Ala Met
            260                 265                 270

Ala Thr Gly Trp Tyr Gln Asp Gly Ser Thr Trp Tyr Tyr Leu Asn Ala
        275                 280                 285

Gly Asn Gly Asp Met Lys Thr Gly Trp Phe Gln Val Asn Gly Asn Trp
    290                 295                 300

Tyr Tyr Ala Tyr Ser Ser Gly Ala Leu Ala Val Asn Thr Thr Val Asp
305                 310                 315                 320

Gly Tyr Ser Val Asn Tyr Asn Gly Glu Trp Val Arg
                325                 330

<210> 191
<211> 627
<212> PRT

<213> Streptococcus pneumoniae

<400> 191
Met Lys Lys Lys Leu Thr Ser Leu Ala Leu Val Gly Ala Phe Leu Gly
1               5                   10                  15

Leu Ser Trp Tyr Gly Asn Val Gln Ala Gln Glu Ser Ser Gly Asn Lys
            20                  25                  30

Ile His Phe Ile Asn Val Gln Glu Gly Gly Ser Asp Ala Ile Ile Leu
        35                  40                  45

Glu Ser Asn Gly His Phe Ala Met Val Asp Thr Gly Glu Asp Tyr Asp
    50                  55                  60

Phe Pro Asp Gly Ser Asp Ser Arg Tyr Pro Trp Arg Glu Gly Ile Glu
65                  70                  75                  80

Thr Ser Tyr Lys His Val Leu Thr Asp Arg Val Phe Arg Arg Leu Lys
                85                  90                  95

Glu Leu Gly Val Gln Lys Leu Asp Phe Ile Leu Val Thr His Thr His
            100                 105                 110

Ser Asp His Ile Gly Asn Val Asp Glu Leu Leu Ser Thr Tyr Pro Val
        115                 120                 125

Asp Arg Val Tyr Leu Lys Lys Tyr Ser Asp Ser Arg Ile Thr Asn Ser
    130                 135                 140

Glu Arg Leu Trp Asp Asn Leu Tyr Gly Tyr Asp Lys Val Leu Gln Thr
145                 150                 155                 160

Ala Ala Glu Lys Gly Val Ser Val Ile Gln Asn Ile Thr Gln Gly Asp
                165                 170                 175

Ala His Phe Gln Phe Gly Asp Met Asp Ile Gln Leu Tyr Asn Tyr Glu
            180                 185                 190

Asn Glu Thr Asp Ser Ser Gly Glu Leu Lys Lys Ile Trp Asp Asp Asn
        195                 200                 205

Ser Asn Ser Leu Ile Ser Val Val Lys Val Asn Gly Lys Lys Ile Tyr
    210                 215                 220

Leu Gly Gly Asp Leu Asp Asn Val His Gly Ala Glu Asp Lys Tyr Gly
225                 230                 235                 240

Pro Leu Ile Gly Lys Val Asp Leu Met Lys Phe Asn His His His Asp
                245                 250                 255

Thr Asn Lys Ser Asn Thr Lys Asp Phe Ile Lys Asn Leu Ser Pro Ser
            260                 265                 270

Leu Ile Val Gln Thr Ser Asp Ser Leu Pro Trp Lys Asn Gly Val Asp
        275                 280                 285

Ser Glu Tyr Val Asn Trp Leu Lys Glu Arg Gly Ile Glu Arg Ile Asn
    290                 295                 300

Ala Ala Ser Lys Asp Tyr Asp Ala Thr Val Phe Asp Ile Arg Lys Asp

305                 310                 315                 320

Gly Phe Val Asn Ile Ser Thr Ser Tyr Lys Pro Ile Pro Ser Phe Gln
                325                 330                 335

Ala Gly Trp His Lys Ser Ala Tyr Gly Asn Trp Trp Tyr Gln Ala Pro
            340                 345                 350

Asp Ser Thr Gly Glu Tyr Ala Val Gly Trp Asn Glu Ile Glu Gly Glu
        355                 360                 365

Trp Tyr Tyr Phe Asn Gln Thr Gly Ile Leu Leu Gln Asn Gln Trp Lys
    370                 375                 380

Lys Trp Asn Asn His Trp Phe Tyr Leu Thr Asp Ser Gly Ala Ser Ala
385                 390                 395                 400

Lys Asn Trp Lys Lys Ile Ala Gly Ile Trp Tyr Tyr Phe Asn Lys Glu
                405                 410                 415

Asn Gln Met Glu Ile Gly Trp Ile Gln Asp Lys Glu Gln Trp Tyr Tyr
            420                 425                 430

Leu Asp Val Asp Gly Ser Met Lys Thr Gly Trp Leu Gln Tyr Met Gly
        435                 440                 445

Gln Trp Tyr Tyr Phe Ala Pro Ser Gly Glu Met Lys Met Gly Trp Val
    450                 455                 460

Lys Asp Lys Glu Thr Trp Tyr Tyr Met Asp Ser Thr Gly Val Met Lys
465                 470                 475                 480

Thr Gly Glu Ile Glu Val Ala Gly Gln His Tyr Tyr Leu Glu Asp Ser
                485                 490                 495

Gly Ala Met Lys Gln Gly Trp His Lys Lys Ala Asn Asp Trp Tyr Phe
            500                 505                 510

Tyr Lys Thr Asp Gly Ser Arg Ala Val Gly Trp Ile Lys Asp Lys Asp
        515                 520                 525

Lys Trp Tyr Phe Leu Lys Glu Asn Gly Gln Leu Leu Val Asn Gly Lys
    530                 535                 540

Thr Pro Glu Gly Tyr Thr Val Asp Ser Ser Gly Ala Trp Leu Val Asp
545                 550                 555                 560

Val Ser Ile Glu Lys Ser Ala Thr Ile Lys Thr Thr Ser His Ser Glu
                565                 570                 575

Ile Lys Glu Ser Lys Glu Val Val Lys Lys Asp Leu Glu Asn Lys Glu
            580                 585                 590

Thr Ser Gln His Glu Ser Val Thr Asn Phe Ser Thr Ser Gln Asp Leu
        595                 600                 605

Thr Ser Ser Thr Ser Gln Ser Ser Glu Thr Ser Val Asn Lys Ser Glu
    610                 615                 620

Ser Glu Gln
625

<210> 192
<211> 318
<212> PRT
<213> Streptococcus pneumoniae

<400> 192
Met Glu Ile Asn Val Ser Lys Leu Arg Thr Asp Leu Pro Gln Val Gly
1               5                   10                  15

Val Gln Pro Tyr Arg Gln Val His Ala His Ser Thr Gly Asn Pro His
            20                  25                  30

Ser Thr Val Gln Asn Glu Ala Asp Tyr His Trp Arg Lys Asp Pro Glu
        35                  40                  45

Leu Gly Phe Phe Ser His Ile Val Gly Asn Gly Cys Ile Met Gln Val
    50                  55                  60

Gly Pro Val Asp Asn Gly Ala Trp Asp Val Gly Gly Gly Trp Asn Ala
65                  70                  75                  80

Glu Thr Tyr Ala Ala Val Glu Leu Ile Glu Ser His Ser Thr Lys Glu
                85                  90                  95

Glu Phe Met Thr Asp Tyr Arg Leu Tyr Ile Glu Leu Leu Arg Asn Leu
            100                 105                 110

Ala Asp Glu Ala Gly Leu Pro Lys Thr Leu Asp Thr Gly Ser Leu Ala
        115                 120                 125

Gly Ile Lys Thr His Glu Tyr Cys Thr Asn Asn Gln Pro Asn Asn His
    130                 135                 140

Ser Asp His Val Asp Pro Tyr Pro Tyr Leu Ala Lys Trp Gly Ile Ser
145                 150                 155                 160

Arg Glu Gln Phe Lys His Asp Ile Glu Asn Gly Leu Thr Ile Glu Thr
                165                 170                 175

Gly Trp Gln Lys Asn Asp Thr Gly Tyr Trp Tyr Val His Ser Asp Gly
            180                 185                 190

Ser Tyr Pro Lys Asp Lys Phe Glu Lys Ile Asn Gly Thr Trp Tyr Tyr
        195                 200                 205

Phe Asp Ser Ser Gly Tyr Met Leu Ala Asp Arg Trp Arg Lys His Thr
    210                 215                 220

Asp Gly Asn Trp Tyr Trp Phe Asp Asn Ser Gly Glu Met Ala Thr Gly
225                 230                 235                 240

Trp Lys Lys Ile Ala Asp Lys Trp Tyr Tyr Phe Asn Glu Glu Gly Ala
                245                 250                 255

Met Lys Thr Gly Trp Val Lys Tyr Lys Asp Thr Trp Tyr Tyr Leu Asp
            260                 265                 270

Ala Lys Glu Gly Ala Met Val Ser Asn Ala Phe Ile Gln Ser Ala Asp

275                 280                 285

Gly Thr Gly Trp Tyr Tyr Leu Lys Pro Asp Gly Thr Leu Ala Asp Lys
    290                 295                 300

Pro Glu Phe Thr Val Glu Pro Asp Gly Leu Ile Thr Val Lys
305                 310                 315

<210> 193
<211> 513
<212> PRT
<213> Streptococcus pneumoniae

<400> 193
Met Gly Thr Thr Gly Phe Thr Ile Ile Asp Leu Ile Ile Leu Ile Val
  1               5                  10                  15

Tyr Leu Leu Ala Val Leu Val Ala Gly Ile Tyr Phe Ser Lys Lys Glu
             20                  25                  30

Met Lys Gly Lys Glu Phe Phe Lys Gly Asp Gly Ser Val Pro Trp Tyr
         35                  40                  45

Val Thr Ser Val Ser Ile Phe Ala Thr Met Leu Ser Pro Ile Ser Phe
     50                  55                  60

Leu Gly Leu Ala Gly Ser Ser Tyr Ala Gly Ser Trp Ile Leu Trp Phe
 65                  70                  75                  80

Ala Gln Leu Gly Met Val Val Ala Ile Pro Leu Thr Ile Arg Phe Ile
                 85                  90                  95

Leu Pro Ile Phe Ala Arg Ile Asp Ile Asp Thr Ala Tyr Asp Tyr Leu
            100                 105                 110

Asp Lys Arg Phe Asn Ser Lys Ala Leu Arg Ile Ile Ser Ala Leu Leu
        115                 120                 125

Phe Ile Ile Tyr Gln Leu Gly Arg Met Ser Ile Ile Met Tyr Leu Pro
    130                 135                 140

Ser Ala Gly Leu Ser Val Leu Thr Gly Ile Asp Ile Asn Ile Leu Ile
145                 150                 155                 160

Ile Leu Met Gly Val Val Ala Ile Val Tyr Ser Tyr Thr Gly Gly Leu
                165                 170                 175

Lys Ser Val Leu Trp Thr Asp Phe Ile Gln Gly Val Ile Leu Ile Ser
            180                 185                 190

Gly Val Val Leu Ala Leu Phe Val Leu Ile Ala Asn Ile Lys Gly Gly
        195                 200                 205

Phe Gly Ala Val Ala Glu Thr Leu Ala Asn Gly Lys Phe Leu Ala Ala
    210                 215                 220

Asn Glu Lys Leu Phe Asp Pro Asn Leu Leu Ser Asn Ser Ile Phe Leu
225                 230                 235                 240

Ile Val Met Gly Ser Gly Phe Thr Ile Leu Ser Ser Tyr Ala Ser Ser
                245                 250                 255

Gln Asp Leu Val Gln Arg Phe Thr Thr Thr Gln Asn Ile Lys Lys Leu
            260                 265                 270

Asn Lys Met Leu Phe Thr Asn Gly Val Leu Ser Leu Ala Thr Ala Thr
        275                 280                 285

Val Phe Tyr Leu Ile Gly Thr Gly Leu Tyr Val Phe Tyr Gln Val Gln
    290                 295                 300

Asn Ala Asp Ser Ala Ala Ser Asn Ile Pro Gln Asp Gln Ile Phe Met
305                 310                 315                 320

Tyr Phe Ile Ala Tyr Gln Leu Pro Val Gly Ile Thr Gly Leu Ile Leu
                325                 330                 335

Ala Ala Ile Tyr Ala Ala Ser Gln Ser Thr Ile Ser Thr Gly Leu Asn
            340                 345                 350

Ser Val Ala Thr Ser Trp Thr Leu Asp Ile Gln Asp Val Ile Ser Lys
        355                 360                 365

Asn Met Ser Asp Asn Arg Arg Thr Lys Ile Ala Gln Phe Val Ser Leu
    370                 375                 380

Ala Val Gly Leu Phe Ser Ile Gly Val Ser Ile Val Met Ala His Ser
385                 390                 395                 400

Asp Ile Lys Ser Ala Tyr Glu Trp Phe Asn Ser Phe Met Gly Leu Val
                405                 410                 415

Leu Gly Leu Leu Gly Gly Val Phe Ile Leu Gly Phe Val Ser Lys Lys
            420                 425                 430

Ala Asn Lys Gln Gly Ala Tyr Ala Ala Leu Ile Val Ser Thr Ile Val
        435                 440                 445

Met Val Phe Ile Lys Tyr Phe Leu Pro Pro Thr Ala Val Ser Tyr Trp
    450                 455                 460

Ala Tyr Ser Leu Ile Ser Ile Ser Val Ser Val Val Ser Gly Tyr Ile
465                 470                 475                 480

Val Ser Val Leu Thr Gly Asn Lys Val Ser Ala Pro Lys Tyr Thr Thr
                485                 490                 495

Ile His Asp Ile Thr Glu Ile Lys Ala Asp Ser Ser Trp Glu Val Arg
            500                 505                 510

His

<210> 194
<211> 826
<212> PRT
<213> Streptococcus pneumoniae

<400> 194
Met Lys Ile Asn Lys Lys Tyr Leu Ala Gly Ser Val Ala Val Leu Ala
1               5                   10                  15

Leu Ser Val Cys Ser Tyr Glu Leu Gly Arg His Gln Ala Gly Gln Asp
            20                  25                  30

Lys Lys Glu Ser Asn Arg Val Ala Tyr Ile Asp Gly Asp Gln Ala Gly
        35                  40                  45

Gln Lys Ala Glu Asn Leu Thr Pro Asp Glu Val Ser Lys Arg Glu Gly
    50                  55                  60

Ile Asn Ala Glu Gln Ile Val Ile Lys Ile Thr Asp Gln Gly Tyr Val
65                  70                  75                  80

Thr Ser His Gly Asp His Tyr His Tyr Tyr Asn Gly Lys Val Pro Tyr
                85                  90                  95

Asp Ala Ile Ile Ser Glu Glu Leu Leu Met Lys Asp Pro Asn Tyr Gln
            100                 105                 110

Leu Lys Asp Ser Asp Ile Val Asn Glu Ile Lys Gly Gly Tyr Val Ile
        115                 120                 125

Lys Val Asp Gly Lys Tyr Tyr Val Tyr Leu Lys Asp Ala Ala His Ala
    130                 135                 140

Asp Asn Ile Arg Thr Lys Glu Glu Ile Lys Arg Gln Lys Gln Glu Arg
145                 150                 155                 160

Ser His Asn His Gly Ser Gly Ala Asn Asp His Ala Val Ala Ala Ala
                165                 170                 175

Arg Ala Gln Gly Arg Tyr Thr Thr Asp Asp Gly Tyr Ile Phe Asn Ala
            180                 185                 190

Ser Asp Ile Ile Glu Asp Thr Gly Asp Ala Tyr Ile Val Pro His Gly
        195                 200                 205

Asp His Tyr His Tyr Ile Pro Lys Asn Glu Leu Ser Ala Ser Glu Leu
    210                 215                 220

Ala Ala Ala Glu Ala Tyr Trp Asn Gly Lys Gln Gly Ser Arg Pro Ser
225                 230                 235                 240

Ser Ser Ser Ser Tyr Asn Ala Asn Pro Ala Gln Pro Arg Leu Ser Glu
                245                 250                 255

Asn His Asn Leu Thr Val Thr Pro Thr Tyr His Gln Asn Gln Gly Glu
            260                 265                 270

Asn Ile Ser Ser Leu Leu Arg Glu Leu Tyr Ala Lys Pro Leu Ser Glu
        275                 280                 285

Arg His Val Glu Ser Asp Gly Leu Ile Phe Asp Pro Ala Gln Ile Thr
    290                 295                 300

Ser Arg Thr Ala Arg Gly Val Ala Val Pro His Gly Asn His Tyr His
305                 310                 315                 320

Phe Ile Pro Tyr Glu Gln Met Ser Glu Leu Glu Lys Arg Ile Ala Arg
                325                 330                 335

Ile Ile Pro Leu Arg Tyr Arg Ser Asn His Trp Val Pro Asp Ser Arg
            340                 345                 350

Pro Glu Gln Pro Ser Pro Gln Ser Thr Pro Glu Pro Ser Pro Ser Pro
        355                 360                 365

Gln Pro Ala Pro Asn Pro Gln Pro Ala Pro Ser Asn Pro Ile Asp Glu
    370                 375                 380

Lys Leu Val Lys Glu Ala Val Arg Lys Val Gly Asp Gly Tyr Val Phe
385                 390                 395                 400

Glu Glu Asn Gly Val Ser Arg Tyr Ile Pro Ala Lys Asp Leu Ser Ala
                405                 410                 415

Glu Thr Ala Ala Gly Ile Asp Ser Lys Leu Ala Lys Gln Glu Ser Leu
            420                 425                 430

Ser His Lys Leu Gly Ala Lys Lys Thr Asp Leu Pro Ser Ser Asp Arg
        435                 440                 445

Glu Phe Tyr Asn Lys Ala Tyr Asp Leu Leu Ala Arg Ile His Gln Asp
    450                 455                 460

Leu Leu Asp Asn Lys Gly Arg Gln Val Asp Phe Glu Ala Leu Asp Asn
465                 470                 475                 480

Leu Leu Glu Arg Leu Lys Asp Val Pro Ser Asp Lys Val Lys Leu Val
                485                 490                 495

Asp Asp Ile Leu Ala Phe Leu Ala Pro Ile Arg His Pro Glu Arg Leu
            500                 505                 510

Gly Lys Pro Asn Ala Gln Ile Thr Tyr Thr Asp Asp Glu Ile Gln Val
        515                 520                 525

Ala Lys Leu Ala Gly Lys Tyr Thr Thr Glu Asp Gly Tyr Ile Phe Asp
    530                 535                 540

Pro Arg Asp Ile Thr Ser Asp Glu Gly Asp Ala Tyr Val Thr Pro His
545                 550                 555                 560

Met Thr His Ser His Trp Ile Lys Lys Asp Ser Leu Ser Glu Ala Glu
                565                 570                 575

Arg Ala Ala Ala Gln Ala Tyr Ala Lys Glu Lys Gly Leu Thr Pro Pro
            580                 585                 590

Ser Thr Asp His Gln Asp Ser Gly Asn Thr Glu Ala Lys Gly Ala Glu
        595                 600                 605

Ala Ile Tyr Asn Arg Val Lys Ala Ala Lys Lys Val Pro Leu Asp Arg
    610                 615                 620

Met Pro Tyr Asn Leu Gln Tyr Thr Val Glu Val Lys Asn Gly Ser Leu
625                 630                 635                 640

Ile Ile Pro His Tyr Asp His Tyr His Asn Ile Lys Phe Glu Trp Phe

645                 650                 655

Asp Glu Gly Leu Tyr Glu Ala Pro Lys Gly Tyr Thr Leu Glu Asp Leu
            660                 665                 670

Leu Ala Thr Val Lys Tyr Tyr Val Glu His Pro Asn Glu Arg Pro His
        675                 680                 685

Ser Asp Asn Gly Phe Gly Asn Ala Ser Asp His Val Gln Arg Asn Lys
    690                 695                 700

Asn Gly Gln Ala Asp Thr Asn Gln Thr Glu Lys Pro Ser Glu Glu Lys
705                 710                 715                 720

Pro Gln Thr Glu Lys Pro Glu Glu Glu Thr Pro Arg Glu Glu Lys Pro
                725                 730                 735

Gln Ser Glu Lys Pro Glu Ser Pro Lys Pro Thr Glu Glu Pro Glu Glu
            740                 745                 750

Ser Pro Glu Glu Ser Glu Glu Pro Gln Val Glu Thr Glu Lys Val Glu
        755                 760                 765

Glu Lys Leu Arg Glu Ala Glu Asp Leu Leu Gly Lys Ile Gln Asp Pro
    770                 775                 780

Ile Ile Lys Ser Asn Ala Lys Glu Thr Leu Thr Gly Leu Lys Asn Asn
785                 790                 795                 800

Leu Leu Phe Gly Thr Gln Asp Asn Asn Thr Ile Met Ala Glu Ala Glu
                805                 810                 815

Lys Leu Leu Ala Leu Leu Lys Glu Ser Lys
            820                 825

<210> 195
<211> 295
<212> PRT
<213> Streptococcus pneumoniae

<400> 195
Met Glu Gly Leu Val Arg Val His Leu Leu Pro Val Phe Gly Asp Tyr
1               5                   10                  15

Lys Leu Ser Lys Leu Thr Thr Pro Ile Leu Gln Gln Gln Val Asn Lys
            20                  25                  30

Trp Ala Asp Lys Ala Asn Lys Gly Glu Lys Gly Ala Phe Ala Asn Tyr
        35                  40                  45

Ser Leu Leu His Asn Met Asn Lys Arg Ile Leu Lys Tyr Gly Val Ala
    50                  55                  60

Ile Gln Val Ile Gln Tyr Asn Pro Ala Asn Asp Val Ile Val Pro Arg
65                  70                  75                  80

Lys Gln Gln Lys Glu Lys Ala Ala Val Lys Tyr Leu Asp Asn Lys Glu
                85                  90                  95

Leu Lys Gln Phe Leu Asp Tyr Leu Asp Ala Leu Asp Gln Ser Asn Tyr
            100                 105                 110

Glu Asn Leu Phe Asp Val Val Leu Tyr Lys Thr Leu Leu Ala Thr Gly
        115                 120                 125

Cys Arg Ile Ser Glu Ala Leu Ala Leu Glu Trp Ser Asp Ile Asp Leu
    130                 135                 140

Glu Ser Gly Val Ile Ser Ile Asn Lys Thr Leu Asn Arg Tyr Gln Glu
145                 150                 155                 160

Ile Asn Ser Pro Lys Ser Ser Ala Gly Tyr Arg Asp Ile Pro Ile Asp
                165                 170                 175

Lys Ala Thr Leu Leu Leu Leu Lys Gln Tyr Lys Asn Arg Gln Gln Ile
            180                 185                 190

Gln Ser Trp Lys Leu Gly Arg Ser Glu Thr Val Val Phe Ser Val Phe
        195                 200                 205

Thr Glu Lys Tyr Ala Tyr Ala Cys Asn Leu Arg Lys Arg Leu Asn Lys
    210                 215                 220

His Phe Asp Ala Ala Gly Val Thr Asn Val Ser Phe His Gly Phe Arg
225                 230                 235                 240

His Thr His Thr Thr Met Met Leu Tyr Ala Gln Val Ser Pro Lys Asp
                245                 250                 255

Val Gln Tyr Arg Leu Gly His Ser Asn Leu Met Ile Thr Glu Asn Thr
            260                 265                 270

Tyr Trp His Thr Asn Gln Glu Asn Ala Lys Lys Ala Val Ser Asn Tyr
        275                 280                 285

Glu Thr Ala Ile Asn Asn Leu
    290                 295

<210> 196
<211> 2235
<212> DNA
<213> Streptococcus pneumoniae

<400> 196
atgaataaga aaaaaatgat tttaacaagt ctagccagcg tcgctatctt aggggctggt 60
tttgttacgt ctcagcctac ttttgtaaga gcagaagaat ctccacaagt tgtcgaaaaa 120
tcttcattag agaagaaata tgaggaagca aaagcaaaag ctgatactgc caagaaagat 180
tacgaaacgg ctaaaaagaa agcagaagac gctcagaaaa agtatgaaga tgatcagaag 240
agaactgagg agaaagctcg aaaagaagca gaagcatctc aaaaattgaa tgatgtggcg 300
cttgttgttc aaaatgcata taaagagtac cgagaagttc aaaatcaacg tagtaaatat 360
aaatctgacg ctgaatatca gaaaaaatta acagaggtcg actctaaaat agagaaggct 420
aggaaagagc aacaggactt gcaaaataaa tttaatgaag taagagcagt tgtagttcct 480
gaaccaaatg cgttggctga gactaagaaa aaagcagaag aagctaaagc agaagaaaaa 540
gtagctaaga gaaaatatga ttatgcaact ctaaaggtag cactagcgaa gaaagaagta 600
gaggctaagg aacttgaaat tgaaaaactt caatatgaaa tttctacttt ggaacaagaa 660
gttgctactg ctcaacatca agtagataat ttgaaaaaac ttcttgctgg tgcggatcct 720
gatgatggca cagaagttat agaagctaaa ttaaaaaaag gagaagctga gctaaacgct 780
aaacaagctg agttagcaaa aaaacaaaca gaacttgaaa aacttcttga cagccttgat 840

cctgaaggta agactcagga tgaattagat aaagaagcag aagaagctga gttggataaa 900
aaagctgatg aacttcaaaa taaagttgct gatttagaaa aagaaattag taaccttgaa 960
atattacttg gaggggctga tcctgaagat gatactgctg ctcttcaaaa taaattagct 1020
gctaaaaaag ctgagttagc aaaaaaacaa acagaacttg aaaaacttct tgacagcctt 1080
gatcctgaag gtaagactca ggatgaatta gataaagaag cagaagaagc tgagttggat 1140
aaaaaagctg atgaacttca aaataaagtt gctgatttag aaaaagaaat tagtaacctt 1200
gaaatattac ttggaggggc tgattctgaa gatgatactg ctgctcttca aaataaatta 1260
gctactaaaa aagctgaatt ggaaaaaact caaaaagaat tagatgcagc tcttaatgag 1320
ttaggccctg atggagatga agaagaaact ccagcgccgg ctcctcaacc agagcaacca 1380
gctcctgcac caaaaccaga gcaaccagct ccagctccaa aaccagagca accagctcct 1440
gcaccaaaac cagagcaacc agctccagct ccaaaaccag agcaaccagc tccagctcca 1500
aaaccagagc aaccagctaa gccggagaaa ccagctgaag agcctactca accagaaaaa 1560
ccagccactc caaaaacagg ctggaaacaa gaaaacggta tgtggtattt ctacaatact 1620
gatggttcaa tggcaatagg ttggctccaa aacaacggtt catggtacta cctaaacgct 1680
aacggcgcta tggcaacagg ttgggtgaaa gatggagata cctggtacta tcttgaagca 1740
tcaggtgcta tgaaagcaag ccaatggttc aaagtatcag ataaatggta ctatgtcaac 1800
agcaatggcg ctatggcgac aggctggctc caatacaatg gctcatggta ctacctcaac 1860
gctaatggtg atatggcgac aggatggctc caatacaacg gttcatggta ttacctcaac 1920
gctaatggtg atatggcgac aggatgggct aaagtcaacg gttcatggta ctacctaaac 1980
gctaacggtg ctatggctac aggttgggct aaagtcaacg gttcatggta ctacctaaac 2040
gctaacggtt caatggcaac aggttgggtg aaagatggag atacctggta ctatcttgaa 2100
gcatcaggtg ctatgaaagc aagccaatgg ttcaaagtat cagataaatg gtactatgtc 2160
aatggcttag gtgcccttgc agtcaacaca actgtagatg gctataaagt caatgccaat 2220
ggtgaatggg tttaa 2235

<210> 197
<211> 2082
<212> DNA
<213> Streptococcus pneumoniae

<400> 197
atgtttgcat caaaaagcga aagaaaagta cattattcaa ttcgtaaatt tagtgttgga 60
gtagctagtg tagttgttgc cagtcttgtt atgggaagtg tggttcatgc gacagagaac 120
gagggagcta cccaagtacc cacttcttct aatagggcaa atgaaagtca ggcagaacaa 180
ggagaacaac ctaaaaaact cgattcagaa cgagataagg caaggaaaga ggtcgaggaa 240
tatgtaaaaa aaatagtggg tgagagctat gcaaaatcaa ctaaaaagcg acatacaatt 300
actgtagctc tagttaacga gttgaacaac attaagaacg agtatttgaa taaaatagtt 360
gaatcaacct cagaaagcca actacagata ctgatgatgg agagtcgatc aaaagtagat 420
gaagctgtgt ctaagtttga aaaggactca tcttcttcgt caagttcaga ctcttccact 480
aaaccggaag cttcagatac agcgaagcca aacaagccga cagaaccagg agaaaaggta 540
gcagaagcta agaagaaggt tgaagaagct gagaaaaaag ccaaggatca aaaagaagaa 600
gatcgtcgta actacccaac cattacttac aaaacgcttg aacttgaaat tgctgagtcc 660
gatgtggaag ttaaaaaagc ggagcttgaa ctagtaaaag tgaaagctaa cgaacctcga 720
gacgagcaaa aaattaagca agcagaagcg gaagttgaga gtaaacaagc tgaggctaca 780
aggttaaaaa aaatcaagac agatcgtgaa gaagcagaag aagaagctaa acgaagagca 840
gatgctaaag agcaaggtaa accaaagggg cggcaaaaac gaggagttcc tggagagcta 900
gcaacacctg ataaaaaaga aaatgatgcg aagtcttcag attctagcgt aggtgaagaa 960
actcttccaa gcccatccct gaaaccagaa aaaaaggtag cagaagctga gaagaaggtt 1020
gaagaagcta agaaaaaagc cgaggatcaa aaagaagaag atcgccgtaa ctacccaacc 1080
aatacttaca aaacgcttga acttgaaatt gctgagtccg atgtggaagt taaaaaagcg 1140
gagcttgaac tagtaaaaga ggaagctaag gaacctcgaa acgaggaaaa agttaagcaa 1200
gcaaaagcgg aagttgagag taaaaaagct gaggctacaa ggttagaaaa aatcaagaca 1260
gatcgtaaaa aagcagaaga agaagctaaa cgaaaagcag cagaagaaga taaagttaaa 1320
gaaaaaccag ctgaacaacc acaaccagcg ccggctccaa aagcagaaaa accagctcca 1380
gctccaaaac cagagaatcc agctgaacaa ccaaaagcag aaaaaccagc tgatcaacaa 1440
gctgaagaag actatgctcg tagatcagaa gaagaatata atcgcttgac tcaacagcaa 1500
ccgccaaaaa ctgaaaaacc agcacaacca tctactccaa aaacaggctg gaaacaagaa 1560
aacggtatgt ggtacttcta caatactgat ggttcaatgg cgacaggatg gctccaaaac 1620
aatggctcat ggtactacct caacagcaat ggcgctatgg cgacaggatg gctccaaaac 1680
aatggttcat ggtactatct aaacgctaat ggttcaatgg caacaggatg gctccaaaac 1740

aatggttcat ggtactacct aaacgctaat ggttcaatgg cgacaggatg gctccaatac 1800
aatggctcat ggtactacct aaacgctaat ggttcaatgg cgacaggatg gctccaatac 1860
aatggctcat ggtactacct aaacgctaat ggtgatatgg cgacaggttg ggtgaaagat 1920
ggagatacct ggtactatct tgaagcatca ggtgctatga aagcaagcca atggttcaaa 1980
gtatcagata aatggtacta tgtcaatggc tcaggtgccc ttgcagtcaa cacaactgta 2040
gatggctatg gagtcaatgc caatggtgaa tgggtaaact aa 2082

<210> 198
<211> 390
<212> DNA
<213> Streptococcus pneumoniae

<400> 198
atggtaaaaa gacgtataag gagagggacg agagaacctg aaaaagttgt tgttcctgag 60
caatcatcta ttccttcgta tcctgtatct gttacatcta accaaggaac agatgtagca 120
gtagaaccag ctaaagcagt tgctccaaca acagactgga aacaagaaaa tggtatgtgg 180
tatttttata atactgatgg ttccatggca acaggttggg tacaagttaa tagttcatgg 240
tactacctca acagcaacgg ttctatgaaa gtcaatcaat ggttccaagt tggtggtaaa 300
tggtattatg taaatacatc gggtgagtta gcggtcaata caagtataga tggctataga 360
gtcaatgata atggtgaatg ggtgcgttaa 390

<210> 199
<211> 693
<212> DNA
<213> Streptococcus pneumoniae

<400> 199
ctgaatacaa gttttgttca tgctgctgat gggattcaat atgtcagaga tgatactaga 60
gataaagaag agggaataga gtatgatgac gctgacaatg gggatattat tgtaaaagta 120
gcgactaaac ctaaggtagt aaccaagaaa atttcaagta cgcgaattcg ttatgaaaaa 180
gatgaaacaa aagaccgtag tgaaaatcct gttacaattg atggagagga tggctatgta 240
actacgacaa ggacctacga tgttaatcca gagactggtt atgttaccga acaggttact 300
gttgatagaa aagaagccac ggatacagtt atcaaagttc cagctaaaag caaggttgaa 360
gaagttcttg ttccatttgc tactaaatat gaagcagaca atgacctttc tgcaggacag 420
gagcaagaga ttactctagg aaagaatggg aaaacagtta caacgataac ttataatgta 480
gatggaaaga gtggacaagt aactgagagt actttaagtc aaaaaaaaga ctctcaaaca 540
agagttgtta aaaaaagaac caagccccaa gttcttgtcc aagaaattcc aatcgaaaca 600
gaatatctcg atggcccaac tcttgataaa agtcaagaag tagaagaagt aggagaaatt 660
ggtaaattac tcttactaca atctatactg tag 693

<210> 200
<211> 1023
<212> DNA
<213> Streptococcus pneumoniae

<400> 200
atgaagcttt tgaaaaaaat gatgcaaatc gcactagcca catttttctt cggtttgtta 60
gcgacaaata cagtatttgc agatgattct gaaggatggc agtttgtcca agaaaatggt 120
agaacctact acaaaaaggg ggatctaaaa gaaacctact ggagagtgat agatgggaag 180
tactattatt ttgatccttt atccggagag atggttgtcg gctggcaata tatacctgct 240
ccacacaagg gggttacgat tggtccttct ccaagaatag agattgctct tagaccagat 300
tggttttatt ttggtcaaga tggtgtatta caagaatttg ttggcaagca agttttagaa 360
gcaaaaactg ctacgaatac caacaaacat catggggaag aatatgatag ccaagcagag 420
aaacgagtct attattttga agatcagcgt agttatcata ctttaaaaac tggttggatt 480
tatgaagagg gtcattggta ttatttacag aaggatggtg gctttgattc gcgcatcaac 540
agattgacgg ttggagagct agcacgtggt tgggttaagg attaccctct tacgtatgat 600
gaagagaagc taaaagcagc tccatggtac tatctaaatc cagcaactgg cattatgcaa 660
acaggttggc aatatctagg taatagatgg tactacctcc attcgtcagg agctatggca 720

actggctggt ataaggaagg ctcaacttgg tactatctag atgctgaaaa tggtgatatg 780
agaactggct ggcaaaacct tgggaacaaa tggtactatc tccgttcatc aggagctatg 840
gcaactggtt ggtatcagga aagttcgact tggtactatc taaatgcaag taatggagat 900
atgaaaacag gctggttcca agtcaatggt aactggtact atgcctatga ttcaggtgct 960
ttagctgtta ataccacagt aggtggttac tacttaaact ataatggtga atgggttaag 1020
taa 1023

<210> 201
<211> 546
<212> DNA
<213> Streptococcus pneumoniae

<400> 201
ttggctggta gatatggttc tgctgttcag tgtacagaag tgactgcctc aaacctttca 60
acagttaaaa ctaaagctac ggttgtagaa aaaccactga aagattttag agcgtctacg 120
tctgatcagt ctggttgggt ggaatctaat ggtaaatggt atttctatga gtctggtgat 180
gtgaagacag gttgggtgaa aacagatggt aaatggtact atttgaatga cttaggtgtc 240
atgcagactg gatttgtaaa attttctggt agctggtatt acttgagcaa ttcaggtgct 300
atgtttacag gctggggaac agatggtagc agatggttct actttgacgg ctcaggagct 360
atgaagacag gctggtacaa ggaaaatggc acttggtatt accttgacga agcaggtatc 420
atgaagacag gttggtttaa agtcggacca cactggtact atgcctacgg ttcaggagct 480
ttggctgtga gcacaacaac accagatggt taccgtgtaa atggtaatgg tgaatgggta 540
aactag 546

<210> 202
<211> 999
<212> DNA
<213> Streptococcus pneumoniae

<400> 202
atgaaaattt tgaaaaaaac tatgcaagtt ggactgacag tatttttctt tggtttgcta 60
gggaccagta cagtatttgc agatgattct gaaggatggc agtttgtcca agaaaacgga 120
agaacctact acaaaaaggg ggacctcaaa gaaacctact ggcgagtgat tgatggtaag 180
tactattatt ttgattctct atctggagag atggttgtcg gctggcaata tatcccgttt 240
ccatctaaag gtagtacaat tggtccttac ccaaatggta tcagattaga aggttttcca 300
aagtcagagt ggtactactt cgataaaaat ggagtgctac aagagtttgt tggttggaaa 360
acattagaga ttaaaactaa agacagtgtt ggaagaaagt acggggaaaa acgtgaagat 420
tcagaagata aagaagagaa gcgttattat acgaactatt actttaatca aaatcattct 480
ttagagacag gttggcttta tgatcagtct aactggtatt atctagctaa gacggaaatt 540
aatggagaaa actaccttgg tggtgaaaga cgtgcggggt ggataaacga tgattcgact 600
tggtactacc tagatccaac aactggtatt atgcaaacag gttggcaata tctaggtaat 660
aagtggtact acctccgttc ctcaggagca atggccactg gctggtatca ggaaggtacc 720
acttggtatt atttagacca cccaaatggc gatatgaaaa caggttggca aaaccttggg 780
aacaaatggt actatctccg ttcatcagga gctatggcaa ctggttggta tcaagatggt 840
tcaacttggt actacctaaa tgcaggtaat ggagacatga agacaggttg gttccaggtc 900
aatggcaact ggtactatgc ttatagctca ggtgctttgg cagtgaatac gaccgtagat 960
ggctattctg tcaactataa tggcgaatgg gttcggtaa 999

<210> 203
<211> 1884
<212> DNA
<213> Streptococcus pneumoniae

<400> 203
atgaaaaaga aattaactag tttagcactt gtaggcgctt ttttaggttt gtcatggtat 60
gggaatgttc aggctcaaga aagttcagga aataaaatcc actttatcaa tgttcaagaa 120
ggtggcagtg atgcgattat tcttgaaagc aatggacatt ttgccatggt ggatacagga 180
gaagattatg atttcccaga tggaagtgat tctcgctatc catggagaga aggaattgaa 240

acgtcttata agcatgttct aacagaccgt gtctttcgtc gtttgaagga attgggtgtc 300
caaaaacttg attttatttt ggtgacccat acccacagtg atcatattgg aaatgttgat 360
gaattactgt ctacctatcc agttgaccga gtctatctta agaaatatag tgatagtcgt 420
attactaatt ctgaacgtct atgggataat ctgtatggct atgataaggt tttacagact 480
gctgcagaaa aaggtgtttc agttattcaa aatatcacac aaggggatgc tcattttcag 540
tttggggaca tggatattca gctctataat tatgaaaatg aaactgattc atcgggtgaa 600
ttaaagaaaa tttgggatga caattccaat tccttgatta gcgtggtgaa agtcaatggc 660
aagaaaattt accttggggg cgatttagat aatgttcatg gagcagaaga caagtatggt 720
cctctcattg gaaaagttga tttgatgaag tttaatcatc accatgatac caacaaatca 780
aataccaagg atttcattaa aaatttgagt ccgagtttga ttgttcaaac ttcggatagt 840
ctaccttgga aaaatggtgt tgatagtgag tatgttaatt ggctcaaaga acgaggaatt 900
gagagaatca acgcagccag caaagactat gatgcaacag tttttgatat tcgaaaagac 960
ggttttgtca atatttcaac atcctacaag ccgattccaa gttttcaagc tggttggcat 1020
aagagtgcat atgggaactg gtggtatcaa gcgcctgatt ctacaggaga gtatgctgtc 1080
ggttggaatg aaatcgaagg tgaatggtat tactttaacc aaacgggtat cttgttacag 1140
aatcaatgga aaaaatggaa caatcattgg ttctatttga cagactctgg tgcttctgct 1200
aaaaattgga agaaaatcgc tggaatctgg tattatttta acaaagaaaa ccagatggaa 1260
attggttgga ttcaagataa agagcagtgg tattatttgg atgttgatgg ttctatgaag 1320
acaggatggc ttcaatatat ggggcaatgg tattactttg ctccatcagg ggaaatgaaa 1380
atgggctggg taaaagataa agaaacctgg tactatatgg attctactgg tgtcatgaag 1440
acaggtgaga tagaagttgc tggtcaacat tattatctgg aagattcagg agctatgaag 1500
caaggctggc ataaaaaggc aaatgattgg tatttctaca agacagacgg ttcacgagct 1560
gtgggttgga tcaaggacaa ggataaatgg tacttcttga aagaaaatgg tcaattactt 1620
gtgaacggta agacaccaga aggttatact gtggattcaa gtggtgcctg gttagtggat 1680
gtttcgatcg agaaatctgc tacaattaaa actacaagtc attcagaaat aaaagaatcc 1740
aaagaagtag tgaaaaagga tcttgaaaat aaagaaacga gtcaacatga aagtgttaca 1800
aatttttcaa ctagtcaaga tttgacatcc tcaacttcac aaagctctga aacgagtgta 1860
aacaaatcgg aatcagaaca gtag 1884

<210> 204
<211> 957
<212> DNA
<213> Streptococcus pneumoniae

<400> 204
atggaaatta atgtgagtaa attaagaaca gatttgcctc aagtcggcgt gcaaccatat 60
aggcaagtac acgcacactc aactgggaat ccgcattcaa ccgtacagaa tgaagcggat 120
tatcactggc ggaaagaccc agaattaggt tttttctcgc acattgttgg gaacggttgc 180
atcatgcagg taggacctgt tgataatggt gcctgggacg ttgggggcgg ttggaatgct 240
gagacctatg cagcggttga actgattgaa agccattcaa ccaaagaaga gttcatgacg 300
gactaccgcc tttatatcga actcttacgc aatctagcag atgaagcagg tttgccgaaa 360
acgcttgata cagggagttt agctggaatt aaaacgcacg agtattgcac gaataaccaa 420
ccaaacaacc actcagacca cgttgaccct tatccatatc ttgctaaatg gggcattagc 480
cgtgagcagt ttaagcatga tattgagaac ggcttgacga ttgaaacagg ctggcagaag 540
aatgacactg gctactggta cgtacattca gacggctctt atccaaaaga caagtttgag 600
aaaatcaatg gcacttggta ctactttgac agttcaggct atatgcttgc agaccgctgg 660
aggaagcaca cagacggcaa ctggtactgg ttcgacaact caggcgaaat ggctacaggc 720
tggaagaaaa tcgctgataa gtggtactat ttcaacgaag aaggtgccat gaagacaggc 780
tgggtcaagt acaaggacac ttggtactac ttagacgcta aagaaggcgc catggtatca 840
aatgccttta tccagtcagc ggacggaaca ggctggtact acctcaaacc agacggaaca 900
ctggcagaca agccagaatt cacagtagag ccagatggct tgattacagt aaaataa 957

<210> 205
<211> 1542
<212> DNA
<213> Streptococcus pneumoniae

<400> 205
atgggcacaa caggatttac aataattgac ttaattatct tgattgttta tttacttgcg 60

gtgttggttg caggtatcta tttctctaaa aaagagatga aaggaaaaga gttctttaaa 120
ggagatggtt cggttccttg gtatgttact tcggtatcca tttttgccac aatgctcagt 180
ccgatttcct tcttgggact cgctggtagc tcttatgcag gtagctggat tttatggttt 240
gctcaattag ggatggtagt agctattcca ctgacaattc gttttatctt acctatcttt 300
gcacggatag acatcgatac ggcatatgat tacttggata aacgttttaa ttctaaagca 360
cttcgtatta tttcagcact cttgtttatt atttatcaat tgggacgtat gtctatcatt 420
atgtacctcc catcagctgg tttatcagta ttgacaggaa ttgacatcaa tattttgatt 480
attttgatgg gtgtagttgc aattgtttat tcttatactg gtggtctaaa atccgtatta 540
tggacagact ttattcaagg tgtgattctg attagtggtg tcgttttagc tttatttgta 600
ctgattgcta atattaaagg tggctttggt gcagtagcag aaacattagc aaacgggaaa 660
ttccttgctg caaatgaaaa acttttcgat cctaacttgc tttcaaactc catcttttta 720
attgtgatgg gttcaggctt tacaatcttg tcttcctatg cttcatctca agatttggtt 780
caacgtttta ctacaacaca aaatattaag aaacttaata agatgttgtt cacaaacggt 840
gttttgtcac ttgcaactgc aacagtcttt tacttgattg gtacaggctt gtacgtattc 900
tatcaagtac aaaatgcaga tagtgcagct agcaatatcc ctcaagacca aatctttatg 960
tactttattg cataccagtt accagtaggt atcacaggtt tgatcttggc agcgatttat 1020
gcagcatctc aatcaactat ttcaacaggt ttgaactctg ttgcaacttc atggacattg 1080
gatattcaag atgtcatttc taaaaatatg tcagacaatc gtcgtacgaa aattgcacaa 1140
ttcgtatctc tagcagtagg tttattctca attggtgttt ccattgtcat ggctcactca 1200
gatattaaat ctgcatacga atggttcaat agtttcatgg gacttgtact tggtctactt 1260
ggtggtgtat ttattcttgg atttgtttct aaaaaagcaa ataaacaagg tgcttatgca 1320
gcgctgattg tatcaaccat cgtcatggta tttattaaat acttccttcc tccaacagct 1380
gttagctact gggcatattc attgatttca atctctgtat cagtagtttc aggttatatt 1440
gtatctgttc ttactggaaa taaagtatct gcacctaaat atacaacgat tcatgatatt 1500
acagaaatta aagcggattc aagttgggaa gttcgtcact aa 1542

<210> 206
<211> 2481
<212> DNA
<213> Streptococcus pneumoniae

<400> 206
atgaaaatta ataaaaaata tctagcaggt tcagtggcag tccttgccct aagtgtttgt 60
tcctatgagc ttggtcgtca ccaagctggt caggataaga aagagtctaa tcgagttgct 120
tatatagatg gtgatcaggc tggtcaaaag gcagaaaact tgacaccaga tgaagtcagt 180
aagagggagg ggatcaacgc cgaacaaatc gtcatcaaga ttacggatca aggttatgtg 240
acctctcatg gagaccatta tcattactat aatggcaagg tcccttatga tgccatcatc 300
agtgaagagc tcctcatgaa agatccgaat tatcagttga aggattcaga cattgtcaat 360
gaaatcaagg gtggttatgt catcaaggta gacggaaaat actatgttta ccttaaggat 420
gcagctcatg cggataatat tcggacaaaa gaagagatta aacgtcagaa gcaggaacgc 480
agtcataatc acgggtcagg agctaacgat catgcagtag ctgcagccag agcccaagga 540
cgctatacaa cggatgatgg gtatatcttc aatgcatctg atatcattga ggacacgggt 600
gatgcttata tcgttcctca cggcgaccat taccattaca ttcctaagaa tgagttatca 660
gctagcgagt tagctgctgc agaagcctat tggaatggga agcagggatc tcgtccttct 720
tcaagttcta gttataatgc aaatccagct caaccaagat tgtcagagaa ccacaatctg 780
actgtcactc caacttatca tcaaaatcaa ggggaaaaca tttcaagcct tttacgtgaa 840
ttgtatgcta aacccttatc agaacgccat gtggaatctg atggccttat tttcgaccca 900
gcgcaaatca caagtcgaac cgccagaggt gtagctgtcc ctcatggtaa ccattaccac 960
tttatccctt atgaacaaat gtctgaattg gaaaaacgaa ttgctcgtat tattcccctt 1020
cgttatcgtt caaaccattg ggtaccagat tcaagaccag aacaaccaag tccacaatcg 1080
actccggaac ctagtccaag tccgcaacct gcaccaaatc ctcaaccagc tccaagcaat 1140
ccaattgatg agaaattggt caaagaagct gttcgaaaag taggcgatgg ttatgtcttt 1200
gaggagaatg gagtttctcg ttatatccca gccaaggatc tttcagcaga aacagcagca 1260
ggcattgata gcaaactggc caagcaggaa agtttatctc ataagctagg agctaagaaa 1320
actgacctcc catctagtga tcgagaattt tacaataagg cttatgactt actagcaaga 1380
attcaccaag atttacttga taataaaggt cgacaagttg attttgaggc tttggataac 1440
ctgttggaac gactcaagga tgtcccaagt gataaagtca agttagtgga tgatattctt 1500
gccttcttag ctccgattcg tcatccagaa cgtttaggaa aaccaaatgc gcaaattacc 1560
tacactgatg atgagattca agtagccaag ttggcaggca agtacacaac agaagacggt 1620
tatatctttg atcctcgtga tataaccagt gatgaggggg atgcctatgt aactccacat 1680

atgacccata gccactggat taaaaaagat agtttgtctg aagctgagag agcggcagcc 1740
caggcttatg ctaaagagaa aggtttgacc cctccttcga cagaccatca ggattcagga 1800
aatactgagg caaaaggagc agaagctatc tacaaccgcg tgaaagcagc taagaaggtg 1860
ccacttgatc gtatgcctta caatcttcaa tatactgtag aagtcaaaaa cggtagttta 1920
atcatacctc attatgacca ttaccataac atcaaatttg agtggtttga cgaaggcctt 1980
tatgaggcac ctaaggggta tactcttgag gatcttttgg cgactgtcaa gtactatgtc 2040
gaacatccaa acgaacgtcc gcattcagat aatggttttg gtaacgctag cgaccatgtt 2100
caaagaaaca aaaatggtca agctgatacc aatcaaacgg aaaaaccaag cgaggagaaa 2160
cctcagacag aaaaacctga ggaagaaacc cctcgagaag agaaaccgca aagcgagaaa 2220
ccagagtctc caaaaccaac agaggaacca gaagaatcac cagaggaatc agaagaacct 2280
caggtcgaga ctgaaaaggt tgaagaaaaa ctgagagagg ctgaagattt acttggaaaa 2340
atccaggatc caattatcaa gtccaatgcc aaagagactc tcacaggatt aaaaaataat 2400
ttactatttg gcacccagga caacaatact attatggcag aagctgaaaa actattggct 2460
ttattaaagg agagtaagta a 2481

<210> 207
<211> 888
<212> DNA
<213> Streptococcus pneumoniae

<400> 207
atggagggat tggttagagt gcatttattg cctgtatttg gcgattacaa gctatctaaa 60
cttactacgc ctattcttca acagcaagta aacaaatggg ctgacaaggc aaataaaggc 120
gaaaaagggg catttgctaa ctactctttg ctccataaca tgaataagcg tattttgaaa 180
tatggcgtag ctatccaggt aatacaatac aacccagcta atgatgtcat cgttccacgc 240
aaacagcaaa aagaaaaggc tgctgtcaaa tacttagaca acaaagaatt aaaacagttt 300
cttgattatt tagatgctct ggatcaatca aattatgaga acttatttga tgttgttctg 360
tataagactt tattggccac tggttgccgt attagtgagg ctctggctct tgaatggtct 420
gatattgacc tagaaagcgg tgttatcagc atcaataaga cactaaaccg ctatcaggaa 480
ataaactcac ctaaatcaag cgctggttat cgtgatatac caatagacaa agccacatta 540
cttttactga aacaatacaa aaaccgtcaa caaattcagt cttggaaatt aggccgatct 600
gaaacagttg tattctctgt atttacggag aaatatgctt atgcttgtaa cttacgcaaa 660
cgcctaaata agcattttga tgctgctgga gtaactaacg tatcatttca tggtttccgc 720
catacacata ctactatgat gctctatgct caggttagcc cgaaagatgt tcagtataga 780
ttaggccact ctaatttaat gatcactgaa aatacttact ggcatactaa ccaagagaat 840
gcaaaaaaag ccgtctcaaa ttatgaaaca gctatcaaca atttataa 888

<210> 208
<211> 624
<212> DNA
<213> Streptococcus pneumoniae

<400> 208
atggaagagt tagtgacctt agattgtttg tttattgaca gaactaagat tgaagccaat 60
gccaacaagt atagttttgt gtggaagaaa acgacagaga aattctccgc caaacttcaa 120
gaacagatac aggtctattt tcaagaagaa atcactcccc ttctgattaa atatgccatg 180
tttgataaga aacaaaagag agggtataaa gagtcagcta aaaacttagc gaattggcac 240
tataatgaca aggaggatag ctacacacat cctgatggct ggtattatcg ttttcaccat 300
accaaatatc agaaaacaca gacagacttt caacaagaaa tcaaggttta ctacgccgac 360
gaacctgaat cagcccctca aaagggactg tatatgaacg aacgctatca aaacttgaaa 420
gctaaagaat gtcaggcgct tttatctccc caaggtagac agattttcgc tcaacgcaag 480
attgatgtgg aacctgtctt tgggcagata aaggcttctt tgggttacaa gagatgtaat 540
ctgagaggga agcgtcaagt gagaattgac atgggattgg tacttatggc caataacctc 600
ctaaaatata gtaaaatgaa ataa 624

<210> 209
<211> 6702
<212> DNA

<213> Streptococcus pneumoniae

<400> 209
atggggaaag gccattggaa tcggaaaaga gtttatagca ttcgtaagtt tgctgtggga 60
gcttgctcag taatgattgg gacttgtgca gttttattag gaggaaatat agctggagaa 120
tctgtagttt atgcggatga aacacttatt actcatactg ctgagaaacc taaagaggaa 180
aaaatgatag tagaagaaaa ggctgataaa gctttggaaa ctaaaaatat agttgaaagg 240
acagaacaaa gtgaacctag ttcaactgag gctattgcat ctgagaagaa agaagatgaa 300
gccgtaactc caaaagagga aaaagtgtct gctaaaccgg aagaaaaagc tccaaggata 360
gaatcacaag cttcaaatca agaaaaaccg ctcaaggaag atgctaaagc tgtaacaaat 420
gaagaagtga atcaaatgat tgaagacagg aaagtggatt ttaatcaaaa ttggtacttt 480
aaactcaatg caaattctaa ggaagccatt aaacctgatg cagacgtatc tacgtggaaa 540
aaattagatt taccgtatga ctggagtatc tttaacgatt tcgatcatga atctcctgca 600
caaaatgaag gtggacagct caacggtggg gaagcttggt atcgcaagac tttcaaacta 660
gatgaaaaag acctcaagaa aaatgttcgc cttacttttg atggcgtcta catggattct 720
caagtttatg tcaatggtca gttagtgggg cattatccaa atggttataa ccagttctca 780
tatgatatca ccaaatacct tcaaaaagat ggtcgtgaga atgtgattgc tgtccatgca 840
gtcaacaaac agccaagtag ccgttggtat tcaggaagtg gtatctatcg tgatgtgact 900
ttacaagtga cagataaggt gcatgttgag aaaaatggga caactatttt aacaccaaaa 960
cttgaagaac aacaacatgg caaggttgaa actcatgtga ccagcaaaat cgtcaatacg 1020
gacgacaaag accatgaact tgtagccgaa tatcaaatcg ttgaacgagg tggtcatgct 1080
gtaacaggct tagttcgtac agcgagtcgt accttaaaag cacatgaatc aacaagccta 1140
gatgcgattt tagaagttga aagaccaaaa ctctggactg ttttaaatga caaacctgcc 1200
ttgtacgaat tgattacgcg tgtttaccgt gacggtcaat tggttgatgc taagaaggat 1260
ttgtttggtt accgttacta tcactggact ccaaatgaag gtttctcttt gaatggtgaa 1320
cgtattaaat tccatggagt atccttgcac cacgaccatg gggcgcttgg agcagaagaa 1380
aactataaag cagaatatcg ccgtctcaaa caaatgaagg agatgggagt taactccatc 1440
cgtacaaccc acaaccctgc tagtgagcaa accttgcaaa tcgcagcaga actaggttta 1500
ctcgttcagg aagaggcctt tgatacgtgg tatggtggca agaaacctta tgactatgga 1560
cgtttctttg aaaaagatgc cactcaccca gaagctcgaa aaggtgaaaa atggtctgat 1620
tttgacctac gtaccatggt cgaaagaggc aaaaacaacc ctgctatctt catgtggtca 1680
attggtaatg aaataggtga agctaatggt gatgcccact ctttagcaac tgttaaacgt 1740
ttggttaagg ttatcaagga tgttgataag actcgctatg ttaccatggg agcagataaa 1800
ttccgtttcg gtaatggtag cggagggcat gagaaaattg ctgatgaact cgatgctgtt 1860
ggatttaact attctgaaga taattacaaa gcccttagag ctaagcatcc aaaatggttg 1920
atttatggat cagaaacatc ttcagctacc cgtacacgtg gaagttacta tcgccctgaa 1980
cgtgaattga aacatagcaa tggacctgag cgtaattatg aacagtcaga ttatggaaat 2040
gatcgtgtgg gttgggggaa aacagcaacc gcttcatgga cttttgaccg tgacaacgct 2100
ggctatgctg gacagtttat ctggacaggt acggactata ttggtgaacc tacaccatgg 2160
cacaaccaaa atcaaactcc tgttaagagc tcttactttg gtatcgtaga tacagccggc 2220
attccaaaac atgacttcta tctctaccaa agccaatggg tttctgttaa gaagaaaccg 2280
atggtacacc ttcttcctca ctggaactgg gaaaacaaag aattagcatc caaagtagct 2340
gactcagaag gtaagattcc agttcgtgct tattcgaatg cttctagtgt agaattgttc 2400
ttgaatggaa aatctcttgg tcttaagact ttcaataaaa aacaaaccag cgatgggcgg 2460
acttaccaag aaggtgcaaa tgctaatgaa ctttatcttg aatggaaagt tgcctatcaa 2520
ccaggtacct tggaagcaat tgctcgtgat gaatctggca aggaaattgc tcgagataag 2580
attacgactg ctggtaagcc agcggcagtt cgtcttatta aggaagacca tgcgattgca 2640
gcagatggaa aagacttgac ttacatctac tatgaaattg ttgacagcca ggggaatgtg 2700
gttccaactg ctaataatct ggttcgcttc caattgcatg gccaaggtca actggtcggt 2760
gtagataacg gagaacaagc cagccgtgaa cgctataagg cgcaagcaga tggttcttgg 2820
attcgtaaag catttaatgg taaaggtgtt gccattgtca aatcaactga acaagcaggg 2880
aaattcaccc tgactgccca ctctgatctc ttgaaatcga accaagtcac tgtctttact 2940
ggtaagaaag aaggacaaga gaagactgtt ttggggacag aagtgccaaa agtacagacc 3000
attattggag aggcacctga aatgcctacc actgttccgt ttgtatacag tgatggtagc 3060
cgtgcagaac gtcctgtaac ctggtcttca gtagatgtga gcaagcctgg tattgtaacg 3120
gtgaaaggta tggctgacgg acgagaagta gaagctcgtg tagaagtgat tgctcttaaa 3180
tcagagctac cagttgtgaa acgtattgct ccaaatactg acttgaattc tgtagacaaa 3240
tctgtttcct atgttttgat tgatggaagt gttgaagagt atgaagtgga caagtgggag 3300
attgccgaag aagataaagc taagttagca attccaggtt ctcgtattca agcgaccggt 3360
tatttagaag gtcaaccaat tcatgcaacc cttgtggtag aagaaggcaa tcctgcggca 3420
cctgcagtac caactgtaac ggttggtggt gaggcagtaa caggtcttac tagtcaaaaa 3480

ccaatgcaat accgcactct tgcttatgga gctaagttgc cagaagtcac agcaagtgct 3540
aaaaatgcag ctgttacagt tcttcaagca agcgcagcaa acggcatgcg tgcgagcatc 3600
tttattcagc ctaaagatgg tggccctctt caaacctatg caattcaatt ccttgaagaa 3660
gcgccaaaaa ttgctcactt gagcttgcaa gtggaaaaag ctgacagtct caaagaagac 3720
caaactgtca aattgtcggt tcgagctcac tatcaagatg gaacgcaagc tgtattacca 3780
gctgataaag taaccttctc tacaagtggt gaaggggaag tcgcaattcg taaaggaatg 3840
cttgagttgc ataagccagg agcagtcact ctgaacgctg aatatgaggg agctaaagac 3900
caagttgaac tcactatcca agccaatact gagaagaaga ttgcgcaatc catccgtcct 3960
gtaaatgtag tgacagattt gcatcaggaa ccaagtcttc cagcaacagt aacagttgag 4020
tatgacaaag gtttccctaa aactcataaa gtcacttggc aagctattcc gaaagaaaaa 4080
ctagactcct atcaaacatt tgaagtacta ggtaaagttg aaggaattga ccttgaagcg 4140
cgtgcaaaag tctctgtaga aggtatcgtt tcagttgaag aagtcagtgt gacaactcca 4200
atcgcagaag caccacaatt accagaaagt gttcggacat atgattcaaa tggtcacgtt 4260
tcatcagcta aggttgcatg ggatgcgatt cgtccagagc aatacgctaa ggaaggtgtc 4320
tttacagtta atggtcgctt agaaggtacg caattaacaa ctaaacttca tgttcgcgta 4380
tctgctcaaa ctgagcaagg tgcaaacatt tctgaccaat ggaccggttc agaattgcca 4440
cttgcctttg cttcagactc aaatccaagc gacccagttt caaatgttaa tgacaagctc 4500
atttcctaca ataaccaacc agccaatcgt tggacaaact ggaatcgtac taatccagaa 4560
gcttcagtcg gtgttctgtt tggagattca ggtatcttga gcaaacgctc cgttgataat 4620
ctaagtgtcg gattccatga agaccatgga gttggtgtac cgaagtctta tgtgattgag 4680
tattatgttg gtaagactgt cccaacagct cctaaaaacc ctagttttgt tggtaatgag 4740
gaccatgtct ttaatgattc tgccaactgg aaaccagtta ctaatctaaa agcccctgct 4800
caactcaagg ctggagaaat gaaccacttt agctttgata aagttgaaac ctatgctgtt 4860
cgtattcgca tggttaaagc agataacaag cgtggaacgt ctatcacaga ggtacaaatc 4920
tttgcgaaac aagttgcggc agccaagcaa ggacaaacaa gaatccaagt tgacggcaaa 4980
gacttagcaa acttcaaccc tgatttgaca gactactacc ttgagtctgt agatggaaaa 5040
gttccggcag tcacagcaag tgttagcaac aatggtctcg ctaccgtcgt tccaagcgtt 5100
cgtgaaggtg agccagttcg tgtcatcgcg aaagctgaaa atggcgacat cttaggagaa 5160
taccgtctgc acttcactaa ggataagagc ttactttctc ataaaccagt tgctgcggtt 5220
aaacaagctc gcttgctaca agtaggtcaa gcacttgaat tgccgactaa ggttccagtt 5280
tacttcacag gtaaagacgg ctacgaaaca aaagacctga cagttgaatg ggaagaagtt 5340
ccagcggaaa atctgacaaa agcaggtcaa tttactgttc gaggccgtgt ccttggtagt 5400
aaccttgttg ctgagatcac tgtacgagtg acagacaaac ttggtgagac tctttcagat 5460
aaccctaact atgatgaaaa cagtaaccag gcctttgctt cagcaaccaa tgatattgac 5520
aaaaactctc atgaccgcgt tgactatctc aatgacggag atcattcaga aaatcgtcgt 5580
tggacaaact ggtcaccaac accatcttct aatccagaag tatcagcggg tgtgattttc 5640
cgtgaaaatg gtaagattgt agaacggact gttacacaag gaaaagttca gttctttgca 5700
gatagtggta cggatgcacc atctaaactc gttttagaac gctatgtcgg tccagagttt 5760
gaagtgccaa cctactattc aaactaccaa gcctacgacg cagaccatcc attcaacaat 5820
ccagaaaatt gggaagctgt tccttatcgt gcggataaag acattgcagc tggtgatgaa 5880
atcaacgtaa catttaaagc tatcaaagcc aaagctatga gatggcgtat ggagcgtaaa 5940
gcagataaga gcggtgttgc gatgattgag atgaccttcc ttgcaccaag tgaattgcct 6000
caagaaagca ctcaatcaaa gattcttgta gatggaaaag aacttgctga tttcgctgaa 6060
aatcgtcaag actatcaaat tacctataaa ggtcaacggc caaaagtctc agttgaagaa 6120
aacaatcaag tagcttcaac tgtggtagat agtggagaag atagctttcc agtacttgtt 6180
cgcctcgttt cagaaagtgg aaaacaagtc aaggaatacc gtatccactt gactaaggaa 6240
aaaccagttt ctgagaagac agttgctgct gtacaagaag atcttccaaa aatcgaattt 6300
gttgaaaaag atttggcata caagacagtt gagaaaaaag attcaacact gtatctaggt 6360
gaaactcgtg tagaacaaga aggaaaagtt ggaaaagaac gtatctttac agcgattaat 6420
cctgatggaa gtaaggaaga aaaactccgt gaagtggtag aagttccgac agaccgcatc 6480
gtcttggttg gaaccaaacc agtagctcaa gaagctaaaa aaccacaagt gtcagaaaaa 6540
gcagatacaa aaccaattga ttcaagtgaa gctagtcaaa ctaataaagc ccagttacca 6600
agtacaggta gtgcggcaag ccaagcagca gtagcagcag gtttaactct tctaggtttg 6660
agtgcaggat tagtagttac taaaggtaaa aaagaagact ag 6702

<210> 210
<211> 1476
<212> DNA
<213> Streptococcus pneumoniae

<400> 210
atggatgcaa tctttgacct aatcggaaag gttttcaatc ccatcttaga aatgggtgga 60
cctgtcatca tgttaatcat tttgacagta ttggctttac tttttggagt gaaattctcc 120
aaagcgcttg aaggtggtat caaacttgcc atcgctctta caggtatcgg tgctatcatc 180
ggtatgctaa acactgcttt ctcagcatca ctagcaaaat tcgttgaaaa cactggtatc 240
caattgagta ttaccgacgt tggttgggca ccacttgcta caatcacttg gggttctgct 300
tggacactat acttcttgct catcatgttg attgtcaaca tagtgatgct agctatgaag 360
aaaacagata cacttgatgt cgatatcttt gatatctggc acttgtctat cacaggtctc 420
ttgattaaat ggtatgctga taacaatggt gtgagtcaag gggtttcact ctttattgct 480
acagcagcta tcgtccttgt cggtgtgttg aaaattatca actctgactt gatgaaacct 540
acatttgatg accttcttaa cgccccaagt tcatcaccaa tgacatcaac tcacatgaac 600
tacatgatga acccagttat catggttttg gataagattt ttgaaaaatt cttcccaggc 660
cttgataaat atgactttga tgctgctaaa ttgaacaaga aaatcggttt ctggggatct 720
aaattcttca tcggtttcat ccttggtatc gttatcggta ttatgggaac tccacatcca 780
attgcaggtg ttgcagatgc agataaatgg cgtcttgtta tcaaaggatg gttgtctctt 840
ggtttgactg ccggtgtatc tttggaactc ttctcactta tcggttcatg gttcatcgca 900
gccgtagaac cactatcaca aggtattaca aacgttgcta ctaaacgtct tcaaggacgt 960
aaattcaata tcggtcttga ctggccattc atcgctggtc gtgctgaaat ctgggcttgt 1020
gccaacgtac ttgcaccaat catgttgatt gaagcagtgc ttctttcaaa agttggaaat 1080
ggtatcttgc cacttgcagg tatcatcgct atgggtgtta ctccagctct cttggttgta 1140
actcgtggta aattgctccg tatgattatc ttcggaacac tcttgttgcc actcttcctt 1200
ctttcaggta cacttattgc accatttgca acagaacttg ctaaaggtgt aggtgccttc 1260
ccagaaggtg tgagccaaac tcaattgatt actcactcta ctcttgaagg accaatcgaa 1320
aaacttcttg gttggacaat tggtaacact acaactggtg atatcaaagc aatccttggt 1380
gcagtagtct tccttgtatt ctatatcggt atctttgctt ggtacagaaa acaaatgatc 1440
aaacgtaacg aagagtacgc agcaaaagca aaataa 1476

<210> 211
<211> 1413
<212> DNA
<213> Streptococcus pneumoniae

<400> 211
atgaagatta tgaaaaaaaa atattggact ttagcgatat tattcttttg tttgttcaat 60
aattctgtta ctgctcaaga aatacctaaa aatcttgatg gcaatataac tcacactcag 120
actagcgaaa gtttttctga atctgatgaa aaacaggttg actattctaa taaaaatcaa 180
gaagaagtag accaaaataa atttcgtatt caaatcgata agacagaatt atttgtaaca 240
acagataaac atttagaaaa aaactgttgt aaattggaac ttgaaccaca aataaataac 300
gatattgtta actctgaaag taataattta ctaggcgaag ataatttaga taataaaatt 360
aaggaaaatg tttctcatct agataataga ggaggaaata tagagcatga caaagataac 420
ttagaatcgt cgattgtaag aaaatatgaa tgggatatag ataaagttac tggtggaggc 480
gaaagttata aattatattc taaaagtaat tctaaagttt caattgctat tttagattca 540
ggagtcgatt tacaaaatac tggattactg aaaaatcttt caaatcactc aaaaaactat 600
gtccccaata aaggatattt aggaaaagag gagggagagg aaggaataat atcagatatt 660
caagatagat taggtcatgg tacggctgtt gtagctcaaa ttgtagggga tgacaatatt 720
aatggagtaa atcctcacgt taatattaac gtctatagaa tatttggtaa gtcgtcagct 780
agtccagatt ggattgtaaa agcaattttt gatgctgtag atgatggcaa tgatattatc 840
aatcttagta ctggacaata tttaatgatt gatggagaat atgaggacgg aacaaatgat 900
tttgaaacat ttttgaagta taaaaaggct attgattacg cgaatcaaaa aggagtaatt 960
atagtagctg cattagggaa tgactcccta aatgtatcaa atcagtcaga tttattgaaa 1020
cttattagtt cacgcaaaaa agtaagaaaa ccaggattag tagttgatgt tccaagttat 1080
ttctcatcta caatttcggt cggaggcata gatcgcttag gtaatttatc agattttagc 1140
aataaagggg attctgatgc aatatatgcg cctgcaggct caacattatc tctttcagaa 1200
ttaggactta ataactttat taatgcagaa aaatataaag aagattggat tttttcggca 1260
acactaggag gatatacgta tctttatgga aactcatttg ctgctcctaa agtttctggt 1320
gcgattgcaa tgattattga taaatacaaa ttaaaagatc agccctataa ttatatgttt 1380
gtaaaaaaat tctggaagaa acattaccag taa 1413

<210> 212

<211> 1008
<212> DNA
<213> Streptococcus pneumoniae

<400> 212
atgaagaaaa catggaaagt gtttttaacg cttgtaacag ctcttgtagc tgttgtgctt 60
gtggcctgtg gtcaaggaac tgcttctaaa gacaacaaag aggcagaact taagaaggtt 120
gactttatcc tagactggac accaaatacc aaccacacag ggctttatgt tgccaaggaa 180
aaaggttatt tcaaagaagc tggagtggat gttgatttga aattgccacc agaagaaagt 240
tcttctgact tggttatcaa cggaaaggca ccatttgcag tgtatttcca agactacatg 300
gctaagaaat tggaaaaagg agcaggaatc actgccgttg cagctattgt tgaacacaat 360
acatcaggaa tcatctctcg taaatctgat aatgtaagca gtccaaaaga cttggttggt 420
aagaaatatg ggacatggaa tgacccaact gaacttgcta tgttgaaaac cttggtagaa 480
tctcaaggtg gagactttga gaaggttgaa aaagtaccaa ataacgactc aaactcaatc 540
acaccgattg ccaatggcgt ctttgatact gcttggattt actacggttg ggatggtatc 600
cttgctaaat ctcaaggtgt agatgctaac ttcatgtact tgaaagacta tgtcaaggag 660
tttgactact attcaccagt tatcatcgca aacaacgact atctgaaaga taacaaagaa 720
gaagctcgca aagtcatcca agccatcaaa aaaggctacc aatatgccat ggaacatcca 780
gaagaagctg cagatattct catcaagaat gcacctgaac tcaaggaaaa acgtgacttt 840
gtcatcgaat ctcaaaaata cttgtcaaaa gaatacgcaa gcgacaagga aaaatggggt 900
caatttgacg cagctcgctg gaatgctttc tacaaatggg ataaagaaaa tggtatcctt 960
aaagaagact tgacagacaa aggcttcacc aacgaatttg tgaaataa 1008

<210> 213
<211> 1332
<212> DNA
<213> Streptococcus pneumoniae

<400> 213
atgataaaaa atcctaaatt attaaccaag tcttttttaa gaagttttgc aattctaggt 60
ggtgttggtc tagtcattca tatagctatt tatttgacct ttccttttta ttatattcaa 120
ctggaggggg aaaagtttaa tgagagcgca agagtgttta cggagtattt aaagactaag 180
acatctgatg aaattccaag cttactccag tcttattcaa agtccttgac catatctgct 240
caccttaaaa gagatattgt agataagcgg ctccctcttg tgcatgactt ggatattaaa 300
gatggaaagc tatcaaatta tatcgtgatg ttagatatgt ctgttagtac agcagatggt 360
aaacaggtaa ccgtgcaatt tgttcacggg gtggatgtct acaaagaagc aaagaatatt 420
ttgcttttgt atctcccata tacatttttg gttacaattg ctttttcctt tgttttttct 480
tatttttata ctaaacgctt gctcaatcct cttttttaca tttcagaagt gactagtaaa 540
atgcaagatt tggatgacaa tattcgtttt gatgaaagta ggaaagatga agttggtgaa 600
gttggaaaac agattaatgg tatgtatgag cacttgttga aggttattta tgagttggaa 660
agtcgtaatg agcaaattgt aaaattgcaa aatcaaaagg tttcctttgt ccgcggagca 720
tcacatgagt tgaaaacccc tttagccagt cttagaatta tcctagagaa tatgcagcat 780
aatattggag attacaaaga tcatccaaaa tatattgcaa agagtataaa taagattgac 840
cagatgagcc acttattaga agaagtactg gagtcttcta aattccaaga gtggacagag 900
tgtcgtgaga ccttgactgt taagccagtt ttagtagata ttttatcacg ttatcaagaa 960
ttagctcatt caataggtgt tacaattgaa aatcaattga cagatgctac cagggtcgtc 1020
atgagtctta gggcattgga taaggttttg acaaacctga ttagtaatgc aattaaatat 1080
tcagataaaa atgggcgtgt aatcatatcc gagcaagatg gctatctctc tatcaaaaat 1140
acatgtgcgc ctctaagtga ccaagaacta gaacatttat ttgatatatt ctatcattct 1200
caaatcgtga cagataagga tgaaagttcc ggtttgggtc tttacattgt gaataatatt 1260
ttagaaagct atcaaatgga ttatagtttt ctcccttatg aacacggtat ggaatttaag 1320
attagcttgt ag 1332

<210> 214
<211> 1428
<212> DNA
<213> Streptococcus pneumoniae

<400> 214

atgtatttag gagatttgat ggagaaagcc gagtgtggtc aattttcaat actttccttt 60
ctattacaag agtctcagac gaccgtcaag gctgtaatgg aagaaacagg atttcaaaaa 120
gcaaccctaa ccaaatatgt caccctgctc aatgacaagg ctttggatag tggcttagag 180
ctggctattc actcagaaga tgaaaatctg cgtctgtcta tcggtgcagc taccaagggg 240
agagatattc ggagcttgtt tttggagagt gctgttaaat accagatttt ggtttatctt 300
ctctaccacc aacagttttt agcccatcag ctggctcaag aattggtgat tagcgaggct 360
acgcttggtc gtcacttggc tggtttaaat cagattttgt cagaatttga tttatccatc 420
caaaatggcc gttggcgagg tccagagcat cagattcact atttctattt ctgtcttttc 480
cgaaaggtct ggtcgagtca ggaatgggaa ggtcacatgc agaaaccaga gagaaaacag 540
gagattgcca atttagagga aatctgcggt gcaagtttgt ctgcggggca gaaattggac 600
ttggttctct gggctcacat cagtcaacaa cgtcttcggg tcaatgcttg tcagtttcaa 660
gtcatagaag agaaaatgcg agggtatttt gacaatatct tttatcttcg tttgctgaga 720
aaggttccgt ccttttttgc tgggcaacat attccactag gagttgagga tggtgagatg 780
atgatattct tctcttttct cctatctcat cgcattcttc ctcttcatac tatggagtat 840
attcttggtt ttggagggca gttggcagat ttactgacgc aattgattca agaaatgaag 900
aaggaggaac tattggggga ttatacagag gaccatgtca cctatgaact cagtcagctt 960
tgtgctcaag tctatctcta taagggctat attttacagg atcgctacaa gtaccagtta 1020
gagaatcgtc atccatattt actgatggaa catgatttta aagagacagc agaggagatt 1080
tttcatgctc tacctgcttt tcaacagggg acagatttag ataagaagat tctctgggaa 1140
tggctccagt taatcgaata tatggctgaa aacggtggcc agcatatgcg gattggtctg 1200
gatttgacat ctggttttct tgtcttttca aggatggcag ccattttgaa acggtatttg 1260
gaatacaatc gttttattac cattgaagct tatgacccta gtcggcatta tgatttgctg 1320
gttaccaata acccgattca taagaaggaa cagacaccag tctattattt aaaaaatgac 1380
ttggatatgg aggatttggt agcgattcgc cagttattat tcacttaa 1428

<210> 215
<211> 1827
<212> DNA
<213> Streptococcus pneumoniae

<400> 215
atggaatttt caaagaaaac acgtgaattg tcaattaaaa aaatgcagga acgtaccctg 60
gacctcttga ttatcggtgg aggaatcaca ggagctggtg tagccttgca ggcggcagct 120
agcggtcttg agactggttt gattgaaatg caagactttg cagaaggaac atctagtcgt 180
tcaacaaaat tggttcacgg aggacttcgt tacctcaaac aatttgacgt agaagtggtc 240
tcagatacgg tttctgaacg tgcagtggtt caacaaatcg ctccacacat tccaaaatca 300
gatccaatgc tcttaccagt ttacgatgaa gatggagcaa cctttagcct cttccgtctt 360
aaagtagcca tggacttgta cgacctcttg gcaggtgtta gcaacacacc agctgcgaac 420
aaggttttga gcaaggatca agtcttggaa cgccagccaa acttgaagaa ggaaggcttg 480
gtaggaggtg gagtgtatct tgacttccgt aacaacgatg cgcgtctcgt gattgaaaac 540
atcaaacgtg ccaaccaaga cggtgccctc attgccaacc acgtgaaggc agaaggcttc 600
ctctttgacg aaagtggcaa gattacaggt gttgtagctc gtgatctctt gacagaccaa 660
gtgtttgaaa tcaaggcccg tctggttatt aatacaacag gtccttggag tgataaagta 720
cgtaatttgt ctaataaggg aacgcaattc tcacaaatgc gcccaactaa gggagttcac 780
ttggtagtag attcaagcaa aatcaaggtt tcacagccag tttacttcga cacaggtttg 840
ggtgacggtc gtatggtctt tgttctccca cgtgaaaaca agacttactt tggtacaact 900
gatacagact acacaggtga tttggagcat ccaaaagtaa ctcaagaaga tgtagattat 960
ctacttggca ttgtcaacaa ccgcttccca gaatccaaca tcaccattga tgatatcgaa 1020
agcagctggg caggtcttcg tccattgatt gcagggaaca gtgcctctga ctataatggt 1080
ggaaataacg gtaccatcag tgatgaaagc tttgacaact tgattgcgac tgttgaatct 1140
tatctctcca aagaaaaaac acgtgaagat gttgagtctg ctgtcagcaa gcttgaaagt 1200
agcacatctg agaaacattt ggatccatct gcagtttctc gtgggtctag cttggaccgt 1260
gatgacaatg gtctcttgac tcttgctggt ggtaaaatca cagactaccg taagatggct 1320
gaaggagcta tggagcgcgt ggttgacatc ctcaaagcag aatttgaccg tagctttaaa 1380
ttgatcaatt ctaaaactta ccctgtttca ggtggagaat tgaacccagc aaatgtggat 1440
tcagaaatcg aagcctttgc gcaacttgga gtatcacgtg gtttggatag caaggaagct 1500
cactatctgg caaatcttta cggttcaaat gcaccgaaag tctttgcact tgctcacagc 1560
ttggaacaag cgccaggact cagcttggca gatactttgt cccttcacta tgcaatgcgc 1620
aatgagttga ctcttagccc agttgacttc cttcttcgtc gtaccaatca catgctcttt 1680
atgcgtgata gcttggatag tatcgttgag ccaattttgg atgaaatggg acgattctat 1740

gactggacag aagaagaaaa agcaacttac cgtgctgatg tcgaagcagc tctcgctaac 1800
aacgatttag cagaattaaa aaattaa 1827

<210> 216
<211> 705
<212> DNA
<213> Streptococcus pneumoniae

<400> 216
atgatgaatg aattatttgg agaatttcta gggactttaa tcctgattct tctaggaaat 60
ggtgttgttg caggtgtggt tcttcctaaa accaagagca atagctcagg ttggattgtg 120
attactatgg gttgggggat tgcagttgcg gttgcagtct ttgtatctgg caagctcagt 180
ccagcttatt taaacccagc tgtgaccatc ggtgtggcct taaaaggtgg tttgccttgg 240
gcttccgttt tgccttatat cttagcccag ttcgcagggg ccatgctggg tcagattttg 300
gtttggttgc aattcaaacc tcactatgag gcagaagaaa atgcaggcaa tatcctggca 360
accttcagta ctggaccagc catcaaggat actgtatcaa acttgattag cgaaatcctt 420
ggaacttttg ttttggtgtt gacaatcttt gctttgggtc tttacgactt tcaggcaggt 480
atcggaacct ttgcagtggg aactttgatt gtcggtatcg gtctatcact aggtgggaca 540
acaggttatg ccttgaaccc agctcgtgac cttggacctc gtatcatgca cagcatcttg 600
ccaattccaa acaagggaga cggagactgg tcttacgctt ggattcctgt tgtaggccct 660
gttatcggag cagccttggc agtgcttgta ttctcacttt tctag 705

<210> 217
<211> 1008
<212> DNA
<213> Streptococcus pneumoniae

<400> 217
atgaaaagga cctggaggaa ctcattcgtg acaaatctta atacaccttt tatgattggc 60
aatattgaga ttcccaatcg taccgtttta gcgcctatgg ctggcgtgac caactcagcc 120
tttcgtacta tcgcaaagga gctcggagct ggactcgttg taatggaaat ggtctctgac 180
aagggaatcc aatacaacaa cgaaaaaacc ctgcacatgc ttcatatcga tgagggcgaa 240
aaccctgtct ctatccaact ttttggtagc gatgaagaca gcctagcacg cgcagcagaa 300
ttcatccaag aaaacaccaa gaccgatatc gtcgatatca acatgggctg ccctgtcaac 360
aaaatcgtga agaacgaagc tggtgctatg tggctcaagg atccagacaa gatttactcc 420
atcatcaaca aggtccagtc tgtccttgat atcccactta ctgtcaaaat gcgtaccggc 480
tgggcggacc catctcttgc agtagaaaat gctctcgctg ctgaagctgc aggtgtttct 540
gccctcgcca tgcatggccg tacccgtgaa caaatgtata ctggccacgc agaccttgag 600
accctttaca aggttgccca agctctaacc aagattccat tcatcgccaa cggtgatatc 660
cgtactgtcc aagaagccaa gcaacgcatc gaagaagttg gtgctgacgc agtcatgatt 720
ggccgagctg ccatgggaaa tccttacctc ttcaaccaaa tcaaccatta ctttgaaaca 780
ggagaaatcc tacctgattt gacctttgaa gacaagatga agatcgccta cgaacacttg 840
aaacgattga ttaacctcaa aggagaaaac gtcgcagttc gtgaattccg cggtctcgct 900
cctcactatc tccgtggaac atctggcgct gccaaactcc gtggagccat ttcgcaagcc 960
agcaccctgg cagagattga aaccctcttg caattggaga aggcttaa 1008

<210> 218
<211> 411
<212> DNA
<213> Streptococcus pneumoniae

<400> 218
atgacaaaga agaaaattga gcgtatttct gtaatacacc gagaaaagat tttatggctc 60
aagtggtatt tcatgcgaga taaagaacaa cctaagtata gtgtccttga gcgtaaaatg 120
tttgatgctg ctaaaaatca agatatgcta gcttatcaaa aatacgcaac tatcaagcag 180
ataacagata ttagggtaca aacaagtgag gctgacattt tagaggctgt aaaagaggtt 240
tatgtgtaca atcacatgaa tgttatcgga gcttgtcagc ggatattatt tatcagtcaa 300
tcaccagctt atgataagtt aaataagtgg tttaatatct attctgattt gtattttagc 360

gttgtaccct tgcccaaaat gggggtatat catgagatgg taggtatcta g 411

<210> 219
<211> 3300
<212> DNA
<213> Streptococcus pneumoniae

<400> 219
atgaaaaatt ccaacgaggc tgagatgaaa ttactttata ctgatattcg gacttctttg 60
acagaaattc taacaagaga ggcagaagag ctagttgcag ctggcaagcg ggtcttctac 120
attgccccca actctctttc ttttgaaaag gaacgcgccg tgctggaata cttgtcccag 180
caggcttctt tttcgattac cgtcacgcgc tttgctcaaa tggctcgcta tctggtcttg 240
aatgatttac cagctaaaac tactcttgat gatatcggtc ttgggttggc cttttacaaa 300
tgccttgccg aactcgatcc caaggacttg cgtgtttatg gcgctattaa gcaggatcct 360
caattgatcc agcagttaat tgagctttac catgagatga ccaaatctca gatgagtttt 420
ttggacttgg agaatttaac agatgaggat aagagggcgg atttactctt gatttttgag 480
aaagtaacag cctatcttaa tcaaggtcag ttagcccagg aaagtcagtt gtcccatttg 540
attgaggcta ttgagaatga caaggtaagt agtgatttta atcaaatcgc cttggtcatt 600
gacggcttta ctcgtttttc tgctgaggaa gagcgggttg tggacttact tcacggcaaa 660
ggtgttgaga ttgttatcgg ggcttatgct agtaagaaag cctataccag tccttttagc 720
gagggcaatc tctaccaagc cagcgtaaaa tttctccatc atctggcttc taaataccaa 780
acgcctgctc aggactgttc tcaaactcat gagaagatgg atagttttga caaggcctct 840
cgtttgttgg agtcttctta tgacttttca gaactcgctt tggatgtcga tgagaaagac 900
cgtgaaaatt tacaaatctg gtcttgtttg acgcaaaagg aggagttgga gctagtagcc 960
cgtagtattc gtcagaaatt acatgagaac tcagacctga gctacaagca ttttcgtatt 1020
ctcttggggg atgtagcttc ttaccagtta tctctcaaaa ccatttttga ccagtatcag 1080
attccttttt atcttggtag aagcgaagcc atggctcatc atcccttgac tcagtttgtc 1140
gagtctattt tagctttaaa acgttaccgt tttcgtcagg aggatttgat taatcttctt 1200
agaactgatt tgtatactga cctcagtcag tctgatattg atgcttttga gcaatatatc 1260
cgctatcttg gtatcaatgg cttgccagcc tttcagcaaa ccttcaccaa atcccaccat 1320
ggaaaattta atcttgagcg tttgaatgtc ctccgcctga gaattttagc acctcttgaa 1380
accctctttg ccagccgaaa acaaaaggct gaaaaactcc tacaaaaatg gagtgtcttt 1440
ctaaaagaag gagctgtgac caagcagtta caagatttga caaccacttt ggaagctgta 1500
gaacaggaaa gacaagccga agtttggaag gctttctgcc atgttttaga acaatttgcg 1560
actgtttttg ctggttcaca ggttagtctg gaagacttcc tagccttgct ccattctgga 1620
atgagtttgt cccaataccg taccattcca gcaacagtgg acactgttct ggtgcagagt 1680
tacgatttga ttgcaccatt gactgctgac tttgtctatg ctattggact aactcaggac 1740
aatttaccaa aaatttctca aaacaccagt cttctgacag atgaagaaag gcaaaaccta 1800
aaccaagcga ccgaagaagg cgttcaatta ctgattgcca gcagtgaaaa tctcaagaaa 1860
aatcgctaca ctatgctttc cttggtcaat tctgctcgta agcagttgtt cttgtcggct 1920
ccaagccttt ttaacgaaag tgaaagtaag gaatctgcct atcttcaaga gttgatccat 1980
tttggattta ggcggagaga gaagaggatg aatcacaaag gactgtctaa ggaggatatg 2040
gggtcctatc acagtctttt gtctagtctg gttgcctatc accagcaggg tgagatgagc 2100
gatactgagc aagatttgac ttttgtcaag gttctgtcgc gtgtcatagg taaaaaacta 2160
gatcagcaag gtctggaaaa tccagctatc ccaaccagtc caagcagcaa gaccttagcc 2220
aaggacacct tgcaagctct ctatcctgcc aaacaggagt tttacctgtc tacgtcgggt 2280
ttgacagagt tttatcgcaa tgaatacagt tatttcctac gctacgtttt aggcttgcag 2340
gaggaattac gtttgcatcc tgatgcccgt agtcacggga atttcttgca tcgtatcttt 2400
gaacgcgcct tacagttgcc taatgaagat tcctttgacc aacgtctaga acaagctatt 2460
caagaaacca gtcaagaacg cgaatttgaa gctatttatc aagaaagttt ggaagcccag 2520
tttaccaagg aagttttgct tgatgttgca cggacaactg gacatattct ccgacacaat 2580
ccagccatcg aaaccatcaa agaagaagca aattttggtg gaaaagacca agcctttatt 2640
caattagaca atggacgcag tgtctttgta cgaggcaagg tggaccggat tgaccgtttg 2700
aaagctaatg gagcgatagg agtagtagac tacaaatcca gtctgactca gttccagttt 2760
cctcatttct ttaatgggct caattctcag ttaccaacct atcttgctgc cctaaaaaga 2820
gaaggggagc agaacttttt cggcgccatg tacttggaaa tggctgaacc tgtccaatct 2880
ctgatggcgg taaaaagtct ggcaggagca gtggtagaag ccagcaaatc tatgaaatac 2940
caagggctct tcttggaaaa agaaagcagt tatttaggcg aattttataa caaaaacaag 3000
gctaatcaac tgacagatga ggaatttcag ctcctactgg actacaatgc ctatctttac 3060
aagaaagctg ctgagaagat tttagcaggc cggttcgcca tcaatcctta tactgaaaat 3120

ggcagaagca ttgccccata cgtccagcaa catcaggcta ttacaggctt tgaagccaat 3180
taccatctgg gccaagcccg tttcctagaa aagttggacc tagctgatgg caagcgtctg 3240
gtcggagaaa aactcaagca agcttggctt gaaaaaataa gagaggagtt gaatcgatga 3300

<210> 220
<211> 3651
<212> DNA
<213> Streptococcus pneumoniae

<400> 220
atgaagctta ttcccttttt aagtgaggag gagattcaaa aactgcaaga agcagaagca 60
aattcgagca aggaacagaa gaaaactgcc gagcaaatcg aagctatcta cacttctgcc 120
cagaatatcc tggtctcagc atcggctggt tctggaaaga cctttgtcat ggcagagcgc 180
attctggacc aattggcgcg tggtgtcgaa atttctcaac tctttatctc aacctttacc 240
gtcaaggctg caactgaact taaagaacgt ttagagaaaa aaatcagcaa gaaaatccaa 300
gaaacagatg atgtcgacct caaacaacac ttgggtcgcc agttggcaga cctacccaac 360
gctgccattg gaaccatgga ttctttcaca caaaaattcc ttggcaaaca tggttatctg 420
cttgatattg cacctaattt ccgtatttta caaaaccaaa gcgagcaact tattctcgaa 480
aacgaagtct ttcatgaggt ctttgaagcg cattaccaag gtaaacagaa agagaccttt 540
agtcatttgc tgaaaaactt tgctgggcgt ggcaaggacg aacggggtct gcgccagcag 600
gtctataaaa tctatgactt cctccaatcc accagtaatc ctcaaaagtg gctgagtgaa 660
tctttcctca aaggatttga gaaagctgat tttaccagtg aaaaagaaaa actgaccgag 720
caaatcaaac aagccctttg ggatttggaa agcttttccc gttaccatct ggataacgat 780
gccaaggagt ttgcaaaggc tgcctattta gaaaatgttc agttaattct ggatgaaatt 840
ggctccctaa atcaggagtc cgatagtcag gcttatcagg cagtgcttgc gcgtgttgtc 900
gccatctcta aggagaaaaa cggtcgagct ctgactaatg ccagccgtaa ggctgatttg 960
aagcccctgg ctgatgccta caacgaagag agaaagaccc agtttgctaa actaggacaa 1020
ttatcagacc agatagcgat tctcgactat caagaacgtt atcatggaga cacttggaaa 1080
ctagctaaaa ccttccaatc tttcatgagc gattttgtag aggcttatcg tcagagaaaa 1140
cgacaggaaa atgccttcga attcgctgat atcagccatt acaccattga gattttagag 1200
aatttcccac aagttcgtga gtcttatcag gagcgcttcc atgaagtcat ggtcgatgag 1260
tatcaggata ccaaccatat tcaagaacgg atgctggaat tgttgtctaa tggccacaat 1320
cgctttatgg tgggagatat caagcaatcc atctatcgtt tccgtcaggc agacccgcag 1380
attttcaatg agaaattcca acgctatgcg caaaatcccc aagaaggcag gctcattatc 1440
ctcaaggaaa atttccgtag tagttcagaa gtgctgtcag caaccaatga tgtctttgaa 1500
cgtctcatgg accaagaggt cggcgaaatc aactatgata acaagcacca gcttgttttt 1560
gccaatacca aactgactcc caatccagac aacaaggcag catttctcct ctacgacaag 1620
gacgatacag gtgaggaaga agagagtcaa acagaaacga aactaacagg cgaaatgcgc 1680
ttagttatca aggagattct gaaacttcat caagaaaaag gtgttgcctt taaggaaatt 1740
gcccttctga cctccagccg cagtcgtaat gaccagattc tcctcgccct gtctgagtac 1800
ggaattcctg tcaaaactga cggagagcaa aacaattatc tccaatccct agaagtgcaa 1860
gtcatgctag acactcttcg tgtcattcac aatcccctgc aagactacgc cttggttgcc 1920
cttatgaagt ctccaatgtt tggttttgat gaggatgagc tagcacgttt gtcccttcag 1980
aaagcagagg ataaagtcca cgaaaatctc tatgagaaac tggtcaatgc acaaaaaatg 2040
gcaagtagtc aaaaaggctt gattcacaca gctctagctg aaaaactaaa gcaattcatg 2100
gatatcctag cttcttggcg cttgtatgcc aaaacccact ctctctatga cttgatttgg 2160
aagatttaca acgaccgttt ttattatgac tatgttgggg ctttgccgaa tggtcctgct 2220
aggcaggcca atctctatgc cctagcactg cgtgctgatc aatttgaaaa gagcaatttc 2280
aaaggtttgt cgcgttttat tcgtatgatt gaccaagtct tagaagccca gcacgatttg 2340
gcaagcgtgg ccgtcgcacc gccaaaagat gcagtagagc tcatgaccat ccacaagagt 2400
aaagggctgg agtttcctta cgtctttatc ctcaatatgg atcaagattt caacaagcaa 2460
gactctatgt cagaagtcat tctcagtcgt cagaatggtc ttggtgtcaa atatattgcc 2520
aagatggaga caggggcagt agaagaccac tatcctaaaa ccatcaaact ctccattcct 2580
agtctgacct ataggcagaa cgaagaggaa ttacagctag caagctattc tgagcagatg 2640
cgtttgctgt atgttgctat gacgcgggct gagaaaaagc tctatcttgt cggcaagggt 2700
tctcgtgaaa agctggaatc caaggaatac ccagcagcca aaaatggaaa actaaatagc 2760
aatactagac tgcaagcacg gaatttccaa gattggcttt gggctatcag taaagtgttt 2820
actaaggaca agctcaactt tagttatcgt tttattggcg aagatcagtt gaccagagaa 2880
gctatcggag agttggaaac caagagtcct ctccaagata gctcccaagc agacaatcgt 2940
cagtcagata ccatcaaaga agctctggaa atgctgaagg aggtggaagt ttataatact 3000

cttcaccgcg cagctattga acttcctagt gttcaaaccc caagtcaaat caagaaattc 3060
tacgaaccag ttatggatat ggaaggtgtc gagattgctg gtcaaggtca gtcagtaggc 3120
aagaaaatca gcttcgattt gccagatttt tcaaccaaag aaaaggtaac tggagctgag 3180
attggtagtg ctactcacga actcatgcag agaattgacc tcagccagca actaaccctt 3240
gctagcctaa cagaaacact caaacaagtt caaactagcc aagctgtcag agacaagatc 3300
aatcttgata aaattcttgc tttctttgac acagtactcg gtcaggaaat tcttgctaat 3360
accgaccatc tttatcgcga gcaacctttc tccatgctca aacgagacca aaagagtcag 3420
gaagactttg ttgtccgtgg tatccttgat ggctatctgc tttacgaaaa caaaattgtt 3480
ctgttcgact acaagacaga ccgctatgat gaaccaagtc aactcgtaga ccgctatcgt 3540
ggtcagttag ctctatacga agaggcttta tcacgagcct attcgattga aaatattgaa 3600
aaatacttga ttttactcgg taaagacgag gttcaagttg taaaagtata a 3651

<210> 221
<211> 648
<212> DNA
<213> Streptococcus pneumoniae

<400> 221
atggaacttg ctcgccatgc tgaaagcttg ggagtagatg ctattgcaac gattccacca 60
atttatttcc gcttgccaga atactcagtt gccaaatact ggaacgatat cagttctgca 120
gctccaaaca cagactacgt gatttacaac attcctcaat tggcaggggt tgctttgact 180
ccaagccttt acacagaaat gttgaaaaat cctcgtgtta tcggtgtgaa gaactcttct 240
atgccagttc aagatatcca aacctttgtc agccttggtg gagaagacca tatcgtcttt 300
aatggtcctg atgagcagtt cctaggagga cgcctcatgg gggctagggc tggtatcggt 360
ggtacttatg gtgctatgcc agaactcttc ttgaaactca atcagttgat tgcggataag 420
gacctagaaa cagcgcgtga attgcagtat gctatcaacg caatcattgg taaactcact 480
tctgctcatg gaaatatgta cggtgtcatc aaagaagtct tgaaaatcaa tgaaggcttg 540
aatattggat ctgttcgttc accattgaca ccagtgactg aagaagatcg tccagttgta 600
gaagcggctg ctgccttgat tcgtgaaacc aaggagcgct tcctctaa 648

<210> 222
<211> 1224
<212> DNA
<213> Streptococcus pneumoniae

<400> 222
atgtataaga caaagtgttt acgagagaag ttagtattat ttttaaaaat tttcttccca 60
atcctgatct accaatttgc caattattct gcctcttttg ttgatactgc aatgacaggt 120
caatacaaca ctatggactt ggctggtgta tctatggcaa ccagtatctg gaatcctttc 180
tttacatttc taacagggat tgtgtcagcc ttggtgccta tcattggtca ccatcttggt 240
cgaggcaaaa aggaagaagt tgcgtctgat ttttaccaat ttatttattt ggccttgggc 300
ctatctgtgg tcttgctggg gatggtactt ttcttggcac caataatctt gaatcatatt 360
gggttagaag cagcagtagc ggcagtagcg gttcgctatc tttggttttt atctatcggg 420
attatcccct tgttgctctt tagcgtcatt cgttccttgc tggattcgct gggcttgacc 480
aaactgtcca tgtacctcat gcttttgtta ctccctctca atagcggatt taactatctc 540
ttgatttacg gtgcctttgg tgttccagaa ctgggagggg ctggtgctgg tttaggaaca 600
tccttggcct actgggtctt gcttgggatt tctgttctgg ttttatttaa acaggagaag 660
ctcaaagcct tacaccttga gaaacgaatt ccacttaata tggataaaat taaggaagga 720
gttcgtttag gtctgcctat tgggggaact gtcttcgcgg aagtggctat cttttcagtg 780
gttggcttga ttatggctaa gttttcgccc ttgattatag ctagtcacca gtcagctatg 840
aacttttcaa gtcttatgta cgcctttcct atgagtatct catcggctat ggctattgtc 900
gtttcctatg aagtgggagc caagcgattt gatgatgcga aaacctatat tggtctagga 960
agatggactg ccctcatttt tgcggccttc accttaacct tcctttacat ttttagggga 1020
aatgtggcca gtctttatgg taacgaccca aaatttatcg atttgacagt gcgtttttta 1080
acttatagtc ttttcttcca gttagcagat acctttgcgg cgccgcttca gggaattttg 1140
cgggggtata aggatacagt tattcctttt taccttggtt tgcttggtta ttggggcgta 1200
gcaatccctg tgtacgctat ttga 1224

<210> 223
<211> 570
<212> DNA
<213> Streptococcus pneumoniae

<400> 223
atgagtactt tagcaaaaat agaagcgctc ttgtttgtag cgggtgaaga tgggattcgg 60
gtccgccagt tagctgaact cctctctctg ccaccgacag gcatccagca aagtttagga 120
aaattagccc agaagtatga aaaggaccca gattccagtt tggctttgat tgagacaagt 180
ggtgcttata gattggtgac caagcctcaa tttgcagaga ttttgaagga atactctaag 240
gcgcctatca accagagctt gtctcgggct gcccttgaga ccttgtccat tattgcctac 300
aaacagccga ttacgcggat agaaattgat gccatccgtg gagttaactc gagtggagcc 360
ttggcaaagt tgcaggcttt tgacctgata aaggaagacg ggaaaaagga agtattgggg 420
cgccccaacc tctatgtgac tacggattat ttcctagatt acatggggat aaaccattta 480
gaagaattac cagtgattga tgagcttgag attcaagccc aagaaagcca attatttggt 540
gaaaggatag aagaagatga gaatcaataa 570

<210> 224
<211> 936
<212> DNA
<213> Streptococcus pneumoniae

<400> 224
atggatacga tgattagtag attttttcgc catttatttg aagccttaaa aagtttgaaa 60
cgaaatggtt ggatgacagt agctgctgtc agttcagtca tgattacttt gaccttggtg 120
gcaatatttg catctgttat tttcaataca gcgaaactag ctacagatat tgaaaataat 180
gtccgtgtag tagtttatat ccgaaaggat gtggaagata atagtcagac aattgaaaaa 240
gaaggtcaaa ctgttacaaa taatgactac cacaaggtat atgattcttt gaagaacatg 300
tctacggtta aaagtgttac cttttcaagt aaagaagaac aatatgaaaa attaaccgag 360
ataatgggag ataactggaa aatctttgaa ggagatgcca atcctctcta tgatgcctat 420
attgtagagg caaacactcc aaatgatgta aaaactatag ccgaagatgc taaaaaaatt 480
gaaggtgtct ctgaggttca agatggcggt gccaatacag aaagactctt caagttagct 540
tcatttatcc gtgtttgggg actagggatt gctgctttgt taatttttat cgcagttttc 600
ttgatttcaa ataccattcg tattaccatt atttcccgca gtcgcgaaat tcaaatcatg 660
cgcttggtcg gagctaaaaa cagttatatc cgtggaccgt tcttgttaga aggagccttt 720
atcggtttat tgggagctat cgcaccatct gttttggtct ttattgttta tcaaattgtt 780
taccaatctg tcaacaaatc gttggtaggg caaaatctat ccatgattag tccagattta 840
tttagtccgt tgatgattgc cctactattt gtgattgggg ttttcattgg ttcattggga 900
tcaggaatat ccatgcgccg attcttgaag atttag 936

<210> 225
<211> 1977
<212> DNA
<213> Streptococcus pneumoniae

<400> 225
atgaagaaag taagatttat ttttttagct ctgctatttt tcttagctag tccagagggt 60
gcaatggcta gtgatggtac ttggcaagga aaacagtatc tgaaagaaga tggcagtcaa 120
gcagcaaatg agtgggtttt tgatactcat tatcaatctt ggttctatat aaaagcagat 180
gctaactatg ctgaaaatga atggctaaag caaggtgacg actattttta cctcaaatct 240
ggtggctata tggccaaatc agaatgggta gaagacaagg gagcctttta ttatcttgac 300
caagatggaa agatgaaaag aaatgcttgg gtaggaactt cctatgttgg tgcaacaggt 360
gccaaagtaa tagaagactg ggtctatgat tctcaatacg atgcttggtt ttatatcaaa 420
gcagatggac agcacgcaga gaaagaatgg ctccaaatta aagggaagga ctattatttc 480
aaatccggtg gttatctact gacaagtcag tggattaatc aagcttatgt gaatgctagt 540
ggtgccaaag tacagcaagg ttggcttttt gacaaacaat accaatcttg gttttacatc 600
aaagaaaatg gaaactatgc tgataaagaa tggattttcg agaatggtca ctattattat 660
ctaaaatccg gtggctacat ggcagccaat gaatggattt gggataagga atcttggttt 720
tatctcaaat ttgatgggaa aatggctgaa aaagaatggg tctacgattc tcatagtcaa 780

gcttggtact acttcaaatc cggtggttac atgacagcca atgaatggat ttgggataag 840
gaatcttggt tttatctcaa atctgatggg aaaatagctg aaaaagaatg ggtctacgat 900
tctcatagtc aagcttggta ctacttcaaa tccggtggtt acatgacagc caatgaatgg 960
atttgggata aggaatcttg gttttacctc aaatctgatg ggaaaatagc tgaaaaagaa 1020
tgggtctacg attctcatag tcaagcttgg tactacttca aatctggtgg ctacatggcg 1080
aaaaatgaga cagtagatgg ttatcagctt ggaagcgatg gtaaatggct tggaggaaaa 1140
actacaaatg aaaatgctgc ttactatcaa gtagtgcctg ttacagccaa tgtttatgat 1200
tcagatggtg aaaagctttc ctatatatcg caaggtagtg tcgtatggct agataaggat 1260
agaaaaagtg atgacaagcg cttggctatt actatttctg gtttgtcagg ctatatgaaa 1320
acagaagatt tacaagcgct agatgctagt aaggacttta tcccttatta tgagagtgat 1380
ggccaccgtt tttatcacta tgtggctcag aatgctagta tcccagtagc ttctcatctt 1440
tctgatatgg aagtaggcaa gaaatattat tcggcagatg gcctgcattt tgatggtttt 1500
aagcttgaga atcccttcct tttcaaagat ttaacagagg ctacaaacta cagtgctgaa 1560
gaattggata aggtatttag tttgctaaac attaacaata gccttttgga gaacaagggc 1620
gctactttta aggaagccga agaacattac catatcaatg ctctttatct ccttgcccat 1680
agtgccctag aaagtaactg gggaagaagt aaaattgcca aagataagaa taatttcttt 1740
ggcattacag cctatgatac gaccccttac ctttctgcta agacatttga tgatgtggat 1800
aagggaattt taggtgcaac caagtggatt aaggaaaatt atatcgatag gggaagaact 1860
ttccttggaa acaaggcttc tggtatgaat gtggaatatg cttcagaccc ttattggggc 1920
gaaaaaattg ctagtgtgat gatgaaaatc aatgagaagc taggtggcaa agattag 1977

<210> 226
<211> 867
<212> DNA
<213> Streptococcus pneumoniae

<400> 226
atgaaaaaag tattacaaaa atattgggca tgggcttttg tggtcatccc cctcttgtta 60
caagcaattt tcttctatgt gccgatgttt caaggagcct tttacagttt taccaactgg 120
acaggattga cttataacta caaatttgtt ggcttaaaca actttaagct cctcttcatg 180
gatccaaaat tcatgaatgc gattggcttt accgcaatca ttgcgattgc catggtggtt 240
ggtgagattg cactcgggat cttcattgcg cgtgtcttga attctaaaat caaaggccaa 300
accttcttcc gtgcttggtt cttcttccca gctgttttat ctggtttgac agtggctttg 360
atcttcaagc aagtcttcaa ctacggtctt ccagcgattg gaaatgccct tcatattgaa 420
tttttccaaa ccagtctttt agggactaag tggggagcaa tctttgcggc tgtctttgtc 480
cttctttggc aaggggtggc tatgcccatc atcatcttcc tagctggttt gcaatctatt 540
ccaactgaga ttacagaggc agcaaggatt gatggtgcga ctagcaagca agttttctgg 600
aacattgaat tgccttactt gctaccaagt gtctctatgg tctttatcct agccctaaaa 660
ggtgggctga ctgcctttga ccaagtcttt gccatgaccg gtggtggtcc aaacaatgcc 720
acaacctcac ttgggctctt ggtttataac tatgccttta aaaacaacca attcggttat 780
gccaatgcca ttgccgtaat cttgttcttc ttaattgtag tgatttcgat catccaattg 840
agagtatcta agaaatttga aatttaa 867

<210> 227
<211> 837
<212> DNA
<213> Streptococcus pneumoniae

<400> 227
atgatgaaac aagatgaaag aaaagccctg attggcaaat acattctatt gattctagga 60
tcggttctga ttttagtgcc gctccttgct accctcttta gttccttcaa acccactaag 120
gatattgtag ataatttctt tggctttcca accaacttca catgggacaa ctttagccgt 180
ctcttagctg atgggattgg aggctattat tggaactctg tcgtcatcac tgtcttgtct 240
ttacttgcag taatgatctt tatccctatg gcagcctact ccatcgctcg caatatgagt 300
aaaagaaaag cctttaccat catgtatacc ctcttaatcc tcggaatctt cgtacctttc 360
caagtcatca tgattccgat tacggttatg atgagtaaac tcggtttggc taataccttt 420
ggtttgatct tgctctactt gacctatgcg attccacaga ccctctttct ctatgttggc 480
tatatcaaaa tctcgattcc agaaagtctg gatgaagcag cagagatcga tggggctaat 540
caatttacaa cctatttccg catcatcttc ccaatgatga aaccgatgca tgcgacaacc 600

atgatcatca atgccctttg gttctggaat gacttcatgt tgccactcct tgtcttgaac 660
cgggattcca aaatgtggac tctgcctttg ttccaataca actacgcagg ccaatatttc 720
aacgactacg gaccaagctt tgcctcttac gtggtcggca ttatcagtat caccattgtc 780
tatctcttct tccaacgcca tatcatttca ggaatgagca acggggcagt gaagtaa 837

<210> 228
<211> 1044
<212> DNA
<213> Streptococcus pneumoniae

<400> 228
atgaaaagta ttcttcagaa aatgggggag catccgatgc tgcttctttt tcttagctat 60
agtactgtta tatccattct tgcacaaaat tggatgggtc ttgtggcttc agtaggaatg 120
tttctattta ctattttctt tttgcactat cagtcgattt tatcccataa attctttcga 180
ttgattttgc agtttgtctt gtttggtagt gtcttgtcag ctgcttttgc cagtttagaa 240
catttccaaa ttgtgaagaa atttaactat gcttttcttt cacccaatat gcaggtgtgg 300
catcagaacc gggcagaagt gaccttcttt aatcctaatt attatggaat tatttgttgt 360
ttctgtatta tgattgcttt ctatctgttt acaacgacca agttgaattg gttgaaagta 420
ttctgtgtga ttgcaggctt tgttaatctc tttggtttga actttactca aaatcgaact 480
gcctttcctg ctattatcgc tggagcaatt atctatctct ttacgactat taaaaactgg 540
aaggcctttt ggcttagtat tggggtcttc gcgattggtt tgagtttcct cttttctagt 600
gatttgggag ttcgaatggg tactttagac tcttctatgg aagaacgcat ttctatctgg 660
gatgctggga tggccttgtt taagcaaaat cctttttggg gtgaaggccc attgacctat 720
atgaactctt atcctcggat acatgctcct tatcatgaac atgcccacag tctttatatt 780
gatacgattc tgagttacgg aattgtgggg actattttat tagttttgtc ttctgttgct 840
cctgttcgct tgatgatgga tatgagtcag gagtcgggga aacgtccgat tatcggcctt 900
tatctatctt tccttacagt ggttgctgtg cacggaattt ttgacttggc tctcttctgg 960
attcagtcag gctttatttt cttgctagtt atgtgcagta ttccattgga gcatcgaatg 1020
ttggtatcgg acatgacgga ttaa 1044

<210> 229
<211> 1299
<212> DNA
<213> Streptococcus pneumoniae

<400> 229
atgtcaaaga tggatgttca gaaaatcatt gcaccgatga tgaagtttgt gaatatgcgt 60
ggcattatag ctctaaaaga tgggatgtta gcaattttgc cattgacagt agttggtagt 120
ttgttcttga ttatgggaca attgccgttc gaaggattaa ataagagcat tgctagtgtt 180
tttggagcta attggacaga gccgtttatg caagtatatt caggaacttt tgctattatg 240
ggtctaattt cttgtttttc aattgcctat tcttatgcta agaatagcgg agtagaggct 300
ttaccagctg gagttctatc tgtatctgca ttctttattt tgctaagatc atcttatatc 360
cctaaacaag gtgaggcgat tggggacgct attagtaaag tttggtttgg aggccaagga 420
attatcggtg ctatcattat aggtttggta gtaggaagta tttatacctt ctttataaag 480
agaaaaattg ttattaagat gccagaacaa gttccacaag ctattgccaa acagtttgaa 540
gcaatgattc cagcatttgt aattttctta tcttctatga ttgtatatat tttagcgaag 600
tcattgacta atggcggaac attcatagaa atgatttatt ctgctattca agttccgttg 660
caaggtttaa ctggatcttt gtatggtgct attggaattg cattctttat atcatttttg 720
tggtggtttg gtgttcatgg gcaatcggta gtaaatggag tagtgacagc tctgctttta 780
tctaatcttg atgctaataa agctatgtta gcctctgcta atctatcatt agaaaatggt 840
gcacatattg ttactcaaca atttttagat tcatttttaa ttctatcagg ttcagggatt 900
acgtttggtc ttgtagttgc catgcttttt gcagcaaaat caaaacaata ccaagcctta 960
ggaaaagttg cagcttttcc agcaatattt aacgtaaatg agccagttgt atttggattt 1020
ccgattgtca tgaatccagt tatgtttgta cctttcattc ttgttcctgt acttgcagct 1080
gtgatagtat atggagctat tgcaacaggt ttcatgcagc cattctcagg ggtaacattg 1140
ccttggagta caccagctat tttatcagga tttttggtgg gtggatggca aggagttatt 1200
actcagctgg tgatattagc gatgtctaca ttggtttatt ttccattctt taaagtacag 1260
gatcgtttag cttaccaaaa tgaaatcaaa caatcttag 1299

<210> 230
<211> 303
<212> DNA
<213> Streptococcus pneumoniae

<400> 230
atgaagaaaa aggacttagt agaccaacta gtctcagaga tcgagacggg gaaagtcagg 60
acactgggaa tatacggtca tggagcttca ggtaaatcaa cctttgcaca ggaattgtac 120
caagctttag attctactac agtaaatttg ctagagacag atccttatat cacctcagga 180
cgccatctgg tagtacccaa ggacgcgccg aatcaaaagg tgacagccag tctgccagtg 240
gcgcatgaac tggagagttt gcagagagat atccttgctt gcaggcgggt atggatgtct 300
tga 303

<210> 231
<211> 717
<212> DNA
<213> Streptococcus pneumoniae

<400> 231
atgaagaaaa gatacctagt cttgacagct ttgctagcct tgagtctagc agcttgttca 60
caagaaaaaa caaaaaatga agatggagaa actaagacag aacagacagc caaagctgat 120
ggaacagtcg gtagtaagtc tcaaggagct gcccagaaga aagcagaagt ggtcaataaa 180
ggtgattact acagcattca agggaaatac gatgaaatca tcgtagccaa caaacactat 240
ccattgtcta aagactataa tccaggggaa aatccaacag ccaaggcaga gttggtcaaa 300
ctcatcaaag cgatgcaaga ggcaggtttc cctattagtg atcattacag tggttttaga 360
agttatgaaa ctcagaccaa gctctatcaa gattatgtca accaagatgg aaaggcagca 420
gctgaccgtt actctgcccg tcctggctat agcgaacacc agacaggctt ggcctttgat 480
gtgattggga ctgatggtga tttggtgaca gaagaaaaag cagcccaatg gctcttggat 540
catgcagctg attatggctt tgttgtccgt tatctcaaag gcaaggaaaa ggaaacaggc 600
tatatggctg aagaatggca cctgcgttat gtaggaaaag aagctaaaga aattgctgca 660
agtggtctca gtttggaaga atactatggc tttgaaggcg gagactacgt cgattaa 717

<210> 232
<211> 1269
<212> DNA
<213> Streptococcus pneumoniae

<400> 232
atgcgtaaat tcttaattat tttgttgcta ccaagttttt tgaccatttc aaaagtcgtt 60
agcacagaaa aagaagtcgt ctatacttcg aaagaaattt attacctttc acaatctgac 120
tttggtattt attttagaga aaaattaagt tctcccatgg tttatggaga ggttcctgtt 180
tatgcgaatg aagatttagt agtggaatct gggaaattga ctcccaaaac aagttttcaa 240
ataaccgagt ggcgcttaaa taaacaagga attccagtat ttaagctatc aaatcatcaa 300
tttatagctg cggacaaacg atttttatat gatcaatcag aggtaactcc aacaataaaa 360
aaagtatggt tagaatctga ctttaaactg tacaatagtc cttatgattt aaaagaagtg 420
aaatcatcct tatcagctta ttcgcaagta tcaatcgaca agaccatgtt tgtagaagga 480
agagaatttc tacatattga tcaggctgga tgggtagcta aagaatcaac ttctgaagaa 540
gataatcgga tgagtaaagt tcaagaaatg ttatctgaaa aatatcagaa agattctttc 600
tctatttatg ttaagcaact gactactgga aaagaagctg gtatcaatca agatgaaaag 660
atgtatgcag ccagcgtttt gaaactctct tatctctatt atacgcaaga aaaaataaat 720
gagggtcttt atcagttaga tacgactgta aaatacgtat ctgcagtcaa tgattttcca 780
ggttcttata aaccagaggg aagtggtagt cttcctaaaa aagaagataa taaagaatat 840
tctttaaagg atttaattac gaaagtatca aaagaatctg ataatgtagc tcataatcta 900
ttgggatatt acatttcaaa ccaatctgat gccacattca aatccaagat gtctgccatt 960
atgggagatg attgggatcc aaaagaaaaa ttgatttctt ctaagatggc cgggaagttt 1020
atggaagcta tttataatca aaatggattt gtgctagagt ctttgactaa aacagatttt 1080
gatagtcagc gaattgccaa aggtgtttct gttaaagtag ctcataaaat tggagatgcg 1140
gatgaattta agcatgatac gggtgttgtc tatgcagatt ctccatttat tctttctatt 1200

ttcactaaga attctgatta tgatacgatt tctaagatag ccaaggatgt ttatgaggtt 1260
ctaaaatga 1269

<210> 233
<211> 1959
<212> DNA
<213> Streptococcus pneumoniae

<400> 233
atgaaaaaac aaaataatgg tttaattaaa aatccttttc tatggttatt atttatcttt 60
ttccttgtga caggattcca gtatttctat tctgggaata actcaggagg aagtcagcaa 120
atcaactata ctgagttggt acaagaaatt accgatggta atgtaaaaga attaacttac 180
caaccaaatg gtagtgttat cgaagtttct ggtgtctata aaaatcctaa aacaagtaaa 240
gaagaaacag gtattcagtt tttcacgcca tctgttacta aggtagagaa atttaccagc 300
actattcttc ctgcagatac taccgtatca gaattgcaaa aacttgctac tgaccataaa 360
gcagaagtaa ctgttaagca tgaaagttca agtggtatat ggattaatct actcgtatcc 420
attgtgccat ttggaattct attcttcttc ctattctcta tgatgggaaa tatgggagga 480
ggcaatggcc gtaatccaat gagttttgga cgtagtaagg ctaaagcagc aaataaagaa 540
gatattaaag taagattttc agatgttgct ggagctgagg aagaaaaaca agaactagtt 600
gaagttgttg agttcttaaa agatccaaaa cgattcacaa aacttggagc ccgtattcca 660
gcaggtgttc ttttggaggg acctccgggg acaggtaaaa ctttgcttgc taaggcagtc 720
gctggagaag caggtgttcc attctttagt atctcaggtt ctgactttgt agaaatgttt 780
gtcggagttg gagctagtcg tgttcgctct ctttttgagg atgccaaaaa agcagcacca 840
gctatcatct ttatcgatga aattgatgct gttggacgtc aacgtggagt cggtctcggc 900
ggaggtaatg acgaacgtga acaaaccttg aaccaacttt tgattgagat ggatggtttt 960
gagggaaatg aagggattat cgtcatcgct gcgacaaacc gttcagatgt acttgaccct 1020
gcccttttgc gtccaggacg ttttgataga aaagtattgg ttggtcgtcc tgatgttaaa 1080
ggtcgtgaag caatcttgaa agttcacgct aagaataagc ctttagcaga agatgttgat 1140
ttgaaattag tggctcaaca aactccaggc tttgttggtg ctgatttaga gaatgtcttg 1200
aatgaagcag ctttagttgc tgctcgtcgc aataaatcga taattgatgc ttcagatatt 1260
gatgaagcag aagatagagt tattgctgga ccttctaaga aagataagac agtttcacaa 1320
aaagaacgag aattggttgc ttaccatgag gcaggacata ccattgttgg tctagtcttg 1380
tcgaatgctc gcgttgtcca taaggttaca attgtaccac gcggccgtgc aggcggatac 1440
atgattgcac ttcctaaaga ggatcaaatg cttctatcta aagaagatat gaaagagcaa 1500
ttggctggct taatgggtgg acgtgtagct gaagaaatta tctttaatgt ccaaaccaca 1560
ggagcttcaa acgactttga acaagcgaca caaatggcac gtgcaatggt tacagagtac 1620
ggtatgagtg aaaaacttgg cccagtacaa tatgaaggaa accatgctat gcttggtgca 1680
cagagtcctc aaaaatcaat ttcagaacaa acagcttatg aaattgatga agaggttcgt 1740
tcattattaa atgaggcacg aaataaagct gctgaaatta ttcagtcaaa tcgtgaaact 1800
cacaagttaa ttgcagaagc attattgaaa tacgaaacat tggatagtac acaaattaaa 1860
gctctttacg aaacaggaaa gatgcctgaa gcagtagaag aggaatctca tgcactatcc 1920
tatgatgaag taaagtcaaa aatgaatgac gaaaaataa 1959

<210> 234
<211> 1278
<212> DNA
<213> Streptococcus pneumoniae

<400> 234
atgagggaac cagatttttt aaatcatttt ctcaagaagg gatatttcaa aaagcatgct 60
aaggcggttc tagctctttc tggtggatta gattccatgt ttctatttaa ggtattgtct 120
acttatcaaa aagagttaga gattgaattg attctagctc atgtgaatca taagcagaga 180
attgaatcag attgggaaga aaaggaatta aggaagttgg ctgctgaagc agagcttcct 240
atttatatca gcaatttttc aggagaattt tcagaagcgc gtgcacgaaa ttttcgttat 300
gatttttttc aagaggtcat gaaaaagaca ggtgcgacag ctttagtcac tgcccaccat 360
gctgatgatc aggtggaaac gatttttatg cgcttgattc gaggaactcg cttgcgctat 420
ctatcaggaa ttaaggagaa gcaagtagtc ggagagatag aaatcattcg tcccttcttg 480
cattttcaga aaaaagactt tccatcaatt tttcactttg aagatacatc aaatcaggag 540
aatcattatt ttcgaaatcg tattcgaaat tcttacttac cagaattgga aaaagaaaat 600

cctcgattta gggatgcaat cttaggcatt ggcaatgaaa ttttagatta tgatttggca 660
atagctgaat tatctaacaa tattaatgtg gaagatttac agcagttatt ttcttactct 720
gagtctacac aaagagtttt acttcaaact tatctgaatc gttttccaga tttgaatctt 780
acaaaagctc agtttgctga agttcagcag attttaaaat ctaaagccca gtatcgtcat 840
ccgattaaaa atggctatga attgataaaa gagtaccaac agtttcagat ttgtaaaatc 900
agtccgcagg ctgatgaaaa ggaagatgaa cttgtgttac actatcaaaa tcaggtagct 960
tatcaaggat atttattttc ttttggactt ccattagaag gtgaattaat tcaacaaata 1020
cctgtttcac gtgaaacatc catacacatt cgtcatcgaa aaacaggaga tgttttgatt 1080
aaaaatgggc atagaaaaaa actcagacgt ttatttattg atttgaaaat ccctatggaa 1140
aagagaaact ctgctcttat tattgagcaa tttggtgaaa ttgtctcaat tttgggaatt 1200
gcgaccaata atttgagtaa aaaaacgaaa aatgatataa tgaacactgt actttatata 1260
gaaaaaatag ataggtaa 1278

<210> 235
<211> 1692
<212> DNA
<213> Streptococcus pneumoniae

<400> 235
atgaagcgtt cttctctttt agttagaatg gttatttcca tctttctggt ctttctcatt 60
ctcctagctc tggttggaac tttctactat caatcaagtt cttcagccat tgaggccacc 120
attgagggca acagccaaac gaccatcagc cagactagcc actttattca gtcttatatc 180
aaaaaactag aaaccacctc gactggtttg acccagcaga cggatgttct ggcctatgct 240
gagaatccca gtcaagacaa ggtcgaggga atccgagatt tgtttttgac catcttgaag 300
tcagataagg acttgaaaac tgttgtgctg gtgaccaaat ctggtcaggt catttctaca 360
gatgacagtg tgcagatgaa aacttcctct gatatgatgg ctgaggattg gtaccaaaag 420
gccattcatc agggagctat gcctgttttg actccagctc gtaaatcaga tagtcagtgg 480
gtcatttctg tcactcaaga acttgttgat gcaaagggag ccaatcttgg tgtgcttcgt 540
ttggatattt cttatgaaac tctggaagcc tatctcaatc aactccagtt ggggcagcag 600
ggctttgcct tcattatcaa tgaaaaccat gaatttgtct accatcctca acacacagtt 660
tatagttcgt ctagcaaaat ggaggctatg aaaccctaca tcgatacagg tcagggttat 720
actcctggtc acaaatccta cgtcagtcaa gagaagattg caggaactga ttggacggtg 780
cttggcgtgt catcattgga aaagttagac caggttcgga gtcagctctt gtggaccttg 840
cttggggcca gtgtcacatc tcttcttgtc tgtctctgct tagtgtggtt cagtcttaaa 900
cgctggattg ctcctttgaa ggatttgaga gaaaccatgt tggaaattgc ttctggtgct 960
caaaatcttc gtgccaagga agttggtgcc tatgaactga gagaagtaac tcgccaattt 1020
aatgctatgt tggatcagat tgatcagttg atggtagcta ttcgtagcca ggaagaaacg 1080
acccgtcagt accaacttca agccctttcg agccagatta atccacattt cctctataac 1140
actttggaca ccatcatctg gatggctgaa tttcatgata gtcagcgagt ggtgcaggtg 1200
accaagtcct tggcaaccta tttccgcttg gcgctcaatc aaggcaagga cttgatttgt 1260
ctctctgacg aaatcaatca tgtccgccag tatctcttta tccagaaaca acgctatgga 1320
gataagctgg aatacgaaat taatgaaaat gttgcctttg ataatttagt cttacccaag 1380
ctggtcctac aaccccttgt agaaaatgct ctttaccatg gcattaagga aaaggaaggt 1440
cagggccata ttaaactttc tgtccagaaa caggattcgg gattggtcat ccgtattgag 1500
gatgatggcg ttggcttcca agatgctggt gatagtagtc aaagtcaact caaacgtggg 1560
ggagttggtc ttcaaaatgt cgatcaacgg ctcaaacttc attttggagc caattaccat 1620
atgaagattg attctagacc ccaaaaaggg acgaaagttg aaatatatat aaatagaata 1680
gaaactagct aa 1692

<210> 236
<211> 1692
<212> DNA
<213> Streptococcus pneumoniae

<400> 236
atgaagcgtt cttctctttt agttagaatg gttatttcca tctttctggt ctttctcatt 60
ctcctagctc tggttggaac tttctactat caatcaagtt cttcagccat tgaggccacc 120
attgagggca acagccaaac gaccatcagc cagactagcc actttattca gtcttatatc 180
aaaaaactag aaaccacctc gactggtttg acccagcaga cggatgttct ggcctatgct 240

gagaatccca gtcaagacaa ggtcgaggga atccgagatt tgtttttgac catcttgaag 300
tcagataagg acttgaaaac tgttgtgctg gtgaccaaat ctggtcaggt catttctaca 360
gatgacagtg tgcagatgaa aacttcctct gatatgatgg ctgaggattg gtaccaaaag 420
gccattcatc agggagctat gcctgttttg actccagctc gtaaatcaga tagtcagtgg 480
gtcatttctg tcactcaaga acttgttgat gcaaagggag ccaatcttgg tgtgcttcgt 540
ttggatattt cttatgaaac tctggaagcc tatctcaatc aactccagtt ggggcagcag 600
ggctttgcct tcattatcaa tgaaaaccat gaatttgtct accatcctca acacacagtt 660
tatagttcgt ctagcaaaat ggaggctatg aaaccctaca tcgatacagg tcagggttat 720
actcctggtc acaaatccta cgtcagtcaa gagaagattg caggaactga ttggacggtg 780
cttggcgtgt catcattgga aaagttagac caggttcgga gtcagctctt gtggaccttg 840
cttggggcca gtgtcacatc tcttcttgtc tgtctctgct tagtgtggtt cagtcttaaa 900
cgctggattg ctcctttgaa ggatttgaga gaaaccatgt tggaaattgc ttctggtgct 960
caaaatcttc gtgccaagga agttggtgcc tatgaactga gagaagtaac tcgccaattt 1020
aatgctatgt tggatcagat tgatcagttg atggtagcta ttcgtagcca ggaagaaacg 1080
acccgtcagt accaacttca agccctttcg agccagatta atccacattt cctctataac 1140
actttggaca ccatcatctg gatggctgaa tttcatgata gtcagcgagt ggtgcaggtg 1200
accaagtcct tggcaaccta tttccgcttg gcgctcaatc aaggcaagga cttgatttgt 1260
ctctctgacg aaatcaatca tgtccgccag tatctcttta tccagaaaca acgctatgga 1320
gataagctgg aatacgaaat taatgaaaat gttgcctttg ataatttagt cttacccaag 1380
ctggtcctac aaccccttgt agaaaatgct ctttaccatg gcattaagga aaaggaaggt 1440
cagggccata ttaaactttc tgtccagaaa caggattcgg gattggtcat ccgtattgag 1500
gatgatggcg ttggcttcca agatgctggt gatagtagtc aaagtcaact caaacgtggg 1560
ggagttggtc ttcaaaatgt cgatcaacgg ctcaaacttc attttggagc caattaccat 1620
atgaagattg attctagacc ccaaaaaggg acgaaagttg aaatatatat aaatagaata 1680
gaaactagct aa 1692

<210> 237
<211> 1140
<212> DNA
<213> Streptococcus pneumoniae

<400> 237
atgtttttta aattattaag agaagctctt aaagtcaagc aggttcgatc aaaaatttta 60
tttacaattt ttatcgtttt ggtctttcgt atcggaacta gcattacagt tcctggtgtg 120
aatgccaata gcttgaatgc tttaagtgga ttatccttct taaacatgtt gagcttggtg 180
tcggggaatg ccctaaaaaa cttttcgatt tttgccctag gagttagtcc ctatatcacc 240
gcttctattg ttgtccaact cttgcaaatg gatattttac ccaagtttgt agagtggggt 300
aaacaagggg aagtaggtcg aagaaaattg aatcaagcta ctcgttatat tgctctagtt 360
ctcgcttttg tgcaatctat cgggattaca gctggtttta ataccttggc tggagctcaa 420
ttgattaaaa ctgctttaac tccacaagtt tttctgacga ttggtatcat cttaacagct 480
ggtagtatga ttgtcacttg gttgggtgag caaattacag ataagggata cggaaacggt 540
gtttccatga ttatctttgc cgggattgtt tcctcaattc cagagatgat tcagggcatc 600
tatgtggact actttgtgaa cgtcccaagt agccgtatca cttcatctat cattttcgta 660
atcattttga ttattactgt attgttgatt atttacttta caacttatgt tcaacaagca 720
gaatacaaaa ttccaatcca atatactaag gttgcacaag gtgctccatc tagctcttac 780
cttccgttaa aagtaaaccc tgctggagtt atccctgtta tctttgccag ttcgattact 840
gcagcgcctg cggctattct tcagtttttg agtgccacag gtcatgattg ggcttgggta 900
agggtagcac aagagatgtt ggcaactact tctccaactg gtattgccat gtatgctttg 960
ttgattattc tctttacatt cttctatacg tttgtacaga ttaatcctga aaaagcagca 1020
gagacctaca aaagagtggt gcctatatcc atggagttcg tcctggtaaa ggtacagaag 1080
aatatatgtc taaacttctt cgtcgtcttg caactgttgg ttccctcttc cttggtgtga 1140

<210> 238
<211> 1338
<212> DNA
<213> Streptococcus pneumoniae

<400> 238
atggatatta gacaagttac tgaaaccatc gccatgattg aggagcaaaa cttcgatatt 60

agaaccatta ccatggggat ttctcttttg gactgtatcg atccagatat caatcgtgct 120
gcggagaaaa tctatcaaaa aattacgaca aaggcggcta atttagtagc tgttggtgat 180
gaaattgcgg ctgagttggg aattcctatc gttaataagc gtgtatcggt gacacctatt 240
tctctgattg gggcagcgac agatgcgacg gactacgtgg ttctggcaaa agcgcttgat 300
aaggctgcga aagagattgg tgtggacttt attggtggtt tttctgcctt agtacaaaaa 360
ggttatcaaa agggagatga gattctcatc aattccattc ctcgcgcttt ggctgagacg 420
gataaggtct gctcgtcagt caatatcggc tcaaccaagt ctggtattaa tatgacggct 480
gtggcagata tgggacgaat tatcaaggaa acagcaaatc tttcagatat gggagtggcc 540
aagttggttg tattcgctaa tgctgttgag gacaatccat ttatggcggg tgcctttcat 600
ggtgttgggg aagcagatgt tatcatcaat gtcggagttt ctggtcctgg tgttgtgaaa 660
cgtgctttgg aaaaagttcg tggacagagc tttgatgtag tagccgaaac agttaagaaa 720
actgccttta aaatcactcg tatcggtcaa ttggttggtc aaatggccag tgagagactg 780
ggtgtggagt ttggtattgt ggacttgagt ttggcaccaa cccctgcggt tggagactct 840
gtggcacgtg tccttgagga aatggggcta gaaacagttg gcacgcatgg aacgacggct 900
gccttggccc tcttgaacga ccaagttaaa aagggtggag tgatggcctg caaccaagtc 960
ggtggtttat ctggtgcctt tatccctgtt tctgaggatg aaggaatgat tgctgcagtg 1020
caaaatggct ctcttaattt agaaaaacta gaagctatga cggctatctg ttctgttgga 1080
ttggatatga ttgccatccc agaagatacg cctgctgaaa ctattgcggc tatgattgcg 1140
gatgaagcag caatcggtgt tatcaacatg aaaacaacag ctgttcgtat cattcccaaa 1200
ggaaaagaag gcgatatgat tgagtttggt ggtctattag gaactgcacc cgttatgaag 1260
gttaatgggg cttcgtctgt cgacttcatc tctcgcggtg gacaaatccc agcaccaatt 1320
catagtttta aaaattaa 1338

<210> 239
<211> 858
<212> DNA
<213> Streptococcus pneumoniae

<400> 239
atgacacaga ttattgatgg gaaagcttta gcggccaaat tgcaggggca gttggctgaa 60
aagactgcaa aattaaagga agaaacaggt ctagtgcctg gtttggtagt gattttggtt 120
ggggacaatc cagccagcca agtctacgtt cgcaacaagg agaggtcagc ccttgcggct 180
ggtttccgta gcgaagtagt acgggttcca gagaccatta ctcaagagga attgttagac 240
ctgattgcta aatacaatca ggatccagct tggcatggga ttttggttca gttgccatta 300
ccaaaacaca ttgatgaaga ggcggttcta ttggctattg acccagaaaa ggatgtggat 360
ggtttccatc ctctaaacat ggggcgtctt tggtctggtc atccagtcat gattccttcg 420
acaccggcag gaattatgga aatgttccat gaatatggga ttgacttgga aggtaaaaat 480
gcagtcgtca tcggtcgatc caatattgtc ggaaaaccta tggcccagct tcttttggca 540
aagaatgcaa cagtaacctt gactcactca cgtactcata atctttccaa ggtggctgca 600
aaagcagata ttctggttgt tgcaatcggt cgtgccaagt ttgtgactgc tgactttgtc 660
aaaccaggtg cggtagtcat tgacgttggg atgaaccgcg atgaaaatgg taagctctgt 720
ggggatgttg attatgaggc ggttgcccca cttgctagcc acattacgcc agtccctgga 780
ggtgtcggtc ctatgaccat tactatgctg atggagcaaa cctatcaggc agcacttagg 840
acattggata gaaaataa 858

<210> 240
<211> 1212
<212> DNA
<213> Streptococcus pneumoniae

<400> 240
atgtctaaat ttaatcgtat tcatttggtg gtactggatt ctgtaggaat cggtgcagca 60
ccagatgcta ataactttgt caatgcaggg gttccagatg gagcttctga cacactggga 120
cacatttcaa aaacagttgg tttgaatgtc ccaaacatgg ctaaaatagg tcttggaaat 180
attcctcgtg aaactcctct taagactgta gcagctgaaa gcaatccaac tggatatgca 240
acaaaattag aggaagtatc tcttggtaag gatactatga ctggacactg ggaaatcatg 300
ggactcaaca ttactgagcc tttcgatact ttctggaacg gattcccaga agaaatcctg 360
acaaaaatcg aagaattctc aggacgcaag gttattcgtg aagccaacaa accttattca 420
ggaacggctg ttatctatga ttttggacca cgtcagatgg aaactggaga gttgattatc 480

tatacttcag ctgaccctgt tttgcagatt gctgcccacg aagacattat tcctttggat 540
gaattgtacc gtatctgtga atacgctcgt tcgattaccc ttgagcgtcc tgcccttctt 600
ggtcgcatca ttgctcgccc ttatgtaggt gaaccaggta acttcactcg tacggcaaac 660
cgtcgtgact tggctgtatc tccatttttc ccaactgttt tggataaatt gaatgaggct 720
ggtatcgata cttatgctgt gggtaaaatc aacgatatct ttaacggtgc tggtatcaac 780
catgacatgg gtcacaacaa gtcaaatagt catggaattg atacactatt gaagactatg 840
ggacttgctg agtttgaaaa aggattctca ttcacaaacc tagttgactt tgatgccctt 900
tacggccatc gtcgtaatgc tcacggttac cgtgattgct tgcatgagtt tgatgaacgc 960
ttacctgaaa ttatcgcagc tatgagagag aatgaccttc tcttgattac tgcggaccat 1020
ggaaatgacc caacgtatgc aggaacggat cacactcggg aatatattcc attgttggcc 1080
tatagccctg cctttaaagg aaatggtctc attccagtag gacattttgc agatatttca 1140
gcgactgttg ccgataactt tggtgtggaa actgctatga ttggggaaag tttcttagat 1200
aaattggtat aa 1212

<210> 241
<211> 981
<212> DNA
<213> Streptococcus pneumoniae

<400> 241
atgtttattt ccatcagtgc tggaattgtg acatttttac taactttagt agaaattccg 60
gcctttatcc aattttatag aaaggcgcaa attacaggcc agcagatgca tgaggatgtc 120
aaacagcatc aggcaaaagc tgggactcct acaatgggag gtttggtttt cttgattact 180
tctgttttgg ttgctttctt tttcgcccta tttagtagcc aattcagcaa taatgtggga 240
atgattttgt tcatcttggt cttgtatggc ttggtcggat ttttagatga ctttctcaag 300
gtctttcgta aaatcaatga ggggcttaat cctaagcaaa aattagctct tcagcttcta 360
ggtggagtta tcttctatct tttctatgag cgcggtggcg atatcctgtc tgtctttggt 420
tatccagttc atttgggatt tttctatatt ttcttcgctc ttttctggct agtcggtttt 480
tcaaacgcag taaacttgac agacggtgtt gacggtttag ctagtatttc cgttgtgatt 540
agtttgtctg cctatggagt tattgcctat gtgcaaggtc agatggatat tcttctagtg 600
attcttgcca tgattggtgg tttgctcggt ttcttcatct ttaaccataa gcctgccaag 660
gtctttatgg gtgatgtggg aagtttggcc ctaggtggga tgctggcagc tatctctatg 720
gctctccacc aagaatggac tctcttgatt atcggaattg tgtatgtttt tgaaacaact 780
tctgttatga tgcaagtcag ttatttcaaa ctgacaggtg gtaaacgtat tttccgtatg 840
acgcctgtac atcaccattt tgagcttggg ggattgtctg gtaaaggaaa tccttggagc 900
gagtggaagg ttgacttctt cttttgggga gtgggacttc tagcaagtct cctgacccta 960
gcaattttat atttgatgta a 981

<210> 242
<211> 1281
<212> DNA
<213> Streptococcus pneumoniae

<400> 242
ttgtttaaga aaaataaaga cattcttaat attgcattgc cagctatggg tgaaaacttt 60
ttgcagatgc taatgggaat ggtggacagt tatttggttg ctcatttagg attgatagct 120
atttcagggg tttcagtagc tggtaatatt atcaccattt atcaggcgat tttcatcgct 180
ctgggagctg ctatttccag tgttatttca aaaagcatag ggcagaaaga ccagtcgaag 240
ttggcctatc atgtgactga ggcgttgaag attaccttac tattaagttt ccttttagga 300
tttttgtcca tcttcgctgg gaaagagatg ataggacttt tggggacgga gagggatgta 360
gctgagagtg gtggactgta tctatctttg gtaggcggat cgattgttct cttaggttta 420
atgactagtc taggagcctt gattcgtgca acgcataatc cacgtctgcc tctctatgtt 480
agttttttat ccaatgcctt gaatattctt ttttcaagtc tagctatttt tgttctggat 540
atggggatag ctggtgttgc ttgggggaca attgtgtctc gtttggttgg tcttgtgatt 600
ttgtggtcac aattaaaact gccttatggg aagccaactt ttggtttaga taaggaactg 660
ttgaccttgg ctttaccagc agctggagag cgacttatga tgagggctgg agatgtagtg 720
atcattgcct tggtcgtttc ttttgggacg gaggcagttg ctgggaatgc aatcggagaa 780
gtcttgaccc agtttaacta tatgcctgcc tttggcgtcg ctacggcaac ggtcatgctg 840
ttggcccgag cagttggaga ggatgattgg aaaagagttg ctagtttgag taaacaaacc 900

ttttggcttt ctctgttcct catgttgccc ctgtccttta gtatatatgt cttgggtgta 960
ccattaactc atctctatac gactgattct ctagcggtgg aggctagtgt tctagtgaca 1020
ctgttttcac tacttgggac ccctatgacg acaggaacag tcatctatac ggcagtctgg 1080
cagggattag gaaatgcacg cctccctttt tatgcgacaa gtataggaat gtggtgtatc 1140
cgcattggga caggatatct gatggggatt gtgcttggtt ggggcttgcc tggtatttgg 1200
gcagggtctc tcttggataa tggttttcgc tggttatttc tacgctatcg ttaccagcgc 1260
tatatgagct tgaaaggata g 1281

<210> 243
<211> 1263
<212> DNA
<213> Streptococcus pneumoniae

<400> 243
atgcaaactc aagaaaaaca ctcgcaagca gccgttcttg gcttgcagca cttactagcc 60
atgtactcag gatctatcct ggttcccatc atgattgcga cagcccttgg ctattcagct 120
gagcagttga cctacctgat ttctacagat atcttcatgt gtggggtggc aaccttcctc 180
caactccaac tcaacaaata ctttgggatt ggactcccag tcgttcttgg agttgcattc 240
cagtcggtcg ctcccttgat tatgattggg caaagccatg gtagtggcgc tatgtttggt 300
gcccttatcg catctgggat ttacgtggtt cttgtttcag gcatcttctc aaaagtagcc 360
aatctcttcc catctatcgt aacaggatct gttattacca cgattggttt aaccttgatc 420
cctgtcgcta ttggaaatat gggaaataac gttccagagc caactggtca aagtctcttg 480
cttgcagcta ttactgttct gattatcctc ttgatcaaca tctttaccaa aggatttatc 540
aagtctatct ctattttgat tggtctggtt gttggaactg ccattgctgc tactatgggc 600
ttggtggact tctctcctgt tgcggtagct ccacttgtcc atgtcccaac tccactctac 660
tttgggatgc caacctttga aatctcatct attgtcatga tgtgtatcat cgcaacggtg 720
tctatggttg agtcaactgg tgtttatctg gccttgtctg atatcacaaa ggatccaatc 780
gacagcacgc gccttcgcaa cggataccgc gcagaaggtt tggccgtact tctcggagga 840
atctttaaca ccttccctta caccggattt tcacaaaacg ttggtttggt taaattgtca 900
ggcatcaaaa aacgcctgcc aatctactac gcagctggtt tcctggttct ccttggactg 960
cttcctaagt ttggcgccct tgcccaaatc attccaagct ccgtcctcgg tggtgccatg 1020
ctggtaatgt ttggttttgt atcaattcaa gggatgcaaa tcctcgcccg tgttgacttt 1080
gctaacaatg aacacaactt ccttatcgca gctgtttcaa tcgctgcagg tgtcggtctc 1140
aacaacagta atctctttgt cagcatgccg acagccttcc aaatgttctt ctcaaacgga 1200
atcgtcgtag ccagcctact cgctattgtc ctcaatgccg tattaaatca taaaaagaaa 1260
taa 1263

<210> 244
<211> 2355
<212> DNA
<213> Streptococcus pneumoniae

<400> 244
atgaaagata gaataaaaga atatttacaa gacaaggaaa aggtgactgt taatgatttg 60
gctcaggctt tgggaaaaga cagttccaag gattttcgtg agttgattaa aaccttgtcc 120
ttaatggaaa gaaagcacca aattcgtttt gaagaagatg gtagtctgac attagaaatt 180
aagaaaaaac atgagattac cctcaagggg atttttcatg cccataaaaa tggctttggc 240
tttgttagtc tggaaggcga ggaggacgac ctttttgtag ggaaaaatga tgtcaactat 300
gctattgatg gtgataccgt cgaggtagtg attaagaaag tcgctgaccg caataaggga 360
acagcagcag aagccaaaat tattgatatc ctagaacaca gtttgacaac agttgtcggg 420
caaatcgttc tggatcagga aaaacctaag tatgctggct atattcgttc aaaaaatcag 480
aaaatcagtc aaccgattta tgttaagaaa ccagccctaa aattagaagg aacagaagtt 540
ctcaaagtct ttatcgataa atacccaagc aagaaacatg atttctttgt cgcgagtgtt 600
ctcgatgtag tgggacactc aacggatgtc ggaattgatg ttcttgaggt cttggaatca 660
atggacattg tatccgagtt tccagaagct gttgttaagg aagcagaaag tgtgcctgat 720
gctccgtctc aaaaggatat ggaaggtcgt ctggatctaa gagatgaaat tacctttacc 780
attgacggtg cggatgccaa ggacttggac gatgcagtgc atatcaaggc tctgaaaaat 840
ggcaatctgg agtttggggt tcacatcgca gatgtttctt attatgtgac cgaggggtct 900
gcccttgaca aggaagccct taaccgtgcg acttctgttt acgtgacaga ccgagtggtg 960

ccaatgcttc cagaacgact atcaaatggc atctgctctc tcaatcccca agttgaccgc 1020
ctgacccagt ctgctattat ggagattgat aaacatggtc gtgtggtcaa ctataccatt 1080
acacaaacag ttatcaagac cagttttcgt atgacctata gcgatgtcaa tgatatccta 1140
gctggcgatg aagaaaagag aaaagaatat cataaaattg tatcaagtat cgaactcatg 1200
gccaagcttc atgaaacttt agaaaacatg cgtgtgaaac gtggagctct caattttgat 1260
accaatgaag cgaagatttt agtggataaa caaggtaagc ctgttgatat cgttcttcgg 1320
cagcgtggta ttgccgagcg gatgattgag tcttttatgt tgatggctaa tgaaacagtt 1380
gccgaacatt tcagcaagtt ggatttgcct tttatctatc gaattcacga ggagcctaag 1440
gctgaaaagg ttcagaagtt tattgattat gcttcgagtt ttggcttgcg catttatgga 1500
actgccagtg agattagtca ggaggcactt caagacatca tgcgtgctgt tgagggagaa 1560
ccttatgcag atgtattgtc catgatgctt cttcgctcta tgcagcaggc tcgttattcg 1620
gagcacaatc acggccacta tggactagct gctgactatt atactcactt taccagtcca 1680
attcgtcgtt atccagacct tcttgttcac cgtatgattc gggattacgg ccgttctaag 1740
gaaatagcag agcattttga acaagtgatt ccagagattg cgacccagtc ttccaaccgt 1800
gaacgtcgtg ccatagaagc tgagcgtgaa gtcgaagcca tgaaaaaggc tgagtatatg 1860
gaagaatacg tgggtgaaga gtatgatgca gttgtatcaa gtattgtcaa attcggtctc 1920
tttgtcgaat tgccaaacac agttgaaggc ttgattcaca tcactaatct gcctgaattt 1980
tatcatttca atgagcgtga tttgactctt cgtggagaaa aatcaggtat cactttccga 2040
gtgggtcagc agatccgtat ccgtgttgaa agagcggata aaatgactgg agagattgat 2100
ttttcattcg tacctagtga gtttgatgtg attgaaaaag gcttgaaaca gtctagtcgt 2160
agtggcagag ggcgtgattc aaatcgtcgt tcggataaga aggaagacaa gagaaaatca 2220
ggacgctcaa atgataagcg taagcattca caaaaagaca agaagaaaaa aggaaagaaa 2280
cctttttaca aggaagtagc taagaaagga gccaagcatg gcaaagggcg agggaaaggt 2340
cgtcgcacaa aataa 2355

<210> 245
<211> 1542
<212> DNA
<213> Streptococcus pneumoniae

<400> 245
atgggcacaa caggatttac aataattgac ttaattatct tgattgttta tttacttgcg 60
gtgttggttg caggtatcta tttctctaaa aaagagatga aggaaaaaga gttctttaaa 120
ggagatggtt cggttccttg gtatgttact tcggtatcca tttttgccac aatgctcagt 180
ccgatttcct tcttgggact cgctggtagc tcttatgcag gtagctggat tttatggttt 240
gctcaattag ggatggtagt agctattcca ctgacaattc gttttatctt acctatcttt 300
gcacggatag acatcgatac ggcatatgat tacttggata aacgttttaa ttctaaagca 360
cttcgtatta tttcagcact cttgtttatt atttatcaat tgggacgtat gtctatcatt 420
atgtacctcc catcagctgg tttatcagta ttgacaggaa ttgacatcaa tattttgatt 480
attttgatgg gtgtagttgc aattgtttat tcttatactg gtggtctaaa atccgtatta 540
tggacagact ttattcaagg tgtgattctg attagtggtg tcgttttagc tttatttgta 600
ctgattgcta atattaaagg tggctttggt gcagtagcag aaacattagc aaacggaaaa 660
ttccttgctg caaatgaaaa acttttcgat cctaacttgc tttcaaactc catcttttta 720
attgtgatgg gttcaggctt tacaatcttg tcttcctatg cttcatctca agatttggtt 780
caacgtttta ctacaacaca aaatattaag aaacttaata agatgttgtt cacaaacggt 840
gttttgtcac ttgcaactgc aacagtcttt tacttgattg gtacaggctt gtacgtattc 900
tatcaagtac aaaatgcaga tagtgcagct agcaatatcc ctcaagacca aatctttatg 960
tactttattg cataccagtt accagtaggt atcacaggtt tgatcttggc agcgatttat 1020
gcagcatctc aatcaactat ttcaacaggt ttgaactctg ttgcaacttc atggacattg 1080
gatattcaag atgtcatttc taaaaatatg tcagacaatc gtcgtacgaa aattgcacaa 1140
ttcgtatctc tagcagtagg tttattctca attggtgttt ccattgtcat ggctcactca 1200
gatattaaat ctgcatacga atggttcaat agtttcatgg gacttgtact tggtctactt 1260
ggtggtgtat ttattcttgg atttgtttct aaaaaagcaa ataaacaagg tgcttatgca 1320
gcgctgattg tatcaaccat cgtcatggta tttattaaat acttccttcc tccaacagct 1380
gttagctact gggcatattc attgatttca atctctgtat cagtagtttc aggttatatt 1440
gtatctgttc ttactggaaa taaagtatct gcacctaaat atacaacgat tcatgatatt 1500
acagaaatta aagcggattc aagttgggaa gttcgtcact aa 1542

<210> 246

<211> 1455
<212> DNA
<213> Streptococcus pneumoniae

<400> 246
atgaaattta gtaaaaaata tatagcagct ggatcagctg ttatcgtatc cttgagtcta 60
tgtgcctatg cactaaacca gcatcgttcg caggaaaata aggacaataa tcgtgtctct 120
tatgtggatg gcagccagtc aagtcagaaa agtgaaaact tgacaccaga ccaggttagc 180
cagaaagaag gaattcaggc tgagcaaatt gtaatcaaaa ttacagatca gggctatgta 240
acgtcacacg gtgaccacta tcattactat aatgggaaag ttccttatga tgccctcttt 300
agtgaagaac tcttgatgaa ggatccaaac tatcaactta aagacgctga tattgtcaat 360
gaagtcaagg gtggttatat catcaaggtc gatggaaaat attatgtcta cctgaaagat 420
gcagctcatg ctgataatgt tcgaactaaa gatgaaatca atcgtcaaaa acaagaacat 480
gtcaaagata atgagaaggt taactctaat gttgctgtag caaggtctca gggacgatat 540
acgacaaatg atggttatgt ctttaatcca gctgatatta tcgaagatac gggtaatgct 600
tatatcgttc ctcatggagg tcactatcac tacattccca aaagcgattt atctgctagt 660
gaattagcag cagctaaagc acatctggct ggaaaaaata tgcaaccgag tcagttaagc 720
tattcttcaa cagctagtga caataacacg caatctgtag caaaaggatc aactagcaag 780
ccagcaaata aatctgaaaa tctccagagt cttttgaagg aactctatga ttcacctagc 840
gcccaacgtt acagtgaatc agatggcctg gtctttgacc ctgctaagat tatcagtcgt 900
acaccaaatg gagttgcgat tccgcatggc gaccattacc actttattcc ttacagcaag 960
ctttctgcct tagaagaaaa gattgccaga atggtgccta tcagtggaac tggttctaca 1020
gtttctacaa atgcaaaacc taatgaagta gtgtctagtc taggcagtct ttcaagcaat 1080
ccttcttctt taacgacaag taaggagctc tcttcagcat ctgatggtta tatttttaat 1140
ccaaaagata tcgttgaaga aacggctaca gcttatattg taagacatgg tgatcatttc 1200
cattacattc caaaatcaaa tcaaattggg caaccgactc ttccaaacaa tagtctagca 1260
acaccttctc catctcttcc aatcaatcca ggaacttcac atgagaaaca tgaagaagat 1320
ggatacggat ttgatgctaa tcgtattatc gctgaagatg aatcaggttt tgtcatgagt 1380
cacggagacc acaatcatta tttcttcaag aaggacttga cagaagagca aattaaggtg 1440
cgcaaaaaca tttag 1455

<210> 247
<211> 462
<212> DNA
<213> Streptococcus pneumoniae

<400> 247
atgaaaaaaa gagcaatagt ggcagtcatt gtactgcttt tgattgggct ggatcagttg 60
gtcaaatcct atatcgtcca gcagattcca ctgggtgaag tgcgctcctg gatccccaat 120
ttcgttagct tgacctacct gcaaaatcga ggtgcagcct tttctatctt acaagatcag 180
cagctgttat tcgctgtcat tactctggtt gtcgtgatag gtgccatttg gtatttacat 240
aaacacatgg aggactcatt ctggatggtc ttgggtttga ctctaataat cgcgggtggt 300
cttggaaact ttattgacag ggtcagtcag ggctttgttg tggatatgtt ccaccttgac 360
tttatcaact ttgcaatttt caatgtggca gatagctatc tgacggttgg agtgattatt 420
ttattgattg caatgctaaa agaggaaata aatggaaatt aa 462

<210> 248
<211> 855
<212> DNA
<213> Streptococcus pneumoniae

<400> 248
atgaatacaa atcttgcaag ttttatcgtt ggactgatca tcgatgaaaa cgaccgtttt 60
tactttgtgc aaaaggatgg tcaaacctat gctcttgcta aggaagaagg ccaacataca 120
gtaggggata cggtcaaagg ttttgcatac acggatatga agcaaaaact ccgcctgaca 180
accttagaag tgactgccac tcaggaccaa tttggttggg gacgtgtcac agaggttcgt 240
aaggacttgg gtgtctttgt ggatacaggc cttcctgaca aggaaatcgt tgtgtcactc 300
gatattctcc ctgagctcaa ggaactctgg cctaagaagg gcgaccaact ctacatccgt 360
cttgaagtgg ataagaaaga ccgtatctgg ggcctcttgg cttatcaaga agacttccaa 420

cgtcttgctc gtcctgccta caacaacatg cagaaccaaa actggccagc cattgtttac 480
cgtctcaagc tgtcaggaac ttttgtttac ctaccagaaa ataatatgct tggttttatt 540
catcctagcg agcgttacgc agagccacgt ttggggcaag tattagatgc gcgcgttatt 600
ggtttccgtg aagtggaccg cactctgaac ctctccctca aaccacgctc ctttgaaatg 660
ttggaaaacg atgctcagat gattttgact tatttggaaa gcaatggcgg tttcatgacc 720
ttaaatgaca agtcatctcc agacgacatc aaggcaacct ttggcatttc taaaggtcag 780
ttcaagaaag ctttaggtgg tcttatgaag gctggtaaaa tcaagcagga ccagtttggg 840
acagagttga tttag 855

<210> 249
<211> 1185
<212> DNA
<213> Streptococcus pneumoniae

<400> 249
atgaaagatg ttagtctatt tttattgaaa aaagttttca aaagccgctt aaactggatt 60
gtcttagctt tatttgtatc tgtactcggt gttacctttt atttaaatag tcagactgca 120
aactcacaca gcttggagag caggttggaa agtcgcattg cagccaacga gagggctatc 180
aatgaaaatg aagagaaact ctcccaaatg tctgatacca gctcggagga ataccagttt 240
gctaaaaata atttagacgt gcaaaaaaat cttttgacgc gaaagacaga aattctgact 300
ttattaaaag aagggcgctg gaaagaagcc tactatttgc agtggcaaga tgaagagaag 360
aattatgaat ttgtatcaaa tgacccgact gctagccctg gcttaaaaat gggggttgac 420
cgcgaacgga agatttacca agccctgtat cccttgaaca taaaagcaca tactttggag 480
tttccgaccc acgggattga tcagattgtc tggattttag aggttatcat cccaagtttg 540
tttgtggttg ctattatttt tatgctaaca caactatttg cagaaagata tcaaaatcat 600
ctggacacag ctcacttata tcctgtttca aaagtgacat ttgcaatatc ctctcttgga 660
gttggagtgg gatatgtaac tgtgctgttt atcggaatct gtggcttttc ttttctagtg 720
ggaagtctga taagtggttt tggacagtta gattatccct acccaattta tagcttagtg 780
aatcaagaag taactattgg gaaaatacaa gatgtattat ttcctggctt gctcttagct 840
ttcttagcct ttatcgtcat tgtggaagtt gtgtacttga ttgcttactt tttcaagcaa 900
aaaatgcctg tcctctttct ttcactcatt gggattgttg gcttattgtt tggtatccaa 960
accattcagc ctcttcaaag gattgcacat ctgattccct ttacttactt gcgttcagtg 1020
gagattttat ctggaagatt acctaagcag attgataatg tcgatctaaa ttggagcatg 1080
ggaatggtct tacttccttg cctgattatc tttttgctat tgggaattct atttattgaa 1140
agatggggaa gttcacagaa aaaagaattt tttaatagat tctag 1185

<210> 250
<211> 1347
<212> DNA
<213> Streptococcus pneumoniae

<400> 250
atgatgaagt tcatattgga tattgttagt acaccagcta ttttagtagc tttaattgca 60
atcttaggat tagttcttca gaagaagaaa ttacctgata ttattaaagg tggaattaag 120
acctttgttg gtttcttagt tgtatctggt ggtgcaggaa ttgtacaaaa ttctttaaat 180
ccatttggta ccatgtttga gcatgctttt catttatctg gcgttgtgcc gaataatgaa 240
gcaattgtag ctgtagcttt aacaacatat ggctcagcta ctgcaatgat tatgtttgca 300
ggcatggtgt tcaatatctt aatcgctcgt tttactcgat ttaaatatat ttttttaaca 360
gggcaccaca ctctatatat ggcatgtatg attgcggtca ttttatcagt tgctggcttt 420
actagcttgc ctctcatctt actaggagga ttagcactcg gtattattat gagtatttcc 480
ccagcatttg tgcaaaaata tatggttcaa ttaactggaa atgacaaggt agctttaggt 540
catttcagtt ctttgggata ttggttgagt ggttttactg gtagccttat cggtgacaaa 600
tcaaaatcaa cagaggacat taaatttcca aagagtttag cttttttacg tgatagtact 660
gttagtatta ctttatccat ggcagttatt tacattattg tagctatctt tgcagggtca 720
gaatatatag aaaaagaaat cagtagtggt acaagtggtc tagtttatgc tttacaatta 780
gcaggtcaat ttgcagcagg ggtatttgtt attttagcag gtgttcgcct tattttgggc 840
gaaattgttc cagcctttaa aggtatttca gagcgtcttg tacctaattc aaaacctgct 900
ttggattgtc cgattgttta tacttatgca cccaatgcag ttctaattgg atttatctct 960
agttttgttg gtggtttagt aagtatggta attatgattg cttcaggaac ggttgttatc 1020

ttaccaggtg ttgtgcctca tttcttctgt ggagcgactg caggtgtcat tgggaatgca 1080
tctggtggtg ttcgtggagc cactattgga gcatttttac aaggtatttt aatcagtttt 1140
cttccagtct ttttaatgcc agttttggga ggacttggtt tccaaggatc aactttctca 1200
gatgcagatt ttggtctatc aggaattatt ttaggaatgt taaatcaatt tggctcacaa 1260
gcaggcattg tgattggtct tgttcttatt ctagcagtta tgtttggagt atcctttatt 1320
aaaaagccat ctgcaacgga ggaataa 1347

<210> 251
<211> 1002
<212> DNA
<213> Streptococcus pneumoniae

<400> 251
atgattaaaa catttctctc tgccctttcg gtcattctct tttctatccc tatcataact 60
tattcttttt tcccatcttc taatcttaac atttggctat ctacccaacc tatcttggca 120
cagatttatg ccttcccctt agctactgca actatggctg ctattttaag tttcttattt 180
tttttcctat ctttttacaa gaaaaataaa caaatacggt tttactctgg cattttgctc 240
ttactatcgc tcatattact attattcgga acagataaaa ccctttcttc tgcatcaaat 300
aagactaaaa ccttaaaatt agtaacttgg aacgtcgcta atcaaataga agcacaacat 360
attgagcgaa tttttagcca ttttgacgcc gatatggcta tattccctga actagctacc 420
aatatcagag gtgagcaaga aaaccagaga atcaaactat tgtttcatca agttggactt 480
tctatggcca actatgatat tttcacttct ccacctacca atagtggaat agctcctgtg 540
actgtgattg tcaagaaaag ttatggtttc tatacagaag ctaaaacttt tcatacaaca 600
cggttcggga caattgtatt acattcgaga aaacaaaata taccagatat cattgccttg 660
catactgcgc ctcctctgcc aggtttaatg gaaatctgga agcaagactt aaacatcatt 720
cataatcaat tggcttcaaa atatccaaag gctattattg caggtgattt taatgcaact 780
atgcgtcatg gagcacttgc aaaaataagc tctcataggg acgcattaaa tgcactgcca 840
ccttttgaaa gaggaacttg gaatagccaa agtccaaaac tttttaatgc aacaatagat 900
catattttat tgcctaaaaa ccactactat gttaaagatt tagacattgt aagttttcaa 960
aactctgatc atagatgtat ttttacagaa atcacatttt aa 1002

<210> 252
<211> 1278
<212> DNA
<213> Streptococcus pneumoniae

<400> 252
atgaatccaa tccaaagatc ttgggcttat gtcagcagaa agcgactgag aagttttatt 60
ttatttctga ttttattggt cttattggcc ggaatttcag cctgtttgac tctgatgaag 120
tccaacaaaa cagtagaaag caatctttat aaatcactca atacatcttt ttctattaag 180
aagatagaga atggtcagac attcaagttg tcagacctag catctgtaag caagattaag 240
gggctggaaa atgtctctcc tgaacttgag acggtcgcaa aactaaaaga caaggaagca 300
gtgactggcg agcagagcgt ggagcgtgat gatttatcag ctgcagacaa taacttggtt 360
agcttaacgg ctcttgagga ttcatccaag gatgtaacct ttaccagttc ggctttcaat 420
ctaaaagaag ggcgacacct tcaaaaaggg gattccaaga aaatccttat ccacgaagaa 480
ttggctaaga agaacggtct ttcgcttcat gacaagattg gcttggatgc tggtcagtct 540
gaatctggaa aaggacaaac agtagagttt gagattatcg gcatcttttc tggtaaaaaa 600
caagagaaat tcacaggctt gtcttctgac ttcagtgaaa atcaagtctt tacagactat 660
gaaagtagcc aaaccctttt gggcaatagt gaagctcaag tcagtgcagc acgcttctat 720
gtagaaaatc ctaaggaaat ggacggactc atgaagcagg tagaaaactt ggccttggaa 780
aatcaaggct accaagtcga aaaggaaaac aaggcttttg aacaaatcaa agactcagtt 840
gcaactttcc aaaccttcct gaccatcttc ctttatggga tgttgatagc aggagctgga 900
gccttaattc tggttttgtc tctctggttg agagaacggg tctatgaagt ggggatttta 960
cttgcacttg gaaaaggcaa gagctcgatc ttcctacaat tctgtttaga ggtagttttg 1020
gtatctcttg gagctttgct tccagcattt gttgcaggaa acgcaatcac aacttaccta 1080
ctccaaactc tactagcaag tggagatcag gcaagcttac aagatacact agccaaagca 1140
agcagtttat caactagcat cttatctttt gcagaatcct atgtttttct agttctgctt 1200
agttgcttat ctgtagccct ttgtttccta ttcttattta gaaaatcacc gaaagaaatt 1260
ttatcatcta ttagttaa 1278

<210> 253
<211> 1380
<212> DNA
<213> Streptococcus pneumoniae

<400> 253
atgttacaca acgcatttgc ctatgttaca aggaagtttt tcaaatcgat tgtcatcttc 60
ctgattattc tcctcatggc gagcttgagt ttggtcggct tgtcaatcaa gggagctact 120
gccaaggctt ctcaggagac ctttaaaaat atcaccaata gcttctccat gcaaatcaat 180
cgtcgcgtca accaaggaac gcctcgtggt gctgggaata tcaagggtga agacatcaaa 240
aaaatcaccg aaaacaaggc cattgagtct tatgtcaaac gtatcaacgc tatcggagat 300
ttgactggat atgacctgat tgaaacgcca gaaaccaaga agaatctcac tgctgatcgt 360
gccaagcgtt ttggaagtag cttgatgatt acaggtgtca atgactcctc taaagaagac 420
aagtttgtct ctggttctta taaactagtc gaaggagagc acttaaccaa cgacgacaag 480
gataaaatcc tcttgcacaa ggacttggca gccaaacacg gctggaaagt aggggacaag 540
gttaaactgg actctaatat ctacgatgca gataatgaaa aaggagccaa ggaaacagtt 600
gaagtgacaa tcaagggact ctttgatggt cataataagt cagcagtaac ctactcacaa 660
gaactttacg aaaacacagc tattacagac attcacactg ctgcaaaact ttatggatac 720
acagaagaca cagccattta tggggacgca accttctttg taacagcaga caagaacttg 780
gatgatgtta tgaaagagtt gaatggcatc agtggtatca actggaagag ctacacactc 840
gtcaagagct cctctaacta cccagctctt gagcaatcta tctctggtat gtacaagatg 900
gccaacctcc tcttctgggg tagcttgagc ttctcagttc tcctccttgc cctcttgctc 960
agcctttgga tcaacgcccg tcgcaaggaa gtgggaattc tcctctctat cggcctcaag 1020
caggcaagta tcttgggtca attcatcacc gaatctatct tgattgctat ccctgctcta 1080
gtttctgctt acttcctagc taattacact gcccgtgcaa ttggaaacac tgtccttgcc 1140
aatgtgactt caggtgttgc caaacaggct agtaaggcgg ctcaagcctc taaccttggt 1200
ggtggtgcag aagtagatgg ctttagcaag accttgtcga gcctagacat ttccattcag 1260
acatcagact ttatcatcat ttttgtcctt gccttggttc tagtggttct cgttatggcg 1320
cttgcttcaa gcaatctcct tagaaaacaa ccaaaagagc tcttgctgga tggtgaataa 1380

<210> 254
<211> 618
<212> DNA
<213> Streptococcus pneumoniae

<400> 254
atgtcacagg ataaacaaat gaaagctgtt tctccccttc tgcagcgagt tatcaatatc 60
tcatcgattg tcggtggggt tgggagtttg attttctgta tttgggctta tcaggctggg 120
attttacaat ccaaggaaac cctctctgcc tttatccagc aggcaggcat ctggggtcca 180
cctctcttta tctttttaca gattttacag actgtcgtcc ctatcattcc aggggccttg 240
acctcggtgg ctggggtctt tatctacggg cacatcatcg ggactatcta caactatatc 300
ggcatcgtga ttggctgtgc cattatcttt tatctagtgc gcctatacgg agctgccttt 360
gtccagtctg tcgtcagcaa gcgcacctac gacaagtaca tcgactggct agataagggc 420
aatcgttttg accgcttctt tatttttatg atgatttggc ccattagccc agctgacttt 480
ctctgtatgc tggctgccct gaccaagatg agcttcaagc gctacatgac catcatcatt 540
ctgaccaaac cctttaccct cgtggtttat acctacggtc tgacctatat tattgacttt 600
ttctggcaaa tgctttga 618

<210> 255
<211> 1200
<212> DNA
<213> Streptococcus pneumoniae

<400> 255
atgagaaata tgtgggttgt aatcaaggaa acctatcttc gacatgtcga gtcatggagt 60
ttcttcttta tggtgatttc gccgttcctc tttttaggaa tctctgtagg aattgggcat 120
ctccaaggtt cttctatggc taaaaataat aaagtggcag tagtgacaac agtgccatct 180

gtagcagaag gactgaagaa tgtaaatggt gttaacttcg actataaaga cgaagcaagt 240
gccaaagaag caattaaaga agaaaaatta aaaggttatt tgaccattga tcaagaagat 300
agtgttctaa aggcagttta tcatggcgaa acatcgcttg aaaatggaat taaatttgag 360
gttacaggta cactcaatga actgcaaaat cagcttaatc gttcaactgc ttccttgtct 420
caagagcagg aaaaacgctt agcgcagaca attcaattca cagaaaagat tgatgaagcc 480
aaggaaaata aaaagtttat tcaaacaatt gcagcaggtg ccttaggatt ctttctttat 540
atgattctga ttacctatgc gggtgtaaca gctcaggaag ttgccagtga aaaaggcacc 600
aaaattatgg aagtcgtttt ttctagcata agggcaagtc actatttcta tgcgcggatg 660
atggctctgt ttctagtgat tttaacgcat attgggatct atgttgtagg tggtctggct 720
gccgttttgc tctttaaaga tttgccattc ttggctcagt ctggtatttt ggatcacttg 780
ggagatgcta tctcactgaa taccttgctc tttattttga tcagtctttt catgtacgta 840
gtcttggcag ccttcctagg atctatggtt tctcgtcctg aggactcagg gaaagccttg 900
tcgcctttga tgattttgat tatgggtggt ttttttggag tgacagctct aggtgcagct 960
ggtgacaatc tcctcttgaa gattggttct tatattccct ttatttcgac cttctttatg 1020
ccgtttcgaa cgattaatga ctatgcgggg ggagcagaag catggatttc acttgctatt 1080
acagtgattt ttgcggtggt agcaacagga tttatcggac gcatgtatgc tagtctcgtt 1140
cttcaaacgg atgatttagg gatttggaaa acctttaaac gtgccttatc ttataaatag 1200


<210> 256
<211> 714
<212> DNA
<213> Streptococcus pneumoniae

<400> 256
atgacagaaa ccattaaatt gatgaaggct catacttcag tgcgcaggtt taaagagcaa 60
gaaattcccc aagtagactt aaatgagatt ttgacagcag cccagatggc atcatcttgg 120
aagaatttcc aatcctactc tgtgattgtg gtacgaagtc aagagaagaa agatgccttg 180
tatgaattgg tacctcaaga agccattcgc cagtctgctg ttttccttct ctttgtcgga 240
gatttgaacc gagcagaaaa gggagcccga cttcataccg acaccttcca accccaaggt 300
gtggaaggtc tcttgattag ttcggtcgat gcagctcttg ctggacaaaa cgccttgttg 360
gcagctgaaa gcttgggcta tggtggtgtg attatcggtt tggttcgata caagtctgaa 420
gaagtggcag agctctttaa cctacctgac tacacctatt ctgtctttgg gatggcactg 480
ggtgtgccaa atcaacatca tgatatgaaa ccgagactgc cactagagaa tgttgtcttt 540
gaggaagaat accaagaaca gtcaactgag gcaatccaag cttatgaccg tgttcaggct 600
gactatgctg gggcgcgtgc gaccacaagc tggagtcagc gcctagcaga acagtttggt 660
caagctgaac caagctcaac tagaaaaaat cttgaacaga agaaattatt gtag 714


<210> 257
<211> 606
<212> DNA
<213> Streptococcus pneumoniae

<400> 257
atgttaaaac ttattgctat tgttggaaca aattcaaaac gttctacaaa ccgtcaattg 60
cttcaataca tgcaaaaaca ctttactgac aaagctgaaa ttgaacttgt tgaaatcaag 120
gccattcctg tcttcaacaa accagctgac aagcaagtac ctgctgaaat attggaaatt 180
gctgctaaaa tcgaagaggc agatggcgtt attatcggta ctcctgagta tgatcactct 240
attccagctg ttttgatgag cgctcttgct tggttgtctt atggtattta cccacttttg 300
aacaaaccaa tcatgattac aggtgcttct tacggtacgc ttggttcatc tcgtgcccaa 360
ttgcaacttc gtcaaatctt gaatgctcct gaaatcaagg caaatgttct tccagatgaa 420
ttcttgctct cacactctct tcaagcattt aacccaagtg gcgacttggt tgaccttgat 480
gttatcaaga aattggatgc catctttgat gacttccgta tctttgtaaa aatcacagaa 540
aaattacgta atgcacaaga attacttcgc aaagatgctg aagactttga ctgggaaaat 600
ttgtaa 606


<210> 258
<211> 843
<212> DNA

<213> Streptococcus pneumoniae

<400> 258
atgaatacct atcaattaaa taatggagta gaaattccag tattgggatt tggaactttt 60
aaggctaagg atggagaaga agcctatcgt gcagtgttag aagccttgaa ggctggttat 120
cgtcatattg atacggcggc gatttatcag aatgaagaaa gtgttggtca agcaatcaaa 180
gatagcggag ttccacgtga agaaatgttc gtaactacca agctttggaa tagtcagcaa 240
acctatgagc aaactcgtca agctttggaa aaatctatag aaaaactggg cttggattat 300
ttggatttgt atttgattca ttggccgaac ccaaaaccgc tcagagaaaa tgacgcatgg 360
aaaactcgca atgcggaagt ttggagagcg atggaagacc tctatcaaga agggaaaatc 420
cgtgctatcg gcgttagcaa ttttcttccc catcatttgg atgccttgct tgaaactgca 480
actatcgttc ctgcggtcaa tcaagttcgc ttggcgccag gtgtgtatca agatcaagtc 540
gtagcttact gtcgtgaaaa gggaatttta ttggaagctt ggggggcctt tggacaagga 600
gaactgtttg atagcaagca agtccaagaa atagcagcaa atcacggaaa atcggttgct 660
cagatagcct tggcctggag cttggcagaa ggatttttac cacttccaaa atctgtcaca 720
acctctcgta ttcaagctaa tcttgattgc tttggaattg aactgagtca tgaggagaga 780
gaaaccttaa aaacgattgc tgttcaatcg ggtgctccac gagttgatga tgtggatttc 840
tag 843

<210> 259
<211> 801
<212> DNA
<213> Streptococcus pneumoniae

<400> 259
atgaggtgca aaatgcttga tccaattgct attcaactag gacccctagc cattcgttgg 60
tatgccttat gtattgtgac aggcttgatt cttgcggttt atttgaccat gaaagaagca 120
cctagaaaga agatcatacc agacgatatt ttagatttta tcttagtagc ctttcccttg 180
gctattttag gagctcgtct ctactatgtt attttccgat ttgattacta tagtcagaat 240
ttaggagaga tttttgccat ttggaatggt ggtttggcca tttacggtgg tttgataact 300
ggggctcttg tgctctatat ctttgctgac cgtaaactca tcaatacttg ggattttcta 360
gatattgcgg cgcctagcgt tatgattgct caaagtttgg ggcgttgggg taatttcttt 420
aaccaagaag cttatggtgc aacagtggat aatctggatt atctacctgg ctttatccgt 480
gaccagatgt atattgaggg gagctaccgt caaccgactt tcctttatga gtctctatgg 540
aatctgcttg gctttgcctt gattctgatt tttagacgga aatggaagag tctcagacga 600
ggtcatatca cggcctttta cttgatttgg tatggtttcg gtcgtatggt tatcgaaggt 660
atgcgaacag atagtctcat gttcttcggc tttcgagtgt cccaatggct gtcagttgtc 720
cttatcggtc tcggtataat gatcgttatt tatcaaaatc gaaagaaggc cccttactat 780
attacagagg aggaaaacta a 801

<210> 260
<211> 390
<212> DNA
<213> Streptococcus pneumoniae

<400> 260
atgggtaaat tatcctcaat ccttttagga accgtttcag gtgcagctct tgccttgttt 60
ttaacaagtg ataagggcaa acaagtttgc agtcaggctc aagattttct agatgatttg 120
agagaagatc cggagtatgc caaggagcaa gtctgtgaaa aactgacaga agttaaggag 180
caggctacag attttgttct gaaaacaaaa gaacaggttg agtcaggtga aatcactgtg 240
gacagtatac ttgctcaaac taaatcctat gcttttcaag cgacagaagc atcaaaaaat 300
caattaaata atctcaagga gcaatggcaa gaaaaagccg aagctcttga tgactcagaa 360
gagattgtga ttgatataac agaagaataa 390

<210> 261
<211> 618
<212> DNA
<213> Streptococcus pneumoniae

<400> 261
atgaaaacta aattgatctt ttggggctct atgctctttc tcctctccct ctccatcctt 60
ctgaccattt atctggcttg gattttctat cctatggaga ttcagtggct aaacttaacg 120
aatcgagtct atctaaaacc agaaaccatt caatacaatt ttcatatctt gatgaattat 180
ctgaccaatc cttttagtca ggtcttacag atgcctgatt ttcgttcgtc agcagctggt 240
ctgcaccatt tcgcagtggt caagaatctc tttcatttgg ttcagctagt agctctagtg 300
acactgccaa gtttctatgt ctttgtcaat aggattgtga aaaaggactt tttgtctctt 360
tatcgaaaaa gtctcctggc tctagtagtc ttacctgtga tgattggact tgggggagtt 420
ttgattggtt ttgaccaatt ctttactctt ttccatcaaa ttctctttgt gggagatgat 480
acctggcttt ttgatccagc caaggatcct gttattatga ttttgccaga gaccttcttt 540
cttcatgcct tcctcctctt ttttgccctc tatgaaaact tctttggcta tctgtatctg 600
aaaagtcgta ggaagtga 618

<210> 262
<211> 1914
<212> DNA
<213> Streptococcus pneumoniae

<400> 262
atgacttatc attttactga agaatacgat attattgtaa ttggtgcggg acacgctggg 60
gttgaggctt ccttggccgc tagccgtatg ggctgtaagg tcctgcttgc gaccatcaat 120
attgaaatgc tggctttcat gccttgtaat ccctctatcg gtggttctgc caaggggatt 180
gtcgtgcgtg aagtcgatgc cctcggtggc gagatggcca aaaccattga caagacttac 240
atccagatga agatgctaaa cacagggaag gggccagctg tccgtgccct tcgtgcgcag 300
gctgacaagg aactttactc taaggagatg cgcaagacgg ttgaaaacca agaaaatctg 360
acccttcgtc aaaccatgat tgatgagatt ttggtggaag atggcaaggt tgtcggtgtg 420
cgtacagcca cccatcaaga atatgctgct aaggctgtta ttgtgacgac agggactgct 480
ctccgtgggg aaattatcat cggagacctc aagtactcat caggtcctaa ccacagcttg 540
gcttctatta acctagctga caatctcaag gaactgggtc tcgaaatcgg tcgtttcaag 600
acaggaaccc ctccacgtgt caaggcttct tctatcaatt acgatgtgac agaaattcag 660
ccaggagacg aagtgcctaa tcatttctca tacacttcac gtgatgagga ttatgtcaag 720
gaccaagtac catgctggtt gacctatacc aatggtacca gtcatgagat tatccaaaac 780
aacctccacc gtgcgcctat gtttacaggt gtggtcaagg gagtggggcc tcgttactgt 840
ccgtcgattg aagacaagat tgtgcgcttt gcggacaagg aacgtcacca actcttcctt 900
gagccagaag ggcgcaatac tgaggaagtc tatgtgcaag gactttcaac cagtctgcct 960
gaggatgtcc agcgtgactt ggtgcattcc atcaaaggtt tggaaaatgc agagatgatg 1020
cggacaggtt atgctattga gtatgatatg gtcttgcctc atcagttgcg tgcgactttg 1080
gaaaccaaga aaatctcagg tctcttcact gctggtcaga caaatggaac atcaggttac 1140
gaagaggcag caggccaagg gattatcgcg ggtatcaatg cggctctgaa aatccaaggc 1200
aagcctgaat tgattttgaa gcgcagtgat ggttatatcg gggtgatgat cgacgacttg 1260
gtgaccaagg gaaccattga accctaccgt ctcttgacca gtcgtgctga ataccgtctc 1320
attcttcgtc atgacaatgc tgatatgcgc ttgactgaga tgggacgcga gattggcctt 1380
gtggacgatg aacgctgggc tcgttttgaa atcaagaaaa atcaatttga taatgagatg 1440
aagcgcctag acagtatcaa actcaagcca gtcaaggaaa ccaatgccaa ggttgaggag 1500
atgggcttca aacccttgac cgatgcagtg acagccaagg aattccttcg ccgtccagaa 1560
gtttcttacc aagatgtggt ggccttcatc ggaccagctg cagaagactt ggatgacaag 1620
attatcgaat tgattgaaac agaaatcaag tatgaaggct atatttccaa agccatggac 1680
caggttgcca agatgaaacg catggaagaa aaacgcattc cggccaatat cgactgggat 1740
gacattgatt ctatcgcaac cgaagcccgt cagaagttca aactcatcaa tccagaaacc 1800
atcggccaag ccagccgtat ttcgggagta aacccagcag atatttctat tttgatggtg 1860
tatctggaag gtaaaaatcg tagtatttct aaaactcttc aaaaatcaaa atga 1914

<210> 263
<211> 684
<212> DNA
<213> Streptococcus pneumoniae

<400> 263

atgaaagtat tagcttttga tacgtccagc aaggctcttt ctctggctat tttagaggat 60
aagcaggttc ttgccgagac gacgattaat attaagaaaa atcacagtat tactcttatg 120
cctgccatcg attttttgat ggcaagtttg gattggacac ccaaggattt ggaccgaatc 180
gtggtagctg aagggccggg tagctataca ggcttgcgaa ttgcggtagc aactgctaag 240
accttagctc acaccctgaa catcgagttg gttggtatgt cgagtctctt ggctctggtg 300
ccccatcaac aagaaggttt gtttgtcccc ttgatggatg cgcgtcgcaa taatgtttat 360
gcaggatttt atgaaaatgc caaacctgtc atggcagaag cgcacctatc ttttgaagag 420
gtgctagaaa aagtcaaggg tactagtcag gtaacctttg tcggagaagt tggccccttt 480
gttgagcaga ttcaaaaaca cttgccaagg actgattaca aagaaacatt gcccaatgca 540
gctaatctag ctcttttggc ctgggacaag gaagcagact ccttgcatga ttttgtgccg 600
aattacctca aacgagtcga ggctgaggaa aactggctca agaaccatac cgagtctggc 660
gagtcttaca ttaaacgcct atga 684

<210> 264
<211> 438
<212> DNA
<213> Streptococcus pneumoniae

<400> 264
atgatagaaa tcaagcgaat tcaacaacag cctgacctag ctcaagccat ctacgctgtt 60
atggcagctg tttacctagt cagtccttgg actctggagc aaatccaagc agatctgtcc 120
caagaccaga cttggtatgc attggcttat gatggggcag aagtgattgg atttctagct 180
gtgcaggaga atctttttga agcagaagtc ctgcaaatcg ctgtcaaagg agcttatcag 240
ggtcagggga ttgcgtcagc cttgtttgct caattgccga cagacaagga aattttcctc 300
gaagtcagac agtcaaatca acgagcgcaa gcattttaca agaaagaaaa gatgacagtt 360
atcgctgagc gaaaggccta ctaccatgac ccagtcgagg acgccattat catgaagaga 420
gaaatagatg aaggatag 438

<210> 265
<211> 558
<212> DNA
<213> Streptococcus pneumoniae

<400> 265
atgacaaaac aagtcttatt agtggatgat gaagaacaca ttctgaaatt gcttgactac 60
catttaagta aggaaggctt ttctactcaa ttggtgacaa atggacggaa ggccttagct 120
ttggcagaaa cagaaccctt tgattttatc ttgcttgata tcatgttacc acaattagat 180
ggcatggaag tttgtaagcg gctgagagcc aaaggcgtca aaactccaat tatgatggtt 240
tctgcgaaaa gtgatgaatt tgataaggtt ttggccttgg aattaggggc tgatgactac 300
ctgaccaagc cttttagccc tagagaattg ctggcgcgtg tcaaggctgt cctcaggcga 360
actaaaggag aacaagaagg agatgattca gataatatcg ctgacgattc ttggctattt 420
gggaccttga aagtataccc tgagcgtcat gaagtctaca aggcgaataa gttactgagt 480
ttgaccccaa aagaatttga aagcgataaa aatccgtttt ttgaagtttt caaagtttcg 540
aaagtaaccg cccaataa 558

<210> 266
<211> 1437
<212> DNA
<213> Streptococcus pneumoniae

<400> 266
atgactactt ttaaagatgg atttttatgg ggtggtgctg ttgctgctca tcaacttgaa 60
ggtggatggc aagaaggtgg caagggaatt agtgttgctg atgttatgac tgctggtcgt 120
catggagtag ctcgtgaaat tactttggga gttttagagg gtaaatatta tccaaatcat 180
gaggcgatag atttttatca ccgttataaa gaagatatag cactttttgc tgaaatggga 240
ttcaagtgct tccgtacctc tattgcatgg acacgtatct ttccaaaagg tgatgagtta 300
gagccgaatg aagaaggatt acagttttat gataatcttt ttgatgaatg cttaaagaat 360
ggtattgaac ctgtcatcac tctatctcat tttgaaatgc cttatcactt agtgaccgaa 420

tatggtggtt ggaaaaatag gaaattgatt gatttctttg ctcgttttgc agaagtcgta 480
tttaaacgtt acaaagataa ggttaaatat tggatgactt tcaatgaaat caataatcaa 540
gcgaattatc aggaagattt tgcaccattt actaactcag gtattgtata tgaggaaggt 600
gataatagag aagcaattat gtatcaagca gcacattacg aattagttgc ttctgcacga 660
gctgtaaaaa ttggtcatga gattaatcca gattttcaaa taggttgtat gattgcgatg 720
tgtccaattt atccagttac ttgcaatcct aaggatatct taatggcaat gaaagctatg 780
cagaagcgtt attattttgc tgatgtgcat gttttaggta aatatcctga gcatattttc 840
aagtattggg aacgaaaagg tatttcagtt gattttactg cccaggataa agaagattta 900
cttggtggga ctgtagatta cattggtttc agttactata tgtcctttgc tatcgactct 960
catcgtgaaa ataatcctta ttttgattat cttgaaacag aagatttagt gaaaaataat 1020
tatgttaagg cttctgaatg ggagtggcaa attgatccag aaggtttgcg ttatgcgtta 1080
aattggttta cagaccacta tcacttacca ctctttattg ttgaaaatgg ttttggagct 1140
atagatcaag ttgcagcaga tggtatggta catgatgatt atagaattga atatctaggt 1200
gcccatattc gtgaaatgaa aaaggctgta gttgaagatg gtgttgattt aatgggttat 1260
actccatggg gatgtattga tttggtttca gctggtaccg gtgaaatgcg gaaacgttat 1320
ggctttattt atgtagataa agatgataat gggaagggaa gttataatcg ttccccgaaa 1380
aaatcttttg gctggtataa ggaagttatt tcatctaacg gtgaatcagt agaatag 1437

<210> 267
<211> 1347
<212> DNA
<213> Streptococcus pneumoniae

<400> 267
atggatcaac aaaacgggtt gtttggtttt cttgaaaacc atgttatggg accaatgggc 60
aaacttgctc agtttaaagt agtacgtgct atcacggctg caggtatggc tgctgtacca 120
tttactattg taggatcaat gtttttggta ttcagtattt tgccacaagc tttctcattt 180
tggccaattg tggcagatat tttctctgct tcatttgata aattcacatc actttacatg 240
gttgcaaact atgcgactat gggttctcta tctctttatt tcgttctatc acttgcatat 300
gaattgacaa aaatttatgc agaggaagaa gaactcaata tgaatcctct taatggtgcc 360
ttgcttgcct tgatggcttt tgtcatgaca gtaccgcaaa tcatttttga tggtggaatg 420
atgaagactg tgacaagtct aaaagaaggt gcagtaattg cagatggatg ggcaatggga 480
aatgtagtcg cacgttttgg gacaacaggg atttttaccg caatcattat ggcaattgtg 540
actgttctta tttatcgtat gtgtgttaaa cataattggg ttattaaaat gcctgaagct 600
gttccagaag gagtttctcg tggatttacc gctttggttc cgggatttgt tgttgcattt 660
gttgttatct ttatcaacgg tcttcttgta gcaatgggaa cagatatttt taaagtcatt 720
gcaattccat ttggttttgt atccaatctg actaattcgt ggattggttt aatgattatt 780
tatctattga ctcaactact ttggattgta ggtatccacg gtgcgaacat tgtttttgca 840
tttgttagtc caattgctct tgctaacatg gctgaaaatg ctgctggcgg gcacttcgct 900
gttgcaggtg aattttctaa tatgtttgta attgcaggtg gttctggtgc aactttagga 960
ctatgtttat atattgcttt tgcctctaaa tctgaacagc ttaaagcaat aggacgagca 1020
tctgtagttc cagccttatt taatattaat gaaccattaa tttttggatt acctattatc 1080
tataatccag ccttggctat accatttatt ttagcaccaa tggttactgc tactatttat 1140
tacgtagcga attctctaaa ctttattaag ccaattatcg cacaggttcc atggccaact 1200
ccagtaggga ttggagcttt cttagggaca gcagatcttc gagctgtatt agttgctcta 1260
gtatgtgcat ttgcagcatt cctagtctat cttccattca tccgtgtata tgatcaaaaa 1320
ttggtgaaag aagagcaagg tatctaa 1347

<210> 268
<211> 1974
<212> DNA
<213> Streptococcus pneumoniae

<400> 268
atgaaaaaat tttatgtaag tccaattttt cctattctag taggattgat tgcgtttgga 60
gtcttatcca ctttcattat ttttgttaat aataatctgt tgacggtttt aatttgtttt 120
ctttttgtag gaggctatgt ttttttattt aagaaactga gagtgcatta tacaaggagt 180
gatgtagaac agatacagta tgtaaaccac caagcggaag aaagtttgac agctctattg 240
gaacagatgc ctgtaggtgt tatgaaattg aatttatctt ctggagaggt tgagtggttt 300

aatccctatg ctgaattgat tttgaccaag gaagatggtg attttgattt agaagctgtt 360
caaacgatta tcaaggcttc agtaggaaat ccgtctactt atgccaagct tggtgagaag 420
cgttatgctg ttcatatgga tgcttcttcc ggtgttttgt attttgtaga tgtatccagg 480
gaacaagcca taacagatga attggtaaca agtagaccag tgattgggat tgtctctgtg 540
gataattatg atgatttgga ggatgaaact tctgagtcag atattagtca aatcaatagt 600
tttgtagcta attttatatc agagttttca gaaaaacaca tgatgttttc tcgtcgggta 660
agtatggatc gattttatct atttactgac tacacggtgc ttgagggctt gatgaatgat 720
aaattttctg ttattgatgc tttcagagaa gagtcgaaac agagacagtt gcccttgacc 780
ttaagtatgg gattttctta tggcgatgga aatcatgatg agatagggaa agttgctttg 840
ctcaatttga acttggctga agtacgtggt ggcgaccagg tggttgttaa ggaaaacgac 900
gaaacgaaaa atccagttta ttttggtggt gggtctgctg cttcaatcaa gcgtacacgg 960
actcgtacgc gcgctatgat gacagctatt tcagataaga ttcggagtgt agatcaggtt 1020
tttgtagtcg gtcacaaaaa tttagacatg gatgctttgg gctctgctgt aggtatgcag 1080
ttgttcgcca gcaatgtgat tgaaaatagc tatgctcttt atgatgaaga acaaatgtct 1140
ccagatattg aacgagctgt ttcattcata gaaaaagaag gagttacgaa gttgttgtct 1200
gttaaggatg caatggggat ggtgaccaat cgttctttgt tgattcttgt agaccattca 1260
aagacagcct taacattatc aaaagaattt tatgatttat ttacccaaac cattgttatt 1320
gaccaccata gaagggatca ggatttccca gataatgcgg ttattactta tatcgaaagt 1380
ggtgcaagta gtgccagtga gttggtaacg gaattgattc agttccagaa ttctaagaaa 1440
aatcgtttga gtcgtatgca agcaagtgtc ttgatggctg gtatgatgtt ggatactaaa 1500
aatttcacct cgcgagtaac tagtcggaca tttgatgttg ctagctatct cagaacgcgc 1560
ggaagtgata gtattgctat ccaggaaatc gctgcgacag attttgaaga atatcgtgag 1620
gtcaatgaac ttattttaca ggggcgtaaa ttaggttcag atgtactaat agcagaggct 1680
aaggacatga aatgctatga tacagttgtt attagtaagg cagcagatgc catgttagcc 1740
atgtcaggta ttgaagcgag ttttgttctt gcgaagaata cacaaggatt tatctctatc 1800
tcagctcgaa gtcgtagtaa actgaatgta caacggatta tggaagagtt aggcggtgga 1860
ggccacttta atttggcagc agctcaaatt aaagatgtaa ccttgtcaga agcaggtgaa 1920
aaactgacag aaattgtatt aaatgaaatg aaggaaaagg agaaagaaga atga 1974

<210> 269
<211> 1416
<212> DNA
<213> Streptococcus pneumoniae

<400> 269
atgaaagaga aaaatatgtg gaaagaattg ttgaatcgtg caggctggat tttggtcttt 60
ttacttgccg tccttttata tcaggttccc ctagtggtta cctctatttt gactttaaaa 120
gaagtagccc tgctacagtc agggctgata gttgctggcc tttcaattgt ggttctggct 180
ctatttatta tgggagctcg taaaaccaag ttagctagtt ttaatttttc tttttttaga 240
gctaaagatt tggcacgttt gggcttgagt tatctagtta ttgtcgggtc aaatatactt 300
ggttccattt tattgcaact gtcaaatgag acgacaacag ctaaccagtc tcagattaat 360
gatatggttc aaaatagttc gttgatttcc agtttcttct tgctagcctt gcttgctccg 420
atttgtgagg aaatcttgtg tcgtgggatt gttcctaaaa agattttccg aggcaaggag 480
aacttgggat ttgtagtcgg tacgattgtg tttgctttat tgcatcaacc aagtaattta 540
ccttctttat tgatttatgg aggtatgtcg acagttctat cttggacagc ctacaagacc 600
caacgtttgg aaatgtcgat cttgcttcac atgattgtta atgggattgc tttctgtttg 660
ttggctcttg tggtgattat gagtcggaca ttaggaattt ctgtttaaat gaaagagaaa 720
aatatgtgga aagaattgtt gaatcgtgca ggctggattt tggtcttttt acttgccgtc 780
cttttatatc aggttcccct agtggttacc tctattttga ctttaaaaga agtagccctg 840
ctacagtcag ggctgatagt tgctggcctt tcaattgtgg ttctggctct atttattatg 900
ggagctcgta aaaccaagtt agctagtttt aatttttctt tttttagagc taaagatttg 960
gcacgtttgg gcttgagtta tctagttatt gtcgggtcaa atatacttgg ttccatttta 1020
ttgcaactgt caaatgagac gacaacagct aaccagtctc agattaatga tatggttcaa 1080
aatagttcgt tgatttccag tttcttcttg ctagccttgc ttgctccgat ttgtgaggaa 1140
atcttgtgtc gtgggattgt tcctaaaaag attttccgag gcaaggagaa cttgggattt 1200
gtagtcggta cgattgtgtt tgctttattg catcaaccaa gtaatttacc ttctttattg 1260
atttatggag gtatgtcgac agttctatct tggacagcct acaagaccca acgtttggaa 1320
atgtcgatct tgcttcacat gattgttaat gggattgctt tctgtttgtt ggctcttgtg 1380
gtgattatga gtcggacatt aggaatttct gtttaa 1416

<210> 270
<211> 555
<212> DNA
<213> Streptococcus pneumoniae

<400> 270
atggatacac aaaagattga agcggctgta aaaatgatta tcgaggctgt aggagaggac 60
gctaatcgcg agggcttgca ggaaacacct gctcgtgtag cccgtatgta tcaagagatt 120
ttttcaggtc ttggtcaaac agcagaggaa catttgtcaa aatcctttga aattattgac 180
gataatatgg tggtagaaaa ggatatcttt ttccatacca tgtgtgaaca ccacttcttg 240
ccattttatg gtagagcgca cattgcctac attccagatg gtcgtgtggc aggcttgtct 300
aagctagccc gtacggttga agtttattcg aaaaaaccac aaattcaaga acgtttgaat 360
atcgaagtgg ccgatgcctt gatggactat ctaggtgcta aaggagcctt tgttgtcatt 420
gaggcggaac atatgtgtat gagtatgcgt ggtgttagaa aaccaggcac tgcaaccttg 480
acgacagtag ctcgtggtct atttgaaaca gataaggatc tccgtgacca agcttatcgt 540
ttaatggggc tataa 555

<210> 271
<211> 1050
<212> DNA
<213> Streptococcus pneumoniae

<400> 271
atgaaagact tgtttttaaa gagaaagcag gcctttcgta aggagtgtct tggttatctg 60
cgctatgtgc tcaatgacca ctttgtcttg ttcctgcttg tcctgttggg ctttctagcc 120
taccagtaca gtcaactctt acaacatttt cctgaaaatc attggcctat ccttttgttt 180
gtaggaatta cgtctgtttt acttttactt tggggaggaa ctgccaccta tatggaggct 240
ccagacaagc tctttctctt agttggagaa gaggaaatta agctccatct caagcgtcaa 300
actggcattt ccctagtctt ttggctcttt gtacagaccc ttttcttgct gttatttgcg 360
cctttatttt tagcaatggg ttatggcttg ccagtttttc tgctctatgt gcttttattg 420
ggggtaggaa aatatttcca cttttgtcaa aaggccagca aatttttcac tgaaactgga 480
ctggactggg actatgttat ttctcaagaa agcaagcgta agcaagtctt gcttcgtttc 540
tttgccctct ttacgcaggt caagggaatt tcaaacagcg ttaagcgtcg tgcctatctg 600
gactttattt taaaggctgt tcagaaggtg cctgggaaga tttggcaaaa tctctatctg 660
cgttcttatc tgcgaaatgg cgacctcttt gctctcagtc ttcgtcttct cttgctttcc 720
ttgctggcgc aggtttttat cgagcaagct tggattgcga cagcagtggt agttctcttt 780
aactacctct tgctcttcca gttgctggcc ctctatcatg cctttgacta ccagtatttg 840
acccaactct ttccgctgga caaggggcaa aaggaaaaag gcttacagga ggtagttcga 900
ggattgacca gttttgtttt acttgtggaa ttagttgttg ggttgattac cttccaagaa 960
aaactagccc ttctagcctt actaggagct ggtttggttt tactagtctt gtatttgcct 1020
tatcaggtaa aacgtcagat gcaggactaa 1050

<210> 272
<211> 1197
<212> DNA
<213> Streptococcus pneumoniae

<400> 272
atgagaaaat caatagtatt agcggcagat aatgcctatc ttattccttt agagacgact 60
ataaagtctg tattgtatca caatagagat gttgattttt atattctcaa cagtgatata 120
gctcctgaat ggtttaaatt attggggaga aaaatggaag ttgtgaattc tacaattcgc 180
agtgtacaca ttgataaaga actttttgaa agctataaaa caggacctca tataaattat 240
gcttcttact ttagattttt tgcgacagaa gtggttgaat ctgatagggt attgtatctg 300
gattccgata tcattgtaac tggggaacta gctactttgt ttgagataga tctcaaagga 360
tattcaattg gtgctgttga tgatgtctat gcctatgaag gacgaaaatc tggatttaat 420
actggtatgt tactaatgga tgttgcaaag tggaaagaac attctattgt caatagttta 480
ttggaattag cggccgagca gaatcaagtt gttcatcttg gggatcagag tattttaaat 540
atttattttg aggataattg gctagcctta gataaaacat ataattatat ggtgggtatt 600

gatatttatc accttgctca agaatgtgaa cgtctagatg acaatccacc tacaattgtt 660
cactatgcta gtcatgataa accttggaat acatatagta tatctagact acgtgaatta 720
tggtgggttt atagagattt ggattggtca gagattgctt ttcaacgttc cgatttaaat 780
tattttgaaa gaagcaatca gtctaaaaaa caagtgatgc ttgtgacatg gagtgcagat 840
ataaaacatt tagagtattt agtacaacgg ttacctgatt ggcattttca tttggctgca 900
ccgtgtgatt gttctgagga gctgacctct ctatcacagt atacgaatgt aacagtatat 960
caaaatgtat tacatagtag aattgattgg ctattggacg attctatagt ttatttagat 1020
attaatacag gtggagaggt ttttaatgta gttacaaggg cacaagaaag tggcaagaaa 1080
atcttcgctt ttgatatcac acgtaaaagt atggatgatg gactctatga cggtattttt 1140
tctgtggaga gaccagatga tttagtggat agaatgaaga atatagagat agagtaa 1197

<210> 273
<211> 1218
<212> DNA
<213> Streptococcus pneumoniae

<400> 273
atgactaaga tttattcgtc aatagcagta aaaaaaggac tatttacctc atttctactg 60
tttatctatg tattgggaag tcgtattatt ctcccttttg ttgacctaaa tactaaagat 120
tttttaggag gttcaacagc ctatctagcc ttctcagccg ccctaacagg tgggaatcta 180
agaagtttat caattttttc tgttggatta tccccttgga tgtccgccat gattttatgg 240
cagatgtttt ctttttctaa acggttgggt ttaacatcta cgtctataga aatacaagat 300
cgccgtaaaa tgtacctgac cttgctaatt gctgtgattc aatccttggc agttagcttg 360
agactgccag tacaatcctc ctattctgca atattggttg ttctaatgaa tacaatattg 420
ctgatagcag gaacattttt tcttgtttgg ttgtcagatt taaatgcgag tatggggatt 480
ggaggttcta ttgtaatcct cctatccagt atggttttaa atattcctca ggatgttttg 540
gaaacatttc agacagtaca cattccaaca gggattattg tgttacttgc tttattaacc 600
cttgtctttt cttatttact tgcccttatg tatcgagctc gctatttggt tcctgttaat 660
aaaattggct tacacaatcg atttaaacgc tattcttatc tcgaaatcat gttgaatcct 720
gcaggtggga tgccttatat gtatgtgatg agtttcttta gtgtaccagc ttatttgttc 780
atcttgttgg gatttatttt ccctaatcat tcagggttag cggctttatc aaaggaattt 840
atggttggaa agcctttgtg ggtctatgtt tatatttcgg tcttattttt atttagtatc 900
atttttgctt ttgttacgat gaatggagaa gagattgcag accgtatgaa aaaatctgga 960
gaatacattt atggtattta tccaggtgcg gatactagtc gatttattaa tcgattggtc 1020
cttcgtttct cagtcatagg tggtctcttt aatgtgatta tggcaggtgg tcccatgctt 1080
tttgttttgt ttgatgaaaa gttattacga ttggcaatga ttccaggctt atttatgatg 1140
ttcgggggca tgatttttac gattagagac gaggtcaagg ctttaaggct aaatgagacc 1200
tatagacctt tgatttag 1218

<210> 274
<211> 2292
<212> DNA
<213> Streptococcus pneumoniae

<400> 274
atgtcctctc tttcggatca agaattagta gctaaaacag tagagtttcg tcagcgtctt 60
tccgagggag aaagtctaga cgatattttg gttgaagctt ttgctgtggt gcgtgaagca 120
gataagcgga ttttagggat gtttccttat gatgttcaag tcatgggagc tattgtcatg 180
cactatggaa atgttgctga gatgaatacg ggggaaggta agaccttgac agctaccatg 240
cctgtctatt tgaacgcttt ttcaggagaa ggagtgatgg ttgtgactcc taatgagtat 300
ttatcaaagc gtgatgccga ggaaatgggt caagtttatc gttttctagg attgaccatt 360
ggtgtaccat ttacggaaga tccaaagaag gagatgaaag ctgaagaaaa gaagcttatc 420
tatgcttcgg atatcatcta cacaaccaat agtaatttag gttttgatta tctaaatgat 480
aacctagcct cgaatgaaga aggtaagttt ttacgaccgt ttaactatgt gattattgat 540
gaaattgatg atatcttgct tgatagtgca caaactcctc tgattattgc gggttctcct 600
cgtgttcagt ctaattacta tgcgatcatt gatacacttg taacaacctt ggtcgaagga 660
gaggattata tctttaaaga ggagaaagag gaggtttggc tcactactaa gggggccaag 720
tctgctgaga atttcctagg gattgataat ttatacaagg aagagcatgc gtcttttgct 780
cgtcatttgg tttatgcgat tcgagctcat aagctcttta ctaaagataa ggactatatc 840

attcgtggaa atgagatggt actggttgat aagggaacag ggcgtctaat ggaaatgact 900
aaacttcaag gaggtctcca tcaggctatt gaagccaagg aacatgtcaa attatctcct 960
gagacgcggg ctatggcctc gatcacctat cagagtcttt ttaagatgtt taataagata 1020
tctggtatga cagggacagg taaggtcgcg gaaaaagagt ttattgaaac ttacaatatg 1080
tctgtagtac gcattccaac caatcgtccg agacaacgga ttgactatcc agataatcta 1140
tatatcactt tacctgaaaa agtgtatgca tccttggagt acatcaagca ataccatgct 1200
aagggaaatc ctttactcgt ttttgtaggc tcagttgaaa tgtctcaact ctattcgtct 1260
ctcttgtttc gtgaagggat tgcccataat gtcctaaatg ctaataatgc ggcgcgtgag 1320
gctcagatta tctccgagtc aggtcagatg ggggctgtga cagtggctac ctctatggca 1380
ggacgtggta cggatatcaa gcttggtaaa ggagtcgcag agcttggggg cttgattgtt 1440
attgggactg agcggatgga aagtcagcgg atcgacctac aaattcgtgg ccgttctggt 1500
cgtcagggag atcctggtat gagtaaattt tttgtatcct tagaggatga tgttatcaag 1560
aaatttggtc catcttgggt gcataaaaag tacaaagact atcaggttca agatatgact 1620
caaccggaag tattgaaagg tcgtaaatac cggaaactag tcgaaaaggc tcagcatgcc 1680
agtgatagtg ctggacgttc agcacgtcgt cagactctgg agtatgctga aagtatgaat 1740
atacaacggg atatagtcta taaagagaga aatcgtctaa tagatggttc tcgtgactta 1800
gaggatgttg ttgtggatat cattgagaga tatacagaag aggtagcggc tgatcactat 1860
gctagtcgtg aattattgtt tcactttatt gtgaccaata ttagttttca tgttaaagag 1920
gttccagatt atatagatgt aactgacaaa actgcagttc gtagctttat gaagcaggtg 1980
attgataaag aactttctga aaagaaagaa ttacttaatc aacatgactt atatgaacag 2040
tttttacgac tttcactgct taaagccatt gatgacaact gggtagagca ggtagactat 2100
ctacaacagc tatccatggc tatcggtggt caatctgcta gtcagaaaaa tccaatcgta 2160
gagtactatc aagaagccta cgcgggcttt gaagctatga aagaacagat tcatgcggat 2220
atggtgcgta atctcctgat ggggctggtt gaggtcactc caaaaggtga aatcgtgact 2280
cattttccat aa 2292

<210> 275
<211> 960
<212> DNA
<213> Streptococcus pneumoniae

<400> 275
atgataggga ctttcgccgc tgctcttgta gctgtactag caaatttcat cgtccctatt 60
gaaattaccc caaatagtgc caatactgaa attgcaccac cagatgggat tgggcaggtt 120
ctcagcaacc tcttgctcaa actggttgac aacccagtca acgccctgct tactgctaac 180
tatattagaa tcttatcttg ggcagtcatt tttggaatcg ctatgagaga agccagtaaa 240
aatagtcaag aattgctaaa aactatcgct gacgtgactt ctaaaattgt cgaatggatc 300
atcaatctgg ctccatttgg aatccttggt cttgttttta aaaccatttc tgacaaggga 360
gtcggaagcc ttgccaacta cggtatttta ttggttctat tagtaacgac tatgcttttt 420
gttgcccctg tggtcaaccc tttgattgcc ttcttcttta tgagacgcaa tccttaccct 480
ctagtttgga actgcctccg tgtcagcggt gtgacagcct tttcactcgg tagttctgcg 540
actaacattc ctgtcaacat gaaactctgc catgaccttg gactcaaccc agatacctat 600
tctgtttcta tcccactcgg ttctactatc aatatggctg gagtagcgat taccattaac 660
cttttgaccc ttgctgcagt taacactctt ggaattcctg ttgactttgc cacagccttt 720
gtcctcagtg tggtagcagc tatctcatcc tgtgatgctt caggtattgc cggaggttcc 780
ctccttctta tcccagttgc ttgtagcctt ttcggtattt ctaacgatat tgccatacaa 840
attgttgggg ttggttttgt gattggtgtc atccaagact catgtgaaac agcccttaac 900
tcttctacag atgtcctctt taccgccgtt gccgaatacg cagcaacccg taaaaaataa 960

<210> 276
<211> 954
<212> DNA
<213> Streptococcus pneumoniae

<400> 276
atgtctatta gccaacgtac gaccaagctc atcttagcta cctgtcttgc ctgcctgctt 60
gcttattttc tcaatctttc gtcagcagtt tcggctggaa ttatcgctct cttgagccta 120
tctgatacgc gtagaagtac tttaaaactg gctcgcaatc gtcttttttc tatgcttcta 180
gctctggcta tcggtgttct agcttttcac ttgagcggat ttcatatctg gagtctcggc 240

ctctatctgg ccttctacgt tcctttagcc tacaagatgg gctgggaaat tggcatcaca 300
ccaagcactg ttttggttag ccatctcttg gttcaagagt caacctctcc agaccttcta 360
gtcaatgaat tccttctctt tgctattggt acaggatttg ccttgcttgt taatctctat 420
atgccttcac gagaagagga aatccagcac taccacacgc tggtggaaga aaagttaaaa 480
gatatcctcc agcgcttcaa atactattta tccagaggag acggacgcaa ccgagcacag 540
ctggtagcag aattagacac gcttttgaaa gaagccctca gactggtcta tttggatcac 600
tctgaccacc tctttcacca gacagactac catatccact actttgagat gagacagcga 660
caaagtcgta tcctgagaaa catggcccaa cagattaaca cttgtcacct tgccgccagt 720
gaaagcctga tcttagcgca actcttttca aaaattgcag gtcaactgag ccagaccaat 780
cctgcttctg atttgctaga tgaaattgaa cgttatctgg aagtcttccg gaaccgcagt 840
ctgcccaaga caagagaaga atttgaaacc cgcgccaccc ttcttcaact cctacgtgaa 900
gccaaaacct tcatccaagt aaaagttgat ttttaccaaa aatatagaca gtaa 954

<210> 277
<211> 1614
<212> DNA
<213> Streptococcus pneumoniae

<400> 277
atggaaatca tgtcgcttgc gattgctgtt tttgccgtca tcattggttt agtcattgga 60
tatgtcagca tctcagctaa gatgaaatca tctcaggaag ctgcagagtt gatgctttta 120
aatgctgaac aagaagcaac taatttacgt ggacaagctg agcgtgaagc ggatttactt 180
gttaatgaag ccaaacgtga aagcaagtct cttaaaaaag aagcactatt ggaggccaaa 240
gaagaagcca gaaaataccg tgaagaagtg gacgctgaat tcaaatcaga acgtcaagaa 300
ctcaaacaaa tcgaaagtcg tttgacagag agagctacta gccttgaccg taaggacgac 360
aatttgacga gtaaagaaca aacacttgaa caaaaagaac aaagtatttc tgatagagcg 420
aaaaaccttg atgcgcgtga agagcaatta gaggaagtcg aaagacaaaa agaagcagaa 480
ctagagcgta ttggtgcgct gtctcaggca gaagcacgag atattatctt ggctcagaca 540
gaggaaaact tgaccaggga gattgccagt cgcattcgcg aagctgagca agaggtcaag 600
gaacgttctg acaaaatggc caaggacatc ttggttcaag ctatgcaacg tatcgctggt 660
gaatatgtag cggagtcaac aaactcaaca gttcatctgc cagacgatac tatgaaggga 720
cgcattattg gtcgtgaagg tcgtaacatt cgtacctttg aaagtttgac aggggtcgat 780
gtgattatcg acgatacacc agaagtggtg accttgtcag gatttgatcc gattcgtcgt 840
gagattgccc gtatgactat ggaaatgttg ctcaaagatg gtcgtataca tccagctcgt 900
atcgaagagt tggttgagaa aaaccgtcaa gagattgaca ataagattcg tgaatacggt 960
gaggctgctg cctatgaaat tggtgcgcca aaccttcatc cagacttgat gaagattatg 1020
ggacgtttgc agttccgtac ttcatatgga caaaatgttt tgcgccattc gattgaggtt 1080
gctaagttgg ctggtatcat ggcgagcgaa cttggtgaaa atgcggctct tgcccgtcgt 1140
gctggattcc ttcacgatat cgggaaagcc attgaccatg aggttgaagg tagccacgtt 1200
gaaatcggta tggaattggc ccgtaagtac aaggaacccc cagttgtggt gaatacgatt 1260
gctagtcacc acggagatgt tgaagctgag agcgtgatag cagttatcgt cgctgcagca 1320
gatgccttga gcgcagcccg tccaggtgct cgtagtgagt ctcttgaaag ctacatcaag 1380
cgtctccatg atttggaaga aattgctaac ggctttgaag gagtgcaaac tagctttgcc 1440
cttcaagcag gacgtgaaat tcgtatcatg gtcaatccag gaaaaatcaa ggacgacaaa 1500
gtcacaatct tggctcacaa agttcgtaag aaaattgaaa acaatctcga ttatccagga 1560
aatatcaagg taaccgtgat tcgcgagctt cgtgcagtag attatgctaa ataa 1614

<210> 278
<211> 315
<212> DNA
<213> Streptococcus pneumoniae

<400> 278
atgatgttaa aaccctctat tgataccttg ctcgacaagg ttccttcaaa atattcactc 60
gtaatcttgg aagcaaaacg tgcccacgaa ttggaagcag gtgccccagc aactcaaggt 120
ttcaagtctg aaaaatcaac tcttcgcgct ttagaagaaa tcgaatcagg aaacgttaca 180
attcacccag atccagaagg aaaacgtgaa gcagtgcgtc gccgtatcga agaagaaaaa 240
cgccgcaaag aagaagaaga aaagaaaatc aaagagcaaa ttgctaaaga aaaagaagat 300
ggtgaaaaaa tttaa 315

<210> 279
<211> 792
<212> DNA
<213> Streptococcus pneumoniae

<400> 279
atgtcagcat atcaattacc gaccgtatgg caggatgaag ctagtaatca aggagctttt 60
acggggctaa acagaccaac agcaggtgcc cgtttcgaac aaaacttgcc aaaaggagaa 120
caagcttttc agctttattc actgggaaca ccaaatggtg tgaaggttac tatcttattg 180
gaagaattac tagaagctgg ttttaaggaa gcggcttacg acttgtataa gattgctatc 240
atggatgggg atcaattcgg atcagacttt gtgaagctca atccaaattc caagattcca 300
gccttattgg accagtcagg tactgaaaac gtaagagtct ttgagtctgc tcatattctt 360
ctttaccttg ctgagaaatt tggagccttt ttaccaagta atcctgtgga aaaggtagaa 420
gttttgaatt ggctattctg gcaagcaggt gcagcacctt ttctaggtgg gggatttgga 480
catttcttca attatgctcc tgaaaaattg gaatatccta ttaaccgttt tacgatggaa 540
gtgaaacgcc agttggattt attggataag gaattggctc agaaacctta tattgcaggc 600
aatgactata cgattgcaga tattgctatc tggtcttggt atggacagtt agttcaagga 660
aatctttacc aaggttctgc aaaattcttg gatgcctcaa gttatcaaaa tctagtaaaa 720
tgggcagaaa aaattgccaa tcgtccagct gttaagcgtg gcttggaagt aacttataca 780
gaaattaaat ag 792

<210> 280
<211> 939
<212> DNA
<213> Streptococcus pneumoniae

<400> 280
ttggcaagct tgatcacttc tatcatcatg ttctatgtcg gtttcgatgt tctaagagat 60
accattcaaa agattctcag tcgggaagaa acggtcattg atcctcttgg tgcaactcta 120
ggaatcattt ctgcagcgat tatgtttgtg gtctatctct acaatactcg cctcagtaag 180
aaatccaact ccaatgcgct gaaggcagct gctaaggaca atctttctga cgctgttacc 240
tcacttggaa ccgccattgc catcctagct agtagtttca attatccgat tgtggataaa 300
ctggttgcta tcatcatcac tttctttatc ttgaagactg cctatgatat cttcatcgag 360
tcttccttta gtctttcaga tggctttgac gaccgcctgc tcgaggacta ccaaaaggct 420
atcatggaaa ttcccaaaat cagcaaggtc aaatcgcaaa gaggtcgcac ctacggtagc 480
aacatctacc tggatattac actagagatg aatcctgact tgtctgtttt tgaaagccat 540
gaaatcgcgg atcaggtcga gtctatgctg gaggagcgtt ttggcgtctt tgataccgat 600
gtccatatcg aaccagcacc tatccctgag gatgaaattt tagacaatgt ctataaaaaa 660
ttgcttatgc gtgaacaatt gattgaccaa ggaaaccaac tagaagaact cttgactgat 720
gattttgtct atattcgcca agatggagag cagatggata aagaggctta taagaccaaa 780
aaagagttaa attctgctat caaggacatt caaattactt ccatcagtca aaaaaccaaa 840
ctcatctgct atgagttaga tggtatcatc cataccagta tctggcgtcg ccacgaaacc 900
tggcaaaata tctttcatca agaaaccaaa aaagaatag 939

<210> 281
<211> 795
<212> DNA
<213> Streptococcus pneumoniae

<400> 281
atgacaatta aactagtagc aacggatatg gacggaacct tcctagatgg gaatggacgc 60
tttgatatgg atcgtctcaa gtctctcttg gtttcctaca aggaaaaagg gatttacttt 120
gcggtagctt cgggtcgggg atttctgtct ctagaaaaat tatttgctgg tgttcgtgat 180
gacattattt tcatcgcgga aaatggcagt ttggtagagt atcaaggtca ggacttgtat 240
gaagcgacta tgtctcgtga cttttatctg gcaacttttg aaaagctgaa aacttcacct 300
tatgtagata tcaataaact gctcttgacg ggtaagaagg gttcatatgt tctagatacg 360
gttgatgaga cctatttgaa agtgagtcag cactataatg aaaatatcca aaaagtagcg 420

agtttggaag atatcacaga tgacattttc aaatttacaa ccaacttcac agaagaaacg 480
ctggaagatg gggaggcttg ggtaaacgaa aacgttcctg gtgttaaggc catgacaact 540
ggctttgaat ccattgatat tgttctggac tatgtcgata agggagtggc cattgttgaa 600
ttagttaaaa aacttggtat cacaatggat caggtcatgg cttttggaga caatcttaat 660
gacttacata tgatgcaggt tgtgggacat cctgtagctc ctgaaaatgc acgacctgaa 720
attttagaat tagcaaagac tgtgattggt caccataagg aacggtcggt tatagcttat 780
atggagggct tataa 795

<210> 282
<211> 819
<212> DNA
<213> Streptococcus pneumoniae

<400> 282
atggcagata taaaattgat tgcattggac ttggacggga ccttgctgac tactgataaa 60
aggctgacgg atcgtaccaa ggaaaccttg caagctgcgc gtgatcgtgg tatcaaggtc 120
gtattgacaa ctggtcgtcc cttaaaagcc atggatttct ttctccatga gttagggact 180
gacggtcagg aagatgagta taccattact tttaatggtg gattagttca gaaaaataca 240
ggagaaatcc ttgataaaac agtcttttca tatgatgatg tggcacgttt gtatgaagaa 300
acagagaaat tatcactgcc tcttgatgcc atctcagaag gaacagttta tcaaatccaa 360
tcggaccaag aaagtcttta tgccaaattc aatccagctt tgacctttgt tccagtggac 420
tttgaagact tatctagtca aatgacctac aacaaatgcg tgactgcctt tgctcaagaa 480
cccttggatg cagccattca gaagatttct ccagaattgt ttgaccaata tgaaatcttt 540
aaatcacgtg aaatgttgct agaatggtca ccaaagaatg ttcataaagc aacaggtttg 600
gcaaaactaa tcagccatct tggaatcgac caaagtcaag tgatggcttg tggtgacgag 660
gccaatgacc tctctatgat tgaatgggca ggtcttggtg ttgctatgca aaacgctgtt 720
cctgaagtaa aggcagccgc aaatgtagtg acgccgatga ccaacgatga ggaagctgtc 780
gcctgggcta tcgaagaata tgtgctaaag gagaactaa 819

<210> 283
<211> 1257
<212> DNA
<213> Streptococcus pneumoniae

<400> 283
atggaaagtt tacttattct attattaatt gccaatctag ctggtctctt tctgatttgg 60
caaaggcagg ataggcagga gaaacactta agtaagagct tggaggatca ggcagatcat 120
ttgtcagacc agttggatta ccgctttgac caagccagac aagccagcca gttagaccaa 180
aaagatttgg aagtggttgt cagcgaccgt ttgcaagaag tgcggattga attgcaccaa 240
ggtctgaccc aagtccgtca agaaatgaca gataatctcc tccaaactag agacaagaca 300
gaccaacgtc tccaagcctt gcaggaatca aatgagcaac gtttggaaca aatgcgccag 360
acggtcgagg aaaaactaga aaagaccttg cagacacgct tacaggcttc ctttgagaca 420
gtttctaaac aactggagtc tgtcaatcgt ggccttggag aaatgcagac agttgcccgt 480
gatgtcggag ctcttaacaa ggttctctct ggaaccaaga cgcgagggat tctgggagaa 540
ttgcaactgg ggcaaattat tgaagacatc atgacacctg cccagtacga acgagaatac 600
gcaacggttg aaaactctag tgaacgagtg gagtatgcca tcaagttacc cggacaaggc 660
gaccaagaat acgtctatct gccaattgac tctaagtttc cactggcaga ttattaccgc 720
ttggaagaag cctatgagac aggtgacaag gatgagattg aacgctgtcg taagtcactc 780
ctagcaagcg tcaagcgctt tgctagggat attaggaaca agtacatagc accacctcgg 840
acgaccaatt ttggagtttt gtttgttccg acagaaggtc tctactcaga aatcgtccgc 900
aatccggtct tctttgatga tttgagacgg gaagaacaga ttattgttgc aggaccaagt 960
accctatcag cccttcttaa ctccctatca gttggtttca agacccttaa tatccaaaag 1020
agtgccgacc atatcagcaa gactcttgcc agtgtcaaga ccgagtttgg caagtttggt 1080
ggtattctgg tcaaggcaca aaaacatctc caacatgcct ctggcaatat tgatgaatta 1140
ttaaaccgtc gtaccatagc tatcgagcgg acgctccgtc acattgagtt gtcagaaggt 1200
gagcctgcgc ttgatctact ccattttcaa gaaaatgagg aagaatatga agattag 1257

<210> 284

<211> 942
<212> DNA
<213> Streptococcus pneumoniae

<400> 284
atgaagatta gtcacatgaa aaaagatgag ttatttgaag gcttttacct aatcaaatca 60
gctgacctga ggcaaactcg agctgggaaa aactacctag cctttacctt ccaagatgat 120
agtggcgaga ttgatgggaa gctctgggat gcccaacctc ataacattga ggcctttacc 180
gcaggtaagg ttgtccacat gaaaggacgc cgagaagttt ataacaatac ccctcaagtc 240
aatcaaatta ctctccgcct gcctcaagct ggtgaaccca atgacccagc tgatttcaag 300
gtcaagtcac cagttgatgt caaggaaatt cgtgactaca tgtcgcaaat gattttcaaa 360
attgaaaatc ctgtctggca acggattgtc cgaaatctct acaccaagta tgataaggaa 420
ttctactcct atccagctgc caagaccaac caccatgcct ttgaaacggg cttggcctat 480
catacggcga ccatggtgcg tttggcagac gctattagcg aagtttatcc tcagctcaat 540
aagagcctgc tctatgcggg gattatgttg catgacttag ctaaggtcat cgagttgacg 600
gggccagacc agacagagta cacagtgcga ggtaatcttc ttggacatat cgctctcatt 660
gatagcgaaa ttaccaagac agttatggaa ctcggcatcg atgataccaa ggaagaagtc 720
gttttgcttc gtcatgtcat cctcagtcac cacggcttgc ttgagtatgg aagcccagtc 780
cgtccacgca ttatggaagc agagattatc catatgattg acaatctgga tgcaagcatg 840
atgatgatgt caacagctct tgctttggtg gataaaggag agatgaccaa taaaatcttc 900
gctatggata atcgttcctt ctataaacca gatttagatt aa 942

<210> 285
<211> 1512
<212> DNA
<213> Streptococcus pneumoniae

<400> 285
atgagtgaaa aagctaaaaa agggtttaag atgccttcat cttacaccgt attattgata 60
atcattgcta ttatggcagt gctaacttgg tttatccctg cgggggcctt tatagaaggt 120
atttacgaga ctcagcctca aaatccacaa gggatttggg atgtcctcat ggcaccgatt 180
cgggctatgc taggtactca tccagaggaa ggttcgctca ttaaagaaac gagcgcagcg 240
attgatgtag ccttcttcat ccttatggtt ggtggtttcc ttggcattgt caacaaaact 300
ggtgctcttg acgtagggat tgcctctatc gtgaagaagt ataagggccg cgaaaaaatg 360
ttaattttgg tactgatgcc tttgtttgcc ctcggtggta caacttatgg tatgggtgaa 420
gaaacaatgg ccttctatcc actccttgtg ccagttatga tggccgttgg ttttgatagc 480
ctgactggtg ttgcaattat tttgctcggt tctcaaatcg gctgtttggc atctactctg 540
aatccatttg cgacaggtat tgcttcagcg actgcgggag ttggtacagg ggacggtatc 600
gtacttcgtc tgatcttctg ggttaccttg actgctctta gtacttggtt tgtttaccgt 660
tatgcggata agattcaaaa agatccgact aagtcactgg tttatagtac tcgcaaagaa 720
gatttgaaac actttaacgt agaagaatct tcatctgtag aatctacact tagcagcaaa 780
caaaaatcag ttctcttctt atttgtgttg acattcatct tgatggtatt gagcttcatt 840
ccatggacag accttggcgt taccattttt gatgacttta atacttggtt gactggtctt 900
ccagttattg gtaatattgt cggttcatct acttctgcac taggtacttg gtacttccca 960
gaaggcgcaa tgctctttgc ctttatgggt atcctgattg gtgttattta tggtcttaaa 1020
gaagataaga ttatctcttc cttcatgaat ggtgctgctg acttgctcag tgttgccttg 1080
atcgtagcga ttgctcgtgg tattcaagtt atcatgaacg acggtatgat taccgataca 1140
atcctcaact ggggtaaaga aggcttgagc ggtctatctt cacaagtctt tatcgttgta 1200
acttatatct tctatctacc tatgtcattc ttgatcccat cttcatctgg tcttgccagc 1260
gcaactatgg gtatcatggc tccacttgga gaatttgtaa atgtccgtcc tagcttgatt 1320
atcactgctt accaatctgc ttcaggtgtc ttgaacttga ttgcaccaac atctggtatt 1380
gtgatgggag ctcttgcact tggacgtatc aacattggta cttggtggaa attcatgggc 1440
aaactcgtag tcgctattat tgtagtgacc atcgcccttc ttctccttgg aaccttcctt 1500
ccattcctat aa 1512

<210> 286
<211> 1332
<212> DNA
<213> Streptococcus pneumoniae

<400> 286
atgaaaatag atataacaaa tcaagttaaa gatgaatttc ttatatcatt aaaaaccttg 60
atttcctatc cttcagtact caatgaagga gaaaatggaa caccttttgg acaagcaatc 120
caagatgtcc tagaaaaaac tttagagatt tgtcgagaca taggtttcac tacctatctt 180
gaccctaaag gttattacgg atatgcagaa atcggtcagg gagcagagct tctggccatt 240
ctctgtcatt tggatgttgt tccatcaggt gatgaagcag attggcagac accgccattt 300
gaagcaacta tcaaagacgg ctgggtattc ggacgtggtg tccaagatga taaaggccct 360
tcgctcgcag ctctctatgc agtaaaaagc ttgctggacc aaggtattca gttcaaaaag 420
cgcgtacgct ttatctttgg taccgatgag gaaaccctct ggcgctgcat ggcacgctac 480
aataccatcg aagaacaggc cagtatgggc tttgcacctg actcatcttt tcctctgacc 540
tatgctgaaa aagggcttct acaggtcaaa cttcatggcc ctggatcgga tcaactagag 600
cttgaagtag gaggcgcctt taacgttgta ccagacaagg ccaactacca aggtctcctc 660
tatgaacagg tttgtaacgg tctcaaagaa gctggttatg attaccaaac cactgaacaa 720
accgtaacgg ttctcggagt gccaaagcat gctaaggatg ctagtcaagg tatcaatgct 780
gtcatccgac tagctaccat tcttgctcct ctccaagaac accctgctct cagttttctt 840
gcaacacaag caggtcaaga cggcacagga agacaaatct ttggtgatat agcagatgaa 900
ccttctggtc acctatcctt taatgtcgca ggtctcatga tcaatcatga acgttctgaa 960
atccgtattg acattcggac tcctgtctta gctgacaagg aagaactagt agagttgctt 1020
acaagatgtg cacaaaacta ccaactccgc tacgaagagt ttgactatct agcgcctcta 1080
tacgtcgcag aagacagtaa actcgttagc acactgatgc aaatctacca agaaaagact 1140
ggcgataaca gtcctgctat ttcatccggt ggtgccactt ttgctcgcac catgccaaat 1200
tgtgtagcct tcggcgcctt attcccagga gcgaagcaga cagaacatca ggcaaatgaa 1260
tgtgccgttc tagaagattt gtaccgtgct atggatattt atgccgaagc cgtctatcga 1320
cttgcaactt aa 1332

<210> 287
<211> 2349
<212> DNA
<213> Streptococcus pneumoniae

<400> 287
atgtctaatt catttgtcaa gttgttagtc tctcaattat ttgcaaattt agcagatatt 60
ttctttagag taacaatcat tgctaacata tacattattt caaaatcagt aattgccaca 120
tcactagttc ctatcttaat aggaatatcc tcttttgttg cgagtctttt agttccgttg 180
gttactaaaa ggttagcgct aaatagggtt ttatctttat ctcaatttgg aaagactata 240
ttattggcga tactggtagg aatgtttacc gtaatgcaat ccgtagcgcc tttggtgacc 300
tatctatttg ttgttgcaat ttccatacta gatggttttg cagcacccgt ttcctatgct 360
attgtgccac gctatgcgac cgatttgggt aaggctaatt cagccttatc aatgactggt 420
gaagctgttc aattgatagg ttggggatta ggtggactct tgtttgcaac aattggtctg 480
ttacctacca cgtgtatcaa tttagtcttg tatatcattt ctagctttct gatgttattt 540
cttcctaacg ctgaagtgga ggtgttagag tcagaaacta atcttgaaat tttgctcaaa 600
ggttggaagt tagttgctag aaatcctaga ttaagacttt ttgtatcagc aaatttattg 660
gaaatttttt caaatacgat ttgggtttct tccattatac ttgtttttgt aacggagtta 720
ttaaataaaa cggaaagtta ctggggatat tctaatacag catactctat tggtattata 780
attagtggct taattgcttt taggctatct gaaaagttcc ttgctgctaa atgggaaccc 840
caattattca ccccaaatct aaaaaccatc cagaatcctt gccttagctt agatcctgga 900
tggtttcttt tttcacccaa tgggtgtttt ttactagaca aaaaagagtt tcccctttat 960
ggtataagtg tagaaaaaaa cacaaaaaga aaggaaactc acatgaacag tttaccaaat 1020
catcacttcc aaaacaagtc tttttaccaa ctatctttcg atggaggtca tttaacccag 1080
tatggtggtc ttatcttttt tcaggaactt ttttcccagt tgaaactaaa agagcggatt 1140
tctaagtatt tagtaacgaa tgaccaacgc cgctactgtc gttattcgga ttcagatatc 1200
cttgtccagt tcctctttca actgttaaca ggttatggaa cggactatgc ttgtaaagaa 1260
ttgtcagctg atgcctactt tccaaaattg ttggaaggag ggcagcttgc ttcacagcca 1320
accttatccc gttttctttc cagaactgac gaggaaacag tccatagttt gcgatgcctc 1380
aaccttgaat tggtcgaatt ctttttacag tttcaccagc taaaccaact cattgtagat 1440
atcgattcta cccatttcac aacttatggc aagcaagaag gtgttgctta taacgcccac 1500
tatcgtgctc atggctatca tcctctttat gctttcgagg ggaagacagg ttattgtttc 1560
aatgcccagc ttcgtcctgg taatcgttat tgttctgaag aggcagacag ctttatcaca 1620
cctgttttag aacggtttaa tcaacttctc tttcgaatgg atagtggctt tgcgacccca 1680

aaattatacg atttaattga aaaaacaggg caatactacc tcataaaact caagaaaaat 1740
actgttctga gccgtcttgg agacctttcc ctcccttgcc cacaggatga ggacttaacc 1800
atcttgcccc actccgccta ctcagaaact ctctatcaag caggatcttg gtcgcacaag 1860
cgtcgtgtct gccagttctc tgaacgaaaa gaaggaaact tgttctacga tgttatttct 1920
ctcgttacaa atatgacgag tggaacaagc caagaccagt ttcagcttta tcgtggacgt 1980
ggtcaagccg agaatttcat caaggagatg aaggagggat tttttggcga taaaacggat 2040
agttcaacct taatcaaaaa cgaagttcgt atgatgatga gctgtatcgc ctacaatctc 2100
tatctttttc tcaaacatct agctggaggt gacttccaaa ctttaacaat caaacgcttc 2160
cgccatcttt ttcttcacgt ggtgggaaaa tgtgttcgaa caggacgcaa gcagctcctc 2220
aaattgtcta gtctctatgc ctattccgaa ttgttttcag cactttattc taggattaga 2280
aaagtcaacc tgaatcttcc tgttccttat gaaccaccta gaagaaaagc gtcgttaatg 2340
atgcattaa 2349

<210> 288
<211> 1245
<212> DNA
<213> Streptococcus pneumoniae

<400> 288
atgatggagt tttttcaaca gcttcctcat ttagagccat atggcaatcc tcagtatttt 60
gtttatgtga ttgctgcaac cttgcccatc tttataggtc tctttttcaa gaaacgcttt 120
gcctggtatg aagtgttggt aagtctcttc tttattgtca ccatgttggt gggtggaaag 180
accaatcaac tagctgcctt gggtatttac ctttgctggg aaatattgct cctgcttttc 240
tacaagcatt atcgaaaaag caaggatggc aagtgggtct tctacttagt tagttttctg 300
tccctacttc cgattatctt tgtcaaggtg caaccagcta tcaatggaac gcagtctttg 360
cttgggttct tgggaatttc ttacctgacc tttcgttcgg ttggaattgt catcgagctg 420
agagatggag tgattaagga ttttaccctc tgggaattcc tccgtttcct tctcttcatg 480
ccaactttct cgagtggtcc aatcgatcgc tttaagcgat ttaatgaaaa ttatcaggct 540
attcctgagc gagatgagtt gatggatatg ctggatgaat ctgtccgcta tatcatgtgg 600
ggctttttgt ataagtttat cctagctcat gttttaggag agaccttact acctcctctg 660
aagaatttag ccttgcagtc aggtggcttc tttaatctct atgccttggc agttatgtat 720
acttttggtc tggaactctt ctttgacttt gcaggttatt ctatgtttgc tttggccatc 780
tcaaacttga tgggaatccg tagccctatc aactttaaca agcccttttt atcaagggat 840
ttaaaggagt tttggaatcg ctggcatatg agtctgtcct tctggttccg tgactttgtc 900
tttatgcgaa tggtgatggt gttaaccaga aagaaagtct ttaaaaatcg taatgtaacc 960
tcaagcatgg cctacattgt aaatatgctg attatgggat tttggcatgg tgtgacctgg 1020
tactatatcg cctatggact ctttcatgga ctaggcttgg tcatcaatga tgcctgggtt 1080
cgcaagaaaa aaacgctcaa taaggaacgg aaaaaagcag ggaaggctgc cctacctgag 1140
aatcgctgga ttcagttgct tggcatggtt gtcactttcc atgttgtcat gttgtcattc 1200
ttaatctttt ctggattctt gaataatcta tggtttaaaa aataa 1245

<210> 289
<211> 1269
<212> DNA
<213> Streptococcus pneumoniae

<400> 289
atgcttaaac gcttatggat gatcttcgga ccggtcttga tcgctggttt gttggttttt 60
ctgctcattt tcttttatcc tactgagatg catcataatc taggagctga aaagcgttca 120
gcagtggcta ctactatcga tagttttaag gagcgaagtc aaaaagtcag agcactatct 180
gatccaaatg tgcgttttgt tcccttcttt ggctctagtg aatggcttcg ttttgacggt 240
gctcatcctg cggtattagc tgagaaatac aatcgttcct accgtcctta tcttttagga 300
caggggggag ctgcatcgct taaccaatat tttggaatgc aacagatgtt accacagctg 360
gagaataaac aagttgtgta tgttatctca cctcagtggt tcagtaaaaa tggctatgat 420
ccagcagcct tccagcagta ttttaatgga gaccagttga ctagttttct gaaacatcaa 480
tctggggatc aggctagtca atatgcagcg actcgcttac tgcaacagtt cccaaacgta 540
gctatgaagg acctggttca gaagttggca agtaaagaag aattgtcgac agcagacaat 600
gaaatgattg aattattggc tcgttttaat gaacgccaag cttccttttt tggtcagttt 660
tcggttagag gctatgttaa ctacgataag catgtagcta agtatttaaa aatcttgcca 720

gaccagtttt cttatcaggc aatagaagat gttgtcaaag cagatgctga aaaaaatact 780
tccaataatg agatgggaat ggaaaattat ttctataatg agcagatcaa gaaggatttg 840
aagaaattaa aggattctca gaaaagcttt acctatctca agtcgccaga gtataatgac 900
ttgcagttgg ttttaacaca gttttctaaa tctaaggtaa acccgatttt tatcattcca 960
cctgttaata aaaaatggat gaactatgct ggtctacgag aggatatgta ccaacaaacg 1020
gtgcagaaga ttcgctacca gttagaaagt caaggtttta ccaatatagc agatttttct 1080
aaggacggcg gggagccttt ctttatgaag gacaccattc accttggttg gttgggttgg 1140
ttggcttttg acaaggcagt tgatcctttc ctatccaatc ccacaccagc tccgacttac 1200
catctgaatg agcgcttttt cagcaaagat tgggcgactt atgatggaga tgtcaaagaa 1260
tttcaatag 1269

<210> 290
<211> 621
<212> DNA
<213> Streptococcus pneumoniae

<400> 290
atggagaaaa acctcaaggc tttgaaacaa acaacagacc aagaaggccc agcaattgaa 60
cctgaaaagg cagaggatac caagacagtc caaaatggtt acttcgagga tgcagctgtc 120
aaggaccgca ccttgagtga ctatgcaggt aactggcaat cagtttatcc tttccttgaa 180
gacggcacgt ttgaccaagt ctttgactac aaggctaagt tgactggtaa gatgacccag 240
gctgagtaca aggcttacta tacaaaaggc tatcatacag atgtgactaa gattaacatt 300
actgataata ctatggaatt tgttcaaggt ggacaaagca agaaatacac ttacaagtat 360
gtcggtaaga aaattttgac ttacaagaaa ggcaatcgtg gcgtgcgttt cctctttgaa 420
gccacagatg ctgacgctgg acaattcaag tatgttcagt ttagtgacca caatgttgcc 480
ccagttaagg cagaacattt ccatatcttc tttggaggca caagccaaga agccctcttt 540
gaagaaatgg acaactggcc aacctactac ccagataacc tatctggcca agaaatcgcc 600
caagaaatgt tggcgcattg a 621

<210> 291
<211> 444
<212> DNA
<213> Streptococcus pneumoniae

<400> 291
atgaaagatg gtcatttgct agcccatcat attcgtttgt tgaatgggcg gatttttcaa 60
aagttactga gtcaagatcc tgaggctctt tataggggtg aacagggcaa gattttagcg 120
gttttatgga atagtgaaac tggctgcgca actgcgacag atatcgcgct tgcgactgga 180
cttgcgaata atacgctgac gactatgata aaaaagctag aggaacaaaa gcttgtaatt 240
gttagtccgt gtggaaaaga caagcgtaag aagtatttag ttttaacgga gttaggcaag 300
tcccagaaag aagtggggca tcgtgtcagt cagaaattgg atactatctt ttacaaagga 360
ttttcagagg aagaaattca ccaatttgaa ggttttcaag aaagaatttt ggcgaatctg 420
aaagagaagg gaaatgaggt ttag 444

<210> 292
<211> 1521
<212> DNA
<213> Streptococcus pneumoniae

<400> 292
atgactaatt taattgcaac ttttcaggat cgttttagtg attggttgac agctctatct 60
caacatttgc agttgtcgct tttgaccttg ttactagcta ttttgcttgc gattcccttg 120
gctgtttttc ttcgctatca tgagaagctg gccgactggg tcttgcagat tgcaggtatt 180
ttccagacca tcccgtctct ggccttgttg ggctctttta tccctttgat gggaattggg 240
accttgccgg ctttgacagc tctagtgatt tatgcgattt tccctatttt gcaaaatact 300
atcactgggc tgaagggaat tgatccgaac ctgcaagagg ctgggattgc ctttggcatg 360
accagatggg aacgtctcaa gaaatttgaa attccactcg ccatgcctgt tatcatgtct 420
gggattcgga cggcagctgt tttgattatc ggtacggcaa ccttggcggc cttgattggt 480

gcagggggac taggttcctt tattcttttg ggaattgacc gtaataatgc cagtttgatt 540
ttgattgggg cactttcttc tgcagtgcta gccattgcct ttaacttcct actaaaagtg 600
atggaaaaag caaaattacg gacgattttc tcaggttttg ccttggtggc tttattactg 660
ggtctgtctt atagtccagc tcttttggtt caaaaagaga aggaaaactt ggttattgct 720
gggaaaatag gtccagaacc agaaattttg gccaatatgt ataagttgct gattgaagaa 780
aataccagca tgactgcgac tgttaaaccg aattttggga agacaagctt cctttatgaa 840
gctctgaaaa aaggcgatat tgacatctat cctgaattta ctggtacggt gactgaaagt 900
ttgcttcaac catcacccaa ggtgagtcat gaaccagaac aggtttatca ggtggcgcgt 960
gatggcattg ctaagcagga tcatctagcc tatctcaaac ccatgtctta tcaaaacacc 1020
tatgctgtag ctgttccgaa aaagattgct caagaatatg gcttgaagac catttcagac 1080
ttgaaaaaag tggaaggcca gttgaaggca ggttttacac tcgagtttaa cgaccgtgaa 1140
gatggaaata agggcttgca atcaatgtat ggtctcaatc tcaatgtagc gaccattgag 1200
ccagcccttc gctatcaggc tattcagtca ggggatattc aaatcacgga tgcctattcg 1260
actgatgcgg aattggagcg ttatgattta caggtcttgg aagatgacaa gcaactcttc 1320
ccaccttatc aaggggctcc actcatgaaa gaagctcttc tcaagaaaca cccagagttg 1380
gaaagagttc ttaatacatt ggctggtaag attacagaaa gccagatgag ccagctcaac 1440
taccaagtcg gtgttgaagg caagtcagca aagcaagtag ccaaggagtt tctccaagaa 1500
caaggtttgt tgaagaaatg a 1521

<210> 293
<211> 810
<212> DNA
<213> Streptococcus pneumoniae

<400> 293
atgatgcata cttatttgca aaagaaaatt gaaaatatca aaacaaccct aggtgaaatg 60
tcaggtggtt accgtcgtat ggttgcggct atggctgatt taggattttc aggaactatg 120
aaggctatct gggatgacct ctttgcccat cgtagttttg cccagtggat ttatttgctg 180
gttttaggaa gttttcctct ctggctggag ttggtttacg aacatcgtat tgttgactgg 240
attgggatga tttgtagctt gacagggatt atctgtgtaa tctttgtatc ggaaggtcga 300
gcaagtaatt atctttttgg cttgattaac tctgttattt accttatttt ggccctacag 360
aaaggctttt atggtgaggt gctgacgaca ctttacttca cagtcatgca gccaattgga 420
cttctagttt ggatttatca ggcacagttt aagaaggaaa agcaggagtt tgtcgcgcgt 480
aaactggacg gcaagggctg gacaaagtat ctttccatta gtgtgctttg gtggttggcc 540
tttggcttca tttatcagtc tattggtgcc aatcgtccct atcgtgattc aatcacagat 600
gcaaccaatg gggtagggca aatcctcatg acagctgttt accgtgaaca gtggatattc 660
tgggcggcta ccaatgtctt ttcaatctat ctctggtggg gagaaagcct gcaaattcaa 720
gggaaatatc taatttatct cattaacagt ctagttggtt ggtatcaatg gagcaaggca 780
gctaagcaga atactgattt acttaactag 810

<210> 294
<211> 900
<212> DNA
<213> Streptococcus pneumoniae

<400> 294
atgagaaata tgaaggcaaa atatgctgtt tgggtggctt ttttcttaaa tttgacttat 60
gccattgttg agtttattgc aggtggagta tttggttcta gcgctgttct tgctgactct 120
gtgcatgact tgggagatgc gattgcaatt ggaatatcag cttttctaga aacaatctcc 180
aatcgtgaag aagacaatca gtacaccttg ggctataagc ggtttagcct gctaggagcc 240
ttggtaacag ctgtgattct cgtaacgggc tctgttctag tcattttgga aaatgtcacg 300
aagattttgc atccgcaacc agtcaatgat gaggggattc tctggttagg aattattgcg 360
attactatca atctgttagc gagtctggtg gttggtaagg gaaagacaaa gaatgagtct 420
attctgagtc tgcattttct ggaagatacg ctagggtggg tagctgttat cctgatggcg 480
attgttcttc gatttacgga ctggtatatc ctagatcctc ttttgtccct tgtcatttct 540
ttctttattc tttcaaaagc ccttccacgt ttttggtcta cactcaagat tttcttggat 600
gctgtgccag aaggtcttga tatcaagcaa gtaaagagtg gcctggagcg attggacaat 660
gtggccagcc ttaatcagct taatctctgg actatggatg ctttggaaaa aaatgccatt 720
gtccatgttt gtctaaaaga aatggaacat atggaaactt gtaaagagtc tattcgaatt 780

ttcctaaaag attgtggttt tcaaaatatt accattgaaa ttgatgctga cctagaaact 840
caccaaaccc ataagcgaaa ggtgtgtgac ttggaacgga gttatgagca tcaacattag 900

<210> 295
<211> 729
<212> DNA
<213> Streptococcus pneumoniae

<400> 295
atgattgaat acaaaaatgt agcactgcgc tacacagaaa aggatgtctt gagagatgtc 60
aacttacaga ttgaggatgg ggaatttatg gttttagtag ggccttctgg gtcaggtaag 120
acgaccatgc tcaagatgat taaccgtctt ttggaaccaa ctgatggaaa tatttatatg 180
gatgggaagc gcatcaaaga ctatgatgag cgtgaacttc gtctttctac tggttatgtt 240
ttacaggcta ttgctctttt tccaaatcta acagttgcgg aaaatattgc tctcattcct 300
gaaatgaagg ggtggagcaa ggaagaaatt acgaagaaaa cagaagagct tttggctaag 360
gttggtttac cagtagccga gtatgggcat cgcttaccta gtgaattatc tggtggagaa 420
cagcaacggg tcggtattgt ccgagctatg attggtcagc ccaagatttt cctcatggat 480
gaaccctttt cggccttgga tgctatttcg agaaaacagt tgcaggttct gacaaaagaa 540
ttgcataaag agtttgggat gacaacgatt tttgtaaccc atgatacgga tgaagccttg 600
aagttggcgg accgtattgc tgtcttgcag gatggagaaa ttcgccaggt agcgaatccc 660
gagacaattt taaaagcgcc tgcaacagac tttgtagcag acttgtttgg aggtagtgtt 720
catgactaa 729

<210> 296
<211> 1083
<212> DNA
<213> Streptococcus pneumoniae

<400> 296
atgtcagcag ttgctatttc agctatgacc aaggttatgc aagaaaccca cggaaatcct 60
tctagtattc atggtcatgg tcgtcaagct ggcaaactct tgcgagaagc ccgtcaggaa 120
ctagcccagt tactaaggac aaaacctcaa catatctttt tcacttctgg tgggactgaa 180
ggcaataata ctaccatcat tggctactgt cttcgtcacc aagaacaagg aaaacatatc 240
atcacaactg ccatcgagca ccatgctgtc cttgaaacaa ttgattactt ggttcaacac 300
tttgggtttg aagcaaccat tatccagcca gaaaatcaag aaatcacagc ccagcaaatt 360
caaaaggctt tacgtgacga tacgattttg gtttctacca tgtttgtcaa taatgagaca 420
ggaaacctac tgcccatcgc tgaaattggc caaatactca agcaacaccc tgctgcctat 480
catgttgatg cagttcaggc tattggtaaa atcccaattc attcagaaga attgggcatt 540
gatttctcca ctgcttctgc ccacaaattc catggtccta agggaatcgg ttttctctac 600
gcatctagca tggactttga ttcctatcta catggcggag accaggaaca gaaaaaacgt 660
gcaggaactg aaaatctgcc tgccattgta ggcatggttg cagccctaaa agaagaccta 720
gaaaaacaag aagaacattt tcaacatgta caaaatctag aaactgcctt tctggcagag 780
ctggagggca ttcagtatta cctgaataga ggaaaacatc atctccctta tgttctcaat 840
attggatttc ctggtcagaa aaatgacctc ttactccttc ggctagattt agctggaatt 900
tcaatctcta ctggctcagc ctgtactgca ggcgttgtcc aatccagcca tgttcttgaa 960
gccatgtatg gcgcaaattc agaacgcttg aaggaatccc ttcgcatcag tttgtcgcca 1020
caaaataccg ttgaagacct acaaaccctc gcaaaaacct taaaagaaat tatcggaggt 1080
tag 1083

<210> 297
<211> 1380
<212> DNA
<213> Streptococcus pneumoniae

<400> 297
atgttattca aattatctaa ggaaaaaata gagctaggct tatctcgttt atcgccagcc 60
cgtcgtattt ttttgagttt tgccttggtc atttactagg gctctcttct tttgagcttg 120
ccctttgtcc aagttgaaag ctcacgagcg acttatttgg atcatctttt cactgctgtc 180

tctgcagtct gtgtgacggg tctctcaacc cttccagtag ctcacaccta taatatctgg 240
ggtcaaataa tctgtttgct cttgattcag atcggtggtc tagggctcat gacctttatt 300
ggggtttctt atatccagag caagcaaaag cttagtcttc gtagccgtgc aactattcag 360
gatagtttta gttatggaga aactcgatct ttgagaaagt ttgtctattc tatttttctc 420
acgacctttt tggttgagag cttgggagct attttgctta gttttcgcct tattcctcaa 480
cttggctggg gacgtggtct ttttagttcc atttttctag cgatctcagc cttctgtaat 540
gccggttttg ataatttagg gagcaccagt ttatttgctt ttcagaccga tttactggtc 600
aatctggtga ttgcaggctt gattattaca ggcggccttg gttttatggt ctggtttgat 660
ttggctggtc atgtaggaag aaagaaaaaa ggacgtctgc actttcatac gaagcttgta 720
ctattattga ctataggttt gttgttattt ggaacagcaa ctactctctt tcttgagtgg 780
aacaatgctg gaacgattgg caatctccct gttgccgata aggttttagt tagctttttt 840
caaacagtga cgatgcgaac agctggcttt tctacgatag attatactca ggctcatcct 900
gtgactcttt tgatttatat cttacagatg tttctaggtg gggcacctgg aggaacagct 960
gggggactca agattacgac attttttgtc ctcttggtct ttgcacgaag tgagcttcta 1020
ggcttgcctc atgccaatgt tgcgagacga acgatcgcgc cgcgaacggt tcaaaaatcc 1080
tttagtgtct ttattatctt tttgatgagc ttcttgatag gattgattct gctagggata 1140
acagccaaag gcaatcctcc ctttatccac ctcgtatttg aaaccatttc agctcttagt 1200
acagttggtg taacggcaaa tctgactcct gaccttggga aattggctct cagtgttatc 1260
atgccactta tgtttatggg acgaattggt cccttgacct tgtttgttag cttggcagat 1320
taccatccag aaaagaaaga tatgattcac tatatgaaag cagatattag tattggttaa 1380

<210> 298
<211> 666
<212> DNA
<213> Streptococcus pneumoniae

<400> 298
atgtcagatc gtacgattgg aattttgggc ttgggaattt ttgggagcag tgtcctagct 60
gccctagcca agcaggatat gaatattatc gctattgatg accacgcaga gcgcatcaat 120
cagtttgagc cagttttggc gcgtggagtg attggtgaca tcacagatga agaattattg 180
agatcagcag ggattgatac ctgcgatacc gttgtagtcg cgacaggtga aaatctggag 240
tcgagtgtgc ttgcggttat gcactgtaag agtttggggg taccgactgt tattgctaag 300
gtcaaaagtc agaccgctaa gaaagtgcta gaaaagattg gagctgactc ggttatctcg 360
ccagagtatg aaatggggca gtctctagca cagaccattc ttttccataa tagtgttgat 420
gtctttcagt tggataaaaa tgtgtctatc gtggagatga aaattcctca gtcttgggca 480
ggtcaaagtc tgagtaaatt agacctccgt ggcaaataca atctgaatat tttgggtttc 540
cgagagcagg aaaattcccc attggatgtt gaatttggac cagatgacct cttgaaagca 600
gatacctata ttttggcagt catcaacaac cagtatttgg ataccctagt agcattgaat 660
tcgtaa 666

<210> 299
<211> 540
<212> DNA
<213> Streptococcus pneumoniae

<400> 299
atgaagttat tgtctatcgc aatttctagc tataatgcag cagcctatct tcattactgt 60
gtggagtcgc tagtgattgg tggtgagcaa gttgggattt tgattatcaa tgacgggtct 120
caggatcaga ctcaggaaat cgctgagtgt ttagctagca agtatcctaa tatcgttaga 180
gccatctatc aggaaaataa atgccatggc ggtgcggtca atcgtggctt ggtagaggct 240
tctgggcgct attttaaagt agttgacagt gatgactggg tggatcctcg tgcctacttg 300
aaaattcttg aaaccttgca ggaacttgag agcaaaggtc aagaggtgga tgtctttgtg 360
accaattttg tctatgaaaa ggaagggcag tctcgtaaga agagtatgag ttacgattca 420
gtcttgcctg ttcggcagat ttttggctgg gaccaggtcg gaaatttctc caaaggccag 480
tataccatga tgcactcgct gatttatcgg acagatttgt tgcgtgctag ccagttctaa 540

<210> 300
<211> 1872

<212> DNA
<213> Streptococcus pneumoniae

<400> 300
atgaaattca atccaaatca aagatatact cgttggtcta ttcgccgtct cagtgtcggt 60
gttgcctcag ttgttgtggc tagtggcttc tttgtcctag ttggtcagcc aagttctgta 120
cgtgccgatg ggctcaatcc aaccccaggt caagtcttac ctgaagagac atcgggaacg 180
aaagagggtg acttatcaga aaaaccagga gacaccgttc tcactcaagc gaaacctgag 240
ggcgttactg gaaatacgaa ttcacttccg acacctacag aaagaactga agtgagcgag 300
gaaacaagcc cttctagtct ggatacactt tttgaaaaag atgaagaagc tcaaaaaaat 360
ccagagctaa cagatgtctt aaaagaaact gtagatacag ctgatgtgga tgggacacaa 420
gcaagtccag cagaaactac tcctgaacaa gtaaaaggtg gagtgaaaga aaatacaaaa 480
gacagcatcg atgttcctgc tgcttatctt gaaaaagctg aagggaaagg tcctttcact 540
gccggtgtaa accaagtaat tccttatgaa ctattcgctg gtgatggtat gttaactcgt 600
ctattactaa aagcttcgga taatgctcct tggtctgaca atggtactgc taaaaatcct 660
gctttacctc ctcttgaagg attaacaaaa gggaaatact tctatgaagt agacttaaat 720
ggcaatactg ttggtaaaca aggtcaagct ttaattgatc aacttcgcgc taatggtact 780
caaacttata aagctactgt taaagtttac ggaaataaag acggtaaagc tgacttgact 840
aatctagttg ctactaaaaa tgtagacatc aacatcaatg gattagttgc taaagaaaca 900
gttcaaaaag ccgttgcaga caacgttaaa gacagtatcg atgttccagc agcctaccta 960
gaaaaagcca agggtgaagg tccattcaca gcaggtgtca accatgtgat tccatacgaa 1020
ctcttcgcag gtgatggcat gttgactcgt ctcttgctca aggcatctga caaggcacca 1080
tggtcagata acggcgacgc taaaaaccca gccctatctc cactaggcga aaacgtgaag 1140
accaaaggtc aatacttcta tcaagtagcc ttggacggaa atgtagctgg caaagaaaaa 1200
caagcgctca ttgaccagtt ccgagcaaat ggtactcaaa cttacagcgc tacagtcaat 1260
gtctatggta acaaagacgg taaaccagac ttggacaaca tcgtagcaac taaaaaagtc 1320
actattaaca taaacggttt aatttctaaa gaaacagttc aaaaagccgt tgcagacaac 1380
gttaaagaca gtatcgatgt tccagcagcc tacctagaaa aagccaaggg tgaaggtcca 1440
ttcacagcag gtgtcaacca tgtgattcca tacgaactct tcgcaggtga tggtatgttg 1500
actcgtctct tgctcaaggc atctgacaag gcaccatggt cagataacgg tgacgctaaa 1560
aacccagccc tatctccact aggtgaaaac gtgaagacca aaggtcaata cttctatcaa 1620
ttagccttgg acggaaatgt agctggcaaa gaaaaacaag cgctcattga ccagttccga 1680
gcaaacggta ctcaaactta cagcgctaca gtcaatgtct atggtaacaa agacggtaaa 1740
ccagacttgg acaacatcgt agcaactaaa aaagtcacta ttaacataaa cggtttaatt 1800
tctaaagaaa cagttcaaaa agccgttgca gacaacgtta agacagtatc gatgttccag 1860
cagcctacct ag 1872

<210> 301
<211> 1053
<212> DNA
<213> Streptococcus pneumoniae

<400> 301
atgaaactaa aaagttatat tttggttgga tatattattt caaccctctt aaccattttg 60
gttgtttttt gggctgttca aaaaatgctg attgcgaaag gcgagattta ctttttgctt 120
gggatgacca tcgttgccag ccttgtcggt gctgggatta gtctctttct cctattgcca 180
gtctttacgt cgttgggcaa actcaaggag catgccaagc gggtagcggc caaggatttt 240
ccttcaaatt tggaggttca aggtcctgta gaatttcagc aattagggca aacttttaat 300
gagatgtccc atgatttgca ggtaagcttt gattccttgg aagaaagcga acgagaaaag 360
ggcttgatga ttgcccagtt gtcgcatgat attaagactc ctatcacttc gatccaagcg 420
acggtagaag ggattttgga tgggattatc aaggagtcgg agcaagctca ttatctagca 480
accattggac gccagacgga gaggctcaat aaactggttg aggagttgaa ttttttgacc 540
ctaaacacag ctagaaatca ggtggaaact accagtaaag acagtatttt tctggacaag 600
ctcttaattg agtgcatgag tgaatttcag tttttgattg agcaggagag aagagatgtc 660
cacttgcagg taatcccaga gtctgcccgg attgagggag attatgctaa gctttctcgt 720
atcttggtga atctggtcga taacgctttt aaatattctg ctccaggaac caagctggaa 780
gtggtggcta agctggagaa ggaccagctt tcaatcagtg tgaccgatga agggcagggt 840
attgccccag aggatttgga aaatattttc aaacgccttt atcgtgtcga aacttcgcgt 900
aacatgaaga caggtggtca tggattagga cttgcgattg cgcgtgaatt ggcccatcaa 960
ttgggtgggg aaatcacagt cagcagccag tacggtctag gaagtacctt taccctcgtt 1020

ctcaacctct ctggtagtga aaataaagcc taa 1053

<210> 302
<211> 528
<212> DNA
<213> Streptococcus pneumoniae

<400> 302
atgtttggtc aaacggctca acatggtctt acgaatagcc tgaaagactt ctggattttt 60
ctgctgaata taggtccaca attggcgttt ttttgccaga tgctccgctg ttccagatcg 120
gttgagcagg gtactggaaa tcaccgtcgt gagttcaata tgattcagca gatattctcg 180
cattttggga tgactcactt gggacaaatc aagttggtct atcaagagtc gattgacctt 240
gagttgctgg tcaatgcact taatcatcac ttgctcattg acagactggt cctcacgccc 300
aatcaaataa cgatagaaat cgacaggcag atagtacatg gtcttgacct gctgaagggg 360
cgtaaagaca aagagattat cgacataaaa agtatgttca ggcagttaga actggctagc 420
acgcaacaaa tctgtccgat aaatcagcga gtgcatcatg gtatactggc ctttggagaa 480
atttccgacc tggtcccagc caaaaatctg ccgaacaggc aagactga 528

<210> 303
<211> 477
<212> DNA
<213> Streptococcus pneumoniae

<400> 303
atggaacatt tagcaactta tttttcaacc tatggaggag ctttcttcgc tgcattggga 60
attgtattgg cggttggatt aagcggtatg gggtctgctt atggagttgg taaggctggg 120
caatctgccg cagctttact gaaagaacag cctgaaaagt ttgcctcagc tttgatattg 180
caattattgc ccggaacaca aggattatat ggttttgtta ttggaatttt aatttggttg 240
caattaactc cagaacttcc tttagaaaaa ggcgttgctt atttctttgt agctcttcca 300
attgctattg taggatactt ttcagctaag catcaaggaa atgtagcagt agcgggaatg 360
caaatcttgg ctaaaagacc aaaagaattc atgaagggag caattttagc tgccatggta 420
gaaacctatg caattcttgc ttttgtcgta tcattcattt tgacccttcg tgtataa 477

<210> 304
<211> 498
<212> DNA
<213> Streptococcus pneumoniae

<400> 304
atgttaaaat cagaaaaaca atcacgttat caaatgttaa atgaagaatt gtccttccta 60
ttggaaggcg aaaccaatgt tttggctaat ctttccaacg ccagtgctct cataaaatca 120
cgttttccta ataccgtatt tgcaggcttt tatttgttcg atggaaagga attggtttta 180
ggccccttcc aaggaggtgt ttcctgcatc cgtattgcac taggcaaggg tgtttgtggt 240
gaggcagctc actttcagga aactgttatt gttggagatg tgacgaccta tctcaactat 300
atttcttgtg atagtctagc taaaagtgaa attgtggtgc cgatgatgaa gaatggtcag 360
ttacttggag ttctggatct ggattcttca gagattgagg attacgatgc tatggatcga 420
gattatttgg aacaatttgt cgctattttg cttgaaaaga cagcatggga ctttacgatg 480
tttgaggaaa aatcttaa 498

<210> 305
<211> 771
<212> DNA
<213> Streptococcus pneumoniae

<400> 305
atgtcagtat tagagatcaa agatcttcac gttgagattg aaggaaaaga aattttaaaa 60
ggggttaacc tgaccctgaa aacaggagaa attgccgcta tcatgggacc aaatggtaca 120

ggtaaatcga ctctttctgc cgctatcatg ggaaatccaa actatgaagt aactaaaggt 180
gaagttttgt ttgatggcgt aaacatcctt gagttggaag tggatgagcg tgcgcgtatg 240
ggacttttcc ttgctatgca atacccatca gaaatccctg gaattaccaa tgctgagttt 300
cttcgtgccg ctatgaatgc gggtaaagaa gatgatgaga agatttcagt tcgtgagttt 360
attactaagc tagatgaaaa aatggaattg ctcaacatga aagaagaaat ggcagagcgt 420
tacctcaacg aaggcttctc tggtggtgag aaaaaacgca atgaaattct tcaacttttg 480
atgttggagc caacatttgc tcttttggac gagattgact caggtcttga tattgacgct 540
cttaaagttg tgtctaaagg tgtcaatgcc atgcgtggtg aaggtttggg tgctatgatc 600
atcactcact accaacgtct tttgaactat atcacacctg atgtggtaca cgtgatgatg 660
gaaggtcgtg ttgtcctttc tggtggtcca gaattggctg cgcgtttgga acgtgaagga 720
tacgcaaaat tagctgaaga acttggctac gactacaagg aagaattgta a 771

<210> 306
<211> 471
<212> DNA
<213> Streptococcus pneumoniae

<400> 306
atgccctaca aaagacaaag gagtttttca atggcacttt ctaaactaga tagcctttat 60
atggcagtgg tagcagacca ttcgaaaaat ccacatcacc aagggaagtt agaagatgct 120
gagcaaatca gtctcaacaa tccgacttgt ggggatgtca tcaacctctc tgtcaagttt 180
gatgcagagg accgtttgga agatattgct tttctaaatt caggatgcac gatttcaact 240
gcttctgcta gtatgatgac agatgccgtt ttaggaaaaa ccaaacaaga aattttagaa 300
ctggcgacta tttttctgaa aatggttcaa gggcaaaaag atgagcgtca agaccaactt 360
ggagacgcgg cattcttgtc aggtgttgcc aaattccctc aaagaatcaa gtgtgcaacc 420
ctagcttgga atgcccttaa gaaaacaatt gaaaatcaag aaaaacagta a 471

<210> 307
<211> 1953
<212> DNA
<213> Streptococcus pneumoniae

<400> 307
atgaaaattc aagacctatt gagaaaagat gtcatgttgc tagatttgca ggcaactgaa 60
aaaacagctg tcatcgacga gatgattaaa aatttgacag accacggtta tgtaacagat 120
tttgaaacat ttaaagaagg aattttggcg cgtgaagctt tgacttctac tggtttgggt 180
gatggaatcg caatgcctca cagcaaaaac gctgctgtca aagaagcgac agttctattt 240
gctaagtcaa ataagggtgt tgactacgag agcttggatg gacaagcaac tgacctcttc 300
ttcatgattg cagctccaga aggtgccaat gatactcact tggcagcctt ggcagaattg 360
tctcaatact tgatgaaaga cggttttgca gacaaacttc gtcaagcaac atctgcagac 420
caagttatcg aactttttga ccaagcttca gaaaaaactg aggaacttgt tcaagcacct 480
gctaatgact ctggtgactt tatcgtagct gttacagctt gtacaacagg tattgcccac 540
acttacatgg cccaagaagc ccttcaaaaa gtagctgctg aaatgggggt tggtatcaag 600
gtcgaaacca acggtgctag cggtgttgga aatcaactaa ctgcagaaga tatccgtaag 660
gctaaagcta ttatcattgc agcagacaag gccgttgaaa tggatcgatt tgatggaaaa 720
ccattgatca atcgtccagt tgctgacggt atccgtaaga cagaagagct aattaacttg 780
gctctttcag gagatactga agtctaccgt gccgctaatg gtgccaaagc tgcaacagcc 840
tctaacgaaa aacaaagcct tggtggtgcc ttgtacaaac acttgatgag tggtgtatct 900
caaatgttac cattcgttat cggtggtggt atcatgattg cccttgcctt cttgattgac 960
ggtgctttgg gtgttccaaa tgaaaacctt ggcaatcttg gttcttacca tgagttagct 1020
tctatgttca tgaaaattgg tggagctgcc tttggtttga tgcttccagt ctttgcgggt 1080
tatgttgcct actctattgc tgaaaaaccg ggtttggtag caggtttcgt ggctggtgct 1140
attgccaaag aaggttttgc ctttggtaaa attccttatg ccgcaggtgg tgaagcaact 1200
tcaactcttg caggtgtctc atctggtttc ctaggtgccc ttgttggtgg atttatcgca 1260
ggtgccttgg ttcttgccat caagaaatac gttaaagttc ctcgttcact cgaaggtgct 1320
aaatcaatcc ttctattgcc acttcttgga acaatcttga caggatttgt tatgctagct 1380
gtgaatatcc caatggctgc aatcaacact gctatgaatg acttcctagg cggtcttgga 1440
ggaggttcag ctgtccttct tggtatcgtc cttggtggaa tgatggctgt tgacatgggt 1500
ggaccagtta ataaagcagc ttatgtcttt ggtacaggta cgcttgcagc aactgtttct 1560

tcaggtggtt ctgtagccat ggcagcagtt atggctggag gaatggtgcc accacttgca 1620
atctttgtcg caactcttct tttcaaagat aaatttacta aggaagaacg taactctggt 1680
ttgacaaaca tcatcatggg cttgtcattt atcactgagg gagcgattcc atttggtgcc 1740
gctgacccag ctcgtgcgat tccaagcttc atccttggtt cagcagtagc aggtggactc 1800
gttggtctta ctggtatcaa actcatggcg ccacacggag gaatcttcgt tatcgccctt 1860
acttcaaatg ctctccttta cctcgtttct gtcttggtag gagcaatcgt aagtggtgtg 1920
gtttatggtt acctacgcaa accacaagca taa 1953

<210> 308
<211> 2304
<212> DNA
<213> Streptococcus pneumoniae

<400> 308
atggcaaaca agaatacaag tacaacaaga cggagaccgt ctaaagcaga actggaaaga 60
aaagaagcga ttcaacgaat gttgatttcg ttaggaattg cgattttatt gattttcgca 120
gccttcaaat taggggctgc aggtataacc ctttataatt taattcgctt gctagtgggt 180
agcctagctt atctggcgat attcggccta ttaatctatc tcttcttttt caagtggata 240
cgaaaacagg aaggactctt atctggcttt ttcaccatat ttgctggctt actcttgatt 300
tttgaggcct acttggtttg gaaatatggt ttggacaagt ccgttctaaa agggaccatg 360
gctcaggttg tgacagatct gactggtttt cgaacgacta gctttgctgg agggggcttg 420
atcggggtcg ctctttatat tccaacagcc tttctctttt caaatatcgg aacttacttt 480
attggttcta tcttgatttt agtgggttct ctcctagtca gcccttggtc tgtttacgat 540
attgctgaat ttttcagtag aggctttgcc aaatggtggg aagggcacga gcgtcgaaaa 600
gaggaacgct ttgtcaaaca agaagaaaaa gctcgccaaa aggctgagaa agaggctaga 660
ttagaacaag aagagactga aaaagcctta ctcgatttgc ctcctgttga tatggaaacg 720
ggtgaaattc tgacagagga agctgttcaa aatcttccac ctattccaga agaaaagtgg 780
gtggaaccag aaatcatcct gcctcaagct gaacttaaat tccctgaaca ggaagatgac 840
tcagatgacg aagatgttca ggtcgatttt tcagccaaag aagcccttga atacaaactt 900
ccaagcttac aactctttgc accagataaa ccaaaagatc agtctaaaga gaagaaaatt 960
gtcagagaaa atatcaaaat cttagaagca acctttgcta gctttggtat taaggtaaca 1020
gttgaacggg ccgaaattgg gccatcagtg accaagtatg aagtcaagcc ggctgttggt 1080
gtaagggtca accgcatttc caatctatca gatgacctcg ctctagcctt ggctgccaaa 1140
gatgtccgga ttgaagcacc aatccctggg aaatccctaa tcggaattga agtgcccaac 1200
tccgatattg ccactgtatc tttccgagaa ctatgggaac aatcgcaaac gaaagcagaa 1260
aatttcttgg aaattccttt agggaaggct gttaatggaa ccgcaagagc ttttgacctt 1320
tctaaaatgc cccacttgct agttgcaggt tcaacgggtt cagggaagtc agtagcagtt 1380
aacggcatta ttgctagcat tctcatgaag gcgagaccag atcaagttaa atttatgatg 1440
gtcgatccca agatggttga gttatctgtt tacaatgata ttccccacct cttgattcca 1500
gtcgtgacca atccacgcaa agccagcaag gctctgcaaa aggttgtgga tgaaatggaa 1560
aaccgttatg aactctttgc caaggtggga gttcggaata ttgcaggttt taatgccaag 1620
gtagaagagt tcaattccca gtctgagtac aagcaaattc cgctaccatt cattgtcgtg 1680
attgtggatg agttggctga cctcatgatg gtggccagca aggaagtgga agatgctatc 1740
atccgtcttg ggcagaaggc gcgtgctgca ggtatccaca tgattcttgc aactcagcgt 1800
ccatctgttg atgtcatctc tggtttgatt aaggccaatg ttccatctcg tgtagcattt 1860
gcggtttcat caggaacaga ctcccgtacg attttggatg aaaatggagc agaaaaactt 1920
cttggtcgag gagacatgct ctttaaaccg attgatgaaa atcatccagt tcgtctccaa 1980
ggctccttta tctcggatga cgatgttgag cgcattgtga acttcatcaa gactcaggca 2040
gatgcagact acgatgagag ttttgatcca ggtgaggttt ctgaaaatga aggagaattt 2100
tcggatggag atgctggtgg tgatccgctt tttgaagaag ctaagtcttt ggttatcgaa 2160
acacagaaag ccagtgcgtc tatgattcag cgtcgtttat cagttggatt taaccgtgcg 2220
acccgtctca tggaagaact ggagatagca ggtgtcatcg gtccagctga aggtaccaaa 2280
cctcgaaaag tgttacaaca ataa 2304

<210> 309
<211> 528
<212> DNA
<213> Streptococcus pneumoniae

<400> 309
atgagttatt ttaaaaaata taaattcgat aaatcccagt tcaaacttgg tatgcgaacc 60
tttaaaacag gtattgctgt ttttctagtt ctcttgattt ttggcttttt tggctggaaa 120
ggtcttcaaa ttggtgcttt gacagccgtt tttagcctga gggagagttt tgatgagagt 180
gttcattttg ggacttcgcg tattctagga aatagtatcg gtggactcta tgccttggtc 240
ttcttcttat taaatacctt tttccacgaa gccttttggg tgaccttggt agttgttcca 300
atctgcacca tgttaaccat tatgacaaat gtagccatga ataacaaagc aggggttatt 360
ggtggtgtag cagctatgtt aatcattacc ctatcaattc caagtggtga gacaattttg 420
tacgtgtttg tgcgtgtatt agaaacgttt atgggagttt ttgtcgcaat tatcgtaaat 480
tacgatattg atcgtattcg tctcttttta gagaaaaaag aaaaataa 528

<210> 310
<211> 471
<212> DNA
<213> Streptococcus pneumoniae

<400> 310
atgaataaat cagaacaccg ccaccaactt atacgcgctc ttatcacaaa aaacaagatt 60
catacacagg ctgagttgca agcccttctt gctgagaacg acattcaagt aacccaggca 120
accctctcac gcgacatcaa aaatatgaac ctatcaaaag tccgcgaaga agatagcgct 180
tattatgttc ttaacaatgg ttccatctca aaatgggaaa aacgtctcga actctacatg 240
gaagacgccc ttgtctggat gcgcccagtt caacaccaag tcctactaaa aacccttcct 300
ggactggctc aatcctttgg ttctatcatt gatactttga gcttccctga cgctatcgct 360
accctttgtg gtaatgatgt ctgtcttatc atctgtgaag atgcagatac tgctcaaaag 420
tgctttgaag aactgaaaaa attcgcccca ccatttttct ttgaagaata a 471

<210> 311
<211> 1488
<212> DNA
<213> Streptococcus pneumoniae

<400> 311
atgaaaagta taaaattaaa tgctctatct tacatgggaa ttcgtgtctt gaatattatt 60
tttcccatcc taactggaac ctatgtcgcg cgtgtcttgg accgaactga ctatggttac 120
ttcaactcag tcgacactat tttgtcattt ttcttgccct ttgcaactta tggtgtctat 180
aactacggtt taagggctat cagtaatgtc aaggataaca aaaaagatct taacagaacc 240
ttttctagtc ttttttattt gtgcatcgct tgtacgattt tgaccactgc tgtctatatc 300
ctagcctatc ctctcttctt tactgataat ccaatcgtca aaaaggtcta ccttgttatg 360
gggattcaac tcattgccca gattttttca atcgaatggg tcaatgaagc tctggaaaat 420
tacagttttc tcttttacaa aactgccttc atccgtatcc tgatgctggt ctctattttc 480
ttatttgtta aaaatgaaca cgatattgtt gtctatacac ttgtgatgag tttatcgacg 540
ctgattaact acctgattag ttatttttgg attaaaagag acatcaaact tgttaaaatt 600
cacctaagtg attttaaacc actctttctc cctctgacag ccatgttagt ctttgccaat 660
gccaatatgc tcttcacttt tttagatcgc ctcttcctcg ttaaaacagg gattgatgtc 720
aacgttagtt actataccat agctcagcga attgtgaccg ttatagctgg ggttgtaaca 780
ggtgcaattg gagtgagtgt gcctcgtctc agttactatc tggggaaagg agacaaagaa 840
gcctatgttt ctctggttaa tagaggtagt cgaatcttta acttctttat cattccactg 900
agttttggac tcatggtttt aggaccaaat gccatcctac tttacggtag tgaaaaatat 960
atcggaggcg gcatcttgac ctctctcttc gcttttcgta cgattatcct ggccttagat 1020
accattcttg gttcccaaat tctctttacc aatggctatg aaaaacgtat cacagtctat 1080
acagtctttg ctgggctact caatttgggc ttgaatagtc tccttttttt caaccatatc 1140
gtggctcctg aatactactt actgacaact atgctatcag agacttctct acttgttttc 1200
tatatcattt tcatccatag aaaacaactc atccacttgg gacatatctt tagctatact 1260
gttcgatact ctctcttttc actttccttt gtagcaattt atttcctgat taatttcgtg 1320
tatcctgtag atatggtcat taatttgcca tttttgatta atactggttt gattgtcttg 1380
ctatcagcta tctcttatat tagtctactt gtcttcacaa aagatagcat tttctatgaa 1440
tttttaaacc atgtcctagc cttaaaaaat aaatttaaaa aatcatag 1488

<210> 312
<211> 804
<212> DNA
<213> Streptococcus pneumoniae

<400> 312
atgaaacaac taaccgttga agatgccaaa caaattgaat tagaaatttt ggattatatt 60
gatactctct gtaaaaagca caatatcaac tatattatta actacggtac tctgattggg 120
gcggttcgac atgagggctt tatcccttgg gacgacgata ttgatctgtc catgcctaga 180
gaagactacc aacgatttat taacattttt caaaaggaaa aaagcaagta taagctccta 240
tccttagaaa ctgataagaa ctactttaac aactttatca agataaccga cagtacgact 300
aaaattattg atactcgaaa tacaaaaacc tatgagtctg gtatctttat cgatattttc 360
cctatagatc gctttgatga tcctaaggtc attgatactt gttataaact ggaaagcttc 420
aaactgctgt ctttcagtaa acataaaaat attgtctata aggatagcct tttaaaagat 480
tggatacgaa cagccttctg gttactcctt cgaccggttt ctcctcgtta ttttgcaaat 540
aaaatcgaga aagaaattca aaaatatagt cgtgaaaatg ggcaatatat ggcttttatc 600
ccttcaaaat ttaaggaaaa ggaagtcttc ccaagtggta cctttgataa aacaatcgat 660
ttaccctttg agaatttaag ccttcctgca cctgaaaaat ttgatactat tttgacacaa 720
ttttatggag attatatgac cctaccacca gaagaaaaac gcttctacag tcatgaattt 780
cacgcttata aattggagga ttag 804

<210> 313
<211> 1527
<212> DNA
<213> Streptococcus pneumoniae

<400> 313
atgataaaaa tcaatcatct aaccatcaca caaaacaaag atttacgaga tcttgtatct 60
gacctaacca tgaccatcca agacggggaa aaggttgcta ttattggtga agaaggaaat 120
ggcaaatcaa ccttacttaa aattttaatg ggggaagctt tgtctgattt cactatcaag 180
ggaaacatcc aatctgacta tcagtcactg gcctacattc ctcaaaaagt ccctgaggac 240
ctaaaaaaga aaactttaca cgactacttc tttttagatt ctattgattt agactacagt 300
atcctctatc gtttggcgga ggaattgcat tttgatagca atcgtttcgc aagtgaccaa 360
gagattggca atctatcagg gggcgaagct ttgaaaattc agcttatcca tgagttagcc 420
aaaccctttg agattctatt tttagatgaa ccttcaaatg acctagacct tgagacagtt 480
gattggctaa aaggccagat tcaaaagacc aggcaaaccg ttattttcat ttcccatgat 540
gaagactttc tttctgaaac ggcagacact attgttcact tgcgactggt caaacaccgt 600
aaagaagcgg aaacgctagt agagcattta gactatgata gctatagtga gcagagaaag 660
gctaattttg ccaaacaaag tcagcaagct gctaacaacc aaagagccta cgataaaacc 720
atggaaaaac atcggagagt taagcaaaat gtagaaactg cgcttcgagc taccaaagat 780
agtactgccg gtcgcctatt ggctaaaaag atgaaaactg tcctctcaca agaaaaacgc 840
tacgaaaagg cagctcagtc catgactcaa aagccacttg aagaggaaca aatccaactt 900
ttcttttcag acatccaacc attaccagct tctaaagtct tagtccaact ggaaaaagaa 960
aatttgtcca ttgacgaccg agttttggtt caaaaactac aactaactgt ccgtggccaa 1020
gaaaaaatcg gtattatcgg gccaaatggt gttgggaaat caactctgtt agccaagtta 1080
cagagacttc tgaatgataa aagagagatt tcacttggtt ttatgccaca agattaccac 1140
aaaaaactgc aattggattt atccccaata gcctatctca gtaaaactgg ggaaaaagag 1200
gaactacaga aaatccaatc tcacctagct agtctcaatt tcagttatcc agaaatgcag 1260
catcaaattc gctccttatc tggcggacaa cagggaaaac tcctgctttt ggatttagtc 1320
ctgcgcaaac caaactttct cctgctggat gaacccacac gaaacttttc tcccacttct 1380
caaccccaaa tcagaaaact ctttgctacc tatccaggcg gtctcatcac tgtttcgcat 1440
gaccgtcgtt tcttaaaaga agtctgctcg atcatctatc gcatgacaga acacggtttg 1500
aagctagtta atttagaaga tttataa 1527

<210> 314
<211> 714
<212> DNA
<213> Streptococcus pneumoniae

<400> 314
atgaaaccaa aaacatttta caacttgctt gccgagcaga atcttccact ttcggaccag 60
caaaaagaac aatttgaacg ttattttgag ctcttggtcg agtggaatga gaagattaat 120
ttgacggcga ttacggacaa ggaagaagtt tatctcaaac atttttacga ttcgattgca 180
cccattcttc aaggtttgat tcccaatgaa actatcaaac ttcttgatat cggggctggg 240
gcaggatttc ctagtctacc aatgaaaatt ctctatccgg agttagatgt gaccattatt 300
gattcactca ataagcgcat caacttccta caactcttgg ctcaagaact ggatttgaac 360
ggagttcatt tctaccacgg acgtgccgaa gattttgccc aagacaagaa cttccgtgct 420
caatatgatt ttgtaacagc tcgtgcggtt gcccgtatgc aggtcctatc tgaattgact 480
attccctacc ttaaggttgg tggcaaacta ttagcactca aggctagcaa tgcgcctgag 540
gaattattag aagctaagaa tgccctcaat ctccttttta gtaaggtcga agacaatctc 600
agctacgccc taccgaatag agatccgcgc tatatcacag tggtagaaaa gaaaaaagaa 660
acaccaaata aatatccacg taaggctggt atgccaaata aacgcccact ttaa 714

<210> 315
<211> 807
<212> DNA
<213> Streptococcus pneumoniae

<400> 315
atgagtatta aactaattgc cgttgatatc gacggaaccc ttgtcaacag ccaaaaggaa 60
atcactcctg aagttttttc tgccatccaa gatgccaaag aagctggtgt caaagtcgtg 120
attgcaactg gccgccctat cgcaggcgtt gccaaacttc tagacgactt gcagttgaga 180
gacgaggggg actatgtggt aaccttcaac ggtgcccttg tccaagaaac tgctacagga 240
catgagatta tcagcgaatc cttgacttat gaggattatc tagatatgga attcctcagt 300
cgcaagctcg gtgtccacat gcatgccatt accaaggacg gtatctatac tgcaaatcgc 360
aatatcggaa aatacactgt acacgaatca accctcgtca gcatgcctat cttctaccgt 420
acccctgaag aaatggctgg caaagaaatt gttaaatgta tgtttatcga tgaaccagaa 480
attctcgatg ctgcgattga aaaaattcca gcagaatttt acgagcgcta ctccatcaac 540
aaatctgctc ctttctacct cgaactcctt aaaaagaatg tagacaaggg ttcagccatt 600
actcacttgg ctgaaaaact cggattgacc aaagatgaaa ccatggcaat cggtgatgaa 660
gaaaatgacc gtgccatgct ggaagtcgtt ggaaaccccg ttgtcatgga aaatggaaat 720
ccagaaatca aaaaaatcgc caaatacatc accaaaacaa atgacgaatc cggcgttgcc 780
catgccatcc gaacatgggt actgtaa 807

<210> 316
<211> 561
<212> DNA
<213> Streptococcus pneumoniae

<400> 316
atgacttgga ttattcttgg agttatcgct cttattgtta tttttgtgat tgttagctat 60
aacggtttgg ttaaaaatcg tatgcaaacc aaggaggctt ggagtcagat tgatgttcag 120
ttgaaacgtc gcaatgacct cttgccaaac ttgattgaga ctgtaaaagg ttatgccaaa 180
tatgaaggtt ctacccttga aaaggtggca gaactacgta accaagtggc ggcagcgact 240
tcaccagcag aagctatgaa agccagtgat gccctcactc gtcaggtttc aggtattttt 300
gcagttgcag aaagctatcc agatttgaaa gctagtgcta actttgttaa attgcaagag 360
gagttgacaa acacagaaaa taaaatttct tactctcgtc aactctataa cagtgttgtc 420
agcaactaca atgtaaaatt agaaactttc ccgagcaata ttatcgctgg aatgtttgga 480
tttaaagcgg cagatttcct tcaaacacct gaagaggaaa agtcggttcc taaagttgat 540
tttagcggtt taggtgacta a 561

<210> 317
<211> 900
<212> DNA
<213> Streptococcus pneumoniae

<400> 317

atgttgtttg atcaaattgc aagcaataaa cgaaaaacct ggattttgtt gctggtattt 60
ttcctactct tagctcttgt tggttatgcg gttggttatc tctttataag atctggactt 120
ggtggtttgg ttattgcact gattatcggc tttatctacg ctttgtctat gatttttcaa 180
tcgacagaga ttgtcatgtc catgaatgga gcgcgtgagg tggatgagca aacggcacca 240
gacctctacc atgtagtgga agatatggct ctggtcgctc agattcctat gccccgtgtt 300
ttcatcattg atgatccagc cttaaatgcc tttgcgacag gttctaatcc tcaaaatgcg 360
gctgttgctg cgacttcagg tctactagct atcatgaatc gtgaagaact agaagctgtt 420
atgggacatg aagtcagtca tattcgtaat tatgatatcc gtatttcgac tattgcagtt 480
gcccttgcta gtgctatcac catgctttct agtatggcag gtcgtatgat gtggtggggt 540
ggagcaggtc gcagacgaag tgatgatgac cgagatggaa atggtcttga aatcattatg 600
ctagtggttt ccctactagc tattgtactg gcacctctcg ctgcaacctt ggttcagctc 660
gctatttctc gtcagaggga atttctggca gatgcatcta gtgtcgagct gactcgcaat 720
ccccagggaa tgattaatgc cctagataag ttggacaata gcaaacctat gagtcgccac 780
gtcgatgatg ctagcagtgc cctttatatc aatgatccta agaaaggtgg ggggttccaa 840
aaactctttt atacccaccc acctatctca gaacggattg aacgtttaaa acagatgtaa 900

<210> 318
<211> 543
<212> DNA
<213> Streptococcus pneumoniae

<400> 318
atgaaattaa atattcaaga aattcgtaag cagtctgaag gtttgaactt tgaacaaacg 60
ttagatttag ttgatgacct gcgtgcacgt aatcaagaaa ttttagatgt aaaagatatc 120
cttgcagttg ggaaagtaca atatgaagac cgtatgtatt tcttagatta tcaactatct 180
tataccattg ttcttgcttc gagtcgcagt atggagccag ttgagttagt tgaatcttat 240
ccagtcacgg aagttttcat ggaaggcgca actaaccagc tagatcaaga agttttagat 300
gatgacttgg tcttgcccat cgaaaatggg gagcttgacc ttgctgagag tgtatcagac 360
aatatcctgc taaacattcc tatcaaggtc ttgacggctg aagaagaagc tggtcaagga 420
tttatctcag gaaatgactg gcaaatcatg acagaggaag aataccaagc tcaaaaagca 480
gtaaagaaag aagaaaacag tccttttgct ggcttacaag gactatttga cggagatgaa 540
taa 543

<210> 319
<211> 861
<212> DNA
<213> Streptococcus pneumoniae

<400> 319
atggagttat ttatgaaaat cacaaactat gaaatctata agttaaaaaa atcaggtttg 60
accaatcaac agattttgaa agtgctagaa tacggtgaaa atgttgatca ggagcttttg 120
ttgggtgata ttgcagatat ctcaggttgc cgtaatccag ccgtttttat ggaacgttat 180
tttcagatag acgatgcgca tttgtcgaaa gagtttcaaa aatttccatc tttctctatt 240
ttagatgact gttatccttg ggatttgagt gaaatatatg atgcgcctgt acttttattt 300
tacaagggaa atcttgacct cctgaaattc ccgaaggtag cggtcgtggg cagtcgtgct 360
tgtagcaaac agggagctaa gtcagttgaa aaagtcattc aaggcttgga aaatgaactg 420
gttattgtca gtggtctggc caagggcatt gacacagcag ctcatatggc agctcttcag 480
aatggcggaa aaaccattgc agtgattgga acaggactgg atgtgtttta tcctaaagcc 540
aataaacgct tgcaagacta catcggcaat gaccatctgg ttctaagtga atatggacct 600
ggtgaacaac ctctgaaatt tcattttcct gcccgtaatc gcatcattgc tggactttgt 660
cgtggtgtga ttgtagcaga ggctaagatg cgttcaggta gtctcattac gtgtgagcga 720
gcaatggaag aaggacgcga tgtctttgct attcctggta gcattttaga tggactatca 780
gacggttgcc atcatttgat tcaagaagga gcaaaattgg tcaccagtgg gcaagatgtt 840
cttgcggaat ttgaatttta a 861

<210> 320
<211> 534
<212> DNA

<213> Streptococcus pneumoniae

<400> 320
atgaaacgtc aattagcctt ggtcgtcttt agtggtggtc aagattcaac aacctgcctt 60
ttctgggtca tgcaacacta tgaaacagtc gaagctgtca cctttgccta cggccaacgt 120
catcacctcg aaattcaaat tactagagaa atcgctaagg aacagggcat tcgtcaccat 180
atcctcgata tgtctctgct gggacaaatc actgctcagc cagactttgc gacgattcat 240
atttcctaca ttcctgacaa gctctgtgtc gagtcaaaat ccctcaaact atatctattt 300
agctaccgaa accacggaga tttccacgaa aactgtatca acaccatcgg gaaagacttg 360
gtcaacttgc tagaccctcg ctatttagaa gtctggggaa aattcactcc gcgcggtggc 420
atttcaatcg acccctacta caactacggt aagcaaggaa ctaagtatga gggcttggca 480
gaacaacgcc tcttccaaca cgacctttat ccagagaaaa ttgacaaccg ctaa 534

<210> 321
<211> 3711
<212> DNA
<213> Streptococcus pneumoniae

<400> 321
atgaccgaaa cggtagaaga taaagtaagt cattcaatta ctgggcttga tatcctcaag 60
gggatagttg ctgcgggagc tgtcataagt ggaaccgttg caactcaaac gaaggtattt 120
acaaatgagt cagcagtact tgaaaaaact gtagagaaaa cggatgcttt ggcaacaaat 180
gatacagtag ttctaggtac gatatctaca agtaattcag cgagttcaac tagtttgtca 240
gcttcagagt cggcaagtac atctgcatct gagtcagcct caaccagcgc ttcgacctca 300
gcaagtacaa gtgcatcaga atcagcaagt acatcggctt cgacaagtat ttctgcatca 360
tctactgtgg taggttcaca aacagctgcc gctacagaag caactgctaa gaaggtcgaa 420
gaagatcgta agaaaccagc tagtgattat gtagcatcag ttacaaatgt caatctccaa 480
tcttatgcta agcgacgcaa gcgttcagtg gattccatcg agcaattgct ggcttctata 540
aaaaatgctg ctgttttttc tggcaatacg attgtaaatg gcgcccctgc aattaatgca 600
agtctaaaca ttgctaaaag tgagacaaaa gtttatacag gtgaaggtgt agattcggta 660
tatcgtgttc caatttacta taaattgaaa gtgacaaatg atggttcaaa attgaccttt 720
acctatacgg ttacgtatgt gaatcctaaa acaaatgatc ttggtaatat atcaagtatg 780
cgtcctggat attctatcta taattcaggt acttcaacac aaacaatgtt aacccttggc 840
agtgatcttg gtaaaccttc aggtgtaaag aactacatta ctgacaaaaa tggtagacag 900
gttctatcct ataatacatc tacaatgacg acgcagggta gtgggtatac ttggggaaat 960
ggtgcccaaa tgaatggttt ctttgctaag aaaggatatg gattaacatc atcttggact 1020
gtaccaatta ctggaacgga tacatccttt acatttaccc cttacgctgc tagaacagat 1080
agaattggaa ttaactactt caatggtgga ggaaaggtag ttgaatctag cacgaccagt 1140
cagtcacttt cacagtctaa gtcactctca gtaagtgcta gtcaaagcgc ctcagcttca 1200
gcatcaacaa gtgcgtcggc ttcagcatca accagtgcct cggcttcagc gtcaaccagt 1260
gcgtcagctt cagcaagtac cagtgcttca gtctcagcat caacaagtgc ttcagcctca 1320
gcatcgacaa gtgcctcggc ttcagcaagc acatcagcat ctgaatcagc gtcaaccagt 1380
gcttcggctt cagcaagtac cagtgcttca gcttcagcat caaccagcgc ctcggcctca 1440
gcaagcacct cagcttctga atcggcctca accagcgcct cggcctcagc aagcacctca 1500
gcttctgaat cggcctcaac cagcgcctca gcctcagcat caacgagtgc ttcggcttca 1560
gcaagcacaa gcgcctcggg ttcagcatca acgagtacgt cagcttcagc gtcaaccagt 1620
gcttcagcct cagcatcaac aagtgcgtca gcctcagcaa gtatctcagc gtctgaatcg 1680
gcatcaacga gtgcgtctga gtcagcatca acgagtacgt cagcctcagc aagcacctca 1740
gcttctgaat cggcctcaac cagtgcgtca gcctcagcat cgacaagcgc ctcagcttca 1800
gcaagtacca gtgcttcagc ctcagcgtcg acaagtgcgt cggcctcaac cagtgcatct 1860
gaatcggcat caaccagtgc gtcagcctca gcaagtacta gtgcatcggc ttcagcatca 1920
accagtgcct cggcttcagc gtcaaccagt gcgtcagctt cagcaagtac cagtgcttca 1980
gtctcagcat caacaagtgc ttcagcctca gcatcgacaa gtgcctcggc ttcagcaagc 2040
acatcagcat ctgaatcagc gtcgacaagc gcctcagctt cagcaagtac cagtgcgtca 2100
gcttcagcat caaccagcgc ctcggcctca gcaagcacct cagcttctga atcggcctca 2160
accagcgcct cggcctcagc aagcacctca gcttctgaat cggcctcaac cagcgcctca 2220
gcctcagcat caacgagtgc ttcggcttca gcaagcacaa gcgcctcggg ttcagcatca 2280
acgagtacgt cagcttcagc gtcaaccagt gcttcagcct cagcatcaac aagtgcgtca 2340
gcctcagcaa gtatctcagc gtctgaatcg gcatcaacga gtgcgtctga gtcagcatca 2400
acgagtacgt cagcctcagc aagcacctca gcttctgaat cggcctcaac cagtgcgtca 2460

gcctcagcat cgacaagcgc ctcagcttca gcaagtacca gtgcttcagc ctcagctcga 2520
caagtgcgtc ggcctcaacc agtgcatctg aatcggcatc aaccagtgcg tcagcctcag 2580
caagtactag tgcatcagct tcagcatcaa cgagtgcatc ggcttcagca tcaaccagtg 2640
cctcggcttc agcgtcaacc agtgcgtcag cttcagcaag taccagtgct tcagtctcag 2700
catcaacaag tgcttcagcc tcagcatcga caagtgcctc ggcttcagca agcacatcag 2760
catctgaatc agcgtcgaca agcgcctcag cttcagcaag taccagtgcg tcagcctcag 2820
cgtcgacaag tgcgtcagcc tcagcaagta ctagtgcatc agcttcagca tcaacgagtg 2880
catcggcttc ggcgtcaacc agtgcatcag agtcagcaag taccagtgcg tcagcttccg 2940
catcaacaag tgcctcggct tcagcaagca ccagtgcgtc ggcttcagca agtactagcg 3000
cctcagcctc agcctcaacc agtgcgtcag cctcagcaag tatctcagcg tctgaatcgg 3060
catcaacgag tgcgtccgct tcagcaagta ctagcgcctc agcctcagcg tcaacaagtg 3120
catcggcttc agcgtcaacg agtgcgtctg aatcggcatc aacgagtgcg tccgcttcag 3180
caagtactag cgcctcagcc tcagcgtcaa caagtgcatc ggcttcagca tcaacgagtg 3240
cgtccgcttc agcaagtact agcgcctcag cctcagcgtc aacaagtgca tcggcttcag 3300
cgtcaacgag tgcgtctgag tcagcatcaa cgagtgcgtc agcctcagca agcacatcag 3360
cttctgaatc tgcatcaacc agtgcgtcag cctcagcatc gacaagcgcc tcagcttcag 3420
caagtaccag tgcgtcagcc tcagcgtcga caagtgcgtc ggcttcagca agtaccagtg 3480
cgtcagcctc agcaagtacc agtgcgtcag cctcagcgtc gacaagtgcg tcggcctcaa 3540
ccagtgcatc tgaatcggca tcaaccagtg cgtcagcctc agcaagtact agtgcatcag 3600
cttcagcatc aacgagtgca tcggcttcag catcaaccag tgcatcagag tcagcaagta 3660
ccagtgcgtc agttccgcat caacaagtgc ctcggcttca gcaagtacta g 3711

<210> 322
<211> 1350
<212> DNA
<213> Streptococcus pneumoniae

<400> 322
atgggggtcg aaacttggtt ttattctagc atctgctggc tggccatcgg gcttggttcc 60
gtttggaagt ttccctacat gactgctgct aatggcggtg gaggcttttt actaatcttt 120
ctcatttcca ctattttaat cggtttccct ctcctgctgg ctgagtttgc ccttggccgt 180
agtgctggcg tttccgctat caaaaccttt ggaaaactgg gcaagaataa caagtacaac 240
tttatcggtt ggattggcgc ctttgccctc tttatcctct tatcttttta cagtgttatc 300
ggaggatgga ttctagtcta tctaggtatt gagtttggga aattgttcca acttggtgga 360
acgggtgatt atgctcagtt atttacttca atcatttcaa atccagccat tgccctagga 420
gctcaagcgg cctttatcct attgaatatc ttcattgtat cacgtggggt tcaaaaaggg 480
attgaaagag cttcgaaagt catgatgccc ctgctcttta tcgtctttgt ttttatcatc 540
ggtcgctctc tcagtttgcc aaatgccatg gaaggggttc tttacttcct caaaccagac 600
ttttcaaaac tgactagcac tggtctcctc tatgctctgg gacaatcttt ctttgccctc 660
tcactagggg ttacagtcat gttgacctat gcttcttact tagacaagaa aaccaatcta 720
gtccagtcag gaatctccat cgtagccatg aatatctcga tatccatcat ggcaggtcta 780
gccatttccc aagctcgatc ccccttcaat atccagtctg aagggggacc cagcctgctc 840
tttatcgtct tgcctcaact ctttgacaag atgccttttg gaaccatttt ctacgtcctc 900
ttcctcttgc tcttcctttt tgcgacagtc actttttctg tcgtgatgct ggaaatcaat 960
gtagacaata tcaccaacca ggataacagc aaacgtgcca aatggagtgt tattttagga 1020
attttgacct ttgtctttgg cattccttca gccctatctt acggtgtcat ggcggatgtt 1080
cacatttttg gtaagacctt ctttgacgct atggacttct tggtttccaa tctcctcatg 1140
ccatttggag ctctctacct ttcacttttt acaggctata tctttaaaaa ggctcttgca 1200
atggaggaac tccatctcga tgaaagagca tggaaacaag gactgttcca agtctggctc 1260
ttccttcttc gtttcttcgt ttcgtcattc caatcatcat cattgtggtc ttcattgccc 1320
aatttatgta atcaaaaagg acttgagtag 1350

<210> 323
<211> 561
<212> DNA
<213> Streptococcus pneumoniae

<400> 323
atgttgaaaa aatggcagtt aaaagatgtt atcttgcttg ctttcttgtc tatctttttt 60

ggtggggttt tcgttggttc aggatatgtg tataatattc tcagtctact cttaacacct 120
cttggtttgc aggcctttgc caatgaaatc ctcttcggtc tctggtgtat ggctgcgccc 180
attgctgcca tctttgttcc gagagtcgga agtgcaacga ttggagaagt gctagctgcg 240
cttgctgaag tcctttatgg tagccaattt ggtctaggag ctcttttgtc tggctttgtt 300
caaggtttgg gaagtgaatt tggttttatc gtaactaaga atcgctatga aagttggctc 360
tctctaactg ctaatagtat tgggattacg cttgttagct ttgtctatga atacattaag 420
ttaggttact acgccttttc ccttccgttt gtcctttcct tgcttgtggt acgttttatt 480
tctgtttatt tcttctgtac catcttggtt cgtgccattg tcaaactcta tcatcagttt 540
gcaactggag gaaaagcata g 561

<210> 324
<211> 651
<212> DNA
<213> Streptococcus pneumoniae

<400> 324
atggtcaaag tagcaaccca gacaccgatt atcagtctct tcttgctgat tttatccttg 60
gaaacatctt tcattccttc gattgctctg actctttcgg tagtcgcatt ttgtattctc 120
tttatgctct attaccgtcg atttaaaatg ttagcttgga tgatcatact tgccatttta 180
ccatcttttg ccaactactg ggcagttcag ttacacggag atgcttcaca ggcagtcatg 240
cttggaacga gggcctttgt gacagtttgt atcggccttg tctttgtttc ctctgtttca 300
ctaaaagagc ttctcttgta cttggctcaa aaggggctat cacgctcttg gtcctatgcc 360
ttgattgtgg tattcaattc ttttcctctc attcagcaag aaatcaagtc cctcaaagaa 420
gcttgcctat tacgtggtca agaactacat ttttggtcgc ccttgattta cagtaaggtt 480
ctgatgacag tctttaggtg gcgccatctt tacctgagag ctctatctgc tcacggatat 540
gacgaacatg cacagttgaa gaatagctat cggacttttt atattcctaa aaaaacaaaa 600
ttaatctacc tgcttttctt tttattgctt caaaccagtc tatttttata a 651

<210> 325
<211> 525
<212> DNA
<213> Streptococcus pneumoniae

<400> 325
atgagaaagc accaattaca agttcacaaa ttaaccattt tatctatgat gattgccctt 60
gatgtagtcc ttacacctat ctttcgaatt gagggaatgg caccgatgtc cagtgtagtc 120
aatattctag caggaatcat gatgggacct gtttatgcct tggctatggc tacagtcaca 180
gcctttatcc gtatgacgac tcaagggatt ccgcctttag ctctcacagg agcgactttt 240
ggagcccttc tagcaggtct cttttataag tacggtcgaa aatttcacta ttctgctcta 300
ggagagattt tgggaacagg tattattggt tccattgttt cctatcctgt tatggtactc 360
tttacaggat cagctgctaa gcttagctgg tttatctaca cgcctcgatt tttcggagca 420
accttgattg gtacagcgat ttcctttatt gcctttcgat ttttaatcaa gcaggaattc 480
tttaaaaaag tgcagggata tttctttagt gaaaggatag actga 525

<210> 326
<211> 804
<212> DNA
<213> Streptococcus pneumoniae

<400> 326
atgcaggaat ttacaaatcc ctttcctata ggctctagtt ccctcattca ctgcattacc 60
aatgagattt cttgtgagat gctggcaaat gggattttgg ctctgggatg caaacctgtc 120
atggcagatg attcccgtga agttcttgat tttactaagc aaagtcaggc tctcttcatc 180
aatttggggc atttgtcagc tgagaaggaa aaagcaatcc gcatggcagc ttcgtatgca 240
aaccaatctt ctctcccgat ggtagtagat gcggttggcg taacgacttc atccattcgt 300
aagagcttag ttaaagacct tttagactat agacctacgg tccttaaagg aaacatgtca 360
gaaattcgaa gtcttgttgg attaaagcac cacggcgttg gggtcgatgc gagtgctaaa 420
gatcaagaaa cggaggattt gcttcaagtc ttgaaagact ggtgtcagac ctatcctggt 480

atgtctttct tagtcacagg tcccaaggac ctcgtcgttt cgaaaaatca ggtcgctgta 540
ctgggaaatg gctgtactga attagactgg ataacaggga caggagactt ggttggagcc 600
ttaacagctg tttttctcag ccaaggaaag actggttttg aagcttcttg cttagcagtc 660
tcttatctca atatcgctgc tgagaaaata gttgttcaag gaatgggatt ggaagaattt 720
cgttaccaag tactcaatca gctttcgctc ctaagaagag atgaaaattg gctagatacc 780
atcaaaggag aggtttatga atag 804

<210> 327
<211> 834
<212> DNA
<213> Streptococcus pneumoniae

<400> 327
atgaaccata aaatcgcaat tttatcagat gttcatggca atgcgacggc gctagaagca 60
gtgattgcag atgctaaaaa tcaaggggcc agtgaatatt ggcttctggg agatattttt 120
cttcctggtc caggcgcaaa tgacttagtc gccctgctaa aggaccttcc tatcacagca 180
agtgttcgag gcaattggga tgatcgtgtc cttgaggctt tagatgggca atatggctta 240
gaagacccac aggaagttca gctcttgcgt atgacacagt atttgatgga gcgaatggat 300
cctgcaacga ttgtctggct acgaagcttg cctttgctgg aaaagaaaga aattgacgga 360
ttgcgctttt ctatctctca taatttacct gacaaaaact atggtggtga cttgctagtt 420
gagaatgata cagagaaatt tgaccaactg ctagatgcgg aaacggacgt ggcagtttat 480
ggtcatgttc acaagcagtt gcttcgttat ggaagtcaag ggcaacaaat catcaatcca 540
gggtcgattg gcatgcccta ttttaattgg gaggcgttaa aaaatcaccg ttcccagtat 600
gccgtgatag aagttgaaga tggggaatta ctcaatatcc aatttcgtaa agttgcttat 660
gattacgaag ctgagttaga attggccaag tccaaggggc ttccctttat cgaaatgtat 720
gaagaactgc gtcgtgacga taactatcag gggcacaatc tggaattatt agccagctta 780
atagaaaagc atgggtatgt agaggatgtg aagaattttt ttgatttttt gtaa 834

<210> 328
<211> 726
<212> DNA
<213> Streptococcus pneumoniae

<400> 328
atgaatgtaa atcagattgt acggattatt cctactttaa aagctaataa tagaaaatta 60
aatgaaacat tttatattga aacccttgga atgaaggcct tgttagaaga atcggccttt 120
ctgtcactag gtgaccaaac gggtcttgaa aagctggttt tagaagaagc tcccagtatg 180
cgtactcgta aggtagaggg aagaaaaaaa ctagctagat tgattgtcaa ggtggaaaat 240
cccttagaaa ttgaaggaat cttatctaaa acagattcga ttcatcgatt atataaaggt 300
caaaatggct acgcttttga aattttctca ccagaagatg atttgatttt gattcatgcg 360
gaagatgaca tagcaagtct agtagaagta ggagaaaagc ctgaatttca aacagatttg 420
gcatcaattt ctttaagtaa atttgagatt tctatggaat tacatctccc aactgatatc 480
gaaagtttct tggaatcatc tgaaattggg gcatcccttg attttattcc agctcagggg 540
caggatttga ctgtggacaa tacggttacc tgggacttat ctatgctcaa gttcttggtc 600
aatgaattag acatagcaag tcttcgccag aagtttgagt ctactgaata ttttattcct 660
aagtctgaaa aattcttcct tggtaaagat agaaataatg ttgaattgtg gtttgaagaa 720
gtatga 726

<210> 329
<211> 936
<212> DNA
<213> Streptococcus pneumoniae

<400> 329
atgaagtgga ccaagattat taaaaaaata gaagaacaaa tcgaggcagg gatttatccc 60
ggagcctctt ttgcgtattt taaggacaat caatggacag agttctattt aggccagagt 120
gacccagagc atggcttgca gactgaggca ggactagttt atgacctagc tagtgtcagc 180
aaggttgttg gggttggcac agtttgtacc ttcttgtggg aaataggtca attagatatt 240

gatagactgg taatagattt tttacctgag agtgattatc cagacatcac tattcgccag 300
ctcttgactc atgcaacaga ccttgatcct tttattccta atcgtgatct tttaacagcc 360
cctgaattaa aggaagcgat gtttcatctc aacagacgaa gtcagccagc ctttctttat 420
tcggatgtcc attttttgct gttgggcttt attttggaaa gaatttttaa tcaagatttg 480
gatgtgattt taaaggatca agtctggaaa ccttggggaa tgacggaaac taagtttggg 540
ccagttgagc ttgctgttcc aacagttaga ggtgtagagg caggcatagt gcatgatccc 600
aaggctcgtc tcctgggtag acatgctggg agtgctggtt tattttcgac tataaaggat 660
ttacaaatct ttttagaaca ctatttagca gatgattttg caagagactt aaatcaaaat 720
ttttctcctt tggatgacaa ggaacgttct ttagcatgga atttggaagg agattggcta 780
gaccatacgg gctatacagg tacctttatc atgtggaatc gtcagaagca agaagccact 840
attttcctat cgaatcgtac ctatgaaaag gacgagagag ctcaatggat attagaccgc 900
aatcaagtga tgaacttgat tcgcaaagaa gagtaa 936

<210> 330
<211> 483
<212> DNA
<213> Streptococcus pneumoniae

<400> 330
atgatgaaga agacttataa tcatattttg gtctggggag tcattttcta tagcatttgc 60
attgtctgtt tttgctttac tcctcaagaa caatctaccg tgggagtggg aactccaggt 120
attcagcatc ttggacgcct ggtttttctt ttgactcctt tcaattctct ctggaaactg 180
ggcgaagtga gtgacattgg acaattatgt tggatttttt tacaaaatat cctcaatgtc 240
ttcttgtttt ttcctctgat tttccaactc ctttatctat ttccaaattt gcggaaaaca 300
aaaaaggtcc ttctttttag ttttcttgtg agtcttggaa tcgagtgtac gcaattaatc 360
ttggactttt tctttgattt caatcgcgtc tttgagattg atgatttgtg gaccaacact 420
ttgggtggct atctggcttg gctcctttat aaacgattac ataaaaacaa ggtaaggaat 480
taa 483

<210> 331
<211> 792
<212> DNA
<213> Streptococcus pneumoniae

<400> 331
atgaagattc ctctcttaac ttttgcaagg cataaatttg tttatgtctt gcttactttg 60
ctttttcttg ctttggttta tcgtgatgtt ttgatgactt atttcttttt tgatattcat 120
gcgcccgatc tagctaaatt cgatggacaa gcaattaaaa atgacttatt aaaatcagca 180
ttagattttc gtattctcca gttcaatcta ggtttttatc aatcatttat tattccaatc 240
atcattgttt tgctaggttt tcaatatatt gagctgaaaa ataaagtttt acgattgagt 300
attggaagag aagtgagtta tcaagggtta aaaagaaagt tgactttgca agttgcaagt 360
atcccttgtt tgatatattt agtgactgtg ctgataattg caattataac ctatttcttt 420
gggacttttt ctcctcttgg atggaattct ctattttctg atggaagtgg tttacaaaga 480
ctcctagatg gagagataaa aagctatttg ttctttactt gtgtcctact aatcggtatt 540
ttcatcaatg caatctattt tttacaaata gttgattatg tggggaatgt gactcgttcg 600
gcaatcacct atttgatgtt tctttggctt ggttctatgc tgctttatag tgccttgcct 660
tactatatgg ttcctatgac gagtttgatg caagctagct atggggatgt aagtttgatg 720
aaactcttta ctccttatat cctttatatt gtcccttaca tggtgcttga aaaatatgaa 780
gataatgttt aa 792

<210> 332
<211> 651
<212> DNA
<213> Streptococcus pneumoniae

<400> 332
atgaagataa tgtttaagaa ttttaacaat attttgctaa atagaaagat tgttttacta 60
cttcgtatag ttctgatgat gattttgata aaccatctat tgtcaacagc ggttcaaaag 120

caggatgctg ttatcttttt caagagagaa ttgatttcaa tttttcccta taatgactat 180
tctgaagcga atttagaaat ccccaaacta ttgttaaacc tttcgctttt catggtagga 240
tggctctctg tcattttact tgaaagtgat ttggcagacc attaccatca cttgattcgc 300
tatcaatcaa gctccttttt cgattataca aggaaacgat tggttgtcat ttctaaattt 360
tttactcaag atttgtttgt ctggtttctt ggtttacttc ctctaggaat tcatttcaaa 420
acagtcgcac ttttcttttt acttgctcag ttaatgatgt tgtacttact actgtcttat 480
ctgatagcac tgattagtgc gggcgctggt tttccttttt ttctctattt tttagcattt 540
gtgggacaag aatggatgat ggatcatatt gtaacagtgt atttagtact cttaagttta 600
ttagttatgt tgattgttag tcgcttggaa gagaaattta agaaaggata a 651

<210> 333
<211> 525
<212> DNA
<213> Streptococcus pneumoniae

<400> 333
atgggcaaag gagagatggg caaaggagtt attggcttgg agttcgactc agaagtattg 60
gtcaacaagg ctccaaccct tcaattggca aatggtaaaa cagcgacttt cctaacccag 120
tatgatagca agaccttgtt gtttgcagta gataaggaag atatcggaca ggaaattatt 180
ggtatagcta aaggaagcat cgaaagtatg cataatcttc ctgtaaatct agcaggtgcc 240
agagttcctg gcggagtaaa tggtagcaaa gcagcggtgc atgaagttcc agaatttaca 300
gggggagtta atggtacaga gccagctgtt catgaaatcg cagagtataa gggatctgat 360
tcgcttgtaa ctcttactac aaaaaaagat tatacttaca aagctcctct tgctcagcag 420
gcacttcctg aaacaggaaa caaggagagt gacctcctag cttcactagg actaacagct 480
ttcttccttg gtctgtttac gctagggaaa aagagagaac aataa 525

<210> 334
<211> 1518
<212> DNA
<213> Streptococcus pneumoniae

<400> 334
   atgtttaaag ttttacaaaa agttggaaaa gcttttatgt tacctatagc tatacttcct 60
   gcagcaggtc tacttttggg gattggtggt gcactttcaa acccaaccac gatagcaact 120
   tatccaatac tagacaatag tatttttcaa tcaatattcc aagtaatgag ctctgcagga 180
   gaggttgtat tcagtaattt gtcactactt ctctgtgtgg gattatgtat tggcttagcg 240
   aaacgagata aaggaaccgc tgcgttagca ggagtaactg gttacttagt tatgactgca 300
   acgatcaaag ctttggtaaa actttttatg gcagaaggat ctgcaattga tactggagtt 360
   attggagcat tagttgtcgg aatagttgcc gtatatttgc acaaccgata taacaatatt 420
   caattacctt ccgctttagg attctttgga ggttcacgct tcgttcctat tgttacatcg 480
   ttctcttcta tcttgattgg ctttgtcttc tttgttattt ggccaccttt ccaacaactt 540
   cttgtttcta caggtggata tatttctcag gcgggtccaa ttggaacttt tctatatgga 600
   tttttaatga gactttctgg agcagtaggc ttacatcata taatttaccc tatgttttgg 660
   tatactgaac ttggtggtgt tgaaactgtt gcaggacaaa cagtggttgg agctcaaaaa 720
   atattttttg ctcaattagc cgatttggcc cattctggat tatttacaga aggaacaagg 780
   ttttttgcag gtcgtttctc aacaatgatg ttcggtttac cggctgcctg tttagcgatg 840
   taccatagtg ttcctaaaaa tcgtcgtaaa aaatacgcgg gtttgttttt tggagttgct 900
   ttaacatctt ttattaccgg tattacagaa ccaattgaat ttatgtttct attcgtcagt 960
   ccggttctat atgttgttca cgcattcctt gatggtgtta gcttctttat tgcagacgtc 1020
   ttaaatattt caataggaaa cacattttca ggaggtgtaa tcgatttcac tttatttgga 1080
   attttgcagg ggaacgctaa gacgaattgg gttcttcaga ttccatttgg acttatttgg 1140
   agtgttttgt attatattat ttttagatgg ttcattactc aattcaacgt tctaacgcca 1200
   gggcgaggag aagaagtaga ttctaaagaa atttctgaat ccgcagattc aacttcaaat 1260
   actgcagatt atttaaaaca ggatagccta caaattatca gagccttggg tggatcaaat 1320
   aatatagaag atgtagatgc ttgtgtgaca cgtttacgtg tagctgtaaa agaagttaat 1380
   caagttgata aagcactttt aaaacaaatt ggtgcagttg atgtcttaga agtgaagggt 1440
   ggcattcaag caatctatgg agcaaaagca atcttatata aaaatagtat taatgaaatt 1500
   ttaggtgtag atgattaa 1518

<210> 335
<211> 1329
<212> DNA
<213> Streptococcus pneumoniae

<400> 335
atgaaattta gaaaattagc ttgtacagta cttgcgggtg ctgcggttct tggtcttgct 60
gcttgtggca attctggcgg aagtaaagat gctgccaaat caggtggtga cggtgccaaa 120
acagaaatca cttggtgggc attcccagta tttacccaag aaaaaactgg tgacggtgtt 180
ggaacttatg aaaaatcaat catcgaagcg tttgaaaaag caaacccaga tataaaagtg 240
aaattggaaa ccatcgactt caagtcaggt cctgaaaaaa tcacaacagc catcgaagca 300
ggaacagctc cagacgtact ctttgatgca ccaggacgta tcatccaata cggtaaaaac 360
ggtaaattgg ctgagttgaa tgacctcttc acagatgaat ttgttaaaga tgtcaacaat 420
gaaaacatcg tacaagcaag taaagctgga gacaaggctt atatgtatcc gattagttct 480
gccccattct acatggcaat gaacaagaaa atgttagaag atgctggagt agcaaacctt 540
gtaaaagaag gttggacaac tgatgatttt gaaaaagtat tgaaagcact taaagacaag 600
ggttacacac caggttcatt gttcagttct ggtcaagggg gagaccaagg aacacgtgcc 660
tttatctcta acctttatag cggttctgta acagatgaaa aagttagcaa atatacaact 720
gatgatccta aattcgtcaa aggtcttgaa aaagcaacta gctggattaa agacaatttg 780
atcaataatg gttcacaatt tgacggtggg gcagatatcc aaaactttgc caacggtcaa 840
acatcttaca caatcctttg ggcaccagct caaaatggta tccaagctaa acttttagaa 900
gcaagtaagg tagaagtggt agaagtacca ttcccatcag acgaaggtaa gccagctctt 960
gagtaccttg taaacgggtt tgcagtattc aacaataaag acgacaagaa agtcgctgca 1020
tctaagaaat tcatccagtt tatcgcagat gacaaggagt ggggacctaa agacgtagtt 1080
cgtacaggtg ctttcccagt ccgtacttca tttggaaaac tttatgaaga caaacgcatg 1140
gaaacaatca gcggctggac tcaatactac tcaccatact acaacactat tgatggattt 1200
gctgaaatga gaacactttg gttcccaatg ttgcaatctg tatcaaatgg tgacgaaaaa 1260
ccagcagatg ctttgaaagc cttcactgaa aaagcgaacg aaacaatcaa aaaagctatg 1320
aaacaatag 1329

<210> 336
<211> 840
<212> DNA
<213> Streptococcus pneumoniae

<400> 336
atgcaatcta cagaaaaaaa accattaaca gcctttactg ttatttcaac aatcattttg 60
ctcttgttga ctgtgctgtt catctttcca ttctactgga ttttgacagg ggcattcaaa 120
tcacaacctg atacaattgt tattcctcct cagtggttcc ctaaaatgcc aaccatggaa 180
aacttccaac aactcatggt gcagaaccct gccttgcaat ggatgtggaa ctcagtattt 240
atctcattgg taaccatgtt cttagtttgt gcaacctcat ctctagcagg ttatgtattg 300
gctaaaaaac gtttctatgg tcaacgcatt ctatttgcta tctttatcgc tgctatggcg 360
cttccaaaac aagttgtcct tgtaccattg gtacgtatcg tcaacttcat gggaatccat 420
gatactctct gggcagttat cttgcctttg attggatggc cattcggtgt cttcctcatg 480
aaacagttca gtgaaaatat ccctacagag ttgcttgaat cagctaaaat cgacggttgt 540
ggtgagattc gtaccttctg gagtgtagcc ttcccgattg tgaaaccagg gtttgcagcc 600
cttgcaatct ttaccttcat caatacttgg aatgactact tcatgcaatt ggtaatgttg 660
acttcacgta acaatttgac catctcactt ggggttgcga ccatgcaggc tgaaatggca 720
accaactatg gtttgattat ggcaggagct gcccttgctg ctgttccaat cgtcacagtc 780
ttcctagtct tccaaaaatc cttcacacag ggtattacta tgggagcggt caaaggataa 840

<210> 337
<211> 1890
<212> DNA
<213> Streptococcus pneumoniae

<400> 337
atgaaaaaaa cttttttctt actggtgtta ggcttgtttt gccttcttcc actctctgtt 60

tttgccattg atttcaagat aaactcttat caaggggatt tgtatattca tgcagacaat 120
acggcagagt ttagacagaa gatagtttac cagtttgagg aggactttaa gggccaaatc 180
gtgggacttg gacgtgctgg taagatgcct agcgggtttg acattgaccc tcatccaaag 240
attcaggccg cgaaaaacgg tgcagaacta gcagatgtga ctagcgaagt aacagaagaa 300
gcggatggtt atactgtgag agtctataat ccaggtcagg agggcgacat agttgaagtt 360
gacctcgtct ggaacttaaa aaatttactt ttcctttatg atgatatcgc tgaattaaat 420
tggcaacctc tgacagatag ttcagagtct attgaaaagt ttgaatttca tgtaagggga 480
gacaaggggg ctgaaaaact ctttttccat acagggaaac tttttagaga gggaacgatt 540
gaaaagagta accttgatta tactatccgt ttagacaatc ttccggctaa gcgtggagtt 600
gagttgcatg cctattggcc tcggaccgat tttgctagcg ctagggatca gggattgaaa 660
gggaatcgtt tagaagagtt taataagata gaagactcga ttgttagaga aaaagatcag 720
agtaaacaac tcgttacttg ggtcctccct tcgatccttt ccatctcctt gttattgagt 780
gtctgcttct attttattta tagaagaaag accactcctt cagtcaaata tgccaaaaat 840
catcgtctct atgaaccacc aatggaatta gagcctatgg ttttatcaga agcagtctac 900
tcgacctcct tggaggaagt gagtcccttg gtcaagggag ctggaaaatt cacctttgat 960
caacttattc aagctacctt gctagatgtg atagaccgtg ggaatgtctc tatcatttca 1020
gaaggagatg cagttggttt gaggctagta aaagaagatg gtttgtcaag ctttgagaaa 1080
gactgcctaa atctagcttt ttcaggtaaa aaagaagaaa ctctttccaa tttgtttgcg 1140
gattacaagg tatctgatag tctttatcgt agagccaaag tttctgatga aaaacggatt 1200
caagcaagag ggcttcaact caaatcttct tttgaagagg tattgaacca gatgcaagaa 1260
ggagtgagaa aacgagtttc cttctggggg ctcccagatt attatcgtcc tttaactggt 1320
ggggaaaagg ccttgcaagt gggtatgggt gccttgacta tcctgcccct atttatcgga 1380
tttggtttgt tcttgtacag tttagacgtt catggctatc tttacctccc tttgccaata 1440
cttggttttc tagggttagt tttgtctgtt ttctattatt ggaagcttcg actagataat 1500
cgtgatggtg ttctaaatga agcgggagct gaggtctact atctctggac cagttttgaa 1560
aatatgttgc gtgagattgc acgattggat caggctgaac tggaaagtat tgtggtctgg 1620
aatcgcctct tggtctatgc gaccttattt ggctatgcgg acaaggttag tcatttgatg 1680
aaggttcatc agattcaagt ggaaaatcca gatatcaatc tctatgtagc ttatggctgg 1740
cacagtacgt tttatcattc aacagcacaa atgagccatt atgctagtgt cgcaaataca 1800
gcaagcacct actctgtatc ttctggaagt ggaagttctg gtggtggctt ctctggaggc 1860
ggaggtggcg gcagtatcgg tgccttttaa 1890

<210> 338
<211> 2661
<212> DNA
<213> Streptococcus pneumoniae

<400> 338
atgaaaaaag taagaaagat atttcagaag gcagttgcag gactgtgctg tatatctcag 60
ttgacagctt tttcttcgat agttgcttta gcagaaacgc ctgaaaccag tccagcgata 120
ggaaaagtag tgattaagga gacaggcgaa ggaggagcgc ttctaggaga tgccgtcttt 180
gagttgaaaa acaatacgga tggcacaact gtttcgcaaa ggacagaggc gcaaacagga 240
gaagcgatat tttcaaacat aaaacctggg acatacacct tgacagaagc ccaacctcca 300
gttggttata aaccctctac taaacaatgg actgttgaag ttgagaagaa tggtcggacg 360
actgtccaag gtgaacaggt agaaaatcga gaagaggctc tatctgacca gtatccacaa 420
acagggactt atccagatgt tcaaacacct tatcagatta ttaaggtaga tggttcggaa 480
aaaaacggac agcacaaggc gttgaatccg aatccatatg aacgtgtgat tccagaaggt 540
acactttcaa agagaattta tcaagtgaat aatttggatg ataaccaata tggaatcgaa 600
ttgacggtta gtgggaaaac agtgtatgaa caaaaagata agtctgtgcc gctggatgtc 660
gttatcttgc tcgataactc aaatagtatg agtaacattc gaaacaagaa tgctcgacgt 720
gcggaaagag ctggtgaggc gacacgttct cttattgata aaattacatc tgattcagaa 780
aatagggtag cgcttgtgac ttatgcttcc actatctttg atgggaccga gtttacagta 840
gaaaaagggg tagcagataa aaacggaaag cgattgaatg attctctttt ttggaattat 900
gatcagacga gttttacaac caataccaaa gattatagtt atttaaagct gactaatgat 960
aagaatgaca ttgtagaatt aaaaaataag gtacctaccg aggcagaaga ccatgatgga 1020
aatagattga tgtaccaatt cggtgccact tttactcaga aagctttgat gaaggcagat 1080
gagattttga cacaacaagc gagacaaaat agtcaaaaag tcattttcca tattacggat 1140
ggtgtcccaa ctatgtcgta tccgattaat tttaatcatg ctacgtttgc tccatcatat 1200
caaaatcaac taaatgcatt ttttagtaaa tctcctaata aagatggaat actattaagt 1260
gattttatta cgcaagcaac tagtggagaa catacaattg tacgcggaga tgggcaaagt 1320

taccagatgt ttacagataa gacagtttat gaaaaaggtg ctcctgcagc tttcccagtt 1380
aaacctgaaa aatattctga aatgaaggcg gctggttatg cagttatagg cgatccaatt 1440
aatggtggat atatttggct taattggaga gagagtattc tggcttatcc gtttaattct 1500
aatactgcta aaattaccaa tcatggtgac cctacaagat ggtactataa cgggaatatt 1560
gctcctgatg ggtatgatgt ctttacggta ggtattggta ttaacggaga tcctggtacg 1620
gatgaagcaa cggctactag ttttatgcaa agtatttcta gtaaacctga aaactatacc 1680
aatgttactg acacgacaaa aatattggaa cagttgaatc gttatttcca caccatcgta 1740
actgaaaaga aatcaattga gaatggtacg attacagatc cgatgggtga gttaattgat 1800
ttgcaattgg gcacagatgg aagatttgat ccagcagatt acactttaac tgcaaacgat 1860
ggtagtcgct tggagaatgg acaagctgta ggtggtccac aaaatgatgg tggtttgtta 1920
aaaaatgcaa aagtgctcta tgatacgact gagaaaagga ttcgtgtaac aggtctgtac 1980
cttggaacgg atgaaaaagt tacgttgacc tacaatgttc gtttgaatga tgagtttgta 2040
agcaataaat tttatgatac caatggtcga acaaccttac atcctaagga agtagaacag 2100
aacacagtgc gcgacttccc gattcctaag attcgtgatg tgcggaagta tccagaaatc 2160
acaatttcaa aagagaaaaa acttggtgac attgagttta ttaaggtcaa taaaaatgat 2220
aaaaaaccac tgagaggtgc ggtctttagt cttcaaaaac aacatccgga ttatccagat 2280
atttatggag ctattgatca aaatggcact tatcaaaatg tgagaacagg tgaagatggt 2340
aagttgacct ttaaaaatct gtcagatggg aaatatcgat tatttgaaaa ttctgaacca 2400
gctggttata aacccgttca aaataagcct atcgttgcct tccaaatagt aaatggagaa 2460
gtcagagatg tgacttcaat cgttccacaa gatataccag cgggttacga gtttacgaat 2520
gataagcact atattaccaa tgaacctatt cctccaaaga gagaatatcc tcgaactggt 2580
ggtatcggaa tgttgccatt ctatctgata ggttgcatga tgatgggagg agttctatta 2640
tacacacgga aacatccgta a 2661

<210> 339
<211> 1998
<212> DNA
<213> Streptococcus pneumoniae

<400> 339
atgaaatcaa tcaacaaatt tttaacaatg cttgctgcct tattactgac agcgagtagc 60
ctgttttcag ctgcaacagt ttttgcggct gggacgacaa caacatctgt taccgttcat 120
aaactattgg caacagatgg ggatatggat aaaattgcaa atgagttaga aacaggtaac 180
tatgctggta ataaagtggg tgttctacct gcaaatgcaa aagaaattgc cggtgttatg 240
ttcgtttgga caaatactaa taatgaaatt attgatgaaa atggccaaac tctaggagtg 300
aatattgatc cacaaacatt taaactctca ggggcaatgc cggcaactgc aatgaaaaaa 360
ttaacagaag ctgaaggagc taaatttaac acggcaaatt taccagctgc taagtataaa 420
atttatgaaa ttcacagttt atcaacttat gtcggtgaag atggagcaac cttaacaggt 480
tctaaagcag ttccaattga aattgaatta ccattgaacg atgttgtgga tgcgcatgtg 540
tatccaaaaa atacagaagc aaagccaaaa attgataaag atttcaaagg taaagcaaat 600
ccagatacac cacgtgtaga taaagataca cctgtgaacc accaagttgg agatgttgta 660
gagtacgaaa ttgttacaaa aattccagca cttgctaatt atgcaacagc aaactggagc 720
gatagaatga ctgaaggttt ggcattcaac aaaggtacag tgaaagtaac tgttgatgat 780
gttgcacttg aagcaggtga ttatgctcta acagaagtag caactggttt tgatttgaaa 840
ttaacagatg ctggtttagc taaagtgaat gaccaaaacg ctgaaaaaac tgtgaaaatc 900
acttattcgg caacattgaa tgacaaagca attgtagaag taccagaatc taatgatgta 960
acatttaact atggtaataa tccagatcac gggaatactc caaagccgaa taagccaaat 1020
gaaaacggcg atttgacatt gaccaagaca tgggttgatg ctacaggtgc accaattccg 1080
gctggagctg aagcaacgtt cgatttggtt aatgctcaga ctggtaaagt tgtacaaact 1140
gtaactttga caacagacaa aaatacagtt actgttaacg gattggataa aaatacagaa 1200
tataaattcg ttgaacgtag tataaaaggg tattcagcag attatcaaga aatcactaca 1260
gctggagaaa ttgctgtcaa gaactggaaa gacgaaaatc caaaaccact tgatccaaca 1320
gagccaaaag ttgttacata tggtaaaaag tttgtcaaag ttaatgataa agataatcgt 1380
ttagctgggg cagaatttgt aattgcaaat gctgataatg ctggtcaata tttagcacgt 1440
aaagcagata aagtgagtca agaagagaag cagttggttg ttacaacaaa ggatgcttta 1500
gatagagcag ttgctgctta taacgctctt actgcacaac aacaaactca gcaagaaaaa 1560
gagaaagttg acaaagctca agctgcttat aatgctgctg tgattgctgc caacaatgca 1620
tttgaatggg tggcagataa ggacaatgaa aatgttgtga aattagtttc tgatgcacaa 1680
ggtcgctttg aaattacagg ccttcttgca ggtacatatt acttagaaga aacaaaacag 1740
cctgctggtt atgcattact aactagccgt cagaaatttg aagtcactgc aacttcttat 1800

tcagcgactg gacaaggcat tgagtatact gctggttcag gtaaagatga cgctacaaaa 1860
gtagtcaaca aaaaaatcac tatcccacaa acgggtggta ttggtacaat tatctttgct 1920
gtagcggggg ctgcgattat gggtattgca gtgtacgcat atgttaaaaa caacaaagat 1980
gaggatcaac ttgcttaa 1998

<210> 340
<211> 1173
<212> DNA
<213> Streptococcus pneumoniae

<400> 340
atgacaatgc agaaaatgca gaaaatgatt agtcgtatct tctttgttat ggctctgtgt 60
ttttctcttg tatggggtgc acatgcagtc caagcgcaag aagatcacac gttggtcttg 120
caattggaga actatcagga ggtggttagt caattgccat ctcgtgatgg tcatcggttg 180
caagtatgga agttggatga ttcgtattcc tatgatgatc gggtgcaaat tgtaagagac 240
ttgcattcgt gggatgagaa taaactttct tctttcaaaa agacttcgtt tgagatgacc 300
ttccttgaga atcagattga agtatctcat attccaaatg gtctttacta tgttcgctct 360
attatccaga cggatgcggt ttcttatcca gctgaatttc tttttgaaat gacagatcaa 420
acggtagagc ctttggtcat tgtagcgaaa aaaacagata caatgacaac aaaggtgaag 480
ctgataaagg tggatcaaga ccacaatcgc ttggagggtg tcggctttaa attggtatca 540
gtagcaagag atgtttctga aaaagaggtt cccttgattg gagaataccg ttacagttct 600
tctggtcaag tagggagaac tctctatact gataaaaatg gagagatttt tgtgacaaat 660
cttcctcttg ggaactatcg tttcaaggag gtggagccac tggcaggcta tgctgttacg 720
acgctggata cggatgtcca gctggtagat catcagctgg tgacgattac ggttgtcaat 780
cagaaattac cacgtggcaa tgttgacttt atgaaggtgg atggtcggac caatacctct 840
cttcaagggg caatgttcaa agtcatgaaa gaagaaagcg gacactatac tcctgttctt 900
caaaatggta aggaagtagt tgtaacatca gggaaagatg gtcgtttccg agtggaaggt 960
ctagagtatg ggacatacta tttatgggag ctccaagctc caactggtta tgttcaatta 1020
acatcgcctg tttcctttac aatcgggaaa gatactcgta aggaactggt aacagtggtt 1080
aaaaataaca agcgaccacg gattgatgtg ccagatacag gggaagaaac ccttgtatat 1140
cttgatgctt gttgccattt tgttgtttgg tag 1173

<210> 341
<211> 1566
<212> DNA
<213> Streptococcus pneumoniae

<400> 341
atgagccaca tatacttatc tattttcaca agtctcttgc tgatgctagg acttgtcaat 60
gttgctcaag ccgatgaata tttacgcatc ggtatggaag cagcatatgc tccctttaac 120
tggacccagg atgatgatag caacggagct gtcaaaatcg atgggaccaa tcagtatgcc 180
aacggatacg atgttcaaat cgccaagaaa atcgctaagg acttaggtaa agaacctttg 240
gttgttaaaa ccaagtggga aggtctagtc cctgccctta cttctggtaa gattgacatg 300
attatcgcag gtatgagtcc aactgcagaa cgcaaacaag aaattgcctt ttcgagcagt 360
tactatacta gcgaaccagt tttgcttgtc aaaaaagatt ctgcctacgc aagtgctaaa 420
tctttggatg actttaacgg tgcaaaaatc acttctcaac aaggggtcta cctttataac 480
ttgattgcac aaatcccagg tgctaaaaaa gaaacagcca tgggagactt cgctcaaatg 540
cgacaagctc ttgaggctgg tgtcattgat gcttatgttt ctgaacgtcc agaagcactg 600
actgctgaag ctgcgaactc taagttcaag atgattcaag tagaacctgg tttcaaaact 660
ggggaagaag atacagctat cgctatcggg cttcgtaaaa atgacaatcg tattagccaa 720
atcaatgcca gcattgaaac catttcaaaa gatgaccaag ttgccttgat ggatcgtatg 780
atcaaggaac aacctgccga agctacaaca actgaagaga ctagcagtag tttctttagc 840
caagttgcta aaattctttc tgaaaactgg caacaactct tgcgtggtgc tggtatcact 900
cttttaatct ctatcgtcgg aaccatcata ggtctcatta ttggacttgc cattggtgtc 960
ttccgtactg ctcctctctc tgaaaacaaa gtcatttacg gcctacaaaa actagtcggc 1020
tgggttctca atgtctacat tgaaattttc cgtggtacgc caatgattgt tcaatcgatg 1080
gttatctact atggaactgc ccaagctttc gggatcaacc ttgaccgtac actggctgct 1140
atcttcatcg tttcaatcaa taccggtgcc tacatgactg aaatcgtccg tggtggtatc 1200
ctagcagttg acaaggacaa atttgaagct gcgactgctc ttggtatgac ccataaccag 1260

accatgcgta agattgtcct acctcaggta gtccgtaaca tcctacctgc aactggtaat 1320
gaatttgtca tcaatatcaa agatacatct gtattgaacg ttatctctgt tgtcgaactt 1380
tatttctcag gaaataccgt ggcaacacaa acctatcaat acttccagac atttacaatc 1440
atcgccgtga tttactttgt cctcaccttc accgtaacac gtatcctacg ctttatcgag 1500
cgcagaatgg acatggatac ctacactaca ggtgctaacc aaatgcaaac ggaggatttg 1560
aaataa 1566

<210> 342
<211> 741
<212> DNA
<213> Streptococcus pneumoniae

<400> 342
atgacacaag caatccttga aattaaacac ctcaaaaaat cctatggaca aaacgaagtg 60
ctaaaagaca tttcactcac tgtccacaag ggagaggtca tctctatcat cggaagctct 120
ggaagcggaa aatcgacctt cctacgctcc attaacctac ttgaaacacc aactgatgga 180
caaatccttt atcatggaca aaacgtcctc gaaaaaggct atgacctcac gcaataccgt 240
gaaaagttgg ggatggtttt ccaatccttt aacctctttg aaaatctcaa tgttcttgaa 300
aacacaatcg tcgctcagac aactgtccta aaacgcgaac gcacagaagc tgaaaagatt 360
gccaaagaaa acctggaaaa ggtcggcatg ggagaacgct actggcaagc caaaccaaaa 420
caactctcag gtggtcaaaa acaacgtgtg gccatcgctc gtgccctctc catgaatccg 480
gacgctattc tctttgatga accaacatca gctctcgatc cagaaatggt tggagaagtc 540
ctcaaaatca tgcaggacct ggctcaggaa ggcttgacca tgattgtcgt aacccatgaa 600
atggaatttg cccgtgatgt ctctcaccgt gttatcttta tggataaggg cgtgatcgct 660
gaagaaggta aaccagaaga cctcttcacc aatcctaaag aagaccgaac aaaagagttc 720
cttcaacgct atctcaaata a 741

<210> 343
<211> 690
<212> DNA
<213> Streptococcus pneumoniae

<400> 343
atgaaaaagt atcaacttct attcaaaata agtgcagtct tctcttactt atttttcgta 60
tttagtcttt ctcagctgac gcttatcgtc caaaactatt ggcaattttc ttctcagata 120
ggcaatttat tctggattca aaatatcttg agtttacttt ttattggagt catgattgtg 180
gttcttgtta agacaggcca tggttatctc ttccgcattc caagaaaaaa atggctttgg 240
tattcgattt tgacagtatt agtgctagtg ttccagatct cttttaacgt tcagacagct 300
aaacatgttc agtcaactgc ggaaggttgg gctgtattga ttggttatag tgggactaac 360
tttgcagagc taggtattta tatagccctg ttctttctgg ttccactgat ggaagaattg 420
atttatagag gattactgca acatgctttc tttaagcatt cgcgatttgg tcttgatttg 480
cttcttcctt ctattttatt tgctctccct catttttcaa gcctgcctag tctgttagat 540
atcttcgtct ttgcaacagt tggaatcatc tttgctggtt tgacccgcta taccaagagc 600
atttatccat cctatgcggt gcatgtgatc aataatattg tagcgacctt cccgtttttg 660
ctcacttttc tacatagggt cttggggtaa 690

<210> 344
<211> 1040
<212> DNA
<213> Streptococcus pneumoniae

<400> 344
atgaacaaga aacaatggct aggtcttggc ctagttgcag tggcagcagt tggacttgct 60
gcatgtggta accgctcttc tcgtaacgca gcttcatctt ctgatgtgaa gacaaaagca 120
gcaatcgtca ctgatactgg tggtgttgat gacaaatcat tcaaccaatc agcttgggaa 180
ggtttgcagg cttggggtaa agaacacaat ctttcaaaag ataacggttt cacttacttc 240
caatcaacaa gtgaagctga ctacgctaac aacttgcaac aagcggctgg aagttacaac 300
ctaatcttcg gtgttggttt tgcccttaat aatgcagtta aagatgcagc aaaagaacac 360

actgacttga actatgtctt gattgatgat gtgattaaag accaaaagaa tgttgcgagc 420
gtaactttcg ctgataatga gtcaggttac cttgcaggtg tggctgcagc aaaaacaact 480
aagacaaaac aagttggttt tgtaggtggt atcgaatctg aagttatctc tcgttttgaa 540
gcaggattca aggctggtgt tgcgtcagta gacccatcta tcaaagtcca agttgactac 600
gctggttcat ttggtgatgc ggctaaaggt aaaacaattg cagccgcaca atacgcagcc 660
ggtgcagata ttgtttacca agtagctggt ggtacaggtg caggtgtctt tgcagaggca 720
aaatctctca acgaaagccg tcctgaaaat gaaaaagttt gggttatcgg tgttgatcgt 780
gaccaagaag cagaaggtaa atacacttct aaagatggca aagaatcaaa ctttgttctt 840
gtatctactt tgaaacaagt tggtacaact gtaaaagata tttctaacaa ggcagaaaga 900
ggagaattcc ctggcggtca agtgatcgtt tactcattga aggataaagg ggttgacttg 960
gcagtaacaa acctttcaga agaaggtaaa aaagctgtcg aagatgcaaa agctaaaatc 1020
cttgatggaa gcgtaaaagt 1040

<210> 345
<211> 1059
<212> DNA
<213> Streptococcus pneumoniae

<400> 345
atgtctaaaa aattacaaca aatttcggtt cccttgattt ctgtattcct aggaatttta 60
ctcggagcca ttgtcatgtg gatcttcggt tatgatgcta tttggggcta cgaagaattg 120
ttctatacag cctttggcag tctgcgtggg attggagaaa tcttccgtgc tatgggtcct 180
ctggtcttga ttggtcttgg ttttgccgtt gccagtcgag ctggtttctt taacgtcgga 240
cttcctggtc aggctttggc aggttggatt ctcagtggtt ggtttgccct gtcgcatcca 300
gatatgcccc gtcccttgat gattctagca accatcgtga ttgccttgat tgctggtggg 360
attgtcggag cgattccagg tattcttagg gcctatctag ggacgtcaga ggttattgta 420
accatcatga tgaactacat tgtcttgtat gtagggaatg cctttatcca tgctttccct 480
aaagacttca tgcaaagtac agattcgacc attcgtgttg gggctaatgc aacctatcag 540
acaccttggt tggctgagtt gactggtaac tcacggatga atattggtat tttctttgcc 600
atcattgccg ttgcagttat ttggttcatg ctcaagaaaa caactcttgg ttttgaaatc 660
cgtgcagttg gtcttaatcc acatgcttca gaatatgctg gtatttctgc caagcggact 720
attatcctat ctatgattat ttcaggtgcc ttggcaggtc ttggtggagc tgttgaaggt 780
ttgggaacct tccagaacgt ctatgttcaa ggttcgtcat tagctatcgg atttaacgga 840
atggcggtta gtttgcttgc ggccaactca ccaattggta tactctttgc agccttccta 900
tttggcgttc tccaagttgg ggctcctggt atgaatgcgg cgcaggtacc atctgagctt 960
gtcagcattg taacagcgtc tattatcttc tttgtcagtg ttcattacct tatcgaacgc 1020
tttgtcaaac cgaaaaaaca agttaaagga ggtaagtaa 1059

<210> 346
<211> 831
<212> DNA
<213> Streptococcus pneumoniae

<400> 346
atgggagtga aaaagaaact aaagttgact agtttgctag gactgtctct gttaatcatg 60
acagcctgtg cgactaatgg ggtaactagc gatattacag ccgaatcggc tgatttttgg 120
agtaaattgg tttacttctt tgcggaaatc attcgctttt tatcgtttga tattagtatc 180
ggagtgggga ttattctctt tacggtcttg attcgtacag tcctcttgcc agtctttcag 240
gtgcaaatgg tggcttctag gaaaatgcag gaagctcagc cacgcattaa ggcgcttcga 300
gaacaatatc caggtcgaga tatggaaagc agaaccaaac tagagcagga aatgcgtaaa 360
gtatttaaag aaatgggtgt cagacagtca gactctcttt ggccgatttt gattcagatg 420
ccggttattt tggccctgtt ccaagcccta tcaagagttg actttttaaa gacaggtcat 480
ttcttatgga ttaaccttgg tagtgtggat acaacccttg ttcttccgat tttagcagca 540
gtattcacct ttttaagtac ttggttgtcc aacaaagctt tgtctgagcg aaatggcgct 600
acgactgcga tgatgtatgg gattccagtc ttgattttta tctttgcagt ttatgcgcca 660
ggtggagtcg ccctatactg gacagtgtct aatgcttatc aagtcttgca aacctatttc 720
ttgaataatc cattcaagat tatcgcagag cgcgaggccg tagtacaggc acaaaaagat 780
ttggaaaata gaaaaagaaa agccaagaaa aaggctcaga aaacgaaata a 831

<210> 347
<211> 795
<212> DNA
<213> Streptococcus pneumoniae

<400> 347
atggttatcg atccatttgc tatcaacgaa ctagactatt acttagtttc acacttccac 60
agtgatcata tcgacccata cacagctgca gcaattctca ataatcctaa gttagagcat 120
gttaagttta tcggtcctta ccactgtgga cgaatctggg aaggatgggg tgttccaaaa 180
gaacgtatca tcgttgttaa accaggtgac actatcgaat taaaagatat gaagattcat 240
gcagtagaat catttgaccg tacttgcttg gtaactctcc cagtgaacgg tgctgatgag 300
acaggcggtg aacttgctgg cttggctgtt acagatgaag aaatggctca aaaggctgtt 360
aactatatct ttgaaacacc aggtggaacc atctatcatg gtgcagattc tcacttctca 420
aactattttg caaaacatgg taaagacttt aaaattgatg ttgctttgaa taactatggt 480
gaaaatccgg taggtatcca agacaaaatg acatctatcg accttcttcg tatggcagaa 540
aatctgcgta ccaaagtcat tatcccagtt cactatgata tctggtctaa cttcatggct 600
tctactaatg agattctaga actttggaaa atgcgaaaag atcgcttgca atacgatttc 660
catccattta tctgggaagt tggcggtaag tacacttatc ctcaagatca acacttagta 720
gaataccatc atccacgtgg ttttgatgat tgttttgaac aagactctaa cattcaattt 780
aaagctttgc tataa 795

<210> 348
<211> 585
<212> DNA
<213> Streptococcus pneumoniae

<400> 348
atgttccttt caggctggtt gtctagtttt gctaatactt atatccatga tttactgggg 60
gttcttttcc cagatagtcc atttttaaat gcctttgaaa gtgctattgc ggctcctttg 120
gtagaagaac ccttgaaatt attgtcactt gtttttgttt tggctttgat tcctgtgcga 180
aaattaaaat ctttgttttt acttggaatt gcttccggtt tgggattcca aatgattaag 240
gatattggtt atattcgtac ggatttgcca gagggctttg actttactat ttcgcgaatt 300
ttagagcgta tcatctcagg aattgcctct cactggactt tttcaggtct agctgtagta 360
ggtgtttact tgctttacag agcctataaa ggacagaagg ttggcaagaa acagggcctt 420
atttttctag gtttagcctt gggaactcac ttcttgttta actctccttt tgtggagttg 480
gaaacagagt tgcctttagc gattccagtg gttacggcta ttgctctcta tggtttttat 540
catgcttatt gctttgttga gaaacacaat gagttgatga cctag 585

<210> 349
<211> 390
<212> DNA
<213> Streptococcus pneumoniae

<400> 349
atgaaggtgg aaccacgttg cgacgtcctt tcgaggatgt cgcatttttt tattaggata 60
ctaattatgg agttgcaaga attagtggag cgcagttggg caatccgaca agcttatcac 120
gaactggaag ttaagcatca tgattccaag tggacggtag aagaagacct cttggcttta 180
tctaatgata ttggaaattt ccaacgactg gtgatgacaa agcaaggacg ctactatgat 240
gaaacaccct acacactgga acaaaaactt tcagaaaata tctggtggct attagaactt 300
tctcaacgtt tggatataga cattctgacg gaaatggaaa acttcctctc tgataaagaa 360
aagcaattga acgttaggac ttggaagtag 390

<210> 350
<211> 975
<212> DNA
<213> Streptococcus pneumoniae

<400> 350
atgcttgatt ggaaacaatt ttttctagcc tatctgcgct cccgtagtcg tctttttatc 60
tatctgcttt ctttggcatt tcttgtctta ctctttcagt ttttatttgc cagtctagga 120
atttacttcc tctacttttt cttcttgtgt tgctttgtaa ccatattatt tttcacttgg 180
gacatattgg tggaaacgca ggtctatcgc caggaacttc tctatggaga gagggaagcc 240
aagtctcctt tggaaatagc tttagcagaa aaattagaag cgcgtgagat ggaactctat 300
cagcagaggt caaaagcaga aagaaaactg acggatttgc tggattacta taccttgtgg 360
gtccatcaga taaagacccc cattgcagcc agtcaactct tagttgcaga agtggtcgac 420
cgccaactga agcagcagct agaacaggaa attttcaaaa tcgactccta taccaaccta 480
gttttacagt acctgcgttt agaaagtttc catgatgatt tggtcttaaa gcaggttcaa 540
attgaggact tggtcaagga aataattcgt aaatatgctc ttttctttat tcaaaaaggc 600
ttaaatgtca atctacatga ccttgataaa gaaatcgtga cggataaaaa gtggctgcta 660
gtggttattg agcaaatcat ctcaaacagt ctcaagtaca ccaaggaagg tggtctggag 720
atttatatgg atgaccaaga gctttgtatc aaagatacgg gaatcgggat aaaaaacagt 780
gatgtcctcc gagtatttga acgtggcttt tcaggataca atggccgttt gacccagcag 840
tcctctggac ttggccttta tctatctaag aaaatttctg aagaactggg gcaccagatt 900
cgtatcgagt ctgaggtcgg aaaaggaacg acagtgcgga ttcagtttgc tcaagtgaac 960
ttagtccttg agtaa 975

<210> 351
<211> 588
<212> DNA
<213> Streptococcus pneumoniae

<400> 351
atggaactta atacacacaa tgctgaaatc ttgctcagtg cagctaataa gtcccactat 60
ccgcaggatg aactgccaga gattgcccta gcagggcgtt caaatgttgg taaatccagc 120
tttatcaaca ctatgttgaa ccgtaagaat ctcgcccgta catcaggaaa acctggtaaa 180
acccagctcc tgaacttttt taacattgat gacaagatgc gctttgtgga tgtgcctggt 240
tatggctatg ctcgtgtttc taaaaaggaa cgtgaaaagt gggggtgcat gattgaggag 300
tacttaacga ctcgggaaaa tctccgtgcg gttgtcagtc tagttgacct tcgtcatgac 360
ccgtcagcag atgatgtgca gatgtacgaa tttctcaagt attatgagat tccagtcatc 420
attgtggcga ccaaggcgga caagattcct cgtggtaaat ggaacaagca tgaatcagca 480
atcaaaaaga aattaaactt tgacccgagt gacgatttca tcctcttttc atctgtcagt 540
aaggcaggga tggatgaggc ttgggatgca atcttagaaa aattgtga 588

<210> 352
<211> 891
<212> DNA
<213> Streptococcus pneumoniae

<400> 352
atgacaaaga aacaacttca cttggtgatt gtgacaggga tgagtggcgc agggaaaact 60
gtagccattc agtccttcga ggatctaggt tatttcacca ttgataatat gccgccagct 120
ctcttgccta agtttttgca gctggttgaa attaaggaag acaatcctaa gttggccttg 180
gtagtggata tgcgtagccg ttctttcttt tcagagattc aagctgtttt ggatgagttg 240
gaaaatcaag atggtttgga tttcaaaatc ctctttttgg atgcggctga taaggaattg 300
gtcgctcgtt acaaggaaac cagacggagt cacccactag cagcagacgg tcgtatttta 360
gatggaatca agttggaacg tgaactcttg gcacctttga aaaatatgag ccaaaatgtg 420
gtggatacga ctgaactcac tccacgtgag ctgcgcaaaa cccttgcaga gcagttttca 480
gaccaagaac aagcccagtc tttccgtatc gaagtcatgt ctttcggatt taagtatgga 540
atcccgattg atgcggactt ggtctttgat gtccgtttct tgccaaatcc ctattattta 600
ccagaactga gaaaccaaac gggtgtggat gaacctgttt atgattatgt catgaaccat 660
cctgagtcag aagactttta tcaacattta ttggccttga ttgagccgat tctgccaagt 720
taccaaaagg aaggtaagtc cgttttgacc attgccatgg gatgtacggg tggacaacac 780
cgtagtgtgg catttgctaa acgcttggcg caggacttat ccaagaattg gtctgttaat 840
gaagggcatc gcgacaaaga ccgcagaaag gaaacggtaa accgttcatg a 891

<210> 353
<211> 978
<212> DNA
<213> Streptococcus pneumoniae

<400> 353
atgagaaaac caaagataac ggtgattggt ggagggactg gaagtcccgt cattctaaaa 60
agtctgcggg aaaaagatgt ggaaatcgca gctatcgtga cggtggcaga tgatggtggt 120
tcttcaggtg aactccgaaa aaatatgcaa cagttgacac cgccaggtga tcttcgtaat 180
gtccttgtgg ccatgtcgga tatgcctaag ttttatgaga aggtctttca gtatcggttc 240
tctgaggatg ccggagcctt tgctggccat ccattgggaa atctcatcat tgctggcttg 300
tcagaaatgc agggttcaac ctataatgcc atgcagttat tgagcaaatt tttccataca 360
acagggaaaa tttatccttc cagtgaccat cctttgaccc ttcatgcagt ctttcaggat 420
gggacagaag tggctggaga gagtcatatt gtagaccatc gaggcataat tgacaatgtc 480
tatgtgacca atgccctaaa cgatgatacg cctctggcca gccgtcgagt agtgcagacc 540
atccttgaaa gtgacatgat tgtcctaggg ccaggttccc tctttacctc tattttgccc 600
aatatcgtga ttaaggaaat tgggcgggct cttttggaaa ccaaggcaga aattgcctat 660
gtctgcaata tcatgaccca acgtggggag acggaacact ttacagatag cgaccacgtg 720
gaagtcttgc atcgtcacct tggtcgccct tttatcgaca ctgtcttggt gaatattgaa 780
aaagtgcctc aggaatacat gaattccaac cgttttgatg aatacttagt gcaagtggaa 840
cacgattttg taggtctttg taagcaagtt tcgcgcgtga tttcatctaa cttccttcgt 900
ctggaaaatg gcggtgcctt ccacgatgga gatttgattg tggacgagtt gatgcgcatt 960
atacaggtga aaaaatga 978

<210> 354
<211> 1179
<212> DNA
<213> Streptococcus pneumoniae

<400> 354
atgaaaaatt tgataaagtt gctaataatt agattgattg ttaacttagc agacagtgta 60
ttttatatag tagcattgtg gcacgttagc aataattatt cttcgagcat gttcttagga 120
atatttattg cagtaaatta tctaccggat ttgttactaa tcttttttgg accagttatt 180
gacagagtaa atccgcaaaa aattcttata atatcaattt tggttcaatt agcagtggct 240
gtaatatttt tattattatt aaaccaaata tcattttggg tgataatgag tctagtgttt 300
atttcagtaa tggctagctc cataagttac gtgatagaag atgtgttgat tcctcaagtg 360
gtagaatatg ataagattgt atttgcaaat tctcttttta gtatttcgta taaagtatta 420
gattctattt ttaattcatt cgcatcattt ttacaggtgg cagtaggatt tattttattg 480
gttaagatag atataggcat atttttactt gctctattta tattgttgtt gttaaaattt 540
agaactagca atgcgaatat agaaaacttc tctttcaaat attacaagag agaagtgttg 600
caaggtacaa agtttatttt aaataataaa ttattattta aaaccagtat ttctttaacg 660
cttataaact ttttttattc atttcagaca gtagttgtac cgattttttc tattcgatat 720
tttgatggtc cgatttttta tggtattttt ttaactattg ctggtttggg tggtatattg 780
ggaaatatgc tagcgccaat cgtaataaaa tatttaaaat cgaatcaaat tgttggtgta 840
tttctttttt tgaacggctc aagttggtta gtagcaattg ttataaaaga ctatacttta 900
tcacttattt tatttttcgt ttgttttatg tctaaaggag tcttcaatat tatttttaat 960
tcgttgtacc aacaaatacc tccacatcaa cttcttggta gggtaaatac taccattgat 1020
tctattattt cttttggaat gccaattggt agtttagttg caggaacgct tattgatttg 1080
aatattgaat tagtgttaat tgctattagc ataccttatt ttttgttttc ttatattttt 1140
tatacggata atggattgaa agaatttagt atatattag 1179

<210> 355
<211> 678
<212> DNA
<213> Streptococcus pneumoniae

<400> 355
atgatgtcta acaaaaataa ggaaattctg atttttgcga ttctctatac agtcctcttt 60
atgtttgatg gcgttaaatt gctggcttct ttaatgccat ctgccattgc aaattatctt 120

gtttatgtag ttttagctct atatggctcc ttcttgttca aggatagatt gatccaacaa 180
tggaaggaga ttagaaagac taaaagaaaa ttcttctttg gagtcttaac aggatggctc 240
tttctcattc tgatgactgt tgtctttgaa tttgtatcag agatgttgaa gcagtttgtg 300
ggactagatg gacaaggtct aaatcagtct aatattcaaa gtacctttca agaacaacca 360
ctactgatag ctgtttttgc ttgtgtcatt ggacctctgg tagaagaatt atttttccgt 420
caggtcttat tgcattactt gcaggaacgg ttgtcaggtt tactaagcat tattctggta 480
ggacttgttt ttgctctgac tcatatgcac agtttggctc tatcagagtg gattggtgca 540
gttggttact taggtggagg ccttgccttt tctattattt atgtgaaaga aaaagagaat 600
atctactatc ccctacttgt tcacatgtta agcaacagcc tctccttaat cattttagct 660
atcagtatag taaaatga 678

<210> 356
<211> 1158
<212> DNA
<213> Streptococcus pneumoniae

<400> 356
ttgaaaaagc caattatcga attcaaaaac gtctctaaag tttttgaaga cagcaacacc 60
aaggttctca aagacatcaa ctttgagttg gaagaaggga aattctacac ccttctaggt 120
gcatctggtt cggggaaatc aactatccta aacattattg caggtttact ggatgcgacg 180
acaggagata tcatgctaga cggtgttcgt atcaatgata ttccaaccaa caagcgcgac 240
gtacataccg tcttccaatc ctatgccttg ttcccacata tgaatgtgtt tgaaaatgtt 300
gcctttccac ttcgcttgcg taaaattgat aagaaagaaa tcgagcagcg tgtagcggaa 360
gttctcaaga tggttcagtt ggaaggttat gaaaaacgtt ccatccgcaa actttctgga 420
ggacaacgtc agcgtgtggc catcgcccgt gctatcatca accaaccccg tgtggtcttg 480
ttggacgagc ctttatcagc gctggacttg aaattgagaa cagacatgca gtacgaattg 540
cgtgaattac aacaacgatt gggcattacc tttgtctttg tcactcacga tcaggaagaa 600
gctcttgcca tgagtgactg gattttcgtt atgaatgatg gcgagattgt ccagtctgga 660
acccctgtgg acatctacga tgagccaatc aaccactttg ttgccacctt tatcggggag 720
tcaaacatct tgccaggtac catgattgag gactacttgg tcgaatttaa cggcaaacgc 780
tttgaagcgg ttgatggtgg gatgaagcca aatgaacctg ttgaggtcgt tattcgtcca 840
gaggacttgc gcattaccct tcctgaagaa ggcaagctcc aagttaaggt cgatacccag 900
cttttccgtg gagttcatta tgaaattatc gcctatgacg aacttggaaa tgaatggatg 960
atccactcaa cccgtaaggc tatcgtgggt gaggaaatcg gtctggactt tgaaccagaa 1020
gacatccaca tcatgcgtct caatgaaacc gaagaagagt tcgatgctcg tattgaggag 1080
tacgtagaaa tcgaagagca agaagcaggt ttgatcaatg caatcgagga ggaaagagat 1140
gaagaaaaca agctctaa 1158

<210> 357
<211> 621
<212> DNA
<213> Streptococcus pneumoniae

<400> 357
atgaaatcaa tgagaatctt atttttgtta gctttaattc aaatcagttt gagtagctgt 60
ttcctatgga aggaatgcat cttgtccttt aaacaaagta cagctttttt catcggaagc 120
atggttttcg tttcaggaat ctgtgctgga gtaaattatc tttatacccg taagcaagaa 180
gtccatagtg tcctagccag taagaagtcg gtgaagcttt tttacagtat gttactctta 240
attaatttgt taggagctgt tcttgttttg tcagataact tgttcatcaa aaatacgctg 300
cagcaagaat tagttgactt tttattgcca tccttctttt tcctatttgg gctagatttg 360
ctgatttttt tacccttgaa aaaatacgtg cgcgattttc ttgctatgct ggacagaaaa 420
aagacagtgt tggtgactat tttagcaaca cttcttttct taagaaatcc aatgaccatt 480
gtctcacttc tgatttatat tggactgggc ttgttttttg cagcctatct tgtcccaaat 540
tcggttaaga aggaagtttc cttttatggt catattttcc gagatcttgt attggtcatt 600
gttacgctca ttttctttta g 621

<210> 358
<211> 1017

<212> DNA
<213> Streptococcus pneumoniae

<400> 358
atggttaaaa aaattattgg aatggtgcta gctttacttt ctgtaactgt agtaggagta 60
ggtgtttttg cttatactat ttatcaacaa gggacagaaa ccttagctaa aacctataaa 120
aaaatcggtg aagaaaccaa ggttattgaa gcgactgaac ctctaaccat tctgttaatg 180
ggagtggaca ccggaaatgt tgaacgaact gaaacttggg tcggtagaag tgatagcatg 240
atcttgatga cagtgaatcc taaaacgaaa aaaacaacaa tgatgagttt agagcgggat 300
attctgacgc gcattgaatc agggaatggt caggctcatg aagcgaaact gaactcagca 360
tatgcagatg gtggagcaga gcttgctata gaaaccattc aaaaaatgat gaatatccat 420
attgatcgct atgtgatggt caatatgaga ggattgcaaa aactagtgga tgcagtagga 480
ggtattacag tcaataatat cctaggtttc ccaatttcta tcagtgacca agaagaattt 540
aatactattt ctatcggtgt tggggagcaa catattgggg gagaagaagc cctagtctat 600
gcacgaatgc gttaccaaga tcctgagggg gattatggtc gtcaaaaacg tcaacgtgaa 660
gttattcaaa aagtcatgga aaaagctctc agtttaaata gcattggtca ttatcaagag 720
attctaaaag ctttgagtga caatatgcag accaatattg atttgtctgc aaaaagtatc 780
cctaacttgc taggctataa agattcattt aaaaccattg aaactcagca gttgcagggt 840
gaaggagaga tacttcaagg tgtttcttac cagattgttt cgagagcaca tatgttggaa 900
atgcaaaatc tactccgacg ttctttggga caagaagaag ttactcagct tgaaaccaat 960
gcggttttat ttgaagattt atttggcaga gcacctgttg gtgatgaaga taattaa 1017

<210> 359
<211> 996
<212> DNA
<213> Streptococcus pneumoniae

<400> 359
atgaaaaaac aagcctatgt cattattgct ctcacctcct tcctatttgt cttttttttc 60
tcccacagct tgctggaaat acttgatttt gactggtcta tctttttgca cgatgtcgaa 120
aaaacagaaa aatttgtctt tttattgttg gttttcagca tgtccatgac ctgtctctta 180
gccctgtttt ggcgagggat cgaagagctt tctctaagaa aaatgcaggc taatctcaag 240
cgtttattag cagggcaaga agtggttcag gttgcagatc cagatttgga tgccagtttc 300
aagtccttat caggtaaact taaccttttg acagaggctc ttcaaaaagc tgaaaatcag 360
agccttgctc aggaagagga aatcatcgag aaggaacgga agcgaattgc tcgggatttg 420
cacgatacag tcagtcagga gttgtttgcg gcccacatga ttttatcggg tatcagtcag 480
caggctttga aattggatag agaaaagatg cagacccagt tgcagagtgt cacagctatt 540
ttagaaacag cccagaagga tttgcgggtt ttgctcttgc atttgcgacc agttgaactg 600
gagcagaaga gcttgataga agggattcaa attcttttaa aagagcttga ggacaagagt 660
gatcttaggg ttagtctcaa gcagaatatg acgaaattgc ctaagaaaat cgaggagcat 720
atcttccgta tcctgcaaga gttgattagc aataccctcc gccatgccca ggcatcttgc 780
ctagatgtct acctctatca gacagatgtt gaattgcaac tgaaggtggt ggacaatggg 840
attggtttcc agttagggag cttagacgac ttgagttatg gactgcgaaa tatcaaggag 900
cgggttgaag atatggctgg aacagttcaa ctcttgacag ctcccaagca agggctggcg 960
gttgatatcc gtattcccct gttagataag gaatga 996

<210> 360
<211> 867
<212> DNA
<213> Streptococcus pneumoniae

<400> 360
atgattgttt ccattatttc tcaaggattt gtctgggcta ttctaggtct gggaatcttt 60
atgacattta ggattttaaa ctttccagat atgacgacag aaggttcctt ccctcttggg 120
ggagctgttg ctgtcacttt gataaccaaa ggcgtgaacc catttttagc gacacttgtt 180
gctgtaggag caggttgttt ggctggaatg gcagcaggcc ttctttatac aaaagggaag 240
atcccaacct tgctctcagg gattttggtg atgacttctt gtcactcaat catgctcttg 300
attatgggac gtgcgaattt aggcctgctt ggaaccaagc aaattcagga tgttttgcct 360
tttgattcgg atttgaatca actcttgaca ggtctcatct ttgtgagtat tgttattgct 420

ctcatgctct ttttcttgga cactaaactc ggacaagcct atattgctac aggggataat 480
cctgatatgg ctagaagttt cgggattcat actggacgca tggagctcat gggcttggtc 540
ttatcaaatg gtgtgattgc ccttgcaggt gccctcattg ctcagcaaga aggttatgcc 600
gatgtgtctc gagggatcgg ggttatcgtt gtggggcttg caagtttgat tattggagaa 660
gttattttca agagtttgag cttggcagag cgtttggtta ctatcgttgt aggttctatc 720
gcttatcaat ttttagtgtg ggcagttatc gcacttggct ttaatacaag ttaccttcgt 780
ttatacagtg ccttgatttt agcagtctgc ctcatgattc caacatttaa gcaaacaatc 840
ttgaaaggag ccaagttaag caaatga 867

<210> 361
<211> 1068
<212> DNA
<213> Streptococcus pneumoniae

<400> 361
atgaaaaaaa tgaaagtttg gtctactgta cttgcaacgg gagttgctct tactacactt 60
gctgcttgct ctggaggttc aaattctacg actgcttctt catctgaaga aaaagctgat 120
aaaagtcaag aattagttat ctattcgaac tcagtctcaa atggtcgtgg tgattggtta 180
actgctaaag caaaagaagc tggttttaat ataaaaatgg ttgatatcgc tggcgctcaa 240
ttagcagacc gtgttattgc tgagaagaat aatgcagttg cagatatggt atttggaatt 300
ggtgctgttg attcaaataa aattagagat caaaaattac tagtacagta caagcctaaa 360
tggttagata aaattgatca atctttatca gataaagata attattataa tcctgtgatt 420
gttcaaccat tagttttaat tggggcgcct gatgtaaaag aaatgcctaa agattggact 480
gaattaggta gtaagtataa aggtaaatat tcaatttctg gtcttcaagg aggtacagga 540
cgggcaattc tagcaagtat cttagttcga taccttgatg ataaaggtga attaggtgtt 600
tccgaaaaag gttgggaagt agcaaaagaa tatttgaaaa atgcatacac tcttcaaaag 660
ggagaaagtt caattgttaa gatgttagac aaagaagatc caatacaata tggaatgatg 720
tggggttctg gtgcattagt tggacaaaaa gaacaaaatg ttgttttcaa agttatgact 780
cctgagattg gtgtaccatt tgtaactgaa caaactatgg ttttaagcac tagtaaaaaa 840
caagcgttag ctaaagaatt tattgattgg tttggtcaat cagaaattca agtagaatat 900
agtaagaact ttggatctat tcctgcaaat aaagatgccc tcaaagatct acctgaagat 960
acgaagaaat ttgttgatca agtgaaacca caaaatattg actgggaagc tgttggaaag 1020
catttggatg aatgggtaga aaaagctgaa ttagaatacg tacaataa 1068

<210> 362
<211> 1011
<212> DNA
<213> Streptococcus pneumoniae

<400> 362
atgattaaat ttgataatat tcaaattaaa tatggtgatt ttgttgcaat tgataatctg 60
aatttagata tacatgaagg ggaatttttt acatttcttg ggccttcagg atgtggtaaa 120
tcaactactt tgagagcatt ggtaggtttt ctagatccat catcaggaag tattgaagtt 180
aatggaacag atgtcactca tttggaacct gaaaagcgtg gaattggtat tgtatttcaa 240
tcttatgcgc tatttccaac tatgactgtt tttgataata ttgcatttgg tttaaaagtt 300
aagaaggtag ctccagatgt tattaaagct aaagtatcag cagtggcagc aaaaattaag 360
atctctgatc aacagttaca gcgtaatgta tcagaattat ctgggggtca acaacaaagg 420
gtagcattgg ctcgtgctct ggttcttgaa cctaaaattc tttgtctaga tgaaccattg 480
tcaaaccttg acgcaaaatt acgtgtagat ttgagaaaag agttgaaaag acttcaaaaa 540
gagttaggta ttactacttt atatgttact catgatcaag aggaagcctt gactttatct 600
gatagaattg cagtctttaa caatggatac atcgaacagg tcggtacacc agtagagatt 660
tatcataatt ctcaaactga atttgtatgt gattttattg gagatattaa tgttttgacc 720
gatgaaacag tccacgaagt attattgaaa aatacaagcg ttttcttaga ggataaaaaa 780
ggatacattc gattagagaa agttcgattc aatcgtgaaa ctgaacaaga ttttattcta 840
aaagggacaa ttattgatgt tgagttttct ggagttacaa ttcactatac aataaaagtt 900
tctgaaagtc agattcttaa tgtaacaagt attgatagtc aggctgctat tagatctgtc 960
ggagaaagtg tggaattatt tatcacacca tcagacgttc tgcaatttta a 1011

<210> 363
<211> 1692
<212> DNA
<213> Streptococcus pneumoniae

<400> 363
atgcgtcata aattaaattt aaaagattgg cttattcgtt tagggttaat ctggttctta 60
gtaacattta ttatttatcc aaactttgat ctagtagtga atgtatttgt aaaaggagga 120
gaattttccc ttgatgctgt acatcgtgtt ctaaaatctc agagggcact tcagagtatt 180
atgaacagtt ttaagttagc attttcactc attattacag ttaatgtcgt aggtattctt 240
tgtgttctat ttacagagta ctttgatatt aaaggtgcta aaattttaaa attaggttat 300
atgacctctt taatttatgg aggagtggtt ttagcgactg gatataaatt tgtctatggt 360
ccttatggat tgattacaaa atttttacaa aatgttatcc cttctttaga ccctaactgg 420
tttattgggt atggtgcagt cttattcatt atgacatttt caggaactgc taatcataca 480
ttgtttttaa caaatacaat tcgaagcgtt gactatcaca ctattgaggc tgctcgaaat 540
atgggagcaa aaccatttac tgttttccga aaagtagtgt taccaacctt aattccaact 600
ctatttgcac ttactattat ggtttttctt agtggtttat ctgcagtagc agcacccatg 660
attgttggtg gtaaagaatt tcaaactata aatccaatga ttattacatt tgcagggatg 720
ggaattctcg gtgatttagc tgccctactt gcaattattt taggtattgc aactacaatt 780
ttgcttacta tcatgaataa gatagaaaaa ggtggaaatt atatttctat ctctaagact 840
aaagcgcctc ttaaaaaaca aaaaattgcg tctaagcctt ggaatatcat tgctcacatt 900
gtagcatatg gattgttcac agttttcatg cttccactaa tttttatagt attatactca 960
tttacagatc cagttgcaat tcaaacaggt aacttaacat tatcaaactt tactttagaa 1020
aattatcgct tattctttag taatagtgcg gcattctctc cattcttggt cagctttatt 1080
tattctatta ttgctgcgac aacagcaaca attctcgcag ttgtatttgc tcgtgttgtc 1140
agaaaacata aatctcgttt tgatttctta tttgaatatg gtgctctact tccttggtta 1200
ctaccaagta cacttttagc agtaagttta ttatttactt ttaatcagcc acaatttctt 1260
gtcttgaatc agattttggt aggtagtttg gtaattctac ttattgcata tatagttgta 1320
aaaatcccat tttcttatag aatggtacgt gctattttat ttagtgttga tgatgagatg 1380
gaagatgcag caagaagtat gggtgcttca cctttttata ctatgatgaa ggttatcatt 1440
ccatttattt taccggttgt tctctctgtt attgctttaa actttaactc tttattaact 1500
gacttcgact tatctgtatt cctttaccat cccctagctc aaccattagg tattacgatt 1560
cgatctgcag gtgatgaaac agcaacatct aatgcacaag ctctggtatt tgtttataca 1620
attgttctga tgattatttc tggaacggta ttatacttca cacaaagacc ggggcgtaaa 1680
gtaaggaaat aa 1692

<210> 364
<211> 5
<212> PRT
<213> Artificial Sequence

<220>
<221> SITE
<222> (3)
<223> Xaa=any amino acid

<220>
<223> Description of Artificial Sequence: Cell wall
anchoring motif

<400> 364
Leu Pro Xaa Thr Gly
1               5

<210> 365
<211> 18
<212> DNA
<213> Artificial Sequence

<220>
<223> Description of Artificial Sequence: Primer

<400> 365
gcgggatccg ccaccatg                                                    18


<210> 366
<211> 10
<212> DNA
<213> Artificial Sequence

<220>
<223> Description of Artificial Sequence: Primer

<400> 366
ttgcggccgc                                                             10


<210> 367
<211> 38
<212> DNA
<213> Artificial Sequence

<220>
<223> Description of Artificial Sequence: Primer

<400> 367
cggatccgcc accatgtctt ctaatgaatc tgccgatg                              38


<210> 368
<211> 39
<212> DNA
<213> Artificial Sequence

<220>
<223> Description of Artificial Sequence: Primer

<400> 368
ttgcggccgc ctgtttagat tggatatctg taaagactt                             39


<210> 369
<211> 40
<212> DNA
<213> Artificial Sequence

<220>
<223> Description of Artificial Sequence: Primer

<400> 369
cgcggatccg ccaccatgga ttttccttca aatttggagg                            40


<210> 370
<211> 29
<212> DNA
<213> Artificial Sequence

<220>
<223> Description of Artificial Sequence: Primer

<400> 370
ttgcggccgc accgtactgg ctgctgact                                    29


<210> 371
<211> 39
<212> DNA
<213> Artificial Sequence

<220>
<223> Description of Artificial Sequence: Primer

<400> 371
cggatccgcc accatgagtg agatcaaaat tattaacgc                         39


<210> 372
<211> 29
<212> DNA
<213> Artificial Sequence

<220>
<223> Description of Artificial Sequence: Primer

<400> 372
ttgcggccgc cgttccatgg ttgactcct                                    29


<210> 373
<211> 38
<212> DNA
<213> Artificial Sequence

<220>
<223> Description of Artificial Sequence: Primer

<400> 373
cgcggatccg ccaccatgtg ggacatattg gtggaaac                          38


<210> 374
<211> 32
<212> DNA
<213> Artificial Sequence

<220>
<223> Description of Artificial Sequence: Primer

<400> 374
ttgcggccgc ttcacttgag caaactgaat cc                                32


<210> 375
<211> 42
<212> DNA
<213> Artificial Sequence

<220>

<223> Description of Artificial Sequence: Primer

<400> 375
cgcggatccg ccaccatgtc acaagaaaaa acaaaaaatg aa                    42


<210> 376
<211> 28
<212> DNA
<213> Artificial Sequence

<220>
<223> Description of Artificial Sequence: Primer

<400> 376
ttgcggccgc atcgacgtag tctccgcc                                    28


<210> 377
<211> 43
<212> DNA
<213> Artificial Sequence

<220>
<223> Description of Artificial Sequence: Primer

<400> 377
cgcggatccg ccaccatgct ggttggaact ttctactatc aat                   43


<210> 378
<211> 29
<212> DNA
<213> Artificial Sequence

<220>
<223> Description of Artificial Sequence: Primer

<400> 378
   ttgcggccgc aactttcgtc cctttttgg                                  29


<210> 379
<211> 34
<212> DNA
<213> Artificial Sequence

<220>
<223> Description of Artificial Sequence: Primer

<400> 379
cgcggatccg ccaccatggg caattctggc ggaa                             34


<210> 380
<211> 35
<212> DNA
<213> Artificial Sequence

<220>
<223> Description of Artificial Sequence: Primer

<400> 380
ttgcggccgc ttgtttcata gcttttttga ttgtt                                35

<210> 381
<211> 39
<212> DNA
<213> Artificial Sequence

<220>
<223> Description of Artificial Sequence: Primer

<400> 381
cgcggatccg ccaccatgct attgatacga aatgcaggg                           39

<210> 382
<211> 37
<212> DNA
<213> Artificial Sequence

<220>
<223> Description of Artificial Sequence: Primer

<400> 382
ttgcggccgc aacataatct agtaaataag cgtagcc                             37

<210> 383
<211> 34
<212> DNA
<213> Artificial Sequence

<220>
<223> Description of Artificial Sequence: Primer

<400> 383
cgcggatccg ccaccatgac ggcgacgaat tttc                                34

<210> 384
<211> 35
<212> DNA
<213> Artificial Sequence

<220>
<223> Description of Artificial Sequence: Primer

<400> 384
ttgcggccgc ttaattcgtt tttgaactag ttgct                               35

<210> 385
<211> 40
<212> DNA
<213> Artificial Sequence

<220>
<223> Description of Artificial Sequence: Primer

<400> 385
cgcggatccg ccaccatggc tgtttttctt cgctatcatg                                40

<210> 386
<211> 34
<212> DNA
<213> Artificial Sequence

<220>
<223> Description of Artificial Sequence: Primer

<400> 386
ttgcggccgc tttcttcaac aaaccttgtt cttg                                      34

<210> 387
<211> 39
<212> DNA
<213> Artificial Sequence

<220>
<223> Description of Artificial Sequence: Primer

<400> 387
cgcggatccg ccaccatggg taaccgctct tctcgtaac                                 39

<210> 388
<211> 31
<212> DNA
<213> Artificial Sequence

<220>
<223> Description of Artificial Sequence: Primer

<400> 388
ttgcggccgc gcttccatca aggattttag c                                         31

## Description

The present invention relates to proteins derived from *Streptococcus pneumoniae,* nucleic acid molecules encoding such proteins, the use of the nucleic acid and/or proteins as antigens/immunogens and in detection/diagnosis, as well as methods for screening the proteins/nucleic acid sequences as potential anti-microbial targets.

*Streptococcus pneumoniae,* commonly referred to as the pneumococcus, is an important pathogenic organism. The continuing significance of *Streptoccocus pneumoniae* infections in relation to human disease in developing and developed countries has been authoritatively reviewed (Fiber, G.R., Science, 265: 1385-1387 (1994)). That indicates that on a global scale this organism is believed to be the most common bacterial cause of acute respiratory infections, and is estimated to result in 1 million childhood deaths each year, mostly in developing countries (Stansfield, S.K., Pediatr. Infect. Dis., 6: 622 (1987)). In the USA it has been suggested (Breiman et al, Arch. Intern. Med., 150: 1401 (1990)) that the pneumococcus is still the most common cause of bacterial pneumonia, and that disease rates are particularly high in young children, in the elderly, and in patients with predisposing conditions such as asplenia, heart, lung and kidney disease, diabetes, alcoholism, or with immunosupressive disorders, especially AIDS. These groups are at higher risk of pneumococcal septicaemia and hence meningitis and therefore have a greater risk of dying from pneumococcal infection. The pneumococcus is also the leading cause of otitis media and sinusitis, which remain prevalent infections in children in developed countries, and which incur substantial costs.

The need for effective preventative strategies against pneumococcal infection is highlighted by the recent emergence of penicillin-resistant pneumococci. It has been reported that 6.6% of pneumoccal isolates in 13 US hospitals in 12 states were found to be resistant to penicillin and some isolates were also resistant to other antibiotics including third generation cyclosporins (Schappert, S.M., Vital and Health Statistics of the Centres for Disease Control/National Centre for Health Statistics, 214:1 (1992)). The rates of penicillin resistance can be higher (up to 20%) in some hospitals (Breiman et al, J. Am. Med. Assoc., 271: 1831 (1994)). Since the development of penicillin resistance among pneumococci is both recent and sudden, coming after decades during which penicillin remained an effective treatment, these findings are regarded as alarming.

For the reasons given above, there are therefore compelling grounds for considering improvements in the means of preventing, controlling, diagnosing or treating pneumococcal diseases.

Various approaches have been taken in order to provide vaccines for the prevention of pneumococcal infections. Difficulties arise for instance in view of the variety of serotypes (at least 90) based on the structure of the polysaccharide capsule surrounding the organism. Vaccines against individual serotypes are not effective against other serotypes and this means that vaccines must include polysaccharide antigens from a whole range of serotypes in order to be effective in a majority of cases. An additional problem arises because it ahs been found that the capsular polysaccharides (each of which determines the serotype and is the major protective antigen) when purified and used as a vaccine do not reliably induce protective antibody responses in children under two years of age, the age group which suffers the highest incidence of invasive pneumococcal infection and meningitis.

A modification of the approach using capsule antigens relies on conjugating the polysaccharide to a protein in order to derive an enhanced immune response, particularly by giving the response T-cell dependent character. This approach has been used in the development of a vaccine against *Haemophilus influenza.* There are issues of cost concerning both the multi-polysaccharide vaccines and those based on conjugates.

A third approach is to look for other antigenic components which offer the potential to be vaccine candidates. In the present application we provide a group of proteins antigens which are secreted/exported proteins.

Thus, in a first aspect the present invention provides a *Streptococcus pneumoniae* protein or polypeptide having a sequence selected from those shown in table 2 herein.

A protein or polypeptide of the present invention may be provided in substantially pure form. For example, it may be provided in a form which is substantially free of other proteins.

In a preferred embodiment, a protein or polypeptide having an amino acid sequence as shown in Table 3 is provided.

The invention encompasses any protein coded for by a nucleic acid sequence as shown in Table 1 herein.

As discussed herein, the proteins and polypeptides of the invention are useful as antigenic material. Such material can be "antigenic" and/or "immunogenic". Generally, "antigenic" is taken to mean that the protein or polypeptide is capable of being used to raise antibodies or indeed is capable of inducing an antibody response in a subject. "Immunogenic" is taken to mean that the protein or polypeptide is capable of eliciting a protective immune response in a subject. Thus, in the latter case, the protein or polypeptide may be capable of not only generating an antibody response and in addition non-antibody based immune responses.

The skilled person will appreciate that homologues or derivatives of the proteins or polypeptides of the invention will also find use in the context of the present invention, ie as antigenic/immunogenic material. Thus, for instance proteins or polypeptides which include one or more additions, deletions, substitutions or the like are encompassed by the present invention. In addition, it may be possible to replace one amino acid with another of similar "type". For instance replacing one hydrophobic amino acid with another. One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis are contemplated in the present invention.

In the case of homologues and derivatives, the degree of identity with a protein or polypeptide as described herein is less important than that the homologue or derivative should retain its antigenicity or immunogenicity to streptoccocus pneumoniae. However, suitably, homologues or derivatives having at least 60% similarity (as discussed above) with the proteins or polypeptides described herein are provided.

Preferably, homologues or derivatives having at least 70% similarity, more preferably at least 80% similarity are provided. Most preferably, homologues or derivatives having at least 90% or even 95% similarity are provided.

In an alternative approach, the homologues or derivatives could be fusion proteins, incorporating moieties which render purification easier, for example by effectively tagging the desired protein or polypeptide. It may be necessary to remove the "tag" or it may be the case that the fusion protein itself retains sufficient antigenicity to be useful.

In an additional aspect of the invention there are provided antigenic fragments of the proteins or polypeptides of the invention, or of homologues or derivatives thereof.

For fragments of the proteins or polypeptides described herein, or of homologues or derivatives thereof, the situation is slightly different. It is well known that is possible to screen an antigenic protein or polypeptide to identify epitopic regions, ie those regions which are responsible for the protein or polypeptide's antigenicity or immunogenicity. Methods for carrying out such screening are well known in the art. Thus, the fragments of the present invention should include one or more such epitopic regions or be sufficiently similar to such regions to retain their antigenic/immunogenic properties. Thus, for fragments according to the present invention the degree of identity is perhaps irrelevant, since they may be 100% identical to a particular part of a protein or polypeptide, homologue or derivative as described herein. The key issue, once again, is that the fragment retains the antigenic/immunogenic properties.

Thus, what is important for homologues, derivatives and fragments is that they possess at least a degree of the antigenicity/immunogenicity of the protein or polypeptide from which they are derived.

Gene cloning techniques may be used to provide a protein of the invention in substantially pure form. These techniques are disclosed, for example, in J. Sambrook et al Molecular Cloning 2nd Edition, Cold Spring Harbor Laboratory Press (1989). Thus, in a fourth aspect, the present invention provides a nucleic acid molecule comprising or consisting of a sequence which is:
(i) any of the DNA sequences set out in Table 1 or their RNA equivalents;
(ii) a sequence which is complementary to any of the sequences of (i);
(iii) a sequence which codes for the same protein or polypeptide, as those sequences of (i) or (ii);
(iv) a sequence which is has substantial identity with any of those of (i), (ii) and (iii);
(v) a sequence which codes for a homologue, derivative or fragment of a protein as defined in Table 1.

In a fifth aspect the present invention provides a nucleic acid molecule comprising or consisting of a sequence which is:
(i) any of the DNA sequences set out in Table 4 or their RNA equivalents;
(ii) a sequence which is complementary to any of the sequences of (i);
(iii) a sequence which codes for the same protein or polypeptide, as those sequences of (i) or (ii);
(iv) a sequence which is has substantial identity with any of those of (i), (ii) and (iii);
(v)a sequence which codes for a homologue, derivative or fragment of a protein as defined in Table 4.

The nucleic acid molecules of the invention may include a plurality of such sequences, and/or fragments. The skilled person will appreciate that the present invention can include novel variants of those particular novel nucleic acid molecules which are exemplified herein. Such variants are encompassed by the present invention. These may occur in nature, for example because of strain variation. For example, additions, substitutions and/or deletions are included. In addition, and particularly when utilising microbial expression systems, one may wish to engineer the nucleic acid sequence by making use of known preferred codon usage in the particular organism being used for expression. Thus, synthetic or non-naturally occurring variants are also included within the scope of the invention.

The term "RNA equivalent" when used above indicates that a given RNA molecule has a sequence which is complementary to that of a given DNA molecule (allowing for the fact that in RNA "U" replaces "T" in the genetic code).

When comparing nucleic acid sequences for the purposes of determining the degree of homology or identity one can use programs such as BESTFIT and GAP (both from the Wisconsin Genetics Computer Group (GCG) software package) BESTFIT, for example, compares two sequences and produces an optimal alignment of the most similar segments. GAP enables sequences to be aligned along their whole length and finds the optimal alignment by inserting spaces in either sequence as appropriate. Suitably, in the context of the present invention compare when discussing identity of nucleic acid sequences, the comparison is made by alignment of the sequences along their whole length.

Preferably, sequences which have substantial identity have at least 50% sequence identity, desirably at least 75 % sequence identity and more desirably at least 90 or at least 95 % sequence identity with said sequences. In some cases the sequence identity may be 99% or above.

Desirably, the term "substantial identity" indicates that said sequence has a greater degree of identity with any of the sequences described herein than with prior art nucleic acid sequences.

It should however be noted that where a nucleic acid sequence of the present invention codes for at least part of a novel gene product the present invention includes within its scope all possible sequence coding for the gene product or for a novel part thereof.

The nucleic acid molecule may be in isolated or recombinant form. It may be incorporated into a vector and the vector may be incorporated into a host. Such vectors and suitable hosts form yet further aspects of the present invention.

Therefore, for example, by using probes based upon the nucleic acid sequences provided herein, genes in *Streptococcus pneumoniae* can be identified. They can then be excised using restriction enzymes and cloned into a vector. The vector can be introduced into a suitable host for expression.

Nucleic acid molecules of the present invention may be obtained from *S.pneumoniae* by the use of appropriate probes complementary to part of the sequences of the nucleic acid molecules. Restriction enzymes or sonication techniques can be used to obtain appropriately sized fragments for probing.

Alternatively PCR techniques may be used to amplify a desired nucleic acid sequence. Thus the sequence data provided herein can be used to design two primers for use in PCR so that a desired sequence, including whole genes or fragments thereof, can be targeted and then amplified to a high degree. One primer will normally show a high degree of specificity for a first sequence located on one strand of a DNA molecule, and the other primer will normally show a high degree of specificity for a second sequence located on the complementary strand of the DNA sequence and being spaced from the complementary sequence to the first sequence.

Typically primers will be at least 15-25 nucleotides long.

As a further alternative chemical synthesis may be used. This may be automated. Relatively short sequences may be chemically synthesised and ligated together to provide a longer sequence.

In yet a further aspect the present invention provides an immunogenic/antigenic composition comprising one or more proteins or polypeptides selected from those whose sequences are shown in Tables 2-4, or homologues or derivatives thereof, and/or fragments of any of these. In preferred embodiments, the immunogenic/antigenic composition is a vaccine or is for use in a diagnostic assay.

In the case of vaccines suitable additional excipients, diluents, adjuvants or the like may be included. Numerous examples of these are well known in the art.

It is also possible to utilise the nucleic acid sequences shown in Table 1 in the preparation of so-called DNA vaccines. Thus, the invention also provides a vaccine composition comprising one or more nucleic acid sequences as defined herein. The use of such DNA vaccines is described in the art. See for instance, Donnelly et al , Ann. Rev. Immunol., 15:617-648 (1997).

As already discussed herein the proteins or polypeptides described herein, their homologues or derivatives, and/or fragments of any of these, can be used in methods of detecting/diagnosing *S.pneumoniae.* Such methods can be based on the detection of antibodies against such proteins which may be present in a subject. Therefore the present invention provides a method for the detection/diagnosis of *S.pneumoniae* which comprises the step of bringing into contact a sample to be tested with at least one protein, or homologue, derivative or fragment thereof, as described herein. Suitably, the sample is a biological sample, such as a tissue sample or a sample of blood or saliva obtained from a subject to be tested.

In an alternative approach, the proteins described herein, or homologues, derivatives and/or fragments thereof, can be used to raise antibodies, which in turn can be used to detect the antigens, and hence *S.pneumoniae.* Such antibodies form another aspect of the invention. Antibodies within the scope of the present invention may be monoclonal or polyclonal.

Polyclonal antibodies can be raised by stimulating their production in a suitable animal host (e.g. a mouse, rat, guinea pig, rabbit, sheep, goat or monkey) when a protein as described herein, or a homologue, derivative or fragment thereof, is injected into the animal. If desired, an adjuvant may be administered together with the protein. Well-known adjuvants include Freund's adjuvant (complete and incomplete) and aluminium hydroxide. The antibodies can then be purified by virtue of their binding to a protein as described herein.

Monoclonal antibodies can be produced from hybridomas. These can be formed by fusing myeloma cells and spleen cells which produce the desired antibody in order to form an immortal cell line. Thus the well-known Kohler & Milstein technique (Nature 256 (1975)) or subsequent variations upon this technique can be used.

Techniques for producing monoclonal and polyclonal antibodies that bind to a particular polypeptide/protein are now well developed in the art. They are discussed in standard immunology textbooks, for example in Roitt et al, Immunology second edition (1989), Churchill Livingstone, London.

In addition to whole antibodies, the present invention includes derivatives thereof which are capable of binding to proteins etc as described herein. Thus the present invention includes antibody fragments and synthetic constructs. Examples of antibody fragments and synthetic constructs are given by Dougall et al in Tibtech 12 372-379 (September 1994).

Antibody fragments include, for example, Fab, F(ab')₂ and Fv fragments. Fab fragments (These are discussed in Roitt *et al* [*supra*]). Fv fragments can be modified to produce a synthetic construct known as a single chain Fv (scFv) molecule. This includes a peptide linker covalently joining Vₕ and Vₗ regions, which contributes to the stability of the molecule. Other synthetic constructs that can be used include CDR peptides. These are synthetic peptides comprising antigen-binding determinants. Peptide mimetics may also be used. These molecules are usually conformationally restricted organic rings that mimic the structure of a CDR loop and that include antigen-interactive side chains.

Synthetic constructs include chimaeric molecules. Thus, for example, humanised (or primatised) antibodies or derivatives thereof are within the scope of the present invention. An example of a humanised antibody is an antibody having human framework regions, but rodent hypervariable regions. Ways of producing chimaeric antibodies are discussed for example by Morrison et al in PNAS, 81, 6851-6855 (1984) and by Takeda et al in Nature. 314, 452-454 (1985).

Synthetic constructs also include molecules comprising an additional moiety that provides the molecule with some desirable property in addition to antigen binding. For example the moiety may be a label (e.g. a fluorescent or radioactive label). Alternatively, it may be a pharmaceutically active agent.

Antibodies, or derivatives thereof, find use in detection/diagnosis of *S.pneumoniae.* Thus, in another aspect the present invention provides a method for the detection/diagnosis of *S.pneumoniae* which comprises the step of bringing into contact a sample to be tested and antibodies capable of binding to one or more proteins described herein, or to homologues, derivatives and/or fragments thereof.

In addition, so-called "Affibodies" may be utilised. These are binding proteins selected from combinatorial libraries of an alpha-helical bacterial receptor domain (Nord *et al*, ) Thus, Small protein domains, capable of specific binding to different target proteins can be selected using combinatorial approaches.

It will also be clear that the nucleic acid sequences described herein may be used to detect/diagnose *S.pneumoniae.* Thus, in yet a further aspect, the present invention provides a method for the detection/diagnosis of *S.pneumoniae* which comprises the step of bringing into contact a sample to be tested with at least one nucleic acid sequence as described herein. Suitably, the sample is a biological sample, such as a tissue sample or a sample of blood or saliva obtained from a subject to be tested. Such samples may be pre-treated before being used in the methods of the invention, Trhus, for example, a sample may be treated to extract DNA. Then, DNA probes based on the nucleic acid sequences described herein (ie usually fragments of such sequences) may be used to detect nucleic acid from *S.pneumoniae.*

In additional aspects, the present invention provides:
(a) a method of vaccinating a subject against *S.pneumoniae* which comprises the step of administering to a subject a protein or polypeptide of the invention, or a derivative, homologue or fragment thereof, or an immunogenic composition of the invention;
(b) a method of vaccinating a subject against *S.pneumoniae* which comprises the step of administering to a subject a nucleic acid molecule as defined herein;
(c) a method for the prophylaxis or treatment of *S.pneumoniae* infection which comprises the step of administering to a subject a protein or polypeptide of the invention, or a derivative, homologue or fragment thereof, or an immunogenic composition of the invention;
(d) a method for the prophylaxis or treatment of *S.pneumoniae* infection which comprises the step of administering to a subject a nucleic acid molecule as defined herein;
(e) a kit for use in detecting/diagnosing *S.pneumoniae* infection comprising one or more proteins or polypeptides of the invention, or homologues, derivatives or fragments thereof, or an antigenic composition of the invention; and
(f) a kit for use in detecting/diagnosing *S.pneumoniae* infection comprising one or more nucleic acid molecules as defined herein.

Given that we have identified a group of important proteins, such proteins are potential targets for anti-microbial therapy. It is necessary, however, to determine whether each individual protein is essential for the organism's viability. Thus, the present invention also provides a method of determining whether a protein or polypeptide as described herein represents a potential anti-microbial target which comprises inactivating said protein and determining whether *S.pneumoniae* is still viable, *in vitro* or *in vivo.*

A suitable method for inactivating the protein is to effect selected gene knockouts, ie prevent expression of the protein and determine whether this results in a lethal change. Suitable methods for carrying out such gene knockouts are described in Li et al, P.N.A.S., 94:13251-13256 (1997).

In a final aspect the present invention provides the use of an agent capable of antagonising, inhibiting or otherwise interfering with the function or expression of a protein or polypeptide of the invention in the manufacture of a medicament for use in the treatment or prophylaxis of *S.pneumoniae* infection.

The invention will now be described with reference to the following examples, which should not be construed as in any way limiting the invention. The examples refer to the figures in which:
Figure 1: shows the results of various DNA vaccine trials; and
Figure 2: shows the results of further DNA vaccine trials.

### EXAMPLE 1

The Genome sequencing of *Streptococcus pneumoniae* type 4 is in progress at the

Institute for Genomic Research (TIGR, Rockville, MD, USA). Up to now, the whole sequence has not been completed or published. On 21^{st} November 1997, the TIGR centre released some DNA sequences as contigs which are not accurate reflections of the finished sequence. These contigs can be downloaded from their Webster (www@tigr.org). We downloaded these contigs and created a local database using the application GCGToBLAST (Wisconsin Package Version 9.1, Genetics Computer Group (GCG), Madison, USA). This database can be searched with the FastA and TfastA procedures (using the method of Pearson and Lipman (PNAS USA, 85:2444-2448 (1988)).

Using FastA and TfastA procedures, the local pneumococcus database was searched for putative leader sequence or anchor sequence features. Relevant sequences were used to interrogate for comparative novel sequences. These were:
(i) already described leader sequences of *Streptococcus pneumoniae* (from proteins NanA, NanB, LytA, PapA, pcpA, PsaA and PspA);
(ii) the leader sequence of Usp45, a secreted protein from *Lactococcus lactis;*
(iii) new hypothetical leader sequences derived from the searches in (i) and (ii);
(iv) the anchor motif LPxTG, a feature common to many Gram-positive bacteria surface proteins which are anchored by a mechanism involving the Sortase complex proteins.

Provided below is an example of this approach, with reference to the sequences derived from the database (see table 1).

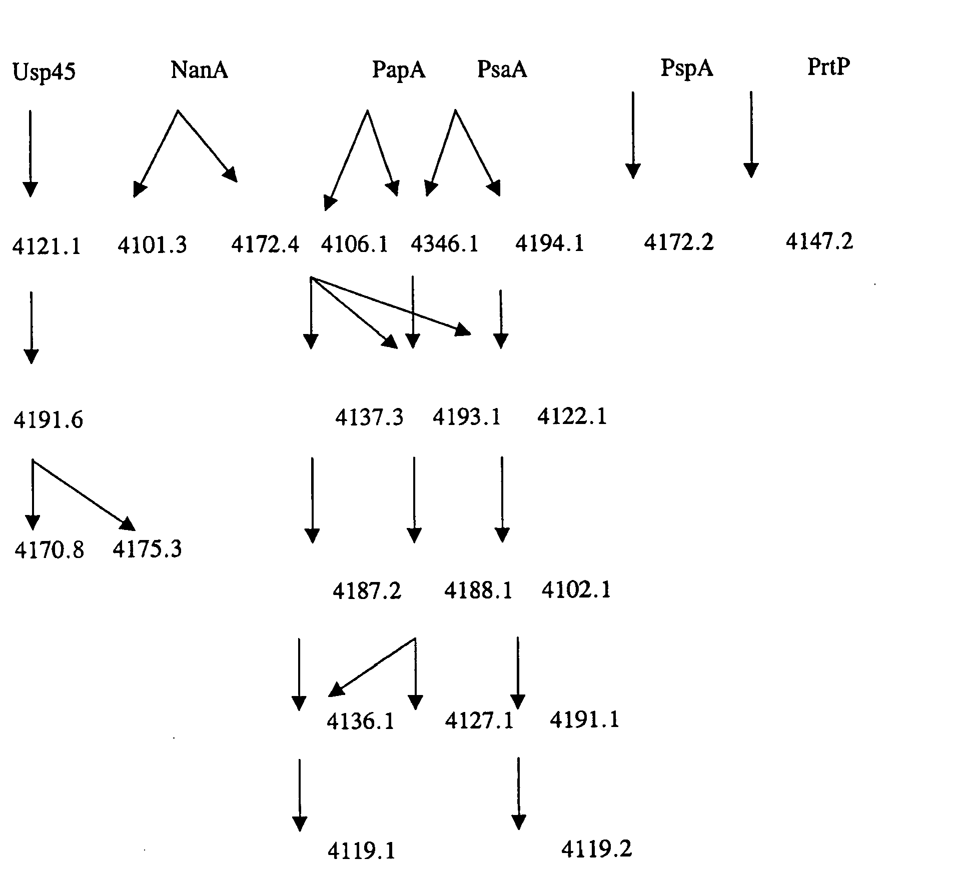

The protein leader sequences of different known exported proteins were used as a starting point for a search of the local pneumococcus database described above. The hypothetical proteins found with this search were then submitted to a Blast search in general databases such as EMBL, Swissprot etc. Proteins remaining unknown in the pneumococcus are kept and annotated. Then the search is performed again using the new potential protein leader sequence as a probe, using the TfastA procedure.

### Example 2: DNA vaccine trials

### pcDNA3.1 + as a DNA vaccine vector

### pcDNA3.1 +

The vector chosen for use as a DNA vaccine vector was pcDNA3.1 (Invitrogen) (actually pcDNA3.1 +, the forward orientation was used in all cases but may be referred to as pcDNA3.1 here on). This vector has been widely and successfully employed as a host vector to test vaccine candidate genes to give protection against pathogens in the literature (Zhang, *et al.,* Kurar and Splitter, Anderson *et al. ).* The vector was designed for high-level stable and non-replicative transient expression in mammalian cells. pcDNA3.1 contains the ColE1 origin of replication which allows convenient high-copy number replication and growth in *E. coli.* This in turn allows rapid and efficient cloning and testing of many genes. The pcDNA3.1 vector has a large number of cloning sites and also contains the gene encoding ampicillin resistance to aid in cloning selection and the human cytomegalovirus (CMV) immediate-early promoter/enhancer which permits efficient, high-level expression of the recombinant protein. The CMV promoter is a strong viral promoter in a wide range of cell types including both muscle and immune (antigen presenting) cells. This is important for optimal immune response as it remains unknown as to which cells types are most important in generating a protective response *in vivo.* A T7 promoter upstream of the multiple cloning site affords efficient expression of the modified insert of interest and which allows *in vitro* transcription of a cloned gene in the sense orientation.
Zhang, D., Yang, X., Berry, J. Shen, C., McClarty, G. and Brunham, R.C. (1997) "DNA vaccination with the major outer-membrane protein genes induces acquired immunity to Chlamydia trachomatis (mouse pneumonitis) infection". Infection and Immunity, 176, 1035-40.
Kurar, E. and Splitter, G.A. (1997) "Nucleic acid vaccination of Brucella abortus ribosomal L7/L12 gene elicits immune response". Vaccine, 15, 1851-57.
Anderson, R., Gao, X.-M., Papakonstantinopoulou, A., Roberts, M. and Dougan, G. (1996) "Immune response in mice following immunisation with DNA encoding fragment C of tetanus toxin". Infection and Immunity, 64, 3168-3173.

### Preparation of DNA vaccines

Oligonucleotide primers were designed for each individual gene of interest derived using the LEEP system. Each gene was examined thoroughly, and where possible, primers were designed such that they targeted that portion of the gene thought to encode only the mature portion of the gene protein. It was hoped that expressing those sequences that encode only the mature portion of a target gene protein, would facilitate its correct folding when expressed in mammalian cells. For example, in the majority of cases primers were designed such that putative N-terminal signal peptide sequences would not be included in the final amplification product to be cloned into the pcDNA3.1 expression vector. The signal peptide directs the polypeptide precursor to the cell membrane via the protein export pathway where it is normally cleaved off by signal peptidase I (or signal peptidase II if a lipoprotein). Hence the signal peptide does not make up any part of the mature protein whether it be displayed on the surface of the bacteria surface or secreted. Where a N-terminal leader peptide sequence was not immediately obvious, primers were designed to target the whole of the gene sequence for cloning and ultimately, expression in pcDNA3.1.

Having said that, however, other additional features of proteins may also affect the expression and presentation of a soluble protein. DNA sequences encoding such features in the genes encoding the proteins of interest were excluded during the design of oligonucleotides. These features included:
1. LPXTG cell wall anchoring motifs.
2. LXXC ipoprotein attachment sites.
3. Hydrophobic C-terminal domain.
4. Where no N-terminal signal peptide or LXXC was present the start codon was excluded.
5. Where no hydrophobic C-terminal domain or LPXTG motif was present the stop codon was removed.

Appropriate PCR primers were designed for each gene of interest and any and all of the regions encoding the above features was removed from the gene when designing these primers. The primers were designed with the appropriate enzyme restriction site followed by a conserved Kozak nucleotide sequence (in all cases) GCCACC was used. The Kozak sequence facilitates the recognition of initiator sequences by eukaryotic ribosomes) and an ATG start codon upstream of the insert of the gene of interest. For example the forward primer using a BamH1 site the primer would begin GCGGGATCCGCCACCATG followed by a small section of the 5' end of the gene of interest. The reverse primer was designed to be compatible with the forward primer and with a Not1 restriction site at the 5' end in all cases (this site is TTGCGGCCGC).

### PCR primers

The following PCR primers were designed and used to amplify the truncated genes of interest.
**ID210** 
Forward Primer 5' CGGATCCGCCACCATGTCTTCTAATGAATCTGCCGATG
3'
Reverse Primer 5' TTGCGGCCGCCTGTTTAGATTGGATATCTGTAAAGACTT
3'
4172.5 
Forward Primer 5'
CGCGGATCCGCCACCATGGATTTTCCTTCAAATTTGGAGG 3'
Reverse Primer 5' TTGCGGCCGCACCGTACTGGCTGCTGACT 3'
ID211 
Forward Primer 5'
CGGATCCGCCACCATGAGTGAGATCAAAATTATTAACGC 3'
Reverse Primer 5' TTGCGGCCGCCGTTCCATGGTTGACTCCT 3'
4197.4 
Forward Primer 5' CGCGGATCCGCCACCATGTGGACATATTGGTGGAAAC
3'
Reverse Primer 5' TTGCGGCCGCTTCACTTGAGCAAACTGAATCC 3'
4122.1 
Forward Primer 5'
CGCGGATCCGCCACCATGTCACAAGAAAAAACAAAAAATGAA 3'
Reverse Primer 5' TTGCGGCCGCATCGACGTAGTCTCCGCC 3'
4126.7 
Forward Primer 5'
CGCGGATCCGCCACCATGCTGGTTGGAACTTTCTACTATCAAT 3'
Reverse Primer 5' TTGCGGCCGCAACTTTCGTCCCTTTTTGG 3'
4188.11 
Forward Primer 5' CGCGGATCCGCCACCATGGGCAATTCTGGCGGAA 3'
Reverse Primer 5' TTGCGGCCGCTTGTTTCATAGCTTTTTGATTGTT 3'
ID209 
Forward Primer 5'
CGCGGATCCGCCACCATGCTATTGATACGAAATGCAGGG 3'
Reverse Primer 5' TTGCGGCCGCAACATAATCTAGTAAATAAGCGTAGCC 3'
ID215 
Forward Primer 5' CGCGGATCCGCCACCATGACGGCGACGAATTTTC 3'
Reverse Primer 5' TTGCGGCCGCTTAATTCGTTTTTGAACTAGTTGCT 3'
4170.4 
Forward Primer 5'
CGCGGATCCGCCACCATGGCTGTTTTCTTCGCTATCATG 3'
Reverse Primer 5' TTGCGGCCGCTTTCTTCAACAAACCTTGTTCTTG 3'
4193.1

Forward Primer 5'
CGCGGATCCGCCACCATGGGTAACCGCTCTTCTCGTAAC 3'
Reverse Primer 5' TTGCGGCCGCGCTTCCATCAAGGATTTTAGC 3'

### Cloning

The insert along with the flanking features described above was amplified using PCR against a template of genomic DNA isolated from type 4 *S*. *pneumoniae* strain 11886 obtained from the National Collection of Type Cultures. The PCR product was cut with the appropriate restriction enzymes and cloned in to the multiple cloning site of pcDNA3.1 using conventional molecular biological techniques. Suitably mapped clones of the genes of interested were cultured and the plasmids isolated on a large scale (> 1.5 mg) using Plasmid Mega Kits (Qiagen). Successful cloning and maintenance of genes was confirmed by restriction mapping and sequencing ∼ 700 base pairs through the 5' cloning junction of each large scale preparation of each construct.

### Strain validation

A strain of type 4 was used in cloning and challenge methods which is the strain from which the *S*. *pneumoniae* genome was sequenced. A freeze dried ampoule of a homogeneous laboratory strain of type 4 *S*. *pneumoniae* strain NCTC 11886 was obtained from the National Collection of Type Strains. The ampoule was opened and the cultured re suspended with 0.5 ml of tryptic soy broth (0.5% glucose, 5% blood). The suspension was subcultured into 10 ml tryptic soy broth (0.5% glucose, 5% blood) and incubated statically overnight at 37°C. This culture was streaked on to 5 % blood agar plates to check for contaminants and confirm viability and on to blood agar slopes and the rest of the culture was used to make 20% glycerol stocks. The slopes were sent to the Public Health Laboratory Service where the type 4 serotype was confirmed.

A glycerol stock of NCTC 11886 was streaked on a 5 % blood agar plate and incubated overnight in a CO2 gas jar at 37°C. Fresh streaks were made and optochin sensitivity was confirmed.

### Pneumococcal challenge

A standard inoculum of type 4 *S*. *pneumoniae* was prepared and frozen down by passaging a culture of pneumococcus 1x through mice, harvesting from the blood of infected animals, and grown up to a predetermined viable count of around 10⁹ cfu/ml in broth before freezing down. The preparation is set out below as per the flow chart.

Streak pneumococcal culture and confirm identity
|
V
Grow over-night culture from 4-5 colonies on plate above
|
V
Animal passage pneumococcal culture
(i.p. injection of cardiac bleed to harvest)
|
V
Grow over-night culture from animal passaged pneumococcus

|
V

Grow day culture (to pre-determined optical density) from over-night of animal
passage and freeze down at -70°C - This is standard minimum

|
V

Thaw one aliquot of standard inoculum to viable count

|
V

Use standard inoculum to determine effective dose (called Virulence Testing)

|
V

All subsequent challenges - use standard inoculum to effective dose

An aliquot of standard inoculum was diluted 500x in PBS and used to inoculate the mice.

Mice were lightly anaesthetised using halothane and then a dose of 1.4 x 10⁵ cfu of pneumococcus was applied to the nose of each mouse. The uptake was facilitated by the normal breathing of the mouse, which was left to recover on its back.

### S. pneumoniae vaccine trials

Vaccine trials in mice were carried out by the administration of DNA to 6 week old CBA/ca mice (Harlan, UK). Mice to be vaccinated were divided into groups of six and each group was immunised with recombinant pcDNA3.1 + plasmid DNA containing a specific target-gene sequence of interest. A total of 100 µg of DNA in Dulbecco's PBS (Sigma) was injected intramuscularly into the tibialis anterior muscle of both legs (50 µl in each leg). A boost was carried using the same procedure 4 weeks later. For comparison, control groups were included in all vaccine trials. These control groups were either unvaccinated animals or those administered with non-recombinant pcDNA3.1 + DNA (sham vaccinated) only, using the same time course described above. 3 weeks after the second immunisation, all mice groups were challenged intra-nasally with a lethal dose of *S*. *pneumoniae* serotype 4 (strain NCTC 11886). The number of bacteria administered was monitored by plating serial dilutions of the inoculum on 5% blood agar plates. A problem with intranasal immunisations is that in some mice the inoculum bubbles out of the nostrils, this has been noted in results table and taken account of in calculations. A less obvious problem is that a certain amount of the inoculum for each mouse may be swallowed. It is assumed that this amount will be the same for each mouse and will average out over the course of innoculations. However, the sample sizes that have been used are small and this problem may have significant effects in some experiments. All mice remaining after the challenge were killed 3 or 4 days after infection. During the infection process, challenged mice were monitored for the development of symptoms associated with the onset of *S*. *pneumoniae* induced-disease. Typical symptoms in an appropriate order included piloerection, an increasingly hunched posture, discharge from eyes, increased lethargy and reluctance to move. The latter symptoms usually coincided with the development of a moribund state at which stage the mice were culled to prevent further suffering. These mice were deemed to be very close to death, and the time of culling was used to determine a survival time for statistical analysis. Where mice were found dead, the survival time was taken as the last time point when the mouse was monitored alive.

### Interpretation of Results

A positive result was taken as any DNA sequence that was cloned and used in challenge experiments as described above which gave protection against that challenge. Protection was taken as those DNA sequences that gave statistically significant protection (to a 95% confidence level (p<0.05)) and also those which were marginal or close to significant using Mann-Whitney or which show some protective features for example there were one or more outlying mice or because the time to the first death was prolonged. It is acceptable to allow marginal or non-significant results to be considered as potential positives when it is considered that the clarity of some of the results may be clouded by the problems associated with the administration of intranasal infections.

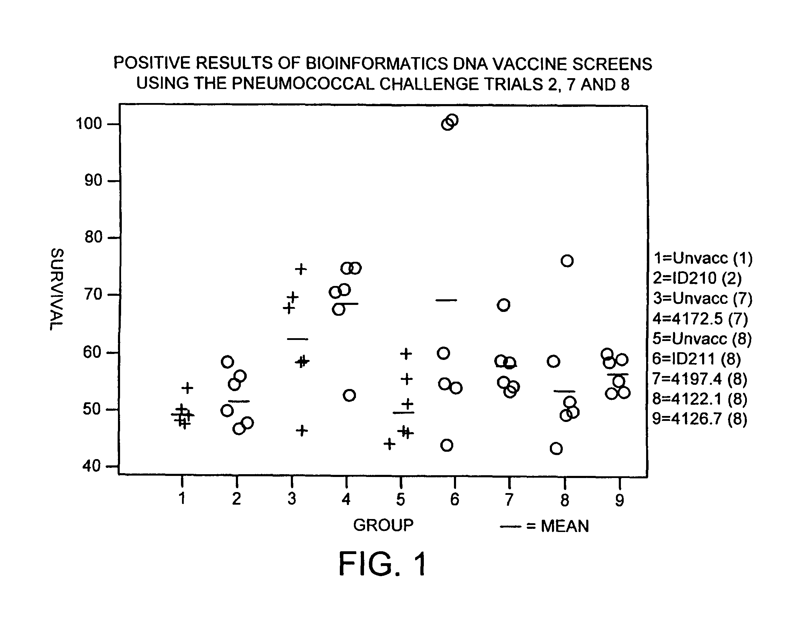

### Results for vaccine trials 2, 7 and 8 (see figure 1)

| **Mouse number** | **Mean survival times (hours)** | | | | | | | | |
|---|---|---|---|---|---|---|---|---|---|
| | **Unvacc control (2)** | **ID210 (2)** | **Unvacc control (7)** | **4172.5 (7)** | **Unvacc control (8)** | **ID211 (8)** | **4197.4 (8)** | **4122.1 (8)** | **4126.7 (8)** |
| 1 | 49.0 | 55.0 | 59.6 | 72.6 | 45.1 | 102.3T | 60.1 | 50.6 | 60.0 |
| 2 | 51.0 | 46.5 | 47.2 | 67.9 | 50.8 | 55.5 | 54.9 | 77.2 | 60.0 |
| 3 | 49.0 | 49.0 | 59.6 | 54.4 | 60.4 | 60.6* | 68.4 | 60.3 | 54.8 |
| 4 | 55.0 | 59.0 | 70.9 | 75.3 | 55.2 | 45.3 | 60.1 | 50.6 | 52.6 |
| 5 | 49.0 | 55.0 | 68.6* | 70.9 | 45.1 | 55.5 | 54.9 | 50.6* | 54.8 |
| 6 | 49.0 | 49.0 | 76.0 | 75.3 | 45.1 | 102.3T | 52.7 | 44.9 | 60 |
| **Mean** | **50.3** | **52.3** | **63.6** | **69.4** | **50.2** | **70.2** | **58.5** | **55.7** | **57.0** |
| **sd** | **2.4** | **4.8** | **10.3** | **7.9** | **6.4** | **25.3** | **5.7** | **11.6** | **3.4** |
| **p value 1** | **-** | **0.3333** | **-** | **0.2104** | **-** | **0.0215** | **0.0621** | **0.4038** | **0.0833** |

| | | | | | | | | | |
|---|---|---|---|---|---|---|---|---|---|
| * - bubbled when dosed so may not have received full inoculum. T - terminated at end of experiment having no symptoms of infection. Numbers in brackets - survival times disregarded assuming incomplete dosing p value 1 refers to significance tests compared to unvaccinated controls | | | | | | | | | |

### Statistical Analyses_{.}

Trial 2 - The group vaccinated with ID210 also had a longer mean survival time than the unvaccinated controls but the results are not statistically significant.
Trial 7 - The group vaccinated with 4172.5 showed much greater survival times than unvaccinated controls although the differences were not statistically significant.
Trial 8 - The group vaccinated with ID211 survived significantly longer than unvaccinated controls. 4197.4, 4122.1 and 4126.7 vaccinated groups showed longer mean survival times than the unvaccinated group but the results were not statistically significant. The 4197.4 and 4126.7 groups also showed a prolonged time to the first death and the 4122.1 group showed 1 outlying result.

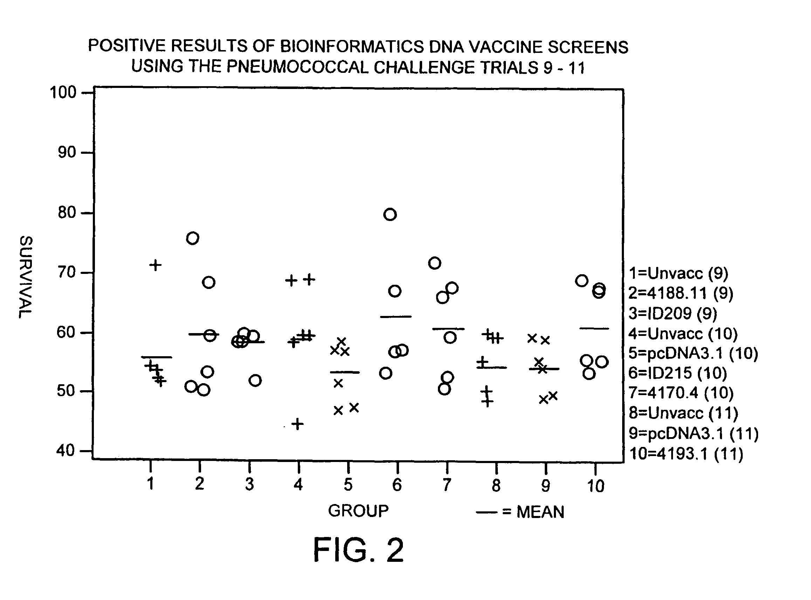

**Results of pneumococcal challenge DNA vaccination trials 9-11 (see** **figure 2****)**

| **Mouse number** | **Mean survival times (hours)** | | | | | | | | | |
|---|---|---|---|---|---|---|---|---|---|---|
| | **Unvacc control (9)** | **4188.1 1 (9)** | **ID209 (9)** | **Unvacc control (10)** | **pcDNA3.1 + (10)** | **ID215 (10)** | **4170. 4 (10)** | **Unvacc control (11)** | **pcDNA3.1 + (11)** | **4193.1 (11)** |
| 1 | (98.5)T | 69.4 | 60.2 | 68.4 | 58.6 | 79.2 | 68.1 | 60.0 | 53.2 | 54.8 |
| 2 | 53.4 | 53.7 | 60.2 | 59.0 | 58.6 | 54.2 | 58.6 | 50.0 | 50.4 | 54.8 |
| 3 | 53.4 | 51.2 | 60.2 | 59.0 | 50.8 | (103.2)*T | 50.9 | 60.0 | 55.4 | 68.7* |
| 4 | 53.4 | 75.0 | (98.0)*T | 45.1* | 58.6 | 58.8 | 72.1 | 55.0 | 60.6 | 54.8 |
| 5 | 70.8 | 51.2 | 60.2 | 68.4 | 46.5 | 68.3 | 68.1 | 60.0 | 50.4 | 68.7 |
| 6 | 53.4 | 61.2 | 52.9 | 59.0 | 48.9 | 58.8 | 54.0 | 50.0 | 60.6 | 68.7* |
| **Mean** | **56.9** | **60.3** | **58.8** | **59.8** | **53.6** | **63.9** | **62.0** | **55.8** | **55.1** | **61.7** |
| **Sd** | **7.8** | **10.0** | **3.3** | **8.5** | **5.6** | **10.0** | **8.7** | **5.0** | **4.6** | **7.6** |
| **p value 1** | **-** | **0.3894** | **0.2519** | **-** | **0.0307** | **<30.0** | **<39. 0** | **-** | **-** | **0.1837** |
| **p value 2** | **-** | **-** | **-** | **-** | **-** | **0.0168** | **0.031 6** | **-** | **-** | **0.0829** |

| | | | | | | | | | | |
|---|---|---|---|---|---|---|---|---|---|---|
| * - bubbled when dosed so may not have received full inoculum. T - terminated at end of experiment having no symptoms of infection. Numbers in brackets - survival times disregarded assuming incomplete dosing p value 1 refers to significance tests compared to unvaccinated controls p value 2 refers to significance tests compared to pcDNA3.1 + vaccinated controls | | | | | | | | | | |

### Statistical Analyses.

Trial 9 - Although not statistically significant the groups vaccinated with 4188.11 and ID209 did have noticeably higher mean survival times than unvaccinated controls.
Trial 10 - The unvaccinated control group survived for a significantly longer period than the pcDNA3.1 + vaccinated group.
The groups vaccinated with ID215 and 4170.4 showed statistically significant longer survival times compared to the sham vaccinated group (p=0.0168 and 0.0316) but not compared to the unvaccinated group.
Trial 11 - The group vaccinated with 4193.1 was the most promising and survived an average of 6.5 hours longer than the pcDNA3.1 + vaccinated group and 6 hours longer than the unvaccinated group although the results were not statistically significant.

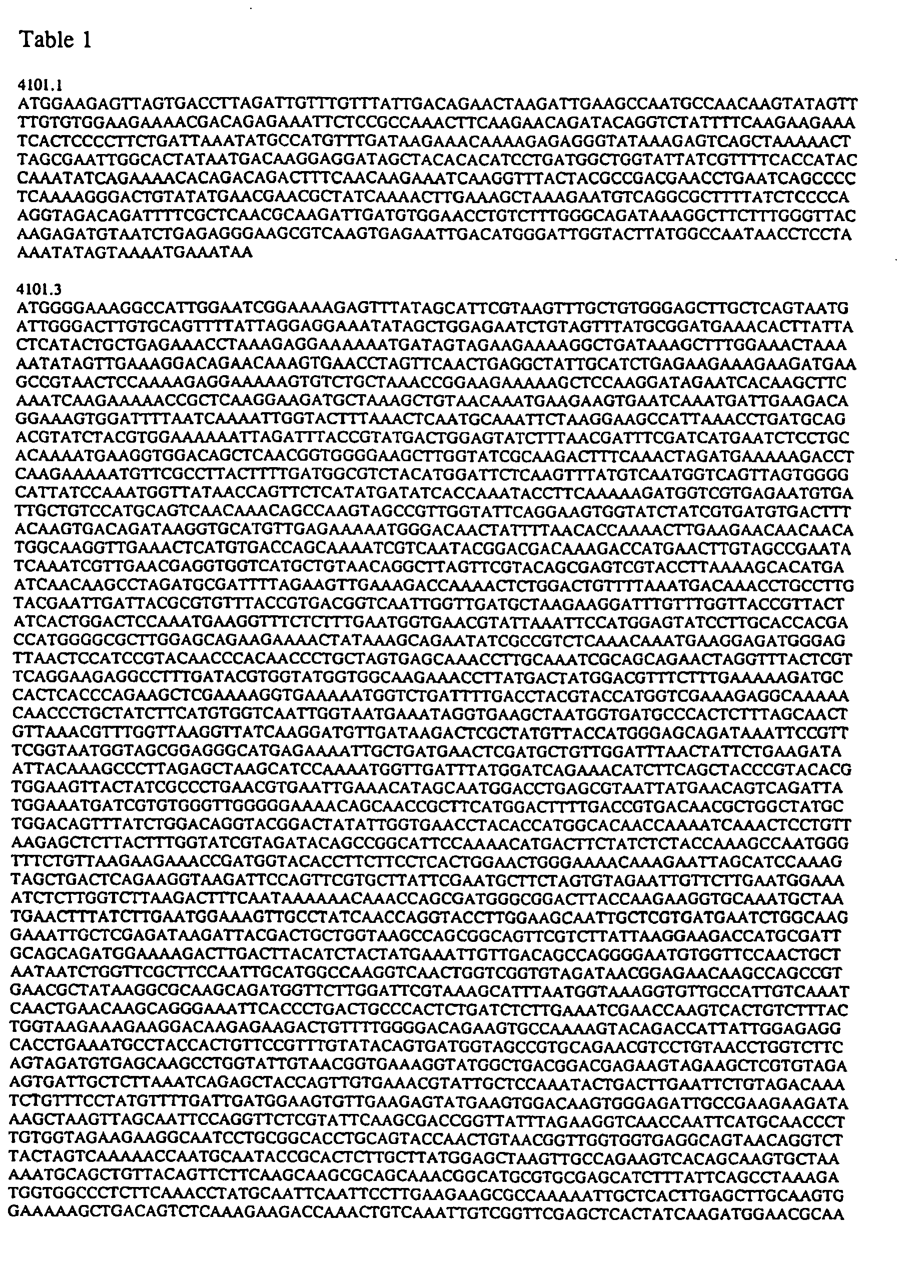

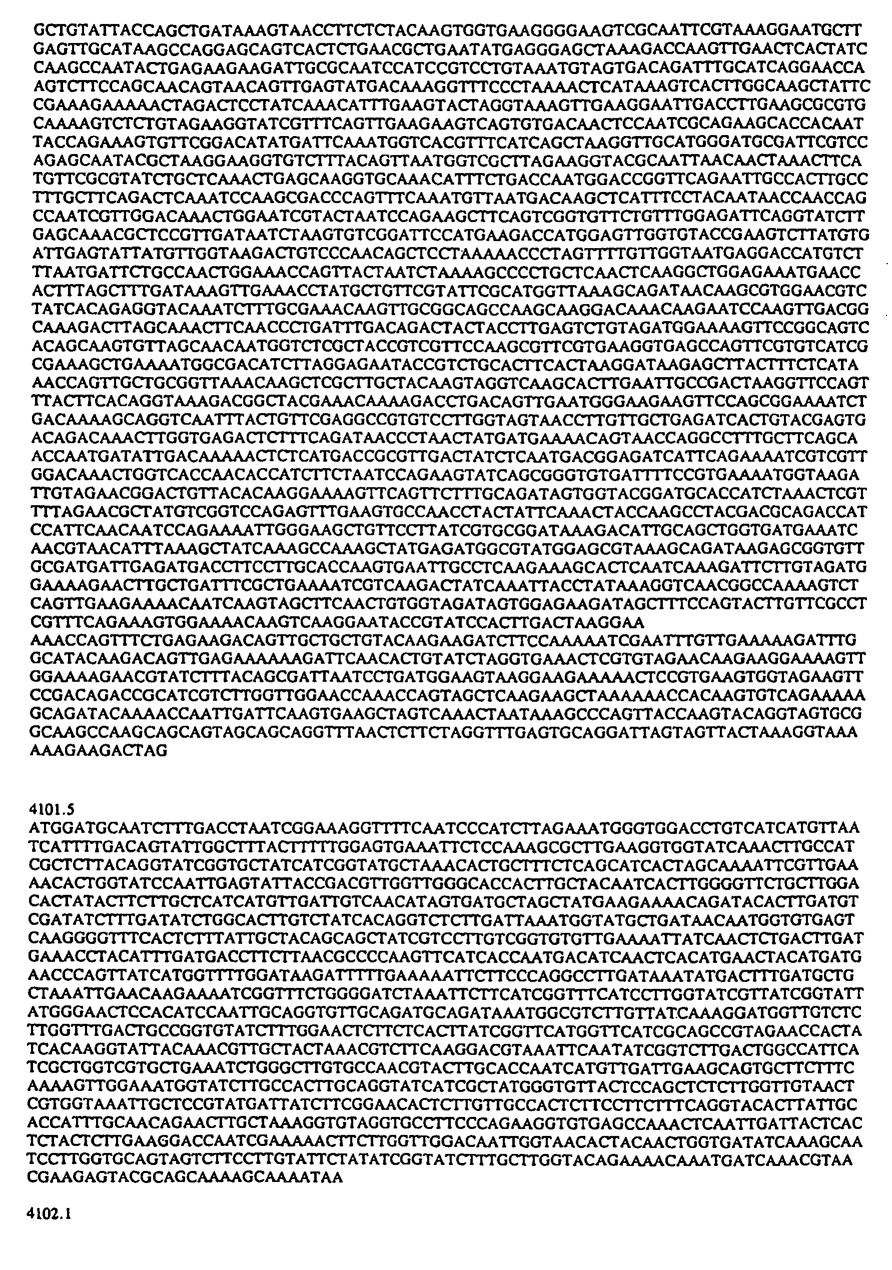

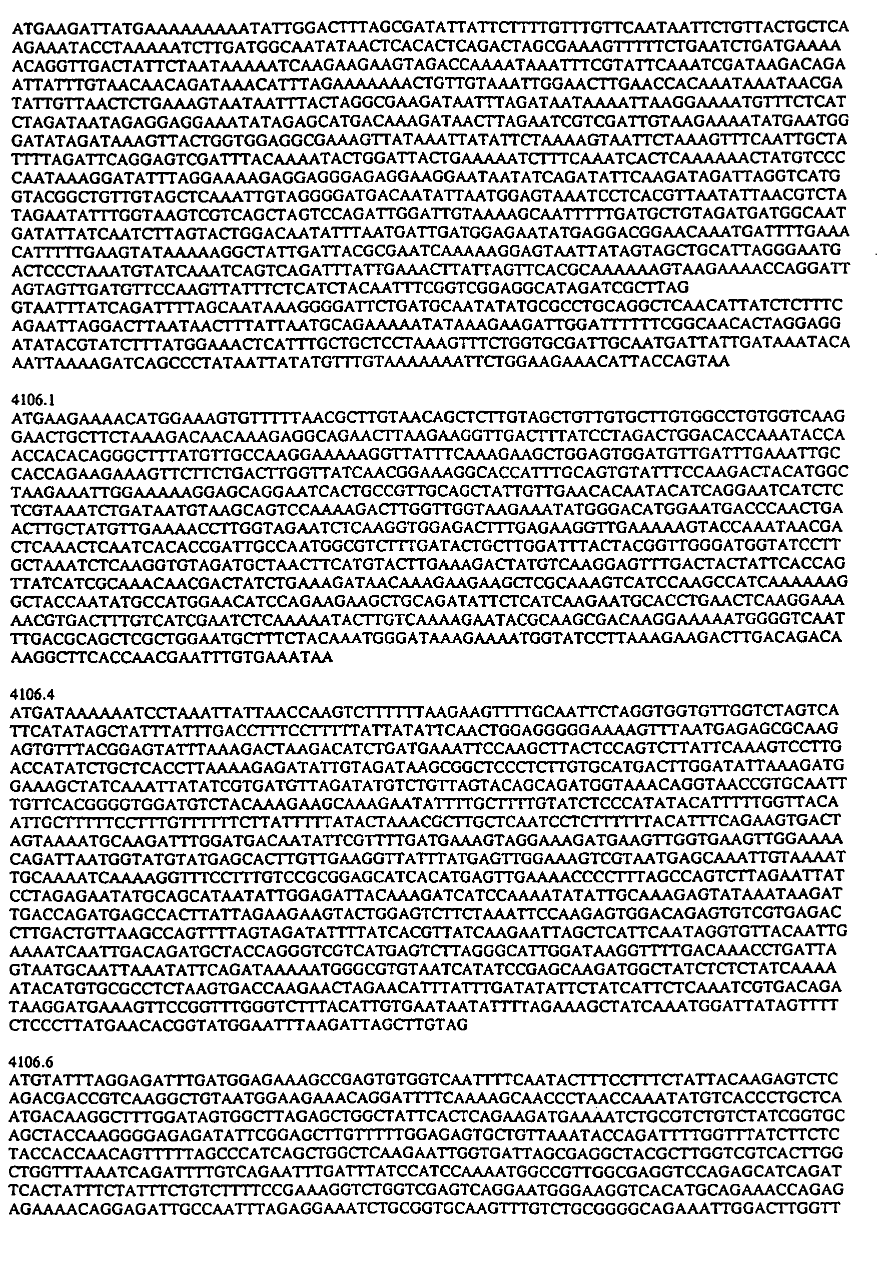

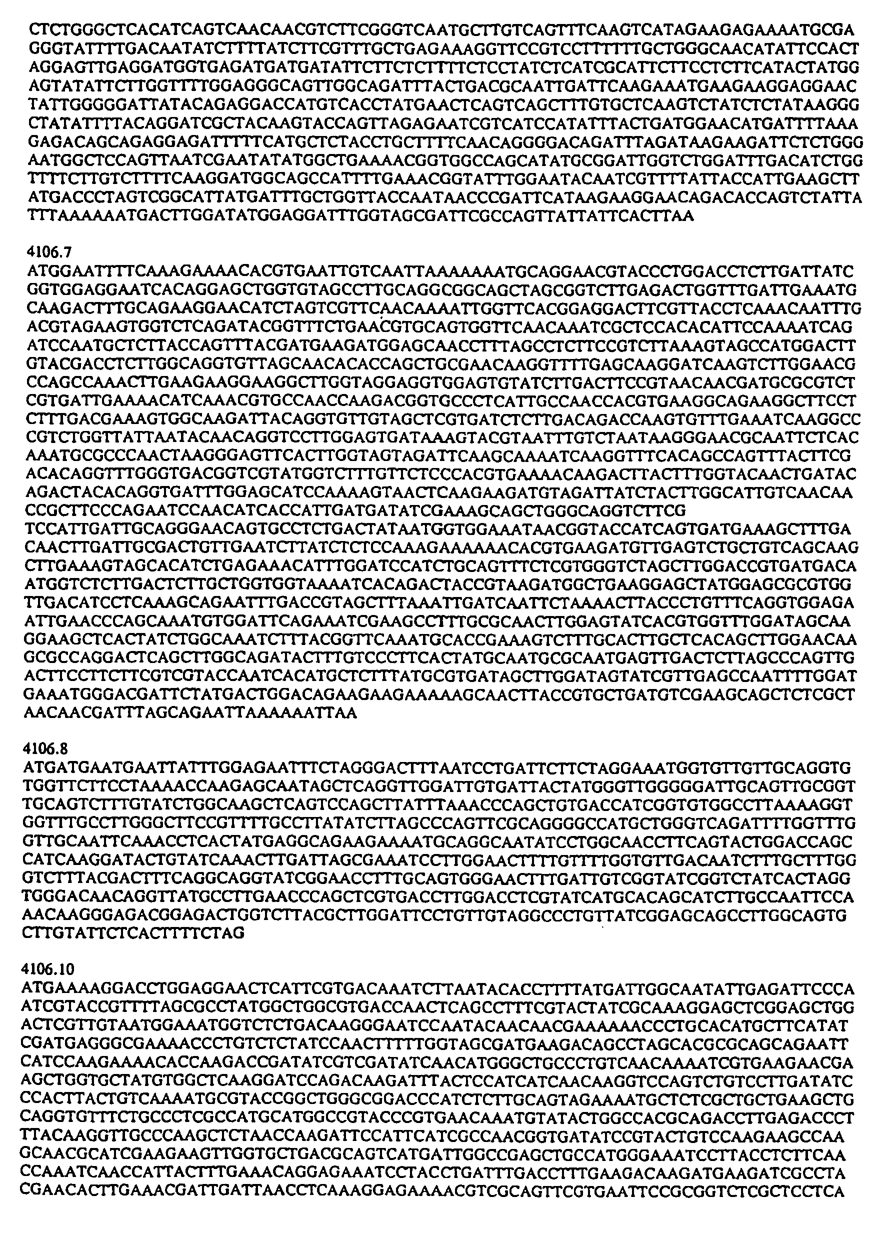

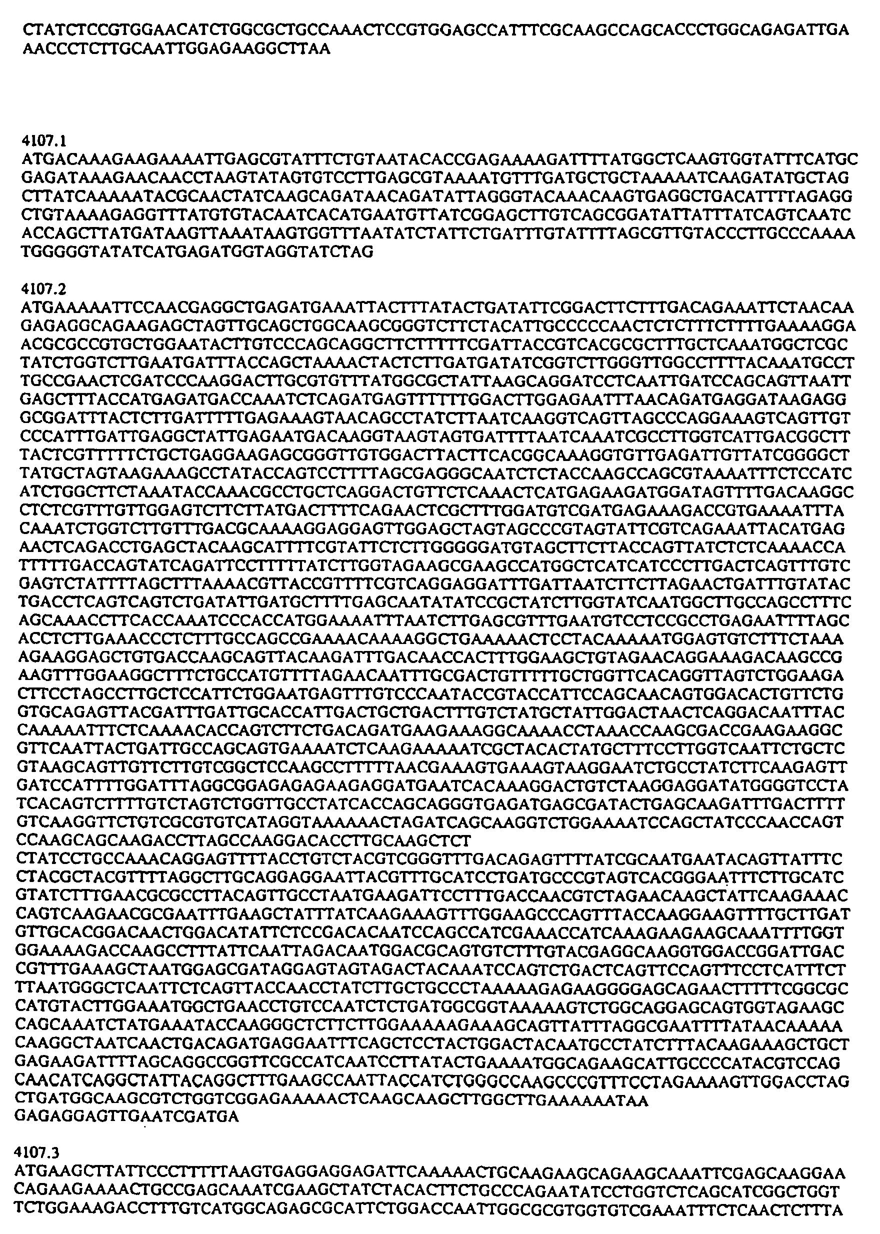

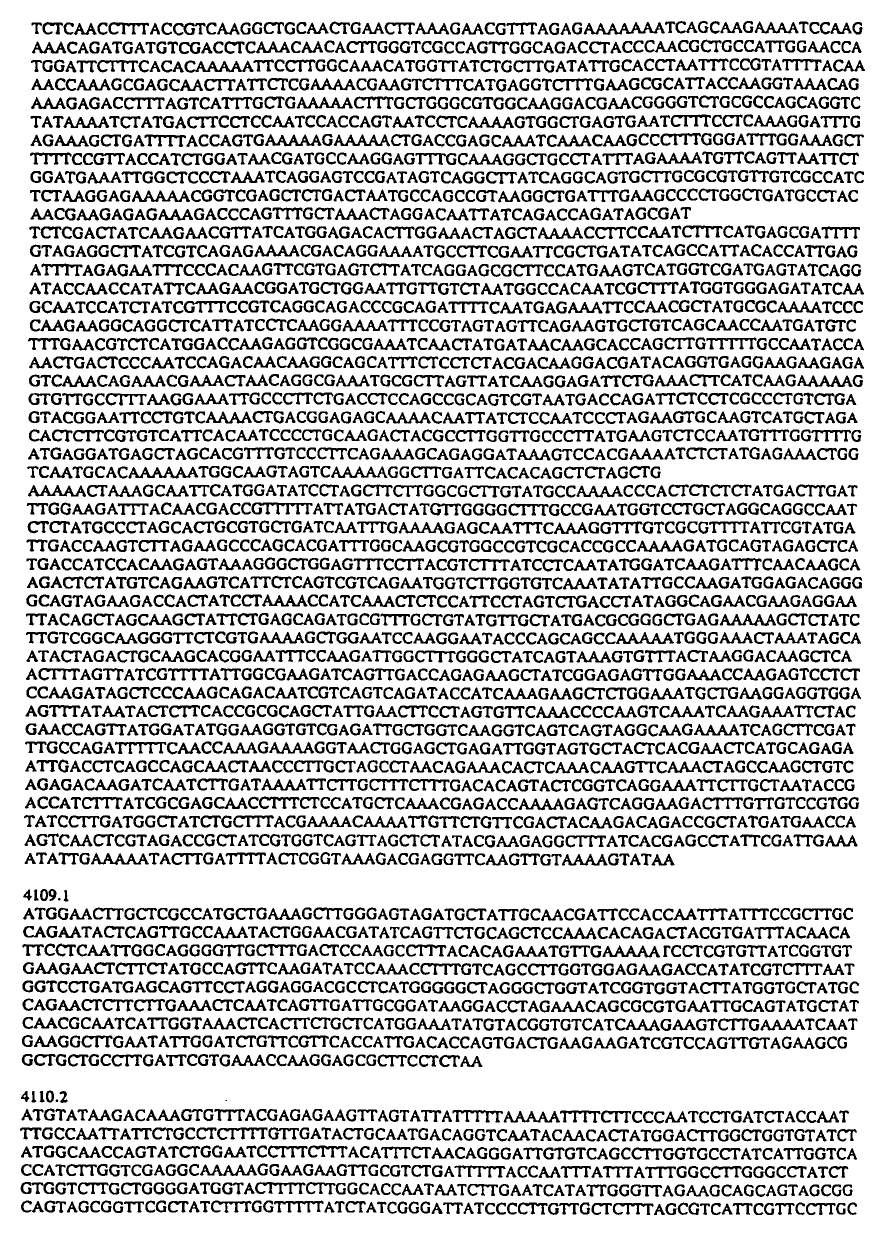

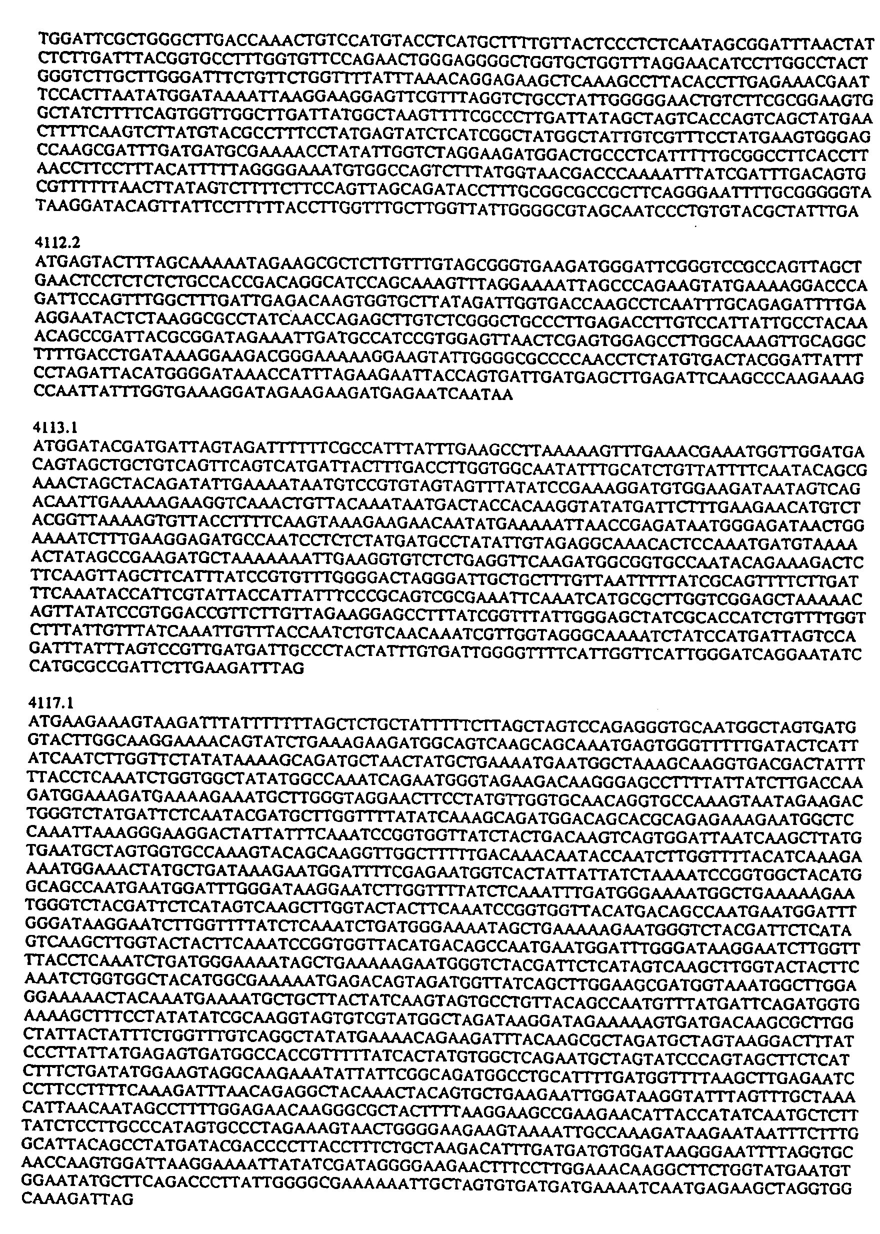

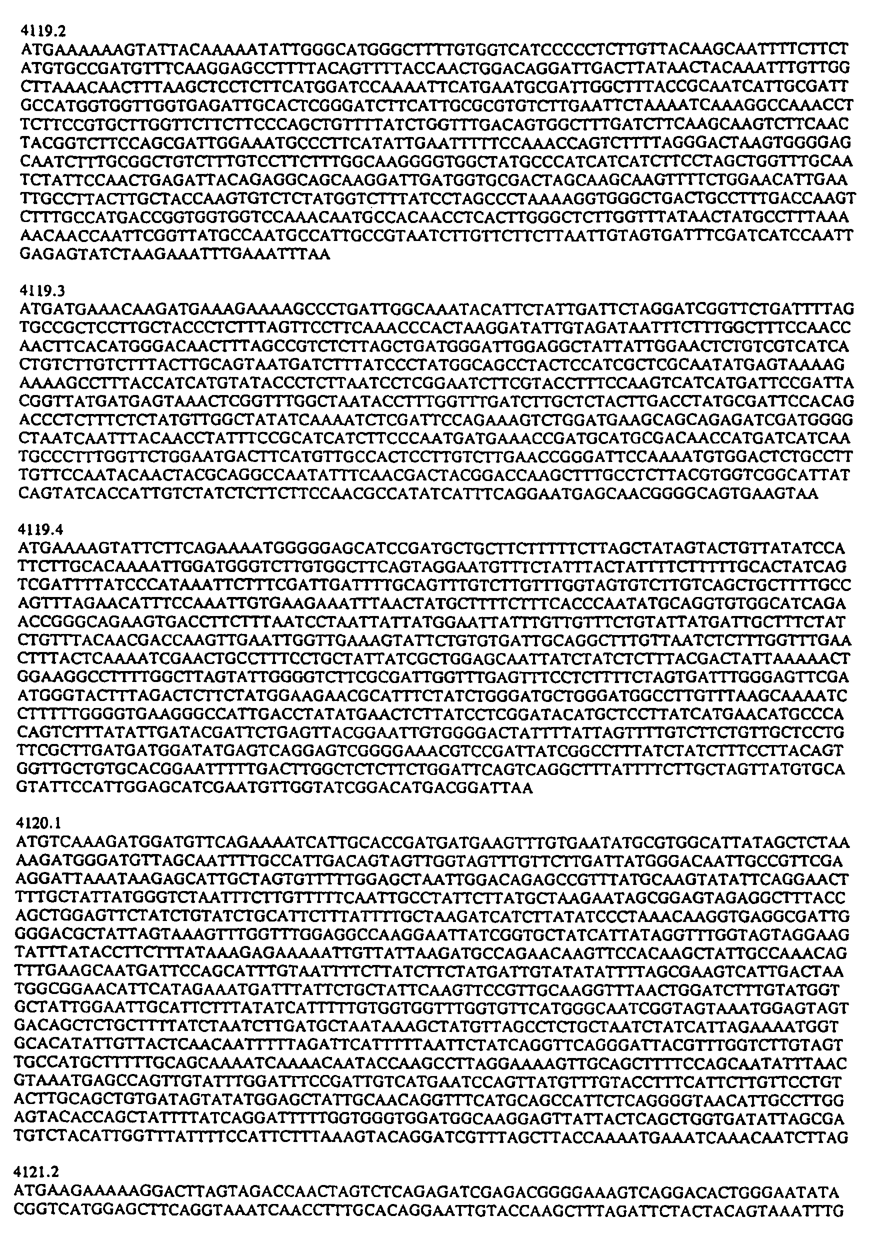

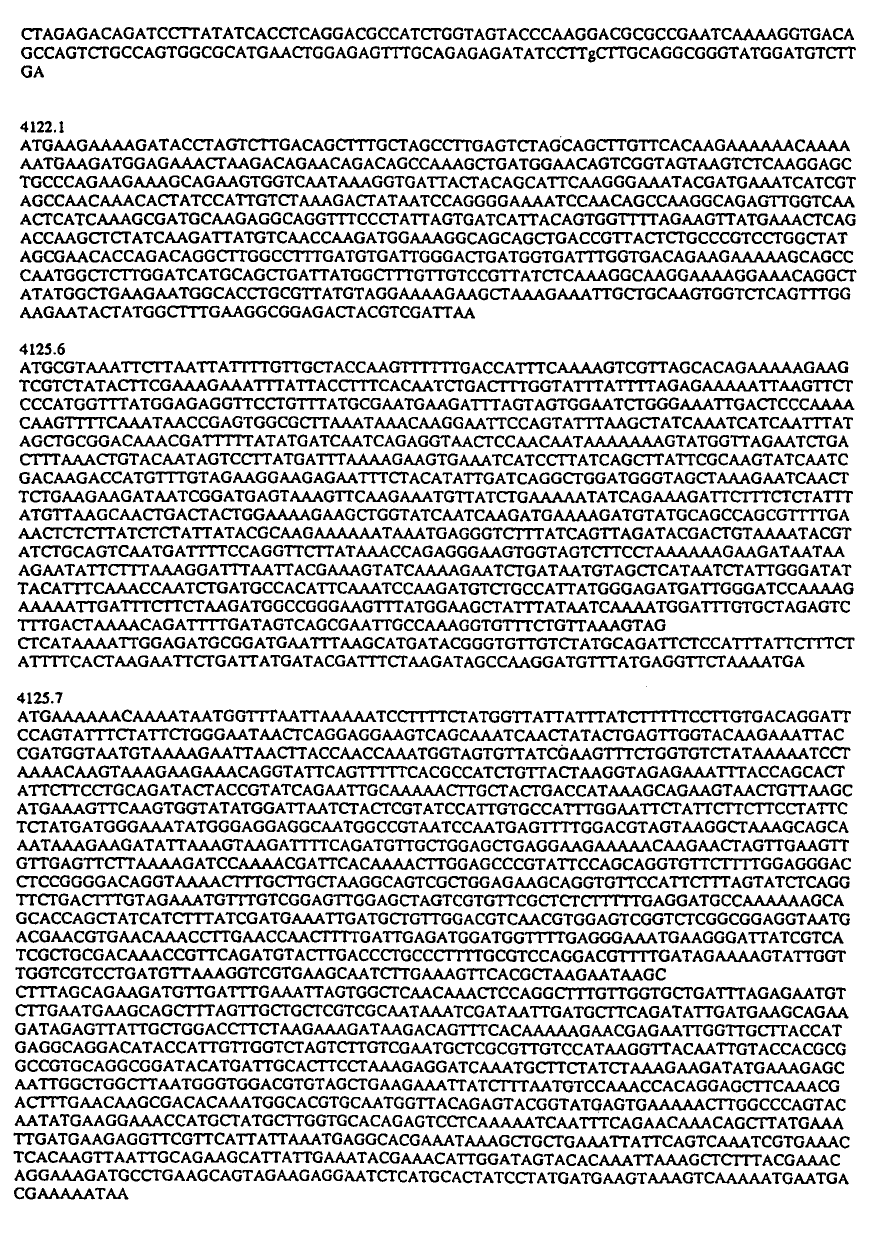

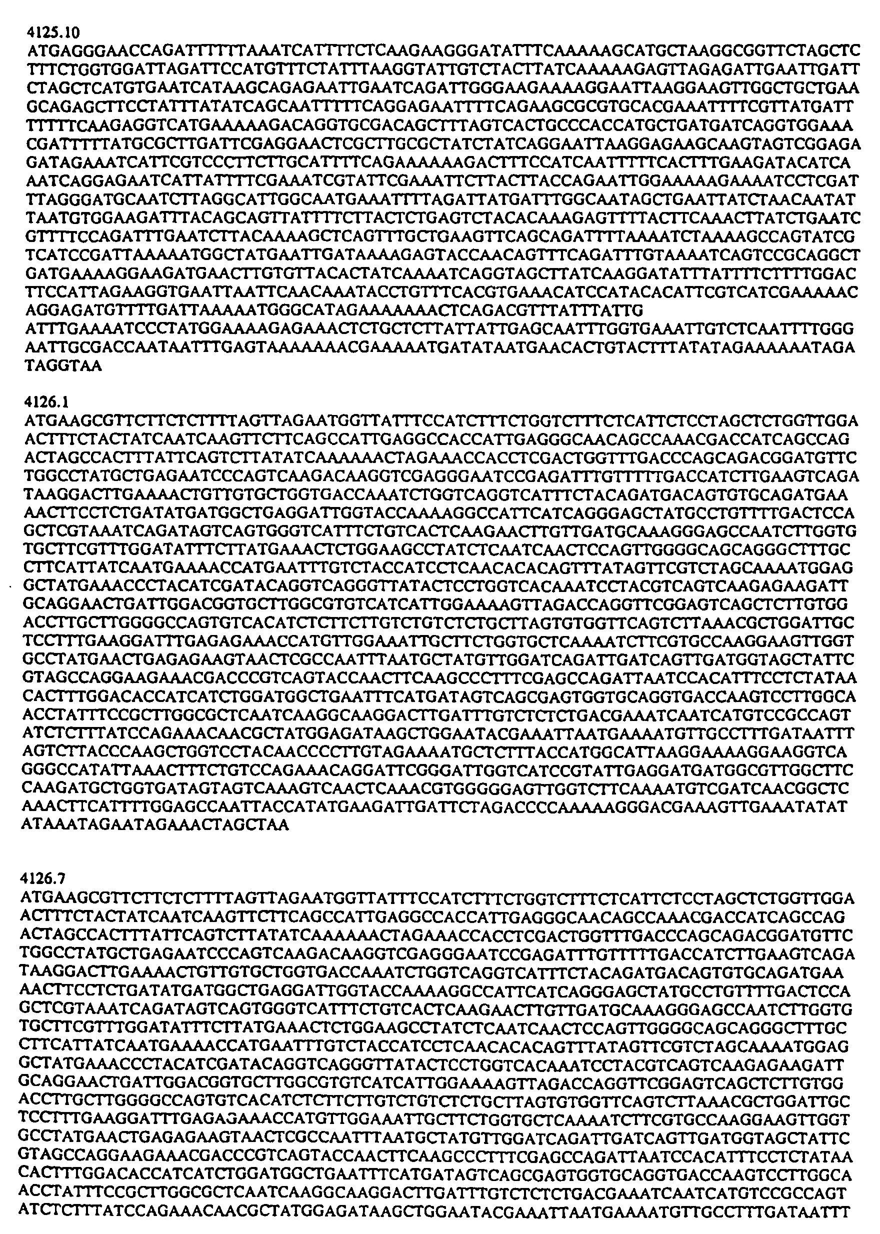

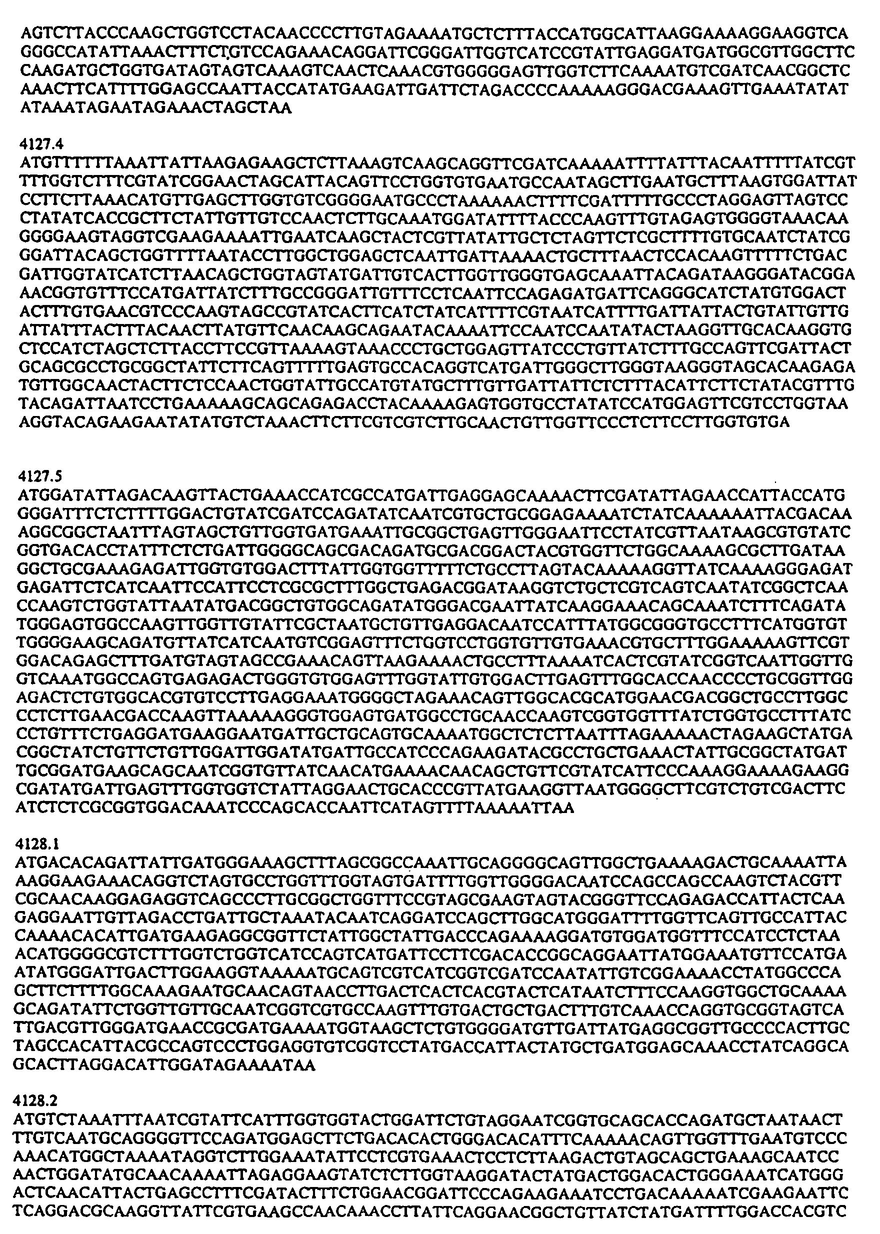

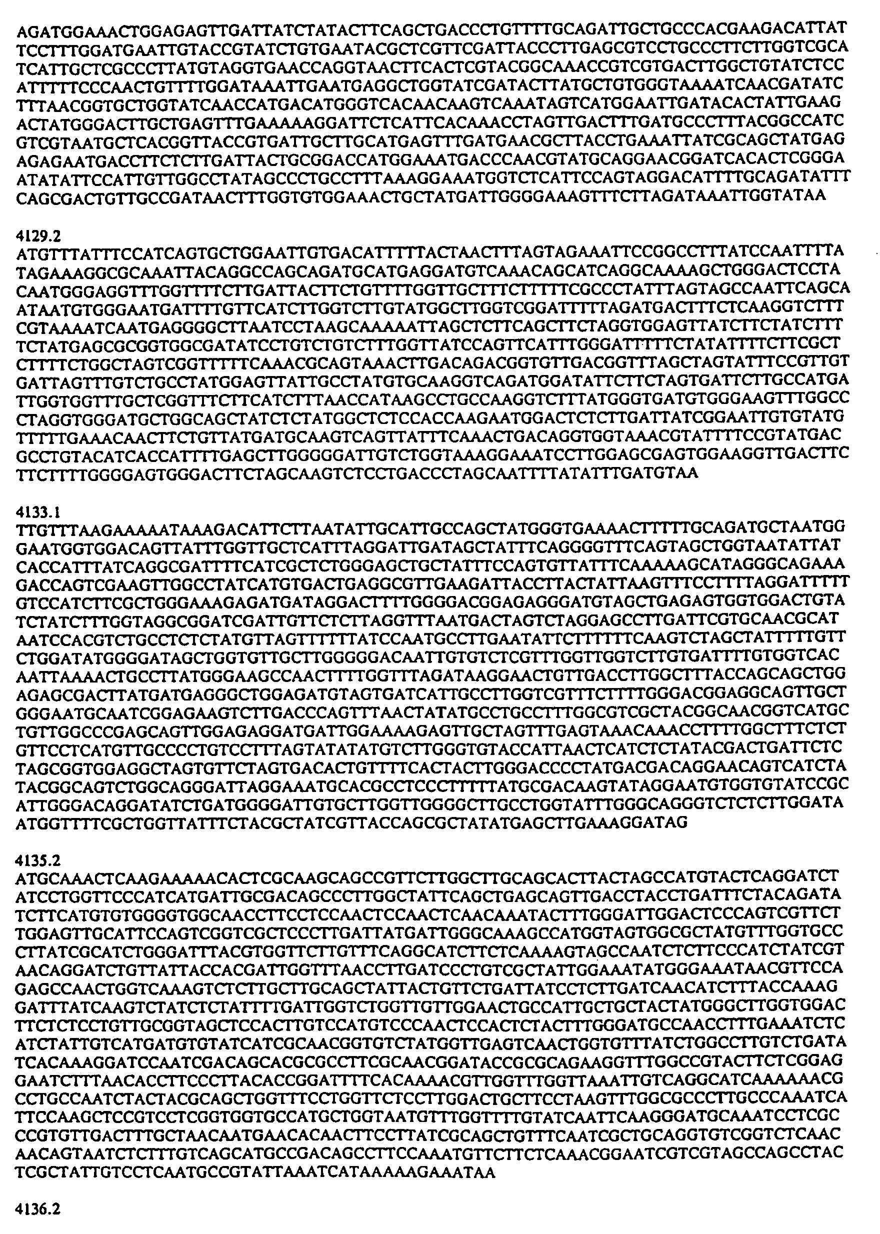

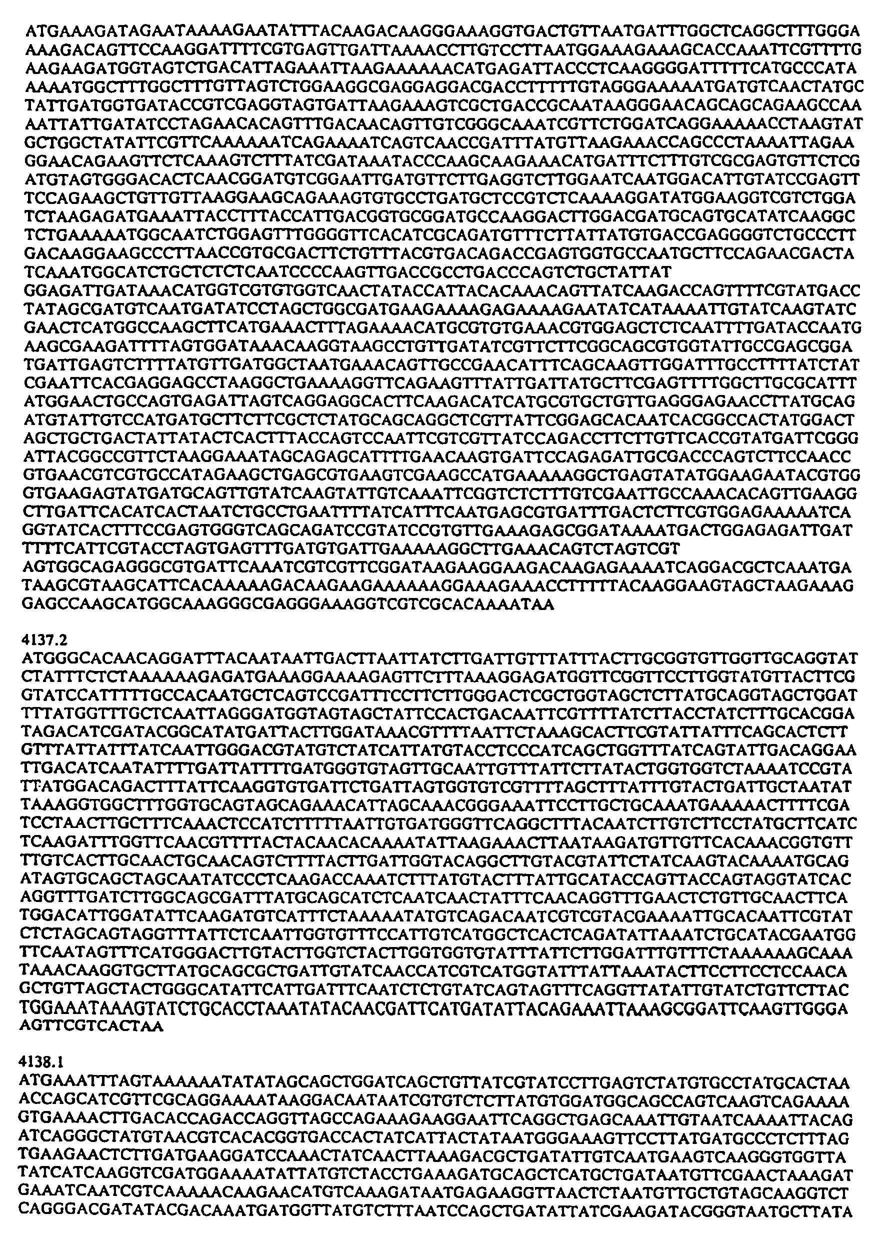

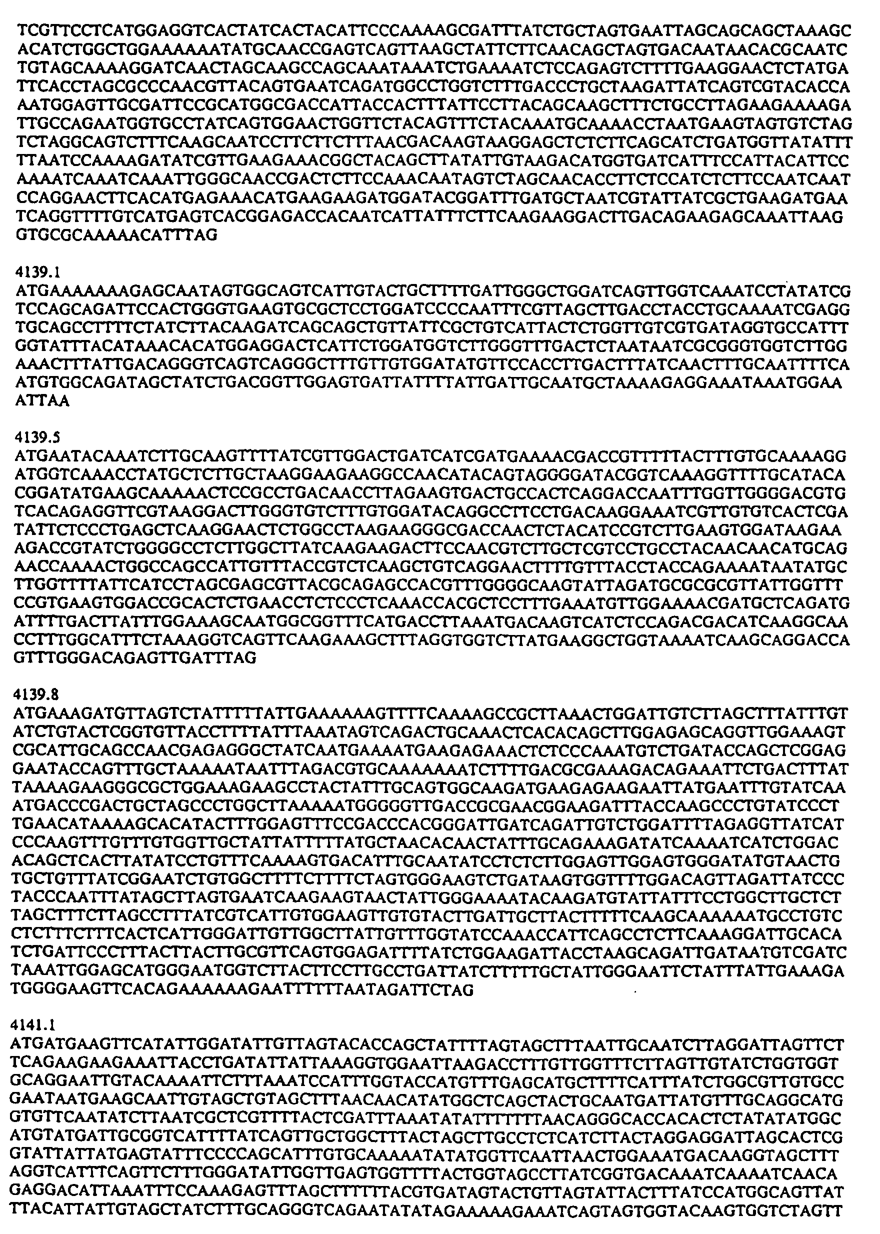

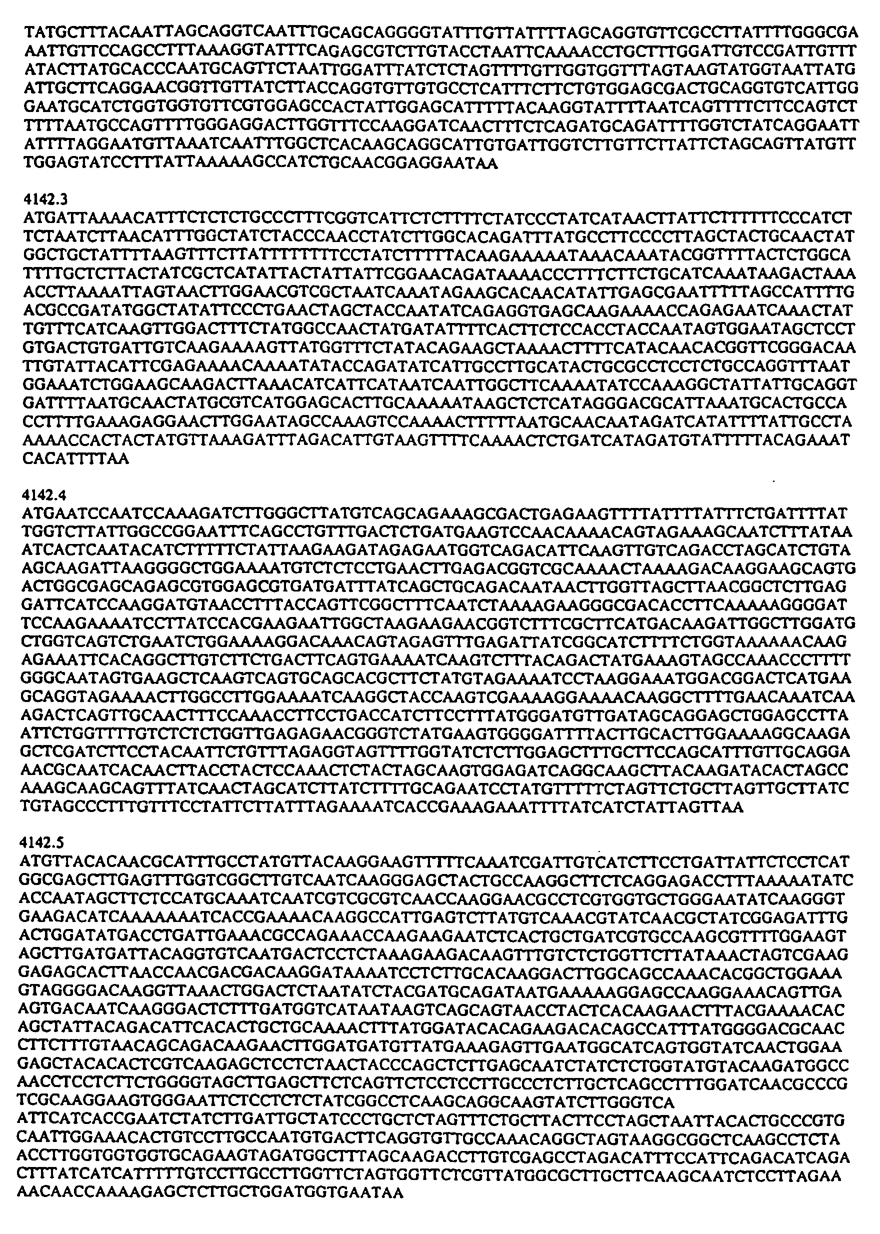

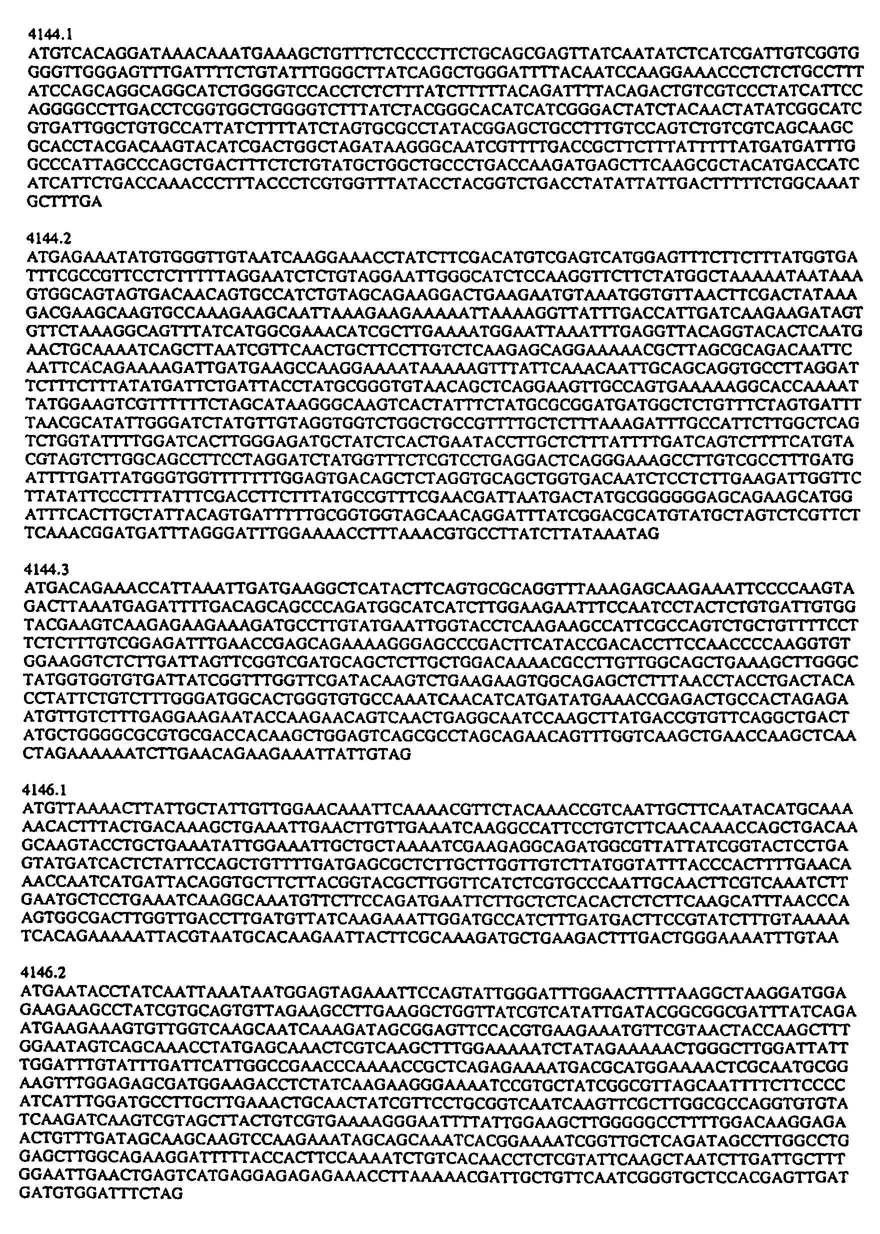

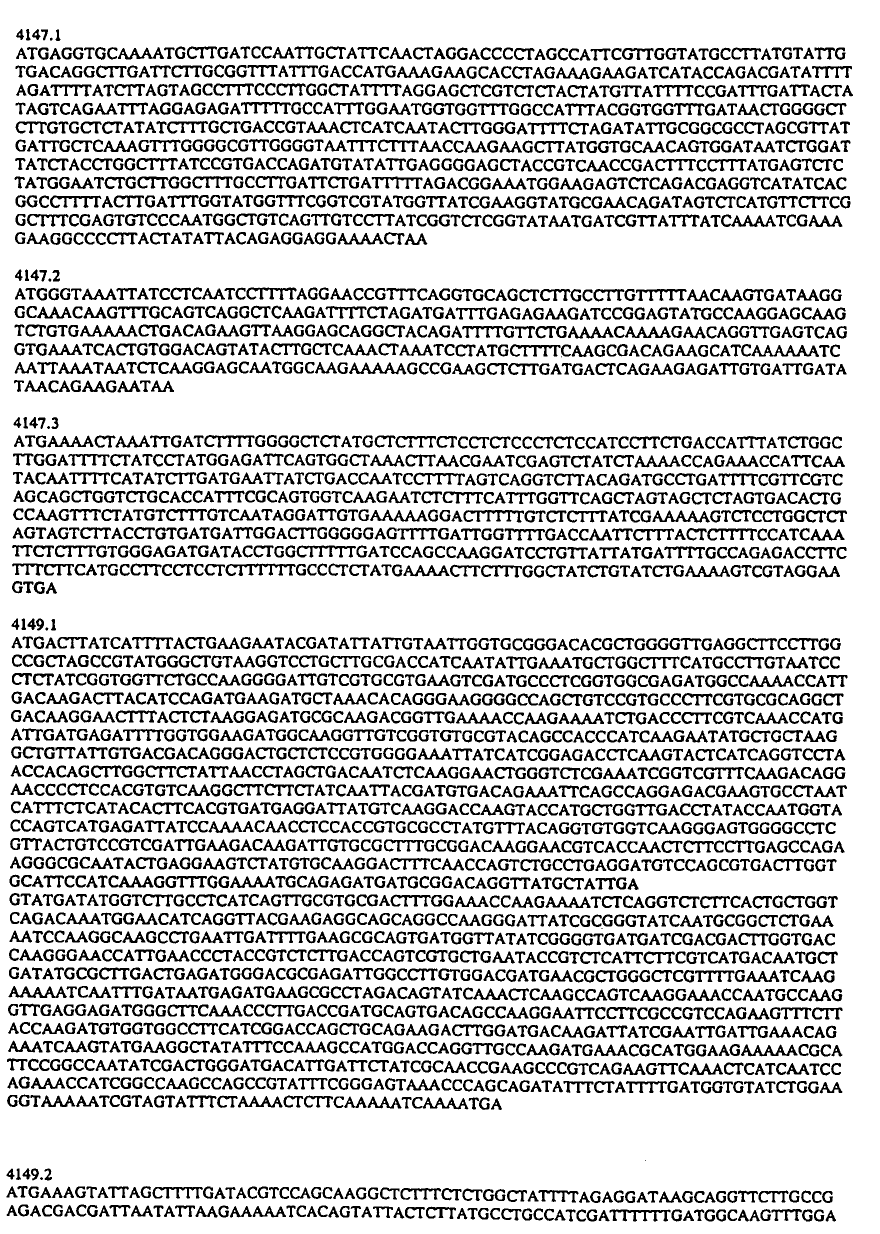

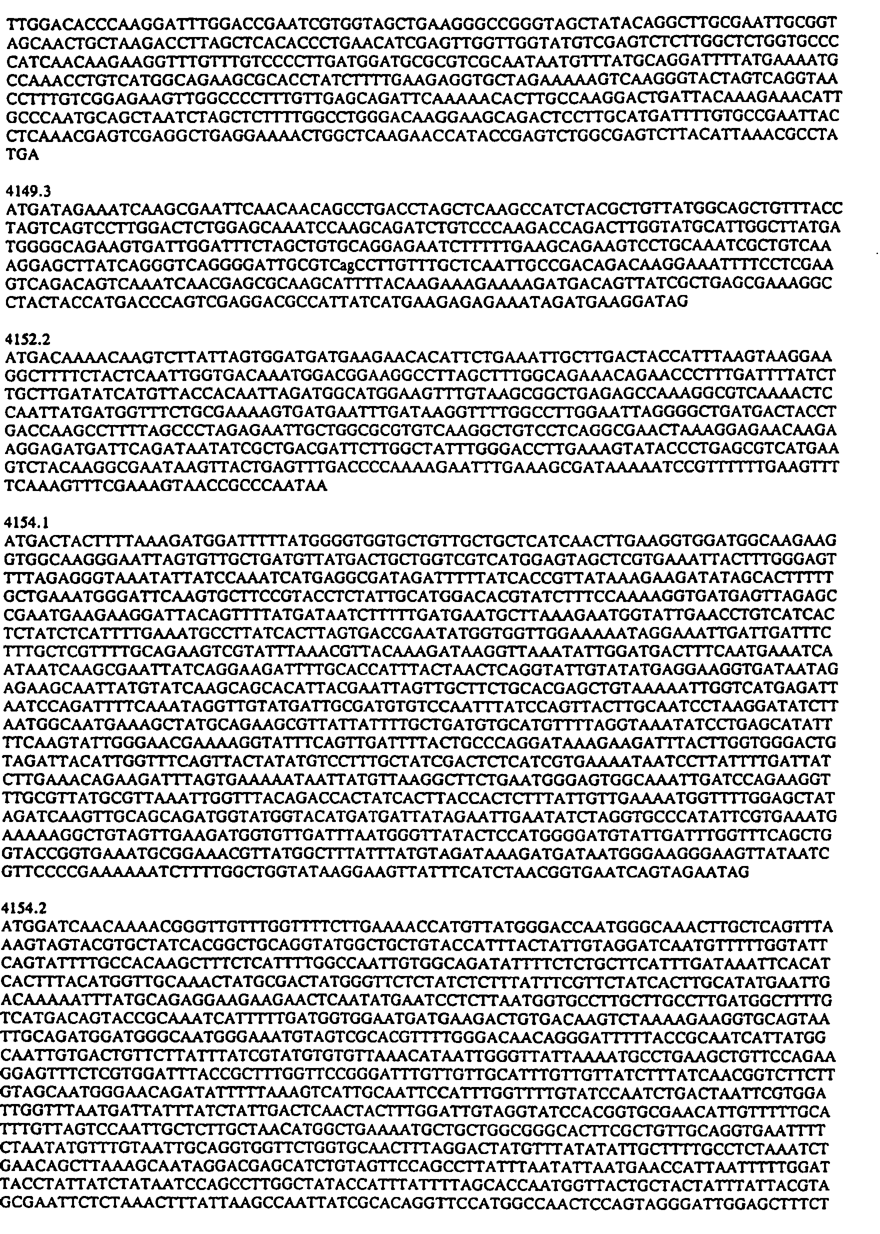

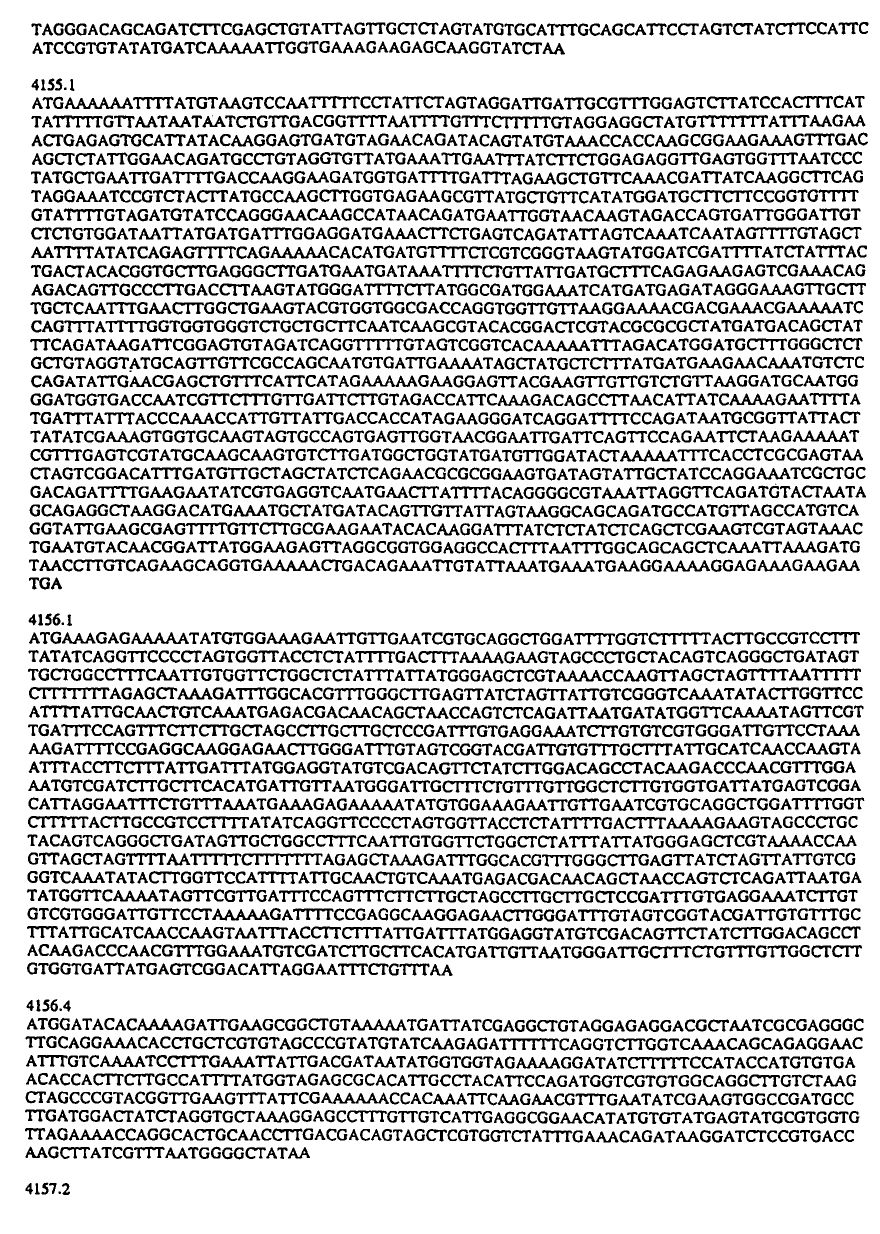

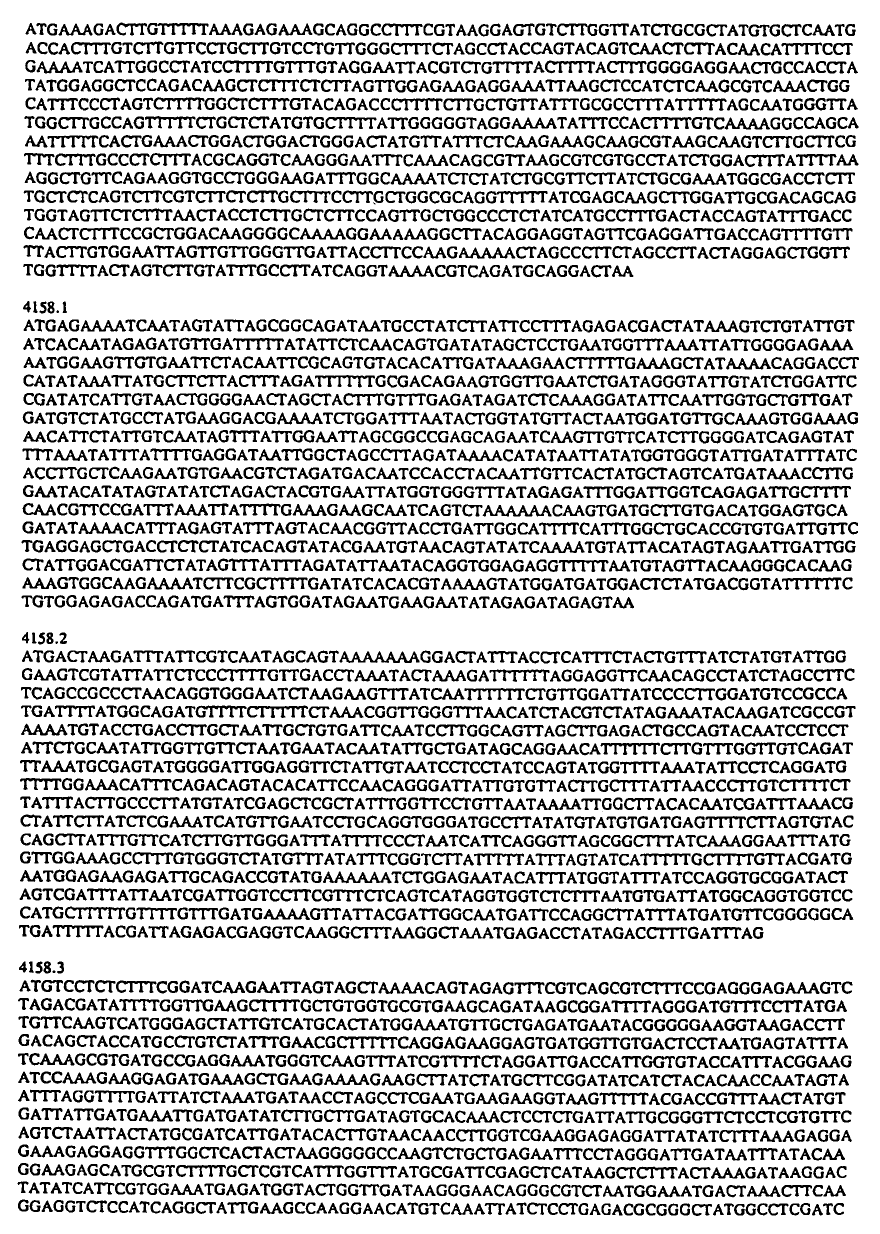

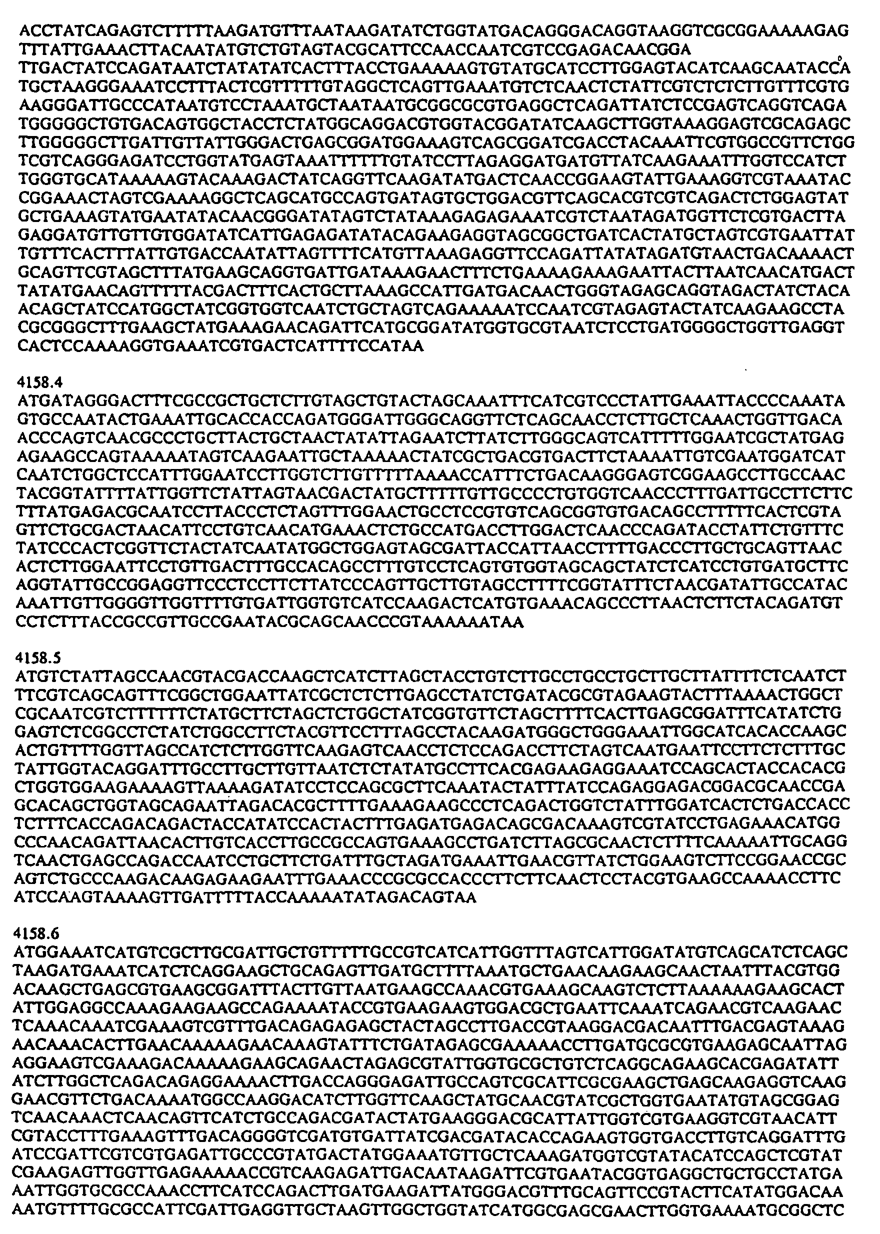

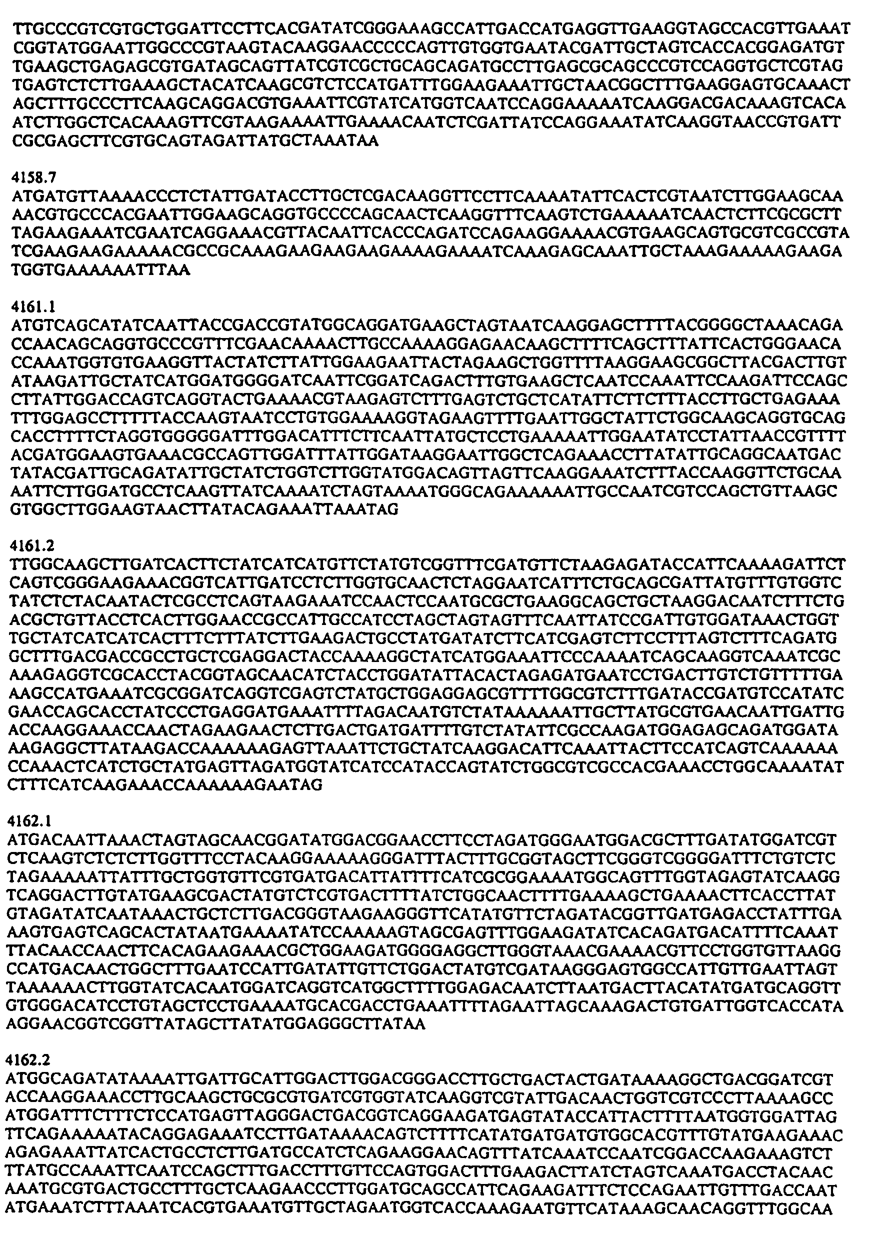

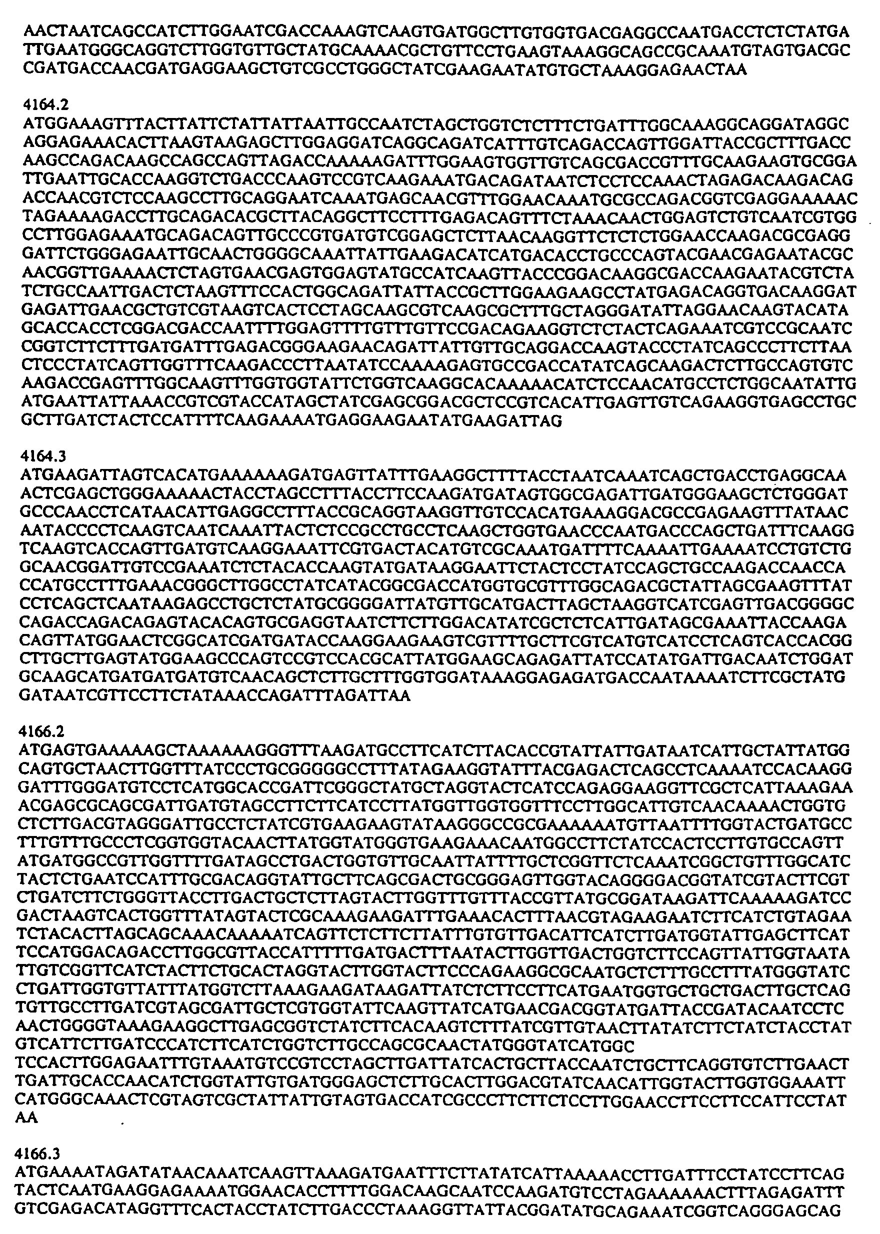

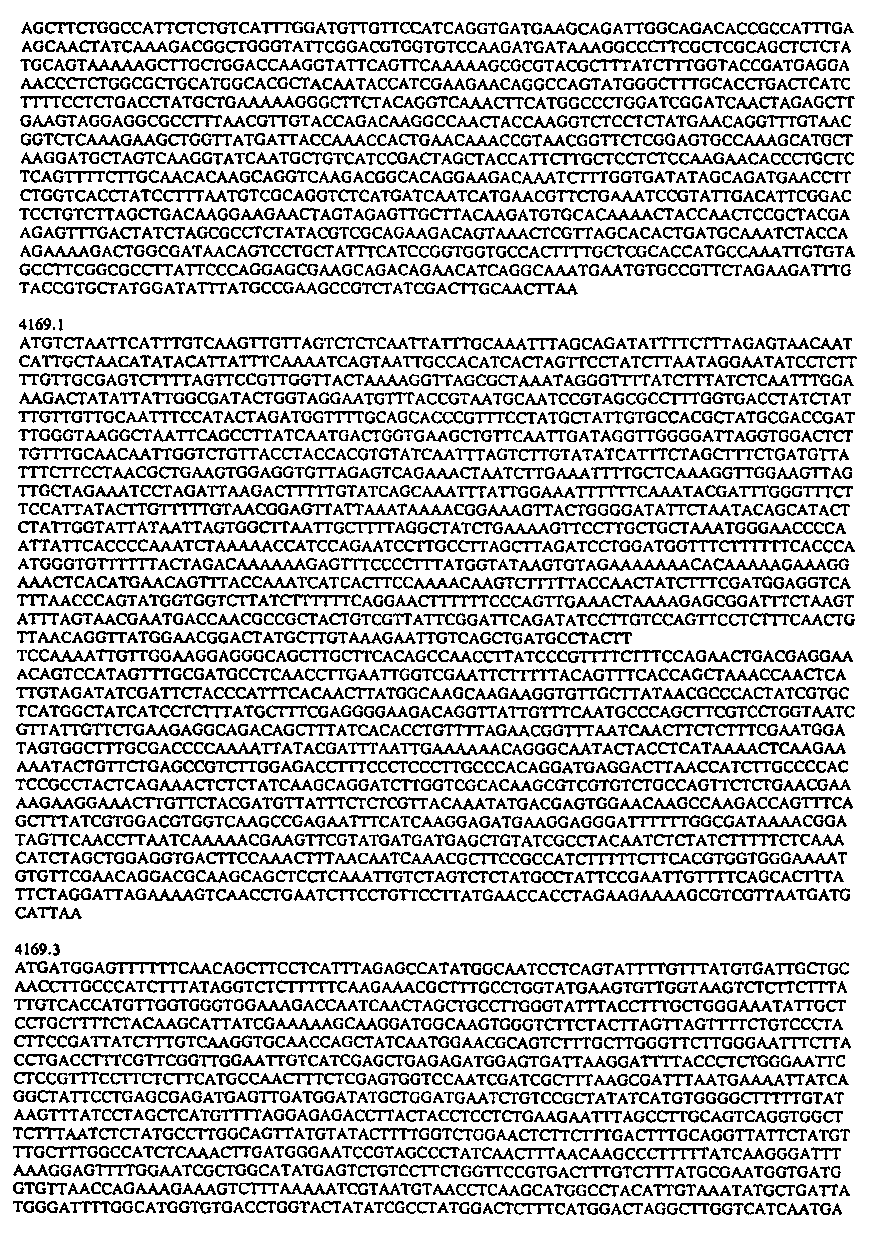

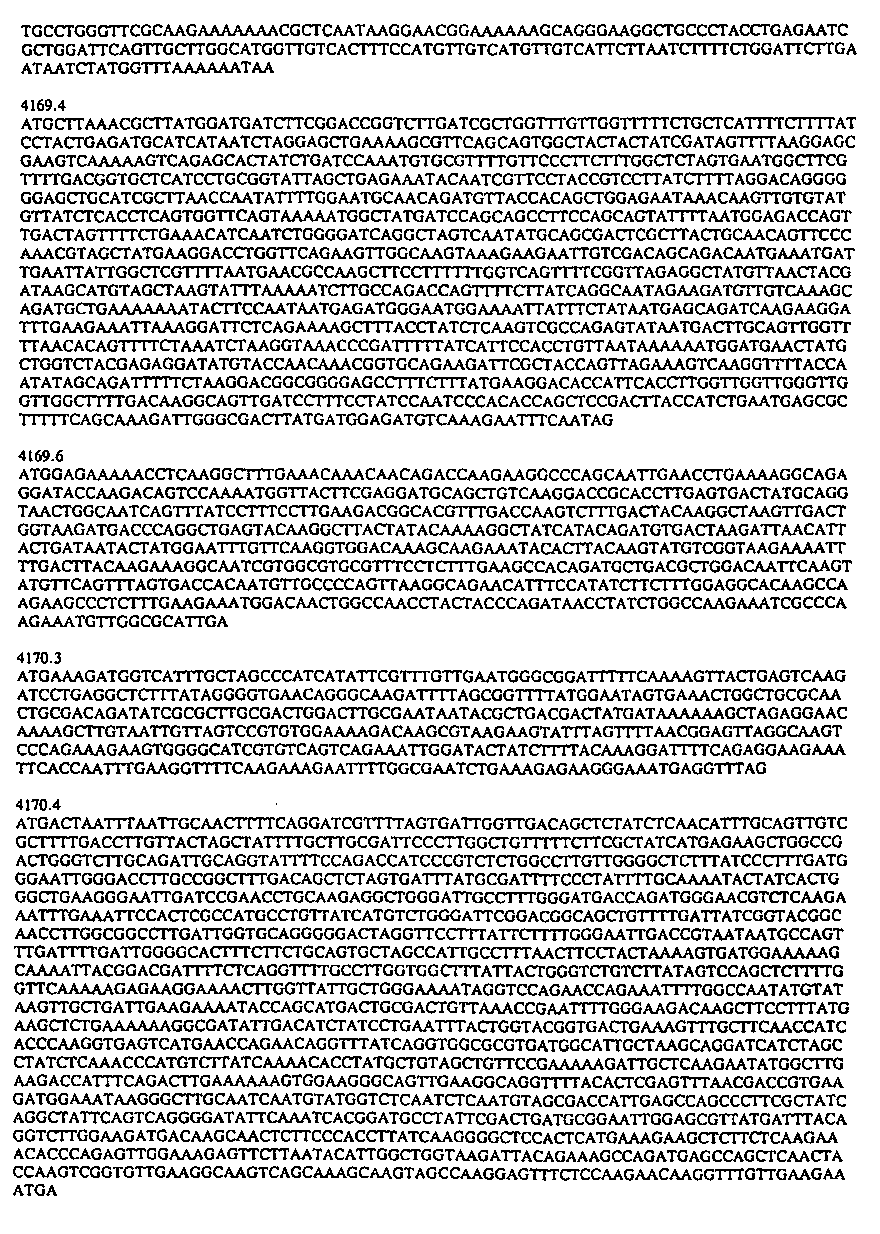

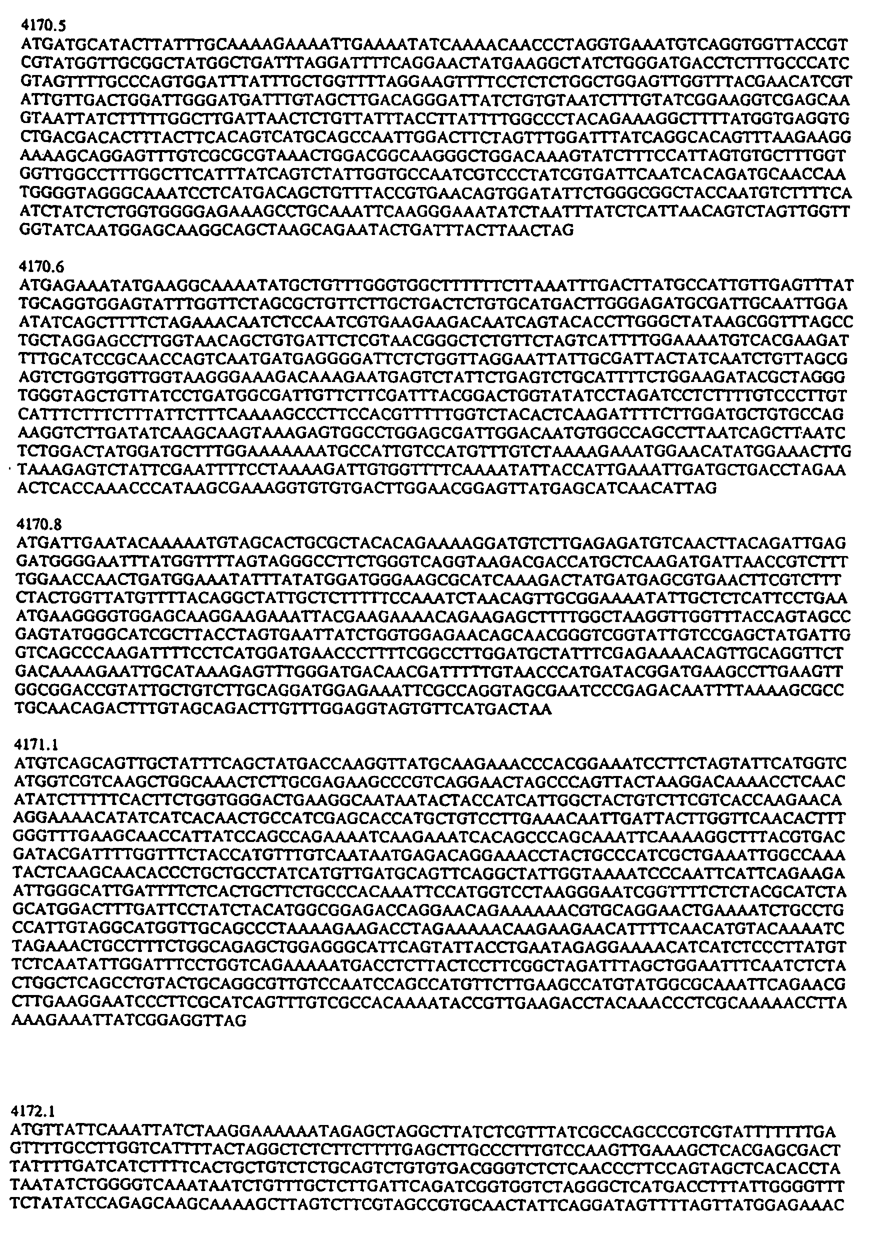

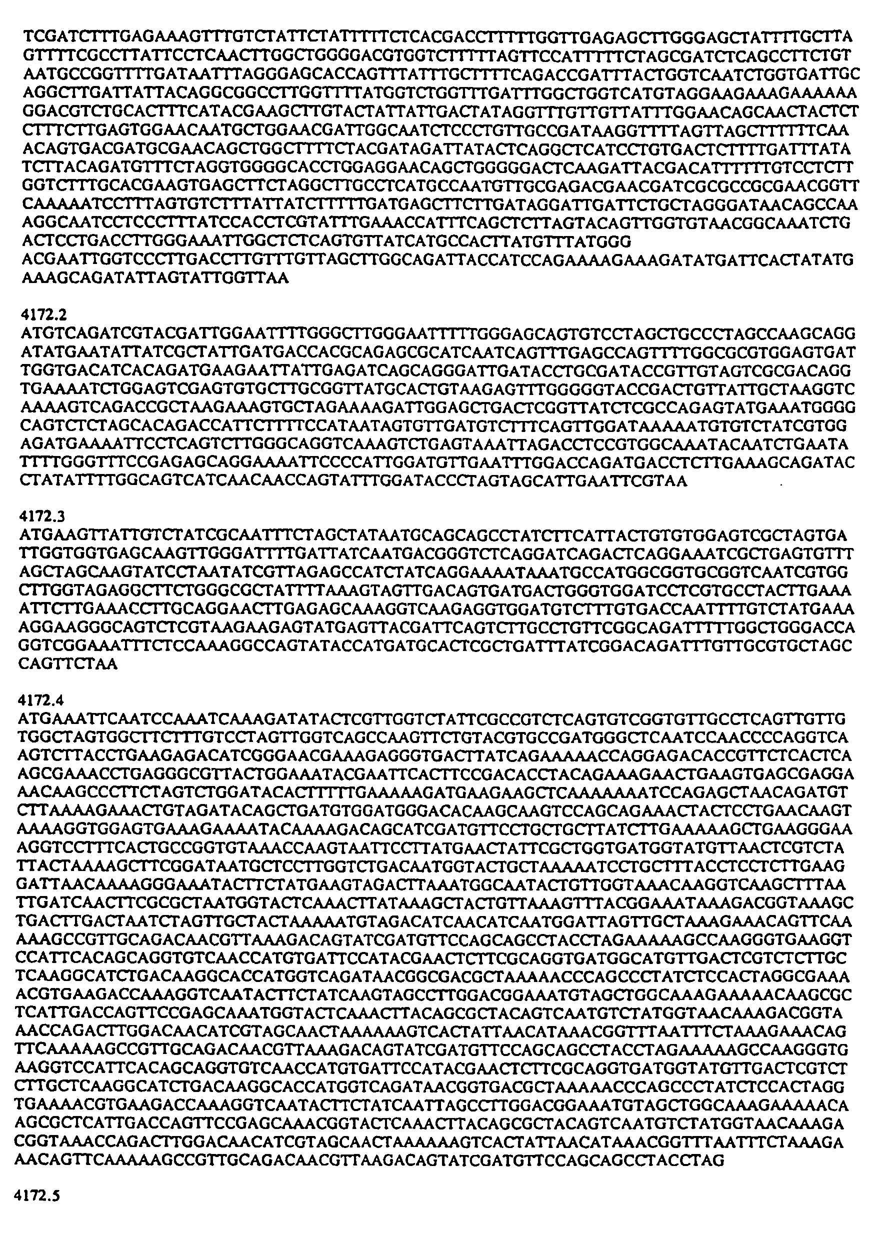

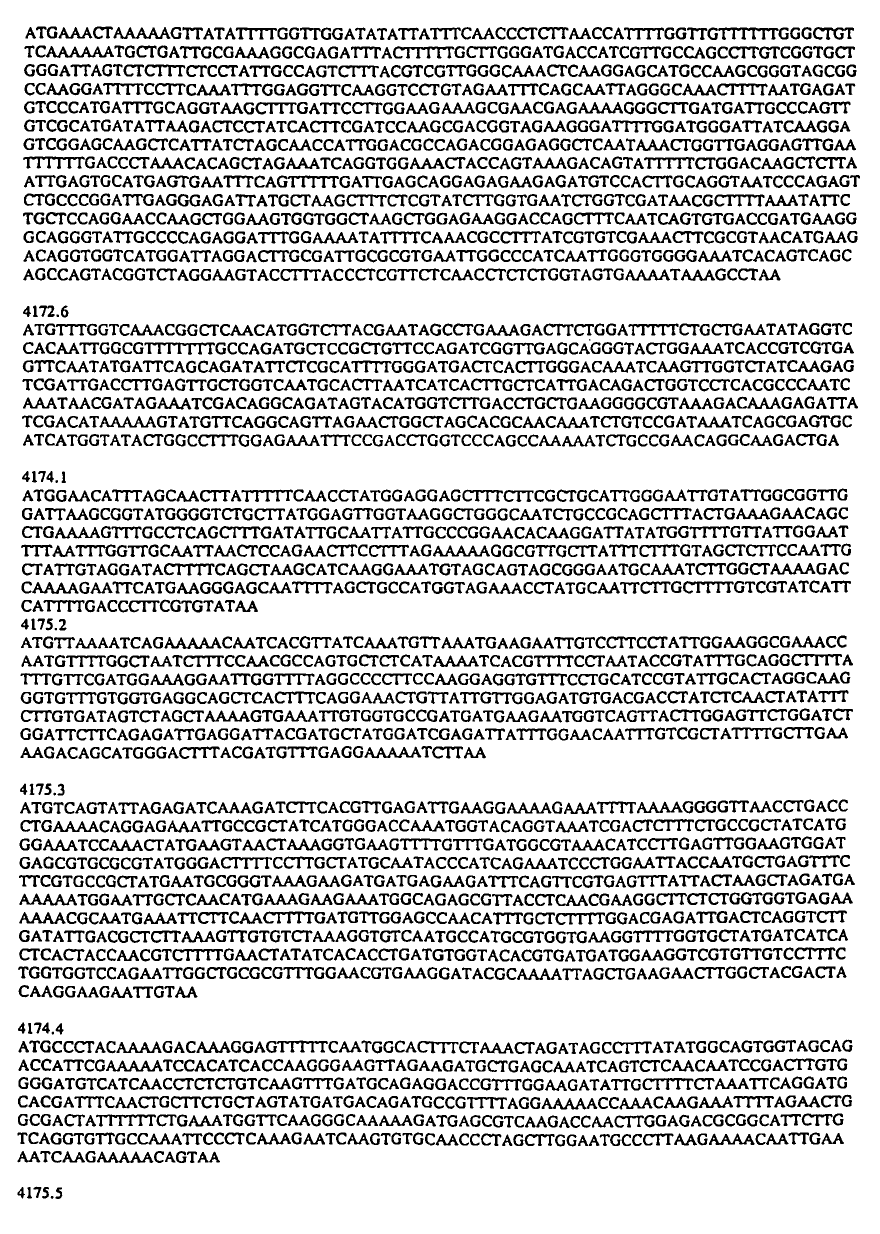

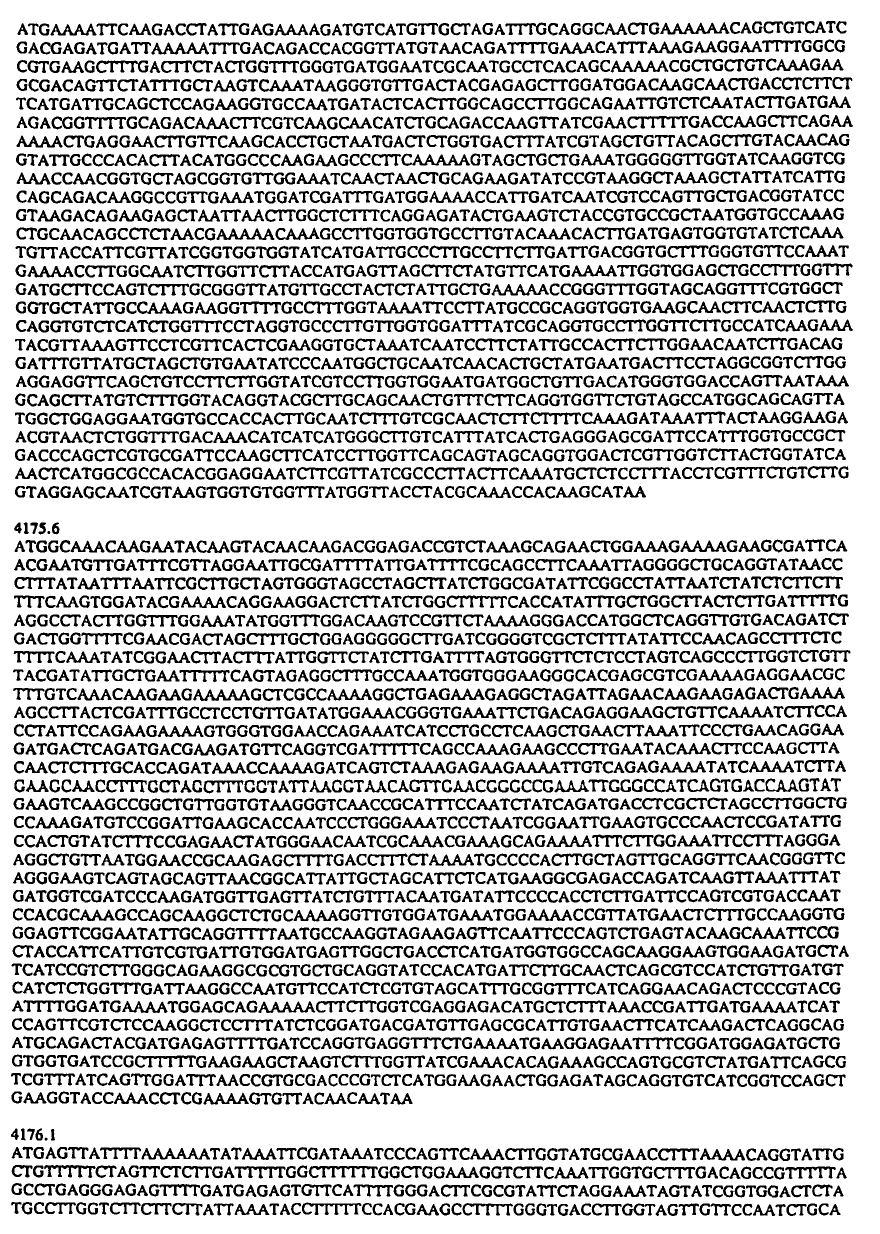

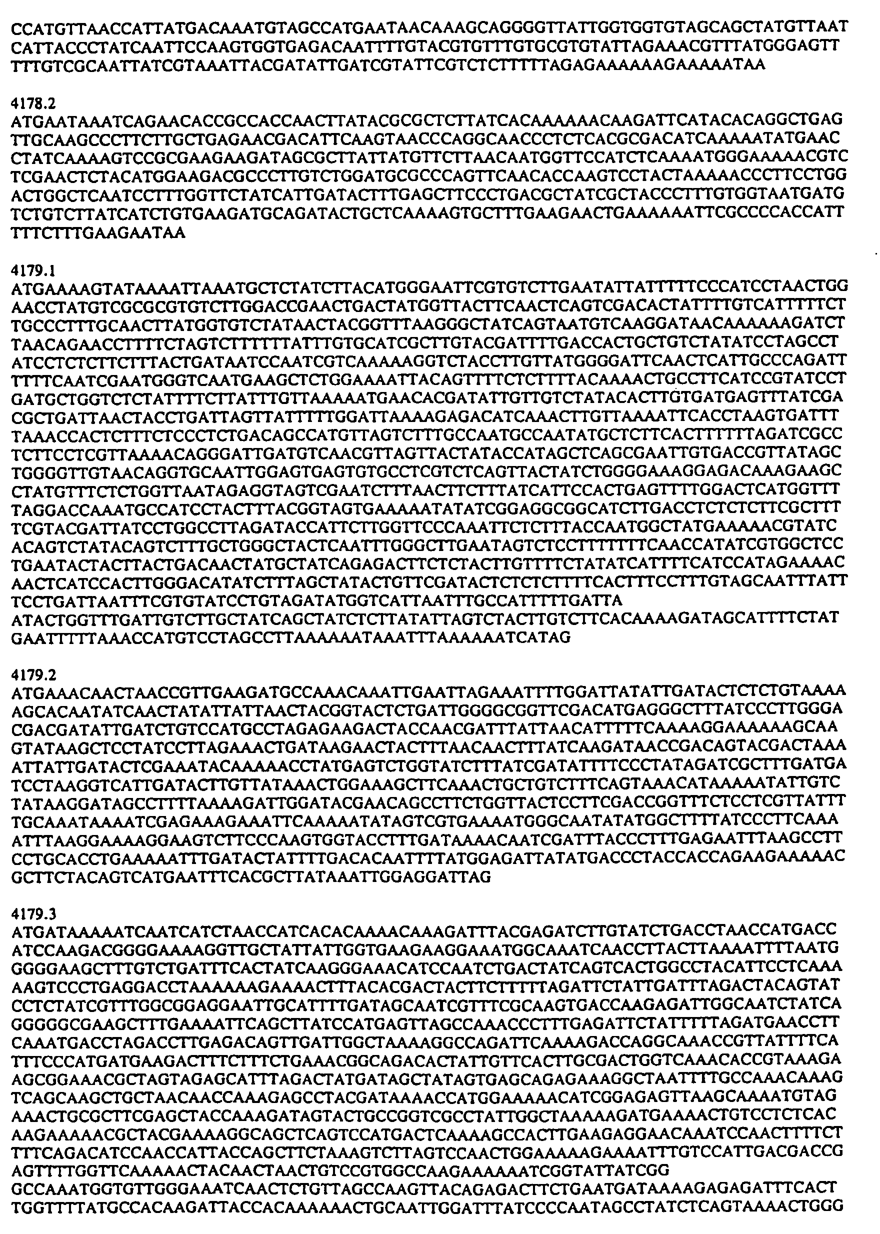

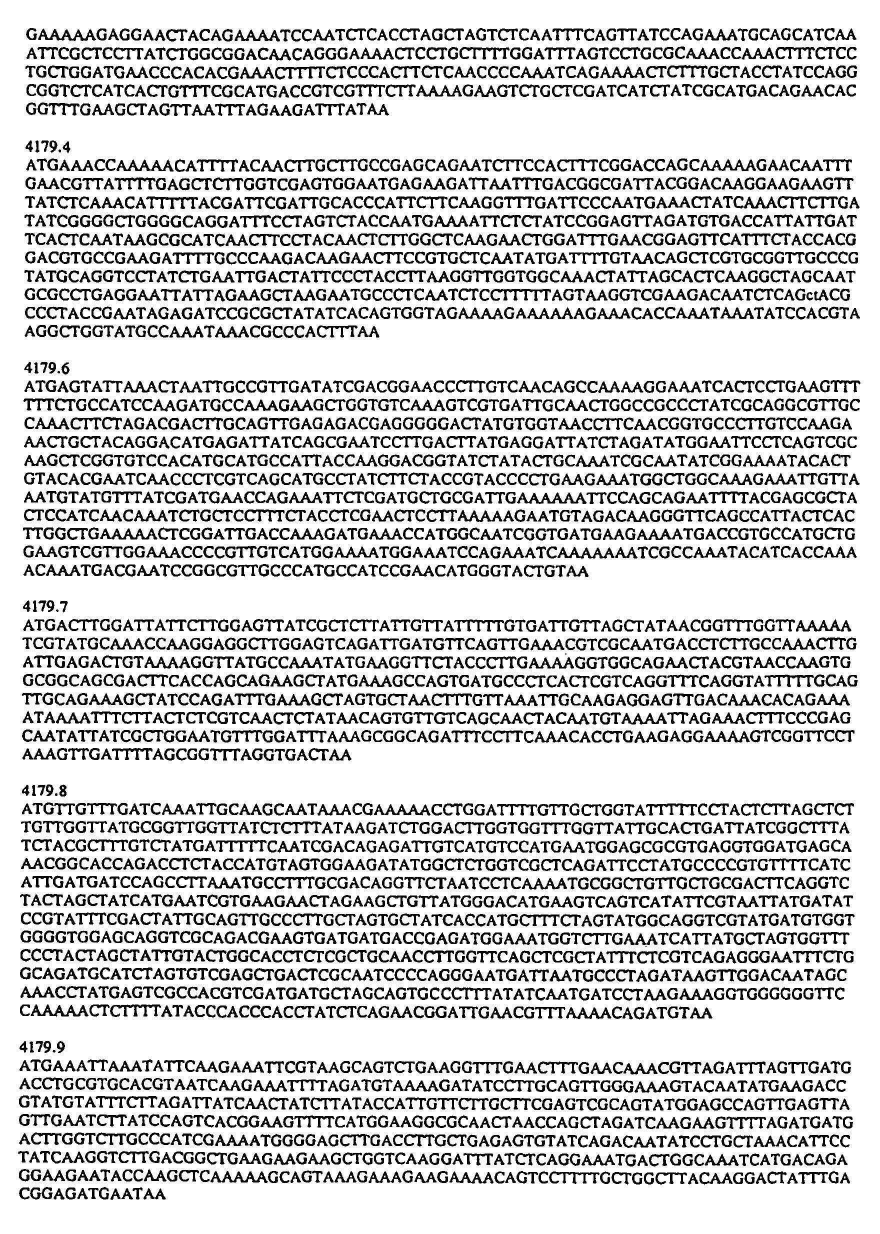

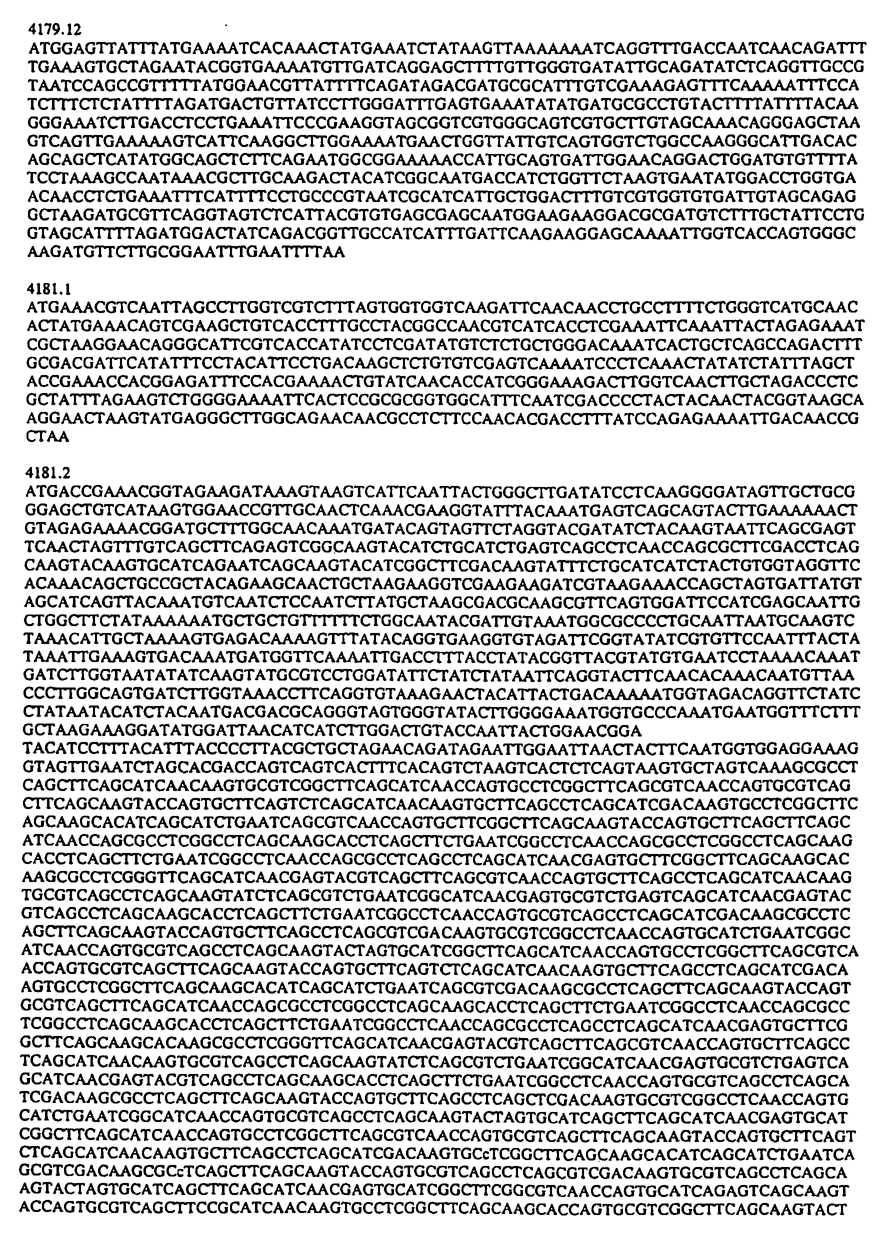

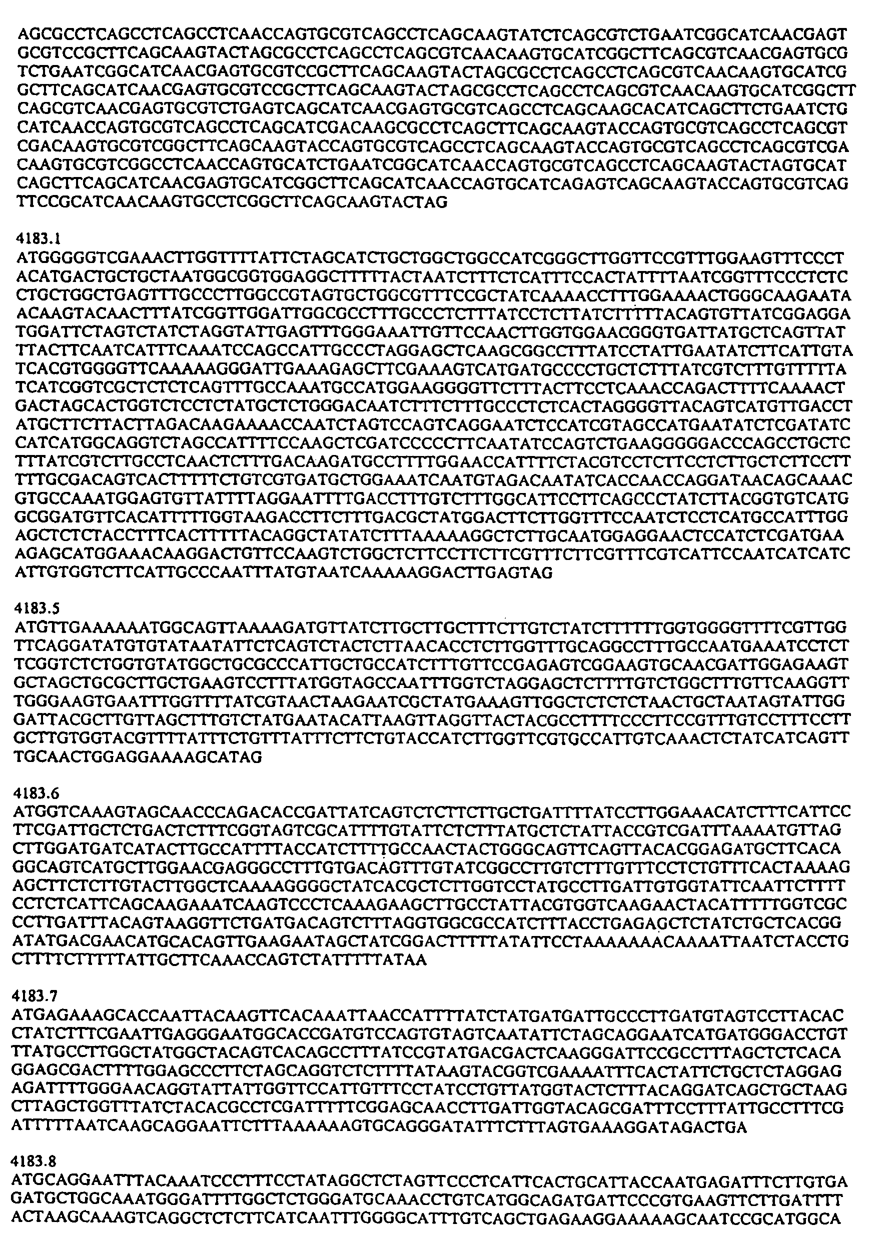

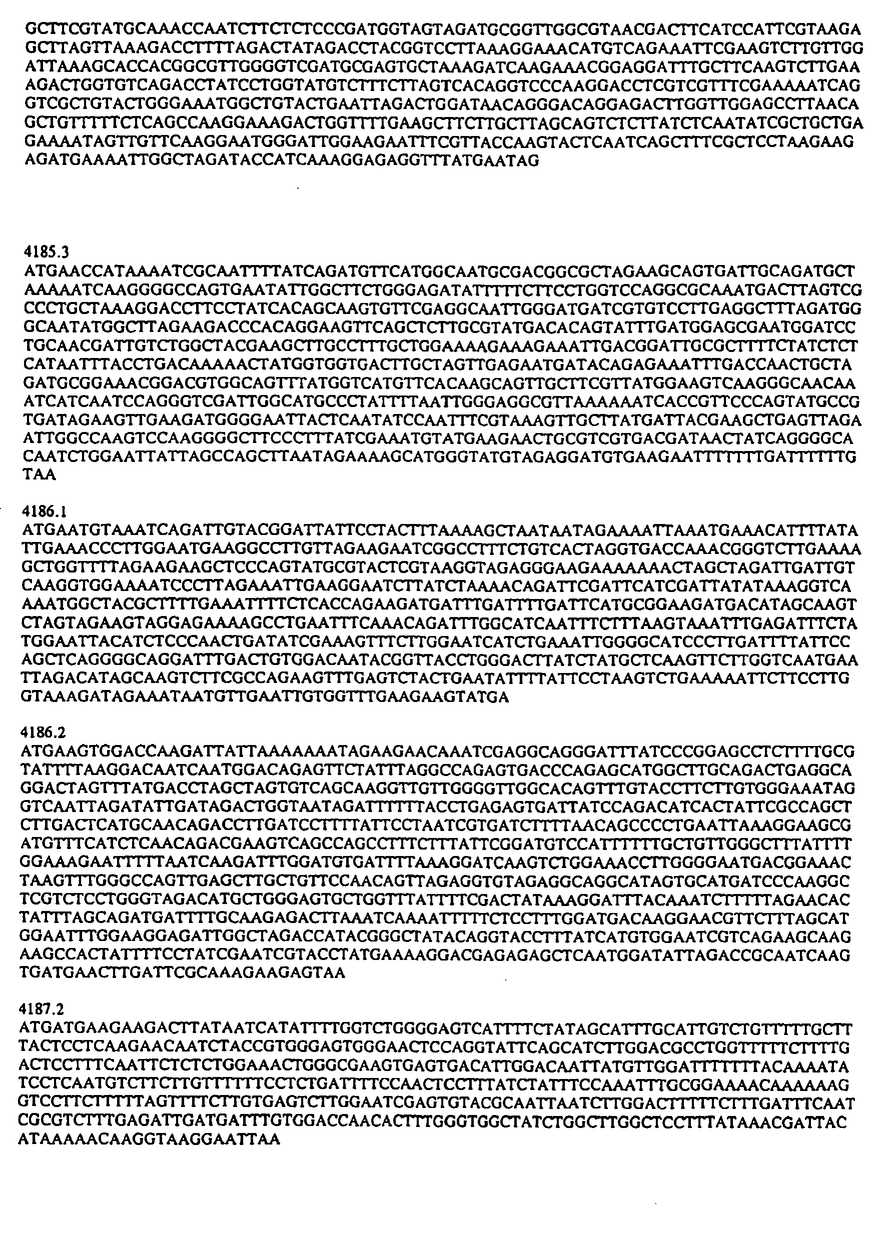

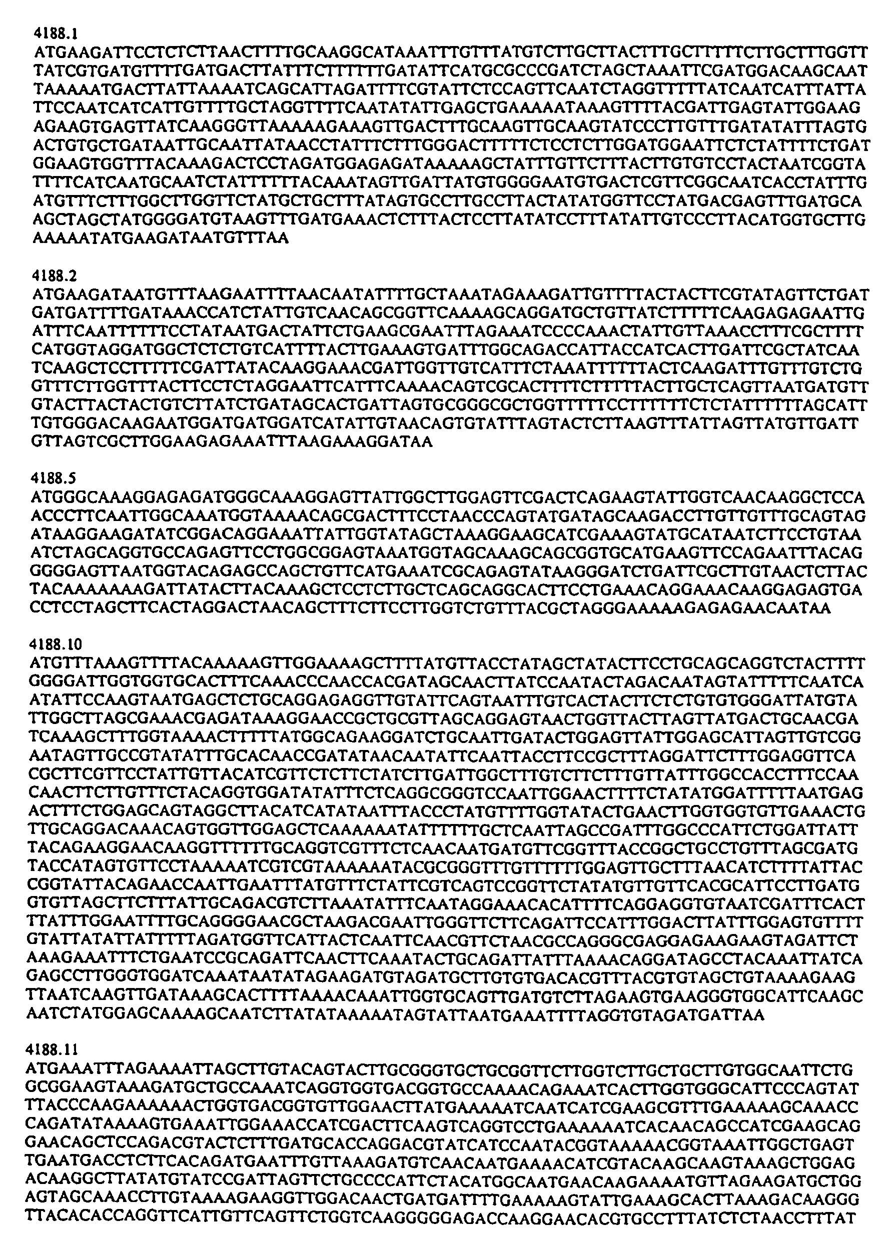

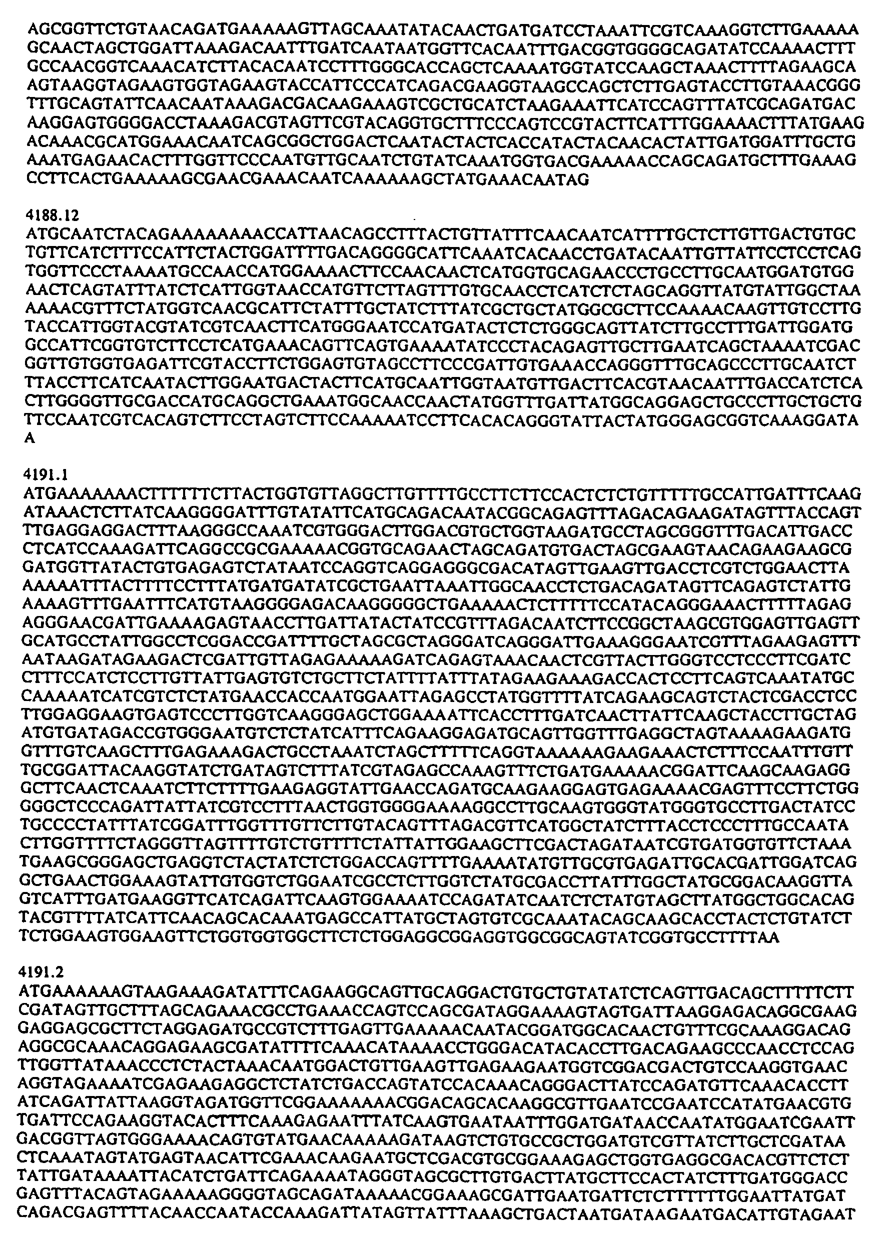

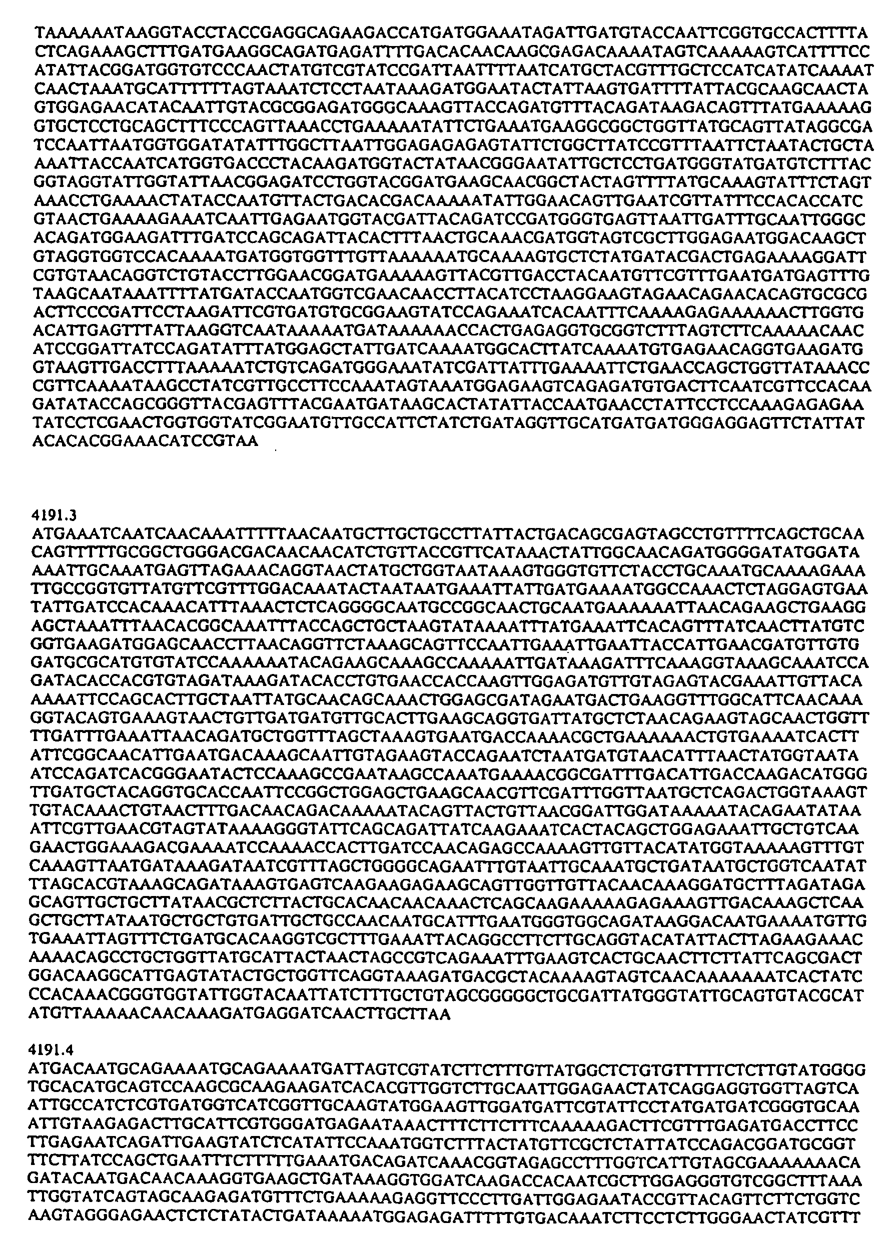

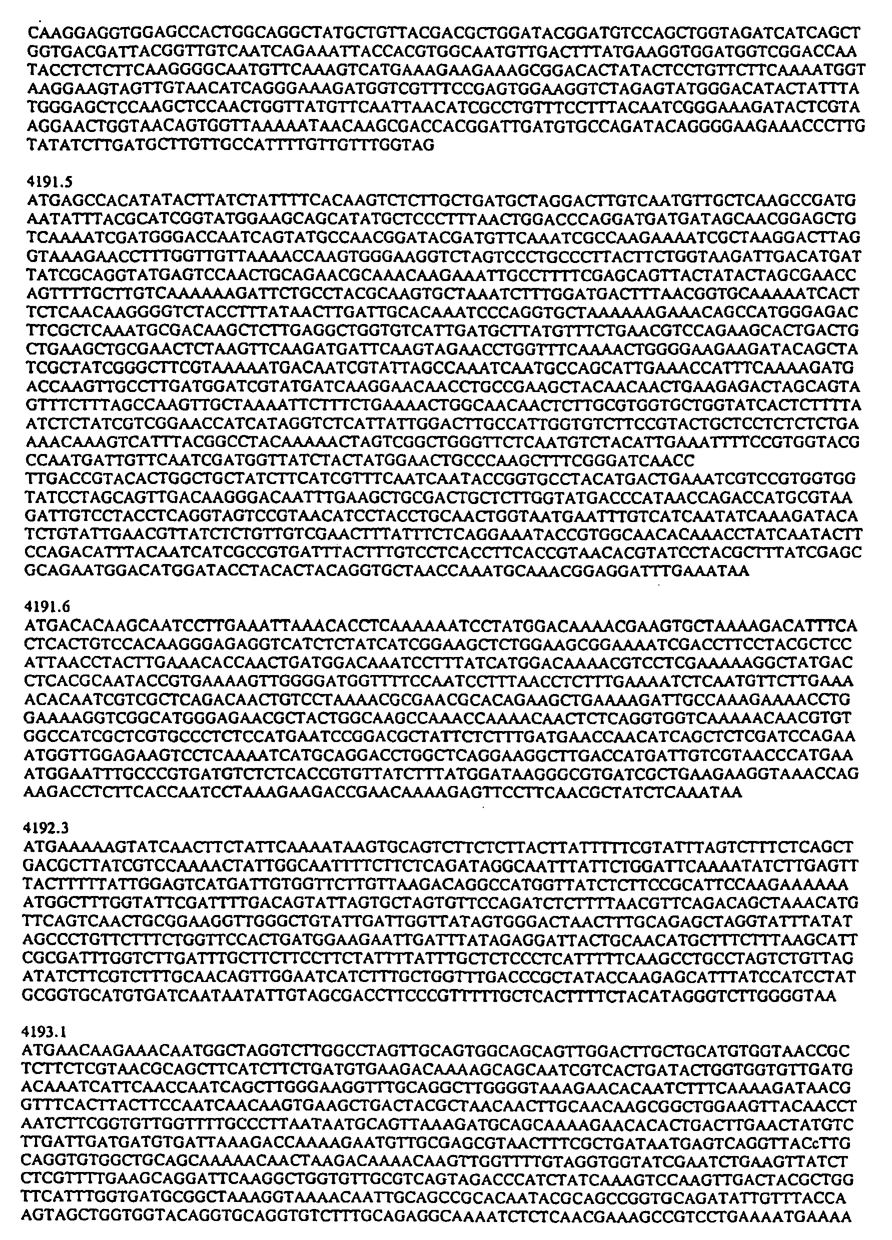

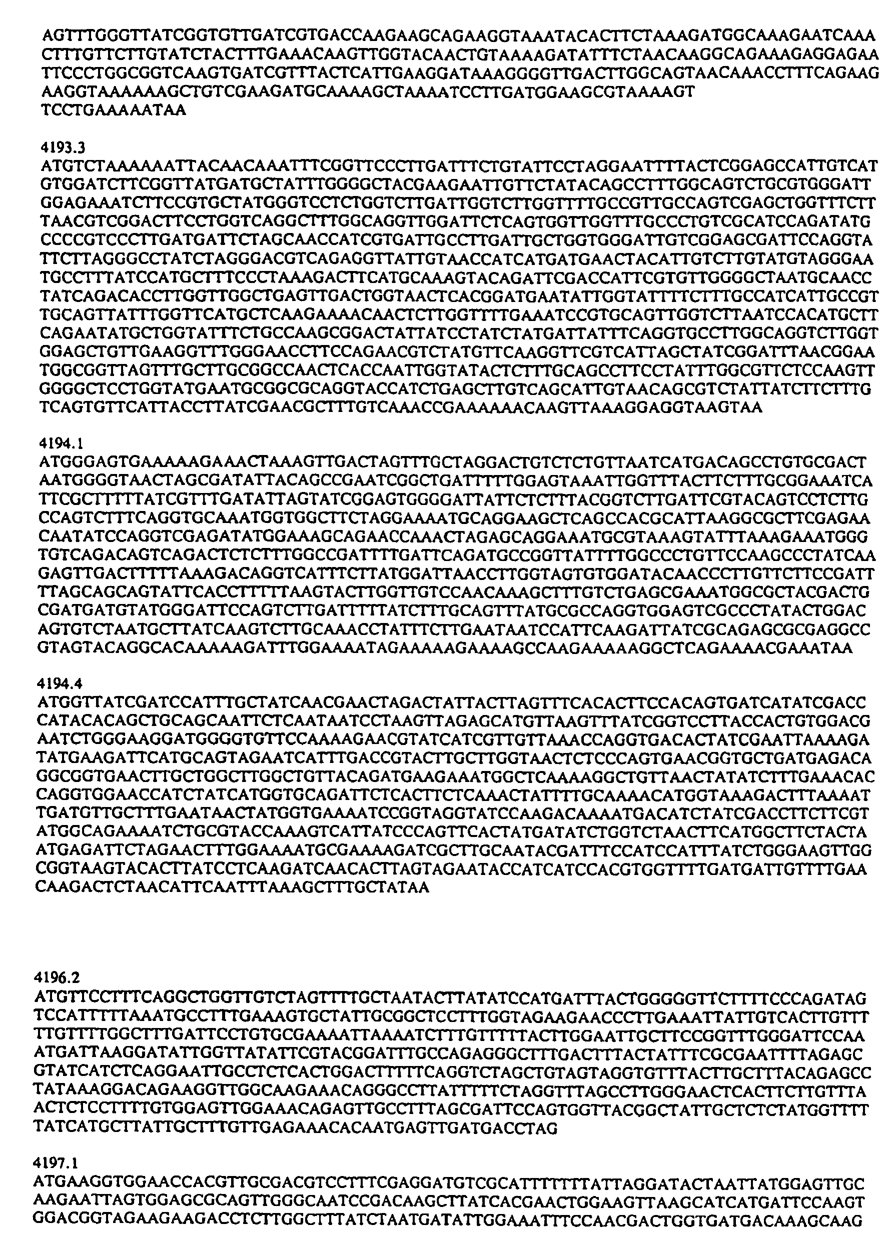

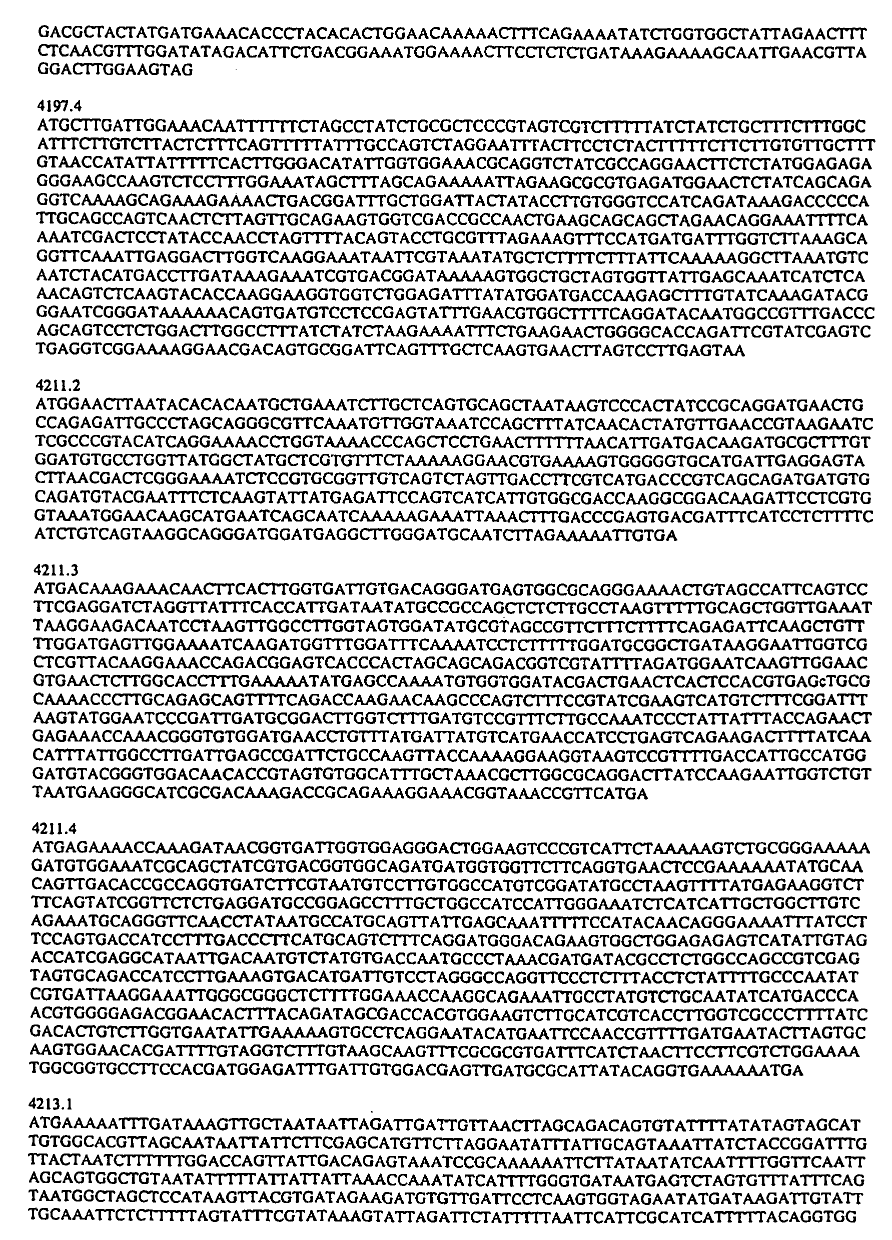

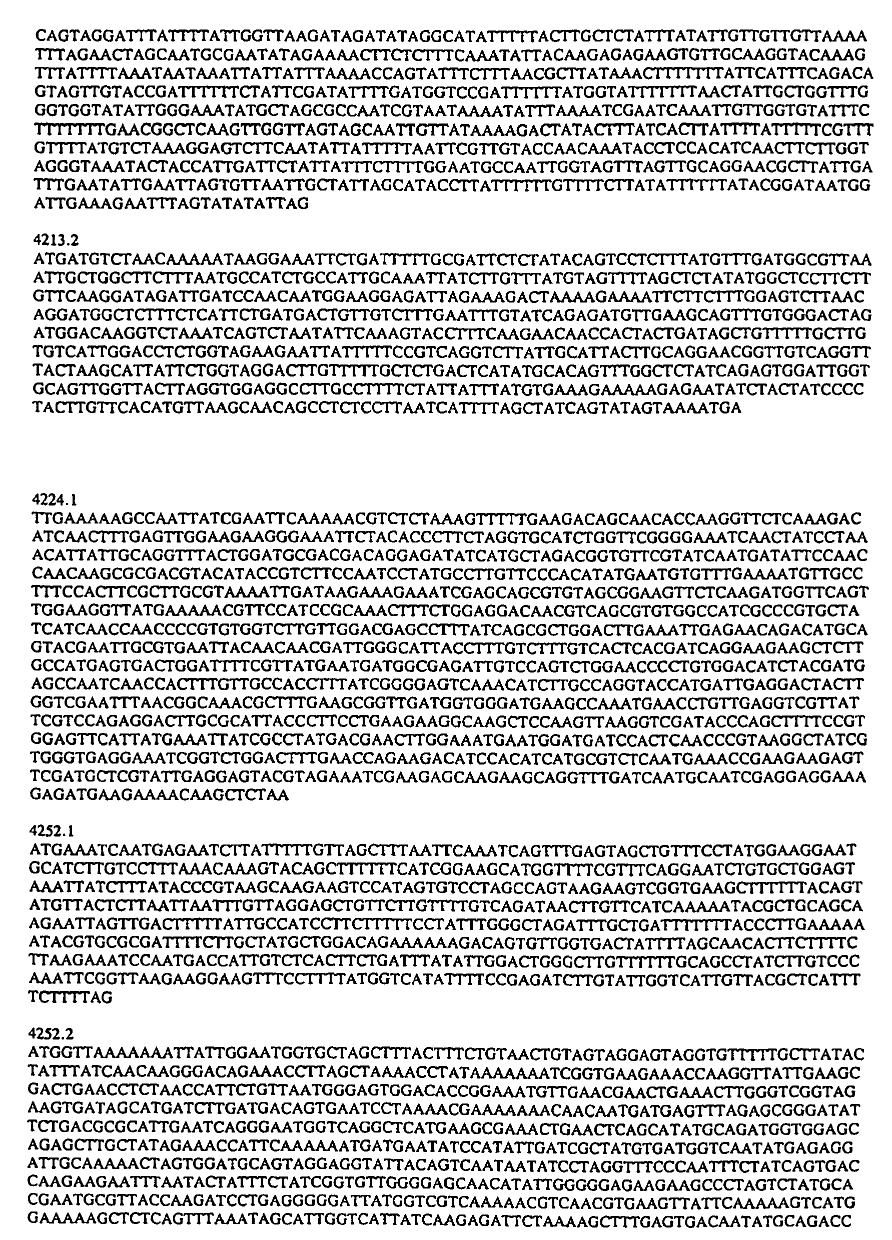

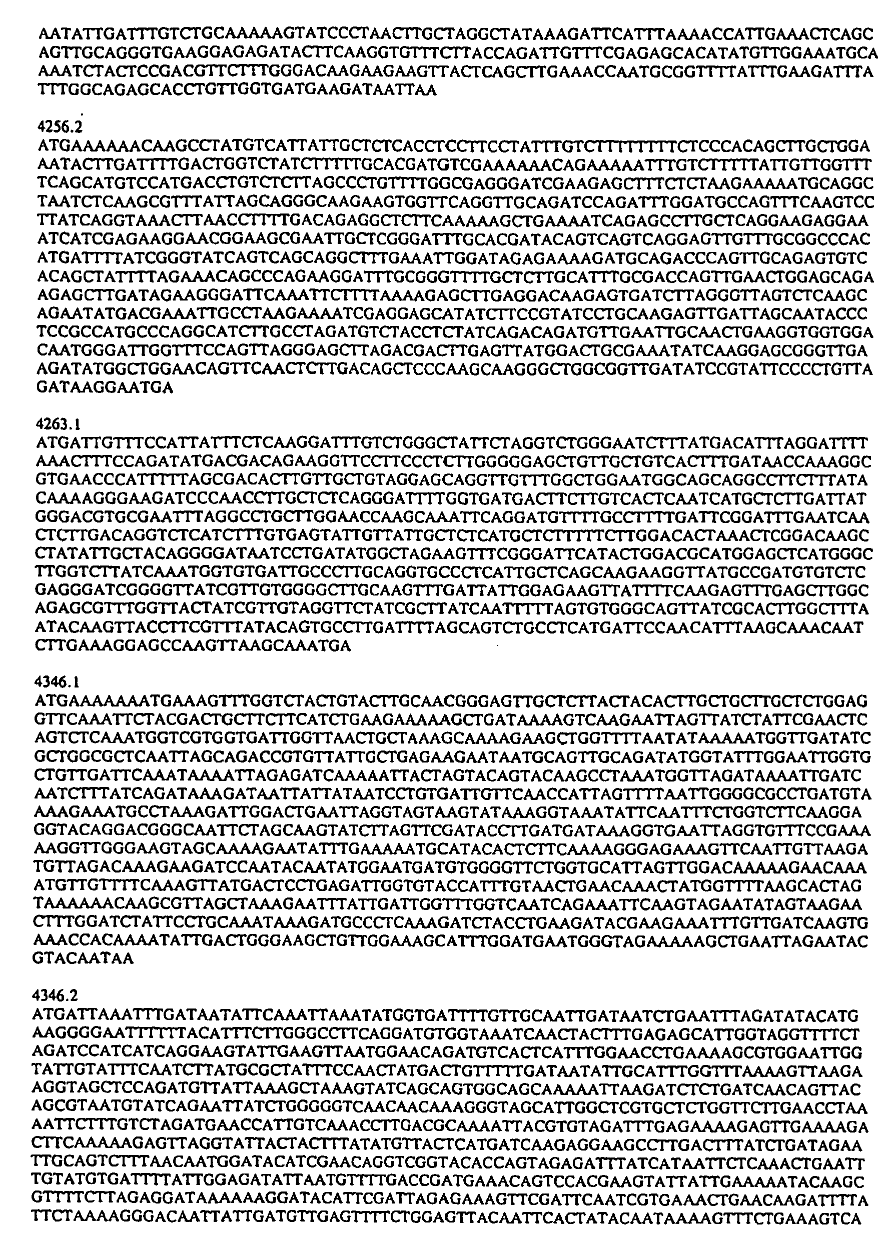

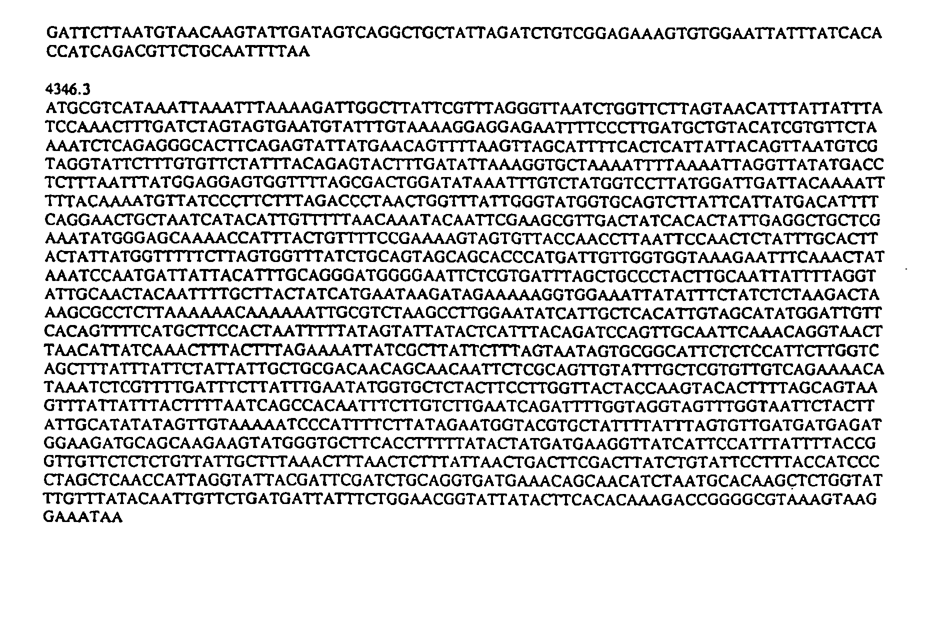

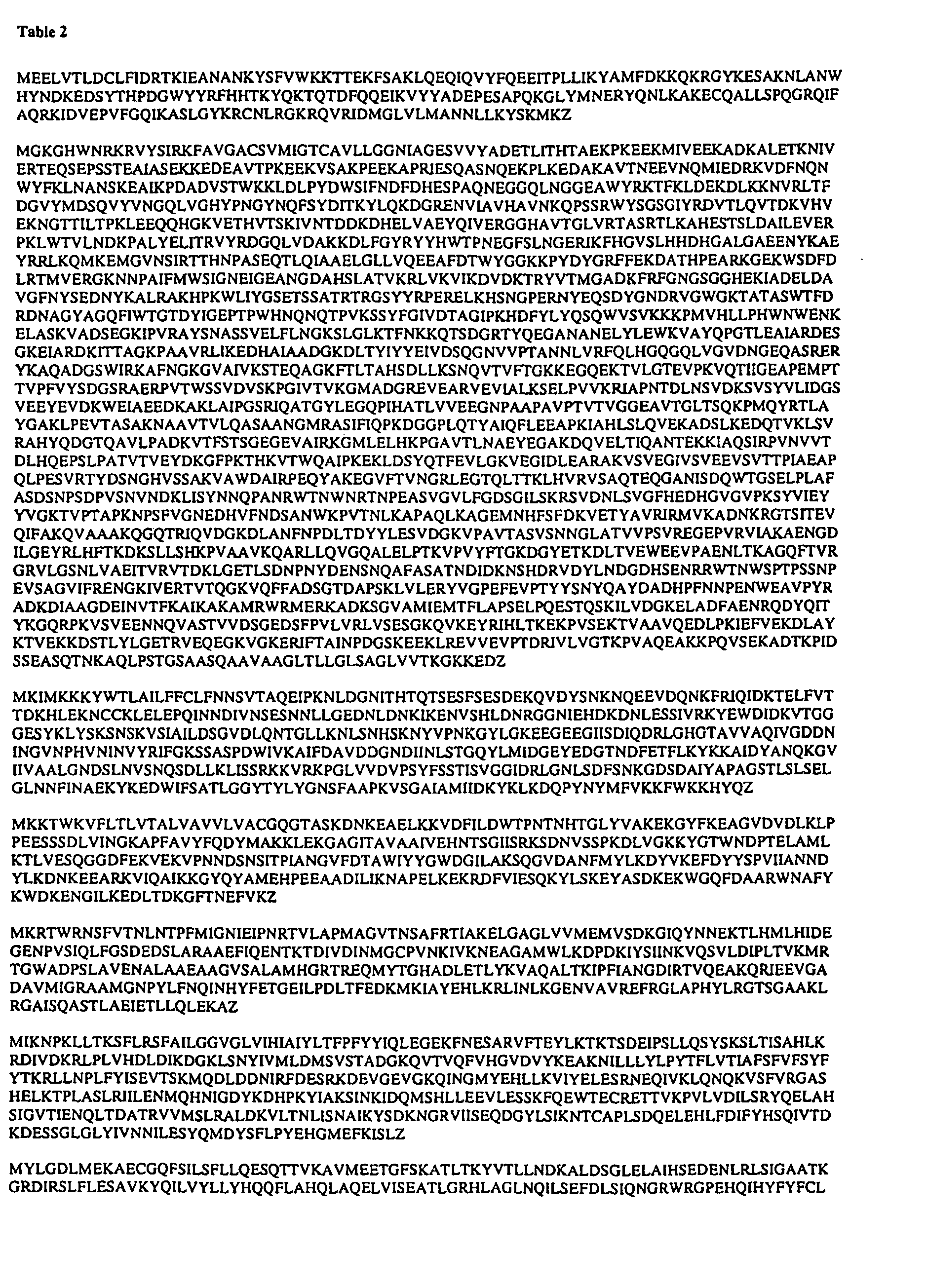

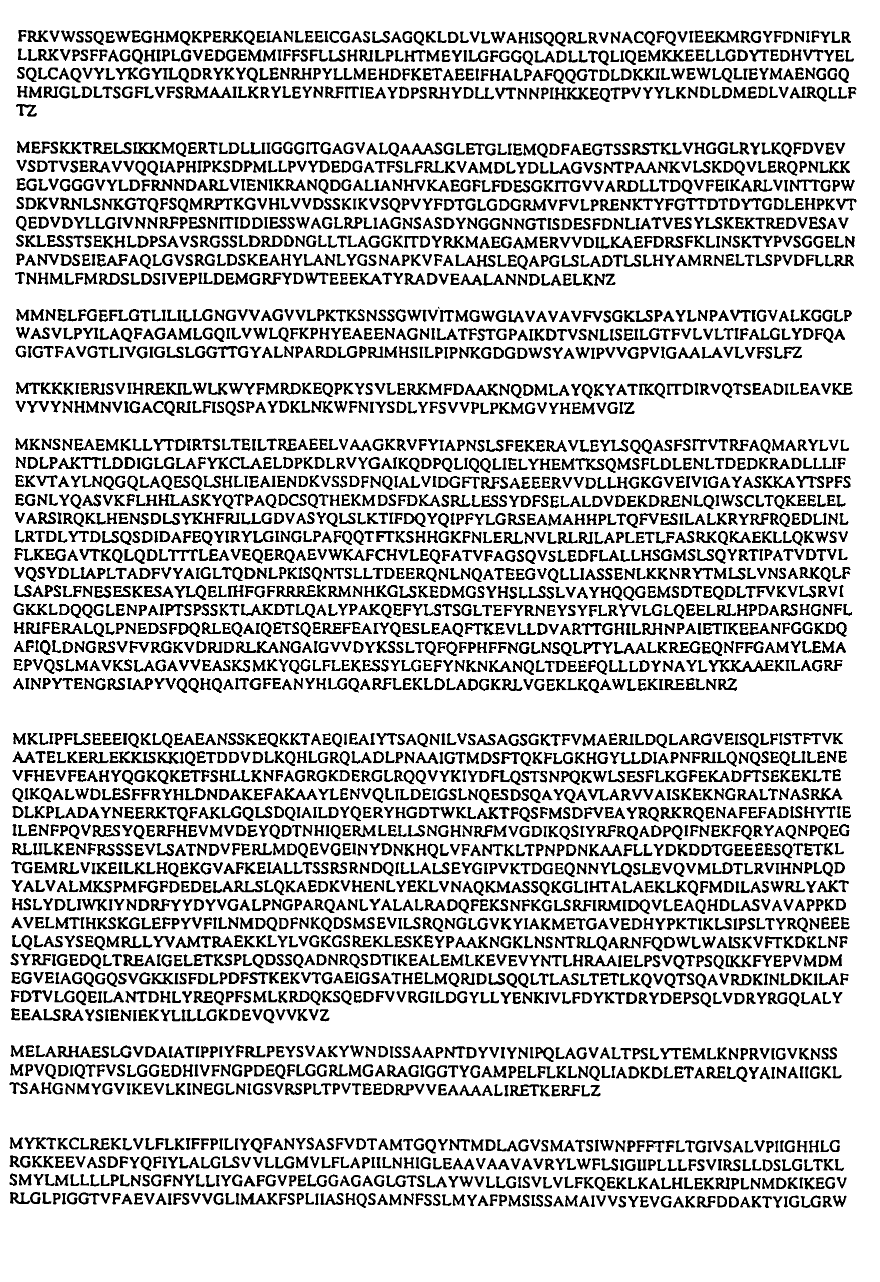

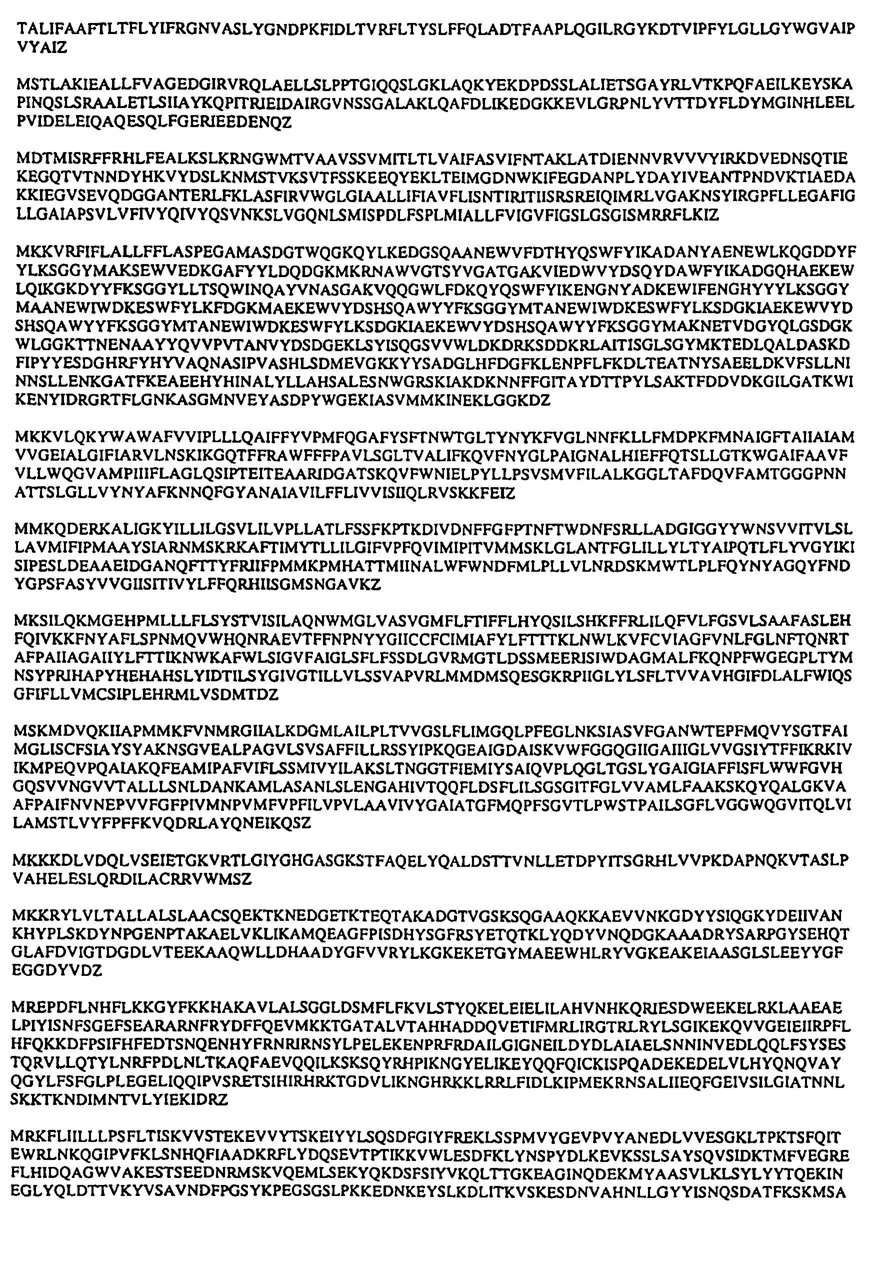

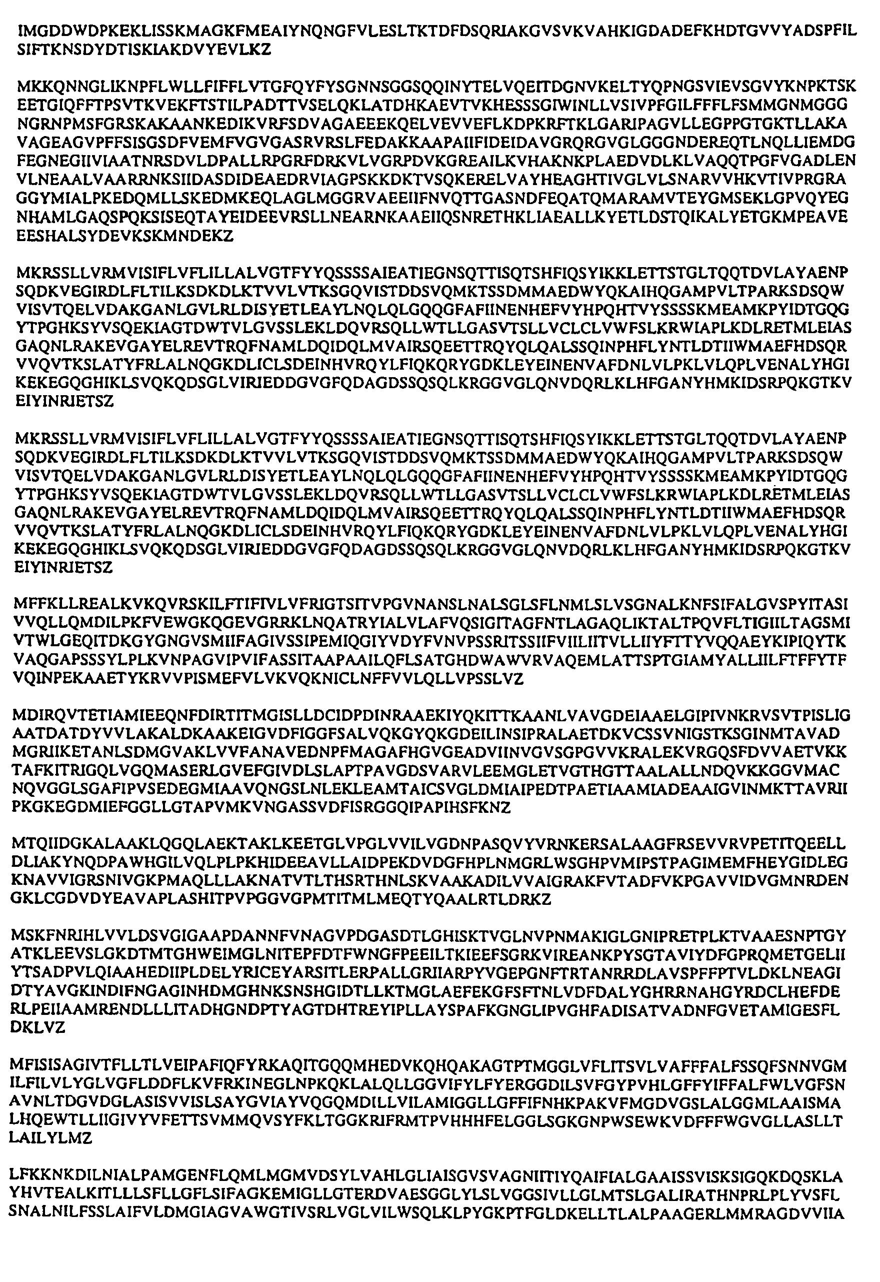

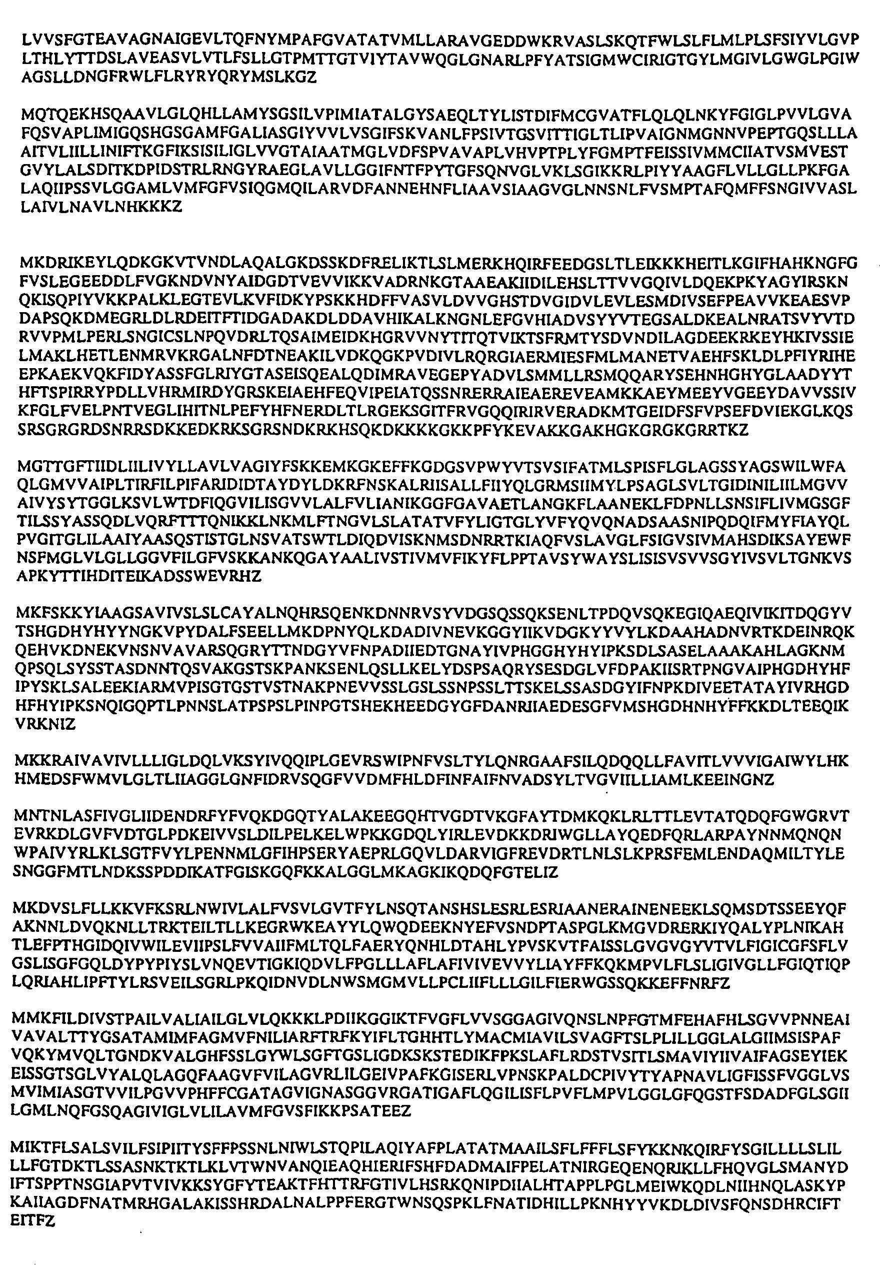

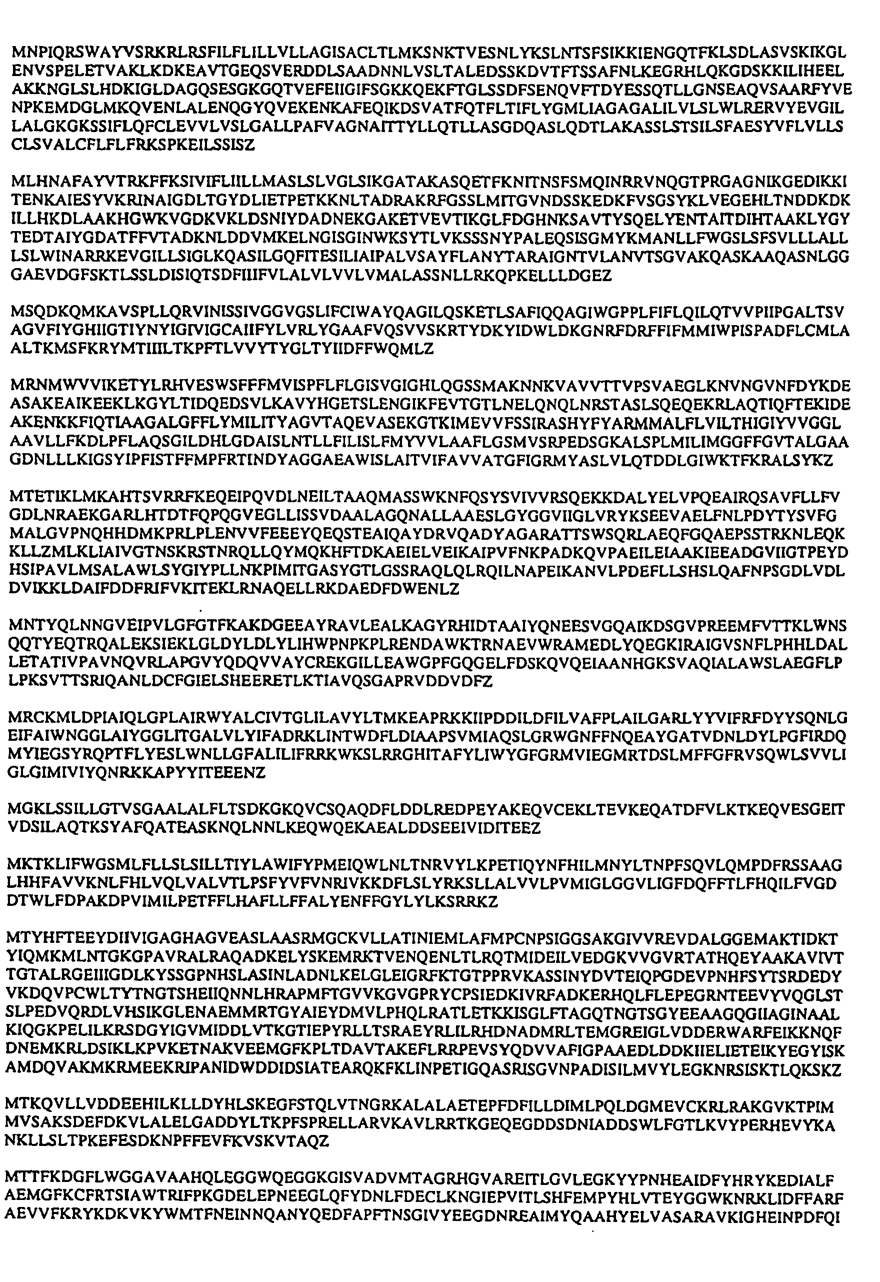

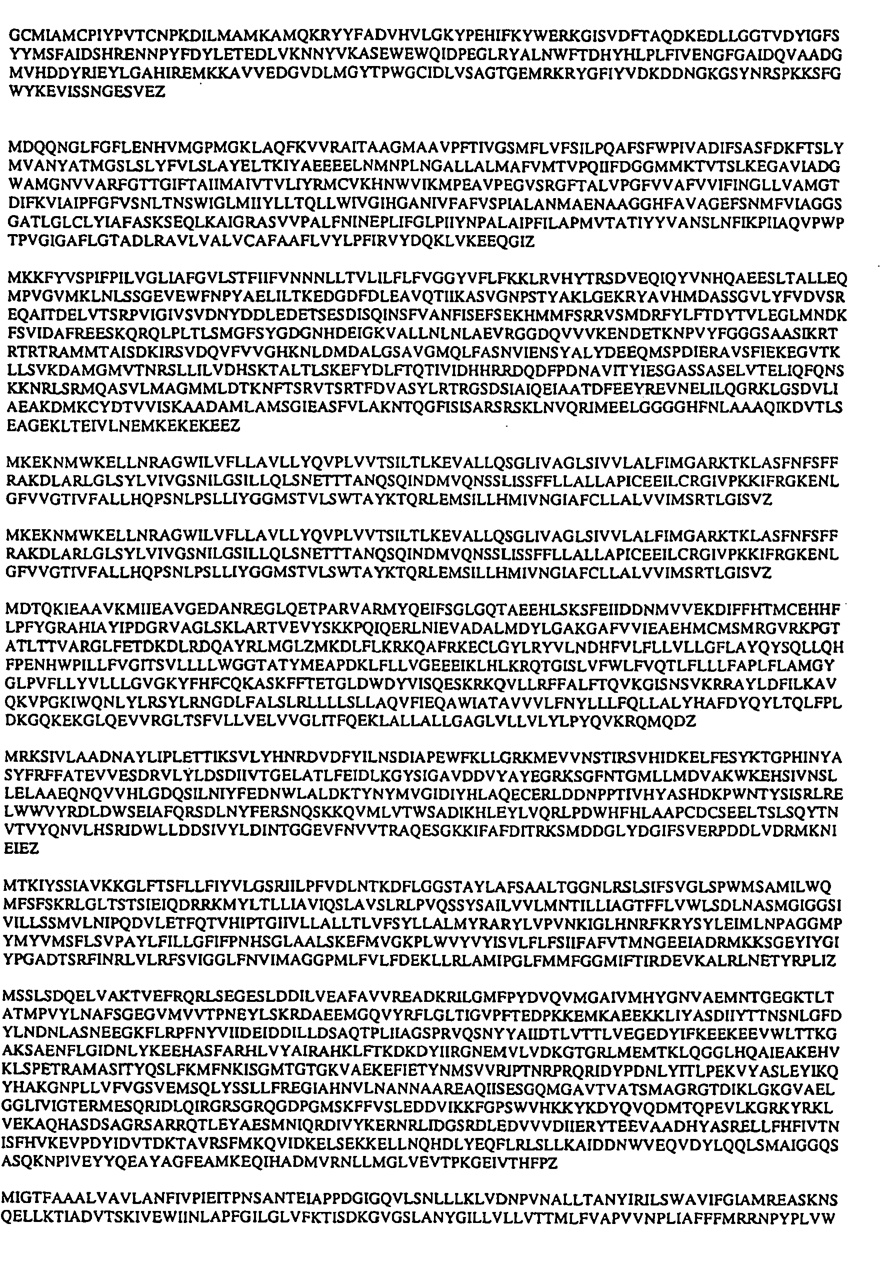

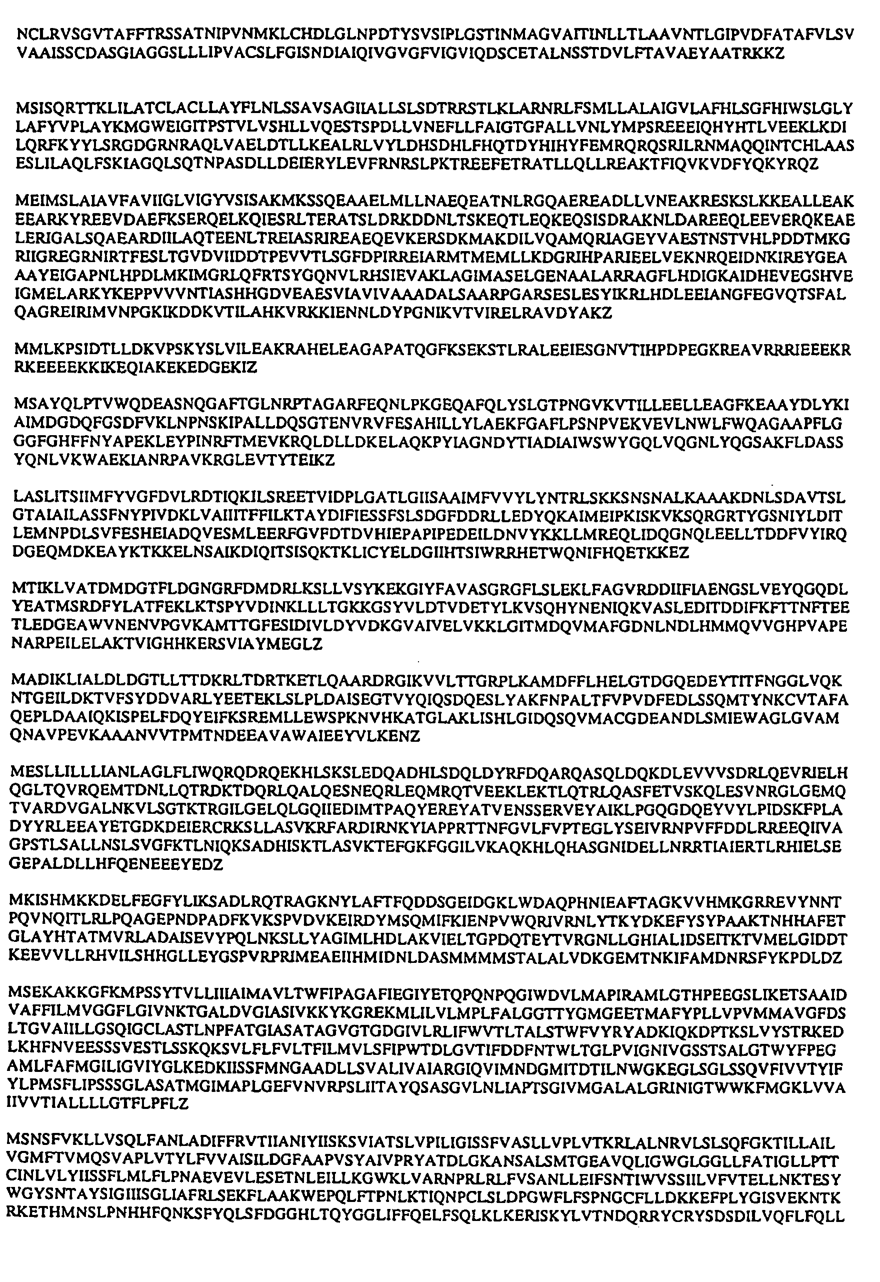

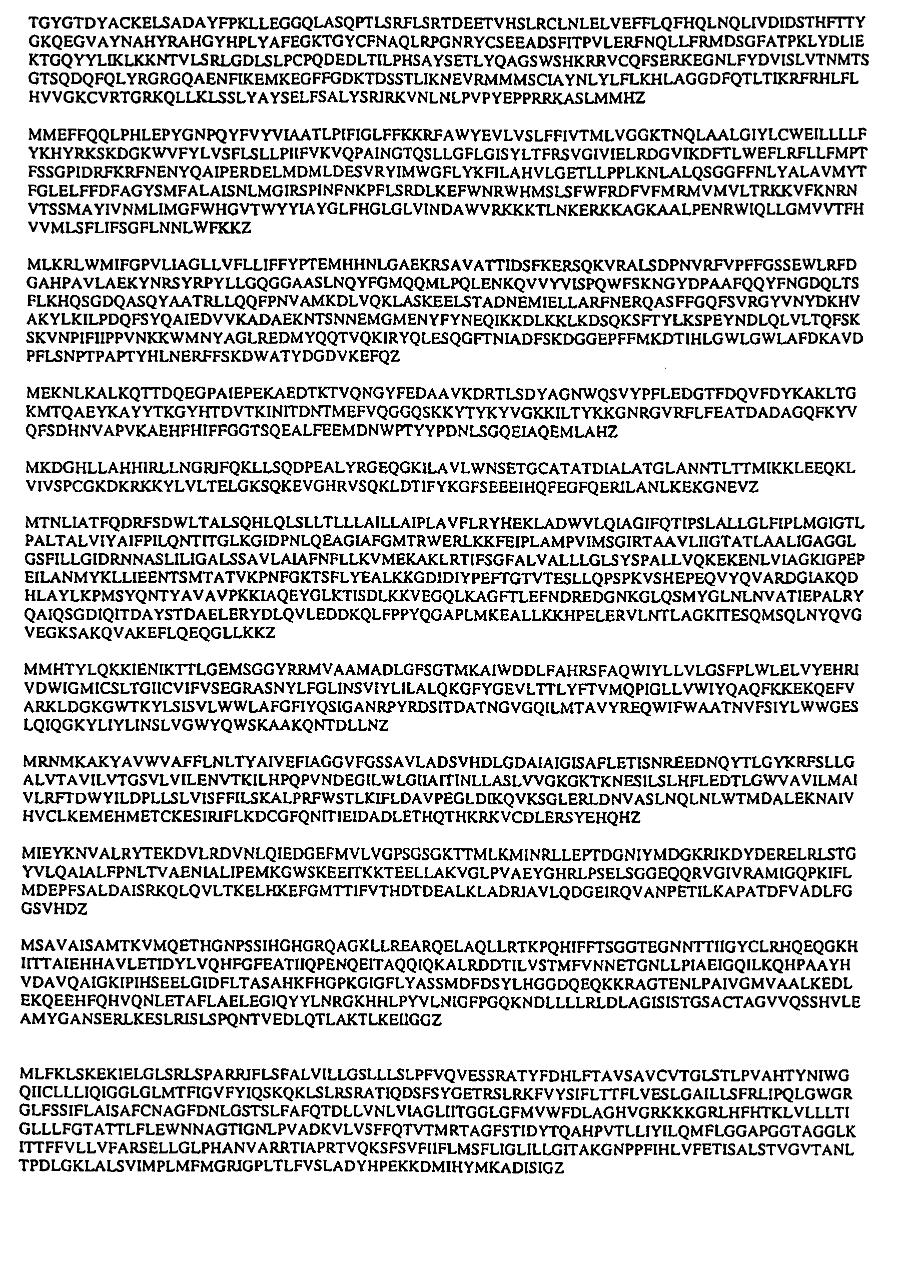

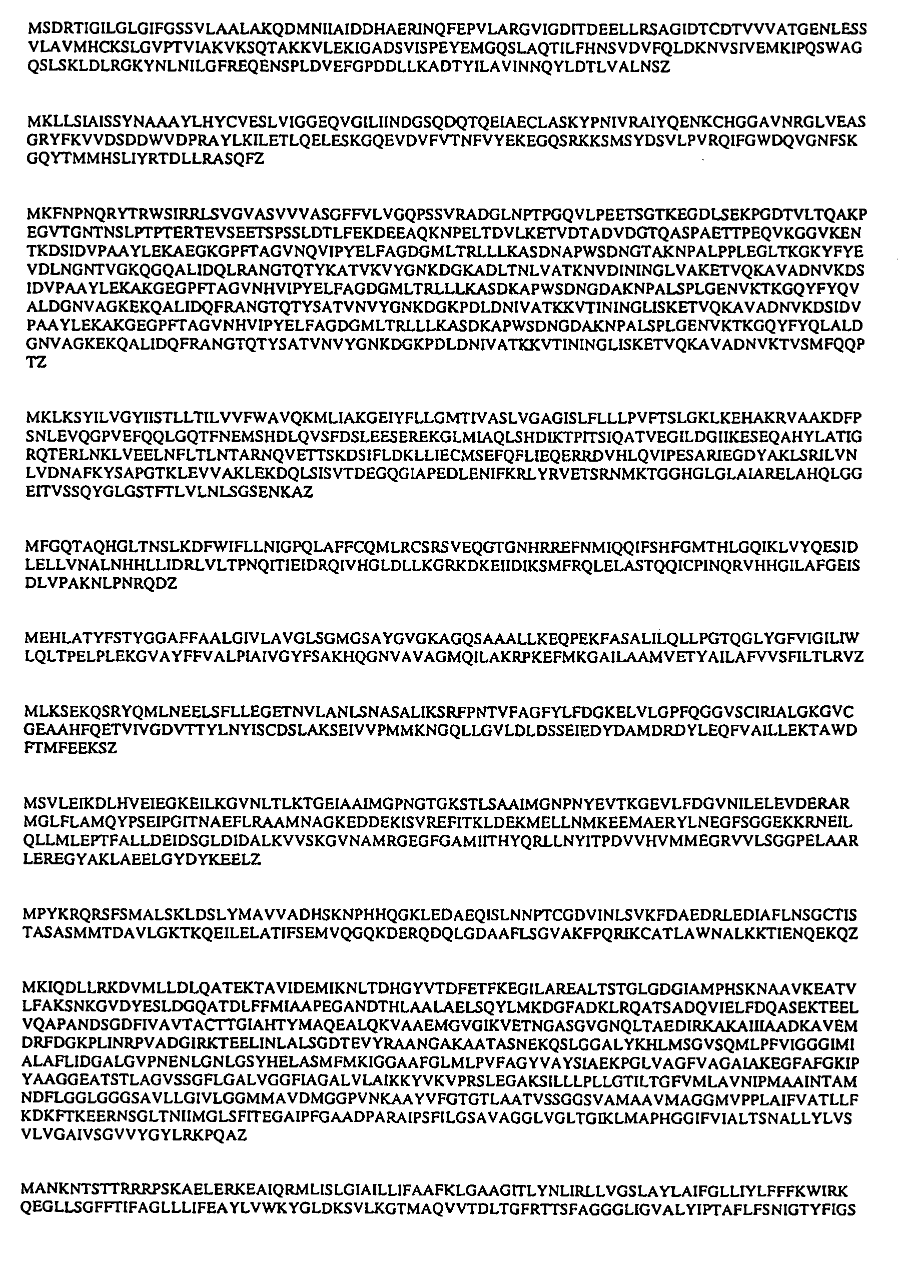

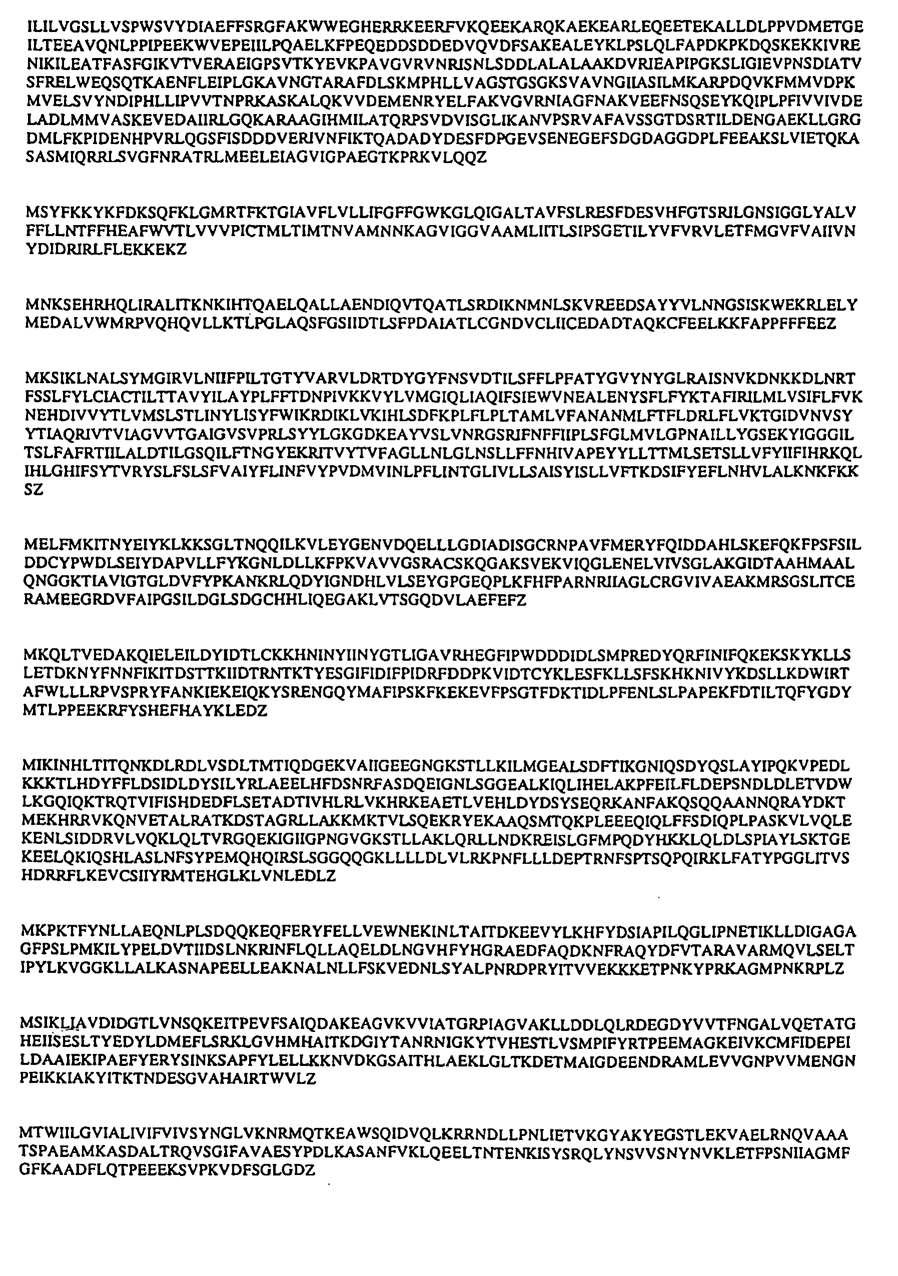

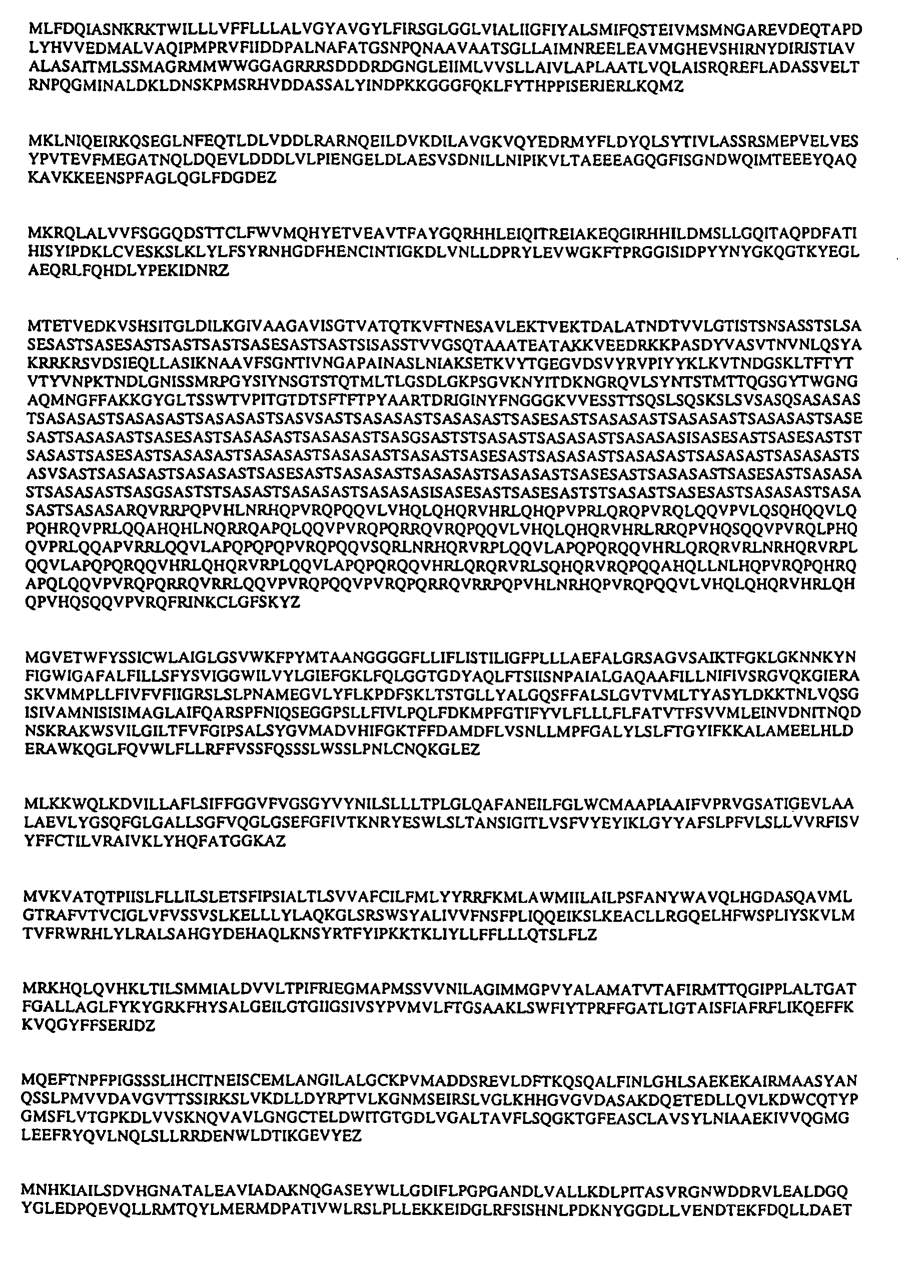

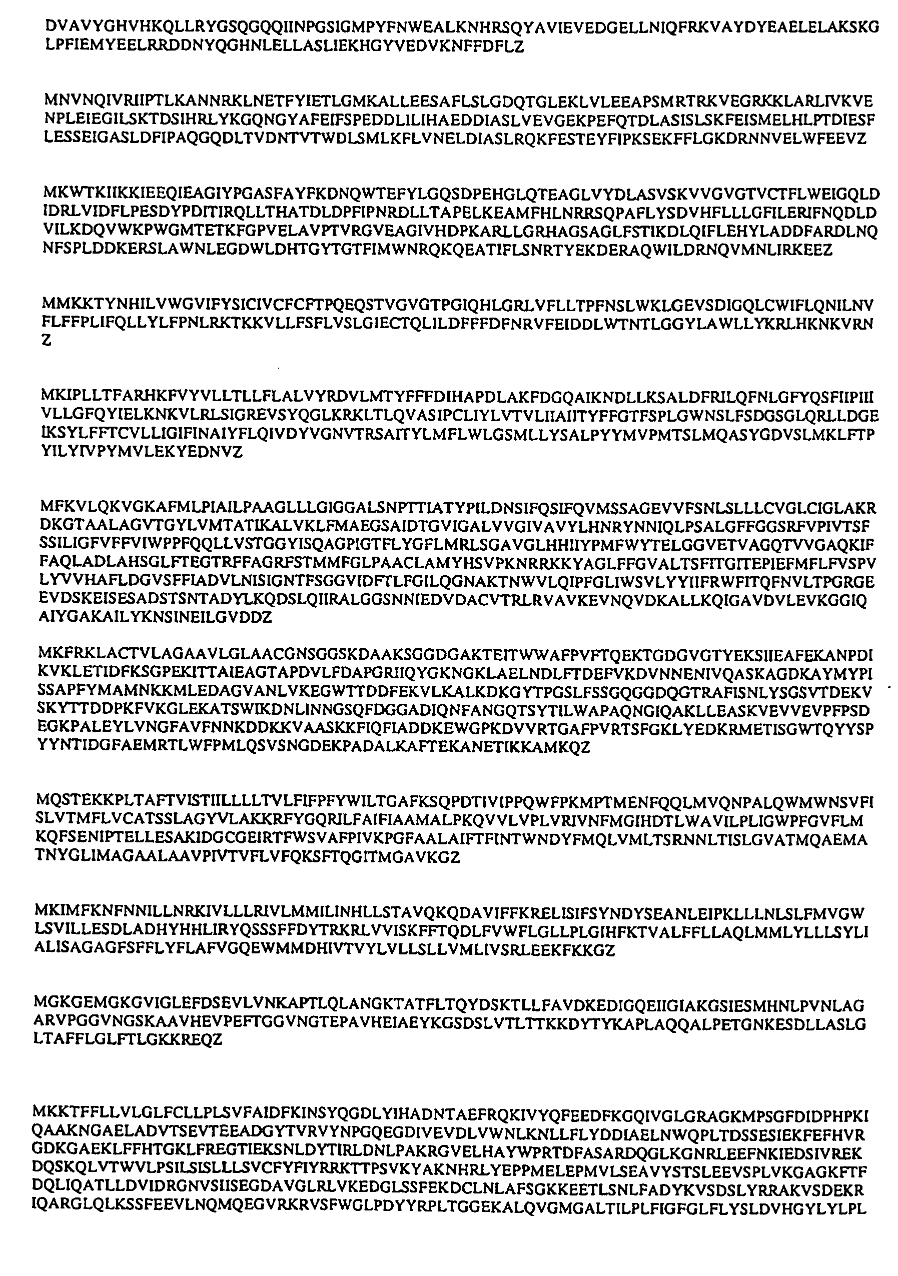

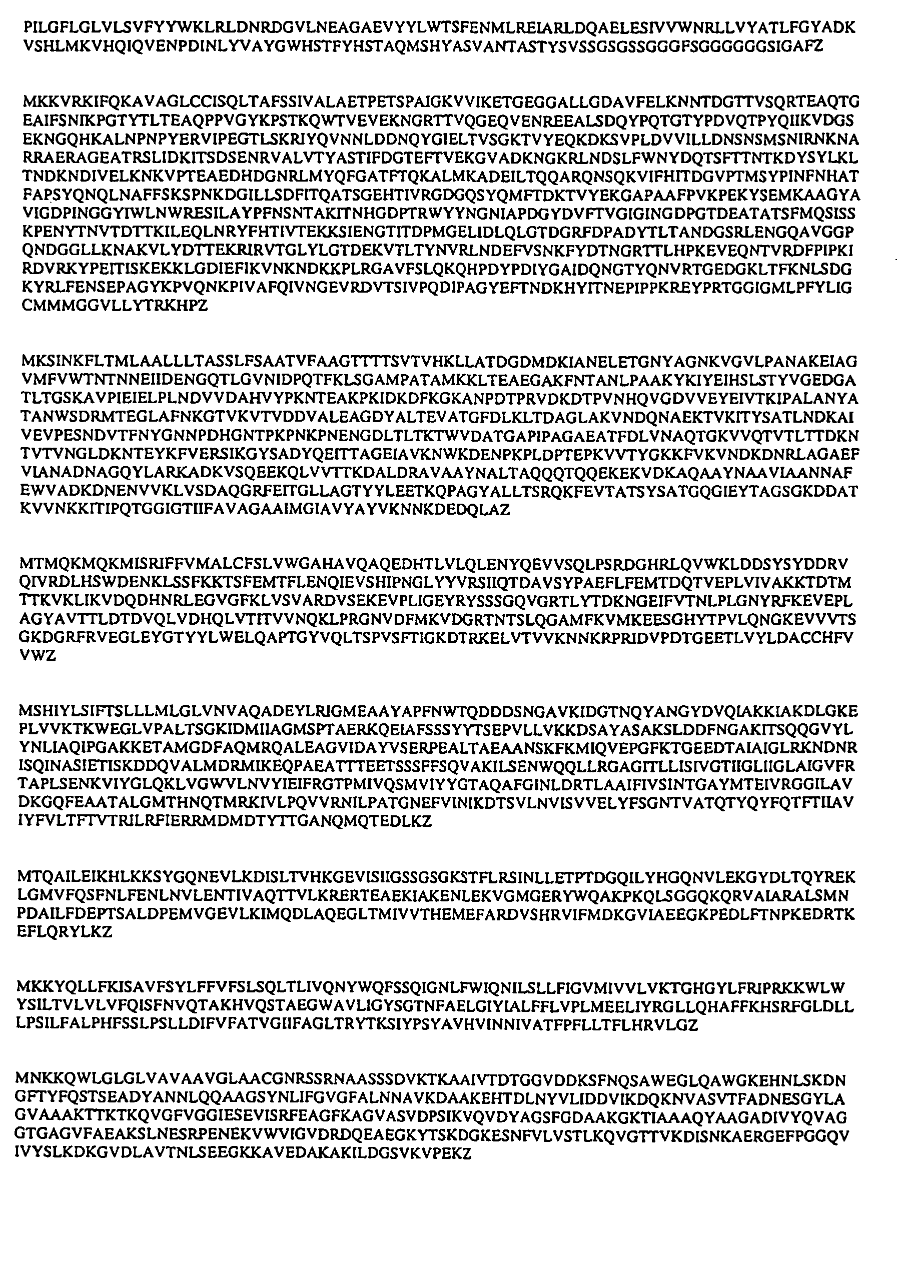

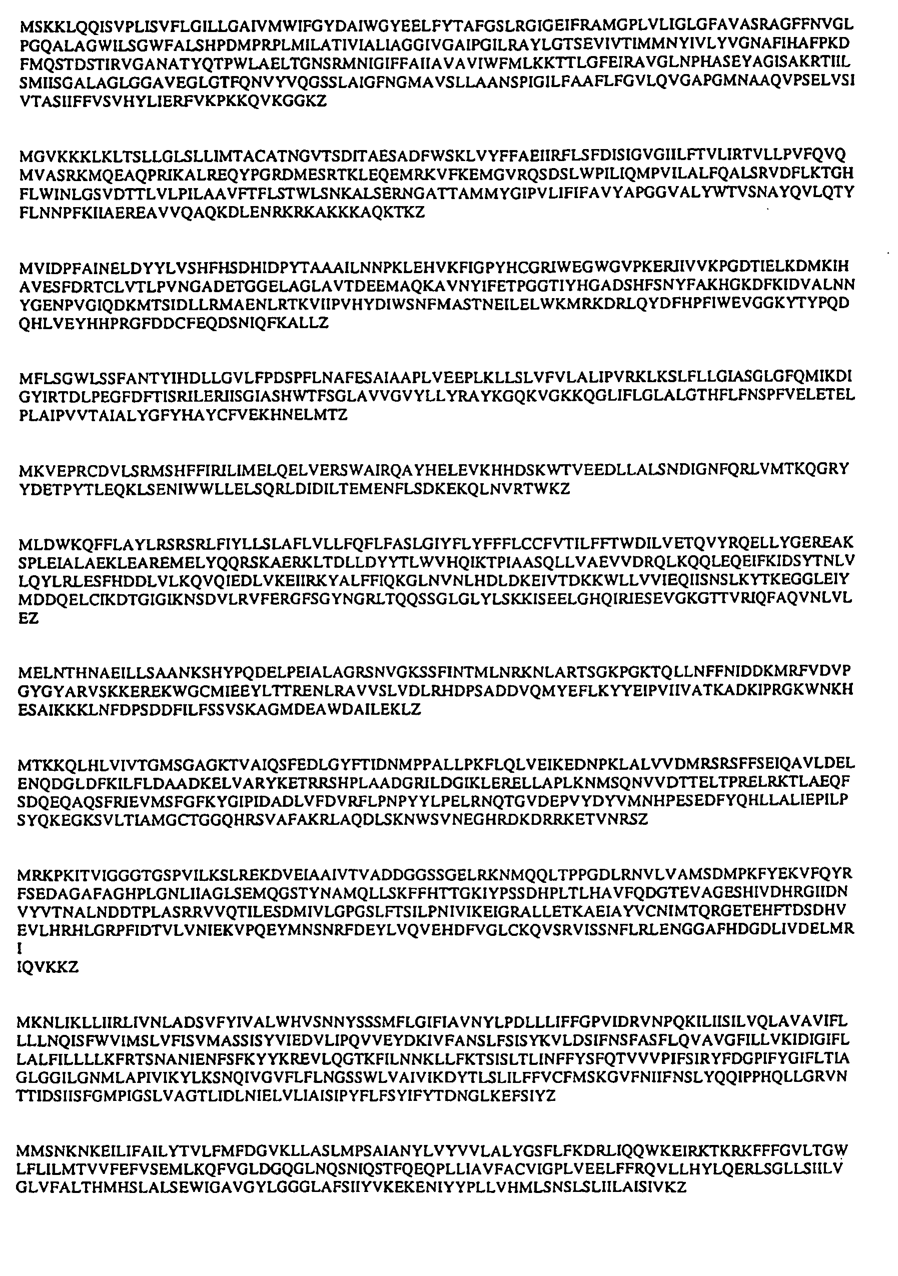

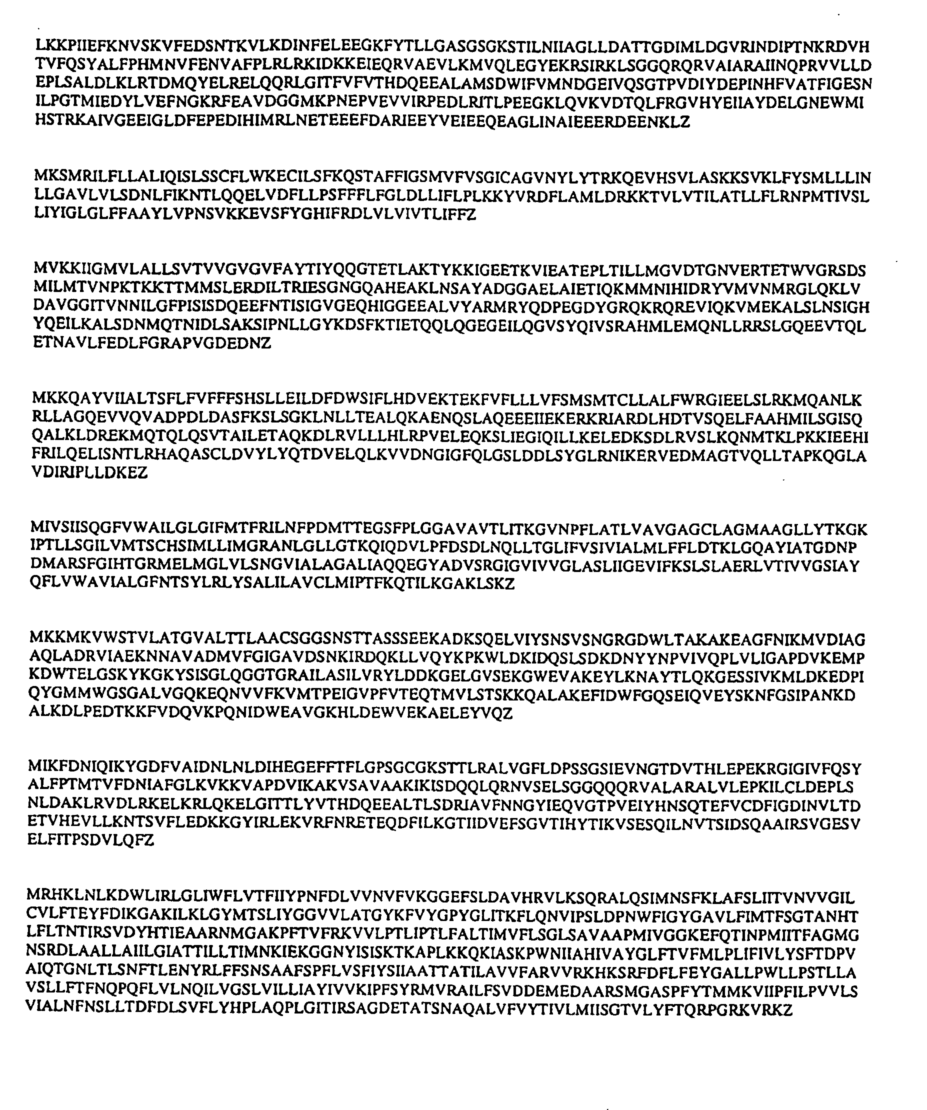

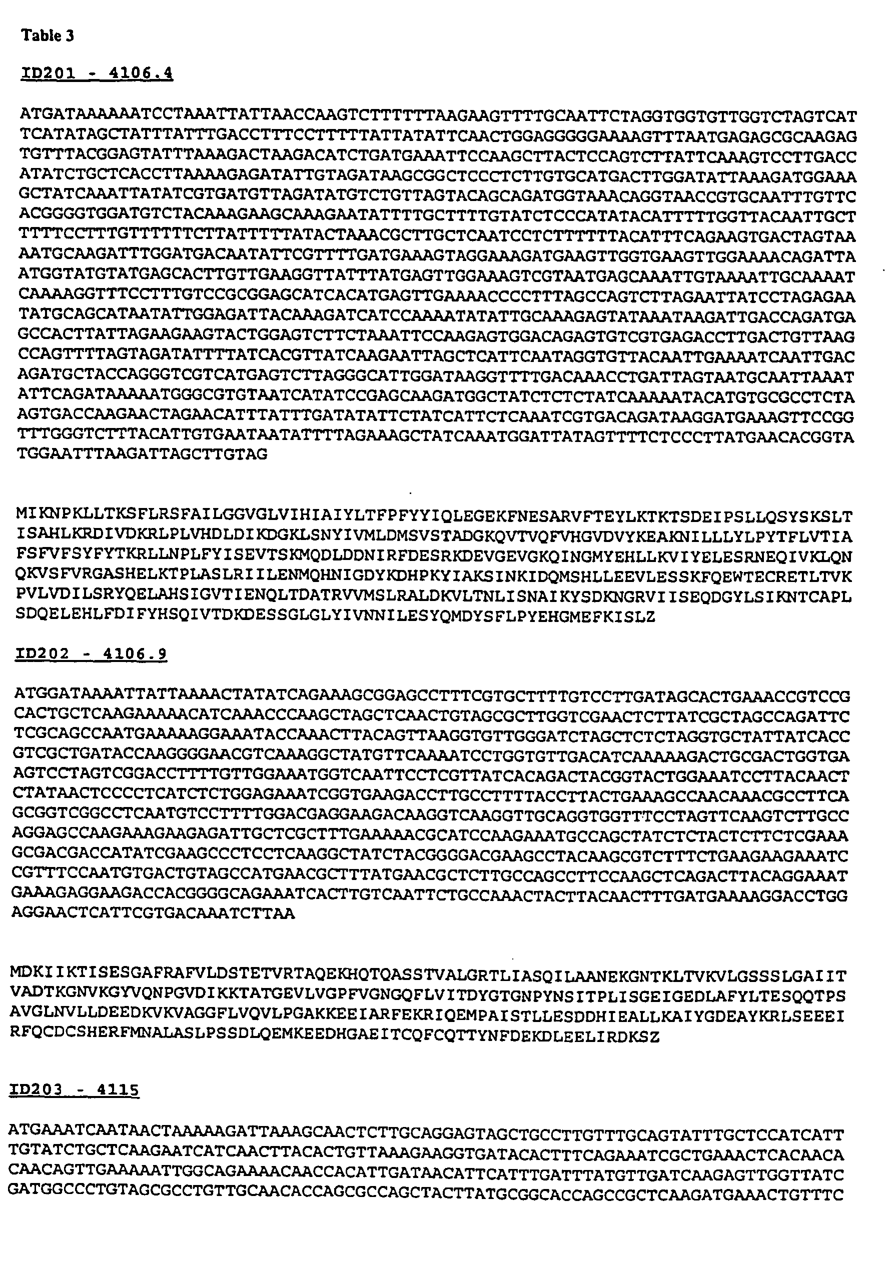

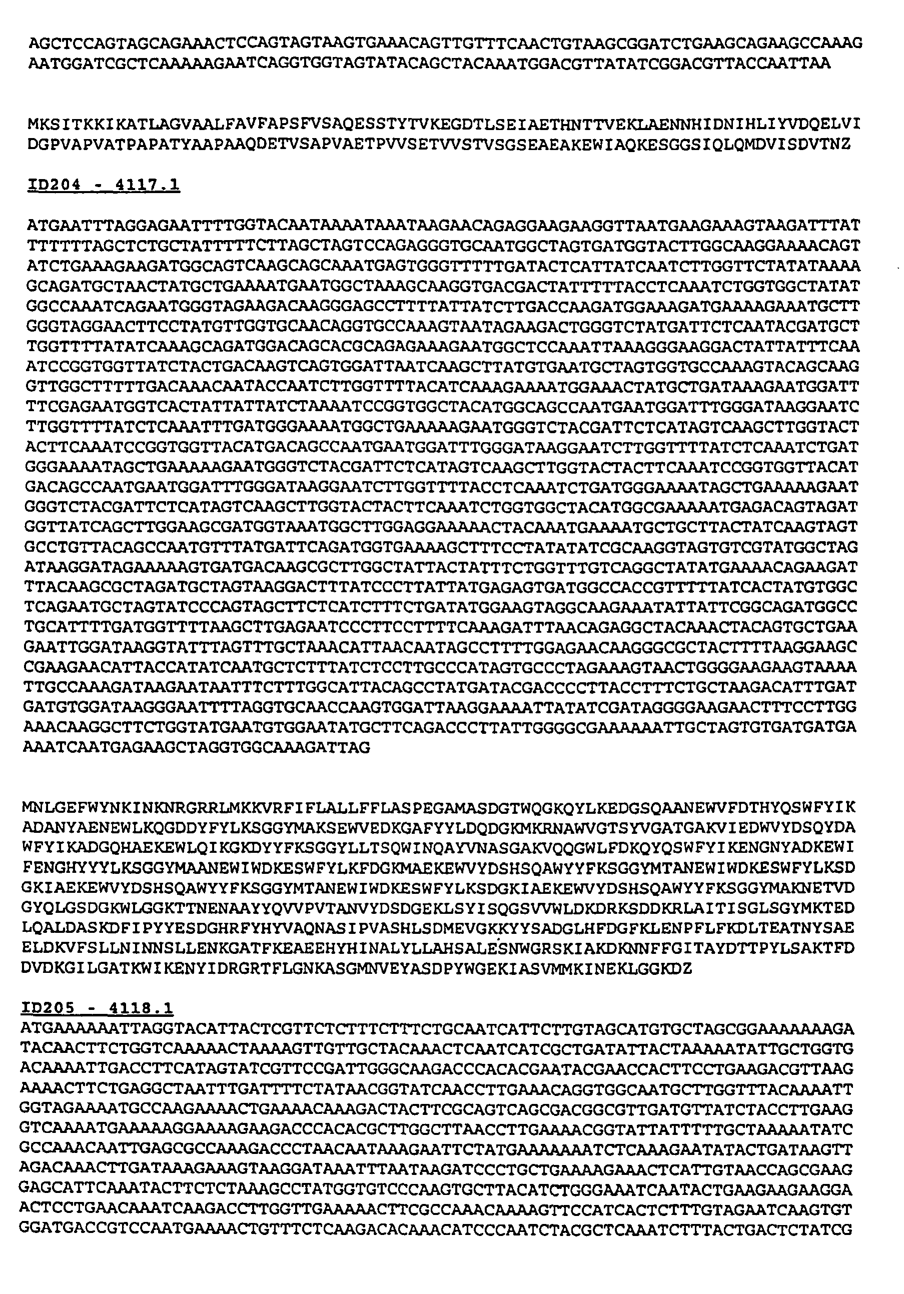

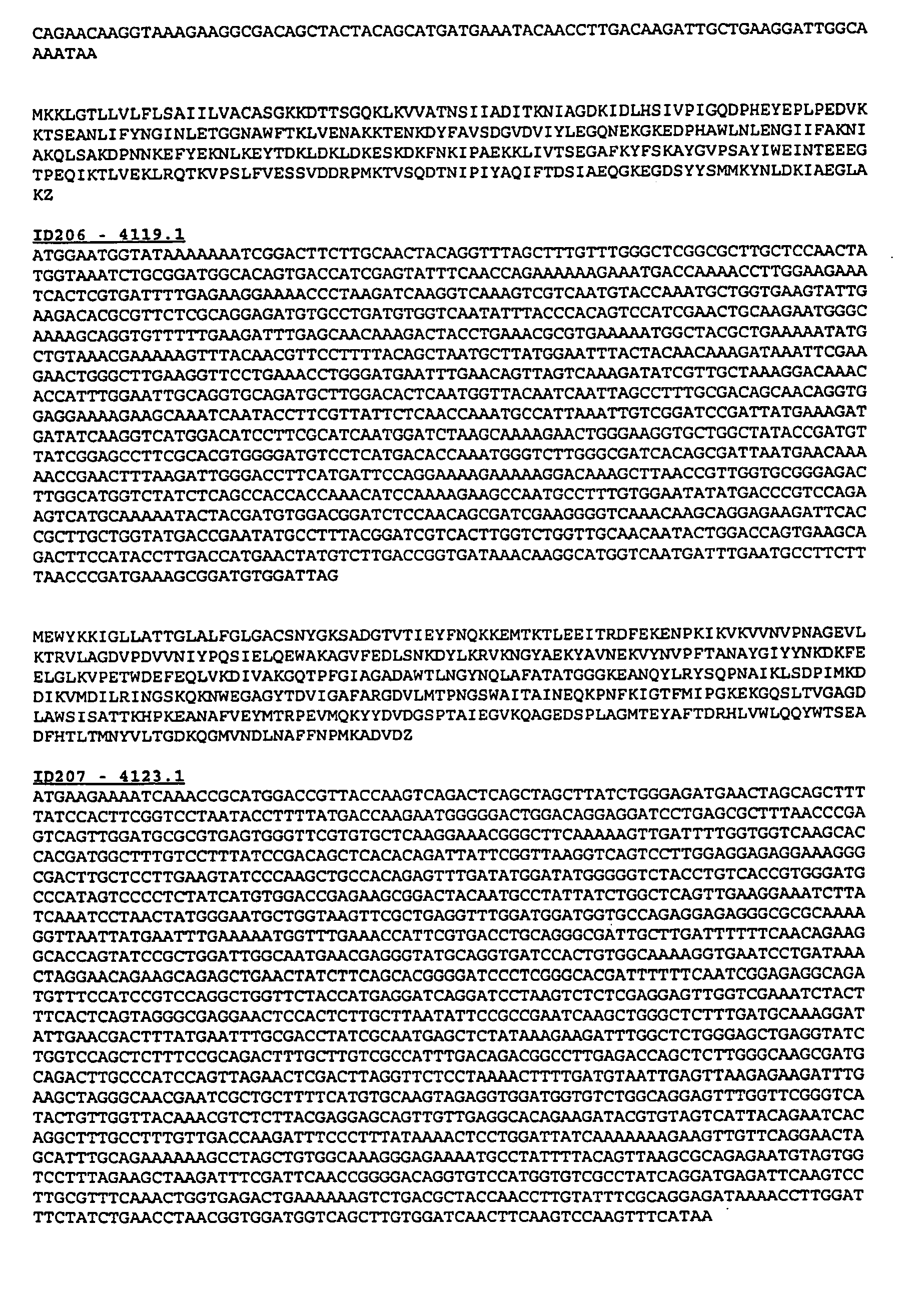

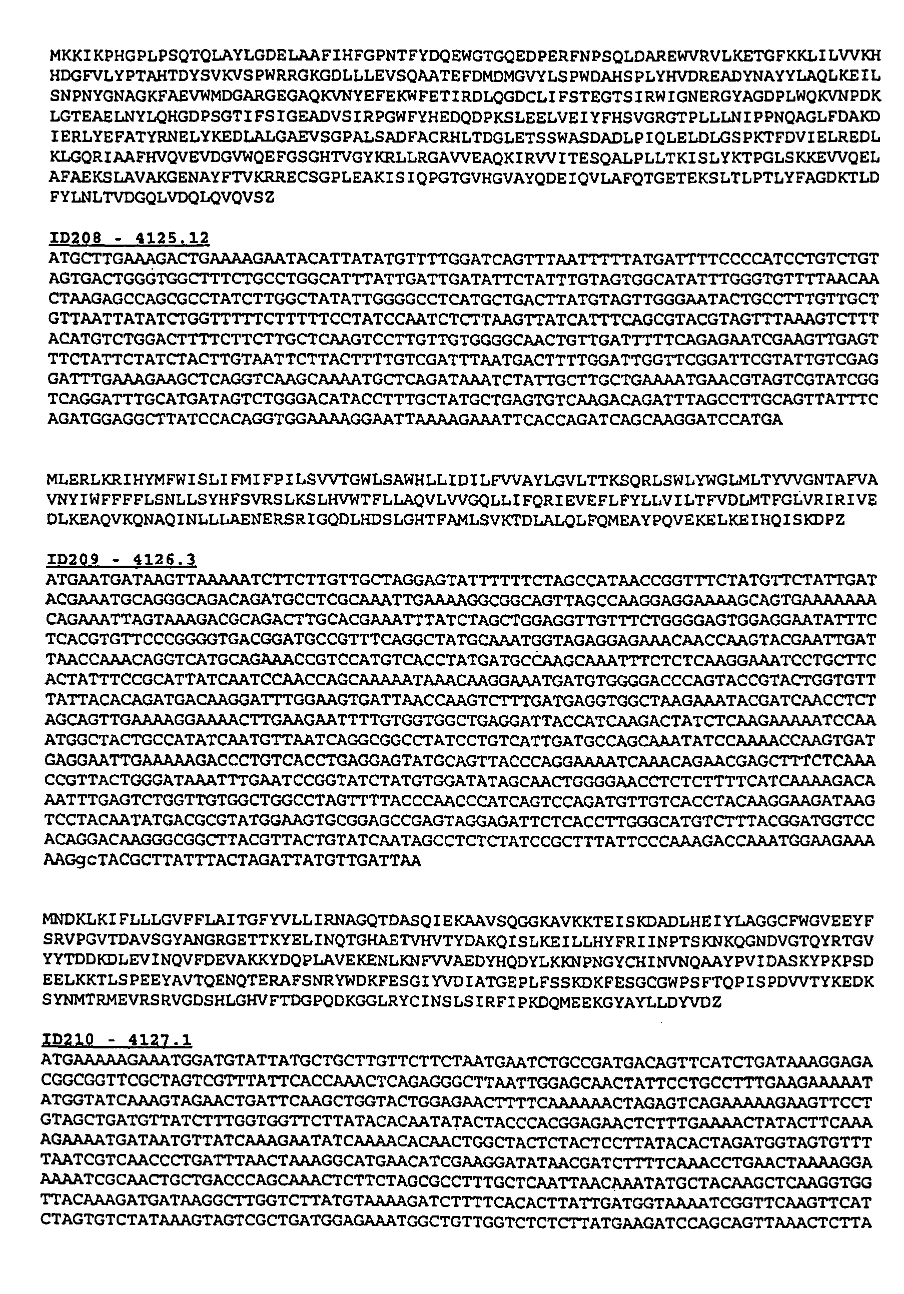

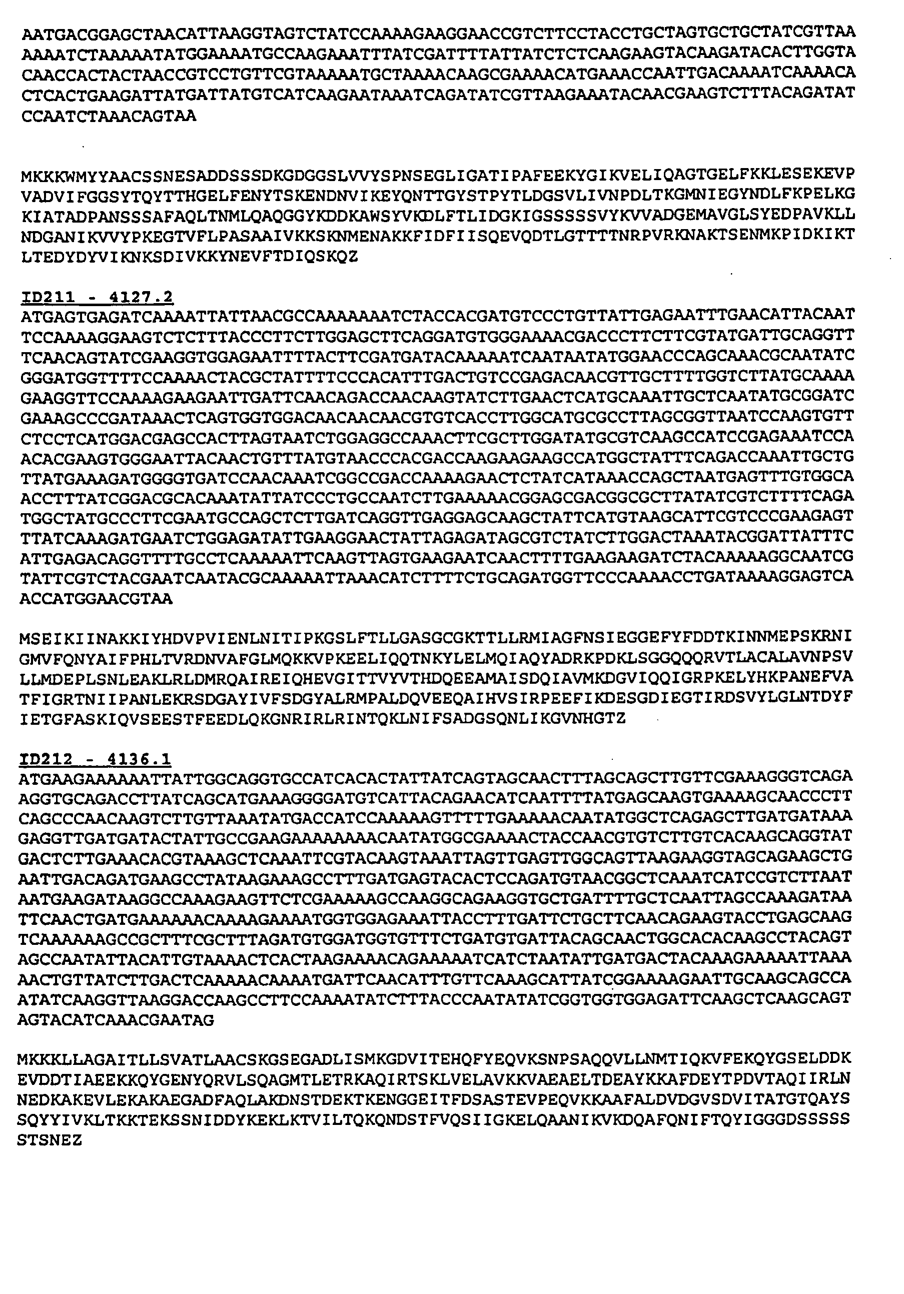

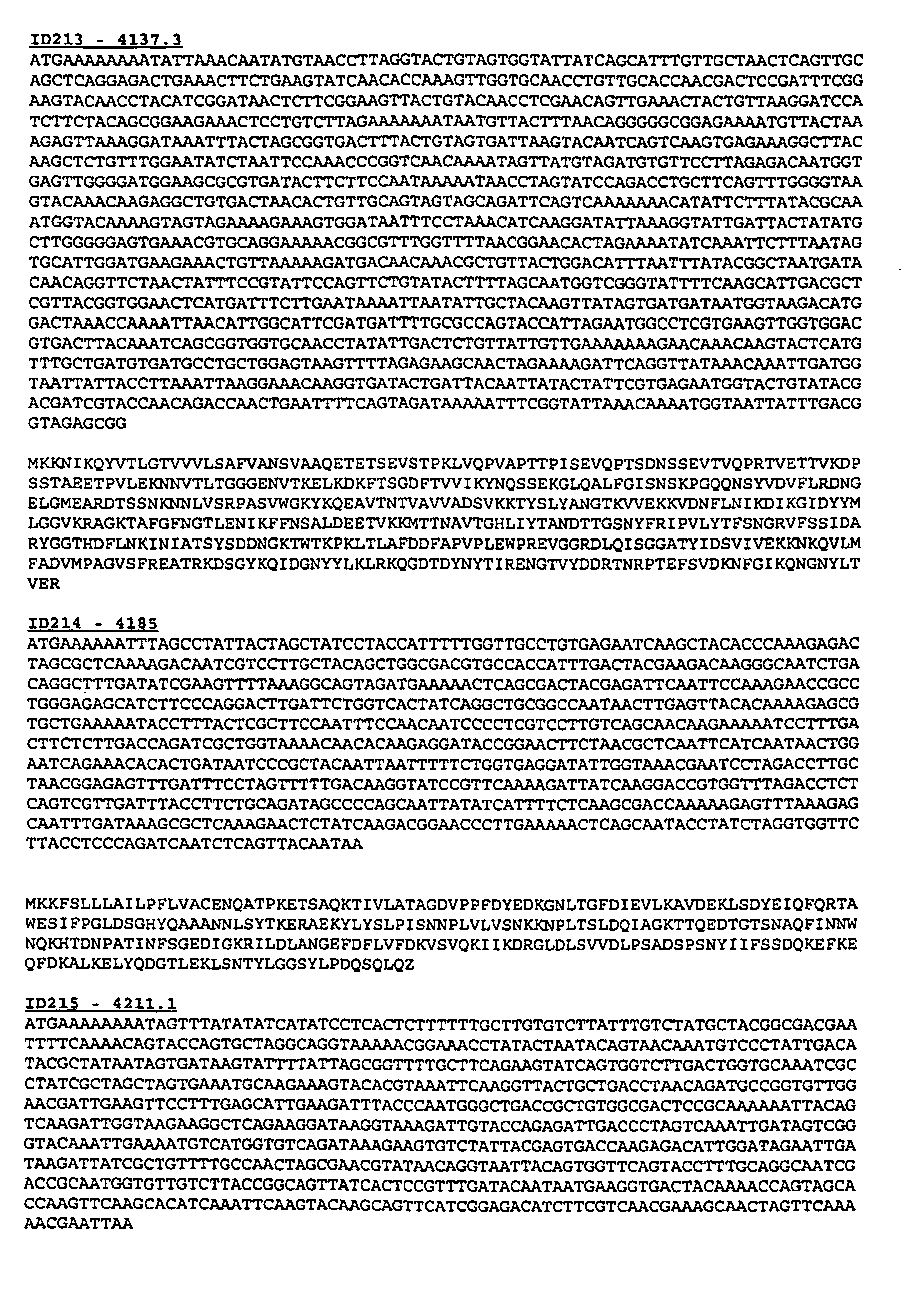

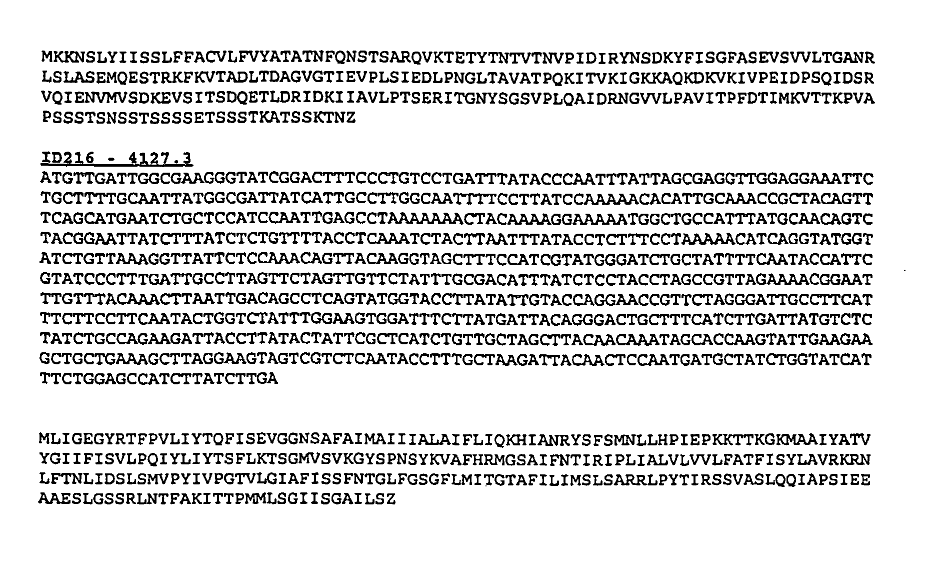

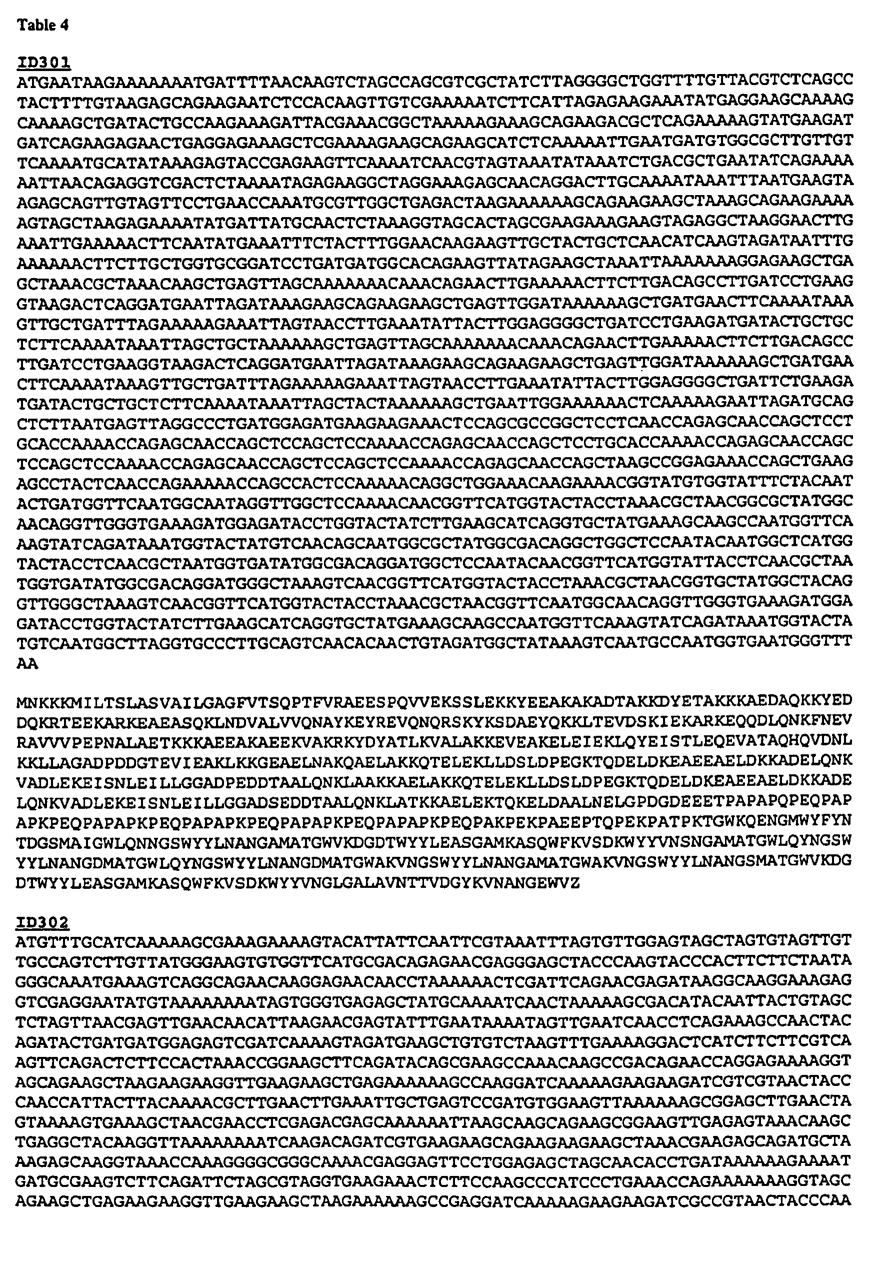

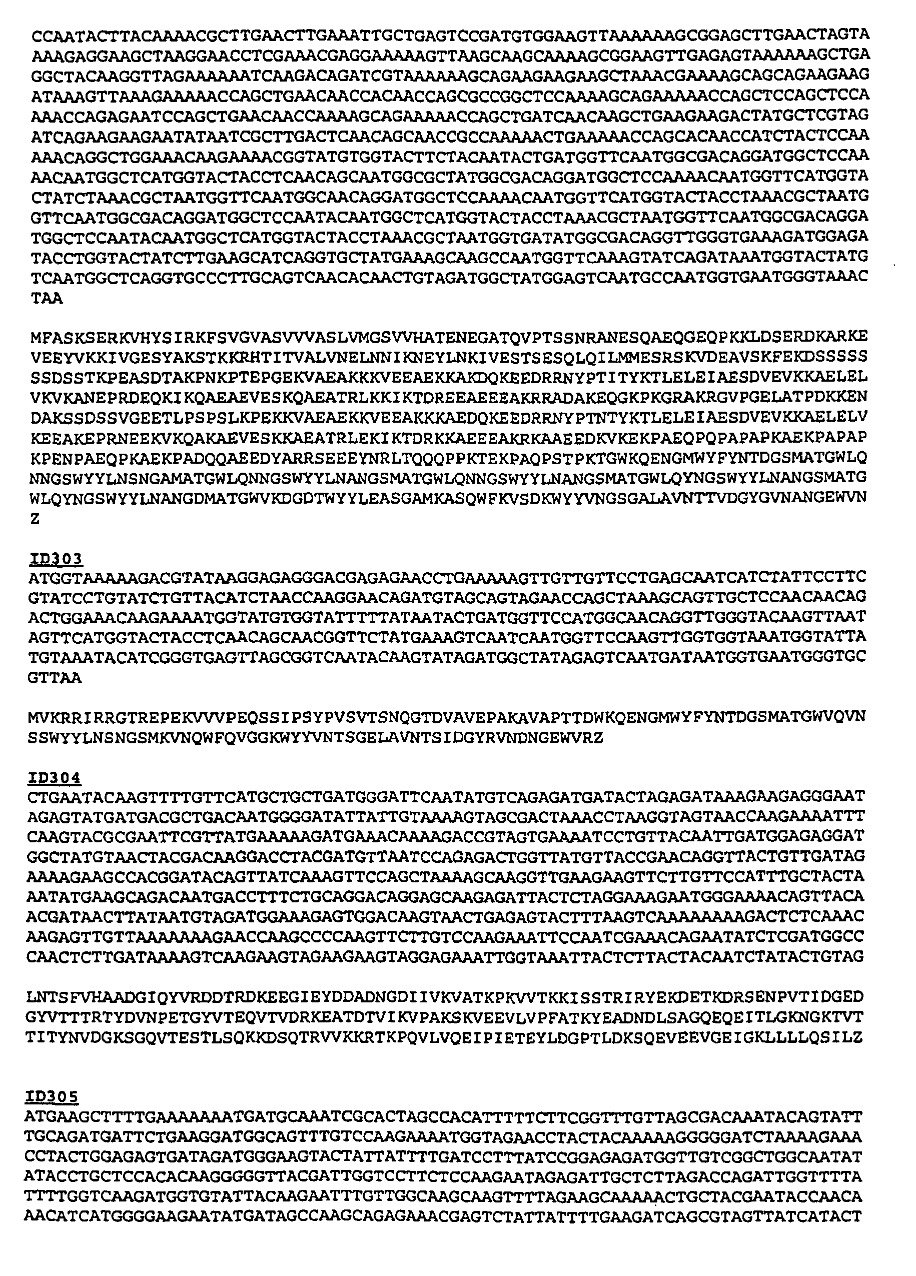

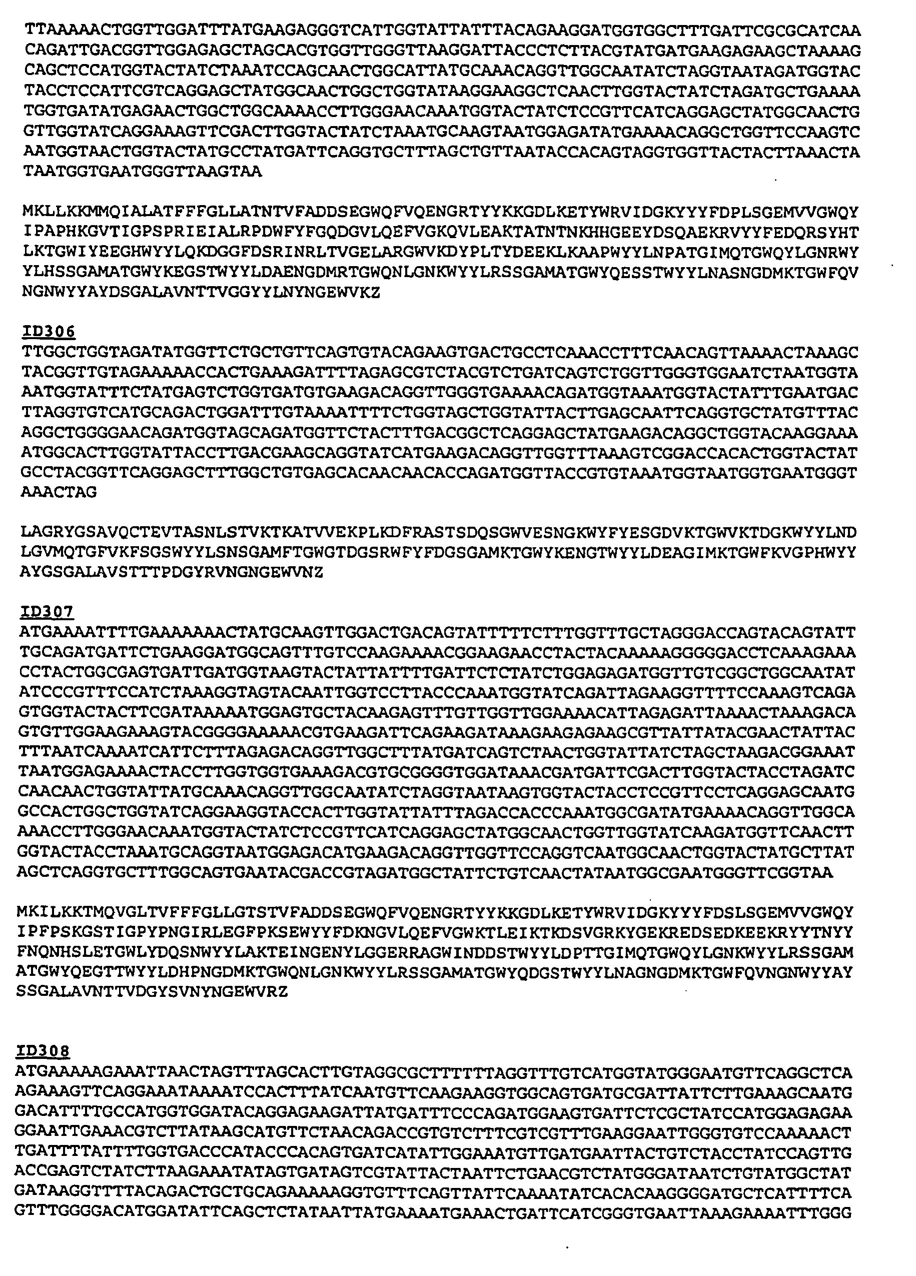

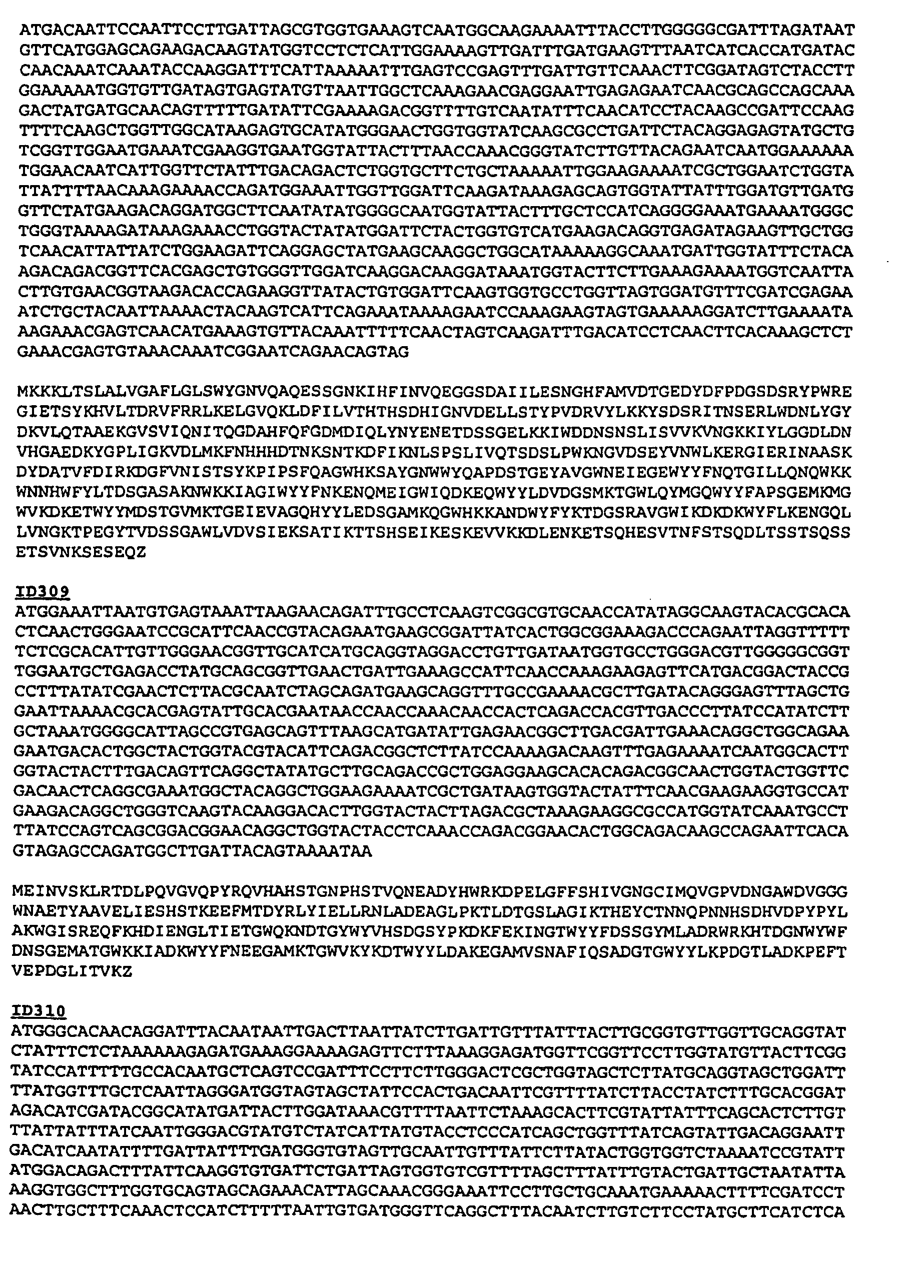

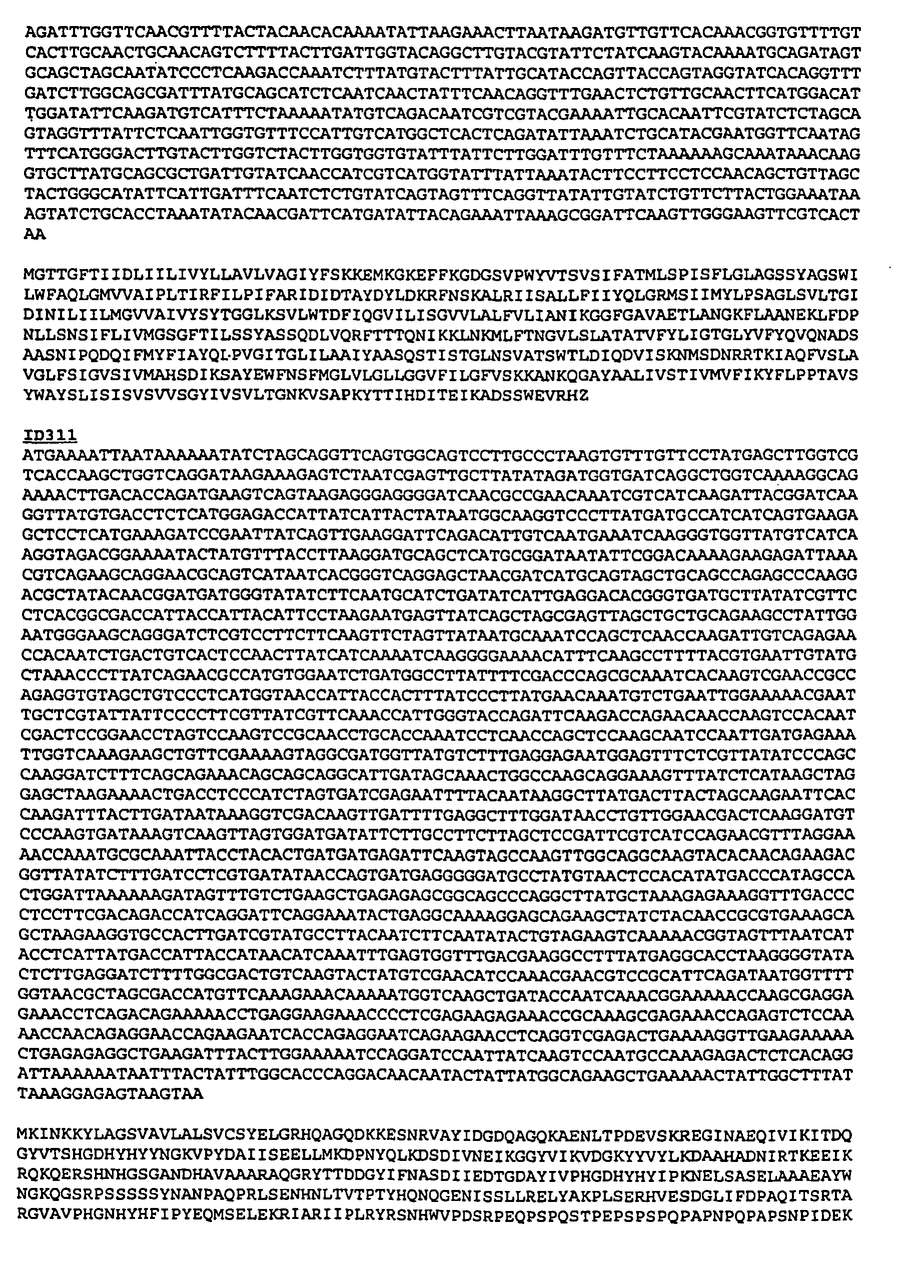

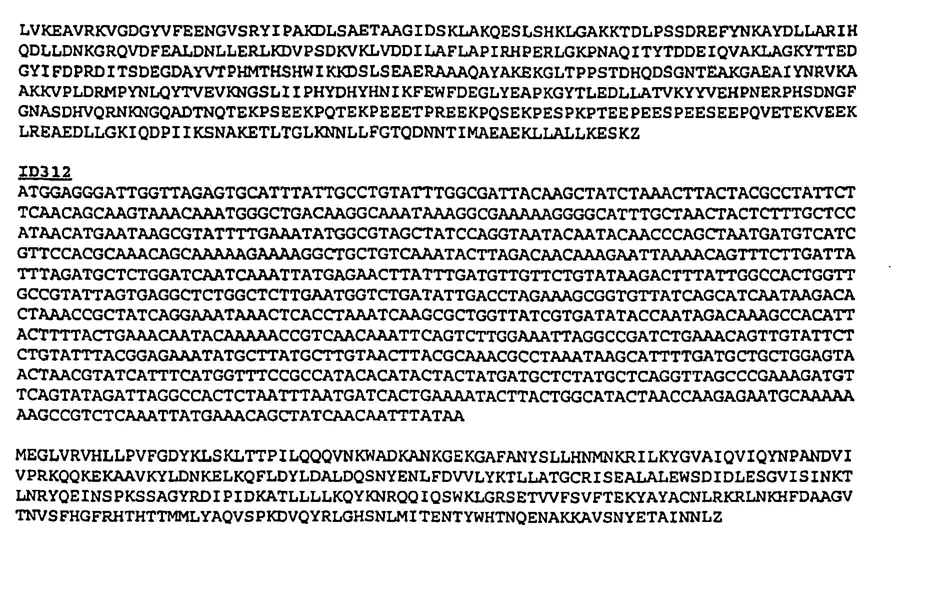

## Claims

1. A *Streptococcus pneumoniae* protein or polypeptide having the following sequence:

MKFSKKYIAAGSAVIVSLSLCAYALNQHRSQENKDNNRVSYVDGSQSSQKSENLTPDQVSQKEGIQAEQIVIKITDQGY
VTSHGDHYHYYNGKVPYDALFSEELLMKDPNYQLKDADIVNEVKGGYIIKVDGKYYVYLKDAAHADNVRTKDEINR
QKQEHVKDNEKVNSNVAVARSQGRYTTNDGYVFNPADIIEDTGNAYIVPHGGHYHYIPKSDLSASELAAAKAHLAGK
NMQPSQLSYSSTASDNNTQSVAKGSTSKPANKSENLQSLLKELYDSPSAQRYSESDGLVFDPAKIISRTPNGVAIPHGDH
YHFIPYSKLSALEEKIARMVPISGTGSTVSTNAKPNEVVSSLGSLSSNPSSLTTSKELSSASDGYIFNPKDIVEETATAYIVR
HGDHFHYIPKSNQIGQPTLPNNSLATPSPSLPINPGTSHEKHEEDGYGFDANRIIAEDESGFVMSHGDHNHYFFKKDLTEE
QIKVRKNI (SEQ ID NO. 38)

2. A protein or polypeptide as claimed in claim 1 provided in substantially pure form.

3. A protein or polypeptide which is substantially identical to one defined in claims 1 or 2.

4. A homologue or derivative of a protein or polypeptide as defined in claims 1 or 2.

5. An antigenic and/or immunogenic fragment of a protein or polypeptide as claimed in any of claims 1 to 4.

6. A nucleic acid molecule comprising or consisting of a sequence which is: (i) 
ATGAAATTTAGTAAAAAATATATAGCAGCTGGATCAGCTGTTATCGTATCCTTGAGTCTATGTGCCT
ATGCACTAAACCAGCATCGTTCGCAGGAAAATAAGGACAATAATCGTGTCTCTTATGTGGATGGCA
GCCAGTCAAGTCAGAAAAGTGAAAACTTGACACCAGACCAGGTTAGCCAGAAAGAAGGAATTCAG
GCTGAGCAAATTGTAATCAAAATTACAGATCAGGGCTATGTAACGTCACACGGTGACCACTATCAT
TACTATAATGGGAAAGTTCCTTATGATGCCCTCTTTAGTGAAGAACTCTTGATGAAGGATCCAAACT
ATCAACTTAAAGACGCTGATATTGTCAATGAAGTCAAGGGTGGTTATATCATCAAGGTCGATGGAA
AATATTATGTCTACCTGAAAGATGCAGCTCATGCTGATAATGTTCGAACTAAAGATGAAATCAATCG
TCAAAAACAAGAACATGTCAAAGATAATGAGAAGGTTAACTCTAATGTTGCTGTAGCAAGGTCTCA
GGGACGATATACGACAAATGATGGTTATGTCTTTAATCCAGCTGATATTATCGAAGATACGGGTAAT
GCTTATATCGTTCCTCATGGAGGTCACTATCACTACATTCCCAAAAGCGATTTATCTGCTAGTGAATT
AGCAGCAGCTAAAGCACATCTGGCTGGAAAAAATATGCAACCGAGTCAGTTAAGCTATTCTTCAAC
AGCTAGTGACAATAACACGCAATCTGTAGCAAAAGGATCAACTAGCAAGCCAGCAAATAAATCTGA
AAATCTCCAGAGTCTTTTGAAGGAACTCTATGATTCACCTAGCGCCCAACGTTACAGTGAATCAGAT
GGCCTGGTCTTTGACCCTGCTAAGATTATCAGTCGTACACCAAATGGAGTTGCGATTCCGCATGGCG
ACCATTACCACTTTATTCCTTACAGCAAGCTTTCTGCCTTAGAAGAAAAGATTGCCAGAATGGTGCC
TATCAGTGGAACTGGTTCTACAGTTTCTACAAATGCAAAACCTAATGAAGTAGTGTCTAGTCTAGGC
AGTCTTTCAAGCAATCCTTCTTCTTTAACGACAAGTAAGGAGCTCTCTTCAGCATCTGATGGTTATAT
 
TTTTAATCCAAAAGATATCGTTGAAGAAACGGCTACAGCTTATATTGTAAGACATGGTGATCATTTC
CATTACATTCCAAAATCAAATCAAATTGGGCAACCGACTCTTCCAAACAATAGTCTAGCAACACCTT
CTCCATCTCTTCCAATCAATCCAGGAACTTCACATGAGAAACATGAAGAAGATGGATACGGATTTG
ATGCTAATCGTATTATCGCTGAAGATGAATCAGGTTTTGTCATGAGTCACGGAGACCACAATCATTA
TTTCTTCAAGAAGGACTTGACAGAAGAGCAAATTAAGGTGCGCAAAAACATTTAG (SEQ ID NO.
246) or its RNA equivalents;
 or its RNA equivalents;
(ii) a sequence which is complementary to any of the sequences of (i);
(iii) a sequence which codes for the same protein or polypeptide, as those sequences of (i) or (ii);
(iv) a sequence which is substantially identical with any of those of (i), (ii) and (iii);
(v) a sequence which codes for a homologue, derivative or fragment of a protein as defined in Table 1.

7. The use of a protein or polypeptide having a sequence selected from those claimed in any one of claims 1 - 3 or homologues or derivatives as claimed in claim 4 and/or fragments thereof as claimed in claim 5, as an immunogen and/or antigen.

8. An immunogenic and/or antigenic composition comprising one or more proteins or polypeptides selected from those claimed in any one of claims 1 - 5 or homologues or derivatives thereof as claimed in claim 4, and/or fragments of any of these as claimed in claim 5.

9. An immunogenic and/or antigenic composition as claimed in claim 8 which is a vaccine or is for use in a diagnostic assay.

10. A vaccine as claimed in claim 9 which comprises one or more additional components selected from excipients, diluents, adjuvants or the like.

11. A vaccine composition comprising one or more nucleic acid sequences as claimed in claim 6.

12. A method for the detection/diagnosis of *S.pneumoniae* which comprises the step of bringing into contact a sample to be tested with at least one protein or polypeptide as claimed in any of claims 1 - 3or homologue or derivative as claimed in claim 4 or fragment thereof as claimed in claim 5.

13. An antibody capable of binding to a protein or polypeptide as claimed in any of claims 1 - 3, or a homologue or derivative as claimed in claim 4 or fragment thereof as claimed in claim 5.

14. An antibody as defined in claim 13 which is a monoclonal antibody.

15. A method for the detection/diagnosis of *S.pneumoniae* which comprises the step of bringing into contact a sample to be tested and at least one antibody as defined in claim 13 or claim 14.

16. A method for the detection/diagnosis of *S.pneumoniae* which comprises the step of bringing into contact a sample to be tested with at least one nucleic acid sequence as defined in claim6.

17. A method of determining whether a protein or polypeptide as claimed in any of claims 1 -5 represents a potential anti-microbial target which comprises inactivating said protein or polypeptide and determining whether *S.pneumoniae* is still viable.

18. The use of an agent capable of antagonising, inhibiting or otherwise interfering with the function or expression of a protein or polypeptide as defined in Tables 1-3 in the manufacture of a medicament for use in the treatment or prophylaxis of *S.pneumoniae* infection
